# EUROPEAN PATENT SPECIFICATION

(11) **EP 2 475 679 B1**
(45) Date of publication and mention of the grant of the patent: **10.02.2016**
(21) Application number: 10790007.8
(22) Date of filing: 14.06.2010
(51) Int. Cl.: C07K 14/195, C12N 15/82

(54) **IMPROVED OPTIMIZED STRAINS OF YARROWIA LIPOLYTICA FOR HIGH EICOSAPENTAENOIC ACID PRODUCTION**
VERBESSERTE OPTIMIERTE YARROWIA LIPOLYTICA-STÄMME ZUR HERSTELLUNG EINER HOCHKONZENTRIERTEN EICOSAPENTAENSÄURE
SOUCHES OPTIMISÉES AMÉLIORÉES DE YARROWIA LIPOLYTICA POUR UNE PRODUCTION ÉLEVÉE D'ACIDE ÉICOSAPENTAÉNOÏQUE

(30) Priority: 16.06.2009 US 187366 P
(43) Date of publication of application: 18.07.2012
(73) Proprietor: E. I. du Pont de Nemours and Company, Wilmington, DE 19805 (US)
(72) Inventor: HONG, Seung-Pyo, Hockessin, Delaware 19707 (US); SHARPE, Pamela, L., Wilmington, Delaware 19808 (US); XUE, Zhixiong, Chadds Ford, Pennsylvania 19317-9728 (US); YADAV, Narendra, S., Wilmington, Delaware 19803 (US); ZHU, Qun, West Chester, Pennsylvania 19382 (US)
(74) Representative: Dzieglewska, Hanna Eva
(86) International application number: PCT/US2010/038535
(87) International publication number: WO 2010/147904

(56) References cited:
- WO-A1-2009/046231
- US-A1- 2006 094 092
- US-A1- 2006 168 687
- US-A1- 2008 254 191
- US-A1- 2009 082 286
- US-A1- 2009 093 543
- DATABASE UniProt [Online] 16 August 2004 (2004-08-16), "SubName: Full=YALI0F19514p;", XP002692015, retrieved from EBI accession no. UNIPROT:Q6C138 Database accession no. Q6C138
- YOUNG ET AL.: 'UniProtKB_Q1 MKL6: Malonyl CoA synthetase' RHIZOBIUM LEGUMINOSARUM BV. VICIAE 3841, [Online] 30 May 2006, Retrieved from the Internet: <URL:http://www.uniprot.org/uniprot/Q1MKL6> [retrieved on 2010-09-20]

### SEQUENCE LISTING

<110> E.I. duPont de Nemours & Company, Inc. Zhu, Quinn
<120> IMPROVED OPTIMIZED STRAINS OF YARROWIA LIPOLYTICA FOR HIGH EICOSAPENTAENOIC ACID PRODUCTION
<130> CL4674
<150> US 61/187366
   <151> 2009-06-16
<150> US 61/187368
   <151> 2009-06-16
<150> US 61/187359
   <151> 2009-06-16
<160> 156
<170> PatentIn version 3.5
<210> 1
   <211> 24
   <212> PRT
   <213> Artificial Sequence
<220>
   <223> Multizyme linker
<300>
   <302> MULTIZYMES AND THEIR USE IN MAKING POLYUNSATURATED FATTY ACIDS
<310> WO 2008/124048
   <311> 2008-04-03
   <312> 2008-10-16
   <313> (1)..(24)
<300>
   <302> MULTIZYMES AND THEIR USE IN MAKING POLYUNSATURATED FATTY ACIDS
<310> US-2008-0254191-A1
   <311> 2008-04-03
   <312> 2008-10-16
   <313> (1)..(24)
<400> 1
   Gly Ala Gly Pro Ala Arg Pro Ala Gly Leu Pro Pro Ala Thr Tyr Tyr
   1               5                   10                  15
   Asp Ser Leu Ala Val Met Gly Ser
               20
<210> 2
   <211> 19
   <212> PRT
   <213> Artificial Sequence
<220>
   <223> Multizyme linker
<300>
   <302> MULTIZYMES AND THEIR USE IN MAKING POLYUNSATURATED FATTY ACIDS
<310> US-2008-0254191-A1
   <311> 2008-04-03
   <312> 2008-10-16
   <313> (1)..(19)
<300>
   <302> MULTIZYMES AND THEIR USE IN MAKING POLYUNSATURATED FATTY ACIDS
<310> WO 2008/124048
   <311> 2008-04-03
   <312> 2008-10-16
   <313> (1)..(19)
<400> 2 Leu Ala Val
<210> 3
   <211> 18
   <212> PRT
   <213> Artificial Sequence
<220>
   <223> Multizyme linker
<300>
   <302> MULTIZYMES AND THEIR USE IN MAKING POLYUNSATURATED FATTY ACIDS
<310> WO 2008/124048
   <311> 2008-04-03
   <312> 2008-10-16
   <313> (1)..(18)
<300>
   <302> MULTIZYMES AND THEIR USE IN MAKING POLYUNSATURATED FATTY ACIDS
<310> US-2008-0254191-A1
   <311> 2008-04-03
   <312> 2008-10-16
   <313> (1)..(18)
<400> 3 Ala Val
<210> 4
   <211> 18
   <212> PRT
   <213> Artificial Sequence
<220>
   <223> Multizyme linker
<300>
   <302> MULTIZYMES AND THEIR USE IN MAKING POLYUNSATURATED FATTY ACIDS
<310> WO 2008/124048
   <311> 2008-04-03
   <312> 2008-10-16
   <313> (1)..(18)
<300>
   <302> MULTIZYMES AND THEIR USE IN MAKING POLYUNSATURATED FATTY ACIDS
<310> US-2008-0254191-A1
   <311> 2008-04-03
   <312> 2008-10-16
   <313> (1)..(18)
<400> 4 Ala Val
<210> 5
   <211> 33
   <212> PRT
   <213> Artificial Sequence
<220>
   <223> Multizyme linker
<300>
   <302> MULTIZYMES AND THEIR USE IN MAKING POLYUNSATURATED FATTY ACIDS
<310> WO 2008/124048
   <311> 2008-04-03
   <312> 2008-10-16
   <313> (1)..(33)
<300>
   <302> MULTIZYMES AND THEIR USE IN MAKING POLYUNSATURATED FATTY ACIDS
<310> US-2008-0254191-A1
   <311> 2008-04-03
   <312> 2008-10-16
   <313> (1)..(33)
<400> 5 Thr
<210> 6
   <211> 22
   <212> PRT
   <213> Artificial Sequence
<220>
   <223> Multizyme linker
<300>
   <302> MULTIZYMES AND THEIR USE IN MAKING POLYUNSATURATED FATTY ACIDS
<310> WO 2008/124048
   <311> 2008-04-03
   <312> 2008-10-16
   <313> (1)..(22)
<300>
   <302> MULTIZYMES AND THEIR USE IN MAKING POLYUNSATURATED FATTY ACIDS
<310> US-2008-0254191-A1
   <311> 2008-04-03
   <312> 2008-10-16
   <313> (1)..(22)
<400> 6
   Pro Ala Arg Pro Ala Gly Leu Pro Pro Ala Thr Tyr Tyr Asp Ser Leu
   1               5                   10                  15
   Ala Val Ser Gly Arg Thr
               20
<210> 7
   <211> 36
   <212> PRT
   <213> Artificial Sequence
<220>
   <223> Multizyme linker
<300>
   <302> MULTIZYMES AND THEIR USE IN MAKING POLYUNSATURATED FATTY ACIDS
<310> WO 2008/124048
   <311> 2008-04-03
   <312> 2008-10-16
   <313> (1)..(36)
<300>
   <302> MULTIZYMES AND THEIR USE IN MAKING POLYUNSATURATED FATTY ACIDS
<310> US-2008-0254191-A1
   <311> 2008-04-03
   <312> 2008-10-16
   <313> (1)..(36)
<400> 7
   Pro Gly Gly Pro Gly Lys Pro Ser Glu Ile Ala Ser Leu Pro Pro Pro
   1               5                   10                  15
   Ile Arg Pro Val Gly Asn Pro Pro Ala Ala Tyr Tyr Asp Ala Leu Ala
               20                  25                  30
   Thr Gly Arg Thr
           35
<210> 8
   <211> 2112
   <212> DNA
   <213> Artificial Sequence
<220>
   <223> Multizyme: EgD9ES/EgD8M gene fusion
<220>
   <221> CDS
   <222> (1)..(2112)
   <223> Multizyme: EgD9ES/EgD8M gene fusion
<300>
   <302> MULTIZYMES AND THEIR USE IN MAKING POLYUNSATURATED FATTY ACIDS
<310> US-2008-0254191-A1
   <311> 2008-04-03
   <312> 2008-10-16
   <313> (1)..(2112)
<300>
   <302> MULTIZYMES AND THEIR USE IN MAKING POLYUNSATURATED FATTY ACIDS
<310> WO 2008/124048
   <311> 2008-04-03
   <312> 2008-10-16
   <313> (1)..(2112)
<400> 8
   atg gag gtc gtg aac gaa atc gtc tcc att ggc cag gag gtt ctt ccc        48
   Met Glu Val Val Asn Glu Ile Val Ser Ile Gly Gln Glu Val Leu Pro
   1               5                   10                  15
   aag gtc gac tat gct cag ctc tgg tct gat gcc tcg cac tgc gag gtg        96
   Lys Val Asp Tyr Ala Gln Leu Trp Ser Asp Ala Ser His Cys Glu Val
               20                  25                  30
   ctg tac ctc tcc atc gcc ttc gtc atc ctg aag ttc acc ctt ggt cct       144
   Leu Tyr Leu Ser Ile Ala Phe Val Ile Leu Lys Phe Thr Leu Gly Pro
           35                  40                  45
   ctc gga ccc aag ggt cag tct cga atg aag ttt gtg ttc acc aac tac       192
   Leu Gly Pro Lys Gly Gln Ser Arg Met Lys Phe Val Phe Thr Asn Tyr
       50                  55                  60
   aac ctg ctc atg tcc atc tac tcg ctg ggc tcc ttc ctc tct atg gcc       240
   Asn Leu Leu Met Ser Ile Tyr Ser Leu Gly Ser Phe Leu Ser Met Ala
   65                  70                  75                  80
   tac gcc atg tac acc att ggt gtc atg tcc gac aac tgc gag aag gct       288
   Tyr Ala Met Tyr Thr Ile Gly Val Met Ser Asp Asn Cys Glu Lys Ala
                   85                  90                  95
   ttc gac aac aat gtc ttc cga atc acc act cag ctg ttc tac ctc agc       336
   Phe Asp Asn Asn Val Phe Arg Ile Thr Thr Gln Leu Phe Tyr Leu Ser
               100                 105                 110
   aag ttc ctc gag tac att gac tcc ttc tat ctg ccc ctc atg ggc aag       384
   Lys Phe Leu Glu Tyr Ile Asp Ser Phe Tyr Leu Pro Leu Met Gly Lys
           115                 120                 125
   cct ctg acc tgg ttg cag ttc ttt cac cat ctc gga gct cct atg gac       432
   Pro Leu Thr Trp Leu Gln Phe Phe His His Leu Gly Ala Pro Met Asp
       130                 135                 140
   atg tgg ctg ttc tac aac tac cga aac gaa gcc gtt tgg atc ttt gtg       480
   Met Trp Leu Phe Tyr Asn Tyr Arg Asn Glu Ala Val Trp Ile Phe Val
   145                 150                 155                 160
   ctg ctc aac ggc ttc att cac tgg atc atg tac ggc tac tat tgg acc       528
   Leu Leu Asn Gly Phe Ile His Trp Ile Met Tyr Gly Tyr Tyr Trp Thr
                   165                 170                 175
   cga ctg atc aag ctc aag ttc cct atg ccc aag tcc ctg att act tct        576
   Arg Leu Ile Lys Leu Lys Phe Pro Met Pro Lys Ser Leu Ile Thr Ser
               180                 185                 190
   atg cag atc att cag ttc aac gtt ggc ttc tac atc gtc tgg aag tac        624
   Met Gln Ile Ile Gln Phe Asn Val Gly Phe Tyr Ile Val Trp Lys Tyr
           195                 200                 205
   cgg aac att ccc tgc tac cga caa gat gga atg aga atg ttt ggc tgg        672
   Arg Asn Ile Pro Cys Tyr Arg Gln Asp Gly Met Arg Met Phe Gly Trp
       210                 215                 220
   ttt ttc aac tac ttc tac gtt ggt act gtc ctg tgt ctg ttc ctc aac        720
   Phe Phe Asn Tyr Phe Tyr Val Gly Thr Val Leu Cys Leu Phe Leu Asn
   225                 230                 235                 240
   ttc tac gtg cag acc tac atc gtc cga aag cac aag gga gcc aaa aag        768
   Phe Tyr Val Gln Thr Tyr Ile Val Arg Lys His Lys Gly Ala Lys Lys
                   245                 250                 255
   att cag ggc gcc ggt ccc gct cga cct gcc gga ctt cct ccc gct acc        816
   Ile Gln Gly Ala Gly Pro Ala Arg Pro Ala Gly Leu Pro Pro Ala Thr
               260                 265                 270
   tac tac gac tct ctg gcc gtc atg gga tcc gtg aag gct tct cga cag        864
   Tyr Tyr Asp Ser Leu Ala Val Met Gly Ser Val Lys Ala Ser Arg Gln
           275                 280                 285
   gct ctg ccc ctc gtc atc gac gga aag gtg tac gac gtc tcc gct tgg        912
   Ala Leu Pro Leu Val Ile Asp Gly Lys Val Tyr Asp Val Ser Ala Trp
       290                 295                 300
   gtg aac ttc cac cct ggt gga gct gaa atc att gag aac tac cag gga        960
   Val Asn Phe His Pro Gly Gly Ala Glu Ile Ile Glu Asn Tyr Gln Gly
   305                 310                 315                 320
   cga gat gct act gac gcc ttc atg gtt atg cac tct cag gaa gcc ttc       1008
   Arg Asp Ala Thr Asp Ala Phe Met Val Met His Ser Gln Glu Ala Phe
                   325                 330                 335
   gac aag ctc aag cga atg ccc aag atc aac cag gct tcc gag ctg cct       1056
   Asp Lys Leu Lys Arg Met Pro Lys Ile Asn Gln Ala Ser Glu Leu Pro
               340                 345                 350
   ccc cag gct gcc gtc aac gaa gct cag gag gat ttc cga aag ctc cga       1104
   Pro Gln Ala Ala Val Asn Glu Ala Gln Glu Asp Phe Arg Lys Leu Arg
           355                 360                 365
   gaa gag ctg atc gcc act ggc atg ttt gac gcc tct ccc ctc tgg tac       1152
   Glu Glu Leu Ile Ala Thr Gly Met Phe Asp Ala Ser Pro Leu Trp Tyr
       370                 375                 380
   tcg tac aag atc ttg acc acc ctg ggt ctt ggc gtg ctt gcc ttc ttc       1200
   Ser Tyr Lys Ile Leu Thr Thr Leu Gly Leu Gly Val Leu Ala Phe Phe
   385                 390                 395                 400
   atg ctg gtc cag tac cac ctg tac ttc att ggt gct ctc gtg ctc ggt       1248
   Met Leu Val Gln Tyr His Leu Tyr Phe Ile Gly Ala Leu Val Leu Gly
                   405                 410                 415
   atg cac tac cag caa atg gga tgg ctg tct cat gac atc tgc cac cac       1296
   Met His Tyr Gln Gln Met Gly Trp Leu Ser His Asp Ile Cys His His
               420                 425                 430
   cag acc ttc aag aac cga aac tgg aat aac gtc ctg ggt ctg gtc ttt        1344
   Gln Thr Phe Lys Asn Arg Asn Trp Asn Asn Val Leu Gly Leu Val Phe
           435                 440                 445
   ggc aac gga ctc cag ggc ttc tcc gtg acc tgg tgg aag gac aga cac        1392
   Gly Asn Gly Leu Gln Gly Phe Ser Val Thr Trp Trp Lys Asp Arg His
       450                 455                 460
   aac gcc cat cat tct gct acc aac gtt cag ggt cac gat ccc gac att        1440
   Asn Ala His His Ser Ala Thr Asn Val Gln Gly His Asp Pro Asp Ile
   465                 470                 475                 480
   gat aac ctg cct ctg ctc gcc tgg tcc gag gac gat gtc act cga gct        1488
   Asp Asn Leu Pro Leu Leu Ala Trp Ser Glu Asp Asp Val Thr Arg Ala
                   485                 490                 495
   tct ccc atc tcc cga aag ctc att cag ttc caa cag tac tat ttc ctg        1536
   Ser Pro Ile Ser Arg Lys Leu Ile Gln Phe Gln Gln Tyr Tyr Phe Leu
               500                 505                 510
   gtc atc tgt att ctc ctg cga ttc atc tgg tgt ttc cag tct gtg ctg        1584
   Val Ile Cys Ile Leu Leu Arg Phe Ile Trp Cys Phe Gln Ser Val Leu
           515                 520                 525
   acc gtt cga tcc ctc aag gac cga gac aac cag ttc tac cga tct cag        1632
   Thr Val Arg Ser Leu Lys Asp Arg Asp Asn Gln Phe Tyr Arg Ser Gln
       530                 535                 540
   tac aag aaa gag gcc att gga ctc gct ctg cac tgg act ctc aag acc        1680
   Tyr Lys Lys Glu Ala Ile Gly Leu Ala Leu His Trp Thr Leu Lys Thr
   545                 550                 555                 560
   ctg ttc cac ctc ttc ttt atg ccc tcc atc ctg acc tcg atg ctg gtg        1728
   Leu Phe His Leu Phe Phe Met Pro Ser Ile Leu Thr Ser Met Leu Val
                   565                 570                 575
   ttc ttt gtt tcc gag ctc gtc ggt ggc ttc gga att gcc atc gtg gtc        1776
   Phe Phe Val Ser Glu Leu Val Gly Gly Phe Gly Ile Ala Ile Val Val
               580                 585                 590
   ttc atg aac cac tac cct ctg gag aag atc ggt gat tcc gtc tgg gac        1824
   Phe Met Asn His Tyr Pro Leu Glu Lys Ile Gly Asp Ser Val Trp Asp
           595                 600                 605
   gga cat ggc ttc tct gtg ggt cag atc cat gag acc atg aac att cga        1872
   Gly His Gly Phe Ser Val Gly Gln Ile His Glu Thr Met Asn Ile Arg
       610                 615                 620
   cga ggc atc att act gac tgg ttc ttt gga ggc ctg aac tac cag atc        1920
   Arg Gly Ile Ile Thr Asp Trp Phe Phe Gly Gly Leu Asn Tyr Gln Ile
   625                 630                 635                 640
   gag cac cat ctc tgg ccc acc ctg cct cga cac aac ctc act gcc gtt        1968
   Glu His His Leu Trp Pro Thr Leu Pro Arg His Asn Leu Thr Ala Val
                   645                 650                 655
   tcc tac cag gtg gaa cag ctg tgc cag aag cac aac ctc ccc tac cga        2016
   Ser Tyr Gln Val Glu Gln Leu Cys Gln Lys His Asn Leu Pro Tyr Arg
               660                 665                 670
   aac cct ctg ccc cat gaa ggt ctc gtc atc ctg ctc cga tac ctg tcc        2064
   Asn Pro Leu Pro His Glu Gly Leu Val Ile Leu Leu Arg Tyr Leu Ser
           675                 680                 685
   cag ttc gct cga atg gcc gag aag cag ccc ggt gcc aag gct cag taa        2112
   Gln Phe Ala Arg Met Ala Glu Lys Gln Pro Gly Ala Lys Ala Gln
       690                 695                 700
<210> 9
   <211> 703
   <212> PRT
   <213> Artificial Sequence
<220>
   <223> Synthetic Construct
<400> 9
   Met Glu Val Val Asn Glu Ile Val Ser Ile Gly Gln Glu Val Leu Pro
   1               5                   10                  15
   Lys Val Asp Tyr Ala Gln Leu Trp Ser Asp Ala Ser His Cys Glu Val
               20                  25                  30
   Leu Tyr Leu Ser Ile Ala Phe Val Ile Leu Lys Phe Thr Leu Gly Pro
           35                  40                  45
   Leu Gly Pro Lys Gly Gln Ser Arg Met Lys Phe Val Phe Thr Asn Tyr
       50                  55                  60
   Asn Leu Leu Met Ser Ile Tyr Ser Leu Gly Ser Phe Leu Ser Met Ala
   65                  70                  75                  80
   Tyr Ala Met Tyr Thr Ile Gly Val Met Ser Asp Asn Cys Glu Lys Ala
                   85                  90                  95
   Phe Asp Asn Asn Val Phe Arg Ile Thr Thr Gln Leu Phe Tyr Leu Ser
               100                 105                 110
   Lys Phe Leu Glu Tyr Ile Asp Ser Phe Tyr Leu Pro Leu Met Gly Lys
           115                 120                 125
   Pro Leu Thr Trp Leu Gln Phe Phe His His Leu Gly Ala Pro Met Asp
       130                 135                 140
   Met Trp Leu Phe Tyr Asn Tyr Arg Asn Glu Ala Val Trp Ile Phe Val
   145                 150                 155                 160
   Leu Leu Asn Gly Phe Ile His Trp Ile Met Tyr Gly Tyr Tyr Trp Thr
                   165                 170                 175
   Arg Leu Ile Lys Leu Lys Phe Pro Met Pro Lys Ser Leu Ile Thr Ser
               180                 185                 190
   Met Gln Ile Ile Gln Phe Asn Val Gly Phe Tyr Ile Val Trp Lys Tyr
   195 200 205
   Arg Asn Ile Pro Cys Tyr Arg Gln Asp Gly Met Arg Met Phe Gly Trp
   210 215 220
   Phe Phe Asn Tyr Phe Tyr Val Gly Thr Val Leu Cys Leu Phe Leu Asn
   225 230 235 240
   Phe Tyr Val Gln Thr Tyr Ile Val Arg Lys His Lys Gly Ala Lys Lys
   245 250 255
   Ile Gln Gly Ala Gly Pro Ala Arg Pro Ala Gly Leu Pro Pro Ala Thr
   260 265 270
   Tyr Tyr Asp Ser Leu Ala Val Met Gly Ser Val Lys Ala Ser Arg Gln
   275 280 285
   Ala Leu Pro Leu Val Ile Asp Gly Lys Val Tyr Asp Val Ser Ala Trp
   290 295 300
   Val Asn Phe His Pro Gly Gly Ala Glu Ile Ile Glu Asn Tyr Gln Gly
   305 310 315 320
   Arg Asp Ala Thr Asp Ala Phe Met Val Met His Ser Gln Glu Ala Phe
   325 330 335
   Asp Lys Leu Lys Arg Met Pro Lys Ile Asn Gln Ala Ser Glu Leu Pro
   340 345 350
   Pro Gln Ala Ala Val Asn Glu Ala Gln Glu Asp Phe Arg Lys Leu Arg
   355 360 365
   Glu Glu Leu Ile Ala Thr Gly Met Phe Asp Ala Ser Pro Leu Trp Tyr
   370 375 380
   Ser Tyr Lys Ile Leu Thr Thr Leu Gly Leu Gly Val Leu Ala Phe Phe
   385 390 395 400
   Met Leu Val Gln Tyr His Leu Tyr Phe Ile Gly Ala Leu Val Leu Gly
   405 410 415
   Met His Tyr Gln Gln Met Gly Trp Leu Ser His Asp Ile Cys His His
   420 425 430
   Gln Thr Phe Lys Asn Arg Asn Trp Asn Asn Val Leu Gly Leu Val Phe
   435 440 445
   Gly Asn Gly Leu Gln Gly Phe Ser Val Thr Trp Trp Lys Asp Arg His
   450 455 460
   Asn Ala His His Ser Ala Thr Asn Val Gln Gly His Asp Pro Asp Ile
   465 470 475 480
   Asp Asn Leu Pro Leu Leu Ala Trp Ser Glu Asp Asp Val Thr Arg Ala
   485 490 495
   Ser Pro Ile Ser Arg Lys Leu Ile Gln Phe Gln Gln Tyr Tyr Phe Leu
   500 505 510
   Val Ile Cys Ile Leu Leu Arg Phe Ile Trp Cys Phe Gln Ser Val Leu
   515 520 525
   Thr Val Arg Ser Leu Lys Asp Arg Asp Asn Gln Phe Tyr Arg Ser Gln
   530 535 540
   Tyr Lys Lys Glu Ala Ile Gly Leu Ala Leu His Trp Thr Leu Lys Thr
   545 550 555 560
   Leu Phe His Leu Phe Phe Met Pro Ser Ile Leu Thr Ser Met Leu Val
   565 570 575
   Phe Phe Val Ser Glu Leu Val Gly Gly Phe Gly Ile Ala Ile Val Val
   580 585 590
   Phe Met Asn His Tyr Pro Leu Glu Lys Ile Gly Asp Ser Val Trp Asp
   595 600 605
   Gly His Gly Phe Ser Val Gly Gln Ile His Glu Thr Met Asn Ile Arg
   610 615 620
   Arg Gly Ile Ile Thr Asp Trp Phe Phe Gly Gly Leu Asn Tyr Gln Ile
   625 630 635 640
   Glu His His Leu Trp Pro Thr Leu Pro Arg His Asn Leu Thr Ala Val
   645 650 655
   Ser Tyr Gln Val Glu Gln Leu Cys Gln Lys His Asn Leu Pro Tyr Arg
   660 665 670
   Asn Pro Leu Pro His Glu Gly Leu Val Ile Leu Leu Arg Tyr Leu Ser
   675 680 685
   Gln Phe Ala Arg Met Ala Glu Lys Gln Pro Gly Ala Lys Ala Gln
   690 695 700
<210> 10
   <211> 2109
   <212> DNA
   <213> Artificial Sequence
<220>
   <223> Multizyme: EaD9ES/EaD8S gene fusion
<220>
   <221> CDS
   <222> (1) .. (2109)
   <223> Multizyme: EaD9ES/EaD8S gene fusion
<300>
   <302> MULTIZYMES AND THEIR USE IN MAKING POLYUNSATURATED FATTY ACIDS
<310> WO 2008/124048
   <311> 2008-04-03
   <312> 2008-10-16
   <313> (1) .. (2109)
<300>
   <302> MULTIZYMES AND THEIR USE IN MAKING POLYUNSATURATED FATTY ACIDS
<310> US-2008-0254191-A1
   <311> 2008-04-03
   <312> 2008-10-16
   <313> (1) .. (2109)
<210> 11
   <211> 702
   <212> PRT
   <213> Artificial Sequence
<220>
   <223> Synthetic Construct
<400> 11
   Met Glu Ala Ala Lys Glu Leu Val Ser Ile Val Gln Glu Glu Leu Pro
   1               5                   10                  15
   Lys Val Asp Tyr Ala Gln Leu Trp Gln Asp Ala Ser Ser Cys Glu Val
               20                  25                  30
   Leu Tyr Leu Ser Val Ala Phe Val Ala Ile Lys Phe Met Leu Arg Pro
           35                  40                  45
   Leu Asp Leu Lys Arg Gln Ala Thr Leu Lys Lys Leu Phe Thr Ala Tyr
       50                  55                  60
   Asn Phe Leu Met Ser Ile Tyr Ser Phe Gly Ser Phe Leu Ala Met Ala
   65                  70                  75                  80
   Tyr Ala Leu Ser Val Thr Gly Ile Leu Ser Gly Asp Cys Glu Thr Ala
                   85                  90                  95
   Phe Asn Asn Asp Val Phe Arg Ile Thr Thr Gln Leu Phe Tyr Leu Ser
               100                 105                 110
   Lys Phe Val Glu Tyr Ile Asp Ser Phe Tyr Leu Pro Leu Met Asp Lys
           115                 120                 125
   Pro Leu Ser Phe Leu Gln Phe Phe His His Leu Gly Ala Pro Ile Asp
       130                 135                 140
   Met Trp Leu Phe Tyr Lys Tyr Arg Asn Glu Gly Val Trp Ile Phe Val
   145                 150                 155                 160
   Leu Leu Asn Gly Phe Ile His Trp Ile Met Tyr Gly Tyr Tyr Trp Thr
                   165                 170                 175
   Arg Leu Ile Lys Leu Asn Phe Pro Met Pro Lys Asn Leu Ile Thr Ser
               180                 185                 190
   Met Gln Ile Ile Gln Phe Asn Val Gly Phe Tyr Ile Val Trp Lys Tyr
   195 200 205
   Arg Asn Val Pro Cys Tyr Arg Gln Asp Gly Met Arg Met Phe Ala Trp
   210 215 220
   Ile Phe Asn Tyr Trp Tyr Val Gly Thr Val Leu Leu Leu Phe Leu Asn
   225 230 235 240
   Phe Tyr Val Gln Thr Tyr Ile Arg Lys Pro Arg Lys Asn Arg Gly Lys
   245 250 255
   Lys Glu Gly Ala Gly Pro Ala Arg Pro Ala Gly Leu Pro Pro Ala Thr
   260 265 270
   Tyr Tyr Asp Ser Leu Ala Val Met Gly Ser Met Val Lys Arg Pro Ala
   275 280 285
   Leu Pro Leu Thr Val Asp Gly Val Thr Tyr Asp Val Ser Ala Trp Leu
   290 295 300
   Asn His His Pro Gly Gly Ala Asp Ile Ile Glu Asn Tyr Arg Gly Arg
   305 310 315 320
   Asp Ala Thr Asp Val Phe Met Val Met His Ser Glu Asn Ala Val Ser
   325 330 335
   Lys Leu Arg Arg Met Pro Ile Met Glu Pro Ser Ser Pro Leu Thr Pro
   340 345 350
   Thr Pro Pro Lys Pro Asn Ser Asp Glu Pro Gln Glu Asp Phe Arg Lys
   355 360 365
   Leu Arg Asp Glu Leu Ile Ala Ala Gly Met Phe Asp Ala Ser Pro Met
   370 375 380
   Trp Tyr Ala Tyr Lys Thr Leu Ser Thr Leu Gly Leu Gly Val Leu Ala
   385 390 395 400
   Val Leu Leu Met Thr Gln Trp His Trp Tyr Leu Val Gly Ala Ile Val
   405 410 415
   Leu Gly Ile His Phe Gln Gln Met Gly Trp Leu Ser His Asp Ile Cys
   420 425 430
   His His Gln Leu Phe Lys Asp Arg Ser Ile Asn Asn Ala Ile Gly Leu
   435 440 445
   Leu Phe Gly Asn Val Leu Gln Gly Phe Ser Val Thr Trp Trp Lys Asp
       450                 455                 460
   Arg His Asn Ala His His Ser Ala Thr Asn Val Gln Gly His Asp Pro
   465                 470                 475                 480
   Asp Ile Asp Asn Leu Pro Leu Leu Ala Trp Ser Lys Glu Asp Val Glu
                   485                 490                 495
   Arg Ala Gly Pro Phe Ser Arg Arg Met Ile Lys Tyr Gln Gln Tyr Tyr
               500                 505                 510
   Phe Phe Phe Ile Cys Ala Leu Leu Arg Phe Ile Trp Cys Phe Gln Ser
           515                 520                 525
   Ile His Thr Ala Thr Gly Leu Lys Asp Arg Ser Asn Gln Tyr Tyr Arg
       530                 535                 540
   Arg Gln Tyr Glu Lys Glu Ser Val Gly Leu Ala Leu His Trp Gly Leu
   545                 550                 555                 560
   Lys Ala Leu Phe Tyr Tyr Phe Tyr Met Pro Ser Phe Leu Thr Gly Leu
                   565                 570                 575
   Met Val Phe Phe Val Ser Glu Leu Leu Gly Gly Phe Gly Ile Ala Ile
               580                 585                 590
   Val Val Phe Met Asn His Tyr Pro Leu Glu Lys Ile Gln Asp Ser Val
           595                 600                 605
   Trp Asp Gly His Gly Phe Cys Ala Gly Gln Ile His Glu Thr Met Asn
       610                 615                 620
   Val Gln Arg Gly Leu Val Thr Asp Trp Phe Phe Gly Gly Leu Asn Tyr
   625                 630                 635                 640
   Gln Ile Glu His His Leu Trp Pro Thr Leu Pro Arg His Asn Leu Thr
                   645                 650                 655
   Ala Ala Ser Ile Lys Val Glu Gln Leu Cys Lys Lys His Asn Leu Pro
               660                 665                 670
   Tyr Arg Ser Pro Pro Met Leu Glu Gly Val Gly Ile Leu Ile Ser Tyr
           675                 680                 685
   Leu Gly Thr Phe Ala Arg Met Val Ala Lys Ala Asp Lys Ala
       690                 695                 700
<210> 12
   <211> 2127
   <212> DNA
   <213> Artificial Sequence
<220>
   <223> Multizyme: E389D9eS/EgD8M gene fusion
<220>
   <221> CDS
   <222> (1)..(2127)
   <223> Multizyme: E389D9eS/EgD8M gene fusion
<300>
   <302> MULTIZYMES AND THEIR USE IN MAKING POLYUNSATURATED FATTY ACIDS
<310> US-2008-0254191-A1
   <311> 2008-04-03
   <312> 2008-10-16
   <313> (1)..(2127)
<300>
   <302> MULTIZYMES AND THEIR USE IN MAKING POLYUNSATURATED FATTY ACIDS
<310> WO 2008/124048
   <311> 2008-04-03
   <312> 2008-10-16
   <313> (1)..(2127)
<400> 12
   atg gct gcc gtc atc gag gtg gcc aac gag ttc gtc gct atc act gcc        48
   Met Ala Ala Val Ile Glu Val Ala Asn Glu Phe Val Ala Ile Thr Ala
   1               5                   10                  15
   gag acc ctt ccc aag gtg gac tat cag cga ctc tgg cga gac atc tac        96
   Glu Thr Leu Pro Lys Val Asp Tyr Gln Arg Leu Trp Arg Asp Ile Tyr
               20                  25                  30
   tcc tgc gag ctc ctg tac ttc tcc att gct ttc gtc atc ctc aag ttt       144
   Ser Cys Glu Leu Leu Tyr Phe Ser Ile Ala Phe Val Ile Leu Lys Phe
           35                  40                  45
   acc ctt ggc gag ctc tcg gat tct ggc aaa aag att ctg cga gtg ctg       192
   Thr Leu Gly Glu Leu Ser Asp Ser Gly Lys Lys Ile Leu Arg Val Leu
       50                  55                  60
   ttc aag tgg tac aac ctc ttc atg tcc gtc ttt tcg ctg gtg tcc ttc       240
   Phe Lys Trp Tyr Asn Leu Phe Met Ser Val Phe Ser Leu Val Ser Phe
   65                  70                  75                  80
   ctc tgt atg ggt tac gcc atc tac acc gtt gga ctg tac tcc aac gaa       288
   Leu Cys Met Gly Tyr Ala Ile Tyr Thr Val Gly Leu Tyr Ser Asn Glu
                   85                  90                  95
   tgc gac aga gct ttc gac aac agc ttg ttc cga ttt gcc acc aag gtc       336
   Cys Asp Arg Ala Phe Asp Asn Ser Leu Phe Arg Phe Ala Thr Lys Val
               100                 105                 110
   ttc tac tat tcc aag ttt ctg gag tac atc gac tct ttc tac ctt ccc       384
   Phe Tyr Tyr Ser Lys Phe Leu Glu Tyr Ile Asp Ser Phe Tyr Leu Pro
           115                 120                 125
   ctc atg gcc aag cct ctg tcc ttt ctg cag ttc ttt cat cac ttg gga       432
   Leu Met Ala Lys Pro Leu Ser Phe Leu Gln Phe Phe His His Leu Gly
       130                 135                 140
   gct cct atg gac atg tgg ctc ttc gtg cag tac tct ggc gaa tcc att       480
   Ala Pro Met Asp Met Trp Leu Phe Val Gln Tyr Ser Gly Glu Ser Ile
   145                 150                 155                 160
   tgg atc ttt gtg ttc ctg aac gga ttc att cac ttt gtc atg tac ggc      528
   Trp Ile Phe Val Phe Leu Asn Gly Phe Ile His Phe Val Met Tyr Gly
                   165                 170                 175
   tac tat tgg aca cgg ctg atg aag ttc aac ttt ccc atg ccc aag cag      576
   Tyr Tyr Trp Thr Arg Leu Met Lys Phe Asn Phe Pro Met Pro Lys Gln
               180                 185                 190
   ctc att acc gca atg cag atc acc cag ttc aac gtt ggc ttc tac ctc      624
   Leu Ile Thr Ala Met Gln Ile Thr Gln Phe Asn Val Gly Phe Tyr Leu
           195                 200                 205
   gtg tgg tgg tac aag gac att ccc tgt tac cga aag gat ccc atg cga      672
   Val Trp Trp Tyr Lys Asp Ile Pro Cys Tyr Arg Lys Asp Pro Met Arg
       210                 215                 220
   atg ctg gcc tgg atc ttc aac tac tgg tac gtc ggt acc gtt ctt ctg      720
   Met Leu Ala Trp Ile Phe Asn Tyr Trp Tyr Val Gly Thr Val Leu Leu
   225                 230                 235                 240
   ctc ttc atc aac ttc ttt gtc aag tcc tac gtg ttt ccc aag cct aag      768
   Leu Phe Ile Asn Phe Phe Val Lys Ser Tyr Val Phe Pro Lys Pro Lys
                   245                 250                 255
   act gcc gac aaa aag gtc cag ggc gcc ggt ccc gct cga cct gcc gga      816
   Thr Ala Asp Lys Lys Val Gln Gly Ala Gly Pro Ala Arg Pro Ala Gly
               260                 265                 270
   ctt cct ccc gct acc tac tac gac tct ctg gcc gtc atg gga tcc gtg      864
   Leu Pro Pro Ala Thr Tyr Tyr Asp Ser Leu Ala Val Met Gly Ser Val
           275                 280                 285
   aag gct tct cga cag gct ctg ccc ctc gtc atc gac gga aag gtg tac      912
   Lys Ala Ser Arg Gln Ala Leu Pro Leu Val Ile Asp Gly Lys Val Tyr
       290                 295                 300
   gac gtc tcc gct tgg gtg aac ttc cac cct ggt gga gct gaa atc att      960
   Asp Val Ser Ala Trp Val Asn Phe His Pro Gly Gly Ala Glu Ile Ile
   305                 310                 315                 320
   gag aac tac cag gga cga gat gct act gac gcc ttc atg gtt atg cac     1008
   Glu Asn Tyr Gln Gly Arg Asp Ala Thr Asp Ala Phe Met Val Met His
                   325                 330                 335
   tct cag gaa gcc ttc gac aag ctc aag cga atg ccc aag atc aac cag     1056
   Ser Gln Glu Ala Phe Asp Lys Leu Lys Arg Met Pro Lys Ile Asn Gln
               340                 345                 350
   gct tcc gag ctg cct ccc cag gct gcc gtc aac gaa gct cag gag gat     1104
   Ala Ser Glu Leu Pro Pro Gln Ala Ala Val Asn Glu Ala Gln Glu Asp
           355                 360                 365
   ttc cga aag ctc cga gaa gag ctg atc gcc act ggc atg ttt gac gcc     1152
   Phe Arg Lys Leu Arg Glu Glu Leu Ile Ala Thr Gly Met Phe Asp Ala
       370                 375                 380
   tct ccc ctc tgg tac tcg tac aag atc ttg acc acc ctg ggt ctt ggc     1200
   Ser Pro Leu Trp Tyr Ser Tyr Lys Ile Leu Thr Thr Leu Gly Leu Gly
   385                 390                 395                 400
   gtg ctt gcc ttc ttc atg ctg gtc cag tac cac ctg tac ttc att ggt     1248
   Val Leu Ala Phe Phe Met Leu Val Gln Tyr His Leu Tyr Phe Ile Gly
                   405                 410                 415
   gct ctc gtg ctc ggt atg cac tac cag caa atg gga tgg ctg tct cat        1296
   Ala Leu Val Leu Gly Met His Tyr Gln Gln Met Gly Trp Leu Ser His
               420                 425                 430
   gac atc tgc cac cac cag acc ttc aag aac cga aac tgg aat aac gtc        1344
   Asp Ile Cys His His Gln Thr Phe Lys Asn Arg Asn Trp Asn Asn Val
           435                 440                 445
   ctg ggt ctg gtc ttt ggc aac gga ctc cag ggc ttc tcc gtg acc tgg        1392
   Leu Gly Leu Val Phe Gly Asn Gly Leu Gln Gly Phe Ser Val Thr Trp
       450                 455                 460
   tgg aag gac aga cac aac gcc cat cat tct gct acc aac gtt cag ggt        1440
   Trp Lys Asp Arg His Asn Ala His His Ser Ala Thr Asn Val Gln Gly
   465                 470                 475                 480
   cac gat ccc gac att gat aac ctg cct ctg ctc gcc tgg tcc gag gac        1488
   His Asp Pro Asp Ile Asp Asn Leu Pro Leu Leu Ala Trp Ser Glu Asp
                   485                 490                 495
   gat gtc act cga gct tct ccc atc tcc cga aag ctc att cag ttc caa        1536
   Asp Val Thr Arg Ala Ser Pro Ile Ser Arg Lys Leu Ile Gln Phe Gln
               500                 505                 510
   cag tac tat ttc ctg gtc atc tgt att ctc ctg cga ttc atc tgg tgt        1584
   Gln Tyr Tyr Phe Leu Val Ile Cys Ile Leu Leu Arg Phe Ile Trp Cys
           515                 520                 525
   ttc cag tct gtg ctg acc gtt cga tcc ctc aag gac cga gac aac cag        1632
   Phe Gln Ser Val Leu Thr Val Arg Ser Leu Lys Asp Arg Asp Asn Gln
       530                 535                 540
   ttc tac cga tct cag tac aag aaa gag gcc att gga ctc gct ctg cac        1680
   Phe Tyr Arg Ser Gln Tyr Lys Lys Glu Ala Ile Gly Leu Ala Leu His
   545                 550                 555                 560
   tgg act ctc aag acc ctg ttc cac ctc ttc ttt atg ccc tcc atc ctg        1728
   Trp Thr Leu Lys Thr Leu Phe His Leu Phe Phe Met Pro Ser Ile Leu
                   565                 570                 575
   acc tcg atg ctg gtg ttc ttt gtt tcc gag ctc gtc ggt ggc ttc gga        1776
   Thr Ser Met Leu Val Phe Phe Val Ser Glu Leu Val Gly Gly Phe Gly
               580                 585                 590
   att gcc atc gtg gtc ttc atg aac cac tac cct ctg gag aag atc ggt        1824
   Ile Ala Ile Val Val Phe Met Asn His Tyr Pro Leu Glu Lys Ile Gly
           595                 600                 605
   gat tcc gtc tgg gac gga cat ggc ttc tct gtg ggt cag atc cat gag        1872
   Asp Ser Val Trp Asp Gly His Gly Phe Ser Val Gly Gln Ile His Glu
       610                 615                 620
   acc atg aac att cga cga ggc atc att act gac tgg ttc ttt gga ggc        1920
   Thr Met Asn Ile Arg Arg Gly Ile Ile Thr Asp Trp Phe Phe Gly Gly
   625                 630                 635                 640
   ctg aac tac cag atc gag cac cat ctc tgg ccc acc ctg cct cga cac        1968
   Leu Asn Tyr Gln Ile Glu His His Leu Trp Pro Thr Leu Pro Arg His
                   645                 650                 655
   aac ctc act gcc gtt tcc tac cag gtg gaa cag ctg tgc cag aag cac        2016
   Asn Leu Thr Ala Val Ser Tyr Gln Val Glu Gln Leu Cys Gln Lys His
               660                 665                 670
   aac ctc ccc tac cga aac cct ctg ccc cat gaa ggt ctc gtc atc ctg         2064
   Asn Leu Pro Tyr Arg Asn Pro Leu Pro His Glu Gly Leu Val Ile Leu
           675                 680                 685
   ctc cga tac ctg tcc cag ttc gct cga atg gcc gag aag cag ccc ggt         2112
   Leu Arg Tyr Leu Ser Gln Phe Ala Arg Met Ala Glu Lys Gln Pro Gly
       690                 695                 700
   gcc aag gct cag taa                                                     2127
   Ala Lys Ala Gln
   705
<210> 13
   <211> 708
   <212> PRT
   <213> Artificial Sequence
<220>
   <223> Synthetic Construct
<400> 13
   Met Ala Ala Val Ile Glu Val Ala Asn Glu Phe Val Ala Ile Thr Ala
   1               5                   10                  15
   Glu Thr Leu Pro Lys Val Asp Tyr Gln Arg Leu Trp Arg Asp Ile Tyr
               20                  25                  30
   Ser Cys Glu Leu Leu Tyr Phe Ser Ile Ala Phe Val Ile Leu Lys Phe
           35                  40                  45
   Thr Leu Gly Glu Leu Ser Asp Ser Gly Lys Lys Ile Leu Arg Val Leu
       50                  55                  60
   Phe Lys Trp Tyr Asn Leu Phe Met Ser Val Phe Ser Leu Val Ser Phe
   65                  70                  75                  80
   Leu Cys Met Gly Tyr Ala Ile Tyr Thr Val Gly Leu Tyr Ser Asn Glu
                   85                  90                  95
   Cys Asp Arg Ala Phe Asp Asn Ser Leu Phe Arg Phe Ala Thr Lys Val
               100                 105                 110
   Phe Tyr Tyr Ser Lys Phe Leu Glu Tyr Ile Asp Ser Phe Tyr Leu Pro
           115                 120                 125
   Leu Met Ala Lys Pro Leu Ser Phe Leu Gln Phe Phe His His Leu Gly
       130                 135                 140
   Ala Pro Met Asp Met Trp Leu Phe Val Gln Tyr Ser Gly Glu Ser Ile
   145                 150                 155                 160
   Trp Ile Phe Val Phe Leu Asn Gly Phe Ile His Phe Val Met Tyr Gly
                   165                 170                 175
   Tyr Tyr Trp Thr Arg Leu Met Lys Phe Asn Phe Pro Met Pro Lys Gln
               180                 185                 190
   Leu Ile Thr Ala Met Gln Ile Thr Gln Phe Asn Val Gly Phe Tyr Leu
           195                 200                 205
   Val Trp Trp Tyr Lys Asp Ile Pro Cys Tyr Arg Lys Asp Pro Met Arg
       210                 215                 220
   Met Leu Ala Trp Ile Phe Asn Tyr Trp Tyr Val Gly Thr Val Leu Leu
   225                 230                 235                 240
   Leu Phe Ile Asn Phe Phe Val Lys Ser Tyr Val Phe Pro Lys Pro Lys
                   245                 250                 255
   Thr Ala Asp Lys Lys Val Gln Gly Ala Gly Pro Ala Arg Pro Ala Gly
               260                 265                 270
   Leu Pro Pro Ala Thr Tyr Tyr Asp Ser Leu Ala Val Met Gly Ser Val
           275                 280                 285
   Lys Ala Ser Arg Gln Ala Leu Pro Leu Val Ile Asp Gly Lys Val Tyr
       290                 295                 300
   Asp Val Ser Ala Trp Val Asn Phe His Pro Gly Gly Ala Glu Ile Ile
   305                 310                 315                 320
   Glu Asn Tyr Gln Gly Arg Asp Ala Thr Asp Ala Phe Met Val Met His
                   325                 330                 335
   Ser Gln Glu Ala Phe Asp Lys Leu Lys Arg Met Pro Lys Ile Asn Gln
               340                 345                 350
   Ala Ser Glu Leu Pro Pro Gln Ala Ala Val Asn Glu Ala Gln Glu Asp
           355                 360                 365
   Phe Arg Lys Leu Arg Glu Glu Leu Ile Ala Thr Gly Met Phe Asp Ala
       370                 375                 380
   Ser Pro Leu Trp Tyr Ser Tyr Lys Ile Leu Thr Thr Leu Gly Leu Gly
   385                 390                 395                 400
   Val Leu Ala Phe Phe Met Leu Val Gln Tyr His Leu Tyr Phe Ile Gly
                   405                 410                 415
   Ala Leu Val Leu Gly Met His Tyr Gln Gln Met Gly Trp Leu Ser His
               420                 425                 430
   Asp Ile Cys His His Gln Thr Phe Lys Asn Arg Asn Trp Asn Asn Val
   435 440 445
   Leu Gly Leu Val Phe Gly Asn Gly Leu Gln Gly Phe Ser Val Thr Trp
   450 455 460
   Trp Lys Asp Arg His Asn Ala His His Ser Ala Thr Asn Val Gln Gly
   465 470 475 480
   His Asp Pro Asp Ile Asp Asn Leu Pro Leu Leu Ala Trp Ser Glu Asp
   485 490 495
   Asp Val Thr Arg Ala Ser Pro Ile Ser Arg Lys Leu Ile Gln Phe Gln
   500 505 510
   Gln Tyr Tyr Phe Leu Val Ile Cys Ile Leu Leu Arg Phe Ile Trp Cys
   515 520 525
   Phe Gln Ser Val Leu Thr Val Arg Ser Leu Lys Asp Arg Asp Asn Gln
   530 535 540
   Phe Tyr Arg Ser Gln Tyr Lys Lys Glu Ala Ile Gly Leu Ala Leu His
   545 550 555 560
   Trp Thr Leu Lys Thr Leu Phe His Leu Phe Phe Met Pro Ser Ile Leu
   565 570 575
   Thr Ser Met Leu Val Phe Phe Val Ser Glu Leu Val Gly Gly Phe Gly
   580 585 590
   Ile Ala Ile Val Val Phe Met Asn His Tyr Pro Leu Glu Lys Ile Gly
   595 600 605
   Asp Ser Val Trp Asp Gly His Gly Phe Ser Val Gly Gln Ile His Glu
   610 615 620
   Thr Met Asn Ile Arg Arg Gly Ile Ile Thr Asp Trp Phe Phe Gly Gly
   625 630 635 640
   Leu Asn Tyr Gln Ile Glu His His Leu Trp Pro Thr Leu Pro Arg His
   645 650 655
   Asn Leu Thr Ala Val Ser Tyr Gln Val Glu Gln Leu Cys Gln Lys His
   660 665 670
   Asn Leu Pro Tyr Arg Asn Pro Leu Pro His Glu Gly Leu Val Ile Leu
   675 680 685
   Leu Arg Tyr Leu Ser Gln Phe Ala Arg Met Ala Glu Lys Gln Pro Gly
   690 695 700
   Ala Lys Ala Gln
   705
<210> 14
   <211> 1860
   <212> DNA
   <213> Saccharomyces cerevisiae
<220>
   <221> CDS
   <222> (1)..(1860)
   <223> GenBank Accession No. NP_014818; "YOR175C"
<300>
   <302> Genes encoding a novel type of lysophophatidylcholine acyltransferases and their use to increase triacylglycerol production and/or modify fatty acid composition
<310> US-2008-0145867-A1
   <311> 2007-06-15
   <312> 2008-06-19
   <313> (1)..(1860)
<300>
   <302> Genes encoding a novel type of lysophophatidylcholine acyltransferases and their use to increase triacylglycerol production and/or modify fatty acid composition
<310> WO 2008/076377
   <311> 2007-12-13
   <312> 2008-06-26
   <313> (1)..(1860)
<300>
   <302> USE OF A CLASS OF GENES ENCODING LYSOPHOSPHOLIPID ACYL TRANSFERASES FOR APPLICATION IN AGRICULTURE, BIOTECHNOLOGY AND MEDICINE
<310> WO 2009/001315
   <311> 2008-06-25
   <312> 2008-12-31
   <313> (1)..(1860)
<400> 14
   atg tac aat cct gtg gac gct gtt tta aca aag ata att acc aac tat        48
   Met Tyr Asn Pro Val Asp Ala Val Leu Thr Lys Ile Ile Thr Asn Tyr
   1               5                   10                  15
   ggg att gat agt ttt aca ctg cga tat gct atc tgc tta ttg gga tcg        96
   Gly Ile Asp Ser Phe Thr Leu Arg Tyr Ala Ile Cys Leu Leu Gly Ser
               20                  25                  30
   ttc cca ctg aat gct att ttg aag aga att ccc gag aag cgt ata ggt       144
   Phe Pro Leu Asn Ala Ile Leu Lys Arg Ile Pro Glu Lys Arg Ile Gly
           35                  40                  45
   tta aaa tgt tgt ttt atc att tct atg tcg atg ttt tac tta ttc ggt       192
   Leu Lys Cys Cys Phe Ile Ile Ser Met Ser Met Phe Tyr Leu Phe Gly
       50                  55                  60
   gtg ctg aat cta gta agt gga ttc agg acc ctg ttt att agt acc atg       240
   Val Leu Asn Leu Val Ser Gly Phe Arg Thr Leu Phe Ile Ser Thr Met
   65                  70                  75                  80
   ttt act tac ttg atc tca aga ttt tac cgt tcc aag ttt atg cca cac        288
   Phe Thr Tyr Leu Ile Ser Arg Phe Tyr Arg Ser Lys Phe Met Pro His
                   85                  90                  95
   ttg aat ttc atg ttt gtt atg ggt cat ttg gca ata aat cat ata cac        336
   Leu Asn Phe Met Phe Val Met Gly His Leu Ala Ile Asn His Ile His
               100                 105                 110
   gcc caa ttc ctt aac gaa cag act caa act acc gtt gac att aca agt        384
   Ala Gln Phe Leu Asn Glu Gln Thr Gln Thr Thr Val Asp Ile Thr Ser
           115                 120                 125
   tca caa atg gtt tta gcc atg aaa cta act tct ttt gca tgg tcg tac        432
   Ser Gln Met Val Leu Ala Met Lys Leu Thr Ser Phe Ala Trp Ser Tyr
       130                 135                 140
   tat gat ggt tca tgc act agc gaa agc gat ttc aaa gat ttg act gag        480
   Tyr Asp Gly Ser Cys Thr Ser Glu Ser Asp Phe Lys Asp Leu Thr Glu
   145                 150                 155                 160
   cat caa aaa tct cgt gct gtc aga ggt cat cca ccc tta tta aag ttc        528
   His Gln Lys Ser Arg Ala Val Arg Gly His Pro Pro Leu Leu Lys Phe
                   165                 170                 175
   ctg gca tat gca ttt ttc tat tca acg ttg cta act ggc cca agt ttc        576
   Leu Ala Tyr Ala Phe Phe Tyr Ser Thr Leu Leu Thr Gly Pro Ser Phe
               180                 185                 190
   gat tat gcc gat ttt gac agc tgg ttg aat tgt gag atg ttc cgt gac        624
   Asp Tyr Ala Asp Phe Asp Ser Trp Leu Asn Cys Glu Met Phe Arg Asp
           195                 200                 205
   ttg cct gaa agc aaa aag cct atg aga aga cac cac cct ggt gaa aga        672
   Leu Pro Glu Ser Lys Lys Pro Met Arg Arg His His Pro Gly Glu Arg
       210                 215                 220
   aga cag att cca aag aat ggt aaa ctt gca tta tgg aaa gtt gtt caa        720
   Arg Gln Ile Pro Lys Asn Gly Lys Leu Ala Leu Trp Lys Val Val Gln
   225                 230                 235                 240
   ggt ctt gct tgg atg att tta agt aca cta gga atg aag cac ttc ccc        768
   Gly Leu Ala Trp Met Ile Leu Ser Thr Leu Gly Met Lys His Phe Pro
                   245                 250                 255
   gta aaa tac gtt ttg gac aaa gat ggc ttc cca acg aga tct ttt ata        816
   Val Lys Tyr Val Leu Asp Lys Asp Gly Phe Pro Thr Arg Ser Phe Ile
               260                 265                 270
   ttc aga atc cat tac tta ttc ttg ctt ggt ttc atc cat aga ttc aag        864
   Phe Arg Ile His Tyr Leu Phe Leu Leu Gly Phe Ile His Arg Phe Lys
           275                 280                 285
   tac tac gct gcc tgg act att tcg gaa gga tct tgt att ttg tgc ggt        912
   Tyr Tyr Ala Ala Trp Thr Ile Ser Glu Gly Ser Cys Ile Leu Cys Gly
       290                 295                 300
   ttg ggt tat aat ggt tat gat tca aag aca caa aag atc aga tgg gat        960
   Leu Gly Tyr Asn Gly Tyr Asp Ser Lys Thr Gln Lys Ile Arg Trp Asp
   305                 310                 315                 320
   cgt gtc aga aat att gac att tgg acc gta gaa acg gcg cag aat acg       1008
   Arg Val Arg Asn Ile Asp Ile Trp Thr Val Glu Thr Ala Gln Asn Thr
                   325                 330                 335
   cgt gaa atg ttg gaa gca tgg aat atg aat act aac aag tgg cta aaa      1056
   Arg Glu Met Leu Glu Ala Trp Asn Met Asn Thr Asn Lys Trp Leu Lys
               340                 345                 350
   tac tct gtt tat tta cgt gtc aca aag aag ggc aaa aaa cct ggt ttc      1104
   Tyr Ser Val Tyr Leu Arg Val Thr Lys Lys Gly Lys Lys Pro Gly Phe
           355                 360                 365
   cgc tca act ttg ttt act ttc cta act tcc gca ttt tgg cat ggt acc      1152
   Arg Ser Thr Leu Phe Thr Phe Leu Thr Ser Ala Phe Trp His Gly Thr
       370                 375                 380
   aga cct ggg tac tat ctg act ttt gcg aca ggg gct ttg tac caa aca      1200
   Arg Pro Gly Tyr Tyr Leu Thr Phe Ala Thr Gly Ala Leu Tyr Gln Thr
   385                 390                 395                 400
   tgt ggt aaa atc tac aga cgc aat ttt aga cca att ttc ttg cga gaa      1248
   Cys Gly Lys Ile Tyr Arg Arg Asn Phe Arg Pro Ile Phe Leu Arg Glu
                   405                 410                 415
   gat ggt gtc act cct ttg cct tct aaa aaa atc tac gat tta gtt ggc      1296
   Asp Gly Val Thr Pro Leu Pro Ser Lys Lys Ile Tyr Asp Leu Val Gly
               420                 425                 430
   ata tat gca att aaa cta gca ttt ggt tac atg gtg caa cca ttt att      1344
   Ile Tyr Ala Ile Lys Leu Ala Phe Gly Tyr Met Val Gln Pro Phe Ile
           435                 440                 445
   atc ctt gat ttg aag cca tct tta atg gta tgg ggc tct gtt tat ttc      1392
   Ile Leu Asp Leu Lys Pro Ser Leu Met Val Trp Gly Ser Val Tyr Phe
       450                 455                 460
   tat gtt cat att att gtt gct ttc tca ttt ttc cta ttc aga gga cca      1440
   Tyr Val His Ile Ile Val Ala Phe Ser Phe Phe Leu Phe Arg Gly Pro
   465                 470                 475                 480
   tat gct aaa caa gtt act gaa ttt ttt aaa tcc aaa caa cct aaa gaa      1488
   Tyr Ala Lys Gln Val Thr Glu Phe Phe Lys Ser Lys Gln Pro Lys Glu
                   485                 490                 495
   ata ttc att aga aaa caa aag aag ttg gaa aaa gat att tct gca agc      1536
   Ile Phe Ile Arg Lys Gln Lys Lys Leu Glu Lys Asp Ile Ser Ala Ser
               500                 505                 510
   tct cca aac ttg ggt ggt ata ttg aag gca aag att gaa cat gaa aag      1584
   Ser Pro Asn Leu Gly Gly Ile Leu Lys Ala Lys Ile Glu His Glu Lys
           515                 520                 525
   gga aag aca gca gaa gaa gaa gaa atg aac tta ggt att cca cca att      1632
   Gly Lys Thr Ala Glu Glu Glu Glu Met Asn Leu Gly Ile Pro Pro Ile
       530                 535                 540
   gag tta gaa aag tgg gac aat gct aag gaa gat tgg gaa gat ttc tgc      1680
   Glu Leu Glu Lys Trp Asp Asn Ala Lys Glu Asp Trp Glu Asp Phe Cys
   545                 550                 555                 560
   aaa gat tac aaa gaa tgg aga aat aaa aat ggt ctt gaa ata gaa gag      1728
   Lys Asp Tyr Lys Glu Trp Arg Asn Lys Asn Gly Leu Glu Ile Glu Glu
                   565                 570                 575
   gaa aac ctt tct aaa gct ttt gaa aga ttc aag cag gaa ttt tct aac      1776
   Glu Asn Leu Ser Lys Ala Phe Glu Arg Phe Lys Gln Glu Phe Ser Asn
               580                 585                 590
   gct gca agt gga tca ggt gaa cgt gtg aga aaa atg agt ttt agt ggt       1824
   Ala Ala Ser Gly Ser Gly Glu Arg Val Arg Lys Met Ser Phe Ser Gly
           595                 600                 605
   tac tca cca aag cct att tca aaa aag gaa gag tag                       1860
   Tyr Ser Pro Lys Pro Ile Ser Lys Lys Glu Glu
       610                 615
<210> 15
   <211> 619
   <212> PRT
   <213> Saccharomyces cerevisiae
<400> 15
   Met Tyr Asn Pro Val Asp Ala Val Leu Thr Lys Ile Ile Thr Asn Tyr
   1               5                   10                  15
   Gly Ile Asp Ser Phe Thr Leu Arg Tyr Ala Ile Cys Leu Leu Gly Ser
               20                  25                  30
   Phe Pro Leu Asn Ala Ile Leu Lys Arg Ile Pro Glu Lys Arg Ile Gly
           35                  40                  45
   Leu Lys Cys Cys Phe Ile Ile Ser Met Ser Met Phe Tyr Leu Phe Gly
       50                  55                  60
   Val Leu Asn Leu Val Ser Gly Phe Arg Thr Leu Phe Ile Ser Thr Met
   65                  70                  75                  80
   Phe Thr Tyr Leu Ile Ser Arg Phe Tyr Arg Ser Lys Phe Met Pro His
                   85                  90                  95
   Leu Asn Phe Met Phe Val Met Gly His Leu Ala Ile Asn His Ile His
               100                 105                 110
   Ala Gln Phe Leu Asn Glu Gln Thr Gln Thr Thr Val Asp Ile Thr Ser
           115                 120                 125
   Ser Gln Met Val Leu Ala Met Lys Leu Thr Ser Phe Ala Trp Ser Tyr
       130                 135                 140
   Tyr Asp Gly Ser Cys Thr Ser Glu Ser Asp Phe Lys Asp Leu Thr Glu
   145                 150                 155                 160
   His Gln Lys Ser Arg Ala Val Arg Gly His Pro Pro Leu Leu Lys Phe
                   165                 170                 175
   Leu Ala Tyr Ala Phe Phe Tyr Ser Thr Leu Leu Thr Gly Pro Ser Phe
               180                 185                 190
   Asp Tyr Ala Asp Phe Asp Ser Trp Leu Asn Cys Glu Met Phe Arg Asp
   195 200 205
   Leu Pro Glu Ser Lys Lys Pro Met Arg Arg His His Pro Gly Glu Arg
   210 215 220
   Arg Gln Ile Pro Lys Asn Gly Lys Leu Ala Leu Trp Lys Val Val Gln
   225 230 235 240
   Gly Leu Ala Trp Met Ile Leu Ser Thr Leu Gly Met Lys His Phe Pro
   245 250 255
   Val Lys Tyr Val Leu Asp Lys Asp Gly Phe Pro Thr Arg Ser Phe Ile
   260 265 270
   Phe Arg Ile His Tyr Leu Phe Leu Leu Gly Phe Ile His Arg Phe Lys
   275 280 285
   Tyr Tyr Ala Ala Trp Thr Ile Ser Glu Gly Ser Cys Ile Leu Cys Gly
   290 295 300
   Leu Gly Tyr Asn Gly Tyr Asp Ser Lys Thr Gln Lys Ile Arg Trp Asp
   305 310 315 320
   Arg Val Arg Asn Ile Asp Ile Trp Thr Val Glu Thr Ala Gln Asn Thr
   325 330 335
   Arg Glu Met Leu Glu Ala Trp Asn Met Asn Thr Asn Lys Trp Leu Lys
   340 345 350
   Tyr Ser Val Tyr Leu Arg Val Thr Lys Lys Gly Lys Lys Pro Gly Phe
   355 360 365
   Arg Ser Thr Leu Phe Thr Phe Leu Thr Ser Ala Phe Trp His Gly Thr
   370 375 380
   Arg Pro Gly Tyr Tyr Leu Thr Phe Ala Thr Gly Ala Leu Tyr Gln Thr
   385 390 395 400
   Cys Gly Lys Ile Tyr Arg Arg Asn Phe Arg Pro Ile Phe Leu Arg Glu
   405 410 415
   Asp Gly Val Thr Pro Leu Pro Ser Lys Lys Ile Tyr Asp Leu Val Gly
   420 425 430
   Ile Tyr Ala Ile Lys Leu Ala Phe Gly Tyr Met Val Gln Pro Phe Ile
   435 440 445
   Ile Leu Asp Leu Lys Pro Ser Leu Met Val Trp Gly Ser Val Tyr Phe
   450 455 460
   Tyr Val His Ile Ile Val Ala Phe Ser Phe Phe Leu Phe Arg Gly Pro
   465 470 475 480
   Tyr Ala Lys Gln Val Thr Glu Phe Phe Lys Ser Lys Gln Pro Lys Glu
   485 490 495
   Ile Phe Ile Arg Lys Gln Lys Lys Leu Glu Lys Asp Ile Ser Ala Ser
   500 505 510
   Ser Pro Asn Leu Gly Gly Ile Leu Lys Ala Lys Ile Glu His Glu Lys
   515 520 525
   Gly Lys Thr Ala Glu Glu Glu Glu Met Asn Leu Gly Ile Pro Pro Ile
   530 535 540
   Glu Leu Glu Lys Trp Asp Asn Ala Lys Glu Asp Trp Glu Asp Phe Cys
   545 550 555 560
   Lys Asp Tyr Lys Glu Trp Arg Asn Lys Asn Gly Leu Glu Ile Glu Glu
   565 570 575
   Glu Asn Leu Ser Lys Ala Phe Glu Arg Phe Lys Gln Glu Phe Ser Asn
   580 585 590
   Ala Ala Ser Gly Ser Gly Glu Arg Val Arg Lys Met Ser Phe Ser Gly
   595 600 605
   Tyr Ser Pro Lys Pro Ile Ser Lys Lys Glu Glu
   610 615
<210> 16
   <211> 1539
   <212> DNA
   <213> Yarrowia lipolytica
<220>
   <221> CDS
   <222> (1)..(1539)
   <223> GenBank Accession No. XP_505624; "YALI0F19514p"
<400> 16
   atg gcc ttt cca tgg gca gat aag tgg gca gcc gat gcg tct gca tct         48
   Met Ala Phe Pro Trp Ala Asp Lys Trp Ala Ala Asp Ala Ser Ala Ser
   1               5                   10                  15
   aca ggg ctg cct ccg gac ctc ctc aag att gca ttc act ctg gtc atg         96
   Thr Gly Leu Pro Pro Asp Leu Leu Lys Ile Ala Phe Thr Leu Val Met
               20                  25                  30
   tct tat ccg ctg agt tct ctc atg aaa cgg ctg cca gat gac gcc aaa        144
   Ser Tyr Pro Leu Ser Ser Leu Met Lys Arg Leu Pro Asp Asp Ala Lys
   35 40 45
   aac ctc aag atc atc tat atc atc tcc gtg tcc atc ttc tac atg gtg 192
   Asn Leu Lys Ile Ile Tyr Ile Ile Ser Val Ser Ile Phe Tyr Met Val
   50 55 60
   ggt gtc ttc tcc ctc tat ggc gga gct gcc act ctg ctc ttc tcc tca 240
   Gly Val Phe Ser Leu Tyr Gly Gly Ala Ala Thr Leu Leu Phe Ser Ser
   65 70 75 80
   atg ggt acc ttc ttc atc acc caa tgg aag agc cct tac atg ccc tgg 288
   Met Gly Thr Phe Phe Ile Thr Gln Trp Lys Ser Pro Tyr Met Pro Trp
   85 90 95
   gtc aat ttt ggt ttt gtc atg acc cat ctc ttc gtc aat cac ctg cgt 336
   Val Asn Phe Gly Phe Val Met Thr His Leu Phe Val Asn His Leu Arg
   100 105 110
   tcg cag ttt ttc ccc gaa aca tac gac ccc aat gtc att gac atc acc 384
   Ser Gln Phe Phe Pro Glu Thr Tyr Asp Pro Asn Val Ile Asp Ile Thr
   115 120 125
   gga gca cag atg gtt ctg tgt atg aag cta tcg tct ttt gga tgg aac 432
   Gly Ala Gln Met Val Leu Cys Met Lys Leu Ser Ser Phe Gly Trp Asn
   130 135 140
   gtc tac gat gga tgg cag att gag aag ggt gag cag ctc agc gag ttc 480
   Val Tyr Asp Gly Trp Gln Ile Glu Lys Gly Glu Gln Leu Ser Glu Phe
   145 150 155 160
   cag act aaa agg gct gtt ctc aag cac ccc agt ctt atg gac ttc cta 528
   Gln Thr Lys Arg Ala Val Leu Lys His Pro Ser Leu Met Asp Phe Leu
   165 170 175
   gct ttt gtg ttc tac ttc cct tcc att ctg aca ggt cct tct tac gac 576
   Ala Phe Val Phe Tyr Phe Pro Ser Ile Leu Thr Gly Pro Ser Tyr Asp
   180 185 190
   tat atg gag ttc cat aac tgg ctc gat ctc agc ctg ttc aag gag ctg 624
   Tyr Met Glu Phe His Asn Trp Leu Asp Leu Ser Leu Phe Lys Glu Leu
   195 200 205
   gag aaa gat aag gac ccc aag cga gct gct cga cga aag cga cac aag 672
   Glu Lys Asp Lys Asp Pro Lys Arg Ala Ala Arg Arg Lys Arg His Lys
   210 215 220
   atc ccc cga tct gga atc gct gct tcc aag aaa ctc gcc gct ggt atc 720
   Ile Pro Arg Ser Gly Ile Ala Ala Ser Lys Lys Leu Ala Ala Gly Ile
   225 230 235 240
   ttc tgg atc gtt ctg tgg acc cag gtg gac tct cga atc tcc acc gcc 768
   Phe Trp Ile Val Leu Trp Thr Gln Val Asp Ser Arg Ile Ser Thr Ala
   245 250 255
   tac gct tac tca gac gca ttc acc aag gag cac aac atc ttt gga cga 816
   Tyr Ala Tyr Ser Asp Ala Phe Thr Lys Glu His Asn Ile Phe Gly Arg
   260 265 270
   att gtg tac ctc tac atg ctc ggt ttc atg tac cga ctc aag tac tac 864
   Ile Val Tyr Leu Tyr Met Leu Gly Phe Met Tyr Arg Leu Lys Tyr Tyr
   275 280 285
   gga gcc tgg tcc att tcc gag gga gcc tgc atc ttg tct ggc ctc gga 912
   Gly Ala Trp Ser Ile Ser Glu Gly Ala Cys Ile Leu Ser Gly Leu Gly
   290 295 300
   ttc cat ggc gtg gac ccc aaa act ggc aag tac aag tgg gac cgt gtc      960
   Phe His Gly Val Asp Pro Lys Thr Gly Lys Tyr Lys Trp Asp Arg Val
   305                 310                 315                 320
   cag aac gtg gac ccg tgg gga ttc gaa act ggt caa aac aca aag gct     1008
   Gln Asn Val Asp Pro Trp Gly Phe Glu Thr Gly Gln Asn Thr Lys Ala
                   325                 330                 335
   ctg ctg gag gcc tgg aac cag aac act aac aag tgg cta cga aac tat     1056
   Leu Leu Glu Ala Trp Asn Gln Asn Thr Asn Lys Trp Leu Arg Asn Tyr
               340                 345                 350
   gtg tac ctc cga gtg gtg ccc aaa ggc caa aag cct gga ttc cga gcc     1104
   Val Tyr Leu Arg Val Val Pro Lys Gly Gln Lys Pro Gly Phe Arg Ala
           355                 360                 365
   act atc ttc aca ttt gtg gtt tcc gcc ttc tgg cat gga act cga cct     1152
   Thr Ile Phe Thr Phe Val Val Ser Ala Phe Trp His Gly Thr Arg Pro
       370                 375                 380
   ggc tac tat ctc acc ttt gtg acc gct gcc atg tac cag tct gtt ggt     1200
   Gly Tyr Tyr Leu Thr Phe Val Thr Ala Ala Met Tyr Gln Ser Val Gly
   385                 390                 395                 400
   aag ttc ttc cga cga tac ctg cga ccc ttc ttc atg gag tct gat gga     1248
   Lys Phe Phe Arg Arg Tyr Leu Arg Pro Phe Phe Met Glu Ser Asp Gly
                   405                 410                 415
   aag act gcc ggt ccc tat aag atc tac tac gac att gtg tgt tgg atc     1296
   Lys Thr Ala Gly Pro Tyr Lys Ile Tyr Tyr Asp Ile Val Cys Trp Ile
               420                 425                 430
   gtt gtc caa acc gca ttt gga tac gct acc cag tcc ttt atg att cta     1344
   Val Val Gln Thr Ala Phe Gly Tyr Ala Thr Gln Ser Phe Met Ile Leu
           435                 440                 445
   gac ttc tgg ctg tcg ctc aag tgt tgg aag aac tcc tgg ttc ctg tac     1392
   Asp Phe Trp Leu Ser Leu Lys Cys Trp Lys Asn Ser Trp Phe Leu Tyr
       450                 455                 460
   cac att gct ctg ggc gcc atc ttt gca att tct agc ccc tac aag gca     1440
   His Ile Ala Leu Gly Ala Ile Phe Ala Ile Ser Ser Pro Tyr Lys Ala
   465                 470                 475                 480
   tgg gcg att ccc aag atc aag aaa aag cag gct gga gcc gtc act gac     1488
   Trp Ala Ile Pro Lys Ile Lys Lys Lys Gln Ala Gly Ala Val Thr Asp
                   485                 490                 495
   aag aag gac gcc aag gag gag gtg aag aag gac acc atc aag acc aag     1536
   Lys Lys Asp Ala Lys Glu Glu Val Lys Lys Asp Thr Ile Lys Thr Lys
               500                 505                 510
   taa                                                                  1539
<210> 17
   <211> 512
   <212> PRT
   <213> Yarrowia lipolytica
<400> 17
   Met Ala Phe Pro Trp Ala Asp Lys Trp Ala Ala Asp Ala Ser Ala Ser
   1               5                   10                  15
   Thr Gly Leu Pro Pro Asp Leu Leu Lys Ile Ala Phe Thr Leu Val Met
               20                  25                  30
   Ser Tyr Pro Leu Ser Ser Leu Met Lys Arg Leu Pro Asp Asp Ala Lys
           35                  40                  45
   Asn Leu Lys Ile Ile Tyr Ile Ile Ser Val Ser Ile Phe Tyr Met Val
       50                  55                  60
   Gly Val Phe Ser Leu Tyr Gly Gly Ala Ala Thr Leu Leu Phe Ser Ser
   65                  70                  75                  80
   Met Gly Thr Phe Phe Ile Thr Gln Trp Lys Ser Pro Tyr Met Pro Trp
                   85                  90                  95
   Val Asn Phe Gly Phe Val Met Thr His Leu Phe Val Asn His Leu Arg
               100                 105                 110
   Ser Gln Phe Phe Pro Glu Thr Tyr Asp Pro Asn Val Ile Asp Ile Thr
           115                 120                 125
   Gly Ala Gln Met Val Leu Cys Met Lys Leu Ser Ser Phe Gly Trp Asn
       130                 135                 140
   Val Tyr Asp Gly Trp Gln Ile Glu Lys Gly Glu Gln Leu Ser Glu Phe
   145                 150                 155                 160
   Gln Thr Lys Arg Ala Val Leu Lys His Pro Ser Leu Met Asp Phe Leu
                   165                 170                 175
   Ala Phe Val Phe Tyr Phe Pro Ser Ile Leu Thr Gly Pro Ser Tyr Asp
               180                 185                 190
   Tyr Met Glu Phe His Asn Trp Leu Asp Leu Ser Leu Phe Lys Glu Leu
           195                 200                 205
   Glu Lys Asp Lys Asp Pro Lys Arg Ala Ala Arg Arg Lys Arg His Lys
       210                 215                 220
   Ile Pro Arg Ser Gly Ile Ala Ala Ser Lys Lys Leu Ala Ala Gly Ile
   225                 230                 235                 240
   Phe Trp Ile Val Leu Trp Thr Gln Val Asp Ser Arg Ile Ser Thr Ala
                   245                 250                 255
   Tyr Ala Tyr Ser Asp Ala Phe Thr Lys Glu His Asn Ile Phe Gly Arg
               260                 265                 270
   Ile Val Tyr Leu Tyr Met Leu Gly Phe Met Tyr Arg Leu Lys Tyr Tyr
           275                 280                 285
   Gly Ala Trp Ser Ile Ser Glu Gly Ala Cys Ile Leu Ser Gly Leu Gly
       290                 295                 300
   Phe His Gly Val Asp Pro Lys Thr Gly Lys Tyr Lys Trp Asp Arg Val
   305                 310                 315                 320
   Gln Asn Val Asp Pro Trp Gly Phe Glu Thr Gly Gln Asn Thr Lys Ala
                   325                 330                 335
   Leu Leu Glu Ala Trp Asn Gln Asn Thr Asn Lys Trp Leu Arg Asn Tyr
               340                 345                 350
   Val Tyr Leu Arg Val Val Pro Lys Gly Gln Lys Pro Gly Phe Arg Ala
           355                 360                 365
   Thr Ile Phe Thr Phe Val Val Ser Ala Phe Trp His Gly Thr Arg Pro
       370                 375                 380
   Gly Tyr Tyr Leu Thr Phe Val Thr Ala Ala Met Tyr Gln Ser Val Gly
   385                 390                 395                 400
   Lys Phe Phe Arg Arg Tyr Leu Arg Pro Phe Phe Met Glu Ser Asp Gly
                   405                 410                 415
   Lys Thr Ala Gly Pro Tyr Lys Ile Tyr Tyr Asp Ile Val Cys Trp Ile
               420                 425                 430
   Val Val Gln Thr Ala Phe Gly Tyr Ala Thr Gln Ser Phe Met Ile Leu
           435                 440                 445
   Asp Phe Trp Leu Ser Leu Lys Cys Trp Lys Asn Ser Trp Phe Leu Tyr
       450                 455                 460
   His Ile Ala Leu Gly Ala Ile Phe Ala Ile Ser Ser Pro Tyr Lys Ala
   465                 470                 475                 480
   Trp Ala Ile Pro Lys Ile Lys Lys Lys Gln Ala Gly Ala Val Thr Asp
                   485                 490                 495
   Lys Lys Asp Ala Lys Glu Glu Val Lys Lys Asp Thr Ile Lys Thr Lys
               500                 505                 510
<210> 18
   <211> 7
   <212> PRT
   <213> Artificial Sequence
<220>
   <223> membrane bound O-acyltransferase motif
<220>
   <221> MISC_FEATURE
   <222> (2)..(2)
   <223> Xaa = Val [V] or Ile [I]
<220>
   <221> misc_feature
   <222> (4)..(5)
   <223> Xaa can be any naturally occurring amino acid
<400> 18
   Met Xaa Leu Xaa Xaa Lys Leu
   1               5
<210> 19
   <211> 8
   <212> PRT
   <213> Artificial Sequence
<220>
   <223> membrane bound O-acyltransferase motif
<220>
   <221> misc_feature
   <222> (2)..(2)
   <223> Xaa can be any naturally occurring amino acid
<220>
   <221> misc_feature
   <222> (6)..(7)
   <223> Xaa can be any naturally occurring amino acid
<400> 19
   Arg Xaa Lys Tyr Tyr Xaa Xaa Trp
   1               5
<210> 20
   <211> 6
   <212> PRT
   <213> Artificial Sequence
<220>
   <223> membrane bound O-acyltransferase motif
<220>
   <221> misc_feature
   <222> (3)..(3)
   <223> Xaa can be any naturally occurring amino acid
<400> 20
   Ser Ala Xaa Trp His Gly
   1 5
<210> 21
   <211> 20
   <212> PRT
   <213> Artificial Sequence
<220>
   <223> motif
<220>
   <221> misc_feature
   <222> (2)..(12)
   <223> Xaa can be any naturally occurring amino acid
<220>
   <221> misc_feature
   <222> (15)..(16)
   <223> Xaa can be any naturally occurring amino acid
<220>
   <221> MISC_FEATURE
   <222> (17)..(17)
   <223> Xaa = Thr [T] or Val [V]
<220>
   <221> misc_feature
   <222> (18)..(19)
   <223> Xaa can be any naturally occurring amino acid
<300>
   <302> GENES ENCODING A NOVEL TYPE OF LYSOPHOPHATIDYLCHOLINE ACYLTRANSFERASES AND THEIR USE TO INCREASE TRIACYLGLYCEROL PRODUCTION AND/OR MODIFY FATTY ACID COMPOSITION
<310> U.S. Patent Publication No. 2008-0145867-A1
   <311> 2007-06-15
   <312> 2008-06-19
   <313> (1)..(15)
<400> 21
   Glu Xaa Xaa Xaa Xaa Xaa Xaa Xaa Xaa Xaa Xaa Xaa Trp Asn Xaa Xaa
   1 5 10 15
   Xaa Xaa Xaa Trp
   20
<210> 22
   <211> 1870
   <212> DNA
   <213> Saccharomyces cerevisiae
<220>
   <221> CDS
   <222> (3)..(1862)
   <223> synthetic Ale1 (codon-optimized for Yarrowia lipolytica)
<400> 22
   ac atg tac aac ccc gtg gac gca gtg ttg act aag att att aca aac          47
      Met Tyr Asn Pro Val Asp Ala Val Leu Thr Lys Ile Ile Thr Asn
      1               5                   10                  15
   tac gga att gat tct ttt acc ctg cga tat gcc att tgt ctg ttg gga      95
   Tyr Gly Ile Asp Ser Phe Thr Leu Arg Tyr Ala Ile Cys Leu Leu Gly
                   20                  25                  30
   tct ttt cct ctt aac gct att ctg aag cgg att cct gaa aag cga atc     143
   Ser Phe Pro Leu Asn Ala Ile Leu Lys Arg Ile Pro Glu Lys Arg Ile
               35                  40                  45
   ggc ctg aag tgt tgt ttt atc att tct atg tcc atg ttt tat ctc ttc     191
   Gly Leu Lys Cys Cys Phe Ile Ile Ser Met Ser Met Phe Tyr Leu Phe
           50                  55                  60
   ggc gtt ctg aat ctc gtg agc gga ttt cga acc ctc ttc att tcc aca     239
   Gly Val Leu Asn Leu Val Ser Gly Phe Arg Thr Leu Phe Ile Ser Thr
       65                  70                  75
   atg ttc aca tac ctt atc tct cgg ttc tac cga tcc aag ttt atg ccc     287
   Met Phe Thr Tyr Leu Ile Ser Arg Phe Tyr Arg Ser Lys Phe Met Pro
   80                  85                  90                  95
   cat ctc aac ttc atg ttc gtc atg ggc cac ttg gct atc aac cac att     335
   His Leu Asn Phe Met Phe Val Met Gly His Leu Ala Ile Asn His Ile
                   100                 105                 110
   cat gct cag ttc ctg aac gaa caa act caa acg acc gtc gat att aca     383
   His Ala Gln Phe Leu Asn Glu Gln Thr Gln Thr Thr Val Asp Ile Thr
               115                 120                 125
   tcc tcg cag atg gtc ctg gct atg aag ctg aca agc ttt gcc tgg tct     431
   Ser Ser Gln Met Val Leu Ala Met Lys Leu Thr Ser Phe Ala Trp Ser
           130                 135                 140
   tac tat gac ggt tcg tgt acg agc gag tcc gac ttc aag gac ctt acc     479
   Tyr Tyr Asp Gly Ser Cys Thr Ser Glu Ser Asp Phe Lys Asp Leu Thr
       145                 150                 155
   gaa cac cag aag tcc cga gcc gtc cga ggc cat cct ccc ctt ctg aaa     527
   Glu His Gln Lys Ser Arg Ala Val Arg Gly His Pro Pro Leu Leu Lys
   160                 165                 170                 175
   ttt ttg gct tac gcc ttt ttc tac tct acc ctt ctc acc ggt ccc tcc     575
   Phe Leu Ala Tyr Ala Phe Phe Tyr Ser Thr Leu Leu Thr Gly Pro Ser
                   180                 185                 190
   ttc gat tac gct gat ttc gac tct tgg ctg aac tgc gaa atg ttc cgg     623
   Phe Asp Tyr Ala Asp Phe Asp Ser Trp Leu Asn Cys Glu Met Phe Arg
               195                 200                 205
   gac ctt ccc gag tcc aag aaa ccc atg cga aga cat cat cct ggt gag     671
   Asp Leu Pro Glu Ser Lys Lys Pro Met Arg Arg His His Pro Gly Glu
           210                 215                 220
   cgg cgt cag att ccc aag aac ggc aag ctc gcc ctg tgg aag gtt gtc     719
   Arg Arg Gln Ile Pro Lys Asn Gly Lys Leu Ala Leu Trp Lys Val Val
       225                 230                 235
   cag ggc ctc gcc tgg atg att ctg agc acg ttg ggt atg aag cac ttc     767
   Gln Gly Leu Ala Trp Met Ile Leu Ser Thr Leu Gly Met Lys His Phe
   240                 245                 250                 255
   ccc gtg aag tac gtg ctg gac aag gac gga ttt cct acc cgt tcc ttt     815
   Pro Val Lys Tyr Val Leu Asp Lys Asp Gly Phe Pro Thr Arg Ser Phe
                   260                 265                 270
   atc ttc cgt att cat tat ctg ttt ctg ctg gga ttc atc cac cga ttt     863
   Ile Phe Arg Ile His Tyr Leu Phe Leu Leu Gly Phe Ile His Arg Phe
               275                 280                 285
   aag tat tac gct gcg tgg acg att agc gaa ggt tcg tgc att ctc tgt      911
   Lys Tyr Tyr Ala Ala Trp Thr Ile Ser Glu Gly Ser Cys Ile Leu Cys
           290                 295                 300
   ggt ctt ggt tat aat gga tac gat tct aag acc cag aag atc cgg tgg      959
   Gly Leu Gly Tyr Asn Gly Tyr Asp Ser Lys Thr Gln Lys Ile Arg Trp
       305                 310                 315
   gat cga gtg cgg aat att gat att tgg aca gtg gag act gca caa aac     1007
   Asp Arg Val Arg Asn Ile Asp Ile Trp Thr Val Glu Thr Ala Gln Asn
   320                 325                 330                 335
   acc cga gag atg ctg gaa gcg tgg aac atg aat act aac aaa tgg ctg     1055
   Thr Arg Glu Met Leu Glu Ala Trp Asn Met Asn Thr Asn Lys Trp Leu
                   340                 345                 350
   aag tat agc gtg tat ctt aga gtg act aag aag ggt aag aag cca ggt     1103
   Lys Tyr Ser Val Tyr Leu Arg Val Thr Lys Lys Gly Lys Lys Pro Gly
               355                 360                 365
   ttt cga tct acc ctg ttt acc ttc ctg acc tcc gcc ttt tgg cac ggt     1151
   Phe Arg Ser Thr Leu Phe Thr Phe Leu Thr Ser Ala Phe Trp His Gly
           370                 375                 380
   acc cgt cct gga tac tac ctt acc ttc gca act ggt gcc ctg tac caa     1199
   Thr Arg Pro Gly Tyr Tyr Leu Thr Phe Ala Thr Gly Ala Leu Tyr Gln
       385                 390                 395
   acc tgt gga aag atc tat aga cga aac ttt cgt ccc atc ttt ctg aga     1247
   Thr Cys Gly Lys Ile Tyr Arg Arg Asn Phe Arg Pro Ile Phe Leu Arg
   400                 405                 410                 415
   gaa gat ggc gtg aca cct ctc ccg tcc aag aag att tac gac ctg gtc     1295
   Glu Asp Gly Val Thr Pro Leu Pro Ser Lys Lys Ile Tyr Asp Leu Val
                   420                 425                 430
   ggc att tac gct att aag ctg gcc ttt ggt tac atg gtt caa ccc ttc     1343
   Gly Ile Tyr Ala Ile Lys Leu Ala Phe Gly Tyr Met Val Gln Pro Phe
               435                 440                 445
   att atc ctt gac ctg aag ccc tct ctt atg gtt tgg gga tcc gtg tat     1391
   Ile Ile Leu Asp Leu Lys Pro Ser Leu Met Val Trp Gly Ser Val Tyr
           450                 455                 460
   ttc tac gtg cat att att gtg gcc ttc tcg ttc ttt ctg ttc cga gga     1439
   Phe Tyr Val His Ile Ile Val Ala Phe Ser Phe Phe Leu Phe Arg Gly
       465                 470                 475
   cca tac gct aag cag gtt act gaa ttt ttc aaa agc aag caa ccg aag     1487
   Pro Tyr Ala Lys Gln Val Thr Glu Phe Phe Lys Ser Lys Gln Pro Lys
   480                 485                 490                 495
   gag atc ttc atc cga aag cag aag aag ttg gaa aaa gac atc tct gcc     1535
   Glu Ile Phe Ile Arg Lys Gln Lys Lys Leu Glu Lys Asp Ile Ser Ala
                   500                 505                 510
   tct tcc ccc aac ctc gga ggt att ctt aag gca aaa atc gaa cat gag     1583
   Ser Ser Pro Asn Leu Gly Gly Ile Leu Lys Ala Lys Ile Glu His Glu
               515                 520                 525
   aag gga aag acg gca gag gag gaa gag atg aac ttg ggc att cca ccc     1631
   Lys Gly Lys Thr Ala Glu Glu Glu Glu Met Asn Leu Gly Ile Pro Pro
           530                 535                 540
   atc gaa ctg gag aag tgg gac aac gcc aag gag gac tgg gag gat ttc        1679
   Ile Glu Leu Glu Lys Trp Asp Asn Ala Lys Glu Asp Trp Glu Asp Phe
       545                 550                 555
   tgc aag gac tac aag gag tgg cgg aac aag aac gga ctg gaa att gaa        1727
   Cys Lys Asp Tyr Lys Glu Trp Arg Asn Lys Asn Gly Leu Glu Ile Glu
   560                 565                 570                 575
   gag gag aac ctg tcc aag gcc ttc gag cga ttt aag cag gaa ttt tcc        1775
   Glu Glu Asn Leu Ser Lys Ala Phe Glu Arg Phe Lys Gln Glu Phe Ser
                   580                 585                 590
   aac gct gcg tcg ggc tct ggt gaa cgg gtt cgg aaa atg tcc ttc tcc        1823
   Asn Ala Ala Ser Gly Ser Gly Glu Arg Val Arg Lys Met Ser Phe Ser
               595                 600                 605
   gga tat tct cct aaa ccc atc tcg aag aaa gaa gaa tag gcggccgc           1870
   Gly Tyr Ser Pro Lys Pro Ile Ser Lys Lys Glu Glu
           610                 615
<210> 23
   <211> 619
   <212> PRT
   <213> Saccharomyces cerevisiae
<400> 23
   Met Tyr Asn Pro Val Asp Ala Val Leu Thr Lys Ile Ile Thr Asn Tyr
   1               5                   10                  15
   Gly Ile Asp Ser Phe Thr Leu Arg Tyr Ala Ile Cys Leu Leu Gly Ser
               20                  25                  30
   Phe Pro Leu Asn Ala Ile Leu Lys Arg Ile Pro Glu Lys Arg Ile Gly
           35                  40                  45
   Leu Lys Cys Cys Phe Ile Ile Ser Met Ser Met Phe Tyr Leu Phe Gly
       50                  55                  60
   Val Leu Asn Leu Val Ser Gly Phe Arg Thr Leu Phe Ile Ser Thr Met
   65                  70                  75                  80
   Phe Thr Tyr Leu Ile Ser Arg Phe Tyr Arg Ser Lys Phe Met Pro His
                   85                  90                  95
   Leu Asn Phe Met Phe Val Met Gly His Leu Ala Ile Asn His Ile His
               100                 105                 110
   Ala Gln Phe Leu Asn Glu Gln Thr Gln Thr Thr Val Asp Ile Thr Ser
           115                 120                 125
   Ser Gln Met Val Leu Ala Met Lys Leu Thr Ser Phe Ala Trp Ser Tyr
       130                 135                 140
   Tyr Asp Gly Ser Cys Thr Ser Glu Ser Asp Phe Lys Asp Leu Thr Glu
   145 150 155 160
   His Gln Lys Ser Arg Ala Val Arg Gly His Pro Pro Leu Leu Lys Phe
   165 170 175
   Leu Ala Tyr Ala Phe Phe Tyr Ser Thr Leu Leu Thr Gly Pro Ser Phe
   180 185 190
   Asp Tyr Ala Asp Phe Asp Ser Trp Leu Asn Cys Glu Met Phe Arg Asp
   195 200 205
   Leu Pro Glu Ser Lys Lys Pro Met Arg Arg His His Pro Gly Glu Arg
   210 215 220
   Arg Gln Ile Pro Lys Asn Gly Lys Leu Ala Leu Trp Lys Val Val Gln
   225 230 235 240
   Gly Leu Ala Trp Met Ile Leu Ser Thr Leu Gly Met Lys His Phe Pro
   245 250 255
   Val Lys Tyr Val Leu Asp Lys Asp Gly Phe Pro Thr Arg Ser Phe Ile
   260 265 270
   Phe Arg Ile His Tyr Leu Phe Leu Leu Gly Phe Ile His Arg Phe Lys
   275 280 285
   Tyr Tyr Ala Ala Trp Thr Ile Ser Glu Gly Ser Cys Ile Leu Cys Gly
   290 295 300
   Leu Gly Tyr Asn Gly Tyr Asp Ser Lys Thr Gln Lys Ile Arg Trp Asp
   305 310 315 320
   Arg Val Arg Asn Ile Asp Ile Trp Thr Val Glu Thr Ala Gln Asn Thr
   325 330 335
   Arg Glu Met Leu Glu Ala Trp Asn Met Asn Thr Asn Lys Trp Leu Lys
   340 345 350
   Tyr Ser Val Tyr Leu Arg Val Thr Lys Lys Gly Lys Lys Pro Gly Phe
   355 360 365
   Arg Ser Thr Leu Phe Thr Phe Leu Thr Ser Ala Phe Trp His Gly Thr
   370 375 380
   Arg Pro Gly Tyr Tyr Leu Thr Phe Ala Thr Gly Ala Leu Tyr Gln Thr
   385 390 395 400
   Cys Gly Lys Ile Tyr Arg Arg Asn Phe Arg Pro Ile Phe Leu Arg Glu
                   405                 410                 415
   Asp Gly Val Thr Pro Leu Pro Ser Lys Lys Ile Tyr Asp Leu Val Gly
               420                 425                 430
   Ile Tyr Ala Ile Lys Leu Ala Phe Gly Tyr Met Val Gln Pro Phe Ile
           435                 440                 445
   Ile Leu Asp Leu Lys Pro Ser Leu Met Val Trp Gly Ser Val Tyr Phe
       450                 455                 460
   Tyr Val His Ile Ile Val Ala Phe Ser Phe Phe Leu Phe Arg Gly Pro
   465                 470                 475                 480
   Tyr Ala Lys Gln Val Thr Glu Phe Phe Lys Ser Lys Gln Pro Lys Glu
                   485                 490                 495
   Ile Phe Ile Arg Lys Gln Lys Lys Leu Glu Lys Asp Ile Ser Ala Ser
               500                 505                 510
   Ser Pro Asn Leu Gly Gly Ile Leu Lys Ala Lys Ile Glu His Glu Lys
           515                 520                 525
   Gly Lys Thr Ala Glu Glu Glu Glu Met Asn Leu Gly Ile Pro Pro Ile
       530                 535                 540
   Glu Leu Glu Lys Trp Asp Asn Ala Lys Glu Asp Trp Glu Asp Phe Cys
   545                 550                 555                 560
   Lys Asp Tyr Lys Glu Trp Arg Asn Lys Asn Gly Leu Glu Ile Glu Glu
                   565                 570                 575
   Glu Asn Leu Ser Lys Ala Phe Glu Arg Phe Lys Gln Glu Phe Ser Asn
               580                 585                 590
   Ala Ala Ser Gly Ser Gly Glu Arg Val Arg Lys Met Ser Phe Ser Gly
           595                 600                 605
   Tyr Ser Pro Lys Pro Ile Ser Lys Lys Glu Glu
       610                 615
<210> 24
   <211> 849
   <212> DNA
   <213> Caenorhabditis elegans
<220>
   <221> CDS
   <222> (1)..(849)
   <223> GenBank Accession No. CAA98276; "clone T06E8.1"
<300>
   <302> METHOD FOR THE PRODUCTION OF POLYUNSATURATED FATTY ACIDS
<310> US 2006-0168687-A1
   <311> 2004-01-29
   <312> 2006-07-27
   <313> (1)..(849)
<300>
   <302> METHOD FOR THE PRODUCTION OF POLYUNSATURATED FATTY ACIDS
<310> WO 2004/076617
   <311> 2004-01-29
   <312> 2004-09-10
   <313> (1)..(849)
<400> 24
   atg gag aac ttc tgg tcg atc gtc gtg ttt ttt cta ctc tca att ctc        48
   Met Glu Asn Phe Trp Ser Ile Val Val Phe Phe Leu Leu Ser Ile Leu
   1               5                   10                  15
   ttc att tta tat aac ata tcg aca gta tgc cac tac tat atg cgg att        96
   Phe Ile Leu Tyr Asn Ile Ser Thr Val Cys His Tyr Tyr Met Arg Ile
               20                  25                  30
   tcg ttt tat tac ttc aca att tta ttg cat gga atg gaa gtt tgt gtt       144
   Ser Phe Tyr Tyr Phe Thr Ile Leu Leu His Gly Met Glu Val Cys Val
           35                  40                  45
   aca atg atc cct tct tgg cta aat ggg aag ggt gct gat tac gtg ttt       192
   Thr Met Ile Pro Ser Trp Leu Asn Gly Lys Gly Ala Asp Tyr Val Phe
       50                  55                  60
   cac tcg ttt ttc tat tgg tgt aaa tgg act ggt gtt cat aca aca gtc       240
   His Ser Phe Phe Tyr Trp Cys Lys Trp Thr Gly Val His Thr Thr Val
   65                  70                  75                  80
   tat gga tat gaa aaa aca caa gtt gaa ggt ccg gct gta gtt att tgt       288
   Tyr Gly Tyr Glu Lys Thr Gln Val Glu Gly Pro Ala Val Val Ile Cys
                   85                  90                  95
   aat cat cag agt tct ctc gac att cta tcg atg gca tca atc tgg ccg       336
   Asn His Gln Ser Ser Leu Asp Ile Leu Ser Met Ala Ser Ile Trp Pro
               100                 105                 110
   aag aat tgt gtt gta atg atg aaa cga att ctt gcc tat gtt cca ttc       384
   Lys Asn Cys Val Val Met Met Lys Arg Ile Leu Ala Tyr Val Pro Phe
           115                 120                 125
   ttc aat ctc gga gcc tac ttt tcc aac aca atc ttc atc gat cga tat       432
   Phe Asn Leu Gly Ala Tyr Phe Ser Asn Thr Ile Phe Ile Asp Arg Tyr
       130                 135                 140
   aac cgt gaa cgt gcg atg gct tca gtt gat tat tgt gca tct gaa atg       480
   Asn Arg Glu Arg Ala Met Ala Ser Val Asp Tyr Cys Ala Ser Glu Met
   145                 150                 155                 160
   aag aac aga aat ctt aaa ctt tgg gta ttt ccg gaa gga aca aga aat       528
   Lys Asn Arg Asn Leu Lys Leu Trp Val Phe Pro Glu Gly Thr Arg Asn
                   165                 170                 175
   cgt gaa gga ggg ttc att cca ttc aag aaa gga gca ttc aat att gca       576
   Arg Glu Gly Gly Phe Ile Pro Phe Lys Lys Gly Ala Phe Asn Ile Ala
               180                 185                 190
   gtt cgt gcg cag att ccc att att cca gtt gta ttc tca gac tat cgg      624
   Val Arg Ala Gln Ile Pro Ile Ile Pro Val Val Phe Ser Asp Tyr Arg
           195                 200                 205
   gat ttc tac tca aag cca ggc cga tat ttc aag aat gat gga gaa gtt      672
   Asp Phe Tyr Ser Lys Pro Gly Arg Tyr Phe Lys Asn Asp Gly Glu Val
       210                 215                 220
   gtt att cga gtt ctg gat gcg att cca aca aaa ggg ctc act ctt gat      720
   Val Ile Arg Val Leu Asp Ala Ile Pro Thr Lys Gly Leu Thr Leu Asp
   225                 230                 235                 240
   gac gtc agc gag ttg tct gat atg tgt cgg gac gtt atg ttg gca gcc      768
   Asp Val Ser Glu Leu Ser Asp Met Cys Arg Asp Val Met Leu Ala Ala
                   245                 250                 255
   tat aag gaa gtt act cta gaa gct cag caa cga aat gcg aca cgg cgt      816
   Tyr Lys Glu Val Thr Leu Glu Ala Gln Gln Arg Asn Ala Thr Arg Arg
               260                 265                 270
   gga gaa aca aaa gac ggg aag aaa tct gag taa                          849
   Gly Glu Thr Lys Asp Gly Lys Lys Ser Glu
           275                 280
<210> 25
   <211> 282
   <212> PRT
   <213> Caenorhabditis elegans
<400> 25
   Met Glu Asn Phe Trp Ser Ile Val Val Phe Phe Leu Leu Ser Ile Leu
   1               5                   10                  15
   Phe Ile Leu Tyr Asn Ile Ser Thr Val Cys His Tyr Tyr Met Arg Ile
               20                  25                  30
   Ser Phe Tyr Tyr Phe Thr Ile Leu Leu His Gly Met Glu Val Cys Val
           35                  40                  45
   Thr Met Ile Pro Ser Trp Leu Asn Gly Lys Gly Ala Asp Tyr Val Phe
       50                  55                  60
   His Ser Phe Phe Tyr Trp Cys Lys Trp Thr Gly Val His Thr Thr Val
   65                  70                  75                  80
   Tyr Gly Tyr Glu Lys Thr Gln Val Glu Gly Pro Ala Val Val Ile Cys
                   85                  90                  95
   Asn His Gln Ser Ser Leu Asp Ile Leu Ser Met Ala Ser Ile Trp Pro
               100                 105                 110
   Lys Asn Cys Val Val Met Met Lys Arg Ile Leu Ala Tyr Val Pro Phe
           115                 120                 125
   Phe Asn Leu Gly Ala Tyr Phe Ser Asn Thr Ile Phe Ile Asp Arg Tyr
   130 135 140
   Asn Arg Glu Arg Ala Met Ala Ser Val Asp Tyr Cys Ala Ser Glu Met
   145 150 155 160
   Lys Asn Arg Asn Leu Lys Leu Trp Val Phe Pro Glu Gly Thr Arg Asn
   165 170 175
   Arg Glu Gly Gly Phe Ile Pro Phe Lys Lys Gly Ala Phe Asn Ile Ala
   180 185 190
   Val Arg Ala Gln Ile Pro Ile Ile Pro Val Val Phe Ser Asp Tyr Arg
   195 200 205
   Asp Phe Tyr Ser Lys Pro Gly Arg Tyr Phe Lys Asn Asp Gly Glu Val
   210 215 220
   Val Ile Arg Val Leu Asp Ala Ile Pro Thr Lys Gly Leu Thr Leu Asp
   225 230 235 240
   Asp Val Ser Glu Leu Ser Asp Met Cys Arg Asp Val Met Leu Ala Ala
   245 250 255
   Tyr Lys Glu Val Thr Leu Glu Ala Gln Gln Arg Asn Ala Thr Arg Arg
   260 265 270
   Gly Glu Thr Lys Asp Gly Lys Lys Ser Glu
   275 280
<210> 26
   <211> 859
   <212> DNA
   <213> Caenorhabditis elegans
<220>
   <221> CDS
   <222> (3)..(851)
   <223> synthetic LPCAT (codon-optimized for Yarrowia lipolytica)
<400> 26
   cc atg gag aac ttc tgg tcc atc gtc gtg ttc ttt ctg ctc tcc att      47
      Met Glu Asn Phe Trp Ser Ile Val Val Phe Phe Leu Leu Ser Ile
      1               5                   10                  15
   ctg ttc atc ctc tac aac att tcg aca gtc tgc cac tac tac atg cga     95
   Leu Phe Ile Leu Tyr Asn Ile Ser Thr Val Cys His Tyr Tyr Met Arg
                   20                  25                  30
   atc tcc ttc tac tac ttt acc atc ctg ctt cac ggc atg gag gtg tgc    143
   Ile Ser Phe Tyr Tyr Phe Thr Ile Leu Leu His Gly Met Glu Val Cys
               35                  40                  45
   gtt acc atg att ccc tct tgg ctc aac ggc aag ggt gcc gac tac gtg    191
   Val Thr Met Ile Pro Ser Trp Leu Asn Gly Lys Gly Ala Asp Tyr Val
           50                  55                  60
   ttt cac tcg ttc ttc tac tgg tgc aag tgg act gga gtc cac acc act      239
   Phe His Ser Phe Phe Tyr Trp Cys Lys Trp Thr Gly Val His Thr Thr
       65                  70                  75
   gtg tat ggc tac gag aag acc cag gtc gaa ggt cct gcc gtg gtc atc      287
   Val Tyr Gly Tyr Glu Lys Thr Gln Val Glu Gly Pro Ala Val Val Ile
   80                  85                  90                  95
   tgc aac cat cag tcc tcg ctc gac att ctg tct atg gct tcc atc tgg      335
   Cys Asn His Gln Ser Ser Leu Asp Ile Leu Ser Met Ala Ser Ile Trp
                   100                 105                 110
   ccc aag aac tgt gtt gtc atg atg aag cgg att ctt gcc tac gtt ccc      383
   Pro Lys Asn Cys Val Val Met Met Lys Arg Ile Leu Ala Tyr Val Pro
               115                 120                 125
   ttc ttc aac ctg gga gcc tac ttt tcc aac acc atc ttc atc gac cga      431
   Phe Phe Asn Leu Gly Ala Tyr Phe Ser Asn Thr Ile Phe Ile Asp Arg
           130                 135                 140
   tac aac cga gag cga gct atg gct tct gtc gac tac tgt gcc tcc gag      479
   Tyr Asn Arg Glu Arg Ala Met Ala Ser Val Asp Tyr Cys Ala Ser Glu
       145                 150                 155
   atg aag aac cga aac ctg aag ctc tgg gtg ttt ccc gaa ggc act cgg      527
   Met Lys Asn Arg Asn Leu Lys Leu Trp Val Phe Pro Glu Gly Thr Arg
   160                 165                 170                 175
   aat cga gag ggt gga ttc att ccc ttc aag aaa ggt gcc ttc aac atc      575
   Asn Arg Glu Gly Gly Phe Ile Pro Phe Lys Lys Gly Ala Phe Asn Ile
                   180                 185                 190
   gct gtt cga gcc cag att ccc atc att cct gtc gtg ttc tct gac tat      623
   Ala Val Arg Ala Gln Ile Pro Ile Ile Pro Val Val Phe Ser Asp Tyr
               195                 200                 205
   cga gac ttc tac tcc aag cct ggc cga tac ttc aag aac gat gga gag      671
   Arg Asp Phe Tyr Ser Lys Pro Gly Arg Tyr Phe Lys Asn Asp Gly Glu
           210                 215                 220
   gtc gtg atc cga gtc ctg gat gcc att ccc acc aag ggt ctg acc ctc      719
   Val Val Ile Arg Val Leu Asp Ala Ile Pro Thr Lys Gly Leu Thr Leu
       225                 230                 235
   gat gac gtc tct gag ctt tcg gac atg tgt cga gac gtc atg ctg gct      767
   Asp Asp Val Ser Glu Leu Ser Asp Met Cys Arg Asp Val Met Leu Ala
   240                 245                 250                 255
   gcc tac aag gaa gtt acc ctc gag gct cag caa cga aac gcc act cga      815
   Ala Tyr Lys Glu Val Thr Leu Glu Ala Gln Gln Arg Asn Ala Thr Arg
                   260                 265                 270
   aga gga gag acc aag gac ggc aag aaa tcc gag taa gcggccgc             859
   Arg Gly Glu Thr Lys Asp Gly Lys Lys Ser Glu
               275                 280
<210> 27
   <211> 282
   <212> PRT
   <213> Caenorhabditis elegans
<400> 27
   Met Glu Asn Phe Trp Ser Ile Val Val Phe Phe Leu Leu Ser Ile Leu
   1               5                   10                  15
   Phe Ile Leu Tyr Asn Ile Ser Thr Val Cys His Tyr Tyr Met Arg Ile
               20                  25                  30
   Ser Phe Tyr Tyr Phe Thr Ile Leu Leu His Gly Met Glu Val Cys Val
           35                  40                  45
   Thr Met Ile Pro Ser Trp Leu Asn Gly Lys Gly Ala Asp Tyr Val Phe
       50                  55                  60
   His Ser Phe Phe Tyr Trp Cys Lys Trp Thr Gly Val His Thr Thr Val
   65                  70                  75                  80
   Tyr Gly Tyr Glu Lys Thr Gln Val Glu Gly Pro Ala Val Val Ile Cys
                   85                  90                  95
   Asn His Gln Ser Ser Leu Asp Ile Leu Ser Met Ala Ser Ile Trp Pro
               100                 105                 110
   Lys Asn Cys Val Val Met Met Lys Arg Ile Leu Ala Tyr Val Pro Phe
           115                 120                 125
   Phe Asn Leu Gly Ala Tyr Phe Ser Asn Thr Ile Phe Ile Asp Arg Tyr
       130                 135                 140
   Asn Arg Glu Arg Ala Met Ala Ser Val Asp Tyr Cys Ala Ser Glu Met
   145                 150                 155                 160
   Lys Asn Arg Asn Leu Lys Leu Trp Val Phe Pro Glu Gly Thr Arg Asn
                   165                 170                 175
   Arg Glu Gly Gly Phe Ile Pro Phe Lys Lys Gly Ala Phe Asn Ile Ala
               180                 185                 190
   Val Arg Ala Gln Ile Pro Ile Ile Pro Val Val Phe Ser Asp Tyr Arg
           195                 200                 205
   Asp Phe Tyr Ser Lys Pro Gly Arg Tyr Phe Lys Asn Asp Gly Glu Val
       210                 215                 220
   Val Ile Arg Val Leu Asp Ala Ile Pro Thr Lys Gly Leu Thr Leu Asp
   225                 230                 235                 240
   Asp Val Ser Glu Leu Ser Asp Met Cys Arg Asp Val Met Leu Ala Ala
                   245                 250                 255
   Tyr Lys Glu Val Thr Leu Glu Ala Gln Gln Arg Asn Ala Thr Arg Arg
   260 265 270
   Gly Glu Thr Lys Asp Gly Lys Lys Ser Glu
   275 280
<210> 28
   <211> 945
   <212> DNA
   <213> Mortierella alpina
<220>
   <221> CDS
   <222> (1)..(945)
   <223> LPAAT1
<300>
   <302> High eicosapentaenoic acid producing strains of Yarrowia lipolytica
<310> US-2006-0115881-A1
   <311> 2005-11-02
   <312> 2006-06-01
   <313> (1)..(945)
<300>
   <302> High eicosapentaenoic acid producing strains of Yarrowia lipolytica
<310> WO 2006/052870
   <311> 2005-11-03
   <312> 2006-05-18
   <313> (1)..(945)
<400> 28
   atg tcc ata ggt tct tcc aat cct gtc ctg ctg gca gcg atc ccc ttc         48
   Met Ser Ile Gly Ser Ser Asn Pro Val Leu Leu Ala Ala Ile Pro Phe
   1               5                   10                  15
   gtc tac ctc ttc gtc ctc cct cgt gtc ctc gcc ttc ctc cct caa aag         96
   Val Tyr Leu Phe Val Leu Pro Arg Val Leu Ala Phe Leu Pro Gln Lys
               20                  25                  30
   gcc cag ttc ctc gca aaa tgc atc gtg gtc ttg atc gcc acc ctt atc        144
   Ala Gln Phe Leu Ala Lys Cys Ile Val Val Leu Ile Ala Thr Leu Ile
           35                  40                  45
   atg tcc gtc gca ggc tgc ttc att tcc atc gtc tgt gcg ctc ctc gat        192
   Met Ser Val Ala Gly Cys Phe Ile Ser Ile Val Cys Ala Leu Leu Asp
       50                  55                  60
   aaa cgc tat gtg atc aac tac gtc gtc tca aga ctc ttc tca ttc ctc        240
   Lys Arg Tyr Val Ile Asn Tyr Val Val Ser Arg Leu Phe Ser Phe Leu
   65                  70                  75                  80
   gct gca aga ccc tgc ggt gtc acc tac aag atc gtc ggc gag gaa cat        288
   Ala Ala Arg Pro Cys Gly Val Thr Tyr Lys Ile Val Gly Glu Glu His
                   85                  90                  95
   ctg gac aag tac ccc gcc att gtc gtc tgc aac cac cag agc tcc atg        336
   Leu Asp Lys Tyr Pro Ala Ile Val Val Cys Asn His Gln Ser Ser Met
               100                 105                 110
   gac atg atg gtc ctg gga cgc gtc ttc cca aag cac tgt gtc gtc atg        384
   Asp Met Met Val Leu Gly Arg Val Phe Pro Lys His Cys Val Val Met
           115                 120                 125
   gca aag aag gaa ctt ctt tac ttt ccg ttc ctg ggc atg ttt atg aag       432
   Ala Lys Lys Glu Leu Leu Tyr Phe Pro Phe Leu Gly Met Phe Met Lys
       130                 135                 140
   ctg agt aac gcc atc ttc att gac cgc aag aac cac aag aag gcg atc       480
   Leu Ser Asn Ala Ile Phe Ile Asp Arg Lys Asn His Lys Lys Ala Ile
   145                 150                 155                 160
   gag tcc acc acc caa gct gtc gcc gac atg aag aag cac aac tct gga       528
   Glu Ser Thr Thr Gln Ala Val Ala Asp Met Lys Lys His Asn Ser Gly
                   165                 170                 175
   atc tgg att ttc ccc gaa gga aca cgt tcc cgc ttg gac aag gcc gat       576
   Ile Trp Ile Phe Pro Glu Gly Thr Arg Ser Arg Leu Asp Lys Ala Asp
               180                 185                 190
   ctc ttg ccc ttc aag aag gga gcc ttc cac ctc gcc att caa gcc caa       624
   Leu Leu Pro Phe Lys Lys Gly Ala Phe His Leu Ala Ile Gln Ala Gln
           195                 200                 205
   ctc ccg atc ctc ccc atc atc tcg caa gga tac tca cac atc tac gat       672
   Leu Pro Ile Leu Pro Ile Ile Ser Gln Gly Tyr Ser His Ile Tyr Asp
       210                 215                 220
   tcg tca aaa cgc tac ttc ccc ggt gga gag ctc gag atc aga gtc ctg       720
   Ser Ser Lys Arg Tyr Phe Pro Gly Gly Glu Leu Glu Ile Arg Val Leu
   225                 230                 235                 240
   gaa cct atc ccc acc acg gga ttg acc aca gac gat gtg aac gac ctg       768
   Glu Pro Ile Pro Thr Thr Gly Leu Thr Thr Asp Asp Val Asn Asp Leu
                   245                 250                 255
   atg gac aag act cgc aac ctg atg ctg aag cac ctc aag gag atg gat       816
   Met Asp Lys Thr Arg Asn Leu Met Leu Lys His Leu Lys Glu Met Asp
               260                 265                 270
   tct caa tac tcc tcc tcc acc gct gaa aac gga tcg acc cat att gac       864
   Ser Gln Tyr Ser Ser Ser Thr Ala Glu Asn Gly Ser Thr His Ile Asp
           275                 280                 285
   gcc gat atc gca aag tca act gcc aca tcg atc gga aac acg gac gat       912
   Ala Asp Ile Ala Lys Ser Thr Ala Thr Ser Ile Gly Asn Thr Asp Asp
       290                 295                 300
   gct atc aca aag agg agg aca cca aaa gag tag                           945
   Ala Ile Thr Lys Arg Arg Thr Pro Lys Glu
   305                 310
<210> 29
   <211> 314
   <212> PRT
   <213> Mortierella alpina
<400> 29
   Met Ser Ile Gly Ser Ser Asn Pro Val Leu Leu Ala Ala Ile Pro Phe
   1               5                   10                  15
   Val Tyr Leu Phe Val Leu Pro Arg Val Leu Ala Phe Leu Pro Gln Lys
               20                  25                  30
   Ala Gln Phe Leu Ala Lys Cys Ile Val Val Leu Ile Ala Thr Leu Ile
           35                  40                  45
   Met Ser Val Ala Gly Cys Phe Ile Ser Ile Val Cys Ala Leu Leu Asp
       50                  55                  60
   Lys Arg Tyr Val Ile Asn Tyr Val Val Ser Arg Leu Phe Ser Phe Leu
   65                  70                  75                  80
   Ala Ala Arg Pro Cys Gly Val Thr Tyr Lys Ile Val Gly Glu Glu His
                   85                  90                  95
   Leu Asp Lys Tyr Pro Ala Ile Val Val Cys Asn His Gln Ser Ser Met
               100                 105                 110
   Asp Met Met Val Leu Gly Arg Val Phe Pro Lys His Cys Val Val Met
           115                 120                 125
   Ala Lys Lys Glu Leu Leu Tyr Phe Pro Phe Leu Gly Met Phe Met Lys
       130                 135                 140
   Leu Ser Asn Ala Ile Phe Ile Asp Arg Lys Asn His Lys Lys Ala Ile
   145                 150                 155                 160
   Glu Ser Thr Thr Gln Ala Val Ala Asp Met Lys Lys His Asn Ser Gly
                   165                 170                 175
   Ile Trp Ile Phe Pro Glu Gly Thr Arg Ser Arg Leu Asp Lys Ala Asp
               180                 185                 190
   Leu Leu Pro Phe Lys Lys Gly Ala Phe His Leu Ala Ile Gln Ala Gln
           195                 200                 205
   Leu Pro Ile Leu Pro Ile Ile Ser Gln Gly Tyr Ser His Ile Tyr Asp
       210                 215                 220
   Ser Ser Lys Arg Tyr Phe Pro Gly Gly Glu Leu Glu Ile Arg Val Leu
   225                 230                 235                 240
   Glu Pro Ile Pro Thr Thr Gly Leu Thr Thr Asp Asp Val Asn Asp Leu
                   245                 250                 255
   Met Asp Lys Thr Arg Asn Leu Met Leu Lys His Leu Lys Glu Met Asp
               260                 265                 270
   Ser Gln Tyr Ser Ser Ser Thr Ala Glu Asn Gly Ser Thr His Ile Asp
           275                 280                 285
   Ala Asp Ile Ala Lys Ser Thr Ala Thr Ser Ile Gly Asn Thr Asp Asp
       290                 295                 300
   Ala Ile Thr Lys Arg Arg Thr Pro Lys Glu
   305                 310
<210> 30
   <211> 1549
   <212> DNA
   <213> Yarrowia lipolytica
<220>
   <221> CDS
   <222> (501)..(1349)
   <223> LPAAT1
<300>
   <302> High eicosapentaenoic acid producing strains of Yarrowia lipolytica
<310> US-2006-0115881-A1
   <311> 2005-11-02
   <312> 2006-06-01
   <313> (1)..(1549)
<300>
   <302> High eicosapentaenoic acid producing strains of Yarrowia lipolytica
<310> WO 2006/052870
   <311> 2005-11-03
   <312> 2006-05-18
   <313> (1)..(1549)
<400> 30
   cacagcataa taccacggca tgaccccgct gactccaacc ttcatttcgg cacatgtagg       60
   tgcacaaggg acttcaagag gggccaattt catgcggaca catggcgcaa aaaacgcccg      120
   actttgatta cacagacacg taataacgac gaagccgaga tgagcacacg tggccaagtc      180
   tgccaatggc cccctggacc cccctgacaa agtttcccaa caagcccagc cgtgcatggt      240
   gtgtttttgt gcggagacac acgccaatta ggctcatttg agggtatgca gcgaaaaaaa      300
   attagtgtgg gtagtttgtt tgcaggaatc aagtgggtgg ttgaaaaaca agaaagagcg      360
   acgacaagag agagagaaaa agagagagag actccataaa gcgtgcatca aaattaaggt      420
   gtgtgactat ccgaaaacca aacatgaaca gttggatata tgtcgctgtg attgcagttg      480
   ctgccgttct cattgcccga atg tcc gtt gca tcc aag ctc gtc ttc tac gtc      533
                         Met Ser Val Ala Ser Lys Leu Val Phe Tyr Val
                         1               5                   10
   cgc gcc gcc atc gcc gtg gtc atc ttt gcc gcc tgt gcc acc tac ggc        581
   Arg Ala Ala Ile Ala Val Val Ile Phe Ala Ala Cys Ala Thr Tyr Gly
               15                  20                  25
   gtg ctg gcg tcc acc att ctc acc gcc atc ggc aag cag ggc ctg gcc        629
   Val Leu Ala Ser Thr Ile Leu Thr Ala Ile Gly Lys Gln Gly Leu Ala
           30                  35                  40
   caa tgg acc gtt gcc aga gcc ttc tac tac tcg gtg cgc atc ttc ctg        677
   Gln Trp Thr Val Ala Arg Ala Phe Tyr Tyr Ser Val Arg Ile Phe Leu
       45                  50                  55
   ggt atc agc atc aag ctg cgt agc cgg cag gtg acc gga acc gcc ggt        725
   Gly Ile Ser Ile Lys Leu Arg Ser Arg Gln Val Thr Gly Thr Ala Gly
   60                  65                  70                  75
   ctg gat gcc tcc aag atc cag gtc gcc aac acc acc aag ccc att gac        773
   Leu Asp Ala Ser Lys Ile Gln Val Ala Asn Thr Thr Lys Pro Ile Asp
                   80                  85                  90
   gac atc acc aaa cac ctg ccc cga cca tgc att ctg att tcc aac cac        821
   Asp Ile Thr Lys His Leu Pro Arg Pro Cys Ile Leu Ile Ser Asn His
               95                  100                 105
   cag aac gaa atg gac att ctg gtg ctc ggt cgc atc ttc ccc cag tac        869
   Gln Asn Glu Met Asp Ile Leu Val Leu Gly Arg Ile Phe Pro Gln Tyr
           110                 115                 120
   tgc tcc gtc acc gcc aaa aag gcc ctc aag tgg tac cct ctg ctg ggc        917
   Cys Ser Val Thr Ala Lys Lys Ala Leu Lys Trp Tyr Pro Leu Leu Gly
       125                 130                 135
   cag ttc atg gcg ctg tcc ggc acc atc ttc ctg gac cga aag gac cga        965
   Gln Phe Met Ala Leu Ser Gly Thr Ile Phe Leu Asp Arg Lys Asp Arg
   140                 145                 150                 155
   acc aag tcc gtg cag acc ctc ggc ggc gcc gtc aag acc atc cag agc       1013
   Thr Lys Ser Val Gln Thr Leu Gly Gly Ala Val Lys Thr Ile Gln Ser
                   160                 165                 170
   ggc aac gga ggc aag ggc cag agc gtc ttc atg ttc ccc gag gga acc       1061
   Gly Asn Gly Gly Lys Gly Gln Ser Val Phe Met Phe Pro Glu Gly Thr
               175                 180                 185
   cga tcc tac tcc aag gac gtc ggc atc atg ccc ttc aag aag ggc tgt       1109
   Arg Ser Tyr Ser Lys Asp Val Gly Ile Met Pro Phe Lys Lys Gly Cys
           190                 195                 200
   ttc cac ctg gcg gtc cag tcg ggc gct ccc att gtc ccc gtg gtg gtc       1157
   Phe His Leu Ala Val Gln Ser Gly Ala Pro Ile Val Pro Val Val Val
       205                 210                 215
   cag aac acc tcc cga atg ttt tct ttc ggc cga ggc aag ctg gac gcc       1205
   Gln Asn Thr Ser Arg Met Phe Ser Phe Gly Arg Gly Lys Leu Asp Ala
   220                 225                 230                 235
   gga gag atc ctt gtc gac gtc ctg agc ccc att gag acc aag ggt ctg       1253
   Gly Glu Ile Leu Val Asp Val Leu Ser Pro Ile Glu Thr Lys Gly Leu
                   240                 245                 250
   gac gcc agc aac gtc gac gct ctc atg gcc acc act tat aag gcc atg       1301
   Asp Ala Ser Asn Val Asp Ala Leu Met Ala Thr Thr Tyr Lys Ala Met
               255                 260                 265
   tgc gag act gcc gac cag att ggc tac gct ggc cag aag act cag tag       1349
   Cys Glu Thr Ala Asp Gln Ile Gly Tyr Ala Gly Gln Lys Thr Gln
           270                 275                 280
   agactgcagc acaagaagtg cttgtagcta ctttaggaga gagataggta atatgaaaca    1409
   tttttcagat cgacacccac ggcgaaccat tggctgtgga gctatgggtg aatggattaa    1469
   tatagcaacg aaatctacct cgattaccaa cgcaaaacga gcccactttc tctgtactgt    1529
   gctatatcgt gtatacccca 1549
<210> 31
   <211> 282
   <212> PRT
   <213> Yarrowia lipolytica
<400> 31
   Met Ser Val Ala Ser Lys Leu Val Phe Tyr Val Arg Ala Ala Ile Ala
   1               5                   10                  15
   Val Val Ile Phe Ala Ala Cys Ala Thr Tyr Gly Val Leu Ala Ser Thr
               20                  25                  30
   Ile Leu Thr Ala Ile Gly Lys Gln Gly Leu Ala Gln Trp Thr Val Ala
           35                  40                  45
   Arg Ala Phe Tyr Tyr Ser Val Arg Ile Phe Leu Gly Ile Ser Ile Lys
       50                  55                  60
   Leu Arg Ser Arg Gln Val Thr Gly Thr Ala Gly Leu Asp Ala Ser Lys
   65                  70                  75                  80
   Ile Gln Val Ala Asn Thr Thr Lys Pro Ile Asp Asp Ile Thr Lys His
                   85                  90                  95
   Leu Pro Arg Pro Cys Ile Leu Ile Ser Asn His Gln Asn Glu Met Asp
               100                 105                 110
   Ile Leu Val Leu Gly Arg Ile Phe Pro Gln Tyr Cys Ser Val Thr Ala
           115                 120                 125
   Lys Lys Ala Leu Lys Trp Tyr Pro Leu Leu Gly Gln Phe Met Ala Leu
       130                 135                 140
   Ser Gly Thr Ile Phe Leu Asp Arg Lys Asp Arg Thr Lys Ser Val Gln
   145                 150                 155                 160
   Thr Leu Gly Gly Ala Val Lys Thr Ile Gln Ser Gly Asn Gly Gly Lys
                   165                 170                 175
   Gly Gln Ser Val Phe Met Phe Pro Glu Gly Thr Arg Ser Tyr Ser Lys
               180                 185                 190
   Asp Val Gly Ile Met Pro Phe Lys Lys Gly Cys Phe His Leu Ala Val
           195                 200                 205
   Gln Ser Gly Ala Pro Ile Val Pro Val Val Val Gln Asn Thr Ser Arg
       210                 215                 220
   Met Phe Ser Phe Gly Arg Gly Lys Leu Asp Ala Gly Glu Ile Leu Val
   225 230 235 240
   Asp Val Leu Ser Pro Ile Glu Thr Lys Gly Leu Asp Ala Ser Asn Val
   245 250 255
   Asp Ala Leu Met Ala Thr Thr Tyr Lys Ala Met Cys Glu Thr Ala Asp
   260 265 270
   Gln Ile Gly Tyr Ala Gly Gln Lys Thr Gln
   275 280
<210> 32
   <211> 303
   <212> PRT
   <213> Saccharomyces cerevisiae
<220>
   <221> MISC_FEATURE
   <222> (1)..(303)
   <223> Slc1p; GenBank Accession No. NP_010231
<400> 32
   Met Ser Val Ile Gly Arg Phe Leu Tyr Tyr Leu Arg Ser Val Leu Val
   1               5                   10                  15
   Val Leu Ala Leu Ala Gly Cys Gly Phe Tyr Gly Val Ile Ala Ser Ile
               20                  25                  30
   Leu Cys Thr Leu Ile Gly Lys Gln His Leu Ala Gln Trp Ile Thr Ala
           35                  40                  45
   Arg Cys Phe Tyr His Val Met Lys Leu Met Leu Gly Leu Asp Val Lys
       50                  55                  60
   Val Val Gly Glu Glu Asn Leu Ala Lys Lys Pro Tyr Ile Met Ile Ala
   65                  70                  75                  80
   Asn His Gln Ser Thr Leu Asp Ile Phe Met Leu Gly Arg Ile Phe Pro
                   85                  90                  95
   Pro Gly Cys Thr Val Thr Ala Lys Lys Ser Leu Lys Tyr Val Pro Phe
               100                 105                 110
   Leu Gly Trp Phe Met Ala Leu Ser Gly Thr Tyr Phe Leu Asp Arg Ser
           115                 120                 125
   Lys Arg Gln Glu Ala Ile Asp Thr Leu Asn Lys Gly Leu Glu Asn Val
       130                 135                 140
   Lys Lys Asn Lys Arg Ala Leu Trp Val Phe Pro Glu Gly Thr Arg Ser
   145 150 155 160
   Tyr Thr Ser Glu Leu Thr Met Leu Pro Phe Lys Lys Gly Ala Phe His
   165 170 175
   Leu Ala Gln Gln Gly Lys Ile Pro Ile Val Pro Val Val Val Ser Asn
   180 185 190
   Thr Ser Thr Leu Val Ser Pro Lys Tyr Gly Val Phe Asn Arg Gly Cys
   195 200 205
   Met Ile Val Arg Ile Leu Lys Pro Ile Ser Thr Glu Asn Leu Thr Lys
   210 215 220
   Asp Lys Ile Gly Glu Phe Ala Glu Lys Val Arg Asp Gln Met Val Asp
   225 230 235 240
   Thr Leu Lys Glu Ile Gly Tyr Ser Pro Ala Ile Asn Asp Thr Thr Leu
   245 250 255
   Pro Pro Gln Ala Ile Glu Tyr Ala Ala Leu Gln His Asp Lys Lys Val
   260 265 270
   Asn Lys Lys Ile Lys Asn Glu Pro Val Pro Ser Val Ser Ile Ser Asn
   275 280 285
   Asp Val Asn Thr His Asn Glu Gly Ser Ser Val Lys Lys Met His
   290 295 300
<210> 33
   <211> 7
   <212> PRT
   <213> Artificial Sequence
<220>
   <223> 1-acyl-sn-glycerol-3-phosphate acyltransferase motif
<220>
   <221> misc_feature
   <222> (3)..(6)
   <223> Xaa can be any naturally occurring amino acid
<400> 33
   Asn His Xaa Xaa Xaa Xaa Asp
   1 5
<210> 34
   <211> 4
   <212> PRT
   <213> Artificial Sequence
<220>
   <223> 1-acyl-sn-glycerol-3-phosphate acyltransferase motif
<400> 34
   Glu Gly Thr Arg
   1
<210> 35
   <211> 955
   <212> DNA
   <213> Mortierella alpina
<220>
   <221> CDS
   <222> (3)..(947)
   <223> synthetic LPAAT1 (codon-optimized for Yarrowia lipolytica)
<400> 35
   ac atg tct att ggt tcg tcc aac ccc gtg ctc ttg gct gcg att ccc        47
      Met Ser Ile Gly Ser Ser Asn Pro Val Leu Leu Ala Ala Ile Pro
      1               5                   10                  15
   ttc gtc tac ctg ttt gtc ctc cca cga gtc ctg gct ttc ctg cct cag        95
   Phe Val Tyr Leu Phe Val Leu Pro Arg Val Leu Ala Phe Leu Pro Gln
                   20                  25                  30
   aag gct cag ttc ctg gcc aaa tgt att gtg gtc ctg att gcc acg ctt       143
   Lys Ala Gln Phe Leu Ala Lys Cys Ile Val Val Leu Ile Ala Thr Leu
               35                  40                  45
   atc atg tcc gtt gca ggc tgc ttc atc tcg atc gtg tgc gct ctt ctg       191
   Ile Met Ser Val Ala Gly Cys Phe Ile Ser Ile Val Cys Ala Leu Leu
           50                  55                  60
   gac aag aga tac gtc atc aat tac gtt gtg tcg cga ttg ttc tcc ttc       239
   Asp Lys Arg Tyr Val Ile Asn Tyr Val Val Ser Arg Leu Phe Ser Phe
       65                  70                  75
   ctt gcc gct cga ccg tgt ggt gtg acc tat aag att gtt ggt gag gaa       287
   Leu Ala Ala Arg Pro Cys Gly Val Thr Tyr Lys Ile Val Gly Glu Glu
   80                  85                  90                  95
   cac ctc gat aag tac cct gct atc gtg gtc tgt aac cat caa tcc tct       335
   His Leu Asp Lys Tyr Pro Ala Ile Val Val Cys Asn His Gln Ser Ser
                   100                 105                 110
   atg gat atg atg gtt ttg gga cga gtt ttt cca aag cac tgc gtt gtc       383
   Met Asp Met Met Val Leu Gly Arg Val Phe Pro Lys His Cys Val Val
               115                 120                 125
   atg gcg aag aag gaa ctc ctg tac ttt ccc ttt ttg gga atg ttt atg       431
   Met Ala Lys Lys Glu Leu Leu Tyr Phe Pro Phe Leu Gly Met Phe Met
           130                 135                 140
   aaa ctg agc aac gct atc ttc atc gac cgg aag aac cac aag aaa gcc       479
   Lys Leu Ser Asn Ala Ile Phe Ile Asp Arg Lys Asn His Lys Lys Ala
       145                 150                 155
   atc gag tct acc acc caa gcc gtg gcg gac atg aag aag cac aac tct       527
   Ile Glu Ser Thr Thr Gln Ala Val Ala Asp Met Lys Lys His Asn Ser
   160                 165                 170                 175
   gga atc tgg att ttc cca gag ggc acc cgg tct aga ctg gac aag gca         575
   Gly Ile Trp Ile Phe Pro Glu Gly Thr Arg Ser Arg Leu Asp Lys Ala
                   180                 185                 190
   gac ctg ctg ccc ttc aag aaa ggt gcc ttt cat ctt gca att cag gcc         623
   Asp Leu Leu Pro Phe Lys Lys Gly Ala Phe His Leu Ala Ile Gln Ala
               195                 200                 205
   cag ctc cct att ctc ccc att atc tcg cag ggc tat tcc cat atc tac         671
   Gln Leu Pro Ile Leu Pro Ile Ile Ser Gln Gly Tyr Ser His Ile Tyr
           210                 215                 220
   gac tct tcg aag cgg tac ttc ccc ggt gga gag ctc gag atc aga gtc         719
   Asp Ser Ser Lys Arg Tyr Phe Pro Gly Gly Glu Leu Glu Ile Arg Val
       225                 230                 235
   ctg gag ccc att cct aca act ggc ctc act act gat gat gtg aac gac         767
   Leu Glu Pro Ile Pro Thr Thr Gly Leu Thr Thr Asp Asp Val Asn Asp
   240                 245                 250                 255
   ctg atg gac aag aca cga aac ctt atg ctc aag cac ttg aag gag atg         815
   Leu Met Asp Lys Thr Arg Asn Leu Met Leu Lys His Leu Lys Glu Met
                   260                 265                 270
   gat tcc cag tat tcg tcg agc act gct gaa aat gga tcc acg cac atc         863
   Asp Ser Gln Tyr Ser Ser Ser Thr Ala Glu Asn Gly Ser Thr His Ile
               275                 280                 285
   gac gcc gat att gcc aag tct aca gcc acc agc att ggc aac act gac         911
   Asp Ala Asp Ile Ala Lys Ser Thr Ala Thr Ser Ile Gly Asn Thr Asp
           290                 295                 300
   gac gca att aca aaa cgt cgt acc cct aag gaa taa gcggccgc                955
   Asp Ala Ile Thr Lys Arg Arg Thr Pro Lys Glu
       305                 310
<210> 36
   <211> 314
   <212> PRT
   <213> Mortierella alpina
<400> 36
   Met Ser Ile Gly Ser Ser Asn Pro Val Leu Leu Ala Ala Ile Pro Phe
   1               5                   10                  15
   Val Tyr Leu Phe Val Leu Pro Arg Val Leu Ala Phe Leu Pro Gln Lys
               20                  25                  30
   Ala Gln Phe Leu Ala Lys Cys Ile Val Val Leu Ile Ala Thr Leu Ile
           35                  40                  45
   Met Ser Val Ala Gly Cys Phe Ile Ser Ile Val Cys Ala Leu Leu Asp
       50                  55                  60
   Lys Arg Tyr Val Ile Asn Tyr Val Val Ser Arg Leu Phe Ser Phe Leu
   65                  70                  75                  80
   Ala Ala Arg Pro Cys Gly Val Thr Tyr Lys Ile Val Gly Glu Glu His
                   85                  90                  95
   Leu Asp Lys Tyr Pro Ala Ile Val Val Cys Asn His Gln Ser Ser Met
               100                 105                 110
   Asp Met Met Val Leu Gly Arg Val Phe Pro Lys His Cys Val Val Met
           115                 120                 125
   Ala Lys Lys Glu Leu Leu Tyr Phe Pro Phe Leu Gly Met Phe Met Lys
       130                 135                 140
   Leu Ser Asn Ala Ile Phe Ile Asp Arg Lys Asn His Lys Lys Ala Ile
   145                 150                 155                 160
   Glu Ser Thr Thr Gln Ala Val Ala Asp Met Lys Lys His Asn Ser Gly
                   165                 170                 175
   Ile Trp Ile Phe Pro Glu Gly Thr Arg Ser Arg Leu Asp Lys Ala Asp
               180                 185                 190
   Leu Leu Pro Phe Lys Lys Gly Ala Phe His Leu Ala Ile Gln Ala Gln
           195                 200                 205
   Leu Pro Ile Leu Pro Ile Ile Ser Gln Gly Tyr Ser His Ile Tyr Asp
       210                 215                 220
   Ser Ser Lys Arg Tyr Phe Pro Gly Gly Glu Leu Glu Ile Arg Val Leu
   225                 230                 235                 240
   Glu Pro Ile Pro Thr Thr Gly Leu Thr Thr Asp Asp Val Asn Asp Leu
                   245                 250                 255
   Met Asp Lys Thr Arg Asn Leu Met Leu Lys His Leu Lys Glu Met Asp
               260                 265                 270
   Ser Gln Tyr Ser Ser Ser Thr Ala Glu Asn Gly Ser Thr His Ile Asp
           275                 280                 285
   Ala Asp Ile Ala Lys Ser Thr Ala Thr Ser Ile Gly Asn Thr Asp Asp
       290                 295                 300
   Ala Ile Thr Lys Arg Arg Thr Pro Lys Glu
   305                 310
<210> 37
   <211> 1185
   <212> DNA
   <213> Yarrowia lipolytica
<220>
   <221> CDS
   <222> (1)..(1185)
   <223> diacylglycerol cholinephosphotransferase (Y1CPT1)
<300>
   <302> HIGH EICOSAPENTAENOIC ACID PRODUCING STRAINS OF
<310> WO 2006/052870
   <311> 2005-11-03
   <312> 2006-05-18
   <313> (1)..(1185)
<300>
   <302> HIGH EICOSAPENTAENOIC ACID PRODUCING STRAINS OF
<310> US 2006-0115881-A1
   <311> 2005-11-02
   <312> 2006-06-01
   <313> (1)..(1185)
<400> 37
   atg ggc gta ttc att aaa cag gag cag ctt ccg gct ctc aag aag tac          48
   Met Gly Val Phe Ile Lys Gln Glu Gln Leu Pro Ala Leu Lys Lys Tyr
   1               5                   10                  15
   aag tac tcc gcc gag gat cac tcg ttc atc tcc aac aac att ctg cgc          96
   Lys Tyr Ser Ala Glu Asp His Ser Phe Ile Ser Asn Asn Ile Leu Arg
               20                  25                  30
   ccc ttc tgg cga cag ttt gtc aaa atc ttc cct ctg tgg atg gcc ccc         144
   Pro Phe Trp Arg Gln Phe Val Lys Ile Phe Pro Leu Trp Met Ala Pro
           35                  40                  45
   aac atg gtg act ctg ttg ggc ttc ttc ttt gtc att gtg aac ttc atc         192
   Asn Met Val Thr Leu Leu Gly Phe Phe Phe Val Ile Val Asn Phe Ile
       50                  55                  60
   acc atg ctc att gtt gat ccc acc cac gac cgc gag cct ccc aga tgg         240
   Thr Met Leu Ile Val Asp Pro Thr His Asp Arg Glu Pro Pro Arg Trp
   65                  70                  75                  80
   gtc tac ctc acc tac gct ctg ggt ctg ttc ctt tac cag aca ttt gat         288
   Val Tyr Leu Thr Tyr Ala Leu Gly Leu Phe Leu Tyr Gln Thr Phe Asp
                   85                  90                  95
   gcc tgt gac gga tcc cat gcc cga cga act ggc cag agt gga ccc ctt         336
   Ala Cys Asp Gly Ser His Ala Arg Arg Thr Gly Gln Ser Gly Pro Leu
               100                 105                 110
   gga gag ctg ttt gac cac tgt gtc gac gcc atg aat acc tct ctg att         384
   Gly Glu Leu Phe Asp His Cys Val Asp Ala Met Asn Thr Ser Leu Ile
           115                 120                 125
   ctc acg gtg gtg gtg tcc acc acc cat atg gga tat aac atg aag ctg         432
   Leu Thr Val Val Val Ser Thr Thr His Met Gly Tyr Asn Met Lys Leu
       130                 135                 140
   ctg att gtg cag att gcc gct ctc gga aac ttc tac ctg tcg acc tgg         480
   Leu Ile Val Gln Ile Ala Ala Leu Gly Asn Phe Tyr Leu Ser Thr Trp
   145                 150                 155                 160
   gag acc tac cat acc gga act ctg tac ctt tct ggc ttc tct ggt cct         528
   Glu Thr Tyr His Thr Gly Thr Leu Tyr Leu Ser Gly Phe Ser Gly Pro
                   165                 170                 175
   gtt gaa ggt atc ttg att ctg gtg gct ctt ttc gtc ctc acc ttc ttc        576
   Val Glu Gly Ile Leu Ile Leu Val Ala Leu Phe Val Leu Thr Phe Phe
               180                 185                 190
   act ggt ccc aac gtg tac gct ctg acc gtc tac gag gct ctt ccc gaa        624
   Thr Gly Pro Asn Val Tyr Ala Leu Thr Val Tyr Glu Ala Leu Pro Glu
           195                 200                 205
   tcc atc act tcg ctg ctg cct gcc agc ttc ctg gac gtc acc atc acc        672
   Ser Ile Thr Ser Leu Leu Pro Ala Ser Phe Leu Asp Val Thr Ile Thr
       210                 215                 220
   cag atc tac att gga ttc gga gtg ctg ggc atg gtg ttc aac atc tac        720
   Gln Ile Tyr Ile Gly Phe Gly Val Leu Gly Met Val Phe Asn Ile Tyr
   225                 230                 235                 240
   ggc gcc tgc gga aac gtg atc aag tac tac aac aac aag ggc aag agc        768
   Gly Ala Cys Gly Asn Val Ile Lys Tyr Tyr Asn Asn Lys Gly Lys Ser
                   245                 250                 255
   gct ctc ccc gcc att ctc gga atc gcc ccc ttt ggc atc ttc tac gtc        816
   Ala Leu Pro Ala Ile Leu Gly Ile Ala Pro Phe Gly Ile Phe Tyr Val
               260                 265                 270
   ggc gtc ttt gcc tgg gcc cat gtt gct cct ctg ctt ctc tcc aag tac        864
   Gly Val Phe Ala Trp Ala His Val Ala Pro Leu Leu Leu Ser Lys Tyr
           275                 280                 285
   gcc atc gtc tat ctg ttt gcc att ggg gct gcc ttt gcc atg caa gtc        912
   Ala Ile Val Tyr Leu Phe Ala Ile Gly Ala Ala Phe Ala Met Gln Val
       290                 295                 300
   ggc cag atg att ctt gcc cat ctc gtg ctt gct ccc ttc ccc cac tgg        960
   Gly Gln Met Ile Leu Ala His Leu Val Leu Ala Pro Phe Pro His Trp
   305                 310                 315                 320
   aac gtg ctg ctc ttc ttc ccc ttt gtg gga ctg gca gtg cac tac att       1008
   Asn Val Leu Leu Phe Phe Pro Phe Val Gly Leu Ala Val His Tyr Ile
                   325                 330                 335
   gca ccc gtg ttt ggc tgg gac gcc gat atc gtg tcg gtt aac act ctc       1056
   Ala Pro Val Phe Gly Trp Asp Ala Asp Ile Val Ser Val Asn Thr Leu
               340                 345                 350
   ttc acc tgt ttt ggc gcc acc ctc tcc att tac gcc ttc ttt gtg ctt       1104
   Phe Thr Cys Phe Gly Ala Thr Leu Ser Ile Tyr Ala Phe Phe Val Leu
           355                 360                 365
   gag atc atc gac gag atc acc aac tac ctc gat atc tgg tgt ctg cga       1152
   Glu Ile Ile Asp Glu Ile Thr Asn Tyr Leu Asp Ile Trp Cys Leu Arg
       370                 375                 380
   atc aag tac cct cag gag aag aag act gag taa                           1185
   Ile Lys Tyr Pro Gln Glu Lys Lys Thr Glu
   385                 390
<210> 38
   <211> 394
   <212> PRT
   <213> Yarrowia lipolytica
<400> 38
   Met Gly Val Phe Ile Lys Gln Glu Gln Leu Pro Ala Leu Lys Lys Tyr
   1               5                   10                  15
   Lys Tyr Ser Ala Glu Asp His Ser Phe Ile Ser Asn Asn Ile Leu Arg
               20                  25                  30
   Pro Phe Trp Arg Gln Phe Val Lys Ile Phe Pro Leu Trp Met Ala Pro
           35                  40                  45
   Asn Met Val Thr Leu Leu Gly Phe Phe Phe Val Ile Val Asn Phe Ile
       50                  55                  60
   Thr Met Leu Ile Val Asp Pro Thr His Asp Arg Glu Pro Pro Arg Trp
   65                  70                  75                  80
   Val Tyr Leu Thr Tyr Ala Leu Gly Leu Phe Leu Tyr Gln Thr Phe Asp
                   85                  90                  95
   Ala Cys Asp Gly Ser His Ala Arg Arg Thr Gly Gln Ser Gly Pro Leu
               100                 105                 110
   Gly Glu Leu Phe Asp His Cys Val Asp Ala Met Asn Thr Ser Leu Ile
           115                 120                 125
   Leu Thr Val Val Val Ser Thr Thr His Met Gly Tyr Asn Met Lys Leu
       130                 135                 140
   Leu Ile Val Gln Ile Ala Ala Leu Gly Asn Phe Tyr Leu Ser Thr Trp
   145                 150                 155                 160
   Glu Thr Tyr His Thr Gly Thr Leu Tyr Leu Ser Gly Phe Ser Gly Pro
                   165                 170                 175
   Val Glu Gly Ile Leu Ile Leu Val Ala Leu Phe Val Leu Thr Phe Phe
               180                 185                 190
   Thr Gly Pro Asn Val Tyr Ala Leu Thr Val Tyr Glu Ala Leu Pro Glu
           195                 200                 205
   Ser Ile Thr Ser Leu Leu Pro Ala Ser Phe Leu Asp Val Thr Ile Thr
       210                 215                 220
   Gln Ile Tyr Ile Gly Phe Gly Val Leu Gly Met Val Phe Asn Ile Tyr
   225                 230                 235                 240
   Gly Ala Cys Gly Asn Val Ile Lys Tyr Tyr Asn Asn Lys Gly Lys Ser
                   245                 250                 255
   Ala Leu Pro Ala Ile Leu Gly Ile Ala Pro Phe Gly Ile Phe Tyr Val
   260 265 270
   Gly Val Phe Ala Trp Ala His Val Ala Pro Leu Leu Leu Ser Lys Tyr
   275 280 285
   Ala Ile Val Tyr Leu Phe Ala Ile Gly Ala Ala Phe Ala Met Gln Val
   290 295 300
   Gly Gln Met Ile Leu Ala His Leu Val Leu Ala Pro Phe Pro His Trp
   305 310 315 320
   Asn Val Leu Leu Phe Phe Pro Phe Val Gly Leu Ala Val His Tyr Ile
   325 330 335
   Ala Pro Val Phe Gly Trp Asp Ala Asp Ile Val Ser Val Asn Thr Leu
   340 345 350
   Phe Thr Cys Phe Gly Ala Thr Leu Ser Ile Tyr Ala Phe Phe Val Leu
   355 360 365
   Glu Ile Ile Asp Glu Ile Thr Asn Tyr Leu Asp Ile Trp Cys Leu Arg
   370 375 380
   Ile Lys Tyr Pro Gln Glu Lys Lys Thr Glu
   385 390
<210> 39
   <211> 1515
   <212> DNA
   <213> Rhizobium leguminosarum bv. viciae 3841
<220>
   <221> CDS
   <222> (1)..(1515)
   <223> GenBank Accession No. YP_766603
<400> 39
   gtg agc aac cat ctt ttc gac gcc atg cgg gct gcc gcg ccc ggt gac          48
   Val Ser Asn His Leu Phe Asp Ala Met Arg Ala Ala Ala Pro Gly Asp
   1               5                   10                  15
   gca ccg ttc atc cgg atc gat aac gcg cgc aca tgg acc tat gat gac          96
   Ala Pro Phe Ile Arg Ile Asp Asn Ala Arg Thr Trp Thr Tyr Asp Asp
               20                  25                  30
   gcg atc gct ctt tcc ggc cgt att gcc ggc gcg atg gac gcg ctc ggc         144
   Ala Ile Ala Leu Ser Gly Arg Ile Ala Gly Ala Met Asp Ala Leu Gly
           35                  40                  45
   att cgc ccc ggc gac cgg gtg gcg gtg cag gtc gag aaa agt gcc gag         192
   Ile Arg Pro Gly Asp Arg Val Ala Val Gln Val Glu Lys Ser Ala Glu
       50                  55                  60
   gcg ttg atc ctc tat ctc gcc tgt ctt cga acc ggc gcc gtc tac ctg         240
   Ala Leu Ile Leu Tyr Leu Ala Cys Leu Arg Thr Gly Ala Val Tyr Leu
   65                  70                  75                  80
   ccg ctc aac acc gcc tat acg ctg gct gag ctc gat tac ttt atc ggc      288
   Pro Leu Asn Thr Ala Tyr Thr Leu Ala Glu Leu Asp Tyr Phe Ile Gly
                   85                  90                  95
   gat gcg gag ccg cgt ttg gtg gtg gtt gcg ccg gcg gct cga ggc ggc      336
   Asp Ala Glu Pro Arg Leu Val Val Val Ala Pro Ala Ala Arg Gly Gly
               100                 105                 110
   gtg gag aca atc gcc aag cgc cac ggc gcg atc gtc gaa acg ctc gat      384
   Val Glu Thr Ile Ala Lys Arg His Gly Ala Ile Val Glu Thr Leu Asp
           115                 120                 125
   gct gat ggc cgc ggc tca ttg ctg gat ctc gca cgc gat gag ccg gcc      432
   Ala Asp Gly Arg Gly Ser Leu Leu Asp Leu Ala Arg Asp Glu Pro Ala
       130                 135                 140
   gac ttt gtc gat gcc tcg cgt tcc gcg gat gat ctg gcc gcg atc ctc      480
   Asp Phe Val Asp Ala Ser Arg Ser Ala Asp Asp Leu Ala Ala Ile Leu
   145                 150                 155                 160
   tac acg tcg gga acg acc gga cgc tcc aag ggg gcg atg ctc acg cat      528
   Tyr Thr Ser Gly Thr Thr Gly Arg Ser Lys Gly Ala Met Leu Thr His
                   165                 170                 175
   ggg aac ctg ctc tcg aac gcc ctg acc ttg cga gac tat tgg cgc gtc      576
   Gly Asn Leu Leu Ser Asn Ala Leu Thr Leu Arg Asp Tyr Trp Arg Val
               180                 185                 190
   acc gct gac gat cgg ctg atc cat gcc ttg ccg atc ttc cac acg cat      624
   Thr Ala Asp Asp Arg Leu Ile His Ala Leu Pro Ile Phe His Thr His
           195                 200                 205
   ggg ctg ttc gtc gcc acg aac gtc aca ctg ctt gcc ggc gcc tcg atg      672
   Gly Leu Phe Val Ala Thr Asn Val Thr Leu Leu Ala Gly Ala Ser Met
       210                 215                 220
   ttc ctg ctg tcg aaa ttc gac gcc gac gag gtc gtt tca ctg atg cca      720
   Phe Leu Leu Ser Lys Phe Asp Ala Asp Glu Val Val Ser Leu Met Pro
   225                 230                 235                 240
   cag gca act atg ctg atg ggc gtg ccg acc ttc tac gtg cgc ctc ctg      768
   Gln Ala Thr Met Leu Met Gly Val Pro Thr Phe Tyr Val Arg Leu Leu
                   245                 250                 255
   caa agc ccg cgc ctc gaa aaa ggg gcg gtc gcc agc atc cgc ctc ttc      816
   Gln Ser Pro Arg Leu Glu Lys Gly Ala Val Ala Ser Ile Arg Leu Phe
               260                 265                 270
   att tcc ggt tcg gct ccc ctg ctg gcc gaa acc cat gcc gag ttc cat      864
   Ile Ser Gly Ser Ala Pro Leu Leu Ala Glu Thr His Ala Glu Phe His
           275                 280                 285
   gcg cgt acc ggt cac gcc att ctc gag cgc tac ggc atg acg gaa acc      912
   Ala Arg Thr Gly His Ala Ile Leu Glu Arg Tyr Gly Met Thr Glu Thr
       290                 295                 300
   aat atg aac acg tcc aac ccc tat gag ggc aaa cgg att gcc gga acg      960
   Asn Met Asn Thr Ser Asn Pro Tyr Glu Gly Lys Arg Ile Ala Gly Thr
   305                 310                 315                 320
   gtt ggt ttc ccg ctg cct gat gtg acg gtg cgc gtc acc gat ccc gcc     1008
   Val Gly Phe Pro Leu Pro Asp Val Thr Val Arg Val Thr Asp Pro Ala
                   325                 330                 335
   acc ggg ctc gtg ctg cca cct gaa gag acg ggc atg atc gag atc aag      1056
   Thr Gly Leu Val Leu Pro Pro Glu Glu Thr Gly Met Ile Glu Ile Lys
               340                 345                 350
   gga ccg aac gtc ttc aag ggc tat tgg cgc atg ccg gaa aag acc gcg      1104
   Gly Pro Asn Val Phe Lys Gly Tyr Trp Arg Met Pro Glu Lys Thr Ala
           355                 360                 365
   gcc gaa ttc acc gcc gac ggt ttc ttc atc agc ggc gat ctc ggc aag      1152
   Ala Glu Phe Thr Ala Asp Gly Phe Phe Ile Ser Gly Asp Leu Gly Lys
       370                 375                 380
   atc gac cgg gaa ggt tat gtc cac atc gtc ggt cgc ggc aag gat ctg      1200
   Ile Asp Arg Glu Gly Tyr Val His Ile Val Gly Arg Gly Lys Asp Leu
   385                 390                 395                 400
   gtg atc tcg ggt gga tac aac atc tat ccg aaa gag gtc gaa ggc gag      1248
   Val Ile Ser Gly Gly Tyr Asn Ile Tyr Pro Lys Glu Val Glu Gly Glu
                   405                 410                 415
   atc gac cag atc gag ggt gtg gtt gag agc gct gtg atc ggt gtg ccg      1296
   Ile Asp Gln Ile Glu Gly Val Val Glu Ser Ala Val Ile Gly Val Pro
               420                 425                 430
   cat ccc gat ttc gga gaa ggc gta acg gct gtc gtc gta tgt aag ccc      1344
   His Pro Asp Phe Gly Glu Gly Val Thr Ala Val Val Val Cys Lys Pro
           435                 440                 445
   ggc gcc gtc ctc gat gaa aag acc atc gtc agc gcc ctc cag gac cgt      1392
   Gly Ala Val Leu Asp Glu Lys Thr Ile Val Ser Ala Leu Gln Asp Arg
       450                 455                 460
   ctc gcc cgc tac aaa caa ccc aag cgc atc atc ttc gcc gac gac ctg      1440
   Leu Ala Arg Tyr Lys Gln Pro Lys Arg Ile Ile Phe Ala Asp Asp Leu
   465                 470                 475                 480
   ccg cgc aac act atg ggt aag gtt cag aag aat atc ctg cgg cag caa      1488
   Pro Arg Asn Thr Met Gly Lys Val Gln Lys Asn Ile Leu Arg Gln Gln
                   485                 490                 495
   tac gcc gat ctt tat acc agg agg taa                                  1515
   Tyr Ala Asp Leu Tyr Thr Arg Arg
               500
<210> 40
   <211> 504
   <212> PRT
   <213> Rhizobium leguminosarum bv. viciae 3841
<400> 40
   Val Ser Asn His Leu Phe Asp Ala Met Arg Ala Ala Ala Pro Gly Asp
   1               5                   10                  15
   Ala Pro Phe Ile Arg Ile Asp Asn Ala Arg Thr Trp Thr Tyr Asp Asp
               20                  25                  30
   Ala Ile Ala Leu Ser Gly Arg Ile Ala Gly Ala Met Asp Ala Leu Gly
           35                  40                  45
   Ile Arg Pro Gly Asp Arg Val Ala Val Gln Val Glu Lys Ser Ala Glu
       50                  55                  60
   Ala Leu Ile Leu Tyr Leu Ala Cys Leu Arg Thr Gly Ala Val Tyr Leu
   65                  70                  75                  80
   Pro Leu Asn Thr Ala Tyr Thr Leu Ala Glu Leu Asp Tyr Phe Ile Gly
                   85                  90                  95
   Asp Ala Glu Pro Arg Leu Val Val Val Ala Pro Ala Ala Arg Gly Gly
               100                 105                 110
   Val Glu Thr Ile Ala Lys Arg His Gly Ala Ile Val Glu Thr Leu Asp
           115                 120                 125
   Ala Asp Gly Arg Gly Ser Leu Leu Asp Leu Ala Arg Asp Glu Pro Ala
       130                 135                 140
   Asp Phe Val Asp Ala Ser Arg Ser Ala Asp Asp Leu Ala Ala Ile Leu
   145                 150                 155                 160
   Tyr Thr Ser Gly Thr Thr Gly Arg Ser Lys Gly Ala Met Leu Thr His
                   165                 170                 175
   Gly Asn Leu Leu Ser Asn Ala Leu Thr Leu Arg Asp Tyr Trp Arg Val
               180                 185                 190
   Thr Ala Asp Asp Arg Leu Ile His Ala Leu Pro Ile Phe His Thr His
           195                 200                 205
   Gly Leu Phe Val Ala Thr Asn Val Thr Leu Leu Ala Gly Ala Ser Met
       210                 215                 220
   Phe Leu Leu Ser Lys Phe Asp Ala Asp Glu Val Val Ser Leu Met Pro
   225                 230                 235                 240
   Gln Ala Thr Met Leu Met Gly Val Pro Thr Phe Tyr Val Arg Leu Leu
                   245                 250                 255
   Gln Ser Pro Arg Leu Glu Lys Gly Ala Val Ala Ser Ile Arg Leu Phe
               260                 265                 270
   Ile Ser Gly Ser Ala Pro Leu Leu Ala Glu Thr His Ala Glu Phe His
           275                 280                 285
   Ala Arg Thr Gly His Ala Ile Leu Glu Arg Tyr Gly Met Thr Glu Thr
       290                 295                 300
   Asn Met Asn Thr Ser Asn Pro Tyr Glu Gly Lys Arg Ile Ala Gly Thr
   305 310 315 320
   Val Gly Phe Pro Leu Pro Asp Val Thr Val Arg Val Thr Asp Pro Ala
   325 330 335
   Thr Gly Leu Val Leu Pro Pro Glu Glu Thr Gly Met Ile Glu Ile Lys
   340 345 350
   Gly Pro Asn Val Phe Lys Gly Tyr Trp Arg Met Pro Glu Lys Thr Ala
   355 360 365
   Ala Glu Phe Thr Ala Asp Gly Phe Phe Ile Ser Gly Asp Leu Gly Lys
   370 375 380
   Ile Asp Arg Glu Gly Tyr Val His Ile Val Gly Arg Gly Lys Asp Leu
   385 390 395 400
   Val Ile Ser Gly Gly Tyr Asn Ile Tyr Pro Lys Glu Val Glu Gly Glu
   405 410 415
   Ile Asp Gln Ile Glu Gly Val Val Glu Ser Ala Val Ile Gly Val Pro
   420 425 430
   His Pro Asp Phe Gly Glu Gly Val Thr Ala Val Val Val Cys Lys Pro
   435 440 445
   Gly Ala Val Leu Asp Glu Lys Thr Ile Val Ser Ala Leu Gln Asp Arg
   450 455 460
   Leu Ala Arg Tyr Lys Gln Pro Lys Arg Ile Ile Phe Ala Asp Asp Leu
   465 470 475 480
   Pro Arg Asn Thr Met Gly Lys Val Gln Lys Asn Ile Leu Arg Gln Gln
   485 490 495
   Tyr Ala Asp Leu Tyr Thr Arg Arg
   500
<210> 41
   <211> 1518
   <212> DNA
   <213> Rhizobium leguminosarum bv. viciae 3841
<220>
   <221> CDS
   <222> (1)..(1518)
   <223> synthetic malonyl-CoA synthetase (codon-optimized for Yarrowia lipolytica)
<400> 41
   atg gtc tcc aac cac ctg ttc gac gcc atg cga gct gcc gct ccc gga 48
   Met Val Ser Asn His Leu Phe Asp Ala Met Arg Ala Ala Ala Pro Gly
   1               5                   10                  15
   gac gca cct ttc att cga atc gac aac gct cgg acc tgg act tac gat        96
   Asp Ala Pro Phe Ile Arg Ile Asp Asn Ala Arg Thr Trp Thr Tyr Asp
               20                  25                  30
   gac gcc att gct ctt tcc ggt cga atc gct gga gct atg gac gca ctc       144
   Asp Ala Ile Ala Leu Ser Gly Arg Ile Ala Gly Ala Met Asp Ala Leu
           35                  40                  45
   ggc att cga ccc gga gac aga gtt gcc gtg cag gtc gag aag tct gcc       192
   Gly Ile Arg Pro Gly Asp Arg Val Ala Val Gln Val Glu Lys Ser Ala
       50                  55                  60
   gag gcg ttg att ctc tac ctg gcc tgt ctt cga acc gga gct gtc tac       240
   Glu Ala Leu Ile Leu Tyr Leu Ala Cys Leu Arg Thr Gly Ala Val Tyr
   65                  70                  75                  80
   ctg cct ctc aac act gcc tac acc ctg gcc gag ctc gac tac ttc atc       288
   Leu Pro Leu Asn Thr Ala Tyr Thr Leu Ala Glu Leu Asp Tyr Phe Ile
                   85                  90                  95
   ggc gat gcc gaa ccg cgt ctg gtg gtc gtt gct ccc gca gct cga ggt       336
   Gly Asp Ala Glu Pro Arg Leu Val Val Val Ala Pro Ala Ala Arg Gly
               100                 105                 110
   ggc gtg gag aca att gcc aag cga cac ggt gct atc gtc gaa acc ctc       384
   Gly Val Glu Thr Ile Ala Lys Arg His Gly Ala Ile Val Glu Thr Leu
           115                 120                 125
   gac gcc gat gga cga ggc tcc ttg ctg gac ctt gct aga gat gag cct       432
   Asp Ala Asp Gly Arg Gly Ser Leu Leu Asp Leu Ala Arg Asp Glu Pro
       130                 135                 140
   gcc gac ttt gtc gat gct tcg cga tct gcc gac gat ctg gct gct att       480
   Ala Asp Phe Val Asp Ala Ser Arg Ser Ala Asp Asp Leu Ala Ala Ile
   145                 150                 155                 160
   ctc tac act tcc ggt aca acc gga cga tcg aag ggt gcc atg ctt act       528
   Leu Tyr Thr Ser Gly Thr Thr Gly Arg Ser Lys Gly Ala Met Leu Thr
                   165                 170                 175
   cat ggc aat ctg ctc tcc aac gct ctc acc ttg cga gac tat tgg aga       576
   His Gly Asn Leu Leu Ser Asn Ala Leu Thr Leu Arg Asp Tyr Trp Arg
               180                 185                 190
   gtt acc gca gac gat cga ctc atc cat gcc ttg cca atc ttt cac act       624
   Val Thr Ala Asp Asp Arg Leu Ile His Ala Leu Pro Ile Phe His Thr
           195                 200                 205
   cat ggt ctg ttc gtt gct acg aac gtc aca ctg ctt gca gga gcc tcg       672
   His Gly Leu Phe Val Ala Thr Asn Val Thr Leu Leu Ala Gly Ala Ser
       210                 215                 220
   atg ttt ctg ctc tcc aag ttc gat gcc gac gag gtc gtt tct ctc atg       720
   Met Phe Leu Leu Ser Lys Phe Asp Ala Asp Glu Val Val Ser Leu Met
   225                 230                 235                 240
   cca cag gcc acc atg ctt atg ggc gtg ccc aca ttc tac gtt cga ttg       768
   Pro Gln Ala Thr Met Leu Met Gly Val Pro Thr Phe Tyr Val Arg Leu
                   245                 250                 255
   ctg cag agt cct cga ctc gag aag ggt gct gtg gcc agc atc aga ctg       816
   Leu Gln Ser Pro Arg Leu Glu Lys Gly Ala Val Ala Ser Ile Arg Leu
               260                 265                 270
   ttc att tct gga tca gct ccc ttg ctt gcc gaa acc cac gcc gag ttt       864
   Phe Ile Ser Gly Ser Ala Pro Leu Leu Ala Glu Thr His Ala Glu Phe
           275                 280                 285
   cat gct cgt act ggt cac gcc att ctc gag cga tac ggc atg acg gaa       912
   His Ala Arg Thr Gly His Ala Ile Leu Glu Arg Tyr Gly Met Thr Glu
       290                 295                 300
   acc aac atg aat act tcc aac ccc tac gag ggc aag cgt att gcc gga       960
   Thr Asn Met Asn Thr Ser Asn Pro Tyr Glu Gly Lys Arg Ile Ala Gly
   305                 310                 315                 320
   acc gtt ggt ttt cct ctg ccc gac gtc act gtg cga gtc acc gat ccc      1008
   Thr Val Gly Phe Pro Leu Pro Asp Val Thr Val Arg Val Thr Asp Pro
                   325                 330                 335
   gcc acc ggt ctc gtt ctt cca cct gaa gag act ggc atg atc gag atc      1056
   Ala Thr Gly Leu Val Leu Pro Pro Glu Glu Thr Gly Met Ile Glu Ile
               340                 345                 350
   aag gga ccc aac gtc ttc aag ggc tat tgg cga atg ccc gaa aag acc      1104
   Lys Gly Pro Asn Val Phe Lys Gly Tyr Trp Arg Met Pro Glu Lys Thr
           355                 360                 365
   gct gcc gag ttt acc gca gac ggt ttc ttt atc tct gga gat ctc ggc      1152
   Ala Ala Glu Phe Thr Ala Asp Gly Phe Phe Ile Ser Gly Asp Leu Gly
       370                 375                 380
   aag atc gac cga gaa ggt tac gtt cac att gtg gga cga ggc aag gac      1200
   Lys Ile Asp Arg Glu Gly Tyr Val His Ile Val Gly Arg Gly Lys Asp
   385                 390                 395                 400
   ctg gtc att tcc ggt ggc tac aac atc tat ccc aaa gag gtc gaa ggc      1248
   Leu Val Ile Ser Gly Gly Tyr Asn Ile Tyr Pro Lys Glu Val Glu Gly
                   405                 410                 415
   gag atc gac cag atc gag ggt gtg gtc gag tct gct gtc att ggt gtt      1296
   Glu Ile Asp Gln Ile Glu Gly Val Val Glu Ser Ala Val Ile Gly Val
               420                 425                 430
   cct cat ccc gat ttc gga gaa ggt gtc acc gct gtt gtc gtg tgc aaa      1344
   Pro His Pro Asp Phe Gly Glu Gly Val Thr Ala Val Val Val Cys Lys
           435                 440                 445
   cct ggt gcc gtt ctc gac gaa aag acc atc gtg tct gct ctg cag gac      1392
   Pro Gly Ala Val Leu Asp Glu Lys Thr Ile Val Ser Ala Leu Gln Asp
       450                 455                 460
   cgt ctt gcc cga tac aag caa ccc aag cgg att atc ttt gcc gac gat      1440
   Arg Leu Ala Arg Tyr Lys Gln Pro Lys Arg Ile Ile Phe Ala Asp Asp
   465                 470                 475                 480
   ctg cct cga aac act atg gga aag gtt cag aag aac att ctt cga cag      1488
   Leu Pro Arg Asn Thr Met Gly Lys Val Gln Lys Asn Ile Leu Arg Gln
                   485                 490                 495
   caa tac gcc gat ctc tac acc aga cga taa                              1518
   Gln Tyr Ala Asp Leu Tyr Thr Arg Arg
               500                 505
<210> 42
   <211> 505
   <212> PRT
   <213> Rhizobium leguminosarum bv. viciae 3841
<400> 42
   Met Val Ser Asn His Leu Phe Asp Ala Met Arg Ala Ala Ala Pro Gly
   1               5                   10                  15
   Asp Ala Pro Phe Ile Arg Ile Asp Asn Ala Arg Thr Trp Thr Tyr Asp
               20                  25                  30
   Asp Ala Ile Ala Leu Ser Gly Arg Ile Ala Gly Ala Met Asp Ala Leu
           35                  40                  45
   Gly Ile Arg Pro Gly Asp Arg Val Ala Val Gln Val Glu Lys Ser Ala
       50                  55                  60
   Glu Ala Leu Ile Leu Tyr Leu Ala Cys Leu Arg Thr Gly Ala Val Tyr
   65                  70                  75                  80
   Leu Pro Leu Asn Thr Ala Tyr Thr Leu Ala Glu Leu Asp Tyr Phe Ile
                   85                  90                  95
   Gly Asp Ala Glu Pro Arg Leu Val Val Val Ala Pro Ala Ala Arg Gly
               100                 105                 110
   Gly Val Glu Thr Ile Ala Lys Arg His Gly Ala Ile Val Glu Thr Leu
           115                 120                 125
   Asp Ala Asp Gly Arg Gly Ser Leu Leu Asp Leu Ala Arg Asp Glu Pro
       130                 135                 140
   Ala Asp Phe Val Asp Ala Ser Arg Ser Ala Asp Asp Leu Ala Ala Ile
   145                 150                 155                 160
   Leu Tyr Thr Ser Gly Thr Thr Gly Arg Ser Lys Gly Ala Met Leu Thr
                   165                 170                 175
   His Gly Asn Leu Leu Ser Asn Ala Leu Thr Leu Arg Asp Tyr Trp Arg
               180                 185                 190
   Val Thr Ala Asp Asp Arg Leu Ile His Ala Leu Pro Ile Phe His Thr
           195                 200                 205
   His Gly Leu Phe Val Ala Thr Asn Val Thr Leu Leu Ala Gly Ala Ser
       210                 215                 220
   Met Phe Leu Leu Ser Lys Phe Asp Ala Asp Glu Val Val Ser Leu Met
   225                 230                 235                 240
   Pro Gln Ala Thr Met Leu Met Gly Val Pro Thr Phe Tyr Val Arg Leu
   245 250 255
   Leu Gln Ser Pro Arg Leu Glu Lys Gly Ala Val Ala Ser Ile Arg Leu
   260 265 270
   Phe Ile Ser Gly Ser Ala Pro Leu Leu Ala Glu Thr His Ala Glu Phe
   275 280 285
   His Ala Arg Thr Gly His Ala Ile Leu Glu Arg Tyr Gly Met Thr Glu
   290 295 300
   Thr Asn Met Asn Thr Ser Asn Pro Tyr Glu Gly Lys Arg Ile Ala Gly
   305 310 315 320
   Thr Val Gly Phe Pro Leu Pro Asp Val Thr Val Arg Val Thr Asp Pro
   325 330 335
   Ala Thr Gly Leu Val Leu Pro Pro Glu Glu Thr Gly Met Ile Glu Ile
   340 345 350
   Lys Gly Pro Asn Val Phe Lys Gly Tyr Trp Arg Met Pro Glu Lys Thr
   355 360 365
   Ala Ala Glu Phe Thr Ala Asp Gly Phe Phe Ile Ser Gly Asp Leu Gly
   370 375 380
   Lys Ile Asp Arg Glu Gly Tyr Val His Ile Val Gly Arg Gly Lys Asp
   385 390 395 400
   Leu Val Ile Ser Gly Gly Tyr Asn Ile Tyr Pro Lys Glu Val Glu Gly
   405 410 415
   Glu Ile Asp Gln Ile Glu Gly Val Val Glu Ser Ala Val Ile Gly Val
   420 425 430
   Pro His Pro Asp Phe Gly Glu Gly Val Thr Ala Val Val Val Cys Lys
   435 440 445
   Pro Gly Ala Val Leu Asp Glu Lys Thr Ile Val Ser Ala Leu Gln Asp
   450 455 460
   Arg Leu Ala Arg Tyr Lys Gln Pro Lys Arg Ile Ile Phe Ala Asp Asp
   465 470 475 480
   Leu Pro Arg Asn Thr Met Gly Lys Val Gln Lys Asn Ile Leu Arg Gln
   485 490 495
   Gln Tyr Ala Asp Leu Tyr Thr Arg Arg
   500 505
<210> 43
   <211> 777
   <212> DNA
   <213> Euglena gracilis
<220>
   <221> CDS
   <222> (1)..(777)
   <223> delta-9 elongase
<300>
   <302> DELTA-9 ELONGASES AND THEIR USE IN MAKING POLYUNSATURATED FATTY ACIDS
<310> WO 2007/061742
   <311> 2006-11-16
   <312> 2007-05-31
   <313> (1)..(777)
<300>
   <302> DELTA-9 ELONGASES AND THEIR USE IN MAKING POLYUNSATURATED FATTY ACIDS
<310> US 2007-0117190-A1
   <311> 2006-11-16
   <312> 2007-05-24
   <313> (1)..(777)
<400> 43
   atg gag gtg gtg aat gaa ata gtc tca att ggg cag gaa gtt tta ccc        48
   Met Glu Val Val Asn Glu Ile Val Ser Ile Gly Gln Glu Val Leu Pro
   1               5                   10                  15
   aaa gtt gat tat gcc caa ctc tgg agt gat gcc agt cac tgt gag gtg        96
   Lys Val Asp Tyr Ala Gln Leu Trp Ser Asp Ala Ser His Cys Glu Val
               20                  25                  30
   ctt tac ttg tcc atc gca ttt gtc atc ttg aag ttc act ctt ggc ccc       144
   Leu Tyr Leu Ser Ile Ala Phe Val Ile Leu Lys Phe Thr Leu Gly Pro
           35                  40                  45
   ctt ggt cca aaa ggt cag tct cgt atg aag ttt gtt ttc acc aat tac       192
   Leu Gly Pro Lys Gly Gln Ser Arg Met Lys Phe Val Phe Thr Asn Tyr
       50                  55                  60
   aac ctt ctc atg tcc att tat tcg ttg gga tca ttc ctc tca atg gca       240
   Asn Leu Leu Met Ser Ile Tyr Ser Leu Gly Ser Phe Leu Ser Met Ala
   65                  70                  75                  80
   tat gcc atg tac acc atc ggt gtt atg tct gac aac tgc gag aag gct       288
   Tyr Ala Met Tyr Thr Ile Gly Val Met Ser Asp Asn Cys Glu Lys Ala
                   85                  90                  95
   ttt gac aac aac gtc ttc agg atc acc acg cag ttg ttc tat ttg agc       336
   Phe Asp Asn Asn Val Phe Arg Ile Thr Thr Gln Leu Phe Tyr Leu Ser
               100                 105                 110
   aag ttc ctg gag tat att gac tcc ttc tat ttg cca ctg atg ggc aag       384
   Lys Phe Leu Glu Tyr Ile Asp Ser Phe Tyr Leu Pro Leu Met Gly Lys
           115                 120                 125
   cct ctg acc tgg ttg caa ttc ttc cat cat ttg ggg gca ccg atg gat       432
   Pro Leu Thr Trp Leu Gln Phe Phe His His Leu Gly Ala Pro Met Asp
       130                 135                 140
   atg tgg ctg ttc tat aat tac cga aat gaa gct gtt tgg att ttt gtg        480
   Met Trp Leu Phe Tyr Asn Tyr Arg Asn Glu Ala Val Trp Ile Phe Val
   145                 150                 155                 160
   ctg ttg aat ggt ttc atc cac tgg atc atg tac ggt tat tat tgg acc        528
   Leu Leu Asn Gly Phe Ile His Trp Ile Met Tyr Gly Tyr Tyr Trp Thr
                   165                 170                 175
   aga ttg atc aag ctg aag ttc ccc atg cca aaa tcc ctg att aca tca        576
   Arg Leu Ile Lys Leu Lys Phe Pro Met Pro Lys Ser Leu Ile Thr Ser
               180                 185                 190
   atg cag atc att caa ttc aat gtt ggt ttc tac att gtc tgg aag tac        624
   Met Gln Ile Ile Gln Phe Asn Val Gly Phe Tyr Ile Val Trp Lys Tyr
           195                 200                 205
   agg aac att ccc tgt tat cgc caa gat ggg atg agg atg ttt ggc tgg        672
   Arg Asn Ile Pro Cys Tyr Arg Gln Asp Gly Met Arg Met Phe Gly Trp
       210                 215                 220
   ttc ttc aat tac ttt tat gtt ggc aca gtc ttg tgt ttg ttc ttg aat        720
   Phe Phe Asn Tyr Phe Tyr Val Gly Thr Val Leu Cys Leu Phe Leu Asn
   225                 230                 235                 240
   ttc tat gtg caa acg tat atc gtc agg aag cac aag gga gcc aaa aag        768
   Phe Tyr Val Gln Thr Tyr Ile Val Arg Lys His Lys Gly Ala Lys Lys
                   245                 250                 255
   att cag tga                                                            777
   Ile Gln
<210> 44
   <211> 258
   <212> PRT
   <213> Euglena gracilis
<400> 44
   Met Glu Val Val Asn Glu Ile Val Ser Ile Gly Gln Glu Val Leu Pro
   1               5                   10                  15
   Lys Val Asp Tyr Ala Gln Leu Trp Ser Asp Ala Ser His Cys Glu Val
               20                  25                  30
   Leu Tyr Leu Ser Ile Ala Phe Val Ile Leu Lys Phe Thr Leu Gly Pro
           35                  40                  45
   Leu Gly Pro Lys Gly Gln Ser Arg Met Lys Phe Val Phe Thr Asn Tyr
       50                  55                  60
   Asn Leu Leu Met Ser Ile Tyr Ser Leu Gly Ser Phe Leu Ser Met Ala
   65                  70                  75                  80
   Tyr Ala Met Tyr Thr Ile Gly Val Met Ser Asp Asn Cys Glu Lys Ala
                   85                  90                  95
   Phe Asp Asn Asn Val Phe Arg Ile Thr Thr Gln Leu Phe Tyr Leu Ser
   100 105 110
   Lys Phe Leu Glu Tyr Ile Asp Ser Phe Tyr Leu Pro Leu Met Gly Lys
   115 120 125
   Pro Leu Thr Trp Leu Gln Phe Phe His His Leu Gly Ala Pro Met Asp
   130 135 140
   Met Trp Leu Phe Tyr Asn Tyr Arg Asn Glu Ala Val Trp Ile Phe Val
   145 150 155 160
   Leu Leu Asn Gly Phe Ile His Trp Ile Met Tyr Gly Tyr Tyr Trp Thr
   165 170 175
   Arg Leu Ile Lys Leu Lys Phe Pro Met Pro Lys Ser Leu Ile Thr Ser
   180 185 190
   Met Gln Ile Ile Gln Phe Asn Val Gly Phe Tyr Ile Val Trp Lys Tyr
   195 200 205
   Arg Asn Ile Pro Cys Tyr Arg Gln Asp Gly Met Arg Met Phe Gly Trp
   210 215 220
   Phe Phe Asn Tyr Phe Tyr Val Gly Thr Val Leu Cys Leu Phe Leu Asn
   225 230 235 240
   Phe Tyr Val Gln Thr Tyr Ile Val Arg Lys His Lys Gly Ala Lys Lys
   245 250 255
   Ile Gln
<210> 45
   <211> 777
   <212> DNA
   <213> Euglena gracilis
<220>
   <221> CDS
   <222> (1)..(777)
   <223> synthetic delta-9 elongase (codon-optimized for Yarrowia lipolytica)
<300>
   <302> DELTA-9 ELONGASES AND THEIR USE IN MAKING POLYUNSATURATED FATTY ACIDS
<310> WO 2007/061742
   <311> 2006-11-16
   <312> 2007-05-31
   <313> (1)..(777)
<300>
   <302> DELTA-9 ELONGASES AND THEIR USE IN MAKING POLYUNSATURATED FATTY ACIDS
<310> US-2007-0117190-A1
   <311> 2006-11-16
   <312> 2007-05-24
   <313> (1)..(777)
<400> 45
   atg gag gtc gtg aac gaa atc gtc tcc att ggc cag gag gtt ctt ccc        48
   Met Glu Val Val Asn Glu Ile Val Ser Ile Gly Gln Glu Val Leu Pro
   1               5                   10                  15
   aag gtc gac tat gct cag ctc tgg tct gat gcc tcg cac tgc gag gtg        96
   Lys Val Asp Tyr Ala Gln Leu Trp Ser Asp Ala Ser His Cys Glu Val
               20                  25                  30
   ctg tac ctc tcc atc gcc ttc gtc atc ctg aag ttc acc ctt ggt cct       144
   Leu Tyr Leu Ser Ile Ala Phe Val Ile Leu Lys Phe Thr Leu Gly Pro
           35                  40                  45
   ctc gga ccc aag ggt cag tct cga atg aag ttt gtg ttc acc aac tac       192
   Leu Gly Pro Lys Gly Gln Ser Arg Met Lys Phe Val Phe Thr Asn Tyr
       50                  55                  60
   aac ctg ctc atg tcc atc tac tcg ctg ggc tcc ttc ctc tct atg gcc       240
   Asn Leu Leu Met Ser Ile Tyr Ser Leu Gly Ser Phe Leu Ser Met Ala
   65                  70                  75                  80
   tac gcc atg tac acc att ggt gtc atg tcc gac aac tgc gag aag gct       288
   Tyr Ala Met Tyr Thr Ile Gly Val Met Ser Asp Asn Cys Glu Lys Ala
                   85                  90                  95
   ttc gac aac aat gtc ttc cga atc acc act cag ctg ttc tac ctc agc       336
   Phe Asp Asn Asn Val Phe Arg Ile Thr Thr Gln Leu Phe Tyr Leu Ser
               100                 105                 110
   aag ttc ctc gag tac att gac tcc ttc tat ctg ccc ctc atg ggc aag       384
   Lys Phe Leu Glu Tyr Ile Asp Ser Phe Tyr Leu Pro Leu Met Gly Lys
           115                 120                 125
   cct ctg acc tgg ttg cag ttc ttt cac cat ctc gga gct cct atg gac       432
   Pro Leu Thr Trp Leu Gln Phe Phe His His Leu Gly Ala Pro Met Asp
       130                 135                 140
   atg tgg ctg ttc tac aac tac cga aac gaa gcc gtt tgg atc ttt gtg       480
   Met Trp Leu Phe Tyr Asn Tyr Arg Asn Glu Ala Val Trp Ile Phe Val
   145                 150                 155                 160
   ctg ctc aac ggc ttc att cac tgg atc atg tac ggc tac tat tgg acc       528
   Leu Leu Asn Gly Phe Ile His Trp Ile Met Tyr Gly Tyr Tyr Trp Thr
                   165                 170                 175
   cga ctg atc aag ctc aag ttc cct atg ccc aag tcc ctg att act tct       576
   Arg Leu Ile Lys Leu Lys Phe Pro Met Pro Lys Ser Leu Ile Thr Ser
               180                 185                 190
   atg cag atc att cag ttc aac gtt ggc ttc tac atc gtc tgg aag tac       624
   Met Gln Ile Ile Gln Phe Asn Val Gly Phe Tyr Ile Val Trp Lys Tyr
           195                 200                 205
   cgg aac att ccc tgc tac cga caa gat gga atg aga atg ttt ggc tgg       672
   Arg Asn Ile Pro Cys Tyr Arg Gln Asp Gly Met Arg Met Phe Gly Trp
       210                 215                 220
   ttt ttc aac tac ttc tac gtt ggt act gtc ctg tgt ctg ttc ctc aac         720
   Phe Phe Asn Tyr Phe Tyr Val Gly Thr Val Leu Cys Leu Phe Leu Asn
   225                 230                 235                 240
   ttc tac gtg cag acc tac atc gtc cga aag cac aag gga gcc aaa aag         768
   Phe Tyr Val Gln Thr Tyr Ile Val Arg Lys His Lys Gly Ala Lys Lys
                   245                 250                 255
   att cag tga                                                             777
   Ile Gln
<210> 46
   <211> 258
   <212> PRT
   <213> Euglena gracilis
<400> 46
   Met Glu Val Val Asn Glu Ile Val Ser Ile Gly Gln Glu Val Leu Pro
   1 5 10 15
   Lys Val Asp Tyr Ala Gln Leu Trp Ser Asp Ala Ser His Cys Glu Val
   20 25 30
   Leu Tyr Leu Ser Ile Ala Phe Val Ile Leu Lys Phe Thr Leu Gly Pro
   35 40 45
   Leu Gly Pro Lys Gly Gln Ser Arg Met Lys Phe Val Phe Thr Asn Tyr
   50 55 60
   Asn Leu Leu Met Ser Ile Tyr Ser Leu Gly Ser Phe Leu Ser Met Ala
   65 70 75 80
   Tyr Ala Met Tyr Thr Ile Gly Val Met Ser Asp Asn Cys Glu Lys Ala
   85 90 95
   Phe Asp Asn Asn Val Phe Arg Ile Thr Thr Gln Leu Phe Tyr Leu Ser
   100 105 110
   Lys Phe Leu Glu Tyr Ile Asp Ser Phe Tyr Leu Pro Leu Met Gly Lys
   115 120 125
   Pro Leu Thr Trp Leu Gln Phe Phe His His Leu Gly Ala Pro Met Asp
   130 135 140
   Met Trp Leu Phe Tyr Asn Tyr Arg Asn Glu Ala Val Trp Ile Phe Val
   145 150 155 160
   Leu Leu Asn Gly Phe Ile His Trp Ile Met Tyr Gly Tyr Tyr Trp Thr
   165 170 175
   Arg Leu Ile Lys Leu Lys Phe Pro Met Pro Lys Ser Leu Ile Thr Ser
   180 185 190
   Met Gln Ile Ile Gln Phe Asn Val Gly Phe Tyr Ile Val Trp Lys Tyr
   195 200 205
   Arg Asn Ile Pro Cys Tyr Arg Gln Asp Gly Met Arg Met Phe Gly Trp
   210 215 220
   Phe Phe Asn Tyr Phe Tyr Val Gly Thr Val Leu Cys Leu Phe Leu Asn
   225 230 235 240
   Phe Tyr Val Gln Thr Tyr Ile Val Arg Lys His Lys Gly Ala Lys Lys
   245 250 255
   Ile Gln
<210> 47
   <211> 792
   <212> DNA
   <213> Eutreptiella sp. CCMP389
<220>
   <221> CDS
   <222> (1)..(792)
   <223> delta-9 elongase
<300>
   <302> DELTA-9 ELONGASES AND THEIR USE IN MAKING POLYUNSATURATED FATTY ACIDS
<310> WO 2007/061742
   <311> 2006-11-16
   <312> 2007-05-31
   <313> (1)..(792)
<300>
   <302> DELTA-9 ELONGASES AND THEIR USE IN MAKING POLYUNSATURATED FATTY ACIDS
<310> US 2007-0117190-A1
   <311> 2006-11-16
   <312> 2007-05-24
   <313> (1)..(792)
<400> 47
   atg gct gcg gtg ata gag gtc gcc aac gag ttt gta gcc atc acg gca        48
   Met Ala Ala Val Ile Glu Val Ala Asn Glu Phe Val Ala Ile Thr Ala
   1               5                   10                  15
   gaa acg ctc ccc aaa gtt gac tat caa cga cta tgg cga gac att tac        96
   Glu Thr Leu Pro Lys Val Asp Tyr Gln Arg Leu Trp Arg Asp Ile Tyr
               20                  25                  30
   agt tgt gag cta ctg tat ttc tcc att gcc ttc gtg atc ttg aag ttt       144
   Ser Cys Glu Leu Leu Tyr Phe Ser Ile Ala Phe Val Ile Leu Lys Phe
           35                  40                  45
   acg ttg ggc gag ttg agc gac agc gga aaa aag att ttg aga gtg ttg       192
   Thr Leu Gly Glu Leu Ser Asp Ser Gly Lys Lys Ile Leu Arg Val Leu
       50                  55                  60
   ttc aag tgg tac aat ctc ttc atg tcc gtg ttc tcc ttg gtg tct ttc      240
   Phe Lys Trp Tyr Asn Leu Phe Met Ser Val Phe Ser Leu Val Ser Phe
   65                  70                  75                  80
   ctt tgc atg ggc tat gcc att tat acc gtg ggc cta tac tct aac gaa      288
   Leu Cys Met Gly Tyr Ala Ile Tyr Thr Val Gly Leu Tyr Ser Asn Glu
                   85                  90                  95
   tgc gac agg gct ttc gac aac tcg ttg ttc cgc ttt gca aca aag gtg      336
   Cys Asp Arg Ala Phe Asp Asn Ser Leu Phe Arg Phe Ala Thr Lys Val
               100                 105                 110
   ttc tac tac agt aag ttt ttg gag tac atc gac tct ttt tat ctt ccg      384
   Phe Tyr Tyr Ser Lys Phe Leu Glu Tyr Ile Asp Ser Phe Tyr Leu Pro
           115                 120                 125
   ctc atg gcc aag ccg ctg tct ttc ctg caa ttc ttc cat cac ttg gga      432
   Leu Met Ala Lys Pro Leu Ser Phe Leu Gln Phe Phe His His Leu Gly
       130                 135                 140
   gcc ccc atg gac atg tgg ctc ttt gtc caa tat tct ggg gaa tct att      480
   Ala Pro Met Asp Met Trp Leu Phe Val Gln Tyr Ser Gly Glu Ser Ile
   145                 150                 155                 160
   tgg atc ttt gtg ttt ttg aat ggg ttc att cac ttt gtt atg tac ggg      528
   Trp Ile Phe Val Phe Leu Asn Gly Phe Ile His Phe Val Met Tyr Gly
                   165                 170                 175
   tac tac tgg act cgg ctg atg aag ttc aat ttc cca atg ccc aag cag      576
   Tyr Tyr Trp Thr Arg Leu Met Lys Phe Asn Phe Pro Met Pro Lys Gln
               180                 185                 190
   ttg att acc gcg atg cag atc acg cag ttc aac gtt ggt ttc tac ctc      624
   Leu Ile Thr Ala Met Gln Ile Thr Gln Phe Asn Val Gly Phe Tyr Leu
           195                 200                 205
   gtg tgg tgg tac aaa gat att ccc tgc tac cga aag gat ccc atg cga      672
   Val Trp Trp Tyr Lys Asp Ile Pro Cys Tyr Arg Lys Asp Pro Met Arg
       210                 215                 220
   atg ttg gcc tgg atc ttc aat tac tgg tat gtt ggg act gtc ttg ctg      720
   Met Leu Ala Trp Ile Phe Asn Tyr Trp Tyr Val Gly Thr Val Leu Leu
   225                 230                 235                 240
   ctg ttc att aat ttc ttc gtc aaa tcc tat gtg ttc cca aag ccg aag      768
   Leu Phe Ile Asn Phe Phe Val Lys Ser Tyr Val Phe Pro Lys Pro Lys
                   245                 250                 255
   act gca gat aaa aag gtc caa tag                                      792
   Thr Ala Asp Lys Lys Val Gln
               260
<210> 48
   <211> 263
   <212> PRT
   <213> Eutreptiella sp. CCMP389
<400> 48
   Met Ala Ala Val Ile Glu Val Ala Asn Glu Phe Val Ala Ile Thr Ala
   1               5                   10                  15
   Glu Thr Leu Pro Lys Val Asp Tyr Gln Arg Leu Trp Arg Asp Ile Tyr
               20                  25                  30
   Ser Cys Glu Leu Leu Tyr Phe Ser Ile Ala Phe Val Ile Leu Lys Phe
           35                  40                  45
   Thr Leu Gly Glu Leu Ser Asp Ser Gly Lys Lys Ile Leu Arg Val Leu
       50                  55                  60
   Phe Lys Trp Tyr Asn Leu Phe Met Ser Val Phe Ser Leu Val Ser Phe
   65                  70                  75                  80
   Leu Cys Met Gly Tyr Ala Ile Tyr Thr Val Gly Leu Tyr Ser Asn Glu
                   85                  90                  95
   Cys Asp Arg Ala Phe Asp Asn Ser Leu Phe Arg Phe Ala Thr Lys Val
               100                 105                 110
   Phe Tyr Tyr Ser Lys Phe Leu Glu Tyr Ile Asp Ser Phe Tyr Leu Pro
           115                 120                 125
   Leu Met Ala Lys Pro Leu Ser Phe Leu Gln Phe Phe His His Leu Gly
       130                 135                 140
   Ala Pro Met Asp Met Trp Leu Phe Val Gln Tyr Ser Gly Glu Ser Ile
   145                 150                 155                 160
   Trp Ile Phe Val Phe Leu Asn Gly Phe Ile His Phe Val Met Tyr Gly
                   165                 170                 175
   Tyr Tyr Trp Thr Arg Leu Met Lys Phe Asn Phe Pro Met Pro Lys Gln
               180                 185                 190
   Leu Ile Thr Ala Met Gln Ile Thr Gln Phe Asn Val Gly Phe Tyr Leu
           195                 200                 205
   Val Trp Trp Tyr Lys Asp Ile Pro Cys Tyr Arg Lys Asp Pro Met Arg
       210                 215                 220
   Met Leu Ala Trp Ile Phe Asn Tyr Trp Tyr Val Gly Thr Val Leu Leu
   225                 230                 235                 240
   Leu Phe Ile Asn Phe Phe Val Lys Ser Tyr Val Phe Pro Lys Pro Lys
                   245                 250                 255
   Thr Ala Asp Lys Lys Val Gln
               260
<210> 49
   <211> 792
   <212> DNA
   <213> Eutreptiella sp. CCMP389
<220>
   <221> CDS
   <222> (1)..(792)
   <223> synthetic delta-9 elongase (codon-optimized for Yarrowia lipolytica)
<300>
   <302> DELTA-9 ELONGASES AND THEIR USE IN MAKING POLYUNSATURATED FATTY ACIDS
<310> WO 2007/061742
   <311> 2006-11-16
   <312> 2007-05-31
   <313> (1)..(792)
<300>
   <302> DELTA-9 ELONGASES AND THEIR USE IN MAKING POLYUNSATURATED FATTY ACIDS
<310> US 2007-0117190-A1
   <311> 2006-11-16
   <312> 2007-05-24
   <313> (1)..(792)
<400> 49
   atg gct gcc gtc atc gag gtg gcc aac gag ttc gtc gct atc act gcc        48
   Met Ala Ala Val Ile Glu Val Ala Asn Glu Phe Val Ala Ile Thr Ala
   1               5                   10                  15
   gag acc ctt ccc aag gtg gac tat cag cga ctc tgg cga gac atc tac        96
   Glu Thr Leu Pro Lys Val Asp Tyr Gln Arg Leu Trp Arg Asp Ile Tyr
               20                  25                  30
   tcc tgc gag ctc ctg tac ttc tcc att gct ttc gtc atc ctc aag ttt        144
   Ser Cys Glu Leu Leu Tyr Phe Ser Ile Ala Phe Val Ile Leu Lys Phe
           35                  40                  45
   acc ctt ggc gag ctc tcg gat tct ggc aaa aag att ctg cga gtg ctg        192
   Thr Leu Gly Glu Leu Ser Asp Ser Gly Lys Lys Ile Leu Arg Val Leu
       50                  55                  60
   ttc aag tgg tac aac ctc ttc atg tcc gtc ttt tcg ctg gtg tcc ttc        240
   Phe Lys Trp Tyr Asn Leu Phe Met Ser Val Phe Ser Leu Val Ser Phe
   65                  70                  75                  80
   ctc tgt atg ggt tac gcc atc tac acc gtt gga ctg tac tcc aac gaa        288
   Leu Cys Met Gly Tyr Ala Ile Tyr Thr Val Gly Leu Tyr Ser Asn Glu
                   85                  90                  95
   tgc gac aga gct ttc gac aac agc ttg ttc cga ttt gcc acc aag gtc        336
   Cys Asp Arg Ala Phe Asp Asn Ser Leu Phe Arg Phe Ala Thr Lys Val
               100                 105                 110
   ttc tac tat tcc aag ttt ctg gag tac atc gac tct ttc tac ctt ccc        384
   Phe Tyr Tyr Ser Lys Phe Leu Glu Tyr Ile Asp Ser Phe Tyr Leu Pro
           115                 120                 125
   ctc atg gcc aag cct ctg tcc ttt ctg cag ttc ttt cat cac ttg gga        432
   Leu Met Ala Lys Pro Leu Ser Phe Leu Gln Phe Phe His His Leu Gly
       130                 135                 140
   gct cct atg gac atg tgg ctc ttc gtg cag tac tct ggc gaa tcc att         480
   Ala Pro Met Asp Met Trp Leu Phe Val Gln Tyr Ser Gly Glu Ser Ile
   145                 150                 155                 160
   tgg atc ttt gtg ttc ctg aac gga ttc att cac ttt gtc atg tac ggc         528
   Trp Ile Phe Val Phe Leu Asn Gly Phe Ile His Phe Val Met Tyr Gly
                   165                 170                 175
   tac tat tgg aca cgg ctg atg aag ttc aac ttt ccc atg ccc aag cag         576
   Tyr Tyr Trp Thr Arg Leu Met Lys Phe Asn Phe Pro Met Pro Lys Gln
               180                 185                 190
   ctc att acc gca atg cag atc acc cag ttc aac gtt ggc ttc tac ctc         624
   Leu Ile Thr Ala Met Gln Ile Thr Gln Phe Asn Val Gly Phe Tyr Leu
           195                 200                 205
   gtg tgg tgg tac aag gac att ccc tgt tac cga aag gat ccc atg cga         672
   Val Trp Trp Tyr Lys Asp Ile Pro Cys Tyr Arg Lys Asp Pro Met Arg
       210                 215                 220
   atg ctg gcc tgg atc ttc aac tac tgg tac gtc ggt acc gtt ctt ctg         720
   Met Leu Ala Trp Ile Phe Asn Tyr Trp Tyr Val Gly Thr Val Leu Leu
   225                 230                 235                 240
   ctc ttc atc aac ttc ttt gtc aag tcc tac gtg ttt ccc aag cct aag         768
   Leu Phe Ile Asn Phe Phe Val Lys Ser Tyr Val Phe Pro Lys Pro Lys
                   245                 250                 255
   act gcc gac aaa aag gtc cag tag                                         792
   Thr Ala Asp Lys Lys Val Gln
               260
<210> 50
   <211> 263
   <212> PRT
   <213> Eutreptiella sp. CCMP389
<400> 50
   Met Ala Ala Val Ile Glu Val Ala Asn Glu Phe Val Ala Ile Thr Ala
   1               5                   10                  15
   Glu Thr Leu Pro Lys Val Asp Tyr Gln Arg Leu Trp Arg Asp Ile Tyr
               20                  25                  30
   Ser Cys Glu Leu Leu Tyr Phe Ser Ile Ala Phe Val Ile Leu Lys Phe
           35                  40                  45
   Thr Leu Gly Glu Leu Ser Asp Ser Gly Lys Lys Ile Leu Arg Val Leu
       50                  55                  60
   Phe Lys Trp Tyr Asn Leu Phe Met Ser Val Phe Ser Leu Val Ser Phe
   65                  70                  75                  80
   Leu Cys Met Gly Tyr Ala Ile Tyr Thr Val Gly Leu Tyr Ser Asn Glu
                   85                  90                  95
   Cys Asp Arg Ala Phe Asp Asn Ser Leu Phe Arg Phe Ala Thr Lys Val
                   100                 105                 110
   Phe Tyr Tyr Ser Lys Phe Leu Glu Tyr Ile Asp Ser Phe Tyr Leu Pro
           115                 120                 125
   Leu Met Ala Lys Pro Leu Ser Phe Leu Gln Phe Phe His His Leu Gly
       130                 135                 140
   Ala Pro Met Asp Met Trp Leu Phe Val Gln Tyr Ser Gly Glu Ser Ile
   145                 150                 155                 160
   Trp Ile Phe Val Phe Leu Asn Gly Phe Ile His Phe Val Met Tyr Gly
                   165                 170                 175
   Tyr Tyr Trp Thr Arg Leu Met Lys Phe Asn Phe Pro Met Pro Lys Gln
               180                 185                 190
   Leu Ile Thr Ala Met Gln Ile Thr Gln Phe Asn Val Gly Phe Tyr Leu
           195                 200                 205
   Val Trp Trp Tyr Lys Asp Ile Pro Cys Tyr Arg Lys Asp Pro Met Arg
       210                 215                 220
   Met Leu Ala Trp Ile Phe Asn Tyr Trp Tyr Val Gly Thr Val Leu Leu
   225                 230                 235                 240
   Leu Phe Ile Asn Phe Phe Val Lys Ser Tyr Val Phe Pro Lys Pro Lys
                   245                 250                 255
   Thr Ala Asp Lys Lys Val Gln
               260
<210> 51
   <211> 774
   <212> DNA
   <213> Euglena anabaena UTEX 373
<220>
   <221> CDS
   <222> (1)..(774)
   <223> delta-9 elongase
<300>
   <302> DELTA-9 ELONGASES AND THEIR USE IN MAKING POLYUNSATURATED FATTY ACIDS
<310> U.S. 2008-0254522-A1
   <311> 2008-04-15
   <312> 2008-10-16
   <313> (1)..(774)
<300>
   <302> DELTA-9 ELONGASES AND THEIR USE IN MAKING POLYUNSATURATED FATTY ACIDS
<310> WO 2008/128241
   <311> 2008-04-16
   <312> 2008-10-23
   <313> (1)..(774)
<400> 51
   atg gaa gca gcc aaa gaa ttg gtt tcc atc gtc caa gag gag ctc ccc 48
   Met Glu Ala Ala Lys Glu Leu Val Ser Ile Val Gln Glu Glu Leu Pro
   1 5 10 15
   aag gtg gac tat gcc cag ctt tgg cag gat gcc agc agc tgt gag gtc 96
   Lys Val Asp Tyr Ala Gln Leu Trp Gln Asp Ala Ser Ser Cys Glu Val
   20 25 30
   ctt tac ctc tcg gtg gca ttc gtg gcg atc aag ttc atg ctg cgc cca 144
   Leu Tyr Leu Ser Val Ala Phe Val Ala Ile Lys Phe Met Leu Arg Pro
   35 40 45
   ctg gac ctg aag cgc cag gcc acc ttg aag aag ctg ttc aca gca tac 192
   Leu Asp Leu Lys Arg Gln Ala Thr Leu Lys Lys Leu Phe Thr Ala Tyr
   50 55 60
   aac ttc ctc atg tcg atc tat tcc ttt ggc tcc ttc ctg gcc atg gcc 240
   Asn Phe Leu Met Ser Ile Tyr Ser Phe Gly Ser Phe Leu Ala Met Ala
   65 70 75 80
   tat gcc cta tca gta act ggc atc ctc tcc ggc gac tgt gag acg gcg 288
   Tyr Ala Leu Ser Val Thr Gly Ile Leu Ser Gly Asp Cys Glu Thr Ala
   85 90 95
   ttc aac aac gat gtg ttc agg atc aca act cag ctg ttc tac ctc agc 336
   Phe Asn Asn Asp Val Phe Arg Ile Thr Thr Gln Leu Phe Tyr Leu Ser
   100 105 110
   aag ttc gta gag tac atc gac tcc ttc tac ctt ccc ctt atg gac aag 384
   Lys Phe Val Glu Tyr Ile Asp Ser Phe Tyr Leu Pro Leu Met Asp Lys
   115 120 125
   cca ctg tcg ttc ctt cag ttc ttc cat cat ttg ggg gcc ccc att gac 432
   Pro Leu Ser Phe Leu Gln Phe Phe His His Leu Gly Ala Pro Ile Asp
   130 135 140
   atg tgg cta ttc tac aaa tac cgc aac gaa gga gtc tgg atc ttt gtc 480
   Met Trp Leu Phe Tyr Lys Tyr Arg Asn Glu Gly Val Trp Ile Phe Val
   145 150 155 160
   ctg ttg aat ggg ttc att cac tgg atc atg tac ggt tac tat tgg acg 528
   Leu Leu Asn Gly Phe Ile His Trp Ile Met Tyr Gly Tyr Tyr Trp Thr
   165 170 175
   cgg ctc atc aag ctg aac ttc ccc atg ccc aag aac ctg atc acc tcc 576
   Arg Leu Ile Lys Leu Asn Phe Pro Met Pro Lys Asn Leu Ile Thr Ser
   180 185 190
   atg cag atc atc cag ttc aat gtc ggg ttc tac atc gtc tgg aag tac 624
   Met Gln Ile Ile Gln Phe Asn Val Gly Phe Tyr Ile Val Trp Lys Tyr
   195 200 205
   cgc aat gtg cca tgc tac cgc cag gat ggg atg cgc atg ttt gcc tgg 672
   Arg Asn Val Pro Cys Tyr Arg Gln Asp Gly Met Arg Met Phe Ala Trp
   210 215 220
   atc ttc aac tac tgg tat gtc ggg acg gtc ttg ctg ctg ttc ctc aac 720
   Ile Phe Asn Tyr Trp Tyr Val Gly Thr Val Leu Leu Leu Phe Leu Asn
   225 230 235 240
   ttt tac gtg cag acg tac atc cgg aag ccg agg aag aac cga ggg aag        768
   Phe Tyr Val Gln Thr Tyr Ile Arg Lys Pro Arg Lys Asn Arg Gly Lys
                   245                 250                 255
   aag gag                                                                774
   Lys Glu
<210> 52
   <211> 258
   <212> PRT
   <213> Euglena anabaena UTEX 373
<400> 52
   Met Glu Ala Ala Lys Glu Leu Val Ser Ile Val Gln Glu Glu Leu Pro
   1               5                   10                  15
   Lys Val Asp Tyr Ala Gln Leu Trp Gln Asp Ala Ser Ser Cys Glu Val
               20                  25                  30
   Leu Tyr Leu Ser Val Ala Phe Val Ala Ile Lys Phe Met Leu Arg Pro
           35                  40                  45
   Leu Asp Leu Lys Arg Gln Ala Thr Leu Lys Lys Leu Phe Thr Ala Tyr
       50                  55                  60
   Asn Phe Leu Met Ser Ile Tyr Ser Phe Gly Ser Phe Leu Ala Met Ala
   65                  70                  75                  80
   Tyr Ala Leu Ser Val Thr Gly Ile Leu Ser Gly Asp Cys Glu Thr Ala
                   85                  90                  95
   Phe Asn Asn Asp Val Phe Arg Ile Thr Thr Gln Leu Phe Tyr Leu Ser
               100                 105                 110
   Lys Phe Val Glu Tyr Ile Asp Ser Phe Tyr Leu Pro Leu Met Asp Lys
           115                 120                 125
   Pro Leu Ser Phe Leu Gln Phe Phe His His Leu Gly Ala Pro Ile Asp
       130                 135                 140
   Met Trp Leu Phe Tyr Lys Tyr Arg Asn Glu Gly Val Trp Ile Phe Val
   145                 150                 155                 160
   Leu Leu Asn Gly Phe Ile His Trp Ile Met Tyr Gly Tyr Tyr Trp Thr
                   165                 170                 175
   Arg Leu Ile Lys Leu Asn Phe Pro Met Pro Lys Asn Leu Ile Thr Ser
               180                 185                 190
   Met Gln Ile Ile Gln Phe Asn Val Gly Phe Tyr Ile Val Trp Lys Tyr
           195                 200                 205
   Arg Asn Val Pro Cys Tyr Arg Gln Asp Gly Met Arg Met Phe Ala Trp
       210                 215                 220
   Ile Phe Asn Tyr Trp Tyr Val Gly Thr Val Leu Leu Leu Phe Leu Asn
   225                 230                 235                 240
   Phe Tyr Val Gln Thr Tyr Ile Arg Lys Pro Arg Lys Asn Arg Gly Lys
                   245                 250                 255
   Lys Glu
<210> 53
   <211> 774
   <212> DNA
   <213> Euglena anabaena UTEX 373
<220>
   <221> CDS
   <222> (1)..(774)
   <223> synthetic delta-9 elongase (codon-optimized for Yarrowia lipolytica)
<300>
   <302> DELTA-9 ELONGASES AND THEIR USE IN MAKING POLYUNSATURATED FATTY ACIDS
<310> U.S. 2008-0254522-A1
   <311> 2008-04-15
   <312> 2008-10-16
   <313> (1)..(774)
<300>
   <302> DELTA-9 ELONGASES AND THEIR USE IN MAKING POLYUNSATURATED FATTY ACIDS
<310> WO 2008/128241
   <311> 2008-04-16
   <312> 2008-10-23
   <313> (1)..(774)
<400> 53
   atg gag gct gcc aag gag ctg gtc tcc atc gtc cag gag gaa ctt ccc        48
   Met Glu Ala Ala Lys Glu Leu Val Ser Ile Val Gln Glu Glu Leu Pro
   1               5                   10                  15
   aag gtg gac tac gcc cag ctc tgg cag gac gcc tcc tct tgc gag gtt        96
   Lys Val Asp Tyr Ala Gln Leu Trp Gln Asp Ala Ser Ser Cys Glu Val
               20                  25                  30
   ctg tac ctc tcg gtc gct ttc gtg gcc atc aag ttc atg ctt cga cct       144
   Leu Tyr Leu Ser Val Ala Phe Val Ala Ile Lys Phe Met Leu Arg Pro
           35                  40                  45
   ctg gac ctc aag cga caa gcc acc ctc aaa aag ctg ttc acc gca tac       192
   Leu Asp Leu Lys Arg Gln Ala Thr Leu Lys Lys Leu Phe Thr Ala Tyr
       50                  55                  60
   aac ttt ctc atg tcc atc tac tcg ttc ggc tcc ttc ctg gcg atg gcc      240
   Asn Phe Leu Met Ser Ile Tyr Ser Phe Gly Ser Phe Leu Ala Met Ala
   65                  70                  75                  80
   tac gct ctc tct gtc act ggt att ctt tcc ggc gat tgt gag act gcc      288
   Tyr Ala Leu Ser Val Thr Gly Ile Leu Ser Gly Asp Cys Glu Thr Ala
                   85                  90                  95
   ttc aac aat gac gtg ttc cga atc acc act cag ctg ttc tac ctc agc      336
   Phe Asn Asn Asp Val Phe Arg Ile Thr Thr Gln Leu Phe Tyr Leu Ser
               100                 105                 110
   aag ttc gtc gag tac atc gac tcc ttc tac ctt ccc ctc atg gac aag      384
   Lys Phe Val Glu Tyr Ile Asp Ser Phe Tyr Leu Pro Leu Met Asp Lys
           115                 120                 125
   ccc ttg tcg ttt ctg cag ttc ttt cac cat ctc gga gct ccc atc gac      432
   Pro Leu Ser Phe Leu Gln Phe Phe His His Leu Gly Ala Pro Ile Asp
       130                 135                 140
   atg tgg ctg ttc tac aag tat cga aac gaa ggc gtc tgg atc ttt gtt      480
   Met Trp Leu Phe Tyr Lys Tyr Arg Asn Glu Gly Val Trp Ile Phe Val
   145                 150                 155                 160
   ctg ctc aac ggc ttc att cac tgg atc atg tac ggt tac tat tgg acg      528
   Leu Leu Asn Gly Phe Ile His Trp Ile Met Tyr Gly Tyr Tyr Trp Thr
                   165                 170                 175
   cga ctc atc aag ctg aac ttc cct atg ccc aag aac ctc att acc tcc      576
   Arg Leu Ile Lys Leu Asn Phe Pro Met Pro Lys Asn Leu Ile Thr Ser
               180                 185                 190
   atg caa att atc cag ttc aac gtc gga ttc tac atc gtc tgg aag tac      624
   Met Gln Ile Ile Gln Phe Asn Val Gly Phe Tyr Ile Val Trp Lys Tyr
           195                 200                 205
   cga aac gtg ccc tgc tac cgg cag gac ggt atg cga atg ttt gcc tgg      672
   Arg Asn Val Pro Cys Tyr Arg Gln Asp Gly Met Arg Met Phe Ala Trp
       210                 215                 220
   atc ttc aac tac tgg tat gtc ggc acg gtg ctg ctt ctg ttc ctc aac      720
   Ile Phe Asn Tyr Trp Tyr Val Gly Thr Val Leu Leu Leu Phe Leu Asn
   225                 230                 235                 240
   ttc tac gtc cag acc tac att cgg aag cct cga aag aac cga ggc aaa      768
   Phe Tyr Val Gln Thr Tyr Ile Arg Lys Pro Arg Lys Asn Arg Gly Lys
                   245                 250                 255
   aag gag                                                              774
   Lys Glu
<210> 54
   <211> 258
   <212> PRT
   <213> Euglena anabaena UTEX 373
<400> 54
   Met Glu Ala Ala Lys Glu Leu Val Ser Ile Val Gln Glu Glu Leu Pro
   1               5                   10                  15
   Lys Val Asp Tyr Ala Gln Leu Trp Gln Asp Ala Ser Ser Cys Glu Val
               20                  25                  30
   Leu Tyr Leu Ser Val Ala Phe Val Ala Ile Lys Phe Met Leu Arg Pro
           35                  40                  45
   Leu Asp Leu Lys Arg Gln Ala Thr Leu Lys Lys Leu Phe Thr Ala Tyr
       50                  55                  60
   Asn Phe Leu Met Ser Ile Tyr Ser Phe Gly Ser Phe Leu Ala Met Ala
   65                  70                  75                  80
   Tyr Ala Leu Ser Val Thr Gly Ile Leu Ser Gly Asp Cys Glu Thr Ala
                   85                  90                  95
   Phe Asn Asn Asp Val Phe Arg Ile Thr Thr Gln Leu Phe Tyr Leu Ser
               100                 105                 110
   Lys Phe Val Glu Tyr Ile Asp Ser Phe Tyr Leu Pro Leu Met Asp Lys
           115                 120                 125
   Pro Leu Ser Phe Leu Gln Phe Phe His His Leu Gly Ala Pro Ile Asp
       130                 135                 140
   Met Trp Leu Phe Tyr Lys Tyr Arg Asn Glu Gly Val Trp Ile Phe Val
   145                 150                 155                 160
   Leu Leu Asn Gly Phe Ile His Trp Ile Met Tyr Gly Tyr Tyr Trp Thr
                   165                 170                 175
   Arg Leu Ile Lys Leu Asn Phe Pro Met Pro Lys Asn Leu Ile Thr Ser
               180                 185                 190
   Met Gln Ile Ile Gln Phe Asn Val Gly Phe Tyr Ile Val Trp Lys Tyr
           195                 200                 205
   Arg Asn Val Pro Cys Tyr Arg Gln Asp Gly Met Arg Met Phe Ala Trp
       210                 215                 220
   Ile Phe Asn Tyr Trp Tyr Val Gly Thr Val Leu Leu Leu Phe Leu Asn
   225                 230                 235                 240
   Phe Tyr Val Gln Thr Tyr Ile Arg Lys Pro Arg Lys Asn Arg Gly Lys
                   245                 250                 255
   Lys Glu
<210> 55
   <211> 1271
   <212> DNA
   <213> Euglena gracilis
<220>
   <221> CDS
   <222> (4)..(1269)
   <223> delta-8 desaturase ("Eg5" or "EgD8")
<300>
   <302> DELTA-8 DESATURASE AND ITS USE IN MAKING POLYUNSATURATED FATTY ACIDS
<310> WO 2006/012325 and WO 2006/012326
   <311> 2005-06-24
   <312> 2006-02-02
   <313> (1)..(1271)
<300>
   <302> DELTA-8 DESATURASE AND ITS USE IN MAKING POLYUNSATURATED FATTY ACIDS
<310> U.S. 7,256,033
   <311> 2005-06-24
   <312> 2007-08-14
   <313> (1)..(1271)
<400> 55
   gaa atg aag tca aag cgc caa gcg ctt ccc ctt aca att gat gga aca        48
       Met Lys Ser Lys Arg Gln Ala Leu Pro Leu Thr Ile Asp Gly Thr
       1               5                   10                  15
   aca tat gat gtg tct gcc tgg gtc aat ttc cac cct ggt ggt gcg gaa        96
   Thr Tyr Asp Val Ser Ala Trp Val Asn Phe His Pro Gly Gly Ala Glu
                   20                  25                  30
   att ata gag aat tac caa gga agg gat gcc act gat gcc ttc atg gtt       144
   Ile Ile Glu Asn Tyr Gln Gly Arg Asp Ala Thr Asp Ala Phe Met Val
               35                  40                  45
   atg cac tct caa gaa gcc ttc gac aag ctc aag cgc atg ccc aaa atc       192
   Met His Ser Gln Glu Ala Phe Asp Lys Leu Lys Arg Met Pro Lys Ile
           50                  55                  60
   aat ccc agt tct gag ttg cca ccc cag gct gca gtg aat gaa gct caa       240
   Asn Pro Ser Ser Glu Leu Pro Pro Gln Ala Ala Val Asn Glu Ala Gln
       65                  70                  75
   gag gat ttc cgg aag ctc cga gaa gag ttg atc gca act ggc atg ttt       288
   Glu Asp Phe Arg Lys Leu Arg Glu Glu Leu Ile Ala Thr Gly Met Phe
   80                  85                  90                  95
   gat gcc tcc ccc ctc tgg tac tca tac aaa atc agc acc aca ctg ggc       336
   Asp Ala Ser Pro Leu Trp Tyr Ser Tyr Lys Ile Ser Thr Thr Leu Gly
                   100                 105                 110
   ctt gga gtg ctg ggt tat ttc ctg atg gtt cag tat cag atg tat ttc       384
   Leu Gly Val Leu Gly Tyr Phe Leu Met Val Gln Tyr Gln Met Tyr Phe
               115                 120                 125
   att ggg gca gtg ttg ctt ggg atg cac tat caa cag atg ggc tgg ctt       432
   Ile Gly Ala Val Leu Leu Gly Met His Tyr Gln Gln Met Gly Trp Leu
           130                 135                 140
   tct cat gac att tgc cac cac cag act ttc aag aac cgg aac tgg aac       480
   Ser His Asp Ile Cys His His Gln Thr Phe Lys Asn Arg Asn Trp Asn
       145                 150                 155
   aac ctc gtg gga ctg gta ttt ggc aat ggt ctg caa ggt ttt tcc gtg 528
   Asn Leu Val Gly Leu Val Phe Gly Asn Gly Leu Gln Gly Phe Ser Val
   160 165 170 175
   aca tgg tgg aag gac aga cac aat gca cat cat tcg gca acc aat gtt 576
   Thr Trp Trp Lys Asp Arg His Asn Ala His His Ser Ala Thr Asn Val
   180 185 190
   caa ggg cac gac cct gat att gac aac ctc ccc ctc tta gcc tgg tct 624
   Gln Gly His Asp Pro Asp Ile Asp Asn Leu Pro Leu Leu Ala Trp Ser
   195 200 205
   gag gat gac gtc aca cgg gcg tca ccg att tcc cgc aag ctc att cag 672
   Glu Asp Asp Val Thr Arg Ala Ser Pro Ile Ser Arg Lys Leu Ile Gln
   210 215 220
   ttc cag cag tac tat ttc ttg gtc atc tgt atc ttg ttg cgg ttc att 720
   Phe Gln Gln Tyr Tyr Phe Leu Val Ile Cys Ile Leu Leu Arg Phe Ile
   225 230 235
   tgg tgt ttc cag agc gtg ttg acc gtg cgc agt ttg aag gac aga gat 768
   Trp Cys Phe Gln Ser Val Leu Thr Val Arg Ser Leu Lys Asp Arg Asp
   240 245 250 255
   aac caa ttc tat cgc tct cag tat aag aag gag gcc att ggc ctc gcc 816
   Asn Gln Phe Tyr Arg Ser Gln Tyr Lys Lys Glu Ala Ile Gly Leu Ala
   260 265 270
   ctg cac tgg acc ttg aag acc ctg ttc cac tta ttc ttt atg ccc agc 864
   Leu His Trp Thr Leu Lys Thr Leu Phe His Leu Phe Phe Met Pro Ser
   275 280 285
   atc ctc aca tcg ctg ttg gtg ttt ttc gtt tcg gag ctg gtt ggc ggc 912
   Ile Leu Thr Ser Leu Leu Val Phe Phe Val Ser Glu Leu Val Gly Gly
   290 295 300
   ttc ggc att gcg atc gtg gtg ttc atg aac cac tac cca ctg gag aag 960
   Phe Gly Ile Ala Ile Val Val Phe Met Asn His Tyr Pro Leu Glu Lys
   305 310 315
   atc ggg gac tca gtc tgg gat ggc cat gga ttc tcg gtt ggc cag atc 1008
   Ile Gly Asp Ser Val Trp Asp Gly His Gly Phe Ser Val Gly Gln Ile
   320 325 330 335
   cat gag acc atg aac att cgg cga ggg att atc aca gat tgg ttt ttc 1056
   His Glu Thr Met Asn Ile Arg Arg Gly Ile Ile Thr Asp Trp Phe Phe
   340 345 350
   gga ggc ttg aat tac cag att gag cac cat ttg tgg ccg acc ctc cct 1104
   Gly Gly Leu Asn Tyr Gln Ile Glu His His Leu Trp Pro Thr Leu Pro
   355 360 365
   cgc cac aac ctg aca gcg gtt agc tac cag gtg gaa cag ctg tgc cag 1152
   Arg His Asn Leu Thr Ala Val Ser Tyr Gln Val Glu Gln Leu Cys Gln
   370 375 380
   aag cac aac ctg ccg tat cgg aac ccg ctg ccc cat gaa ggg ttg gtc 1200
   Lys His Asn Leu Pro Tyr Arg Asn Pro Leu Pro His Glu Gly Leu Val
   385 390 395
   atc ctg ctg cgc tat ctg gcg gtg ttc gcc cgg atg gcg gag aag caa 1248
   Ile Leu Leu Arg Tyr Leu Ala Val Phe Ala Arg Met Ala Glu Lys Gln
   400 405 410 415
   ccc gcg ggg aag gct cta taa gg                                        1271
   Pro Ala Gly Lys Ala Leu
                       420
<210> 56
   <211> 421
   <212> PRT
   <213> Euglena gracilis
<400> 56
   Met Lys Ser Lys Arg Gln Ala Leu Pro Leu Thr Ile Asp Gly Thr Thr
   1               5                   10                  15
   Tyr Asp Val Ser Ala Trp Val Asn Phe His Pro Gly Gly Ala Glu Ile
               20                  25                  30
   Ile Glu Asn Tyr Gln Gly Arg Asp Ala Thr Asp Ala Phe Met Val Met
           35                  40                  45
   His Ser Gln Glu Ala Phe Asp Lys Leu Lys Arg Met Pro Lys Ile Asn
       50                  55                  60
   Pro Ser Ser Glu Leu Pro Pro Gln Ala Ala Val Asn Glu Ala Gln Glu
   65                  70                  75                  80
   Asp Phe Arg Lys Leu Arg Glu Glu Leu Ile Ala Thr Gly Met Phe Asp
                   85                  90                  95
   Ala Ser Pro Leu Trp Tyr Ser Tyr Lys Ile Ser Thr Thr Leu Gly Leu
               100                 105                 110
   Gly Val Leu Gly Tyr Phe Leu Met Val Gln Tyr Gln Met Tyr Phe Ile
           115                 120                 125
   Gly Ala Val Leu Leu Gly Met His Tyr Gln Gln Met Gly Trp Leu Ser
       130                 135                 140
   His Asp Ile Cys His His Gln Thr Phe Lys Asn Arg Asn Trp Asn Asn
   145                 150                 155                 160
   Leu Val Gly Leu Val Phe Gly Asn Gly Leu Gln Gly Phe Ser Val Thr
                   165                 170                 175
   Trp Trp Lys Asp Arg His Asn Ala His His Ser Ala Thr Asn Val Gln
               180                 185                 190
   Gly His Asp Pro Asp Ile Asp Asn Leu Pro Leu Leu Ala Trp Ser Glu
           195                 200                 205
   Asp Asp Val Thr Arg Ala Ser Pro Ile Ser Arg Lys Leu Ile Gln Phe
       210                 215                 220
   Gln Gln Tyr Tyr Phe Leu Val Ile Cys Ile Leu Leu Arg Phe Ile Trp
   225                 230                 235                 240
   Cys Phe Gln Ser Val Leu Thr Val Arg Ser Leu Lys Asp Arg Asp Asn
                   245                 250                 255
   Gln Phe Tyr Arg Ser Gln Tyr Lys Lys Glu Ala Ile Gly Leu Ala Leu
               260                 265                 270
   His Trp Thr Leu Lys Thr Leu Phe His Leu Phe Phe Met Pro Ser Ile
           275                 280                 285
   Leu Thr Ser Leu Leu Val Phe Phe Val Ser Glu Leu Val Gly Gly Phe
       290                 295                 300
   Gly Ile Ala Ile Val Val Phe Met Asn His Tyr Pro Leu Glu Lys Ile
   305                 310                 315                 320
   Gly Asp Ser Val Trp Asp Gly His Gly Phe Ser Val Gly Gln Ile His
                   325                 330                 335
   Glu Thr Met Asn Ile Arg Arg Gly Ile Ile Thr Asp Trp Phe Phe Gly
               340                 345                 350
   Gly Leu Asn Tyr Gln Ile Glu His His Leu Trp Pro Thr Leu Pro Arg
           355                 360                 365
   His Asn Leu Thr Ala Val Ser Tyr Gln Val Glu Gln Leu Cys Gln Lys
       370                 375                 380
   His Asn Leu Pro Tyr Arg Asn Pro Leu Pro His Glu Gly Leu Val Ile
   385                 390                 395                 400
   Leu Leu Arg Tyr Leu Ala Val Phe Ala Arg Met Ala Glu Lys Gln Pro
                   405                 410                 415
   Ala Gly Lys Ala Leu
               420
<210> 57
   <211> 1272
   <212> DNA
   <213> Euglena gracilis
<220>
   <221> CDS
   <222> (2)..(1270)
   <223> synthetic delta-8 desaturase (codon-optimized for Yarrowia lipolytica) ("D8SF" or "EgD8S")
<300>
   <302> DELTA-8 DESATURASE AND ITS USE IN MAKING POLYUNSATURATED FATTY ACIDS
<310> WO 2006/012325 and WO 2006/012326
   <311> 2005-06-24
   <312> 2006-02-02
   <313> (1)..(1272)
<300>
   <302> DELTA-8 DESATURASE AND ITS USE IN MAKING POLYUNSATURATED FATTY ACIDS
<310> U.S. 7,256,033
   <311> 2005-06-24
   <312> 2007-08-14
   <313> (1)..(1272)
<400> 57
   c atg gtg aag tcc aag cga cag gct ctg ccc ctc acc atc gac gga act        49
     Met Val Lys Ser Lys Arg Gln Ala Leu Pro Leu Thr Ile Asp Gly Thr
     1               5                   10                  15
   acc tac gac gtc tcc gct tgg gtg aac ttc cac cct ggt gga gct gaa          97
   Thr Tyr Asp Val Ser Ala Trp Val Asn Phe His Pro Gly Gly Ala Glu
               20                  25                  30
   atc att gag aac tac cag gga cga gat gct act gac gcc ttc atg gtt         145
   Ile Ile Glu Asn Tyr Gln Gly Arg Asp Ala Thr Asp Ala Phe Met Val
           35                  40                  45
   atg cac tct cag gaa gcc ttc gac aag ctc aag cga atg ccc aag atc         193
   Met His Ser Gln Glu Ala Phe Asp Lys Leu Lys Arg Met Pro Lys Ile
       50                  55                  60
   aac ccc tcc tcc gag ctg cct ccc cag gct gcc gtc aac gaa gct cag         241
   Asn Pro Ser Ser Glu Leu Pro Pro Gln Ala Ala Val Asn Glu Ala Gln
   65                  70                  75                  80
   gag gat ttc cga aag ctc cga gaa gag ctg atc gcc act ggc atg ttt         289
   Glu Asp Phe Arg Lys Leu Arg Glu Glu Leu Ile Ala Thr Gly Met Phe
                   85                  90                  95
   gac gcc tct ccc ctc tgg tac tcg tac aag atc tcc acc acc ctg ggt         337
   Asp Ala Ser Pro Leu Trp Tyr Ser Tyr Lys Ile Ser Thr Thr Leu Gly
               100                 105                 110
   ctt ggc gtg ctt gga tac ttc ctg atg gtc cag tac cag atg tac ttc         385
   Leu Gly Val Leu Gly Tyr Phe Leu Met Val Gln Tyr Gln Met Tyr Phe
           115                 120                 125
   att ggt gct gtg ctg ctc ggt atg cac tac cag caa atg gga tgg ctg         433
   Ile Gly Ala Val Leu Leu Gly Met His Tyr Gln Gln Met Gly Trp Leu
       130                 135                 140
   tct cat gac atc tgc cac cac cag acc ttc aag aac cga aac tgg aat         481
   Ser His Asp Ile Cys His His Gln Thr Phe Lys Asn Arg Asn Trp Asn
   145                 150                 155                 160
   aac ctc gtg ggt ctg gtc ttt ggc aac gga ctc cag ggc ttc tcc gtg         529
   Asn Leu Val Gly Leu Val Phe Gly Asn Gly Leu Gln Gly Phe Ser Val
                   165                 170                 175
   acc tgg tgg aag gac aga cac aac gcc cat cat tct gct acc aac gtt         577
   Thr Trp Trp Lys Asp Arg His Asn Ala His His Ser Ala Thr Asn Val
               180                 185                 190
   cag ggt cac gat ccc gac att gat aac ctg cct ctg ctc gcc tgg tcc           625
   Gln Gly His Asp Pro Asp Ile Asp Asn Leu Pro Leu Leu Ala Trp Ser
           195                 200                 205
   gag gac gat gtc act cga gct tct ccc atc tcc cga aag ctc att cag           673
   Glu Asp Asp Val Thr Arg Ala Ser Pro Ile Ser Arg Lys Leu Ile Gln
       210                 215                 220
   ttc caa cag tac tat ttc ctg gtc atc tgt att ctc ctg cga ttc atc           721
   Phe Gln Gln Tyr Tyr Phe Leu Val Ile Cys Ile Leu Leu Arg Phe Ile
   225                 230                 235                 240
   tgg tgt ttc cag tct gtg ctg acc gtt cga tcc ctc aag gac cga gac           769
   Trp Cys Phe Gln Ser Val Leu Thr Val Arg Ser Leu Lys Asp Arg Asp
                   245                 250                 255
   aac cag ttc tac cga tct cag tac aag aaa gag gcc att gga ctc gct           817
   Asn Gln Phe Tyr Arg Ser Gln Tyr Lys Lys Glu Ala Ile Gly Leu Ala
               260                 265                 270
   ctg cac tgg act ctc aag acc ctg ttc cac ctc ttc ttt atg ccc tcc           865
   Leu His Trp Thr Leu Lys Thr Leu Phe His Leu Phe Phe Met Pro Ser
           275                 280                 285
   atc ctg acc tcg ctc ctg gtg ttc ttt gtt tcc gag ctc gtc ggt ggc           913
   Ile Leu Thr Ser Leu Leu Val Phe Phe Val Ser Glu Leu Val Gly Gly
       290                 295                 300
   ttc gga att gcc atc gtg gtc ttc atg aac cac tac cct ctg gag aag           961
   Phe Gly Ile Ala Ile Val Val Phe Met Asn His Tyr Pro Leu Glu Lys
   305                 310                 315                 320
   atc ggt gat tcc gtc tgg gac gga cat ggc ttc tct gtg ggt cag atc          1009
   Ile Gly Asp Ser Val Trp Asp Gly His Gly Phe Ser Val Gly Gln Ile
                   325                 330                 335
   cat gag acc atg aac att cga cga ggc atc att act gac tgg ttc ttt          1057
   His Glu Thr Met Asn Ile Arg Arg Gly Ile Ile Thr Asp Trp Phe Phe
               340                 345                 350
   gga ggc ctg aac tac cag atc gag cac cat ctc tgg ccc acc ctg cct          1105
   Gly Gly Leu Asn Tyr Gln Ile Glu His His Leu Trp Pro Thr Leu Pro
           355                 360                 365
   cga cac aac ctc act gcc gtt tcc tac cag gtg gaa cag ctg tgc cag          1153
   Arg His Asn Leu Thr Ala Val Ser Tyr Gln Val Glu Gln Leu Cys Gln
       370                 375                 380
   aag cac aac ctc ccc tac cga aac cct ctg ccc cat gaa ggt ctc gtc          1201
   Lys His Asn Leu Pro Tyr Arg Asn Pro Leu Pro His Glu Gly Leu Val
   385                 390                 395                 400
   atc ctg ctc cga tac ctg gcc gtg ttc gct cga atg gcc gag aag cag          1249
   Ile Leu Leu Arg Tyr Leu Ala Val Phe Ala Arg Met Ala Glu Lys Gln
                   405                 410                 415
   ccc gct ggc aag gct ctc taa gc                                            1272
   Pro Ala Gly Lys Ala Leu
               420
<210> 58
   <211> 422
   <212> PRT
   <213> Euglena gracilis
<400> 58
   Met Val Lys Ser Lys Arg Gln Ala Leu Pro Leu Thr Ile Asp Gly Thr
   1               5                   10                  15
   Thr Tyr Asp Val Ser Ala Trp Val Asn Phe His Pro Gly Gly Ala Glu
               20                  25                  30
   Ile Ile Glu Asn Tyr Gln Gly Arg Asp Ala Thr Asp Ala Phe Met Val
           35                  40                  45
   Met His Ser Gln Glu Ala Phe Asp Lys Leu Lys Arg Met Pro Lys Ile
       50                  55                  60
   Asn Pro Ser Ser Glu Leu Pro Pro Gln Ala Ala Val Asn Glu Ala Gln
   65                  70                  75                  80
   Glu Asp Phe Arg Lys Leu Arg Glu Glu Leu Ile Ala Thr Gly Met Phe
                   85                  90                  95
   Asp Ala Ser Pro Leu Trp Tyr Ser Tyr Lys Ile Ser Thr Thr Leu Gly
               100                 105                 110
   Leu Gly Val Leu Gly Tyr Phe Leu Met Val Gln Tyr Gln Met Tyr Phe
           115                 120                 125
   Ile Gly Ala Val Leu Leu Gly Met His Tyr Gln Gln Met Gly Trp Leu
       130                 135                 140
   Ser His Asp Ile Cys His His Gln Thr Phe Lys Asn Arg Asn Trp Asn
   145                 150                 155                 160
   Asn Leu Val Gly Leu Val Phe Gly Asn Gly Leu Gln Gly Phe Ser Val
                   165                 170                 175
   Thr Trp Trp Lys Asp Arg His Asn Ala His His Ser Ala Thr Asn Val
               180                 185                 190
   Gln Gly His Asp Pro Asp Ile Asp Asn Leu Pro Leu Leu Ala Trp Ser
           195                 200                 205
   Glu Asp Asp Val Thr Arg Ala Ser Pro Ile Ser Arg Lys Leu Ile Gln
       210                 215                 220
   Phe Gln Gln Tyr Tyr Phe Leu Val Ile Cys Ile Leu Leu Arg Phe Ile
   225                 230                 235                 240
   Trp Cys Phe Gln Ser Val Leu Thr Val Arg Ser Leu Lys Asp Arg Asp
                   245                 250                 255
   Asn Gln Phe Tyr Arg Ser Gln Tyr Lys Lys Glu Ala Ile Gly Leu Ala
               260                 265                 270
   Leu His Trp Thr Leu Lys Thr Leu Phe His Leu Phe Phe Met Pro Ser
           275                 280                 285
   Ile Leu Thr Ser Leu Leu Val Phe Phe Val Ser Glu Leu Val Gly Gly
       290                 295                 300
   Phe Gly Ile Ala Ile Val Val Phe Met Asn His Tyr Pro Leu Glu Lys
   305                 310                 315                 320
   Ile Gly Asp Ser Val Trp Asp Gly His Gly Phe Ser Val Gly Gln Ile
                   325                 330                 335
   His Glu Thr Met Asn Ile Arg Arg Gly Ile Ile Thr Asp Trp Phe Phe
               340                 345                 350
   Gly Gly Leu Asn Tyr Gln Ile Glu His His Leu Trp Pro Thr Leu Pro
           355                 360                 365
   Arg His Asn Leu Thr Ala Val Ser Tyr Gln Val Glu Gln Leu Cys Gln
       370                 375                 380
   Lys His Asn Leu Pro Tyr Arg Asn Pro Leu Pro His Glu Gly Leu Val
   385                 390                 395                 400
   Ile Leu Leu Arg Tyr Leu Ala Val Phe Ala Arg Met Ala Glu Lys Gln
                   405                 410                 415
   Pro Ala Gly Lys Ala Leu
               420
<210> 59
   <211> 1272
   <212> DNA
   <213> Artificial Sequence
<220>
   <223> mutant EgD8S-23 delta-8 desaturase (also "EgD8M")
<220>
   <221> CDS
   <222> (2)..(1270)
<300>
   <302> MUTANT DELTA-8 DESATURASE GENES ENGINEERED BY TARGETED MUTAGENSIS AND THEIR USE IN MAKING POLYUNSATURATED FATTY ACIDS
<310> WO 2008/073271
   <311> 2007-12-05
   <312> 2008-06-19
   <313> (1)..(1272)
<300>
   <302> MUTANT DELTA-8 DESATURASE GENES ENGINEERED BY TARGETED MUTAGENSIS AND THEIR USE IN MAKING POLYUNSATURATED FATTY ACIDS
<310> US 2008-0138868-A1
   <311> 2006-12-07
   <312> 2008-06-12
   <313> (1)..(1272)
<400> 59
   c atg gtg aag gct tct cga cag gct ctg ccc ctc gtc atc gac gga aag        49
     Met Val Lys Ala Ser Arg Gln Ala Leu Pro Leu Val Ile Asp Gly Lys
     1               5                   10                  15
   gtg tac gac gtc tcc gct tgg gtg aac ttc cac cct ggt gga gct gaa          97
   Val Tyr Asp Val Ser Ala Trp Val Asn Phe His Pro Gly Gly Ala Glu
               20                  25                  30
   atc att gag aac tac cag gga cga gat gct act gac gcc ttc atg gtt         145
   Ile Ile Glu Asn Tyr Gln Gly Arg Asp Ala Thr Asp Ala Phe Met Val
           35                  40                  45
   atg cac tct cag gaa gcc ttc gac aag ctc aag cga atg ccc aag atc         193
   Met His Ser Gln Glu Ala Phe Asp Lys Leu Lys Arg Met Pro Lys Ile
       50                  55                  60
   aac cag gct tcc gag ctg cct ccc cag gct gcc gtc aac gaa gct cag         241
   Asn Gln Ala Ser Glu Leu Pro Pro Gln Ala Ala Val Asn Glu Ala Gln
   65                  70                  75                  80
   gag gat ttc cga aag ctc cga gaa gag ctg atc gcc act ggc atg ttt         289
   Glu Asp Phe Arg Lys Leu Arg Glu Glu Leu Ile Ala Thr Gly Met Phe
                   85                  90                  95
   gac gcc tct ccc ctc tgg tac tcg tac aag atc ttg acc acc ctg ggt         337
   Asp Ala Ser Pro Leu Trp Tyr Ser Tyr Lys Ile Leu Thr Thr Leu Gly
               100                 105                 110
   ctt ggc gtg ctt gcc ttc ttc atg ctg gtc cag tac cac ctg tac ttc         385
   Leu Gly Val Leu Ala Phe Phe Met Leu Val Gln Tyr His Leu Tyr Phe
           115                 120                 125
   att ggt gct ctc gtg ctc ggt atg cac tac cag caa atg gga tgg ctg         433
   Ile Gly Ala Leu Val Leu Gly Met His Tyr Gln Gln Met Gly Trp Leu
       130                 135                 140
   tct cat gac atc tgc cac cac cag acc ttc aag aac cga aac tgg aat         481
   Ser His Asp Ile Cys His His Gln Thr Phe Lys Asn Arg Asn Trp Asn
   145                 150                 155                 160
   aac gtc ctg ggt ctg gtc ttt ggc aac gga ctc cag ggc ttc tcc gtg         529
   Asn Val Leu Gly Leu Val Phe Gly Asn Gly Leu Gln Gly Phe Ser Val
                   165                 170                 175
   acc tgg tgg aag gac aga cac aac gcc cat cat tct gct acc aac gtt         577
   Thr Trp Trp Lys Asp Arg His Asn Ala His His Ser Ala Thr Asn Val
               180                 185                 190
   cag ggt cac gat ccc gac att gat aac ctg cct ctg ctc gcc tgg tcc         625
   Gln Gly His Asp Pro Asp Ile Asp Asn Leu Pro Leu Leu Ala Trp Ser
           195                 200                 205
   gag gac gat gtc act cga gct tct ccc atc tcc cga aag ctc att cag         673
   Glu Asp Asp Val Thr Arg Ala Ser Pro Ile Ser Arg Lys Leu Ile Gln
       210                 215                 220
   ttc caa cag tac tat ttc ctg gtc atc tgt att ctc ctg cga ttc atc         721
   Phe Gln Gln Tyr Tyr Phe Leu Val Ile Cys Ile Leu Leu Arg Phe Ile
   225                 230                 235                 240
   tgg tgt ttc cag tct gtg ctg acc gtt cga tcc ctc aag gac cga gac         769
   Trp Cys Phe Gln Ser Val Leu Thr Val Arg Ser Leu Lys Asp Arg Asp
                   245                 250                 255
   aac cag ttc tac cga tct cag tac aag aaa gag gcc att gga ctc gct         817
   Asn Gln Phe Tyr Arg Ser Gln Tyr Lys Lys Glu Ala Ile Gly Leu Ala
               260                 265                 270
   ctg cac tgg act ctc aag acc ctg ttc cac ctc ttc ttt atg ccc tcc         865
   Leu His Trp Thr Leu Lys Thr Leu Phe His Leu Phe Phe Met Pro Ser
           275                 280                 285
   atc ctg acc tcg atg ctg gtg ttc ttt gtt tcc gag ctc gtc ggt ggc         913
   Ile Leu Thr Ser Met Leu Val Phe Phe Val Ser Glu Leu Val Gly Gly
       290                 295                 300
   ttc gga att gcc atc gtg gtc ttc atg aac cac tac cct ctg gag aag         961
   Phe Gly Ile Ala Ile Val Val Phe Met Asn His Tyr Pro Leu Glu Lys
   305                 310                 315                 320
   atc ggt gat tcc gtc tgg gac gga cat ggc ttc tct gtg ggt cag atc        1009
   Ile Gly Asp Ser Val Trp Asp Gly His Gly Phe Ser Val Gly Gln Ile
                   325                 330                 335
   cat gag acc atg aac att cga cga ggc atc att act gac tgg ttc ttt        1057
   His Glu Thr Met Asn Ile Arg Arg Gly Ile Ile Thr Asp Trp Phe Phe
               340                 345                 350
   gga ggc ctg aac tac cag atc gag cac cat ctc tgg ccc acc ctg cct        1105
   Gly Gly Leu Asn Tyr Gln Ile Glu His His Leu Trp Pro Thr Leu Pro
           355                 360                 365
   cga cac aac ctc act gcc gtt tcc tac cag gtg gaa cag ctg tgc cag        1153
   Arg His Asn Leu Thr Ala Val Ser Tyr Gln Val Glu Gln Leu Cys Gln
       370                 375                 380
   aag cac aac ctc ccc tac cga aac cct ctg ccc cat gaa ggt ctc gtc        1201
   Lys His Asn Leu Pro Tyr Arg Asn Pro Leu Pro His Glu Gly Leu Val
   385                 390                 395                 400
   atc ctg ctc cga tac ctg tcc cag ttc gct cga atg gcc gag aag cag        1249
   Ile Leu Leu Arg Tyr Leu Ser Gln Phe Ala Arg Met Ala Glu Lys Gln
                   405                 410                 415
   ccc ggt gcc aag gct cag taa gc                                         1272
   Pro Gly Ala Lys Ala Gln
               420
<210> 60
   <211> 422
   <212> PRT
   <213> Artificial Sequence
<220>
   <223> Synthetic Construct
<210> 61
   <211> 1260
   <212> DNA
   <213> Euglena anabaena UTEX 373
<220>
   <221> CDS
   <222> (1)..(1260)
   <223> delta-8 desaturase
<300>
   <302> DELTA-8 DESATURASES AND THEIR USE IN MAKING POLYUNSATURATED FATTY ACIDS
<310> U.S. 2008-0254521-A1
   <311> 2008-04-09
   <312> 2008-10-16
   <313> (1)..(1260)
<300>
   <302> DELTA-8 DESATURASES AND THEIR USE IN MAKING POLYUNSATURATED FATTY ACIDS
<310> WO 2008/124194
   <311> 2008-04-10
   <312> 2008-10-16
   <313> (1)..(1260)
<210> 62
   <211> 420
   <212> PRT
   <213> Euglena anabaena UTEX 373
<400> 62
   Met Val Lys Arg Pro Ala Leu Pro Leu Thr Val Asp Gly Val Thr Tyr
   1               5                   10                  15
   Asp Val Ser Ala Trp Leu Asn His His Pro Gly Gly Ala Asp Ile Ile
               20                  25                  30
   Glu Asn Tyr Arg Gly Arg Asp Ala Thr Asp Val Phe Met Val Met His
           35                  40                  45
   Ser Glu Asn Ala Val Ser Lys Leu Arg Arg Met Pro Ile Met Glu Pro
       50                  55                  60
   Ser Ser Pro Leu Thr Pro Thr Pro Pro Lys Pro Asn Ser Asp Glu Pro
   65                  70                  75                  80
   Gln Glu Asp Phe Arg Lys Leu Arg Asp Glu Leu Ile Ala Ala Gly Met
                   85                  90                  95
   Phe Asp Ala Ser Pro Met Trp Tyr Ala Tyr Lys Thr Leu Ser Thr Leu
               100                 105                 110
   Gly Leu Gly Val Leu Ala Val Leu Leu Met Thr Gln Trp His Trp Tyr
           115                 120                 125
   Leu Val Gly Ala Ile Val Leu Gly Ile His Phe Gln Gln Met Gly Trp
       130                 135                 140
   Leu Ser His Asp Ile Cys His His Gln Leu Phe Lys Asp Arg Ser Ile
   145                 150                 155                 160
   Asn Asn Ala Ile Gly Leu Leu Phe Gly Asn Val Leu Gln Gly Phe Ser
                   165                 170                 175
   Val Thr Trp Trp Lys Asp Arg His Asn Ala His His Ser Ala Thr Asn
               180                 185                 190
   Val Gln Gly His Asp Pro Asp Ile Asp Asn Leu Pro Leu Leu Ala Trp
           195                 200                 205
   Ser Lys Glu Asp Val Glu Arg Ala Gly Pro Phe Ser Arg Arg Met Ile
       210                 215                 220
   Lys Tyr Gln Gln Tyr Tyr Phe Phe Phe Ile Cys Ala Leu Leu Arg Phe
   225                 230                 235                 240
   Ile Trp Cys Phe Gln Ser Ile His Thr Ala Thr Gly Leu Lys Asp Arg
                   245                 250                 255
   Ser Asn Gln Tyr Tyr Arg Arg Gln Tyr Glu Lys Glu Ser Val Gly Leu
               260                 265                 270
   Ala Leu His Trp Gly Leu Lys Ala Leu Phe Tyr Tyr Phe Tyr Met Pro
           275                 280                 285
   Ser Phe Leu Thr Gly Leu Met Val Phe Phe Val Ser Glu Leu Leu Gly
   290 295 300
   Gly Phe Gly Ile Ala Ile Val Val Phe Met Asn His Tyr Pro Leu Glu
   305 310 315 320
   Lys Ile Gln Asp Ser Val Trp Asp Gly His Gly Phe Cys Ala Gly Gln
   325 330 335
   Ile His Glu Thr Met Asn Val Gln Arg Gly Leu Val Thr Asp Trp Phe
   340 345 350
   Phe Gly Gly Leu Asn Tyr Gln Ile Glu His His Leu Trp Pro Thr Leu
   355 360 365
   Pro Arg His Asn Leu Thr Ala Ala Ser Ile Lys Val Glu Gln Leu Cys
   370 375 380
   Lys Lys His Asn Leu Pro Tyr Arg Ser Pro Pro Met Leu Glu Gly Val
   385 390 395 400
   Gly Ile Leu Ile Ser Tyr Leu Gly Thr Phe Ala Arg Met Val Ala Lys
   405 410 415
   Ala Asp Lys Ala
   420
<210> 63
   <211> 1260
   <212> DNA
   <213> Euglena anabaena UTEX 373
<220>
   <221> CDS
   <222> (1)..(1260)
   <223> synthetic delta-8 desaturase (codon-optimized for Yarrowia lipolytica)
<300>
   <302> DELTA-8 DESATURASES AND THEIR USE IN MAKING POLYUNSATURATED FATTY ACIDS
<310> U.S. 2008-0254521-A1
   <311> 2008-04-09
   <312> 2008-10-16
   <313> (1)..(1260)
<300>
   <302> DELTA-8 DESATURASES AND THEIR USE IN MAKING POLYUNSATURATED FATTY ACIDS
<310> WO 2008/124194
   <311> 2008-04-10
   <312> 2008-10-16
   <313> (1)..(1260)
<210> 64
   <211> 420
   <212> PRT
   <213> Euglena anabaena UTEX 373
<400> 64
   Met Val Lys Arg Pro Ala Leu Pro Leu Thr Val Asp Gly Val Thr Tyr
   1               5                   10                  15
   Asp Val Ser Ala Trp Leu Asn His His Pro Gly Gly Ala Asp Ile Ile
               20                  25                  30
   Glu Asn Tyr Arg Gly Arg Asp Ala Thr Asp Val Phe Met Val Met His
           35                  40                  45
   Ser Glu Asn Ala Val Ser Lys Leu Arg Arg Met Pro Ile Met Glu Pro
   50 55 60
   Ser Ser Pro Leu Thr Pro Thr Pro Pro Lys Pro Asn Ser Asp Glu Pro
   65 70 75 80
   Gln Glu Asp Phe Arg Lys Leu Arg Asp Glu Leu Ile Ala Ala Gly Met
   85 90 95
   Phe Asp Ala Ser Pro Met Trp Tyr Ala Tyr Lys Thr Leu Ser Thr Leu
   100 105 110
   Gly Leu Gly Val Leu Ala Val Leu Leu Met Thr Gln Trp His Trp Tyr
   115 120 125
   Leu Val Gly Ala Ile Val Leu Gly Ile His Phe Gln Gln Met Gly Trp
   130 135 140
   Leu Ser His Asp Ile Cys His His Gln Leu Phe Lys Asp Arg Ser Ile
   145 150 155 160
   Asn Asn Ala Ile Gly Leu Leu Phe Gly Asn Val Leu Gln Gly Phe Ser
   165 170 175
   Val Thr Trp Trp Lys Asp Arg His Asn Ala His His Ser Ala Thr Asn
   180 185 190
   Val Gln Gly His Asp Pro Asp Ile Asp Asn Leu Pro Leu Leu Ala Trp
   195 200 205
   Ser Lys Glu Asp Val Glu Arg Ala Gly Pro Phe Ser Arg Arg Met Ile
   210 215 220
   Lys Tyr Gln Gln Tyr Tyr Phe Phe Phe Ile Cys Ala Leu Leu Arg Phe
   225 230 235 240
   Ile Trp Cys Phe Gln Ser Ile His Thr Ala Thr Gly Leu Lys Asp Arg
   245 250 255
   Ser Asn Gln Tyr Tyr Arg Arg Gln Tyr Glu Lys Glu Ser Val Gly Leu
   260 265 270
   Ala Leu His Trp Gly Leu Lys Ala Leu Phe Tyr Tyr Phe Tyr Met Pro
   275 280 285
   Ser Phe Leu Thr Gly Leu Met Val Phe Phe Val Ser Glu Leu Leu Gly
   290 295 300
   Gly Phe Gly Ile Ala Ile Val Val Phe Met Asn His Tyr Pro Leu Glu
   305 310 315 320
   Lys Ile Gln Asp Ser Val Trp Asp Gly His Gly Phe Cys Ala Gly Gln
                   325                 330                 335
   Ile His Glu Thr Met Asn Val Gln Arg Gly Leu Val Thr Asp Trp Phe
               340                 345                 350
   Phe Gly Gly Leu Asn Tyr Gln Ile Glu His His Leu Trp Pro Thr Leu
           355                 360                 365
   Pro Arg His Asn Leu Thr Ala Ala Ser Ile Lys Val Glu Gln Leu Cys
       370                 375                 380
   Lys Lys His Asn Leu Pro Tyr Arg Ser Pro Pro Met Leu Glu Gly Val
   385                 390                 395                 400
   Gly Ile Leu Ile Ser Tyr Leu Gly Thr Phe Ala Arg Met Val Ala Lys
                   405                 410                 415
   Ala Asp Lys Ala
               420
<210> 65
   <211> 1350
   <212> DNA
   <213> Euglena gracilis
<220>
   <221> CDS
   <222> (1)..(1350)
   <223> delta-5 desaturase
<300>
   <302> DELTA-5 DESATURASE AND ITS USE IN MAKING POLYUNSATURATED FATTY ACIDS
<310> WO 2007/136671
   <311> 2007-05-16
   <312> 2007-11-29
   <313> (1)..(1350)
<300>
   <302> DELTA-5 DESATURASE AND ITS USE IN MAKING POLYUNSATURATED FATTY ACIDS
<310> US 2007-0292924-A1
   <311> 2007-05-15
   <312> 2007-12-20
   <313> (1)..(1350)
<400> 65
   atg gct ctc agt ctt acc aca gaa cag ctg tta gaa cgc cct gat ttg          48
   Met Ala Leu Ser Leu Thr Thr Glu Gln Leu Leu Glu Arg Pro Asp Leu
   1               5                   10                  15
   gtt gcg att gat ggc atc ctc tac gac ctt gaa ggg ctt gcc aaa gtt          96
   Val Ala Ile Asp Gly Ile Leu Tyr Asp Leu Glu Gly Leu Ala Lys Val
               20                  25                  30
   cat cca gga gga gat ttg att ctc gct tct ggt gcc tct gat gcc tcc         144
   His Pro Gly Gly Asp Leu Ile Leu Ala Ser Gly Ala Ser Asp Ala Ser
           35                  40                  45
   cct ctc ttt tat tca atg cat cca tac gtc aaa ccg gag aat tcc aaa         192
   Pro Leu Phe Tyr Ser Met His Pro Tyr Val Lys Pro Glu Asn Ser Lys
       50                  55                  60
   ttg ctt caa cag ttc gtc cga ggg aag cat gac cgc acc tcg aag gac         240
   Leu Leu Gln Gln Phe Val Arg Gly Lys His Asp Arg Thr Ser Lys Asp
   65                  70                  75                  80
   att gtc tac acg tat gat tct ccc ttc gca caa gac gtt aag cgg aca         288
   Ile Val Tyr Thr Tyr Asp Ser Pro Phe Ala Gln Asp Val Lys Arg Thr
                   85                  90                  95
   atg cgc gag gtg atg aaa ggg agg aac tgg tac gca acc cct ggc ttc         336
   Met Arg Glu Val Met Lys Gly Arg Asn Trp Tyr Ala Thr Pro Gly Phe
               100                 105                 110
   tgg ctg cgc acc gtt ggg atc atc gcc gtg acg gcc ttt tgc gag tgg         384
   Trp Leu Arg Thr Val Gly Ile Ile Ala Val Thr Ala Phe Cys Glu Trp
           115                 120                 125
   cac tgg gct acc acg ggg atg gtg ctg tgg ggc ctg ttg act gga ttc         432
   His Trp Ala Thr Thr Gly Met Val Leu Trp Gly Leu Leu Thr Gly Phe
       130                 135                 140
   atg cac atg cag atc ggc tta tcc atc cag cat gat gcg tcc cac ggg         480
   Met His Met Gln Ile Gly Leu Ser Ile Gln His Asp Ala Ser His Gly
   145                 150                 155                 160
   gcc atc agc aag aag cct tgg gtc aac gcc ctc ttc gcc tac ggc att         528
   Ala Ile Ser Lys Lys Pro Trp Val Asn Ala Leu Phe Ala Tyr Gly Ile
                   165                 170                 175
   gac gtc atc gga tcg tcc cgg tgg att tgg ctg cag tcg cac atc atg         576
   Asp Val Ile Gly Ser Ser Arg Trp Ile Trp Leu Gln Ser His Ile Met
               180                 185                 190
   cgg cac cac acc tac acc aac cag cac ggc ctc gac ctg gat gcg gag         624
   Arg His His Thr Tyr Thr Asn Gln His Gly Leu Asp Leu Asp Ala Glu
           195                 200                 205
   tcg gca gag ccg ttc ctg gtg ttc cac aac tac ccc gcc gca aac acc         672
   Ser Ala Glu Pro Phe Leu Val Phe His Asn Tyr Pro Ala Ala Asn Thr
       210                 215                 220
   gcc cga aag tgg ttc cac cgc ttc caa gct tgg tac atg tac ctt gtg         720
   Ala Arg Lys Trp Phe His Arg Phe Gln Ala Trp Tyr Met Tyr Leu Val
   225                 230                 235                 240
   ctg ggg gca tac ggg gta tcg ctg gtg tac aac ccg ctc tac att ttc         768
   Leu Gly Ala Tyr Gly Val Ser Leu Val Tyr Asn Pro Leu Tyr Ile Phe
                   245                 250                 255
   cgg atg cag cac aat gac acc atc cca gag tct gtc acg gcc atg cgg         816
   Arg Met Gln His Asn Asp Thr Ile Pro Glu Ser Val Thr Ala Met Arg
               260                 265                 270
   gag aat ggc ttt ctg cgg cgc tac cgc aca ctt gca ttc gtg atg cga         864
   Glu Asn Gly Phe Leu Arg Arg Tyr Arg Thr Leu Ala Phe Val Met Arg
           275                 280                 285
   gct ttc ttc atc ttc cgg acc gca ttc ttg ccc tgg tac ctc act ggg        912
   Ala Phe Phe Ile Phe Arg Thr Ala Phe Leu Pro Trp Tyr Leu Thr Gly
       290                 295                 300
   acc tca ttg ctg atc acc att cct ctg gtg ccc act gca act ggt gcc        960
   Thr Ser Leu Leu Ile Thr Ile Pro Leu Val Pro Thr Ala Thr Gly Ala
   305                 310                 315                 320
   ttc ttg acg ttc ttc ttc att ttg tcc cac aat ttt gat ggc tcc gaa       1008
   Phe Leu Thr Phe Phe Phe Ile Leu Ser His Asn Phe Asp Gly Ser Glu
                   325                 330                 335
   cgg atc ccc gac aag aac tgc aag gtt aag agc tct gag aag gac gtt       1056
   Arg Ile Pro Asp Lys Asn Cys Lys Val Lys Ser Ser Glu Lys Asp Val
               340                 345                 350
   gag gct gac caa att gac tgg tat cgg gcg cag gtg gag acg tcc tcc       1104
   Glu Ala Asp Gln Ile Asp Trp Tyr Arg Ala Gln Val Glu Thr Ser Ser
           355                 360                 365
   aca tac ggt ggc ccc atc gcc atg ttc ttc act ggc ggt ctc aat ttc       1152
   Thr Tyr Gly Gly Pro Ile Ala Met Phe Phe Thr Gly Gly Leu Asn Phe
       370                 375                 380
   cag atc gag cac cac ctc ttt ccc cgg atg tcg tct tgg cac tac ccc       1200
   Gln Ile Glu His His Leu Phe Pro Arg Met Ser Ser Trp His Tyr Pro
   385                 390                 395                 400
   ttc gtc cag cag gcg gtc cgg gag tgt tgc gaa cgc cat gga gtg cga       1248
   Phe Val Gln Gln Ala Val Arg Glu Cys Cys Glu Arg His Gly Val Arg
                   405                 410                 415
   tat gtt ttc tac cct acc atc gtc ggc aac atc atc tcc acc ctg aag       1296
   Tyr Val Phe Tyr Pro Thr Ile Val Gly Asn Ile Ile Ser Thr Leu Lys
               420                 425                 430
   tac atg cat aag gtg ggt gtc gtc cac tgc gtg aag gac gca cag gat       1344
   Tyr Met His Lys Val Gly Val Val His Cys Val Lys Asp Ala Gln Asp
           435                 440                 445
   tcc tga                                                                1350
   Ser
<210> 66
   <211> 449
   <212> PRT
   <213> Euglena gracilis
<400> 66
   Met Ala Leu Ser Leu Thr Thr Glu Gln Leu Leu Glu Arg Pro Asp Leu
   1               5                   10                  15
   Val Ala Ile Asp Gly Ile Leu Tyr Asp Leu Glu Gly Leu Ala Lys Val
               20                  25                  30
   His Pro Gly Gly Asp Leu Ile Leu Ala Ser Gly Ala Ser Asp Ala Ser
           35                  40                  45
   Pro Leu Phe Tyr Ser Met His Pro Tyr Val Lys Pro Glu Asn Ser Lys
   50 55 60
   Leu Leu Gln Gln Phe Val Arg Gly Lys His Asp Arg Thr Ser Lys Asp
   65 70 75 80
   Ile Val Tyr Thr Tyr Asp Ser Pro Phe Ala Gln Asp Val Lys Arg Thr
   85 90 95
   Met Arg Glu Val Met Lys Gly Arg Asn Trp Tyr Ala Thr Pro Gly Phe
   100 105 110
   Trp Leu Arg Thr Val Gly Ile Ile Ala Val Thr Ala Phe Cys Glu Trp
   115 120 125
   His Trp Ala Thr Thr Gly Met Val Leu Trp Gly Leu Leu Thr Gly Phe
   130 135 140
   Met His Met Gln Ile Gly Leu Ser Ile Gln His Asp Ala Ser His Gly
   145 150 155 160
   Ala Ile Ser Lys Lys Pro Trp Val Asn Ala Leu Phe Ala Tyr Gly Ile
   165 170 175
   Asp Val Ile Gly Ser Ser Arg Trp Ile Trp Leu Gln Ser His Ile Met
   180 185 190
   Arg His His Thr Tyr Thr Asn Gln His Gly Leu Asp Leu Asp Ala Glu
   195 200 205
   Ser Ala Glu Pro Phe Leu Val Phe His Asn Tyr Pro Ala Ala Asn Thr
   210 215 220
   Ala Arg Lys Trp Phe His Arg Phe Gln Ala Trp Tyr Met Tyr Leu Val
   225 230 235 240
   Leu Gly Ala Tyr Gly Val Ser Leu Val Tyr Asn Pro Leu Tyr Ile Phe
   245 250 255
   Arg Met Gln His Asn Asp Thr Ile Pro Glu Ser Val Thr Ala Met Arg
   260 265 270
   Glu Asn Gly Phe Leu Arg Arg Tyr Arg Thr Leu Ala Phe Val Met Arg
   275 280 285
   Ala Phe Phe Ile Phe Arg Thr Ala Phe Leu Pro Trp Tyr Leu Thr Gly
   290 295 300
   Thr Ser Leu Leu Ile Thr Ile Pro Leu Val Pro Thr Ala Thr Gly Ala
   305 310 315 320
   Phe Leu Thr Phe Phe Phe Ile Leu Ser His Asn Phe Asp Gly Ser Glu
   325 330 335
   Arg Ile Pro Asp Lys Asn Cys Lys Val Lys Ser Ser Glu Lys Asp Val
   340 345 350
   Glu Ala Asp Gln Ile Asp Trp Tyr Arg Ala Gln Val Glu Thr Ser Ser
   355 360 365
   Thr Tyr Gly Gly Pro Ile Ala Met Phe Phe Thr Gly Gly Leu Asn Phe
   370 375 380
   Gln Ile Glu His His Leu Phe Pro Arg Met Ser Ser Trp His Tyr Pro
   385 390 395 400
   Phe Val Gln Gln Ala Val Arg Glu Cys Cys Glu Arg His Gly Val Arg
   405 410 415
   Tyr Val Phe Tyr Pro Thr Ile Val Gly Asn Ile Ile Ser Thr Leu Lys
   420 425 430
   Tyr Met His Lys Val Gly Val Val His Cys Val Lys Asp Ala Gln Asp
   435 440 445
   Ser
<210> 67
   <211> 1350
   <212> DNA
   <213> Euglena gracilis
<220>
   <221> CDS
   <222> (1)..(1350)
   <223> synthetic delta-5 desaturase (codon-optimized for Yarrowia lipolytica)
<300>
   <302> DELTA-5 DESATURASE AND ITS USE IN MAKING POLYUNSATURATED FATTY ACIDS
<310> WO 2007/136671
   <311> 2007-05-16
   <312> 2007-11-29
   <313> (1)..(1350)
<300>
   <302> DELTA-5 DESATURASE AND ITS USE IN MAKING POLYUNSATURATED FATTY ACIDS
<310> US 2007-0292924-A1
   <311> 2007-05-15
   <312> 2007-12-20
   <313> (1)..(1350)
<400> 67
   atg gct ctc tcc ctt act acc gag cag ctg ctc gag cga ccc gac ctg        48
   Met Ala Leu Ser Leu Thr Thr Glu Gln Leu Leu Glu Arg Pro Asp Leu
   1               5                   10                  15
   gtt gcc atc gac ggc att ctc tac gat ctg gaa ggt ctt gcc aag gtc        96
   Val Ala Ile Asp Gly Ile Leu Tyr Asp Leu Glu Gly Leu Ala Lys Val
               20                  25                  30
   cat ccc gga ggc gac ttg atc ctc gct tct ggt gcc tcc gat gct tct       144
   His Pro Gly Gly Asp Leu Ile Leu Ala Ser Gly Ala Ser Asp Ala Ser
           35                  40                  45
   cct ctg ttc tac tcc atg cac cct tac gtc aag ccc gag aac tcg aag       192
   Pro Leu Phe Tyr Ser Met His Pro Tyr Val Lys Pro Glu Asn Ser Lys
       50                  55                  60
   ctg ctt caa cag ttc gtg cga ggc aag cac gac cga acc tcc aag gac       240
   Leu Leu Gln Gln Phe Val Arg Gly Lys His Asp Arg Thr Ser Lys Asp
   65                  70                  75                  80
   att gtc tac acc tac gac tct ccc ttt gca cag gac gtc aag cga act       288
   Ile Val Tyr Thr Tyr Asp Ser Pro Phe Ala Gln Asp Val Lys Arg Thr
                   85                  90                  95
   atg cga gag gtc atg aaa ggt cgg aac tgg tat gcc aca cct gga ttc       336
   Met Arg Glu Val Met Lys Gly Arg Asn Trp Tyr Ala Thr Pro Gly Phe
               100                 105                 110
   tgg ctg cga acc gtt ggc atc att gct gtc acc gcc ttt tgc gag tgg       384
   Trp Leu Arg Thr Val Gly Ile Ile Ala Val Thr Ala Phe Cys Glu Trp
           115                 120                 125
   cac tgg gct act acc gga atg gtg ctg tgg ggt ctc ttg act gga ttc       432
   His Trp Ala Thr Thr Gly Met Val Leu Trp Gly Leu Leu Thr Gly Phe
       130                 135                 140
   atg cac atg cag atc ggc ctg tcc att cag cac gat gcc tct cat ggt       480
   Met His Met Gln Ile Gly Leu Ser Ile Gln His Asp Ala Ser His Gly
   145                 150                 155                 160
   gcc atc agc aaa aag ccc tgg gtc aac gct ctc ttt gcc tac ggc atc       528
   Ala Ile Ser Lys Lys Pro Trp Val Asn Ala Leu Phe Ala Tyr Gly Ile
                   165                 170                 175
   gac gtc att gga tcg tcc aga tgg atc tgg ctg cag tct cac atc atg       576
   Asp Val Ile Gly Ser Ser Arg Trp Ile Trp Leu Gln Ser His Ile Met
               180                 185                 190
   cga cat cac acc tac acc aat cag cat ggt ctc gac ctg gat gcc gag       624
   Arg His His Thr Tyr Thr Asn Gln His Gly Leu Asp Leu Asp Ala Glu
           195                 200                 205
   tcc gca gaa cca ttc ctt gtg ttc cac aac tac cct gct gcc aac act       672
   Ser Ala Glu Pro Phe Leu Val Phe His Asn Tyr Pro Ala Ala Asn Thr
       210                 215                 220
   gct cga aag tgg ttt cac cga ttc cag gcc tgg tac atg tac ctc gtg       720
   Ala Arg Lys Trp Phe His Arg Phe Gln Ala Trp Tyr Met Tyr Leu Val
   225                 230                 235                 240
   ctt gga gcc tac ggc gtt tcg ctg gtg tac aac cct ctc tac atc ttc       768
   Leu Gly Ala Tyr Gly Val Ser Leu Val Tyr Asn Pro Leu Tyr Ile Phe
                   245                 250                 255
   cga atg cag cac aac gac acc att ccc gag tct gtc aca gcc atg cga        816
   Arg Met Gln His Asn Asp Thr Ile Pro Glu Ser Val Thr Ala Met Arg
               260                 265                 270
   gag aac ggc ttt ctg cga cgg tac cga acc ctt gca ttc gtt atg cga        864
   Glu Asn Gly Phe Leu Arg Arg Tyr Arg Thr Leu Ala Phe Val Met Arg
           275                 280                 285
   gct ttc ttc atc ttt cga acc gcc ttc ttg ccc tgg tat ctc act gga        912
   Ala Phe Phe Ile Phe Arg Thr Ala Phe Leu Pro Trp Tyr Leu Thr Gly
       290                 295                 300
   acc tcc ctg ctc atc acc att cct ctg gtg ccc act gct acc ggt gcc        960
   Thr Ser Leu Leu Ile Thr Ile Pro Leu Val Pro Thr Ala Thr Gly Ala
   305                 310                 315                 320
   ttc ctc acc ttc ttt ttc atc ttg tct cac aac ttc gat ggc tcg gag       1008
   Phe Leu Thr Phe Phe Phe Ile Leu Ser His Asn Phe Asp Gly Ser Glu
                   325                 330                 335
   cga atc ccc gac aag aac tgc aag gtc aag agc tcc gag aag gac gtt       1056
   Arg Ile Pro Asp Lys Asn Cys Lys Val Lys Ser Ser Glu Lys Asp Val
               340                 345                 350
   gaa gcc gat cag atc gac tgg tac aga gct cag gtg gag acc tct tcc       1104
   Glu Ala Asp Gln Ile Asp Trp Tyr Arg Ala Gln Val Glu Thr Ser Ser
           355                 360                 365
   acc tac ggt gga ccc att gcc atg ttc ttt act ggc ggt ctc aac ttc       1152
   Thr Tyr Gly Gly Pro Ile Ala Met Phe Phe Thr Gly Gly Leu Asn Phe
       370                 375                 380
   cag atc gag cat cac ctc ttt cct cga atg tcg tct tgg cac tat ccc       1200
   Gln Ile Glu His His Leu Phe Pro Arg Met Ser Ser Trp His Tyr Pro
   385                 390                 395                 400
   ttc gtg cag caa gct gtc cga gag tgt tgc gaa cga cac gga gtt cgg       1248
   Phe Val Gln Gln Ala Val Arg Glu Cys Cys Glu Arg His Gly Val Arg
                   405                 410                 415
   tac gtc ttc tac cct acc att gtg ggc aac atc att tcc acc ctc aag       1296
   Tyr Val Phe Tyr Pro Thr Ile Val Gly Asn Ile Ile Ser Thr Leu Lys
               420                 425                 430
   tac atg cac aaa gtc ggt gtg gtt cac tgt gtc aag gac gct cag gat       1344
   Tyr Met His Lys Val Gly Val Val His Cys Val Lys Asp Ala Gln Asp
           435                 440                 445
   tcc taa                                                               1350
   Ser
<210> 68
   <211> 449
   <212> PRT
   <213> Euglena gracilis
<400> 68
   Met Ala Leu Ser Leu Thr Thr Glu Gln Leu Leu Glu Arg Pro Asp Leu
   1               5                   10                  15
   Val Ala Ile Asp Gly Ile Leu Tyr Asp Leu Glu Gly Leu Ala Lys Val
               20                  25                  30
   His Pro Gly Gly Asp Leu Ile Leu Ala Ser Gly Ala Ser Asp Ala Ser
           35                  40                  45
   Pro Leu Phe Tyr Ser Met His Pro Tyr Val Lys Pro Glu Asn Ser Lys
       50                  55                  60
   Leu Leu Gln Gln Phe Val Arg Gly Lys His Asp Arg Thr Ser Lys Asp
   65                  70                  75                  80
   Ile Val Tyr Thr Tyr Asp Ser Pro Phe Ala Gln Asp Val Lys Arg Thr
                   85                  90                  95
   Met Arg Glu Val Met Lys Gly Arg Asn Trp Tyr Ala Thr Pro Gly Phe
               100                 105                 110
   Trp Leu Arg Thr Val Gly Ile Ile Ala Val Thr Ala Phe Cys Glu Trp
           115                 120                 125
   His Trp Ala Thr Thr Gly Met Val Leu Trp Gly Leu Leu Thr Gly Phe
       130                 135                 140
   Met His Met Gln Ile Gly Leu Ser Ile Gln His Asp Ala Ser His Gly
   145                 150                 155                 160
   Ala Ile Ser Lys Lys Pro Trp Val Asn Ala Leu Phe Ala Tyr Gly Ile
                   165                 170                 175
   Asp Val Ile Gly Ser Ser Arg Trp Ile Trp Leu Gln Ser His Ile Met
               180                 185                 190
   Arg His His Thr Tyr Thr Asn Gln His Gly Leu Asp Leu Asp Ala Glu
           195                 200                 205
   Ser Ala Glu Pro Phe Leu Val Phe His Asn Tyr Pro Ala Ala Asn Thr
       210                 215                 220
   Ala Arg Lys Trp Phe His Arg Phe Gln Ala Trp Tyr Met Tyr Leu Val
   225                 230                 235                 240
   Leu Gly Ala Tyr Gly Val Ser Leu Val Tyr Asn Pro Leu Tyr Ile Phe
                   245                 250                 255
   Arg Met Gln His Asn Asp Thr Ile Pro Glu Ser Val Thr Ala Met Arg
               260                 265                 270
   Glu Asn Gly Phe Leu Arg Arg Tyr Arg Thr Leu Ala Phe Val Met Arg
               275                 280                 285
   Ala Phe Phe Ile Phe Arg Thr Ala Phe Leu Pro Trp Tyr Leu Thr Gly
       290                 295                 300
   Thr Ser Leu Leu Ile Thr Ile Pro Leu Val Pro Thr Ala Thr Gly Ala
   305                 310                 315                 320
   Phe Leu Thr Phe Phe Phe Ile Leu Ser His Asn Phe Asp Gly Ser Glu
                   325                 330                 335
   Arg Ile Pro Asp Lys Asn Cys Lys Val Lys Ser Ser Glu Lys Asp Val
               340                 345                 350
   Glu Ala Asp Gln Ile Asp Trp Tyr Arg Ala Gln Val Glu Thr Ser Ser
           355                 360                 365
   Thr Tyr Gly Gly Pro Ile Ala Met Phe Phe Thr Gly Gly Leu Asn Phe
       370                 375                 380
   Gln Ile Glu His His Leu Phe Pro Arg Met Ser Ser Trp His Tyr Pro
   385                 390                 395                 400
   Phe Val Gln Gln Ala Val Arg Glu Cys Cys Glu Arg His Gly Val Arg
                   405                 410                 415
   Tyr Val Phe Tyr Pro Thr Ile Val Gly Asn Ile Ile Ser Thr Leu Lys
               420                 425                 430
   Tyr Met His Lys Val Gly Val Val His Cys Val Lys Asp Ala Gln Asp
           435                 440                 445
   Ser
<210> 69
   <211> 1350
   <212> DNA
   <213> Euglena gracilis
<220>
   <221> CDS
   <222> (1)..(1350)
   <223> mutant delta-5 desaturase
<400> 69
   atg gct ctc agt ctt acc aca gaa cag ctg tta gaa cgc cct gat ttg         48
   Met Ala Leu Ser Leu Thr Thr Glu Gln Leu Leu Glu Arg Pro Asp Leu
   1               5                   10                  15
   gtt gcg att gat ggc atc ctc tac gac ctt gaa ggg ctt gcc aaa gtt         96
   Val Ala Ile Asp Gly Ile Leu Tyr Asp Leu Glu Gly Leu Ala Lys Val
               20                  25                  30
   cat cca gga tcc gat ttg att ctc gct tct ggt gcc tct gat gcc tcc          144
   His Pro Gly Ser Asp Leu Ile Leu Ala Ser Gly Ala Ser Asp Ala Ser
           35                  40                  45
   cct ctc ttt tat tca atg cat cca tac gtc aaa ccg gag aac tcc aaa          192
   Pro Leu Phe Tyr Ser Met His Pro Tyr Val Lys Pro Glu Asn Ser Lys
       50                  55                  60
   ttg ctt caa cag ttc gtc cga ggg aag cat gac cgc acc tcg aag gac          240
   Leu Leu Gln Gln Phe Val Arg Gly Lys His Asp Arg Thr Ser Lys Asp
   65                  70                  75                  80
   att gtc tac acg tat gat tct ccc ttc gca caa gac gtt aag cgg aca          288
   Ile Val Tyr Thr Tyr Asp Ser Pro Phe Ala Gln Asp Val Lys Arg Thr
                   85                  90                  95
   atg cgc gag gtg atg aaa ggg agg aac tgg tac gca acc cct ggc ttc          336
   Met Arg Glu Val Met Lys Gly Arg Asn Trp Tyr Ala Thr Pro Gly Phe
               100                 105                 110
   tgg ctg cgc acc gtt ggg atc atc gcc gtg acg gcc ttt tgc gag tgg          384
   Trp Leu Arg Thr Val Gly Ile Ile Ala Val Thr Ala Phe Cys Glu Trp
           115                 120                 125
   cac tgg gct acc acg ggg atg gtg ctg tgg ggc ctg ttg act gga ttc          432
   His Trp Ala Thr Thr Gly Met Val Leu Trp Gly Leu Leu Thr Gly Phe
       130                 135                 140
   atg cac atg cag atc ggc tta tcc atc cag cat gat gcg tcc cac ggg          480
   Met His Met Gln Ile Gly Leu Ser Ile Gln His Asp Ala Ser His Gly
   145                 150                 155                 160
   gcc atc agc aag aag cct tgg gtc aac gcc ctc ttc gcc tac ggc att          528
   Ala Ile Ser Lys Lys Pro Trp Val Asn Ala Leu Phe Ala Tyr Gly Ile
                   165                 170                 175
   gac gtc atc gga tcg tcc cgg tgg att tgg ctg cag tcg cac atc atg          576
   Asp Val Ile Gly Ser Ser Arg Trp Ile Trp Leu Gln Ser His Ile Met
               180                 185                 190
   cgg cac cac acc tac acc aac cag cac ggc ctc gac ctg gat gcg gag          624
   Arg His His Thr Tyr Thr Asn Gln His Gly Leu Asp Leu Asp Ala Glu
           195                 200                 205
   tcg gca gag ccg ttc ctg gtg ttc cac aac tac ccc gcc gca aac acc          672
   Ser Ala Glu Pro Phe Leu Val Phe His Asn Tyr Pro Ala Ala Asn Thr
       210                 215                 220
   gcc cga aag tgg ttc cac cgc ttc cag gct tgg tac atg tac ctt gtg          720
   Ala Arg Lys Trp Phe His Arg Phe Gln Ala Trp Tyr Met Tyr Leu Val
   225                 230                 235                 240
   ctg ggg gca tac ggg gta tcg ctg gtg tac aac ccg ctc tac att ttc          768
   Leu Gly Ala Tyr Gly Val Ser Leu Val Tyr Asn Pro Leu Tyr Ile Phe
                   245                 250                 255
   cgg atg cag cac aat gac acc atc cca gag tct gtc acg gcc atg cgg          816
   Arg Met Gln His Asn Asp Thr Ile Pro Glu Ser Val Thr Ala Met Arg
               260                 265                 270
   gaa aat ggc ttt ctg cgg cgc tac cgc aca ctt gca ttc gtg atg cga          864
   Glu Asn Gly Phe Leu Arg Arg Tyr Arg Thr Leu Ala Phe Val Met Arg
           275                 280                 285
   gct ttc ttc atc ttc cgg acc gca ttc ttg ccc tgg tac ctc act ggg        912
   Ala Phe Phe Ile Phe Arg Thr Ala Phe Leu Pro Trp Tyr Leu Thr Gly
       290                 295                 300
   acc tca ttg ctg atc acc att cct ctg gtg ccc acc gca act ggt gcc        960
   Thr Ser Leu Leu Ile Thr Ile Pro Leu Val Pro Thr Ala Thr Gly Ala
   305                 310                 315                 320
   ttc ttg acg ttc ttc ttc att ttg tcc cac aat ttt gat ggc tcc gaa       1008
   Phe Leu Thr Phe Phe Phe Ile Leu Ser His Asn Phe Asp Gly Ser Glu
                   325                 330                 335
   cgg atc ccc gac aag aac tgc aag gtt aag cga tct gag aag gac gtt       1056
   Arg Ile Pro Asp Lys Asn Cys Lys Val Lys Arg Ser Glu Lys Asp Val
               340                 345                 350
   gag gct gac caa att gac tgg tat cgg gcg cag gtg gag acg tcc tcc       1104
   Glu Ala Asp Gln Ile Asp Trp Tyr Arg Ala Gln Val Glu Thr Ser Ser
           355                 360                 365
   aca tac ggt ggc ccc atc gcc atg ttc ttc act ggc ggt ctc aat ttc       1152
   Thr Tyr Gly Gly Pro Ile Ala Met Phe Phe Thr Gly Gly Leu Asn Phe
       370                 375                 380
   cag atc gag cac cac ctc ttt ccc cgg atg tcg tct tgg cac tac ccc       1200
   Gln Ile Glu His His Leu Phe Pro Arg Met Ser Ser Trp His Tyr Pro
   385                 390                 395                 400
   ttc gtc cag cag gcg gtc cgg gag tgt tgc gaa cga cat gga gtg cga       1248
   Phe Val Gln Gln Ala Val Arg Glu Cys Cys Glu Arg His Gly Val Arg
                   405                 410                 415
   tat gtt ttc tac cct acc atc gtc ggc aac atc atc tcc acc ctg aag       1296
   Tyr Val Phe Tyr Pro Thr Ile Val Gly Asn Ile Ile Ser Thr Leu Lys
               420                 425                 430
   tac atg cat aag gtg ggt gtc gtc cac tgc gtg aag gac gca cag gat       1344
   Tyr Met His Lys Val Gly Val Val His Cys Val Lys Asp Ala Gln Asp
           435                 440                 445
   tcc taa                                                               1350
   Ser
<210> 70
   <211> 449
   <212> PRT
   <213> Euglena gracilis
<400> 70
   Met Ala Leu Ser Leu Thr Thr Glu Gln Leu Leu Glu Arg Pro Asp Leu
   1               5                   10                  15
   Val Ala Ile Asp Gly Ile Leu Tyr Asp Leu Glu Gly Leu Ala Lys Val
               20                  25                  30
   His Pro Gly Ser Asp Leu Ile Leu Ala Ser Gly Ala Ser Asp Ala Ser
           35                  40                  45
   Pro Leu Phe Tyr Ser Met His Pro Tyr Val Lys Pro Glu Asn Ser Lys
       50                  55                  60
   Leu Leu Gln Gln Phe Val Arg Gly Lys His Asp Arg Thr Ser Lys Asp
   65                  70                  75                  80
   Ile Val Tyr Thr Tyr Asp Ser Pro Phe Ala Gln Asp Val Lys Arg Thr
                   85                  90                  95
   Met Arg Glu Val Met Lys Gly Arg Asn Trp Tyr Ala Thr Pro Gly Phe
               100                 105                 110
   Trp Leu Arg Thr Val Gly Ile Ile Ala Val Thr Ala Phe Cys Glu Trp
           115                 120                 125
   His Trp Ala Thr Thr Gly Met Val Leu Trp Gly Leu Leu Thr Gly Phe
       130                 135                 140
   Met His Met Gln Ile Gly Leu Ser Ile Gln His Asp Ala Ser His Gly
   145                 150                 155                 160
   Ala Ile Ser Lys Lys Pro Trp Val Asn Ala Leu Phe Ala Tyr Gly Ile
                   165                 170                 175
   Asp Val Ile Gly Ser Ser Arg Trp Ile Trp Leu Gln Ser His Ile Met
               180                 185                 190
   Arg His His Thr Tyr Thr Asn Gln His Gly Leu Asp Leu Asp Ala Glu
           195                 200                 205
   Ser Ala Glu Pro Phe Leu Val Phe His Asn Tyr Pro Ala Ala Asn Thr
       210                 215                 220
   Ala Arg Lys Trp Phe His Arg Phe Gln Ala Trp Tyr Met Tyr Leu Val
   225                 230                 235                 240
   Leu Gly Ala Tyr Gly Val Ser Leu Val Tyr Asn Pro Leu Tyr Ile Phe
                   245                 250                 255
   Arg Met Gln His Asn Asp Thr Ile Pro Glu Ser Val Thr Ala Met Arg
               260                 265                 270
   Glu Asn Gly Phe Leu Arg Arg Tyr Arg Thr Leu Ala Phe Val Met Arg
           275                 280                 285
   Ala Phe Phe Ile Phe Arg Thr Ala Phe Leu Pro Trp Tyr Leu Thr Gly
       290                 295                 300
   Thr Ser Leu Leu Ile Thr Ile Pro Leu Val Pro Thr Ala Thr Gly Ala
   305 310 315 320
   Phe Leu Thr Phe Phe Phe Ile Leu Ser His Asn Phe Asp Gly Ser Glu
   325 330 335
   Arg Ile Pro Asp Lys Asn Cys Lys Val Lys Arg Ser Glu Lys Asp Val
   340 345 350
   Glu Ala Asp Gln Ile Asp Trp Tyr Arg Ala Gln Val Glu Thr Ser Ser
   355 360 365
   Thr Tyr Gly Gly Pro Ile Ala Met Phe Phe Thr Gly Gly Leu Asn Phe
   370 375 380
   Gln Ile Glu His His Leu Phe Pro Arg Met Ser Ser Trp His Tyr Pro
   385 390 395 400
   Phe Val Gln Gln Ala Val Arg Glu Cys Cys Glu Arg His Gly Val Arg
   405 410 415
   Tyr Val Phe Tyr Pro Thr Ile Val Gly Asn Ile Ile Ser Thr Leu Lys
   420 425 430
   Tyr Met His Lys Val Gly Val Val His Cys Val Lys Asp Ala Gln Asp
   435 440 445
   Ser
<210> 71
   <211> 1350
   <212> DNA
   <213> Euglena gracilis
<220>
   <221> CDS
   <222> (1)..(1350)
   <223> synthetic mutant delta-5 desaturase (codon-optimized for Yarrowia lipolytica)
<400> 71
   atg gct ctc tcc ctt act acc gag cag ctg ctc gag cga ccc gac ctg        48
   Met Ala Leu Ser Leu Thr Thr Glu Gln Leu Leu Glu Arg Pro Asp Leu
   1               5                   10                  15
   gtt gcc atc gac ggc att ctc tac gat ctg gaa ggt ctt gcc aag gtc        96
   Val Ala Ile Asp Gly Ile Leu Tyr Asp Leu Glu Gly Leu Ala Lys Val
               20                  25                  30
   cat ccc gga tcc gac ttg atc ctc gct tct ggt gcc tcc gat gct tct       144
   His Pro Gly Ser Asp Leu Ile Leu Ala Ser Gly Ala Ser Asp Ala Ser
           35                  40                  45
   cct ctg ttc tac tcc atg cac cct tac gtc aag ccc gag aac tcg aag       192
   Pro Leu Phe Tyr Ser Met His Pro Tyr Val Lys Pro Glu Asn Ser Lys
       50                  55                  60
   ctg ctt caa cag ttc gtg cga ggc aag cac gac cga acc tcc aag gac      240
   Leu Leu Gln Gln Phe Val Arg Gly Lys His Asp Arg Thr Ser Lys Asp
   65                  70                  75                  80
   att gtc tac acc tac gac tct ccc ttt gca cag gac gtc aag cga act      288
   Ile Val Tyr Thr Tyr Asp Ser Pro Phe Ala Gln Asp Val Lys Arg Thr
                   85                  90                  95
   atg cga gag gtc atg aaa ggt cgg aac tgg tat gcc aca cct gga ttc      336
   Met Arg Glu Val Met Lys Gly Arg Asn Trp Tyr Ala Thr Pro Gly Phe
               100                 105                 110
   tgg ctg cga acc gtt ggc atc att gct gtc acc gcc ttt tgc gag tgg      384
   Trp Leu Arg Thr Val Gly Ile Ile Ala Val Thr Ala Phe Cys Glu Trp
           115                 120                 125
   cac tgg gct act acc gga atg gtg ctg tgg ggt ctc ttg act gga ttc      432
   His Trp Ala Thr Thr Gly Met Val Leu Trp Gly Leu Leu Thr Gly Phe
       130                 135                 140
   atg cac atg cag atc ggc ctg tcc att cag cac gat gcc tct cat ggt      480
   Met His Met Gln Ile Gly Leu Ser Ile Gln His Asp Ala Ser His Gly
   145                 150                 155                 160
   gcc atc agc aaa aag ccc tgg gtc aac gct ctc ttt gcc tac ggc atc      528
   Ala Ile Ser Lys Lys Pro Trp Val Asn Ala Leu Phe Ala Tyr Gly Ile
                   165                 170                 175
   gac gtc att gga tcg tcc aga tgg atc tgg ctg cag tct cac atc atg      576
   Asp Val Ile Gly Ser Ser Arg Trp Ile Trp Leu Gln Ser His Ile Met
               180                 185                 190
   cga cat cac acc tac acc aat cag cat ggt ctc gac ctg gat gcc gag      624
   Arg His His Thr Tyr Thr Asn Gln His Gly Leu Asp Leu Asp Ala Glu
           195                 200                 205
   tcc gca gaa cca ttc ctt gtg ttc cac aac tac cct gct gcc aac act      672
   Ser Ala Glu Pro Phe Leu Val Phe His Asn Tyr Pro Ala Ala Asn Thr
       210                 215                 220
   gct cga aag tgg ttt cac cga ttc cag gcc tgg tac atg tac ctc gtg      720
   Ala Arg Lys Trp Phe His Arg Phe Gln Ala Trp Tyr Met Tyr Leu Val
   225                 230                 235                 240
   ctt gga gcc tac ggc gtt tcg ctg gtg tac aac cct ctc tac atc ttc      768
   Leu Gly Ala Tyr Gly Val Ser Leu Val Tyr Asn Pro Leu Tyr Ile Phe
                   245                 250                 255
   cga atg cag cac aac gac acc att ccc gag tct gtc aca gcc atg cga      816
   Arg Met Gln His Asn Asp Thr Ile Pro Glu Ser Val Thr Ala Met Arg
               260                 265                 270
   gag aac ggc ttt ctg cga cgg tac cga acc ctt gca ttc gtt atg cga      864
   Glu Asn Gly Phe Leu Arg Arg Tyr Arg Thr Leu Ala Phe Val Met Arg
           275                 280                 285
   gct ttc ttc atc ttt cga acc gcc ttc ttg ccc tgg tat ctc act gga      912
   Ala Phe Phe Ile Phe Arg Thr Ala Phe Leu Pro Trp Tyr Leu Thr Gly
       290                 295                 300
   acc tcc ctg ctc atc acc att cct ctg gtg ccc act gct acc ggt gcc      960
   Thr Ser Leu Leu Ile Thr Ile Pro Leu Val Pro Thr Ala Thr Gly Ala
   305                 310                 315                 320
   ttc ctc acc ttc ttt ttc atc ttg tct cac aac ttc gat ggc tcg gag       1008
   Phe Leu Thr Phe Phe Phe Ile Leu Ser His Asn Phe Asp Gly Ser Glu
                       325                 330                 335
   cga atc ccc gac aag aac tgc aag gtc aag agc tcc gag aag gac gtt       1056
   Arg Ile Pro Asp Lys Asn Cys Lys Val Lys Ser Ser Glu Lys Asp Val
                   340                 345                 350
   gaa gcc gat cag atc gac tgg tac aga gct cag gtg gag acc tct tcc       1104
   Glu Ala Asp Gln Ile Asp Trp Tyr Arg Ala Gln Val Glu Thr Ser Ser
               355                 360                 365
   acc tac ggt gga ccc att gcc atg ttc ttt act ggc ggt ctc aac ttc       1152
   Thr Tyr Gly Gly Pro Ile Ala Met Phe Phe Thr Gly Gly Leu Asn Phe
           370                 375                 380
   cag atc gag cat cac ctc ttt cct cga atg tcg tct tgg cac tat ccc       1200
   Gln Ile Glu His His Leu Phe Pro Arg Met Ser Ser Trp His Tyr Pro
   385                 390                 395                 400
   ttc gtg cag caa gct gtc cga gag tgt tgc gaa cga cac gga gtt cgg       1248
   Phe Val Gln Gln Ala Val Arg Glu Cys Cys Glu Arg His Gly Val Arg
                       405                 410                 415
   tac gtc ttc tac cct acc att gtg ggc aac atc att tcc acc ctc aag       1296
   Tyr Val Phe Tyr Pro Thr Ile Val Gly Asn Ile Ile Ser Thr Leu Lys
                   420                 425                 430
   tac atg cac aaa gtc ggt gtg gtt cac tgt gtc aag gac gct cag gat       1344
   Tyr Met His Lys Val Gly Val Val His Cys Val Lys Asp Ala Gln Asp
               435                 440                 445
   tcc taa                                                               1350
   Ser
<210> 72
   <211> 449
   <212> PRT
   <213> Euglena gracilis
<400> 72
   Met Ala Leu Ser Leu Thr Thr Glu Gln Leu Leu Glu Arg Pro Asp Leu
   1               5                   10                  15
   Val Ala Ile Asp Gly Ile Leu Tyr Asp Leu Glu Gly Leu Ala Lys Val
               20                  25                  30
   His Pro Gly Ser Asp Leu Ile Leu Ala Ser Gly Ala Ser Asp Ala Ser
           35                  40                  45
   Pro Leu Phe Tyr Ser Met His Pro Tyr Val Lys Pro Glu Asn Ser Lys
       50                  55                  60
   Leu Leu Gln Gln Phe Val Arg Gly Lys His Asp Arg Thr Ser Lys Asp
   65                  70                  75                  80
   Ile Val Tyr Thr Tyr Asp Ser Pro Phe Ala Gln Asp Val Lys Arg Thr
                   85                  90                  95
   Met Arg Glu Val Met Lys Gly Arg Asn Trp Tyr Ala Thr Pro Gly Phe
               100                 105                 110
   Trp Leu Arg Thr Val Gly Ile Ile Ala Val Thr Ala Phe Cys Glu Trp
           115                 120                 125
   His Trp Ala Thr Thr Gly Met Val Leu Trp Gly Leu Leu Thr Gly Phe
       130                 135                 140
   Met His Met Gln Ile Gly Leu Ser Ile Gln His Asp Ala Ser His Gly
   145                 150                 155                 160
   Ala Ile Ser Lys Lys Pro Trp Val Asn Ala Leu Phe Ala Tyr Gly Ile
                   165                 170                 175
   Asp Val Ile Gly Ser Ser Arg Trp Ile Trp Leu Gln Ser His Ile Met
               180                 185                 190
   Arg His His Thr Tyr Thr Asn Gln His Gly Leu Asp Leu Asp Ala Glu
           195                 200                 205
   Ser Ala Glu Pro Phe Leu Val Phe His Asn Tyr Pro Ala Ala Asn Thr
       210                 215                 220
   Ala Arg Lys Trp Phe His Arg Phe Gln Ala Trp Tyr Met Tyr Leu Val
   225                 230                 235                 240
   Leu Gly Ala Tyr Gly Val Ser Leu Val Tyr Asn Pro Leu Tyr Ile Phe
                   245                 250                 255
   Arg Met Gln His Asn Asp Thr Ile Pro Glu Ser Val Thr Ala Met Arg
               260                 265                 270
   Glu Asn Gly Phe Leu Arg Arg Tyr Arg Thr Leu Ala Phe Val Met Arg
           275                 280                 285
   Ala Phe Phe Ile Phe Arg Thr Ala Phe Leu Pro Trp Tyr Leu Thr Gly
       290                 295                 300
   Thr Ser Leu Leu Ile Thr Ile Pro Leu Val Pro Thr Ala Thr Gly Ala
   305                 310                 315                 320
   Phe Leu Thr Phe Phe Phe Ile Leu Ser His Asn Phe Asp Gly Ser Glu
                   325                 330                 335
   Arg Ile Pro Asp Lys Asn Cys Lys Val Lys Ser Ser Glu Lys Asp Val
   340 345 350
   Glu Ala Asp Gln Ile Asp Trp Tyr Arg Ala Gln Val Glu Thr Ser Ser
   355 360 365
   Thr Tyr Gly Gly Pro Ile Ala Met Phe Phe Thr Gly Gly Leu Asn Phe
   370 375 380
   Gln Ile Glu His His Leu Phe Pro Arg Met Ser Ser Trp His Tyr Pro
   385 390 395 400
   Phe Val Gln Gln Ala Val Arg Glu Cys Cys Glu Arg His Gly Val Arg
   405 410 415
   Tyr Val Phe Tyr Pro Thr Ile Val Gly Asn Ile Ile Ser Thr Leu Lys
   420 425 430
   Tyr Met His Lys Val Gly Val Val His Cys Val Lys Asp Ala Gln Asp
   435 440 445
   Ser
<210> 73
   <211> 1392
   <212> DNA
   <213> Peridinium sp. CCMP626
<220>
   <221> CDS
   <222> (1)..(1392)
   <223> delta-5 desaturase
<300>
   <302> DELTA-5 DESATURASE AND ITS USE IN MAKING POLYUNSATURATED FATTY ACIDS
<310> WO 2007/136646
   <311> 2007-05-16
   <312> 2007-11-29
   <313> (1)..(1392)
<300>
   <302> DELTA-5 DESATURASE AND ITS USE IN MAKING POLYUNSATURATED FATTY ACIDS
<310> US 2007-0271632-A1
   <311> 2007-05-15
   <312> 2007-11-22
   <313> (1)..(1392)
<400> 73
   atg gct cca gat gcg gac aag ttg aga cag cgc aag gcg caa tcg att        48
   Met Ala Pro Asp Ala Asp Lys Leu Arg Gln Arg Lys Ala Gln Ser Ile
   1               5                   10                  15
   caa gac acg gct gat tcg caa gct acc gaa ctc aag att ggc acc ctg        96
   Gln Asp Thr Ala Asp Ser Gln Ala Thr Glu Leu Lys Ile Gly Thr Leu
               20                  25                  30
   aag ggc ttg cag ggg aca gaa atc gtc att gat gga gac att tac gat      144
   Lys Gly Leu Gln Gly Thr Glu Ile Val Ile Asp Gly Asp Ile Tyr Asp
           35                  40                  45
   ata aaa gac ttt gat cac ccc ggt ggt gaa tcc atc atg act ttt ggg      192
   Ile Lys Asp Phe Asp His Pro Gly Gly Glu Ser Ile Met Thr Phe Gly
       50                  55                  60
   gga aac gat gtc acc gcc acg tac aag atg atc cac ccc tac cac tct      240
   Gly Asn Asp Val Thr Ala Thr Tyr Lys Met Ile His Pro Tyr His Ser
   65                  70                  75                  80
   aag cac cat ttg gag aag atg aag aaa gtg gga cga gtt ccg gac tac      288
   Lys His His Leu Glu Lys Met Lys Lys Val Gly Arg Val Pro Asp Tyr
                   85                  90                  95
   acc tcg gaa tac aag ttt gat act ccc ttt gag cgt gaa atc aag caa      336
   Thr Ser Glu Tyr Lys Phe Asp Thr Pro Phe Glu Arg Glu Ile Lys Gln
               100                 105                 110
   gag gtc ttc aag att gtg cga cga ggc cgc gag ttt gga aca cct gga      384
   Glu Val Phe Lys Ile Val Arg Arg Gly Arg Glu Phe Gly Thr Pro Gly
           115                 120                 125
   tac ttc ttc cgg gct ttc tgc tac att gga ctt ttc ttt tac ttg cag      432
   Tyr Phe Phe Arg Ala Phe Cys Tyr Ile Gly Leu Phe Phe Tyr Leu Gln
       130                 135                 140
   tat ttg tgg gtc acg act ccc act acc ttt gcc ttg gcg atc ttc tat      480
   Tyr Leu Trp Val Thr Thr Pro Thr Thr Phe Ala Leu Ala Ile Phe Tyr
   145                 150                 155                 160
   ggt gtt tcg caa gct ttc att ggt ttg aac gta caa cat gat gcc aac      528
   Gly Val Ser Gln Ala Phe Ile Gly Leu Asn Val Gln His Asp Ala Asn
                   165                 170                 175
   cac gga gct gcc tcc aag aag cct tgg atc aat aac ttg cta gga ttg      576
   His Gly Ala Ala Ser Lys Lys Pro Trp Ile Asn Asn Leu Leu Gly Leu
               180                 185                 190
   ggg gct gac ttt atc gga ggt tcc aaa tgg ttg tgg atg aac cag cac      624
   Gly Ala Asp Phe Ile Gly Gly Ser Lys Trp Leu Trp Met Asn Gln His
           195                 200                 205
   tgg acg cac cac aca tac acc aac cac cat gag aag gat ccc gat gcc      672
   Trp Thr His His Thr Tyr Thr Asn His His Glu Lys Asp Pro Asp Ala
       210                 215                 220
   ttg ggc gct gaa cca atg ttg ttg ttc aat gat tat ccc ttg ggt cac      720
   Leu Gly Ala Glu Pro Met Leu Leu Phe Asn Asp Tyr Pro Leu Gly His
   225                 230                 235                 240
   cca aag cgt act ttg att cac cac ttc cag gcc ttc tat tac ctt ttc      768
   Pro Lys Arg Thr Leu Ile His His Phe Gln Ala Phe Tyr Tyr Leu Phe
                   245                 250                 255
   gtc ttg gcc gga tac tgg gtc tct tcg gtc ttc aac cct caa att ttg      816
   Val Leu Ala Gly Tyr Trp Val Ser Ser Val Phe Asn Pro Gln Ile Leu
               260                 265                 270
   gac ttg caa cac cgc ggt gct caa gcg gtt gga atg aaa atg gag aac      864
   Asp Leu Gln His Arg Gly Ala Gln Ala Val Gly Met Lys Met Glu Asn
           275                 280                 285
   gat tac att gcc aaa agc cga aag tat gcc atc ttc ttg cgt ctc ttg        912
   Asp Tyr Ile Ala Lys Ser Arg Lys Tyr Ala Ile Phe Leu Arg Leu Leu
       290                 295                 300
   tat att tac acc aac att gtc gct ccg atc caa aac caa ggc ttc tcg        960
   Tyr Ile Tyr Thr Asn Ile Val Ala Pro Ile Gln Asn Gln Gly Phe Ser
   305                 310                 315                 320
   ttg acc gtg gtc gcc cac att ttg acc atg ggc gtc gct tcc agt ttg       1008
   Leu Thr Val Val Ala His Ile Leu Thr Met Gly Val Ala Ser Ser Leu
                   325                 330                 335
   act ttg gcg act ctt ttt gcc ttg tcg cac aat ttt gaa aac gcg gat       1056
   Thr Leu Ala Thr Leu Phe Ala Leu Ser His Asn Phe Glu Asn Ala Asp
               340                 345                 350
   cgc gat ccc act tac gag gcc cgc aag gga gga gag cct gtt tgt tgg       1104
   Arg Asp Pro Thr Tyr Glu Ala Arg Lys Gly Gly Glu Pro Val Cys Trp
           355                 360                 365
   ttc aag tcg caa gtc gaa acc tcg tca act tac gga ggt ttc atc tcg       1152
   Phe Lys Ser Gln Val Glu Thr Ser Ser Thr Tyr Gly Gly Phe Ile Ser
       370                 375                 380
   ggt tgc ttg acg ggc gga ctc aac ttc caa gtg gaa cac cac ttg ttc       1200
   Gly Cys Leu Thr Gly Gly Leu Asn Phe Gln Val Glu His His Leu Phe
   385                 390                 395                 400
   cct cgt atg agt tcg gcc tgg tac ccc tac att gcc cct act gtt cga       1248
   Pro Arg Met Ser Ser Ala Trp Tyr Pro Tyr Ile Ala Pro Thr Val Arg
                   405                 410                 415
   gag gtt tgc aaa aag cac gga gtc aag tac gca tac tat ccc tgg gtc       1296
   Glu Val Cys Lys Lys His Gly Val Lys Tyr Ala Tyr Tyr Pro Trp Val
               420                 425                 430
   tgg caa aac ttg att tca act gtc aag tat ctg cat caa agc gga act       1344
   Trp Gln Asn Leu Ile Ser Thr Val Lys Tyr Leu His Gln Ser Gly Thr
           435                 440                 445
   gga tcc aac tgg aag aat ggc gcc aac ccc tac tcg gga aaa ttg taa       1392
   Gly Ser Asn Trp Lys Asn Gly Ala Asn Pro Tyr Ser Gly Lys Leu
       450                 455                 460
<210> 74
   <211> 463
   <212> PRT
   <213> Peridinium sp. CCMP626
<400> 74
   Met Ala Pro Asp Ala Asp Lys Leu Arg Gln Arg Lys Ala Gln Ser Ile
   1               5                   10                  15
   Gln Asp Thr Ala Asp Ser Gln Ala Thr Glu Leu Lys Ile Gly Thr Leu
               20                  25                  30
   Lys Gly Leu Gln Gly Thr Glu Ile Val Ile Asp Gly Asp Ile Tyr Asp
           35                  40                  45
   Ile Lys Asp Phe Asp His Pro Gly Gly Glu Ser Ile Met Thr Phe Gly
       50                  55                  60
   Gly Asn Asp Val Thr Ala Thr Tyr Lys Met Ile His Pro Tyr His Ser
   65                  70                  75                  80
   Lys His His Leu Glu Lys Met Lys Lys Val Gly Arg Val Pro Asp Tyr
                   85                  90                  95
   Thr Ser Glu Tyr Lys Phe Asp Thr Pro Phe Glu Arg Glu Ile Lys Gln
               100                 105                 110
   Glu Val Phe Lys Ile Val Arg Arg Gly Arg Glu Phe Gly Thr Pro Gly
           115                 120                 125
   Tyr Phe Phe Arg Ala Phe Cys Tyr Ile Gly Leu Phe Phe Tyr Leu Gln
       130                 135                 140
   Tyr Leu Trp Val Thr Thr Pro Thr Thr Phe Ala Leu Ala Ile Phe Tyr
   145                 150                 155                 160
   Gly Val Ser Gln Ala Phe Ile Gly Leu Asn Val Gln His Asp Ala Asn
                   165                 170                 175
   His Gly Ala Ala Ser Lys Lys Pro Trp Ile Asn Asn Leu Leu Gly Leu
               180                 185                 190
   Gly Ala Asp Phe Ile Gly Gly Ser Lys Trp Leu Trp Met Asn Gln His
           195                 200                 205
   Trp Thr His His Thr Tyr Thr Asn His His Glu Lys Asp Pro Asp Ala
       210                 215                 220
   Leu Gly Ala Glu Pro Met Leu Leu Phe Asn Asp Tyr Pro Leu Gly His
   225                 230                 235                 240
   Pro Lys Arg Thr Leu Ile His His Phe Gln Ala Phe Tyr Tyr Leu Phe
                   245                 250                 255
   Val Leu Ala Gly Tyr Trp Val Ser Ser Val Phe Asn Pro Gln Ile Leu
               260                 265                 270
   Asp Leu Gln His Arg Gly Ala Gln Ala Val Gly Met Lys Met Glu Asn
           275                 280                 285
   Asp Tyr Ile Ala Lys Ser Arg Lys Tyr Ala Ile Phe Leu Arg Leu Leu
       290                 295                 300
   Tyr Ile Tyr Thr Asn Ile Val Ala Pro Ile Gln Asn Gln Gly Phe Ser
   305 310 315 320
   Leu Thr Val Val Ala His Ile Leu Thr Met Gly Val Ala Ser Ser Leu
   325 330 335
   Thr Leu Ala Thr Leu Phe Ala Leu Ser His Asn Phe Glu Asn Ala Asp
   340 345 350
   Arg Asp Pro Thr Tyr Glu Ala Arg Lys Gly Gly Glu Pro Val Cys Trp
   355 360 365
   Phe Lys Ser Gln Val Glu Thr Ser Ser Thr Tyr Gly Gly Phe Ile Ser
   370 375 380
   Gly Cys Leu Thr Gly Gly Leu Asn Phe Gln Val Glu His His Leu Phe
   385 390 395 400
   Pro Arg Met Ser Ser Ala Trp Tyr Pro Tyr Ile Ala Pro Thr Val Arg
   405 410 415
   Glu Val Cys Lys Lys His Gly Val Lys Tyr Ala Tyr Tyr Pro Trp Val
   420 425 430
   Trp Gln Asn Leu Ile Ser Thr Val Lys Tyr Leu His Gln Ser Gly Thr
   435 440 445
   Gly Ser Asn Trp Lys Asn Gly Ala Asn Pro Tyr Ser Gly Lys Leu
   450 455 460
<210> 75
   <211> 1392
   <212> DNA
   <213> Peridinium sp. CCMP626
<220>
   <221> CDS
   <222> (1)..(1392)
   <223> synthetic delta-5 desaturase (codon-optimized for Yarrowia lipolytica)
<300>
   <302> DELTA-5 DESATURASE AND ITS USE IN MAKING POLYUNSATURATED FATTY ACIDS
<310> WO 2007/136646
   <311> 2007-05-16
   <312> 2007-11-29
   <313> (1)..(1392)
<300>
   <302> DELTA-5 DESATURASE AND ITS USE IN MAKING POLYUNSATURATED FATTY ACIDS
<310> US 2007-0271632-A1
   <311> 2007-05-15
   <312> 2007-11-22
   <313> (1)..(1392)
<400> 75
   atg gct ccc gac gcc gac aag ctg cga cag cga aag gct cag tcc atc         48
   Met Ala Pro Asp Ala Asp Lys Leu Arg Gln Arg Lys Ala Gln Ser Ile
   1               5                   10                  15
   cag gac act gcc gat tct cag gct acc gag ctc aag att ggc acc ctg         96
   Gln Asp Thr Ala Asp Ser Gln Ala Thr Glu Leu Lys Ile Gly Thr Leu
               20                  25                  30
   aag ggt ctc caa ggc acc gag atc gtc att gat ggc gac atc tac gac        144
   Lys Gly Leu Gln Gly Thr Glu Ile Val Ile Asp Gly Asp Ile Tyr Asp
           35                  40                  45
   atc aaa gac ttc gat cac cct gga ggc gaa tcc atc atg acc ttt ggt        192
   Ile Lys Asp Phe Asp His Pro Gly Gly Glu Ser Ile Met Thr Phe Gly
       50                  55                  60
   ggc aac gac gtt act gcc acc tac aag atg att cat ccc tac cac tcg        240
   Gly Asn Asp Val Thr Ala Thr Tyr Lys Met Ile His Pro Tyr His Ser
   65                  70                  75                  80
   aag cat cac ctg gag aag atg aaa aag gtc ggt cga gtg ccc gac tac        288
   Lys His His Leu Glu Lys Met Lys Lys Val Gly Arg Val Pro Asp Tyr
                   85                  90                  95
   acc tcc gag tac aag ttc gat act ccc ttc gaa cga gag atc aaa cag        336
   Thr Ser Glu Tyr Lys Phe Asp Thr Pro Phe Glu Arg Glu Ile Lys Gln
               100                 105                 110
   gag gtc ttc aag att gtg cga aga ggt cga gag ttt gga aca cct ggc        384
   Glu Val Phe Lys Ile Val Arg Arg Gly Arg Glu Phe Gly Thr Pro Gly
           115                 120                 125
   tac ttc ttt cga gcc ttc tgc tac atc ggt ctc ttc ttt tac ctg cag        432
   Tyr Phe Phe Arg Ala Phe Cys Tyr Ile Gly Leu Phe Phe Tyr Leu Gln
       130                 135                 140
   tat ctc tgg gtt acc act cct acc act ttc gcc ctt gct atc ttc tac        480
   Tyr Leu Trp Val Thr Thr Pro Thr Thr Phe Ala Leu Ala Ile Phe Tyr
   145                 150                 155                 160
   ggt gtg tct cag gcc ttc att ggc ctg aac gtc cag cac gac gcc aac        528
   Gly Val Ser Gln Ala Phe Ile Gly Leu Asn Val Gln His Asp Ala Asn
                   165                 170                 175
   cac gga gct gcc tcc aaa aag ccc tgg atc aac aat ttg ctc ggc ctg        576
   His Gly Ala Ala Ser Lys Lys Pro Trp Ile Asn Asn Leu Leu Gly Leu
               180                 185                 190
   ggt gcc gac ttt atc gga ggc tcc aag tgg ctc tgg atg aac cag cac        624
   Gly Ala Asp Phe Ile Gly Gly Ser Lys Trp Leu Trp Met Asn Gln His
           195                 200                 205
   tgg acc cat cac act tac acc aac cat cac gag aag gat ccc gac gcc        672
   Trp Thr His His Thr Tyr Thr Asn His His Glu Lys Asp Pro Asp Ala
       210                 215                 220
   ctg ggt gca gag cct atg ctg ctc ttc aac gac tat ccc ttg ggt cac        720
   Leu Gly Ala Glu Pro Met Leu Leu Phe Asn Asp Tyr Pro Leu Gly His
   225                 230                 235                 240
   ccc aag cga acc ctc att cat cac ttc caa gcc ttc tac tat ctg ttt        768
   Pro Lys Arg Thr Leu Ile His His Phe Gln Ala Phe Tyr Tyr Leu Phe
                   245                 250                 255
   gtc ctt gct ggc tac tgg gtg tct tcg gtg ttc aac cct cag atc ctg        816
   Val Leu Ala Gly Tyr Trp Val Ser Ser Val Phe Asn Pro Gln Ile Leu
               260                 265                 270
   gac ctc cag cac cga ggt gcc cag gct gtc ggc atg aag atg gag aac        864
   Asp Leu Gln His Arg Gly Ala Gln Ala Val Gly Met Lys Met Glu Asn
           275                 280                 285
   gac tac att gcc aag tct cga aag tac gct atc ttc ctg cga ctc ctg        912
   Asp Tyr Ile Ala Lys Ser Arg Lys Tyr Ala Ile Phe Leu Arg Leu Leu
       290                 295                 300
   tac atc tac acc aac att gtg gct ccc atc cag aac caa ggc ttt tcg        960
   Tyr Ile Tyr Thr Asn Ile Val Ala Pro Ile Gln Asn Gln Gly Phe Ser
   305                 310                 315                 320
   ctc acc gtc gtt gct cac att ctt act atg ggt gtc gcc tcc agc ctg       1008
   Leu Thr Val Val Ala His Ile Leu Thr Met Gly Val Ala Ser Ser Leu
                   325                 330                 335
   acc ctc gct act ctg ttc gcc ctc tcc cac aac ttc gag aac gca gat       1056
   Thr Leu Ala Thr Leu Phe Ala Leu Ser His Asn Phe Glu Asn Ala Asp
               340                 345                 350
   cgg gat ccc acc tac gag gct cga aag gga ggc gag cct gtc tgt tgg       1104
   Arg Asp Pro Thr Tyr Glu Ala Arg Lys Gly Gly Glu Pro Val Cys Trp
           355                 360                 365
   ttc aag tcg cag gtg gaa acc tcc tct act tac ggt ggc ttc att tcc       1152
   Phe Lys Ser Gln Val Glu Thr Ser Ser Thr Tyr Gly Gly Phe Ile Ser
       370                 375                 380
   ggt tgc ctt aca ggc gga ctc aac ttt cag gtc gag cat cac ctg ttt       1200
   Gly Cys Leu Thr Gly Gly Leu Asn Phe Gln Val Glu His His Leu Phe
   385                 390                 395                 400
   cct cga atg tcc tct gcc tgg tac ccc tac atc gct cct acc gtt cga       1248
   Pro Arg Met Ser Ser Ala Trp Tyr Pro Tyr Ile Ala Pro Thr Val Arg
                   405                 410                 415
   gag gtc tgc aaa aag cac ggc gtc aag tac gcc tac tat ccc tgg gtg       1296
   Glu Val Cys Lys Lys His Gly Val Lys Tyr Ala Tyr Tyr Pro Trp Val
               420                 425                 430
   tgg cag aac ctc atc tcg acc gtc aag tac ctg cat cag tcc gga act       1344
   Trp Gln Asn Leu Ile Ser Thr Val Lys Tyr Leu His Gln Ser Gly Thr
           435                 440                 445
   ggc tcg aac tgg aag aac ggt gcc aat ccc tac tct ggc aag ctg taa       1392
   Gly Ser Asn Trp Lys Asn Gly Ala Asn Pro Tyr Ser Gly Lys Leu
       450                 455                 460
<210> 76
   <211> 463
   <212> PRT
   <213> Peridinium sp. CCMP626
<400> 76
   Met Ala Pro Asp Ala Asp Lys Leu Arg Gln Arg Lys Ala Gln Ser Ile
   1               5                   10                  15
   Gln Asp Thr Ala Asp Ser Gln Ala Thr Glu Leu Lys Ile Gly Thr Leu
               20                  25                  30
   Lys Gly Leu Gln Gly Thr Glu Ile Val Ile Asp Gly Asp Ile Tyr Asp
           35                  40                  45
   Ile Lys Asp Phe Asp His Pro Gly Gly Glu Ser Ile Met Thr Phe Gly
       50                  55                  60
   Gly Asn Asp Val Thr Ala Thr Tyr Lys Met Ile His Pro Tyr His Ser
   65                  70                  75                  80
   Lys His His Leu Glu Lys Met Lys Lys Val Gly Arg Val Pro Asp Tyr
                   85                  90                  95
   Thr Ser Glu Tyr Lys Phe Asp Thr Pro Phe Glu Arg Glu Ile Lys Gln
               100                 105                 110
   Glu Val Phe Lys Ile Val Arg Arg Gly Arg Glu Phe Gly Thr Pro Gly
           115                 120                 125
   Tyr Phe Phe Arg Ala Phe Cys Tyr Ile Gly Leu Phe Phe Tyr Leu Gln
       130                 135                 140
   Tyr Leu Trp Val Thr Thr Pro Thr Thr Phe Ala Leu Ala Ile Phe Tyr
   145                 150                 155                 160
   Gly Val Ser Gln Ala Phe Ile Gly Leu Asn Val Gln His Asp Ala Asn
                   165                 170                 175
   His Gly Ala Ala Ser Lys Lys Pro Trp Ile Asn Asn Leu Leu Gly Leu
               180                 185                 190
   Gly Ala Asp Phe Ile Gly Gly Ser Lys Trp Leu Trp Met Asn Gln His
           195                 200                 205
   Trp Thr His His Thr Tyr Thr Asn His His Glu Lys Asp Pro Asp Ala
       210                 215                 220
   Leu Gly Ala Glu Pro Met Leu Leu Phe Asn Asp Tyr Pro Leu Gly His
   225                 230                 235                 240
   Pro Lys Arg Thr Leu Ile His His Phe Gln Ala Phe Tyr Tyr Leu Phe
                   245                 250                 255
   Val Leu Ala Gly Tyr Trp Val Ser Ser Val Phe Asn Pro Gln Ile Leu
               260                 265                 270
   Asp Leu Gln His Arg Gly Ala Gln Ala Val Gly Met Lys Met Glu Asn
   275 280 285
   Asp Tyr Ile Ala Lys Ser Arg Lys Tyr Ala Ile Phe Leu Arg Leu Leu
   290 295 300
   Tyr Ile Tyr Thr Asn Ile Val Ala Pro Ile Gln Asn Gln Gly Phe Ser
   305 310 315 320
   Leu Thr Val Val Ala His Ile Leu Thr Met Gly Val Ala Ser Ser Leu
   325 330 335
   Thr Leu Ala Thr Leu Phe Ala Leu Ser His Asn Phe Glu Asn Ala Asp
   340 345 350
   Arg Asp Pro Thr Tyr Glu Ala Arg Lys Gly Gly Glu Pro Val Cys Trp
   355 360 365
   Phe Lys Ser Gln Val Glu Thr Ser Ser Thr Tyr Gly Gly Phe Ile Ser
   370 375 380
   Gly Cys Leu Thr Gly Gly Leu Asn Phe Gln Val Glu His His Leu Phe
   385 390 395 400
   Pro Arg Met Ser Ser Ala Trp Tyr Pro Tyr Ile Ala Pro Thr Val Arg
   405 410 415
   Glu Val Cys Lys Lys His Gly Val Lys Tyr Ala Tyr Tyr Pro Trp Val
   420 425 430
   Trp Gln Asn Leu Ile Ser Thr Val Lys Tyr Leu His Gln Ser Gly Thr
   435 440 445
   Gly Ser Asn Trp Lys Asn Gly Ala Asn Pro Tyr Ser Gly Lys Leu
   450 455 460
<210> 77
   <211> 1362
   <212> DNA
   <213> Euglena anabaena UTEX 373
<220>
   <221> CDS
   <222> (1)..(1362)
   <223> delta-5 desaturase
<300>
   <302> DELTA-5 DESATURASES AND THEIR USE IN MAKING POLYUNSATURATED FATTY ACIDS
<310> U.S. 2008-0274521-A1
   <311> 2008-04-29
   <312> 2008-11-06
   <313> (1)..(1362)
<300>
   <302> DELTA-5 DESATURASES AND THEIR USE IN MAKING POLYUNSATURATED FATTY ACIDS
<310> WO 2008/137532
   <311> 2008-05-01
   <312> 2008-11-13
   <313> (1)..(1362)
<400> 77
   atg gcc acc atc tct ttg act act gag caa ctt tta gaa cac cca gaa        48
   Met Ala Thr Ile Ser Leu Thr Thr Glu Gln Leu Leu Glu His Pro Glu
   1               5                   10                  15
   ctg gtt gca att gat ggg gtg ttg tac gat ctc ttc gga ctg gcg aaa        96
   Leu Val Ala Ile Asp Gly Val Leu Tyr Asp Leu Phe Gly Leu Ala Lys
               20                  25                  30
   gtg cat cca ggt ggc aac ctc att gaa gcc gcc ggt gcc tcc gac gga       144
   Val His Pro Gly Gly Asn Leu Ile Glu Ala Ala Gly Ala Ser Asp Gly
           35                  40                  45
   acc gcc ctg ttc tac tcc atg cac cct gga gtg aag cca gag aat tcg       192
   Thr Ala Leu Phe Tyr Ser Met His Pro Gly Val Lys Pro Glu Asn Ser
       50                  55                  60
   aag ctg ctg cag caa ttt gcc cga ggc aaa cac gaa cga agc tcg aag       240
   Lys Leu Leu Gln Gln Phe Ala Arg Gly Lys His Glu Arg Ser Ser Lys
   65                  70                  75                  80
   gac cca gtg tac acc ttt gac agt ccc ttc gcc cag gat gtc aag cag       288
   Asp Pro Val Tyr Thr Phe Asp Ser Pro Phe Ala Gln Asp Val Lys Gln
                   85                  90                  95
   agc gtt cgg gag gtc atg aag ggg cgc aac tgg tac gcc acg ccc ggc       336
   Ser Val Arg Glu Val Met Lys Gly Arg Asn Trp Tyr Ala Thr Pro Gly
               100                 105                 110
   ttt tgg ctg cgg acc gcg ctg atc atc gcg tgc act gcc ata ggc gaa       384
   Phe Trp Leu Arg Thr Ala Leu Ile Ile Ala Cys Thr Ala Ile Gly Glu
           115                 120                 125
   tgg tat tgg atc act acc ggg gca gtg atg tgg ggc atc ttc acc ggg       432
   Trp Tyr Trp Ile Thr Thr Gly Ala Val Met Trp Gly Ile Phe Thr Gly
       130                 135                 140
   tac ttc cac agc cag att ggg ttg gcg att caa cac gat gcc tct cac       480
   Tyr Phe His Ser Gln Ile Gly Leu Ala Ile Gln His Asp Ala Ser His
   145                 150                 155                 160
   gga gcc atc agc aaa aag ccc tgg gtg aac gcc ttt ttc gcc tac ggc       528
   Gly Ala Ile Ser Lys Lys Pro Trp Val Asn Ala Phe Phe Ala Tyr Gly
                   165                 170                 175
   atc gac gcc att gga tcc tcc cgc tgg atc tgg ctg cag tcc cac att       576
   Ile Asp Ala Ile Gly Ser Ser Arg Trp Ile Trp Leu Gln Ser His Ile
               180                 185                 190
   atg cgc cac cac acc tac acc aac cag cat ggc ctg gac ctg gac gct       624
   Met Arg His His Thr Tyr Thr Asn Gln His Gly Leu Asp Leu Asp Ala
           195                 200                 205
   gcc tcg gcg gag ccg ttc att ttg ttc cac tcc tac ccg gca aca aat       672
   Ala Ser Ala Glu Pro Phe Ile Leu Phe His Ser Tyr Pro Ala Thr Asn
       210                 215                 220
   gcg tca cga aag tgg tac cat cgg ttc cag gcg tgg tac atg tac atc      720
   Ala Ser Arg Lys Trp Tyr His Arg Phe Gln Ala Trp Tyr Met Tyr Ile
   225                 230                 235                 240
   gtt ttg ggg atg tat ggt gtg tcg atg gtg tac aat ccg atg tac ttg      768
   Val Leu Gly Met Tyr Gly Val Ser Met Val Tyr Asn Pro Met Tyr Leu
                   245                 250                 255
   ttc acg atg cag cac aac gac aca atc cca gag gcc acc tct ctt aga      816
   Phe Thr Met Gln His Asn Asp Thr Ile Pro Glu Ala Thr Ser Leu Arg
               260                 265                 270
   cca ggc agc ttt ttc aac cgg cag cgc gcc ttc gcc gtt tcc ctc cgc      864
   Pro Gly Ser Phe Phe Asn Arg Gln Arg Ala Phe Ala Val Ser Leu Arg
           275                 280                 285
   cta ctg ttc atc ttc cgc aac gcc ttc ctc ccc tgg tac atc gcg ggc      912
   Leu Leu Phe Ile Phe Arg Asn Ala Phe Leu Pro Trp Tyr Ile Ala Gly
       290                 295                 300
   gcc tct ccg ctg ctc acc atc ctg ctg gtg cca acg gtc aca ggc atc      960
   Ala Ser Pro Leu Leu Thr Ile Leu Leu Val Pro Thr Val Thr Gly Ile
   305                 310                 315                 320
   ttc ttg aca ttt gtt ttt gtg ctg tcc cat aac ttt gaa ggc gct gag     1008
   Phe Leu Thr Phe Val Phe Val Leu Ser His Asn Phe Glu Gly Ala Glu
                   325                 330                 335
   cgg acc ccc gaa aag aac tgc aag gcc aaa agg gcc aag gag ggg aag     1056
   Arg Thr Pro Glu Lys Asn Cys Lys Ala Lys Arg Ala Lys Glu Gly Lys
               340                 345                 350
   gag gtc cgc gat gta gag gag gac cgg gtg gac tgg tac cgg gcg cag     1104
   Glu Val Arg Asp Val Glu Glu Asp Arg Val Asp Trp Tyr Arg Ala Gln
           355                 360                 365
   gcc gag acc gcg gcg acc tac ggg ggc agc gtc ggg atg atg ctg acc     1152
   Ala Glu Thr Ala Ala Thr Tyr Gly Gly Ser Val Gly Met Met Leu Thr
       370                 375                 380
   ggc ggt ttg aac ctg cag atc gag cac cac ttg ttc ccc cgc atg tcc     1200
   Gly Gly Leu Asn Leu Gln Ile Glu His His Leu Phe Pro Arg Met Ser
   385                 390                 395                 400
   tct tgg cac tac ccc ttc atc caa gat acg gtg cgg gaa tgt tgc aag     1248
   Ser Trp His Tyr Pro Phe Ile Gln Asp Thr Val Arg Glu Cys Cys Lys
                   405                 410                 415
   cgc cat ggc gtg cgc tac aca tac tac ccg acc atc ctg gag aat ata     1296
   Arg His Gly Val Arg Tyr Thr Tyr Tyr Pro Thr Ile Leu Glu Asn Ile
               420                 425                 430
   atg tcc acg ctc cgc tac atg cag aag gtg ggc gtg gcc cac aca att     1344
   Met Ser Thr Leu Arg Tyr Met Gln Lys Val Gly Val Ala His Thr Ile
           435                 440                 445
   cag gat gcc cag gaa ttc                                             1362
   Gln Asp Ala Gln Glu Phe
       450
<210> 78
   <211> 454
   <212> PRT
   <213> Euglena anabaena UTEX 373
<400> 78
   Met Ala Thr Ile Ser Leu Thr Thr Glu Gln Leu Leu Glu His Pro Glu
   1               5                   10                  15
   Leu Val Ala Ile Asp Gly Val Leu Tyr Asp Leu Phe Gly Leu Ala Lys
               20                  25                  30
   Val His Pro Gly Gly Asn Leu Ile Glu Ala Ala Gly Ala Ser Asp Gly
           35                  40                  45
   Thr Ala Leu Phe Tyr Ser Met His Pro Gly Val Lys Pro Glu Asn Ser
       50                  55                  60
   Lys Leu Leu Gln Gln Phe Ala Arg Gly Lys His Glu Arg Ser Ser Lys
   65                  70                  75                  80
   Asp Pro Val Tyr Thr Phe Asp Ser Pro Phe Ala Gln Asp Val Lys Gln
                   85                  90                  95
   Ser Val Arg Glu Val Met Lys Gly Arg Asn Trp Tyr Ala Thr Pro Gly
               100                 105                 110
   Phe Trp Leu Arg Thr Ala Leu Ile Ile Ala Cys Thr Ala Ile Gly Glu
           115                 120                 125
   Trp Tyr Trp Ile Thr Thr Gly Ala Val Met Trp Gly Ile Phe Thr Gly
       130                 135                 140
   Tyr Phe His Ser Gln Ile Gly Leu Ala Ile Gln His Asp Ala Ser His
   145                 150                 155                 160
   Gly Ala Ile Ser Lys Lys Pro Trp Val Asn Ala Phe Phe Ala Tyr Gly
                   165                 170                 175
   Ile Asp Ala Ile Gly Ser Ser Arg Trp Ile Trp Leu Gln Ser His Ile
               180                 185                 190
   Met Arg His His Thr Tyr Thr Asn Gln His Gly Leu Asp Leu Asp Ala
           195                 200                 205
   Ala Ser Ala Glu Pro Phe Ile Leu Phe His Ser Tyr Pro Ala Thr Asn
       210                 215                 220
   Ala Ser Arg Lys Trp Tyr His Arg Phe Gln Ala Trp Tyr Met Tyr Ile
   225                 230                 235                 240
   Val Leu Gly Met Tyr Gly Val Ser Met Val Tyr Asn Pro Met Tyr Leu
                   245                 250                 255
   Phe Thr Met Gln His Asn Asp Thr Ile Pro Glu Ala Thr Ser Leu Arg
               260                 265                 270
   Pro Gly Ser Phe Phe Asn Arg Gln Arg Ala Phe Ala Val Ser Leu Arg
           275                 280                 285
   Leu Leu Phe Ile Phe Arg Asn Ala Phe Leu Pro Trp Tyr Ile Ala Gly
       290                 295                 300
   Ala Ser Pro Leu Leu Thr Ile Leu Leu Val Pro Thr Val Thr Gly Ile
   305                 310                 315                 320
   Phe Leu Thr Phe Val Phe Val Leu Ser His Asn Phe Glu Gly Ala Glu
                   325                 330                 335
   Arg Thr Pro Glu Lys Asn Cys Lys Ala Lys Arg Ala Lys Glu Gly Lys
               340                 345                 350
   Glu Val Arg Asp Val Glu Glu Asp Arg Val Asp Trp Tyr Arg Ala Gln
           355                 360                 365
   Ala Glu Thr Ala Ala Thr Tyr Gly Gly Ser Val Gly Met Met Leu Thr
       370                 375                 380
   Gly Gly Leu Asn Leu Gln Ile Glu His His Leu Phe Pro Arg Met Ser
   385                 390                 395                 400
   Ser Trp His Tyr Pro Phe Ile Gln Asp Thr Val Arg Glu Cys Cys Lys
                   405                 410                 415
   Arg His Gly Val Arg Tyr Thr Tyr Tyr Pro Thr Ile Leu Glu Asn Ile
               420                 425                 430
   Met Ser Thr Leu Arg Tyr Met Gln Lys Val Gly Val Ala His Thr Ile
           435                 440                 445
   Gln Asp Ala Gln Glu Phe
       450
<210> 79
   <211> 1362
   <212> DNA
   <213> Euglena anabaena UTEX 373
<220>
   <221> CDS
   <222> (1)..(1362)
   <223> synthetic delta-5 desaturase (codon-optimized for Yarrowia lipolytica)
<300>
   <302> DELTA-5 DESATURASES AND THEIR USE IN MAKING POLYUNSATURATED FATTY ACIDS
<310> U.S. 2008-0274521-A1
   <311> 2008-04-29
   <312> 2008-11-06
   <313> (1)..(1362)
<300>
   <302> DELTA-5 DESATURASES AND THEIR USE IN MAKING POLYUNSATURATED FATTY ACIDS
<310> WO 2008/137532
   <311> 2008-05-01
   <312> 2008-11-13
   <313> (1)..(1362)
<400> 79
   atg gcc acc atc tcc ctg act acc gag cag ctc ctg gaa cac ccc gag        48
   Met Ala Thr Ile Ser Leu Thr Thr Glu Gln Leu Leu Glu His Pro Glu
   1               5                   10                  15
   ctc gtt gcc atc gac gga gtc ctg tac gat ctc ttc ggt ctg gcc aag        96
   Leu Val Ala Ile Asp Gly Val Leu Tyr Asp Leu Phe Gly Leu Ala Lys
               20                  25                  30
   gtg cat cca gga ggc aac ctc atc gaa gct gcc ggt gca tcc gac gga       144
   Val His Pro Gly Gly Asn Leu Ile Glu Ala Ala Gly Ala Ser Asp Gly
           35                  40                  45
   acc gct ctg ttc tac tcc atg cat cct gga gtc aag cca gag aac tcg       192
   Thr Ala Leu Phe Tyr Ser Met His Pro Gly Val Lys Pro Glu Asn Ser
       50                  55                  60
   aag ctt ctg cag caa ttt gcc cga ggc aag cac gaa cga agc tcc aag       240
   Lys Leu Leu Gln Gln Phe Ala Arg Gly Lys His Glu Arg Ser Ser Lys
   65                  70                  75                  80
   gat ccc gtg tac acc ttc gac tct ccc ttt gct cag gac gtc aag cag       288
   Asp Pro Val Tyr Thr Phe Asp Ser Pro Phe Ala Gln Asp Val Lys Gln
                   85                  90                  95
   tcc gtt cga gag gtc atg aag ggt cga aac tgg tac gcc act cct ggc       336
   Ser Val Arg Glu Val Met Lys Gly Arg Asn Trp Tyr Ala Thr Pro Gly
               100                 105                 110
   ttc tgg ctg aga acc gca ctc atc atc gct tgt act gcc att ggc gag       384
   Phe Trp Leu Arg Thr Ala Leu Ile Ile Ala Cys Thr Ala Ile Gly Glu
           115                 120                 125
   tgg tac tgg atc aca acc gga gca gtg atg tgg ggt atc ttt act gga       432
   Trp Tyr Trp Ile Thr Thr Gly Ala Val Met Trp Gly Ile Phe Thr Gly
       130                 135                 140
   tac ttc cac tcg cag att ggc ttg gcc att caa cac gat gct tct cac       480
   Tyr Phe His Ser Gln Ile Gly Leu Ala Ile Gln His Asp Ala Ser His
   145                 150                 155                 160
   gga gcc atc agc aaa aag ccc tgg gtc aac gcc ttt ttc gct tat ggc       528
   Gly Ala Ile Ser Lys Lys Pro Trp Val Asn Ala Phe Phe Ala Tyr Gly
                   165                 170                 175
   atc gac gcc att ggt tcc tct cgt tgg atc tgg ctg cag tcc cac att         576
   Ile Asp Ala Ile Gly Ser Ser Arg Trp Ile Trp Leu Gln Ser His Ile
               180                 185                 190
   atg cga cat cac act tac acc aac cag cat ggc ctc gac ctg gat gct         624
   Met Arg His His Thr Tyr Thr Asn Gln His Gly Leu Asp Leu Asp Ala
           195                 200                 205
   gcc tcg gca gag ccg ttc atc ttg ttc cac tcc tat cct gct acc aac         672
   Ala Ser Ala Glu Pro Phe Ile Leu Phe His Ser Tyr Pro Ala Thr Asn
       210                 215                 220
   gcc tct cga aag tgg tac cac cga ttt cag gcg tgg tac atg tac atc         720
   Ala Ser Arg Lys Trp Tyr His Arg Phe Gln Ala Trp Tyr Met Tyr Ile
   225                 230                 235                 240
   gtt ctg gga atg tat ggt gtc tcg atg gtg tac aat ccc atg tac ctc         768
   Val Leu Gly Met Tyr Gly Val Ser Met Val Tyr Asn Pro Met Tyr Leu
                   245                 250                 255
   ttc aca atg cag cac aac gac acc att ccc gag gcc act tct ctc aga         816
   Phe Thr Met Gln His Asn Asp Thr Ile Pro Glu Ala Thr Ser Leu Arg
               260                 265                 270
   cca ggc agc ttt ttc aat cgg cag cga gct ttc gcc gtt tcc ctt cga         864
   Pro Gly Ser Phe Phe Asn Arg Gln Arg Ala Phe Ala Val Ser Leu Arg
           275                 280                 285
   ctg ctc ttc atc ttc cga aac gcc ttt ctt ccc tgg tac att gct ggt         912
   Leu Leu Phe Ile Phe Arg Asn Ala Phe Leu Pro Trp Tyr Ile Ala Gly
       290                 295                 300
   gcc tct cct ctg ctc acc att ctt ctg gtg ccc acg gtc aca ggc atc         960
   Ala Ser Pro Leu Leu Thr Ile Leu Leu Val Pro Thr Val Thr Gly Ile
   305                 310                 315                 320
   ttc ctc acc ttt gtg ttc gtt ctg tcc cat aac ttc gag gga gcc gaa        1008
   Phe Leu Thr Phe Val Phe Val Leu Ser His Asn Phe Glu Gly Ala Glu
                   325                 330                 335
   cgg acc cca gag aag aac tgc aag gcc aaa cga gct aag gaa ggc aag        1056
   Arg Thr Pro Glu Lys Asn Cys Lys Ala Lys Arg Ala Lys Glu Gly Lys
               340                 345                 350
   gag gtc aga gac gtg gaa gag gat cga gtc gac tgg tac cga gca cag        1104
   Glu Val Arg Asp Val Glu Glu Asp Arg Val Asp Trp Tyr Arg Ala Gln
           355                 360                 365
   gcc gag act gct gcc acc tac ggt ggc agc gtg gga atg atg ctt aca        1152
   Ala Glu Thr Ala Ala Thr Tyr Gly Gly Ser Val Gly Met Met Leu Thr
       370                 375                 380
   ggc ggt ctc aac ctg cag atc gag cat cac ttg ttt ccc cga atg tcc        1200
   Gly Gly Leu Asn Leu Gln Ile Glu His His Leu Phe Pro Arg Met Ser
   385                 390                 395                 400
   tct tgg cac tat ccc ttc att caa gac acc gtt cgg gag tgt tgc aag        1248
   Ser Trp His Tyr Pro Phe Ile Gln Asp Thr Val Arg Glu Cys Cys Lys
                   405                 410                 415
   cga cat ggc gtc cgt tac aca tac tat cct acc att ctc gag aac atc        1296
   Arg His Gly Val Arg Tyr Thr Tyr Tyr Pro Thr Ile Leu Glu Asn Ile
               420                 425                 430
   atg tcc act ctt cga tac atg cag aag gtg ggt gtt gct cac acc att        1344
   Met Ser Thr Leu Arg Tyr Met Gln Lys Val Gly Val Ala His Thr Ile
           435                 440                 445
   cag gat gcc cag gag ttc                                          1362
   Gln Asp Ala Gln Glu Phe
       450
<210> 80
   <211> 454
   <212> PRT
   <213> Euglena anabaena UTEX 373
<400> 80
   Met Ala Thr Ile Ser Leu Thr Thr Glu Gln Leu Leu Glu His Pro Glu
   1               5                   10                  15
   Leu Val Ala Ile Asp Gly Val Leu Tyr Asp Leu Phe Gly Leu Ala Lys
               20                  25                  30
   Val His Pro Gly Gly Asn Leu Ile Glu Ala Ala Gly Ala Ser Asp Gly
           35                  40                  45
   Thr Ala Leu Phe Tyr Ser Met His Pro Gly Val Lys Pro Glu Asn Ser
       50                  55                  60
   Lys Leu Leu Gln Gln Phe Ala Arg Gly Lys His Glu Arg Ser Ser Lys
   65                  70                  75                  80
   Asp Pro Val Tyr Thr Phe Asp Ser Pro Phe Ala Gln Asp Val Lys Gln
                   85                  90                  95
   Ser Val Arg Glu Val Met Lys Gly Arg Asn Trp Tyr Ala Thr Pro Gly
               100                 105                 110
   Phe Trp Leu Arg Thr Ala Leu Ile Ile Ala Cys Thr Ala Ile Gly Glu
           115                 120                 125
   Trp Tyr Trp Ile Thr Thr Gly Ala Val Met Trp Gly Ile Phe Thr Gly
       130                 135                 140
   Tyr Phe His Ser Gln Ile Gly Leu Ala Ile Gln His Asp Ala Ser His
   145                 150                 155                 160
   Gly Ala Ile Ser Lys Lys Pro Trp Val Asn Ala Phe Phe Ala Tyr Gly
                   165                 170                 175
   Ile Asp Ala Ile Gly Ser Ser Arg Trp Ile Trp Leu Gln Ser His Ile
               180                 185                 190
   Met Arg His His Thr Tyr Thr Asn Gln His Gly Leu Asp Leu Asp Ala
           195                 200                 205
   Ala Ser Ala Glu Pro Phe Ile Leu Phe His Ser Tyr Pro Ala Thr Asn
       210                 215                 220
   Ala Ser Arg Lys Trp Tyr His Arg Phe Gln Ala Trp Tyr Met Tyr Ile
   225                 230                 235                 240
   Val Leu Gly Met Tyr Gly Val Ser Met Val Tyr Asn Pro Met Tyr Leu
                   245                 250                 255
   Phe Thr Met Gln His Asn Asp Thr Ile Pro Glu Ala Thr Ser Leu Arg
               260                 265                 270
   Pro Gly Ser Phe Phe Asn Arg Gln Arg Ala Phe Ala Val Ser Leu Arg
           275                 280                 285
   Leu Leu Phe Ile Phe Arg Asn Ala Phe Leu Pro Trp Tyr Ile Ala Gly
       290                 295                 300
   Ala Ser Pro Leu Leu Thr Ile Leu Leu Val Pro Thr Val Thr Gly Ile
   305                 310                 315                 320
   Phe Leu Thr Phe Val Phe Val Leu Ser His Asn Phe Glu Gly Ala Glu
                   325                 330                 335
   Arg Thr Pro Glu Lys Asn Cys Lys Ala Lys Arg Ala Lys Glu Gly Lys
               340                 345                 350
   Glu Val Arg Asp Val Glu Glu Asp Arg Val Asp Trp Tyr Arg Ala Gln
           355                 360                 365
   Ala Glu Thr Ala Ala Thr Tyr Gly Gly Ser Val Gly Met Met Leu Thr
       370                 375                 380
   Gly Gly Leu Asn Leu Gln Ile Glu His His Leu Phe Pro Arg Met Ser
   385                 390                 395                 400
   Ser Trp His Tyr Pro Phe Ile Gln Asp Thr Val Arg Glu Cys Cys Lys
                   405                 410                 415
   Arg His Gly Val Arg Tyr Thr Tyr Tyr Pro Thr Ile Leu Glu Asn Ile
               420                 425                 430
   Met Ser Thr Leu Arg Tyr Met Gln Lys Val Gly Val Ala His Thr Ile
           435                 440                 445
   Gln Asp Ala Gln Glu Phe
       450
<210> 81
   <211> 1365
   <212> DNA
   <213> Euglena anabaena UTEX 373
<220>
   <221> CDS
   <222> (1)..(1365)
   <223> synthetic mutant delta-5 desaturase (codon-optimized for Yarrowia lipolytica)
<400> 81
   atg gcc acc atc tcc ctg act acc gag cag ctc ctg gaa cac ccc gag          48
   Met Ala Thr Ile Ser Leu Thr Thr Glu Gln Leu Leu Glu His Pro Glu
   1               5                   10                  15
   ctc gtt gcc atc gac gga gtc ctg tac gat ctc ttc ggt ctg gcc aag          96
   Leu Val Ala Ile Asp Gly Val Leu Tyr Asp Leu Phe Gly Leu Ala Lys
               20                  25                  30
   gtc cat gcc gga ggc aac ctc atc gaa gct gcc ggt gca tcc gac gga         144
   Val His Ala Gly Gly Asn Leu Ile Glu Ala Ala Gly Ala Ser Asp Gly
           35                  40                  45
   acc gct ctg ttc tac tcc atg cat cct gga gtc aag cca gag aac tcg         192
   Thr Ala Leu Phe Tyr Ser Met His Pro Gly Val Lys Pro Glu Asn Ser
       50                  55                  60
   aag ctt ctg cag caa ttt gcc cga ggc aag cac gaa cga agc tcc aag         240
   Lys Leu Leu Gln Gln Phe Ala Arg Gly Lys His Glu Arg Ser Ser Lys
   65                  70                  75                  80
   gat ccc gtg tac acc ttc gac tct ccc ttt gct cag gac gtc aag cag         288
   Asp Pro Val Tyr Thr Phe Asp Ser Pro Phe Ala Gln Asp Val Lys Gln
                   85                  90                  95
   tcc gtt cga gag gtc atg aag ggt cga aac tgg tac gcc act cct ggc         336
   Ser Val Arg Glu Val Met Lys Gly Arg Asn Trp Tyr Ala Thr Pro Gly
               100                 105                 110
   ttc tgg ctg aga acc gca ctc atc atc gct tgt act gcc att ggc gag         384
   Phe Trp Leu Arg Thr Ala Leu Ile Ile Ala Cys Thr Ala Ile Gly Glu
           115                 120                 125
   tgg tac tgg atc aca acc gga gca gtg atg tgg ggt atc ttt act gga         432
   Trp Tyr Trp Ile Thr Thr Gly Ala Val Met Trp Gly Ile Phe Thr Gly
       130                 135                 140
   tac ttc cac tcg cag att ggc ttg gcc att caa cac gat gct tct cac         480
   Tyr Phe His Ser Gln Ile Gly Leu Ala Ile Gln His Asp Ala Ser His
   145                 150                 155                 160
   gga gcc atc agc aaa aag ccc tgg gtc aac gcc ttt ttc gct tat ggc         528
   Gly Ala Ile Ser Lys Lys Pro Trp Val Asn Ala Phe Phe Ala Tyr Gly
                   165                 170                 175
   atc gac gcc att ggt tcc tct cgt tgg atc tgg ctg cag tcc cac att         576
   Ile Asp Ala Ile Gly Ser Ser Arg Trp Ile Trp Leu Gln Ser His Ile
               180                 185                 190
   atg cga cat cac act tac acc aac cag cat ggc ctc gac ctg gat gct         624
   Met Arg His His Thr Tyr Thr Asn Gln His Gly Leu Asp Leu Asp Ala
           195                 200                 205
   gcc tcg gca gag ccg ttc atc ttg ttc cac tcc tat cct gct acc aac        672
   Ala Ser Ala Glu Pro Phe Ile Leu Phe His Ser Tyr Pro Ala Thr Asn
       210                 215                 220
   gcc tct cga aag tgg tac cac cga ttt cag gcg tgg tac atg tac atc        720
   Ala Ser Arg Lys Trp Tyr His Arg Phe Gln Ala Trp Tyr Met Tyr Ile
   225                 230                 235                 240
   gtt ctg gga atg tat ggt gtc tcg atg gtg tac aat ccc atg tac ctc        768
   Val Leu Gly Met Tyr Gly Val Ser Met Val Tyr Asn Pro Met Tyr Leu
                   245                 250                 255
   ttc aca atg cag cac aac gac acc att ccc gag gcc act tct ctc aga        816
   Phe Thr Met Gln His Asn Asp Thr Ile Pro Glu Ala Thr Ser Leu Arg
               260                 265                 270
   cca ggc agc ttt ttc aat cgg cag cga gct ttc gcc gtt tcc ctt cga        864
   Pro Gly Ser Phe Phe Asn Arg Gln Arg Ala Phe Ala Val Ser Leu Arg
           275                 280                 285
   ctg ctc ttc atc ttc cga aac gcc ttt ctt ccc tgg tac att gct ggt        912
   Leu Leu Phe Ile Phe Arg Asn Ala Phe Leu Pro Trp Tyr Ile Ala Gly
       290                 295                 300
   gcc tct cct ctg ctc acc att ctt ctg gtg ccc acg gtc aca ggc atc        960
   Ala Ser Pro Leu Leu Thr Ile Leu Leu Val Pro Thr Val Thr Gly Ile
   305                 310                 315                 320
   ttc ctc acc ttt gtg ttc gtt ctg tcc cat aac ttc gag gga gcc gaa       1008
   Phe Leu Thr Phe Val Phe Val Leu Ser His Asn Phe Glu Gly Ala Glu
                   325                 330                 335
   cgg acc cca gag aag aac tgc aag gcc aaa cga gct aag gaa ggc aag       1056
   Arg Thr Pro Glu Lys Asn Cys Lys Ala Lys Arg Ala Lys Glu Gly Lys
               340                 345                 350
   gag gtc aga gac gtg gaa gag gat cga gtc gac tgg tac cga gca cag       1104
   Glu Val Arg Asp Val Glu Glu Asp Arg Val Asp Trp Tyr Arg Ala Gln
           355                 360                 365
   gcc gag act gct gcc acc tac ggt ggc agc gtg gga atg atg ctt aca       1152
   Ala Glu Thr Ala Ala Thr Tyr Gly Gly Ser Val Gly Met Met Leu Thr
       370                 375                 380
   ggc ggt ctc aac ctg cag atc gag cat cac ttg ttt ccc cga atg tcc       1200
   Gly Gly Leu Asn Leu Gln Ile Glu His His Leu Phe Pro Arg Met Ser
   385                 390                 395                 400
   tct tgg cac tat ccc ttc att caa gac acc gtt cgg gag tgt tgc aag       1248
   Ser Trp His Tyr Pro Phe Ile Gln Asp Thr Val Arg Glu Cys Cys Lys
                   405                 410                 415
   cga cat ggc gtc cgt tac aca tac tat cct acc att ctc gag aac atc       1296
   Arg His Gly Val Arg Tyr Thr Tyr Tyr Pro Thr Ile Leu Glu Asn Ile
               420                 425                 430
   atg tcc act ctt cga tac atg cag aag gtg ggt gtt gct cac acc att       1344
   Met Ser Thr Leu Arg Tyr Met Gln Lys Val Gly Val Ala His Thr Ile
           435                 440                 445
   cag gat gcc cag gag ttc taa                                           1365
   Gln Asp Ala Gln Glu Phe
       450
<210> 82
   <211> 454
   <212> PRT
   <213> Euglena anabaena UTEX 373
<400> 82
   Met Ala Thr Ile Ser Leu Thr Thr Glu Gln Leu Leu Glu His Pro Glu
   1               5                   10                  15
   Leu Val Ala Ile Asp Gly Val Leu Tyr Asp Leu Phe Gly Leu Ala Lys
               20                  25                  30
   Val His Ala Gly Gly Asn Leu Ile Glu Ala Ala Gly Ala Ser Asp Gly
           35                  40                  45
   Thr Ala Leu Phe Tyr Ser Met His Pro Gly Val Lys Pro Glu Asn Ser
       50                  55                  60
   Lys Leu Leu Gln Gln Phe Ala Arg Gly Lys His Glu Arg Ser Ser Lys
   65                  70                  75                  80
   Asp Pro Val Tyr Thr Phe Asp Ser Pro Phe Ala Gln Asp Val Lys Gln
                   85                  90                  95
   Ser Val Arg Glu Val Met Lys Gly Arg Asn Trp Tyr Ala Thr Pro Gly
               100                 105                 110
   Phe Trp Leu Arg Thr Ala Leu Ile Ile Ala Cys Thr Ala Ile Gly Glu
           115                 120                 125
   Trp Tyr Trp Ile Thr Thr Gly Ala Val Met Trp Gly Ile Phe Thr Gly
       130                 135                 140
   Tyr Phe His Ser Gln Ile Gly Leu Ala Ile Gln His Asp Ala Ser His
   145                 150                 155                 160
   Gly Ala Ile Ser Lys Lys Pro Trp Val Asn Ala Phe Phe Ala Tyr Gly
                   165                 170                 175
   Ile Asp Ala Ile Gly Ser Ser Arg Trp Ile Trp Leu Gln Ser His Ile
               180                 185                 190
   Met Arg His His Thr Tyr Thr Asn Gln His Gly Leu Asp Leu Asp Ala
           195                 200                 205
   Ala Ser Ala Glu Pro Phe Ile Leu Phe His Ser Tyr Pro Ala Thr Asn
       210                 215                 220
   Ala Ser Arg Lys Trp Tyr His Arg Phe Gln Ala Trp Tyr Met Tyr Ile
   225                 230                 235                 240
   Val Leu Gly Met Tyr Gly Val Ser Met Val Tyr Asn Pro Met Tyr Leu
                   245                 250                 255
   Phe Thr Met Gln His Asn Asp Thr Ile Pro Glu Ala Thr Ser Leu Arg
               260                 265                 270
   Pro Gly Ser Phe Phe Asn Arg Gln Arg Ala Phe Ala Val Ser Leu Arg
           275                 280                 285
   Leu Leu Phe Ile Phe Arg Asn Ala Phe Leu Pro Trp Tyr Ile Ala Gly
       290                 295                 300
   Ala Ser Pro Leu Leu Thr Ile Leu Leu Val Pro Thr Val Thr Gly Ile
   305                 310                 315                 320
   Phe Leu Thr Phe Val Phe Val Leu Ser His Asn Phe Glu Gly Ala Glu
                   325                 330                 335
   Arg Thr Pro Glu Lys Asn Cys Lys Ala Lys Arg Ala Lys Glu Gly Lys
               340                 345                 350
   Glu Val Arg Asp Val Glu Glu Asp Arg Val Asp Trp Tyr Arg Ala Gln
           355                 360                 365
   Ala Glu Thr Ala Ala Thr Tyr Gly Gly Ser Val Gly Met Met Leu Thr
       370                 375                 380
   Gly Gly Leu Asn Leu Gln Ile Glu His His Leu Phe Pro Arg Met Ser
   385                 390                 395                 400
   Ser Trp His Tyr Pro Phe Ile Gln Asp Thr Val Arg Glu Cys Cys Lys
                   405                 410                 415
   Arg His Gly Val Arg Tyr Thr Tyr Tyr Pro Thr Ile Leu Glu Asn Ile
               420                 425                 430
   Met Ser Thr Leu Arg Tyr Met Gln Lys Val Gly Val Ala His Thr Ile
           435                 440                 445
   Gln Asp Ala Gln Glu Phe
       450
<210> 83
   <211> 1086
   <212> DNA
   <213> Phytophthora ramorum
<220>
   <221> CDS
   <222> (1)..(1086)
   <223> delta-17 desaturase
<300>
   <302> DELTA-17 DESATURASES AND THEIR USE IN MAKING POLYUNSATURATED FATTY ACIDS
<310> WO 2007/123999
   <311> 2007-04-19
   <312> 2007-11-01
   <313> (1)..(1086)
<300>
   <302> DELTA-17 DESATURASES AND THEIR USE IN MAKING POLYUNSATURATED FATTY ACIDS
<310> US 2007-0249026-A1
   <311> 2007-04-18
   <312> 2007-10-25
   <313> (1)..(1086)
<400> 83
   atg gcg act aag cag ccg tac cag ttc ccg acc ctg acg gag atc aag      48
   Met Ala Thr Lys Gln Pro Tyr Gln Phe Pro Thr Leu Thr Glu Ile Lys
   1               5                   10                  15
   cgg tcg ctg ccc agc gag tgc ttt gag gcc tcg gtg ccg ctg tcg ctc      96
   Arg Ser Leu Pro Ser Glu Cys Phe Glu Ala Ser Val Pro Leu Ser Leu
               20                  25                  30
   tac tac acg gtg cgc atc gtg gcc atc gcc gtg gcg ctg gcg ttc ggc      144
   Tyr Tyr Thr Val Arg Ile Val Ala Ile Ala Val Ala Leu Ala Phe Gly
           35                  40                  45
   ctc aac tac gcg cgc gcg ctg ccc gtg gtc gag agc ttg tgg gcg ctg      192
   Leu Asn Tyr Ala Arg Ala Leu Pro Val Val Glu Ser Leu Trp Ala Leu
       50                  55                  60
   gac gct gcg ctc tgc tgc ggt tac gtg ctg ctg cag ggc atc gtg ttc      240
   Asp Ala Ala Leu Cys Cys Gly Tyr Val Leu Leu Gln Gly Ile Val Phe
   65                  70                  75                  80
   tgg ggc ttc ttc acg gtg ggc cat gac gcc ggc cac ggc gcc ttc tcg      288
   Trp Gly Phe Phe Thr Val Gly His Asp Ala Gly His Gly Ala Phe Ser
                   85                  90                  95
   cgt tac cac ctg ctc aac ttc gtg gtg ggc acc ttc atc cac tcg ctc      336
   Arg Tyr His Leu Leu Asn Phe Val Val Gly Thr Phe Ile His Ser Leu
               100                 105                 110
   atc ctc acg ccc ttc gag tcg tgg aag ctc acg cac cgc cac cac cac      384
   Ile Leu Thr Pro Phe Glu Ser Trp Lys Leu Thr His Arg His His His
           115                 120                 125
   aag aac acg ggc aac att gac cgc gac gag atc ttc tac ccg cag cgc      432
   Lys Asn Thr Gly Asn Ile Asp Arg Asp Glu Ile Phe Tyr Pro Gln Arg
       130                 135                 140
   aag gcc gac gac cac ccg ctg tcg cgc aac ctc gtg ctg gcg ctc ggc      480
   Lys Ala Asp Asp His Pro Leu Ser Arg Asn Leu Val Leu Ala Leu Gly
   145                 150                 155                 160
   gcc gcg tgg ttc gcc tac ctg gtc gag ggc ttc ccg ccc cgc aag gtc      528
   Ala Ala Trp Phe Ala Tyr Leu Val Glu Gly Phe Pro Pro Arg Lys Val
                   165                 170                 175
   aac cac ttc aac cca ttc gag ccg ctg ttt gtg cgc cag gtg gcc gcc        576
   Asn His Phe Asn Pro Phe Glu Pro Leu Phe Val Arg Gln Val Ala Ala
                   180                 185                 190
   gtc gtc atc tcg ctc tcc gcg cac ttc gcc gtg ttg gcg ctg tcc gtg        624
   Val Val Ile Ser Leu Ser Ala His Phe Ala Val Leu Ala Leu Ser Val
           195                 200                 205
   tat ctg agc ttc cag ttc ggt ctc aag acc atg gcg ctc tac tac tac        672
   Tyr Leu Ser Phe Gln Phe Gly Leu Lys Thr Met Ala Leu Tyr Tyr Tyr
       210                 215                 220
   ggc ccc gtc ttc gtg ttc ggc agc atg ctt gtg atc acc acc ttc ctg        720
   Gly Pro Val Phe Val Phe Gly Ser Met Leu Val Ile Thr Thr Phe Leu
   225                 230                 235                 240
   cat cac aat gac gag gag acc cca tgg tac gga gac tcc gac tgg acc        768
   His His Asn Asp Glu Glu Thr Pro Trp Tyr Gly Asp Ser Asp Trp Thr
                   245                 250                 255
   tac gtc aag ggc aac ctg tcg tcc gtg gac cgg tcc tac ggc gcg ttc        816
   Tyr Val Lys Gly Asn Leu Ser Ser Val Asp Arg Ser Tyr Gly Ala Phe
               260                 265                 270
   atc gac aac ctg agc cac aac atc ggc acg cac cag atc cac cac ctc        864
   Ile Asp Asn Leu Ser His Asn Ile Gly Thr His Gln Ile His His Leu
           275                 280                 285
   ttc ccc atc atc ccg cac tac aag ctc aac cgc gct acg gcg gca ttc        912
   Phe Pro Ile Ile Pro His Tyr Lys Leu Asn Arg Ala Thr Ala Ala Phe
       290                 295                 300
   cac cag gcc ttc ccc gag ctc gtg cgc aag agc gac gag ccg atc ctc        960
   His Gln Ala Phe Pro Glu Leu Val Arg Lys Ser Asp Glu Pro Ile Leu
   305                 310                 315                 320
   aag gcc ttc tgg cgc gtc ggc cga ctg tac gcc aac tac ggc gtc gtg       1008
   Lys Ala Phe Trp Arg Val Gly Arg Leu Tyr Ala Asn Tyr Gly Val Val
                   325                 330                 335
   gac ccg gac gcc aag ctc ttc acg ctc aag gag gcc aag gcg gcg tcc       1056
   Asp Pro Asp Ala Lys Leu Phe Thr Leu Lys Glu Ala Lys Ala Ala Ser
               340                 345                 350
   gag gcg gcg acc aag acc aag gcc acc taa                               1086
   Glu Ala Ala Thr Lys Thr Lys Ala Thr
           355                 360
<210> 84
   <211> 361
   <212> PRT
   <213> Phytophthora ramorum
<400> 84
   Met Ala Thr Lys Gln Pro Tyr Gln Phe Pro Thr Leu Thr Glu Ile Lys
   1               5                   10                  15
   Arg Ser Leu Pro Ser Glu Cys Phe Glu Ala Ser Val Pro Leu Ser Leu
               20                  25                  30
   Tyr Tyr Thr Val Arg Ile Val Ala Ile Ala Val Ala Leu Ala Phe Gly
           35                  40                  45
   Leu Asn Tyr Ala Arg Ala Leu Pro Val Val Glu Ser Leu Trp Ala Leu
       50                  55                  60
   Asp Ala Ala Leu Cys Cys Gly Tyr Val Leu Leu Gln Gly Ile Val Phe
   65                  70                  75                  80
   Trp Gly Phe Phe Thr Val Gly His Asp Ala Gly His Gly Ala Phe Ser
                   85                  90                  95
   Arg Tyr His Leu Leu Asn Phe Val Val Gly Thr Phe Ile His Ser Leu
               100                 105                 110
   Ile Leu Thr Pro Phe Glu Ser Trp Lys Leu Thr His Arg His His His
           115                 120                 125
   Lys Asn Thr Gly Asn Ile Asp Arg Asp Glu Ile Phe Tyr Pro Gln Arg
       130                 135                 140
   Lys Ala Asp Asp His Pro Leu Ser Arg Asn Leu Val Leu Ala Leu Gly
   145                 150                 155                 160
   Ala Ala Trp Phe Ala Tyr Leu Val Glu Gly Phe Pro Pro Arg Lys Val
                   165                 170                 175
   Asn His Phe Asn Pro Phe Glu Pro Leu Phe Val Arg Gln Val Ala Ala
               180                 185                 190
   Val Val Ile Ser Leu Ser Ala His Phe Ala Val Leu Ala Leu Ser Val
           195                 200                 205
   Tyr Leu Ser Phe Gln Phe Gly Leu Lys Thr Met Ala Leu Tyr Tyr Tyr
       210                 215                 220
   Gly Pro Val Phe Val Phe Gly Ser Met Leu Val Ile Thr Thr Phe Leu
   225                 230                 235                 240
   His His Asn Asp Glu Glu Thr Pro Trp Tyr Gly Asp Ser Asp Trp Thr
                   245                 250                 255
   Tyr Val Lys Gly Asn Leu Ser Ser Val Asp Arg Ser Tyr Gly Ala Phe
               260                 265                 270
   Ile Asp Asn Leu Ser His Asn Ile Gly Thr His Gln Ile His His Leu
           275                 280                 285
   Phe Pro Ile Ile Pro His Tyr Lys Leu Asn Arg Ala Thr Ala Ala Phe
   290 295 300
   His Gln Ala Phe Pro Glu Leu Val Arg Lys Ser Asp Glu Pro Ile Leu
   305 310 315 320
   Lys Ala Phe Trp Arg Val Gly Arg Leu Tyr Ala Asn Tyr Gly Val Val
   325 330 335
   Asp Pro Asp Ala Lys Leu Phe Thr Leu Lys Glu Ala Lys Ala Ala Ser
   340 345 350
   Glu Ala Ala Thr Lys Thr Lys Ala Thr
   355 360
<210> 85
   <211> 1086
   <212> DNA
   <213> Phytophthora ramorum
<220>
   <221> CDS
   <222> (1)..(1086)
   <223> synthetic delta-17 desaturase (codon-optimized for Yarrowia lipolytica)
<300>
   <302> DELTA-17 DESATURASES AND THEIR USE IN MAKING POLYUNSATURATED FATTY ACIDS
<310> WO 2007/123999
   <311> 2007-04-19
   <312> 2007-11-01
   <313> (1)..(1086)
<300>
   <302> DELTA-17 DESATURASES AND THEIR USE IN MAKING POLYUNSATURATED FATTY ACIDS
<310> US 2007-0249026-A1
   <311> 2007-04-18
   <312> 2007-10-25
   <313> (1)..(1086)
<400> 85
   atg gct acc aag cag ccc tac cag ttc cct act ctg acc gag atc aag        48
   Met Ala Thr Lys Gln Pro Tyr Gln Phe Pro Thr Leu Thr Glu Ile Lys
   1               5                   10                  15
   cga tct ctt ccc tcc gag tgc ttt gaa gcc tcg gtc cct ctg tcc ttg        96
   Arg Ser Leu Pro Ser Glu Cys Phe Glu Ala Ser Val Pro Leu Ser Leu
               20                  25                  30
   tac tac acc gtg cga atc gtc gct att gcc gtt gct ctg gcc ttc gga       144
   Tyr Tyr Thr Val Arg Ile Val Ala Ile Ala Val Ala Leu Ala Phe Gly
           35                  40                  45
   ctc aac tac gct cga gcc ctt ccc gtg gtc gag tct ctg tgg gca ctc       192
   Leu Asn Tyr Ala Arg Ala Leu Pro Val Val Glu Ser Leu Trp Ala Leu
       50                  55                  60
   gac gct gcc ctt tgt tgc ggt tac gtt ctg ctc caa ggc att gtc ttc       240
   Asp Ala Ala Leu Cys Cys Gly Tyr Val Leu Leu Gln Gly Ile Val Phe
   65                  70                  75                  80
   tgg gga ttc ttt acc gtg ggt cac gat gct gga cat ggt gcc ttc tct      288
   Trp Gly Phe Phe Thr Val Gly His Asp Ala Gly His Gly Ala Phe Ser
                   85                  90                  95
   cga tac cac ctg ctc aac ttt gtc gtt ggc acc ttt atc cac tcc ctc      336
   Arg Tyr His Leu Leu Asn Phe Val Val Gly Thr Phe Ile His Ser Leu
               100                 105                 110
   att ctt act ccc ttc gag tcg tgg aag ctc aca cat cga cac cat cac      384
   Ile Leu Thr Pro Phe Glu Ser Trp Lys Leu Thr His Arg His His His
           115                 120                 125
   aag aac acc gga aac atc gac cga gac gaa atc ttc tac cct cag cga      432
   Lys Asn Thr Gly Asn Ile Asp Arg Asp Glu Ile Phe Tyr Pro Gln Arg
       130                 135                 140
   aag gcc gac gat cat cct ctg tct cga aac ctc gtc ctg gct ctc ggt      480
   Lys Ala Asp Asp His Pro Leu Ser Arg Asn Leu Val Leu Ala Leu Gly
   145                 150                 155                 160
   gcc gct tgg ttt gcc tac ctt gtc gag ggc ttt cct ccc cga aag gtc      528
   Ala Ala Trp Phe Ala Tyr Leu Val Glu Gly Phe Pro Pro Arg Lys Val
                   165                 170                 175
   aac cac ttc aac ccc ttc gaa cct ctg ttt gtg cga cag gtg gct gcc      576
   Asn His Phe Asn Pro Phe Glu Pro Leu Phe Val Arg Gln Val Ala Ala
               180                 185                 190
   gtt gtc att tcc ctc tct gct cac ttc gcc gtc ctg gca ctg tcc gtg      624
   Val Val Ile Ser Leu Ser Ala His Phe Ala Val Leu Ala Leu Ser Val
           195                 200                 205
   tat ctg agc ttt cag ttc ggt ctc aag aca atg gct ctg tac tac tat      672
   Tyr Leu Ser Phe Gln Phe Gly Leu Lys Thr Met Ala Leu Tyr Tyr Tyr
       210                 215                 220
   gga ccc gtc ttc gtg ttc ggc tcc atg ctc gtc att act acc ttt ctg      720
   Gly Pro Val Phe Val Phe Gly Ser Met Leu Val Ile Thr Thr Phe Leu
   225                 230                 235                 240
   cat cac aat gac gag gaa act cct tgg tac gga gat tcc gac tgg acc      768
   His His Asn Asp Glu Glu Thr Pro Trp Tyr Gly Asp Ser Asp Trp Thr
                   245                 250                 255
   tac gtc aag ggc aac ttg tct tcc gtg gac cga tct tac ggt gcc ttc      816
   Tyr Val Lys Gly Asn Leu Ser Ser Val Asp Arg Ser Tyr Gly Ala Phe
               260                 265                 270
   atc gac aac ctc tcg cac aac att ggc aca cac cag atc cac cat ctg      864
   Ile Asp Asn Leu Ser His Asn Ile Gly Thr His Gln Ile His His Leu
           275                 280                 285
   ttt ccc atc att cct cac tac aag ctc aac cga gcc acc gct gcc ttc      912
   Phe Pro Ile Ile Pro His Tyr Lys Leu Asn Arg Ala Thr Ala Ala Phe
       290                 295                 300
   cac cag gcc ttt ccc gaa ctt gtc cga aag agc gac gag ccc att ctc      960
   His Gln Ala Phe Pro Glu Leu Val Arg Lys Ser Asp Glu Pro Ile Leu
   305                 310                 315                 320
   aag gct ttc tgg aga gtt ggt cga ctt tac gcc aac tac gga gtc gtg     1008
   Lys Ala Phe Trp Arg Val Gly Arg Leu Tyr Ala Asn Tyr Gly Val Val
                   325                 330                 335
   gat ccc gac gca aag ctg ttt act ctc aag gag gcc aaa gct gcc tcc       1056
   Asp Pro Asp Ala Lys Leu Phe Thr Leu Lys Glu Ala Lys Ala Ala Ser
               340                 345                 350
   gag gct gcc acc aag acc aag gct act taa                               1086
   Glu Ala Ala Thr Lys Thr Lys Ala Thr
           355                 360
<210> 86
   <211> 361
   <212> PRT
   <213> Phytophthora ramorum
<400> 86
   Met Ala Thr Lys Gln Pro Tyr Gln Phe Pro Thr Leu Thr Glu Ile Lys
   1               5                   10                  15
   Arg Ser Leu Pro Ser Glu Cys Phe Glu Ala Ser Val Pro Leu Ser Leu
               20                  25                  30
   Tyr Tyr Thr Val Arg Ile Val Ala Ile Ala Val Ala Leu Ala Phe Gly
           35                  40                  45
   Leu Asn Tyr Ala Arg Ala Leu Pro Val Val Glu Ser Leu Trp Ala Leu
       50                  55                  60
   Asp Ala Ala Leu Cys Cys Gly Tyr Val Leu Leu Gln Gly Ile Val Phe
   65                  70                  75                  80
   Trp Gly Phe Phe Thr Val Gly His Asp Ala Gly His Gly Ala Phe Ser
                   85                  90                  95
   Arg Tyr His Leu Leu Asn Phe Val Val Gly Thr Phe Ile His Ser Leu
               100                 105                 110
   Ile Leu Thr Pro Phe Glu Ser Trp Lys Leu Thr His Arg His His His
           115                 120                 125
   Lys Asn Thr Gly Asn Ile Asp Arg Asp Glu Ile Phe Tyr Pro Gln Arg
       130                 135                 140
   Lys Ala Asp Asp His Pro Leu Ser Arg Asn Leu Val Leu Ala Leu Gly
   145                 150                 155                 160
   Ala Ala Trp Phe Ala Tyr Leu Val Glu Gly Phe Pro Pro Arg Lys Val
                   165                 170                 175
   Asn His Phe Asn Pro Phe Glu Pro Leu Phe Val Arg Gln Val Ala Ala
               180                 185                 190
   Val Val Ile Ser Leu Ser Ala His Phe Ala Val Leu Ala Leu Ser Val
           195                 200                 205
   Tyr Leu Ser Phe Gln Phe Gly Leu Lys Thr Met Ala Leu Tyr Tyr Tyr
       210                 215                 220
   Gly Pro Val Phe Val Phe Gly Ser Met Leu Val Ile Thr Thr Phe Leu
   225                 230                 235                 240
   His His Asn Asp Glu Glu Thr Pro Trp Tyr Gly Asp Ser Asp Trp Thr
                   245                 250                 255
   Tyr Val Lys Gly Asn Leu Ser Ser Val Asp Arg Ser Tyr Gly Ala Phe
               260                 265                 270
   Ile Asp Asn Leu Ser His Asn Ile Gly Thr His Gln Ile His His Leu
           275                 280                 285
   Phe Pro Ile Ile Pro His Tyr Lys Leu Asn Arg Ala Thr Ala Ala Phe
       290                 295                 300
   His Gln Ala Phe Pro Glu Leu Val Arg Lys Ser Asp Glu Pro Ile Leu
   305                 310                 315                 320
   Lys Ala Phe Trp Arg Val Gly Arg Leu Tyr Ala Asn Tyr Gly Val Val
                   325                 330                 335
   Asp Pro Asp Ala Lys Leu Phe Thr Leu Lys Glu Ala Lys Ala Ala Ser
               340                 345                 350
   Glu Ala Ala Thr Lys Thr Lys Ala Thr
           355                 360
<210> 87
   <211> 1080
   <212> DNA
   <213> Pythium aphanidermatum
<220>
   <221> CDS
   <222> (1)..(1080)
   <223> delta-17 desaturase
<300>
   <302> DELTA-17 DESATURASES AND THEIR USE IN MAKING POLYUNSATURATED FATTY ACIDS
<310> WO 2008/054565
   <311> 2007-07-19
   <312> 2008-05-08
   <313> (1)..(1080)
<300>
   <302> DELTA-17 DESATURASES AND THEIR USE IN MAKING POLYUNSATURATED FATTY ACIDS
<310> US 2008-0125326-A1
   <311> 2007-07-19
   <312> 2008-05-29
   <313> (1)..(1080)
<400> 87
   atg gct tct tcc act gtt gct gcg ccg tac gag ttc ccg acg ctg acg          48
   Met Ala Ser Ser Thr Val Ala Ala Pro Tyr Glu Phe Pro Thr Leu Thr
   1               5                   10                  15
   gag atc aag cgc tcg ctg cca gcg cac tgc ttt gag gcc tcg gtc ccg          96
   Glu Ile Lys Arg Ser Leu Pro Ala His Cys Phe Glu Ala Ser Val Pro
               20                  25                  30
   tgg tcg ctc tac tac acc gtg cgc gcg ctg ggc atc gcc ggc tcg ctc         144
   Trp Ser Leu Tyr Tyr Thr Val Arg Ala Leu Gly Ile Ala Gly Ser Leu
           35                  40                  45
   gcg ctc ggc ctc tac tac gcg cgc gcg ctc gcg atc gtg cag gag ttt         192
   Ala Leu Gly Leu Tyr Tyr Ala Arg Ala Leu Ala Ile Val Gln Glu Phe
       50                  55                  60
   gcc ctg ctg gat gcg gtg ctc tgc acg ggg tac att ctg ctg cag ggc         240
   Ala Leu Leu Asp Ala Val Leu Cys Thr Gly Tyr Ile Leu Leu Gln Gly
   65                  70                  75                  80
   atc gta ttc tgg ggg ttc ttc acc atc ggc cat gac tgc ggc cac ggc         288
   Ile Val Phe Trp Gly Phe Phe Thr Ile Gly His Asp Cys Gly His Gly
                   85                  90                  95
   gcg ttc tcg cgt tcg cac ctg ctc aac ttc agc gtc ggc acg ctc att         336
   Ala Phe Ser Arg Ser His Leu Leu Asn Phe Ser Val Gly Thr Leu Ile
               100                 105                 110
   cac tcg atc atc ctc acg ccg tac gag tca tgg aag atc tcg cac cgc         384
   His Ser Ile Ile Leu Thr Pro Tyr Glu Ser Trp Lys Ile Ser His Arg
           115                 120                 125
   cac cac cac aag aac acg ggc aac atc gac aag gac gag att ttc tac         432
   His His His Lys Asn Thr Gly Asn Ile Asp Lys Asp Glu Ile Phe Tyr
       130                 135                 140
   ccg cag cgc gag gcc gac tcg cac cca ctg tcc cga cac atg gtg atc         480
   Pro Gln Arg Glu Ala Asp Ser His Pro Leu Ser Arg His Met Val Ile
   145                 150                 155                 160
   tcg ctc ggc tcg gcc tgg ttc gcg tac ctc gtt gcg ggc ttc cct cct         528
   Ser Leu Gly Ser Ala Trp Phe Ala Tyr Leu Val Ala Gly Phe Pro Pro
                   165                 170                 175
   cgc aag gtg aac cac ttc aac cct tgg gaa ccg ttg tac ctg cgc cgc         576
   Arg Lys Val Asn His Phe Asn Pro Trp Glu Pro Leu Tyr Leu Arg Arg
               180                 185                 190
   atg tct gcc gtc atc atc tca ctc ggc tcg ctc gtg gcg ttc gcg ggc         624
   Met Ser Ala Val Ile Ile Ser Leu Gly Ser Leu Val Ala Phe Ala Gly
           195                 200                 205
   ttg tat gcg tat ctc acc tac gtc tat ggc ctt aag acc atg gcg ctg         672
   Leu Tyr Ala Tyr Leu Thr Tyr Val Tyr Gly Leu Lys Thr Met Ala Leu
       210                 215                 220
   tac tac ttc gcc cct ctc ttt ggg ttc gcc acg atg ctc gtg gtc act         720
   Tyr Tyr Phe Ala Pro Leu Phe Gly Phe Ala Thr Met Leu Val Val Thr
   225                 230                 235                 240
   acc ttt ttg cac cac aat gac gag gaa acg cca tgg tac gcc gac tcg        768
   Thr Phe Leu His His Asn Asp Glu Glu Thr Pro Trp Tyr Ala Asp Ser
                   245                 250                 255
   gag tgg acg tac gtc aag ggc aac ctc tcg tcc gtg gac cgc tcg tac        816
   Glu Trp Thr Tyr Val Lys Gly Asn Leu Ser Ser Val Asp Arg Ser Tyr
               260                 265                 270
   ggc gcg ctc atc gac aac ctg agc cac aac atc ggc acg cac cag atc        864
   Gly Ala Leu Ile Asp Asn Leu Ser His Asn Ile Gly Thr His Gln Ile
           275                 280                 285
   cac cac ctg ttt ccg atc atc ccg cac tac aag ctg aac gag gcg acg        912
   His His Leu Phe Pro Ile Ile Pro His Tyr Lys Leu Asn Glu Ala Thr
       290                 295                 300
   gca gcg ttc gcg cag gcg ttc ccg gag ctc gtg cgc aag agc gcg tcg        960
   Ala Ala Phe Ala Gln Ala Phe Pro Glu Leu Val Arg Lys Ser Ala Ser
   305                 310                 315                 320
   ccg atc atc ccg acg ttc atc cgc atc ggg ctc atg tac gcc aag tac       1008
   Pro Ile Ile Pro Thr Phe Ile Arg Ile Gly Leu Met Tyr Ala Lys Tyr
                   325                 330                 335
   ggc gtc gtg gac aag gac gcc aag atg ttt acg ctc aag gag gcc aag       1056
   Gly Val Val Asp Lys Asp Ala Lys Met Phe Thr Leu Lys Glu Ala Lys
               340                 345                 350
   gcc gcc aag acc aag gcc aac tag                                       1080
   Ala Ala Lys Thr Lys Ala Asn
           355
<210> 88
   <211> 359
   <212> PRT
   <213> Pythium aphanidermatum
<400> 88
   Met Ala Ser Ser Thr Val Ala Ala Pro Tyr Glu Phe Pro Thr Leu Thr
   1               5                   10                  15
   Glu Ile Lys Arg Ser Leu Pro Ala His Cys Phe Glu Ala Ser Val Pro
               20                  25                  30
   Trp Ser Leu Tyr Tyr Thr Val Arg Ala Leu Gly Ile Ala Gly Ser Leu
           35                  40                  45
   Ala Leu Gly Leu Tyr Tyr Ala Arg Ala Leu Ala Ile Val Gln Glu Phe
       50                  55                  60
   Ala Leu Leu Asp Ala Val Leu Cys Thr Gly Tyr Ile Leu Leu Gln Gly
   65                  70                  75                  80
   Ile Val Phe Trp Gly Phe Phe Thr Ile Gly His Asp Cys Gly His Gly
                   85                  90                  95
   Ala Phe Ser Arg Ser His Leu Leu Asn Phe Ser Val Gly Thr Leu Ile
               100                 105                 110
   His Ser Ile Ile Leu Thr Pro Tyr Glu Ser Trp Lys Ile Ser His Arg
           115                 120                 125
   His His His Lys Asn Thr Gly Asn Ile Asp Lys Asp Glu Ile Phe Tyr
       130                 135                 140
   Pro Gln Arg Glu Ala Asp Ser His Pro Leu Ser Arg His Met Val Ile
   145                 150                 155                 160
   Ser Leu Gly Ser Ala Trp Phe Ala Tyr Leu Val Ala Gly Phe Pro Pro
                   165                 170                 175
   Arg Lys Val Asn His Phe Asn Pro Trp Glu Pro Leu Tyr Leu Arg Arg
               180                 185                 190
   Met Ser Ala Val Ile Ile Ser Leu Gly Ser Leu Val Ala Phe Ala Gly
           195                 200                 205
   Leu Tyr Ala Tyr Leu Thr Tyr Val Tyr Gly Leu Lys Thr Met Ala Leu
       210                 215                 220
   Tyr Tyr Phe Ala Pro Leu Phe Gly Phe Ala Thr Met Leu Val Val Thr
   225                 230                 235                 240
   Thr Phe Leu His His Asn Asp Glu Glu Thr Pro Trp Tyr Ala Asp Ser
                   245                 250                 255
   Glu Trp Thr Tyr Val Lys Gly Asn Leu Ser Ser Val Asp Arg Ser Tyr
               260                 265                 270
   Gly Ala Leu Ile Asp Asn Leu Ser His Asn Ile Gly Thr His Gln Ile
           275                 280                 285
   His His Leu Phe Pro Ile Ile Pro His Tyr Lys Leu Asn Glu Ala Thr
       290                 295                 300
   Ala Ala Phe Ala Gln Ala Phe Pro Glu Leu Val Arg Lys Ser Ala Ser
   305                 310                 315                 320
   Pro Ile Ile Pro Thr Phe Ile Arg Ile Gly Leu Met Tyr Ala Lys Tyr
                   325                 330                 335
   Gly Val Val Asp Lys Asp Ala Lys Met Phe Thr Leu Lys Glu Ala Lys
               340                 345                 350
   Ala Ala Lys Thr Lys Ala Asn
   355
<210> 89
   <211> 1080
   <212> DNA
   <213> Pythium aphanidermatum
<220>
   <221> CDS
   <222> (1)..(1080)
   <223> synthetic delta-17 desaturase (codon-optimized for Yarrowia lipolytica)
<300>
   <302> DELTA-17 DESATURASES AND THEIR USE IN MAKING POLYUNSATURATED FATTY ACIDS
<310> WO 2008/054565
   <311> 2007-07-19
   <312> 2008-05-08
   <313> (1)..(1080)
<300>
   <302> DELTA-17 DESATURASES AND THEIR USE IN MAKING POLYUNSATURATED FATTY ACIDS
<310> US 2008-0125326-A1
   <311> 2007-07-19
   <312> 2008-05-29
   <313> (1)..(1080)
<400> 89
   atg gct tcc tct acc gtt gcc gct ccc tac gag ttc cct act ctc acc       48
   Met Ala Ser Ser Thr Val Ala Ala Pro Tyr Glu Phe Pro Thr Leu Thr
   1               5                   10                  15
   gag atc aag cga tcc ctg cct gcc cac tgc ttc gaa gcc tct gtt ccc       96
   Glu Ile Lys Arg Ser Leu Pro Ala His Cys Phe Glu Ala Ser Val Pro
               20                  25                  30
   tgg tcc ctc tac tat acc gtg cga gct ctg ggc att gcc ggt tcc ctt      144
   Trp Ser Leu Tyr Tyr Thr Val Arg Ala Leu Gly Ile Ala Gly Ser Leu
           35                  40                  45
   gct ctc gga ctg tac tat gct cga gcc ctt gct atc gtg cag gag ttt      192
   Ala Leu Gly Leu Tyr Tyr Ala Arg Ala Leu Ala Ile Val Gln Glu Phe
       50                  55                  60
   gca ctg ctc gat gcc gtc ctt tgc act ggc tac att ctg ctc cag ggt      240
   Ala Leu Leu Asp Ala Val Leu Cys Thr Gly Tyr Ile Leu Leu Gln Gly
   65                  70                  75                  80
   atc gtg ttc tgg gga ttc ttt acc atc ggt cac gac tgt gga cat ggt      288
   Ile Val Phe Trp Gly Phe Phe Thr Ile Gly His Asp Cys Gly His Gly
                   85                  90                  95
   gcc ttc tcg cga tcc cac ctg ctc aac ttc tct gtt ggc aca ctc att      336
   Ala Phe Ser Arg Ser His Leu Leu Asn Phe Ser Val Gly Thr Leu Ile
               100                 105                 110
   cac tcc atc att ctg act ccc tac gag tcg tgg aag atc agc cat cga      384
   His Ser Ile Ile Leu Thr Pro Tyr Glu Ser Trp Lys Ile Ser His Arg
           115                 120                 125
   cac cat cac aag aac acc ggc aac atc gac aag gat gag atc ttc tac         432
   His His His Lys Asn Thr Gly Asn Ile Asp Lys Asp Glu Ile Phe Tyr
       130                 135                 140
   cct cag cga gaa gcc gac tct cat ccc ctg tcc cga cac atg gtc atc         480
   Pro Gln Arg Glu Ala Asp Ser His Pro Leu Ser Arg His Met Val Ile
   145                 150                 155                 160
   tcc ctt ggt tcg gct tgg ttt gcc tac ctc gtt gct gga ttt cct ccc         528
   Ser Leu Gly Ser Ala Trp Phe Ala Tyr Leu Val Ala Gly Phe Pro Pro
                   165                 170                 175
   cga aag gtc aac cac ttc aat ccc tgg gag cct ctc tac ctg cga aga         576
   Arg Lys Val Asn His Phe Asn Pro Trp Glu Pro Leu Tyr Leu Arg Arg
               180                 185                 190
   atg tct gcc gtc atc att tcc ctc ggc tct ctc gtg gcc ttt gct ggt         624
   Met Ser Ala Val Ile Ile Ser Leu Gly Ser Leu Val Ala Phe Ala Gly
           195                 200                 205
   ctg tac gcc tac ctt acc tac gtc tac ggc ctc aag acc atg gct ctg         672
   Leu Tyr Ala Tyr Leu Thr Tyr Val Tyr Gly Leu Lys Thr Met Ala Leu
       210                 215                 220
   tat tac ttc gca cct ctc ttt gga ttc gcc acc atg ctg gtt gtc act         720
   Tyr Tyr Phe Ala Pro Leu Phe Gly Phe Ala Thr Met Leu Val Val Thr
   225                 230                 235                 240
   acc ttc ctc cat cac aac gac gag gaa act ccc tgg tac gcc gat tcg         768
   Thr Phe Leu His His Asn Asp Glu Glu Thr Pro Trp Tyr Ala Asp Ser
                   245                 250                 255
   gag tgg acc tat gtc aag ggc aac ttg tcc tct gtg gac cga agc tac         816
   Glu Trp Thr Tyr Val Lys Gly Asn Leu Ser Ser Val Asp Arg Ser Tyr
               260                 265                 270
   gga gcc ctc atc gac aac ctg tcc cac aac att ggt aca cat cag atc         864
   Gly Ala Leu Ile Asp Asn Leu Ser His Asn Ile Gly Thr His Gln Ile
           275                 280                 285
   cac cat ctg ttt ccc atc att cct cac tac aag ctc aac gag gcc act         912
   His His Leu Phe Pro Ile Ile Pro His Tyr Lys Leu Asn Glu Ala Thr
       290                 295                 300
   gct gcc ttc gct cag gcc ttt ccc gaa ctg gtg cga aag tcg gct tct         960
   Ala Ala Phe Ala Gln Ala Phe Pro Glu Leu Val Arg Lys Ser Ala Ser
   305                 310                 315                 320
   ccc atc att ccc acc ttc atc cga att ggt ctt atg tac gcc aag tac        1008
   Pro Ile Ile Pro Thr Phe Ile Arg Ile Gly Leu Met Tyr Ala Lys Tyr
                   325                 330                 335
   ggc gtg gtc gac aag gat gcc aag atg ttt acc ctc aag gag gcc aag        1056
   Gly Val Val Asp Lys Asp Ala Lys Met Phe Thr Leu Lys Glu Ala Lys
               340                 345                 350
   gct gcc aag acc aaa gcc aac taa                                        1080
   Ala Ala Lys Thr Lys Ala Asn
           355
<210> 90
   <211> 359
   <212> PRT
   <213> Pythium aphanidermatum
<400> 90
   Met Ala Ser Ser Thr Val Ala Ala Pro Tyr Glu Phe Pro Thr Leu Thr
   1               5                   10                  15
   Glu Ile Lys Arg Ser Leu Pro Ala His Cys Phe Glu Ala Ser Val Pro
               20                  25                  30
   Trp Ser Leu Tyr Tyr Thr Val Arg Ala Leu Gly Ile Ala Gly Ser Leu
           35                  40                  45
   Ala Leu Gly Leu Tyr Tyr Ala Arg Ala Leu Ala Ile Val Gln Glu Phe
       50                  55                  60
   Ala Leu Leu Asp Ala Val Leu Cys Thr Gly Tyr Ile Leu Leu Gln Gly
   65                  70                  75                  80
   Ile Val Phe Trp Gly Phe Phe Thr Ile Gly His Asp Cys Gly His Gly
                   85                  90                  95
   Ala Phe Ser Arg Ser His Leu Leu Asn Phe Ser Val Gly Thr Leu Ile
               100                 105                 110
   His Ser Ile Ile Leu Thr Pro Tyr Glu Ser Trp Lys Ile Ser His Arg
           115                 120                 125
   His His His Lys Asn Thr Gly Asn Ile Asp Lys Asp Glu Ile Phe Tyr
       130                 135                 140
   Pro Gln Arg Glu Ala Asp Ser His Pro Leu Ser Arg His Met Val Ile
   145                 150                 155                 160
   Ser Leu Gly Ser Ala Trp Phe Ala Tyr Leu Val Ala Gly Phe Pro Pro
                   165                 170                 175
   Arg Lys Val Asn His Phe Asn Pro Trp Glu Pro Leu Tyr Leu Arg Arg
               180                 185                 190
   Met Ser Ala Val Ile Ile Ser Leu Gly Ser Leu Val Ala Phe Ala Gly
           195                 200                 205
   Leu Tyr Ala Tyr Leu Thr Tyr Val Tyr Gly Leu Lys Thr Met Ala Leu
       210                 215                 220
   Tyr Tyr Phe Ala Pro Leu Phe Gly Phe Ala Thr Met Leu Val Val Thr
   225                 230                 235                 240
   Thr Phe Leu His His Asn Asp Glu Glu Thr Pro Trp Tyr Ala Asp Ser
                   245                 250                 255
   Glu Trp Thr Tyr Val Lys Gly Asn Leu Ser Ser Val Asp Arg Ser Tyr
               260                 265                 270
   Gly Ala Leu Ile Asp Asn Leu Ser His Asn Ile Gly Thr His Gln Ile
           275                 280                 285
   His His Leu Phe Pro Ile Ile Pro His Tyr Lys Leu Asn Glu Ala Thr
       290                 295                 300
   Ala Ala Phe Ala Gln Ala Phe Pro Glu Leu Val Arg Lys Ser Ala Ser
   305                 310                 315                 320
   Pro Ile Ile Pro Thr Phe Ile Arg Ile Gly Leu Met Tyr Ala Lys Tyr
                   325                 330                 335
   Gly Val Val Asp Lys Asp Ala Lys Met Phe Thr Leu Lys Glu Ala Lys
               340                 345                 350
   Ala Ala Lys Thr Lys Ala Asn
           355
<210> 91
   <211> 1434
   <212> DNA
   <213> Fusarium monoliforme
<220>
   <221> CDS
   <222> (1)..(1434)
   <223> delta-12 desaturase
<300>
   <302> DELTA-12 DESATURASES SUITABLE FOR ALTERING LEVELS OF POLYUNSATURATED FATTY ACIDS IN OLEAGINOUS YEAST
<310> WO 2005/047485
   <311> 2004-11-12
   <312> 2005-05-26
   <313> (1)..(1434)
<300>
   <302> DELTA-12 DESATURASES SUITABLE FOR ALTERING LEVELS OF POLYUNSATURATED FATTY ACIDS IN OLEAGINOUS YEAST
<310> U.S. 7,504,259
   <311> 2004-11-10
   <312> 2009-03-17
   <313> (1)..(1434)
<400> 91
   atg gcg tcc act tcg gct ctg ccc aag cag aac cct gcg ctt aga cgc          48
   Met Ala Ser Thr Ser Ala Leu Pro Lys Gln Asn Pro Ala Leu Arg Arg
   1               5                   10                  15
   acc gtc acc tca act act gtg acg gat tct gag tct gcc gcc gtc tct          96
   Thr Val Thr Ser Thr Thr Val Thr Asp Ser Glu Ser Ala Ala Val Ser
               20                  25                  30
   cct tca gac tct ccc cgc cac tcg gcc tct tcc aca tcg ctc tcg tcc      144
   Pro Ser Asp Ser Pro Arg His Ser Ala Ser Ser Thr Ser Leu Ser Ser
           35                  40                  45
   atg tcc gag gtt gat atc gcc aag ccc aag tcc gag tat ggt gtc atg      192
   Met Ser Glu Val Asp Ile Ala Lys Pro Lys Ser Glu Tyr Gly Val Met
       50                  55                  60
   ctc gac acc tac ggc aac cag ttc gag gtt ccc gac ttt acc atc aag      240
   Leu Asp Thr Tyr Gly Asn Gln Phe Glu Val Pro Asp Phe Thr Ile Lys
   65                  70                  75                  80
   gac atc tac aat gcc atc cct aag cac tgc ttc aag cgc tcc gct ctc      288
   Asp Ile Tyr Asn Ala Ile Pro Lys His Cys Phe Lys Arg Ser Ala Leu
                   85                  90                  95
   aag gga tac ggt tat atc ctc cgc gac att gtc ctc ctg act acc act      336
   Lys Gly Tyr Gly Tyr Ile Leu Arg Asp Ile Val Leu Leu Thr Thr Thr
               100                 105                 110
   ttc agc atc tgg tac aac ttt gtg acc ccc gaa tat atc ccc tcc acc      384
   Phe Ser Ile Trp Tyr Asn Phe Val Thr Pro Glu Tyr Ile Pro Ser Thr
           115                 120                 125
   ccc gcc cgc gct ggt ctg tgg gcc gtg tac acc gtt ctt cag ggt ctt      432
   Pro Ala Arg Ala Gly Leu Trp Ala Val Tyr Thr Val Leu Gln Gly Leu
       130                 135                 140
   ttc ggt act ggt ctc tgg gtt att gcc cat gag tgc ggt cac ggt gct      480
   Phe Gly Thr Gly Leu Trp Val Ile Ala His Glu Cys Gly His Gly Ala
   145                 150                 155                 160
   ttc tcc gat tct cgc atc atc aac gac att act ggc tgg gtt ctt cac      528
   Phe Ser Asp Ser Arg Ile Ile Asn Asp Ile Thr Gly Trp Val Leu His
                   165                 170                 175
   tct tcc ctc ctt gtc ccc tac ttc agc tgg caa atc tcc cac cga aag      576
   Ser Ser Leu Leu Val Pro Tyr Phe Ser Trp Gln Ile Ser His Arg Lys
               180                 185                 190
   cac cac aag gcc act ggc aac atg gag cgt gac atg gtc ttc gtt ccc      624
   His His Lys Ala Thr Gly Asn Met Glu Arg Asp Met Val Phe Val Pro
           195                 200                 205
   cga acc cgc gag cag cag gct act cgt ctc gga aag atg acc cac gag      672
   Arg Thr Arg Glu Gln Gln Ala Thr Arg Leu Gly Lys Met Thr His Glu
       210                 215                 220
   ctc gct cat ctt act gag gag acc ccc gct ttc act ctt ctc atg ctc      720
   Leu Ala His Leu Thr Glu Glu Thr Pro Ala Phe Thr Leu Leu Met Leu
   225                 230                 235                 240
   gtc ctt cag cag ctc gtt ggc tgg ccc aac tac ctc atc acc aat gtt      768
   Val Leu Gln Gln Leu Val Gly Trp Pro Asn Tyr Leu Ile Thr Asn Val
                   245                 250                 255
   acc ggc cac aac tac cac gag cgc cag cgt gag ggt cgc ggc aag ggc      816
   Thr Gly His Asn Tyr His Glu Arg Gln Arg Glu Gly Arg Gly Lys Gly
               260                 265                 270
   aag cat aac ggc ctc ggc ggt ggt gtt aac cac ttc gat ccc cgc agc      864
   Lys His Asn Gly Leu Gly Gly Gly Val Asn His Phe Asp Pro Arg Ser
           275                 280                 285
   cct ctg tac gag aac agt gac gct aag ctc atc gtc ctc agc gat att        912
   Pro Leu Tyr Glu Asn Ser Asp Ala Lys Leu Ile Val Leu Ser Asp Ile
       290                 295                 300
   ggt atc ggt ctg atg gcc act gct ctg tac ttc ctc gtt cag aag ttc        960
   Gly Ile Gly Leu Met Ala Thr Ala Leu Tyr Phe Leu Val Gln Lys Phe
   305                 310                 315                 320
   ggt ttc tac aac atg gcc atc tgg tac ttt gtt ccc tac ctc tgg gtt       1008
   Gly Phe Tyr Asn Met Ala Ile Trp Tyr Phe Val Pro Tyr Leu Trp Val
                   325                 330                 335
   aac cac tgg ctc gtt gcc atc acc ttc ctc cag cac acc gac cct acc       1056
   Asn His Trp Leu Val Ala Ile Thr Phe Leu Gln His Thr Asp Pro Thr
               340                 345                 350
   ctt ccc cac tac acc aac gac gag tgg aac ttc gtc cgt ggt gcc gct       1104
   Leu Pro His Tyr Thr Asn Asp Glu Trp Asn Phe Val Arg Gly Ala Ala
           355                 360                 365
   gct acc att gac cgt gag atg ggc ttc atc ggc cgc cac ctt ctc cac       1152
   Ala Thr Ile Asp Arg Glu Met Gly Phe Ile Gly Arg His Leu Leu His
       370                 375                 380
   ggc atc atc gag act cat gtc ctc cac cac tac gtc agc agc atc ccc       1200
   Gly Ile Ile Glu Thr His Val Leu His His Tyr Val Ser Ser Ile Pro
   385                 390                 395                 400
   ttc tac aac gcg gac gag gcc acc gag gcc att aag ccc atc atg ggc       1248
   Phe Tyr Asn Ala Asp Glu Ala Thr Glu Ala Ile Lys Pro Ile Met Gly
                   405                 410                 415
   aag cac tac cgg gct gat gtc cag gat ggt cct cgt ggc ttc atc cgc       1296
   Lys His Tyr Arg Ala Asp Val Gln Asp Gly Pro Arg Gly Phe Ile Arg
               420                 425                 430
   gcc atg tac cgc agt gcg cgt atg tgc cag tgg gtt gag ccc agc gct       1344
   Ala Met Tyr Arg Ser Ala Arg Met Cys Gln Trp Val Glu Pro Ser Ala
           435                 440                 445
   ggt gcc gag ggt gct ggt aag ggt gtt ctg ttc ttc cgc aac cgc aac       1392
   Gly Ala Glu Gly Ala Gly Lys Gly Val Leu Phe Phe Arg Asn Arg Asn
       450                 455                 460
   aac gtg ggc acc ccc ccc gct gtt atc aag ccc gtt gct taa               1434
   Asn Val Gly Thr Pro Pro Ala Val Ile Lys Pro Val Ala
   465                 470                 475
<210> 92
   <211> 477
   <212> PRT
   <213> Fusarium monoliforme
<400> 92
   Met Ala Ser Thr Ser Ala Leu Pro Lys Gln Asn Pro Ala Leu Arg Arg
   1               5                   10                  15
   Thr Val Thr Ser Thr Thr Val Thr Asp Ser Glu Ser Ala Ala Val Ser
               20                  25                  30
   Pro Ser Asp Ser Pro Arg His Ser Ala Ser Ser Thr Ser Leu Ser Ser
           35                  40                  45
   Met Ser Glu Val Asp Ile Ala Lys Pro Lys Ser Glu Tyr Gly Val Met
       50                  55                  60
   Leu Asp Thr Tyr Gly Asn Gln Phe Glu Val Pro Asp Phe Thr Ile Lys
   65                  70                  75                  80
   Asp Ile Tyr Asn Ala Ile Pro Lys His Cys Phe Lys Arg Ser Ala Leu
                   85                  90                  95
   Lys Gly Tyr Gly Tyr Ile Leu Arg Asp Ile Val Leu Leu Thr Thr Thr
               100                 105                 110
   Phe Ser Ile Trp Tyr Asn Phe Val Thr Pro Glu Tyr Ile Pro Ser Thr
           115                 120                 125
   Pro Ala Arg Ala Gly Leu Trp Ala Val Tyr Thr Val Leu Gln Gly Leu
       130                 135                 140
   Phe Gly Thr Gly Leu Trp Val Ile Ala His Glu Cys Gly His Gly Ala
   145                 150                 155                 160
   Phe Ser Asp Ser Arg Ile Ile Asn Asp Ile Thr Gly Trp Val Leu His
                   165                 170                 175
   Ser Ser Leu Leu Val Pro Tyr Phe Ser Trp Gln Ile Ser His Arg Lys
               180                 185                 190
   His His Lys Ala Thr Gly Asn Met Glu Arg Asp Met Val Phe Val Pro
           195                 200                 205
   Arg Thr Arg Glu Gln Gln Ala Thr Arg Leu Gly Lys Met Thr His Glu
       210                 215                 220
   Leu Ala His Leu Thr Glu Glu Thr Pro Ala Phe Thr Leu Leu Met Leu
   225                 230                 235                 240
   Val Leu Gln Gln Leu Val Gly Trp Pro Asn Tyr Leu Ile Thr Asn Val
                   245                 250                 255
   Thr Gly His Asn Tyr His Glu Arg Gln Arg Glu Gly Arg Gly Lys Gly
               260                 265                 270
   Lys His Asn Gly Leu Gly Gly Gly Val Asn His Phe Asp Pro Arg Ser
           275                 280                 285
   Pro Leu Tyr Glu Asn Ser Asp Ala Lys Leu Ile Val Leu Ser Asp Ile
       290                 295                 300
   Gly Ile Gly Leu Met Ala Thr Ala Leu Tyr Phe Leu Val Gln Lys Phe
   305                 310                 315                 320
   Gly Phe Tyr Asn Met Ala Ile Trp Tyr Phe Val Pro Tyr Leu Trp Val
                   325                 330                 335
   Asn His Trp Leu Val Ala Ile Thr Phe Leu Gln His Thr Asp Pro Thr
               340                 345                 350
   Leu Pro His Tyr Thr Asn Asp Glu Trp Asn Phe Val Arg Gly Ala Ala
           355                 360                 365
   Ala Thr Ile Asp Arg Glu Met Gly Phe Ile Gly Arg His Leu Leu His
       370                 375                 380
   Gly Ile Ile Glu Thr His Val Leu His His Tyr Val Ser Ser Ile Pro
   385                 390                 395                 400
   Phe Tyr Asn Ala Asp Glu Ala Thr Glu Ala Ile Lys Pro Ile Met Gly
                   405                 410                 415
   Lys His Tyr Arg Ala Asp Val Gln Asp Gly Pro Arg Gly Phe Ile Arg
               420                 425                 430
   Ala Met Tyr Arg Ser Ala Arg Met Cys Gln Trp Val Glu Pro Ser Ala
           435                 440                 445
   Gly Ala Glu Gly Ala Gly Lys Gly Val Leu Phe Phe Arg Asn Arg Asn
       450                 455                 460
   Asn Val Gly Thr Pro Pro Ala Val Ile Lys Pro Val Ala
   465                 470                 475
<210> 93
   <211> 1434
   <212> DNA
   <213> Fusarium moniliforme
<220>
   <221> CDS
   <222> (1)..(1434)
   <223> synthetic delta-12 desaturase (codon-optimized for Yarrowia lipolytica)
<300>
   <302> DELTA-12 DESATURASES SUITABLE FOR ALTERING LEVELS OF POLYUNSATURATED FATTY ACIDS IN OLEAGINOUS YEAST
<310> WO 2005/047485
   <311> 2004-11-12
   <312> 2005-05-26
   <313> (1)..(1434)
<300>
   <302> DELTA-12 DESATURASES SUITABLE FOR ALTERING LEVELS OF POLYUNSATURATED FATTY ACIDS IN OLEAGINOUS YEAST
<310> U.S. 7,504,259
   <311> 2004-11-10
   <312> 2009-03-17
   <313> (1)..(1434)
<400> 93
   atg gcc tcc acc tcg gct ctg ccc aag cag aac cct gcc ctc cga cga 48
   Met Ala Ser Thr Ser Ala Leu Pro Lys Gln Asn Pro Ala Leu Arg Arg
   1 5 10 15
   acc gtc act tcc acc act gtg acc gac tcg gag tct gct gcc gtc tct 96
   Thr Val Thr Ser Thr Thr Val Thr Asp Ser Glu Ser Ala Ala Val Ser
   20 25 30
   ccc tcc gat tct ccc aga cac tcg gcc tcc tct aca tcg ctg tct tcc 144
   Pro Ser Asp Ser Pro Arg His Ser Ala Ser Ser Thr Ser Leu Ser Ser
   35 40 45
   atg tcc gag gtg gac att gcc aag ccc aag tcc gag tac ggt gtc atg 192
   Met Ser Glu Val Asp Ile Ala Lys Pro Lys Ser Glu Tyr Gly Val Met
   50 55 60
   ctg gat acc tac ggc aac cag ttc gaa gtt ccc gac ttc acc atc aag 240
   Leu Asp Thr Tyr Gly Asn Gln Phe Glu Val Pro Asp Phe Thr Ile Lys
   65 70 75 80
   gac atc tac aac gct att ccc aag cac tgc ttc aag cga tct gct ctc 288
   Asp Ile Tyr Asn Ala Ile Pro Lys His Cys Phe Lys Arg Ser Ala Leu
   85 90 95
   aag gga tac ggc tac att ctt cga gac att gtc ctc ctg act acc act 336
   Lys Gly Tyr Gly Tyr Ile Leu Arg Asp Ile Val Leu Leu Thr Thr Thr
   100 105 110
   ttc agc atc tgg tac aac ttt gtg aca ccc gag tac att ccc tcc act 384
   Phe Ser Ile Trp Tyr Asn Phe Val Thr Pro Glu Tyr Ile Pro Ser Thr
   115 120 125
   cct gct cga gcc ggt ctg tgg gct gtg tac acc gtt ctt cag gga ctc 432
   Pro Ala Arg Ala Gly Leu Trp Ala Val Tyr Thr Val Leu Gln Gly Leu
   130 135 140
   ttc ggt act gga ctg tgg gtc att gcc cac gag tgt gga cat ggt gct 480
   Phe Gly Thr Gly Leu Trp Val Ile Ala His Glu Cys Gly His Gly Ala
   145 150 155 160
   ttc tcc gat tcc cga atc atc aac gac att act ggc tgg gtg ctt cac 528
   Phe Ser Asp Ser Arg Ile Ile Asn Asp Ile Thr Gly Trp Val Leu His
   165 170 175
   tct tcc ctg ctt gtt ccc tac ttc agc tgg caa atc tcc cac cgg aag 576
   Ser Ser Leu Leu Val Pro Tyr Phe Ser Trp Gln Ile Ser His Arg Lys
   180 185 190
   cat cac aag gcc act gga aac atg gag cga gac atg gtc ttc gtt cct 624
   His His Lys Ala Thr Gly Asn Met Glu Arg Asp Met Val Phe Val Pro
   195 200 205
   cga acc cga gag cag caa gct act cga ctc ggc aag atg acc cac gaa 672
   Arg Thr Arg Glu Gln Gln Ala Thr Arg Leu Gly Lys Met Thr His Glu
       210                 215                 220
   ctc gcc cat ctt acc gag gaa act cct gct ttc acc ctg ctc atg ctt        720
   Leu Ala His Leu Thr Glu Glu Thr Pro Ala Phe Thr Leu Leu Met Leu
   225                 230                 235                 240
   gtg ctt cag caa ctg gtc ggt tgg ccc aac tat ctc att acc aac gtt        768
   Val Leu Gln Gln Leu Val Gly Trp Pro Asn Tyr Leu Ile Thr Asn Val
                   245                 250                 255
   act gga cac aac tac cat gag cgg cag cga gag ggt cga ggc aag gga        816
   Thr Gly His Asn Tyr His Glu Arg Gln Arg Glu Gly Arg Gly Lys Gly
               260                 265                 270
   aag cac aac ggt ctt ggc ggt gga gtt aac cat ttc gat ccc cga tct        864
   Lys His Asn Gly Leu Gly Gly Gly Val Asn His Phe Asp Pro Arg Ser
           275                 280                 285
   cct ctg tac gag aac agc gac gcc aag ctc atc gtg ctc tcc gac att        912
   Pro Leu Tyr Glu Asn Ser Asp Ala Lys Leu Ile Val Leu Ser Asp Ile
       290                 295                 300
   ggc att ggt ctt atg gcc acc gct ctg tac ttt ctc gtt cag aag ttc        960
   Gly Ile Gly Leu Met Ala Thr Ala Leu Tyr Phe Leu Val Gln Lys Phe
   305                 310                 315                 320
   gga ttc tac aac atg gcc atc tgg tac ttc gtt ccc tac ttg tgg gtt       1008
   Gly Phe Tyr Asn Met Ala Ile Trp Tyr Phe Val Pro Tyr Leu Trp Val
                   325                 330                 335
   aac cac tgg ctc gtc gcc att acc ttt ctg cag cac aca gat cct act       1056
   Asn His Trp Leu Val Ala Ile Thr Phe Leu Gln His Thr Asp Pro Thr
               340                 345                 350
   ctt ccc cac tac acc aac gac gag tgg aac ttt gtg cga ggt gcc gct       1104
   Leu Pro His Tyr Thr Asn Asp Glu Trp Asn Phe Val Arg Gly Ala Ala
           355                 360                 365
   gca acc atc gac cga gag atg ggc ttc att gga cgt cat ctg ctc cac       1152
   Ala Thr Ile Asp Arg Glu Met Gly Phe Ile Gly Arg His Leu Leu His
       370                 375                 380
   ggc att atc gag act cac gtc ctg cat cac tac gtc tct tcc att ccc       1200
   Gly Ile Ile Glu Thr His Val Leu His His Tyr Val Ser Ser Ile Pro
   385                 390                 395                 400
   ttc tac aat gcg gac gaa gct acc gag gcc atc aaa cct atc atg ggc       1248
   Phe Tyr Asn Ala Asp Glu Ala Thr Glu Ala Ile Lys Pro Ile Met Gly
                   405                 410                 415
   aag cac tat cga gct gat gtc cag gac ggt cct cga gga ttc att cga       1296
   Lys His Tyr Arg Ala Asp Val Gln Asp Gly Pro Arg Gly Phe Ile Arg
               420                 425                 430
   gcc atg tac cga tct gca cga atg tgc cag tgg gtt gaa ccc tcc gct       1344
   Ala Met Tyr Arg Ser Ala Arg Met Cys Gln Trp Val Glu Pro Ser Ala
           435                 440                 445
   ggt gcc gag gga gct ggc aag ggt gtc ctg ttc ttt cga aac cga aac       1392
   Gly Ala Glu Gly Ala Gly Lys Gly Val Leu Phe Phe Arg Asn Arg Asn
       450                 455                 460
   aat gtg ggc act cct ccc gct gtc atc aag ccc gtt gcc taa               1434
   Asn Val Gly Thr Pro Pro Ala Val Ile Lys Pro Val Ala
   465                 470                 475
<210> 94
   <211> 477
   <212> PRT
   <213> Fusarium moniliforme
<400> 94
   Met Ala Ser Thr Ser Ala Leu Pro Lys Gln Asn Pro Ala Leu Arg Arg
   1               5                   10                  15
   Thr Val Thr Ser Thr Thr Val Thr Asp Ser Glu Ser Ala Ala Val Ser
               20                  25                  30
   Pro Ser Asp Ser Pro Arg His Ser Ala Ser Ser Thr Ser Leu Ser Ser
           35                  40                  45
   Met Ser Glu Val Asp Ile Ala Lys Pro Lys Ser Glu Tyr Gly Val Met
       50                  55                  60
   Leu Asp Thr Tyr Gly Asn Gln Phe Glu Val Pro Asp Phe Thr Ile Lys
   65                  70                  75                  80
   Asp Ile Tyr Asn Ala Ile Pro Lys His Cys Phe Lys Arg Ser Ala Leu
                   85                  90                  95
   Lys Gly Tyr Gly Tyr Ile Leu Arg Asp Ile Val Leu Leu Thr Thr Thr
               100                 105                 110
   Phe Ser Ile Trp Tyr Asn Phe Val Thr Pro Glu Tyr Ile Pro Ser Thr
           115                 120                 125
   Pro Ala Arg Ala Gly Leu Trp Ala Val Tyr Thr Val Leu Gln Gly Leu
       130                 135                 140
   Phe Gly Thr Gly Leu Trp Val Ile Ala His Glu Cys Gly His Gly Ala
   145                 150                 155                 160
   Phe Ser Asp Ser Arg Ile Ile Asn Asp Ile Thr Gly Trp Val Leu His
                   165                 170                 175
   Ser Ser Leu Leu Val Pro Tyr Phe Ser Trp Gln Ile Ser His Arg Lys
               180                 185                 190
   His His Lys Ala Thr Gly Asn Met Glu Arg Asp Met Val Phe Val Pro
           195                 200                 205
   Arg Thr Arg Glu Gln Gln Ala Thr Arg Leu Gly Lys Met Thr His Glu
       210                 215                 220
   Leu Ala His Leu Thr Glu Glu Thr Pro Ala Phe Thr Leu Leu Met Leu
   225                 230                 235                 240
   Val Leu Gln Gln Leu Val Gly Trp Pro Asn Tyr Leu Ile Thr Asn Val
                   245                 250                 255
   Thr Gly His Asn Tyr His Glu Arg Gln Arg Glu Gly Arg Gly Lys Gly
               260                 265                 270
   Lys His Asn Gly Leu Gly Gly Gly Val Asn His Phe Asp Pro Arg Ser
           275                 280                 285
   Pro Leu Tyr Glu Asn Ser Asp Ala Lys Leu Ile Val Leu Ser Asp Ile
       290                 295                 300
   Gly Ile Gly Leu Met Ala Thr Ala Leu Tyr Phe Leu Val Gln Lys Phe
   305                 310                 315                 320
   Gly Phe Tyr Asn Met Ala Ile Trp Tyr Phe Val Pro Tyr Leu Trp Val
                   325                 330                 335
   Asn His Trp Leu Val Ala Ile Thr Phe Leu Gln His Thr Asp Pro Thr
               340                 345                 350
   Leu Pro His Tyr Thr Asn Asp Glu Trp Asn Phe Val Arg Gly Ala Ala
           355                 360                 365
   Ala Thr Ile Asp Arg Glu Met Gly Phe Ile Gly Arg His Leu Leu His
       370                 375                 380
   Gly Ile Ile Glu Thr His Val Leu His His Tyr Val Ser Ser Ile Pro
   385                 390                 395                 400
   Phe Tyr Asn Ala Asp Glu Ala Thr Glu Ala Ile Lys Pro Ile Met Gly
                   405                 410                 415
   Lys His Tyr Arg Ala Asp Val Gln Asp Gly Pro Arg Gly Phe Ile Arg
               420                 425                 430
   Ala Met Tyr Arg Ser Ala Arg Met Cys Gln Trp Val Glu Pro Ser Ala
           435                 440                 445
   Gly Ala Glu Gly Ala Gly Lys Gly Val Leu Phe Phe Arg Asn Arg Asn
       450                 455                 460
   Asn Val Gly Thr Pro Pro Ala Val Ile Lys Pro Val Ala
   465                 470                 475
<210> 95
   <211> 828
   <212> DNA
   <213> Mortierella alpina
<220>
   <221> CDS
   <222> (1)..(828)
   <223> C16/18 elongase
<300>
   <302> A MORTIERELLA ALPINA C16/18 FATTY ACID ELONGASE
<310> WO 2007/046817
   <311> 2005-11-04
   <312> 2007-04-26
   <313> (1)..(828)
<300>
   <302> A MORTIERELLA ALPINA C16/18 FATTY ACID ELONGASE
<310> US 2007-0087420-A1
   <311> 2005-10-19
   <312> 2007-04-19
   <313> (1)..(828)
<400> 95
   atg gag tct gga cct atg cct gcc ggg atc ccc ttc cct gaa tac tat        48
   Met Glu Ser Gly Pro Met Pro Ala Gly Ile Pro Phe Pro Glu Tyr Tyr
   1               5                   10                  15
   gac ttt ttc atg gac tgg aag aca ccc ctg gca att gct gcc acc tac        96
   Asp Phe Phe Met Asp Trp Lys Thr Pro Leu Ala Ile Ala Ala Thr Tyr
               20                  25                  30
   acc gcc gct gtt ggg ctc ttc aac ccc aag gtt ggc aaa gtc tcg cgc       144
   Thr Ala Ala Val Gly Leu Phe Asn Pro Lys Val Gly Lys Val Ser Arg
           35                  40                  45
   gtg gta gcc aag tcg gct aac gcc aag ccg gca gag cgc acg cag tcc       192
   Val Val Ala Lys Ser Ala Asn Ala Lys Pro Ala Glu Arg Thr Gln Ser
       50                  55                  60
   ggc gcc gcc atg acc gcc ttt gtc ttt gtc cac aac ctt atc ctc tgc       240
   Gly Ala Ala Met Thr Ala Phe Val Phe Val His Asn Leu Ile Leu Cys
   65                  70                  75                  80
   gtg tac tct gga atc acc ttc tac tac atg ttc cca gcc atg gtc aag       288
   Val Tyr Ser Gly Ile Thr Phe Tyr Tyr Met Phe Pro Ala Met Val Lys
                   85                  90                  95
   aac ttt aga aca cat acc ctc cat gag gcc tac tgc gat acg gat cag       336
   Asn Phe Arg Thr His Thr Leu His Glu Ala Tyr Cys Asp Thr Asp Gln
               100                 105                 110
   agc ctg tgg aac aac gcc ctt ggc tac tgg ggc tac ctc ttc tac ctt       384
   Ser Leu Trp Asn Asn Ala Leu Gly Tyr Trp Gly Tyr Leu Phe Tyr Leu
           115                 120                 125
   tca aag ttt tac gag gtc att gac acc atc atc atc atc ttg aag ggg       432
   Ser Lys Phe Tyr Glu Val Ile Asp Thr Ile Ile Ile Ile Leu Lys Gly
       130                 135                 140
   cgc cgc tcg tcc ctg ctc cag acc tac cac cac gcc ggc gct atg atc       480
   Arg Arg Ser Ser Leu Leu Gln Thr Tyr His His Ala Gly Ala Met Ile
   145                 150                 155                 160
   acc atg tgg tcc ggc atc aac tac cag gca acg ccc att tgg att ttt      528
   Thr Met Trp Ser Gly Ile Asn Tyr Gln Ala Thr Pro Ile Trp Ile Phe
                   165                 170                 175
   gtc gtc ttc aac tcg ttc atc cac acc atc atg tac tgt tac tat gcc      576
   Val Val Phe Asn Ser Phe Ile His Thr Ile Met Tyr Cys Tyr Tyr Ala
               180                 185                 190
   ttc acc tca atc ggc ttc cac ccc cca ggc aag aag tac ctc acc tcc      624
   Phe Thr Ser Ile Gly Phe His Pro Pro Gly Lys Lys Tyr Leu Thr Ser
           195                 200                 205
   atg cag atc acc cag ttt ttg gtc ggc atc act atc gcc gtc tct tat      672
   Met Gln Ile Thr Gln Phe Leu Val Gly Ile Thr Ile Ala Val Ser Tyr
       210                 215                 220
   ctc ttc gtc cct gga tgt atc cgc aca ccc ggt gct cag atg gct gtc      720
   Leu Phe Val Pro Gly Cys Ile Arg Thr Pro Gly Ala Gln Met Ala Val
   225                 230                 235                 240
   tgg atc aac gtc gga tac ctc ttt ccc ctc act tat ctc ttt gtg gat      768
   Trp Ile Asn Val Gly Tyr Leu Phe Pro Leu Thr Tyr Leu Phe Val Asp
                   245                 250                 255
   ttt gcc aag cgt act tac tcc aag cgt agt gcc atc gcc gct cag aag      816
   Phe Ala Lys Arg Thr Tyr Ser Lys Arg Ser Ala Ile Ala Ala Gln Lys
               260                 265                 270
   aag gcc cag taa                                                      828
   Lys Ala Gln
           275
<210> 96
   <211> 275
   <212> PRT
   <213> Mortierella alpina
<400> 96
   Met Glu Ser Gly Pro Met Pro Ala Gly Ile Pro Phe Pro Glu Tyr Tyr
   1               5                   10                  15
   Asp Phe Phe Met Asp Trp Lys Thr Pro Leu Ala Ile Ala Ala Thr Tyr
               20                  25                  30
   Thr Ala Ala Val Gly Leu Phe Asn Pro Lys Val Gly Lys Val Ser Arg
           35                  40                  45
   Val Val Ala Lys Ser Ala Asn Ala Lys Pro Ala Glu Arg Thr Gln Ser
       50                  55                  60
   Gly Ala Ala Met Thr Ala Phe Val Phe Val His Asn Leu Ile Leu Cys
   65                  70                  75                  80
   Val Tyr Ser Gly Ile Thr Phe Tyr Tyr Met Phe Pro Ala Met Val Lys
                   85                  90                  95
   Asn Phe Arg Thr His Thr Leu His Glu Ala Tyr Cys Asp Thr Asp Gln
                   100                 105                 110
   Ser Leu Trp Asn Asn Ala Leu Gly Tyr Trp Gly Tyr Leu Phe Tyr Leu
           115                 120                 125
   Ser Lys Phe Tyr Glu Val Ile Asp Thr Ile Ile Ile Ile Leu Lys Gly
       130                 135                 140
   Arg Arg Ser Ser Leu Leu Gln Thr Tyr His His Ala Gly Ala Met Ile
   145                 150                 155                 160
   Thr Met Trp Ser Gly Ile Asn Tyr Gln Ala Thr Pro Ile Trp Ile Phe
                   165                 170                 175
   Val Val Phe Asn Ser Phe Ile His Thr Ile Met Tyr Cys Tyr Tyr Ala
               180                 185                 190
   Phe Thr Ser Ile Gly Phe His Pro Pro Gly Lys Lys Tyr Leu Thr Ser
           195                 200                 205
   Met Gln Ile Thr Gln Phe Leu Val Gly Ile Thr Ile Ala Val Ser Tyr
       210                 215                 220
   Leu Phe Val Pro Gly Cys Ile Arg Thr Pro Gly Ala Gln Met Ala Val
   225                 230                 235                 240
   Trp Ile Asn Val Gly Tyr Leu Phe Pro Leu Thr Tyr Leu Phe Val Asp
                   245                 250                 255
   Phe Ala Lys Arg Thr Tyr Ser Lys Arg Ser Ala Ile Ala Ala Gln Lys
               260                 265                 270
   Lys Ala Gln
           275
<210> 97
   <211> 828
   <212> DNA
   <213> Mortierella alpina
<220>
   <221> CDS
   <222> (1)..(828)
   <223> synthetic C16/18 elongase (codon-optimized for Yarrowia lipolytica)
<300>
   <302> A MORTIERELLA ALPINA C16/18 FATTY ACID ELONGASE
<310> U.S. 7,470,532
   <311> 2005-10-19
   <312> 2008-12-30
   <313> (1)..(828)
<300>
   <302> A MORTIERELLA ALPINA C16/18 FATTY ACID ELONGASE
<310> WO 2007/046817
   <311> 2005-11-04
   <312> 2007-04-26
   <313> (1)..(828)
<400> 97
   atg gag tct gga ccc atg cct gct ggc att ccc ttc cct gag tac tat      48
   Met Glu Ser Gly Pro Met Pro Ala Gly Ile Pro Phe Pro Glu Tyr Tyr
   1               5                   10                  15
   gac ttc ttt atg gac tgg aag act ccc ctg gcc atc gct gcc acc tac      96
   Asp Phe Phe Met Asp Trp Lys Thr Pro Leu Ala Ile Ala Ala Thr Tyr
               20                  25                  30
   act gct gcc gtc ggt ctc ttc aac ccc aag gtt ggc aag gtc tcc cga      144
   Thr Ala Ala Val Gly Leu Phe Asn Pro Lys Val Gly Lys Val Ser Arg
           35                  40                  45
   gtg gtt gcc aag tcg gct aac gca aag cct gcc gag cga acc cag tcc      192
   Val Val Ala Lys Ser Ala Asn Ala Lys Pro Ala Glu Arg Thr Gln Ser
       50                  55                  60
   gga gct gcc atg act gcc ttc gtc ttt gtg cac aac ctc att ctg tgt      240
   Gly Ala Ala Met Thr Ala Phe Val Phe Val His Asn Leu Ile Leu Cys
   65                  70                  75                  80
   gtc tac tct ggc atc acc ttc tac tac atg ttt cct gct atg gtc aag      288
   Val Tyr Ser Gly Ile Thr Phe Tyr Tyr Met Phe Pro Ala Met Val Lys
                   85                  90                  95
   aac ttc cga acc cac aca ctg cac gaa gcc tac tgc gac acg gat cag      336
   Asn Phe Arg Thr His Thr Leu His Glu Ala Tyr Cys Asp Thr Asp Gln
               100                 105                 110
   tcc ctc tgg aac aac gca ctt ggc tac tgg ggt tac ctc ttc tac ctg      384
   Ser Leu Trp Asn Asn Ala Leu Gly Tyr Trp Gly Tyr Leu Phe Tyr Leu
           115                 120                 125
   tcc aag ttc tac gag gtc att gac acc atc atc atc atc ctg aag gga      432
   Ser Lys Phe Tyr Glu Val Ile Asp Thr Ile Ile Ile Ile Leu Lys Gly
       130                 135                 140
   cga cgg tcc tcg ctg ctt cag acc tac cac cat gct gga gcc atg att      480
   Arg Arg Ser Ser Leu Leu Gln Thr Tyr His His Ala Gly Ala Met Ile
   145                 150                 155                 160
   acc atg tgg tct ggc atc aac tac caa gcc act ccc att tgg atc ttt      528
   Thr Met Trp Ser Gly Ile Asn Tyr Gln Ala Thr Pro Ile Trp Ile Phe
                   165                 170                 175
   gtg gtc ttc aac tcc ttc att cac acc atc atg tac tgt tac tat gcc      576
   Val Val Phe Asn Ser Phe Ile His Thr Ile Met Tyr Cys Tyr Tyr Ala
               180                 185                 190
   ttc acc tct atc gga ttc cat cct cct ggc aaa aag tac ctg act tcg      624
   Phe Thr Ser Ile Gly Phe His Pro Pro Gly Lys Lys Tyr Leu Thr Ser
           195                 200                 205
   atg cag att act cag ttt ctg gtc ggt atc acc att gcc gtg tcc tac      672
   Met Gln Ile Thr Gln Phe Leu Val Gly Ile Thr Ile Ala Val Ser Tyr
       210                 215                 220
   ctc ttc gtt cct ggc tgc atc cga aca ccc ggt gct cag atg gct gtc        720
   Leu Phe Val Pro Gly Cys Ile Arg Thr Pro Gly Ala Gln Met Ala Val
   225                 230                 235                 240
   tgg atc aac gtc ggc tac ctg ttt ccc ttg acc tat ctg ttc gtg gac        768
   Trp Ile Asn Val Gly Tyr Leu Phe Pro Leu Thr Tyr Leu Phe Val Asp
                   245                 250                 255
   ttt gcc aag cga acc tac tcc aag cga tct gcc att gcc gct cag aaa        816
   Phe Ala Lys Arg Thr Tyr Ser Lys Arg Ser Ala Ile Ala Ala Gln Lys
               260                 265                 270
   aag gct cag taa                                                        828
   Lys Ala Gln
           275
<210> 98
   <211> 275
   <212> PRT
   <213> Mortierella alpina
<400> 98
   Met Glu Ser Gly Pro Met Pro Ala Gly Ile Pro Phe Pro Glu Tyr Tyr
   1               5                   10                  15
   Asp Phe Phe Met Asp Trp Lys Thr Pro Leu Ala Ile Ala Ala Thr Tyr
               20                  25                  30
   Thr Ala Ala Val Gly Leu Phe Asn Pro Lys Val Gly Lys Val Ser Arg
           35                  40                  45
   Val Val Ala Lys Ser Ala Asn Ala Lys Pro Ala Glu Arg Thr Gln Ser
       50                  55                  60
   Gly Ala Ala Met Thr Ala Phe Val Phe Val His Asn Leu Ile Leu Cys
   65                  70                  75                  80
   Val Tyr Ser Gly Ile Thr Phe Tyr Tyr Met Phe Pro Ala Met Val Lys
                   85                  90                  95
   Asn Phe Arg Thr His Thr Leu His Glu Ala Tyr Cys Asp Thr Asp Gln
               100                 105                 110
   Ser Leu Trp Asn Asn Ala Leu Gly Tyr Trp Gly Tyr Leu Phe Tyr Leu
           115                 120                 125
   Ser Lys Phe Tyr Glu Val Ile Asp Thr Ile Ile Ile Ile Leu Lys Gly
       130                 135                 140
   Arg Arg Ser Ser Leu Leu Gln Thr Tyr His His Ala Gly Ala Met Ile
   145                 150                 155                 160
   Thr Met Trp Ser Gly Ile Asn Tyr Gln Ala Thr Pro Ile Trp Ile Phe
                   165                 170                 175
   Val Val Phe Asn Ser Phe Ile His Thr Ile Met Tyr Cys Tyr Tyr Ala
               180                 185                 190
   Phe Thr Ser Ile Gly Phe His Pro Pro Gly Lys Lys Tyr Leu Thr Ser
           195                 200                 205
   Met Gln Ile Thr Gln Phe Leu Val Gly Ile Thr Ile Ala Val Ser Tyr
       210                 215                 220
   Leu Phe Val Pro Gly Cys Ile Arg Thr Pro Gly Ala Gln Met Ala Val
   225                 230                 235                 240
   Trp Ile Asn Val Gly Tyr Leu Phe Pro Leu Thr Tyr Leu Phe Val Asp
                   245                 250                 255
   Phe Ala Lys Arg Thr Tyr Ser Lys Arg Ser Ala Ile Ala Ala Gln Lys
               260                 265                 270
   Lys Ala Gln
           275
<210> 99
   <211> 12
   <212> PRT
   <213> Artificial Sequence
<220>
   <223> membrane bound O-acyltransferase motif
<220>
   <221> misc_feature
   <222> (4)..(6)
   <223> Xaa can be any naturally occurring amino acid
<220>
   <221> misc_feature
   <222> (9)..(11)
   <223> Xaa can be any naturally occurring amino acid
<300>
   <301> Hideo Shindou, Miki Etoa, Ryo Morimotoa and Takao Shimizua <302> Identification of membrane O-acyltransferase family motifs <303> Biochemical and Biophysical Research Communications <304> 383 <305> 3 <306> 320-325 <307> 2009-04-08
   <313> (1)..(12)
<400> 99
   Trp His Gly Xaa Xaa Xaa Gly Tyr Xaa Xaa Xaa Phe
   1               5                   10
<210> 100
   <211> 6
   <212> PRT
   <213> Artificial Sequence
<220>
   <223> membrane bound O-acyltransferase motif
<220>
   <221> misc_feature
   <222> (2)..(5)
   <223> Xaa can be any naturally occurring amino acid
<300>
   <301> Hideo Shindou, Miki Etoa, Ryo Morimotoa and Takao Shimizua <302> Identification of membrane O-acyltransferase family motifs <303> Biochemical and Biophysical Research Communications <304> 383 <305> 3 <306> 320-325 <307> 2009-04-08
   <313> (1)..(6)
<400> 100
   Tyr Xaa Xaa Xaa Xaa Phe
   1 5
<210> 101
   <211> 9
   <212> PRT
   <213> Artificial Sequence
<220>
   <223> membrane bound O-acyltransferase motif
<220>
   <221> misc_feature
   <222> (2)..(4)
   <223> Xaa can be any naturally occurring amino acid
<220>
   <221> misc_feature
   <222> (7)..(8)
   <223> Xaa can be any naturally occurring amino acid
<300>
   <301> Hideo Shindou, Miki Etoa, Ryo Morimotoa and Takao Shimizua <302> Identification of membrane O-acyltransferase family motifs <303> Biochemical and Biophysical Research Communications <304> 383 <305> 3 <306> 320-325 <307> 2009-04-08
   <313> (1)..(9)
<400> 101
   Tyr Xaa Xaa Xaa Tyr Phe Xaa Xaa His
   1 5
<210> 102
   <211> 15
   <212> PRT
   <213> Artificial Sequence
<220>
   <223> motif
<220>
   <221> MISC_FEATURE
   <222> (2)..(2)
   <223> Xaa = Val [V] or Ile [I]
<220>
   <221> MISC_FEATURE
   <222> (3)..(3)
   <223> Xaa = Leu [L] or Ile [I]
<220>
   <221> misc_feature
   <222> (4)..(5)
   <223> Xaa can be any naturally occurring amino acid
<220>
   <221> MISC_FEATURE
   <222> (7)..(7)
   <223> Xaa = Leu [L] or Ile [I] or Val [V]
<220>
   <221> misc_feature
   <222> (8)..(13)
   <223> Xaa can be any naturally occurring amino acid
<300>
   <302> GENES ENCODING A NOVEL TYPE OF LYSOPHOPHATIDYLCHOLINE ACYLTRANSFERASES AND THEIR USE TO INCREASE TRIACYLGLYCEROL PRODUCTION AND/OR MODIFY FATTY ACID COMPOSITION
<310> U.S. Patent Publication No. 2008-0145867-A1
   <311> 2007-06-15
   <312> 2008-06-19
   <313> (1)..(15)
<400> 102
   Met Xaa Xaa Xaa Xaa Lys Xaa Xaa Xaa Xaa Xaa Xaa Xaa Asp Gly
   1               5                   10                  15
<210> 103
   <211> 24
   <212> PRT
   <213> Artificial Sequence
<220>
   <223> motif
<220>
   <221> misc_feature
   <222> (2)..(2)
   <223> Xaa can be any naturally occurring amino acid
<220>
   <221> misc_feature
   <222> (6)..(7)
   <223> Xaa can be any naturally occurring amino acid
<220>
   <221> misc_feature
   <222> (9)..(11)
   <223> Xaa can be any naturally occurring amino acid
<220>
   <221> MISC_FEATURE
   <222> (12)..(12)
   <223> Xaa = Glu [E] or Asp [D]
<220>
   <221> MISC_FEATURE
   <222> (13)..(13)
   <223> Xaa = Ala [A] or Gly [G]
<220>
   <221> misc_feature
   <222> (14)..(18)
   <223> Xaa can be any naturally occurring amino acid
<220>
   <221> misc_feature
   <222> (20)..(20)
   <223> Xaa can be any naturally occurring amino acid
<220>
   <221> MISC_FEATURE
   <222> (22)..(22)
   <223> Xaa = Phe [F] or Tyr [Y]
<220>
   <221> misc_feature
   <222> (23)..(23)
   <223> Xaa can be any naturally occurring amino acid
<300>
   <302> GENES ENCODING A NOVEL TYPE OF LYSOPHOPHATIDYLCHOLINE ACYLTRANSFERASES AND THEIR USE TO INCREASE TRIACYLGLYCEROL PRODUCTION AND/OR MODIFY FATTY ACID COMPOSITION
<310> U.S. Patent Publication No. 2008-0145867-A1
   <311> 2007-06-15
   <312> 2008-06-19
   <313> (1)..(15)
<400> 103
   Arg Xaa Lys Tyr Tyr Xaa Xaa Trp Xaa Xaa Xaa Xaa Xaa Xaa Xaa Xaa
   1               5                   10                  15
   Xaa Xaa Gly Xaa Gly Xaa Xaa Gly
               20
<210> 104
   <211> 15
   <212> PRT
   <213> Artificial Sequence
<220>
   <223> motif
<220>
   <221> misc_feature
   <222> (3)..(3)
   <223> Xaa can be any naturally occurring amino acid
<220>
   <221> misc_feature
   <222> (7)..(8)
   <223> Xaa can be any naturally occurring amino acid
<220>
   <221> misc_feature
   <222> (12)..(13)
   <223> Xaa can be any naturally occurring amino acid
<220>
   <221> MISC_FEATURE
   <222> (14)..(14)
   <223> Xaa = Thr [T] or Phe [F]
<300>
   <302> GENES ENCODING A NOVEL TYPE OF LYSOPHOPHATIDYLCHOLINE ACYLTRANSFERASES AND THEIR USE TO INCREASE TRIACYLGLYCEROL PRODUCTION AND/OR MODIFY FATTY ACID COMPOSITION
<310> U.S. Patent Publication No. 2008-0145867-A1
   <311> 2007-06-15
   <312> 2008-06-19
   <313> (1)..(15)
<400> 104
   Ser Ala Xaa Trp His Gly Xaa Xaa Pro Gly Tyr Xaa Xaa Xaa Phe
   1               5                   10                  15
<210> 105
   <211> 7
   <212> PRT
   <213> Artificial Sequence
<220>
   <223> 1-acyl-sn-glycerol-3-phosphate acyltransferase motif
<220>
   <221> misc_feature
   <222> (2)..(3)
   <223> Xaa can be any naturally occurring amino acid
<220>
   <221> MISC_FEATURE
   <222> (6)..(6)
   <223> Xaa = Asp [D] or Arg [R]
<300>
   <301> Tal M. Lewin, Ping Wang, and Rosalind A. Coleman <302> Analysis of Amino Acid Motifs Diagnostic for the sn-Glycerol-3-phosphate Acyltransferase Reaction <303> Biochemistry <304> 38 <305> 18 <306> 57645771 <307> 1999-04-15
   <313> (1)..(7)
<300>
   <301> Atsushi Yamashita, Hiroki Nakanishia, Hiroshi Suzukia, Ryo Kamataa, Ken Tanakaa, Keizo Wakua and Takayuki Sugiura <302> Topology of acyltransferase motifs and substrate specificity and accessibility in 1-acyl-sn-glycero-3-phosphate acyltransferase 1 <303> Biochimica et Biophysica Acta <304> 1771 <305> 9 <306> 1202-1215 <307> 2007-07-17
   <313> (1)..(7)
<400> 105
   Gly Xaa Xaa Phe Ile Xaa Arg
   1               5
<210> 106
   <211> 6
   <212> PRT
   <213> Artificial Sequence
<220>
   <223> 1-acyl-sn-glycerol-3-phosphate acyltransferase motif
<220>
   <221> MISC_FEATURE
   <222> (1)..(1)
   <223> Xaa = Val [V] or Ile [I]
<220>
   <221> MISC_FEATURE
   <222> (2)..(2)
   <223> Xaa = Pro [P] or Xaa
<220>
   <221> MISC_FEATURE
   <222> (3)..(3)
   <223> Xaa = Ile [I] or Val [V] or Leu [L]
<220>
   <221> MISC_FEATURE
   <222> (4)..(4)
   <223> Xaa = Ile [I] or Val [V]
<220>
   <221> MISC_FEATURE
   <222> (6)..(6)
   <223> Xaa = Val [V] or Ile [I]
<300>
   <301> Atsushi Yamashita, Hiroki Nakanishia, Hiroshi Suzukia, Ryo Kamataa, Ken Tanakaa, Keizo Wakua and Takayuki Sugiura <302> Topology of acyltransferase motifs and substrate specificity and accessibility in 1-acyl-sn-glycero-3-phosphate acyltransferase 1 <303> Biochimica et Biophysica Acta <304> 1771 <305> 9 <306> 1202-1215 <307> 2007-07-17
   <313> (1)..(6)
<400> 106
   Xaa Xaa Xaa Xaa Pro Xaa
   1 5
<210> 107
   <211> 6
   <212> PRT
   <213> Artificial Sequence
<220>
   <223> 1-acyl-sn-glycerol-3-phosphate acyltransferase motif
<300>
   <301> Atsushi Yamashita, Hiroki Nakanishia, Hiroshi Suzukia, Ryo Kamataa, Ken Tanakaa, Keizo Wakua and Takayuki Sugiura <302> Topology of acyltransferase motifs and substrate specificity and accessibility in 1-acyl-sn-glycero-3-phosphate acyltransferase 1 <303> Biochimica et Biophysica Acta <304> 1771 <305> 9 <306> 1202-1215 <307> 2007-07-17
   <313> (1)..(6)
<400> 107
   Ile Val Pro Ile Val Met
   1 5
<210> 108
   <211> 1024
   <212> PRT
   <213> Yarrowia lipolytica
<220>
   <221> MISC_FEATURE
   <222> (1)..(1024)
   <223> YlPex1p; GenBank Accession No. CAG82178
<400> 108
   Met Thr Ser Lys Ser Asp Tyr Ser Gly Lys Asp Lys Ile Glu Leu Asp
   1 5 10 15
   Pro Val Phe Ala Lys Ser Ile Asp Leu Leu Pro Asn Thr Gln Val Val
   20 25 30
   Ile Asp Ile Gln Leu Asn Pro Lys Ile Ala His Thr Ile His Leu Glu
   35 40 45
   Pro Val Thr Val Ala Asp Trp Glu Ile Val Glu Leu His Ala Ala Tyr
   50 55 60
   Leu Glu Ser Arg Met Ile Asn Gln Val Arg Ala Val Ser Pro Asn Gln
   65 70 75 80
   Pro Val Thr Val Tyr Pro Ser Ser Thr Thr Ser Ala Thr Leu Lys Val
   85 90 95
   Ile Arg Ile Glu Pro Asp Leu Gly Ala Ala Gly Phe Ala Lys Leu Ser
   100 105 110
   Pro Asp Ser Glu Val Val Val Ala Pro Lys Gln Arg Lys Lys Glu Glu
   115 120 125
   Lys Gln Val Lys Lys Arg Ser Gly Ser Ala Arg Ser Thr Gly Ser Gln
   130 135 140
   Lys Arg Lys Gly Gly Arg Gly Pro His Ala Leu Arg Arg Ala Ile Ser
   145 150 155 160
   Glu Asp Phe Asp Gly His Leu Arg Leu Glu Val Ser Leu Asp Val Ser
   165 170 175
   Gln Leu Pro Pro Glu Phe His Gln Leu Lys Asn Val Ser Ile Lys Val
   180 185 190
   Ile Thr Pro Pro Asn Leu Ala Ser Pro Gln Gln Ala Ala Ser Ile Ala
   195 200 205
   Val Glu Glu Lys Ser Glu Glu Ser Leu Ser Gln Asn Lys Pro Pro Ser
   210 215 220
   Ser Glu Pro Lys Val Glu Val Pro Pro Asp Ile Ile Asn Pro Ala Ser
   225 230 235 240
   Glu Ile Val Ala Thr Leu Val Asn Asp Thr Thr Ser Pro Thr Gly His
   245 250 255
   Ala Lys Leu Ser Tyr Ala Leu Ala Asp Ala Leu Gly Ile Pro Ser Ser
   260 265 270
   Val Gly His Val Ile Arg Phe Glu Ser Ala Ser Lys Pro Leu Ser Gln
   275 280 285
   Lys Pro Gly Ala Leu Val Ile His Arg Phe Ile Thr Lys Thr Val Gly
   290 295 300
   Ala Ala Glu Gln Lys Ser Leu Arg Leu Lys Gly Glu Lys Asn Ala Asp
   305 310 315 320
   Asp Gly Val Ser Ala Asp Asp Gln Phe Ser Leu Leu Glu Glu Leu Lys
   325 330 335
   Lys Leu Gln Met Leu Glu Gly Pro Ile Thr Asn Phe Gln Arg Leu Pro
   340 345 350
   Pro Ile Pro Glu Leu Leu Pro Leu Gly Gly Val Ile Gly Leu Gln Asn
   355 360 365
   Ser Glu Gly Trp Ile Gln Gly Gly Tyr Leu Gly Glu Glu Pro Ile Pro
   370 375 380
   Phe Val Ser Gly Ser Glu Ile Leu Arg Ser Glu Ser Ser Leu Ser Pro
   385 390 395 400
   Ser Asn Ile Glu Ser Glu Asp Lys Arg Val Val Gly Leu Asp Asn Met
   405 410 415
   Leu Asn Lys Ile Asn Glu Val Leu Ser Arg Asp Ser Ile Gly Cys Leu
   420 425 430
   Val Tyr Gly Ser Arg Gly Ser Gly Lys Ser Ala Val Leu Asn His Ile
   435 440 445
   Lys Lys Glu Cys Lys Val Ser His Thr His Thr Val Ser Ile Ala Cys
   450 455 460
   Gly Leu Ile Ala Gln Asp Arg Val Gln Ala Val Arg Glu Ile Leu Thr
   465 470 475 480
   Lys Ala Phe Leu Glu Ala Ser Trp Phe Ser Pro Ser Val Leu Phe Leu
   485 490 495
   Asp Asp Ile Asp Ala Leu Met Pro Ala Glu Val Glu His Ala Asp Ser
   500 505 510
   Ser Arg Thr Arg Gln Leu Thr Gln Leu Phe Leu Glu Leu Ala Leu Pro
   515 520 525
   Ile Met Lys Ser Arg His Val Ser Val Val Ala Ser Ala Gln Ala Lys
   530 535 540
   Glu Ser Leu His Met Asn Leu Val Thr Gly His Val Phe Glu Glu Leu
   545 550 555 560
   Phe His Leu Lys Ser Pro Asp Lys Glu Ala Arg Leu Ala Ile Leu Ser
   565 570 575
   Glu Ala Val Lys Leu Met Asp Gln Asn Val Ser Phe Ser Gln Asn Asp
   580 585 590
   Val Leu Glu Ile Ala Ser Gln Val Asp Gly Tyr Leu Pro Gly Asp Leu
   595 600 605
   Trp Thr Leu Ser Glu Arg Ala Gln His Glu Met Ala Leu Arg Gln Ile
   610 615 620
   Glu Ile Gly Leu Glu Asn Pro Ser Ile Gln Leu Ala Asp Phe Met Lys
   625 630 635 640
   Ala Leu Glu Asp Phe Val Pro Ser Ser Leu Arg Gly Val Lys Leu Gln
   645 650 655
   Lys Ser Asn Val Lys Trp Asn Asp Ile Gly Gly Leu Lys Glu Thr Lys
   660 665 670
   Ala Val Leu Leu Glu Thr Leu Glu Trp Pro Thr Lys Tyr Ala Pro Ile
   675 680 685
   Phe Ala Ser Cys Pro Leu Arg Leu Arg Ser Gly Leu Leu Leu Tyr Gly
   690 695 700
   Tyr Pro Gly Cys Gly Lys Thr Tyr Leu Ala Ser Ala Val Ala Ala Gln
   705 710 715 720
   Cys Gly Leu Asn Phe Ile Ser Ile Lys Gly Pro Glu Ile Leu Asn Lys
   725 730 735
   Tyr Ile Gly Ala Ser Glu Gln Ser Val Arg Glu Leu Phe Glu Arg Ala
   740 745 750
   Gln Ala Ala Lys Pro Cys Ile Leu Phe Phe Asp Glu Phe Asp Ser Ile
   755 760 765
   Ala Pro Lys Arg Gly His Asp Ser Thr Gly Val Thr Asp Arg Val Val
   770 775 780
   Asn Gln Met Leu Thr Gln Met Asp Gly Ala Glu Gly Leu Asp Gly Val
   785 790 795 800
   Tyr Val Leu Ala Ala Thr Ser Arg Pro Asp Leu Ile Asp Pro Ala Leu
   805 810 815
   Leu Arg Pro Gly Arg Leu Asp Lys Met Leu Ile Cys Asp Leu Pro Ser
   820 825 830
   Tyr Glu Asp Arg Leu Asp Ile Leu Arg Ala Ile Val Asp Gly Lys Met
   835 840 845
   His Leu Asp Gly Glu Val Glu Leu Glu Tyr Val Ala Ser Arg Thr Asp
   850 855 860
   Gly Phe Ser Gly Ala Asp Leu Gln Ala Val Met Phe Asn Ala Tyr Leu
   865 870 875 880
   Glu Ala Ile His Glu Val Val Asp Val Ala Asp Asp Thr Ala Ala Asp
   885 890 895
   Thr Pro Ala Leu Glu Asp Lys Arg Leu Glu Phe Phe Gln Thr Thr Leu
   900 905 910
   Gly Asp Ala Lys Lys Asp Pro Ala Ala Val Gln Asn Glu Val Met Asn
   915 920 925
   Ala Arg Ala Ala Val Ala Glu Lys Ala Arg Val Thr Ala Lys Leu Glu
   930 935 940
   Ala Leu Phe Lys Gly Met Ser Val Gly Val Asp Asn Asp Asp Asp Lys
   945 950 955 960
   Pro Arg Lys Lys Ala Val Val Val Ile Lys Pro Gln His Met Asn Lys
   965 970 975
   Ser Leu Asp Glu Thr Ser Pro Ser Ile Ser Lys Lys Glu Leu Leu Lys
   980 985 990
   Leu Lys Gly Ile Tyr Ser Gln Phe Val Ser Gly Arg Ser Gly Asp Met
   995 1000 1005
   Pro Pro Gly Thr Ala Ser Thr Asp Val Gly Gly Arg Ala Thr Leu
   1010 1015 1020
   Ala
<210> 109
   <211> 381
   <212> PRT
   <213> Yarrowia lipolytica
<220>
   <221> MISC_FEATURE
   <222> (1)..(381)
   <223> YlPex2p; GenBank Accession No. CAG77647
<400> 109
   Met Ser Ser Val Leu Arg Leu Phe Lys Ile Gly Ala Pro Val Pro Asn
   1 5 10 15
   Val Arg Val His Gln Leu Asp Ala Ser Leu Leu Asp Ala Glu Leu Val
   20 25 30
   Asp Leu Leu Lys Asn Gln Leu Phe Lys Gly Phe Thr Asn Phe His Pro
           35                  40                  45
   Glu Phe Arg Asp Lys Tyr Glu Ser Glu Leu Val Leu Ala Leu Lys Leu
       50                  55                  60
   Ile Leu Phe Lys Leu Thr Val Trp Asp His Ala Ile Thr Tyr Gly Gly
   65                  70                  75                  80
   Lys Leu Gln Asn Leu Lys Phe Ile Asp Ser Arg His Ser Ser Lys Leu
                   85                  90                  95
   Gln Ile Gln Pro Ser Val Ile Gln Lys Leu Gly Tyr Gly Ile Leu Val
               100                 105                 110
   Val Gly Gly Gly Tyr Leu Trp Ser Lys Ile Glu Gly Tyr Leu Leu Ala
           115                 120                 125
   Arg Ser Glu Asp Asp Val Ala Thr Asp Gly Thr Ser Val Arg Gly Ala
       130                 135                 140
   Ser Ala Ala Arg Gly Ala Leu Lys Val Ala Asn Phe Ala Ser Leu Leu
   145                 150                 155                 160
   Tyr Ser Ala Ala Thr Leu Gly Asn Phe Val Ala Phe Leu Tyr Thr Gly
                   165                 170                 175
   Arg Tyr Ala Thr Val Ile Met Arg Leu Leu Arg Ile Arg Leu Val Pro
               180                 185                 190
   Ser Gln Arg Thr Ser Ser Arg Gln Val Ser Tyr Glu Phe Gln Asn Arg
           195                 200                 205
   Gln Leu Val Trp Asn Ala Phe Thr Glu Phe Leu Ile Phe Ile Leu Pro
       210                 215                 220
   Leu Leu Gln Leu Pro Lys Leu Lys Arg Arg Ile Glu Arg Lys Leu Gln
   225                 230                 235                 240
   Ser Leu Asn Val Thr Arg Val Gly Asn Val Glu Glu Ala Ser Glu Gly
                   245                 250                 255
   Glu Leu Ala His Leu Pro Gln Lys Thr Cys Ala Ile Cys Phe Arg Asp
               260                 265                 270
   Glu Glu Glu Gln Glu Gly Gly Gly Gly Ala Ser His Tyr Ser Thr Asp
           275                 280                 285
   Val Thr Asn Pro Tyr Gln Ala Asp Cys Gly His Val Tyr Cys Tyr Val
   290 295 300
   Cys Leu Val Thr Lys Leu Ala Gln Gly Asp Gly Asp Gly Trp Asn Cys
   305 310 315 320
   Tyr Arg Cys Ala Lys Gln Val Gln Lys Met Lys Pro Trp Val Asp Val
   325 330 335
   Asp Glu Ala Ala Val Val Gly Ala Ala Glu Met His Glu Lys Val Asp
   340 345 350
   Val Ile Glu His Ala Glu Asp Asn Glu Gln Glu Glu Glu Glu Phe Asp
   355 360 365
   Asp Asp Asp Glu Asp Ser Asn Phe Gln Leu Met Lys Asp
   370 375 380
<210> 110
   <211> 431
   <212> PRT
   <213> Yarrowia lipolytica
<220>
   <221> MISC_FEATURE
   <222> (1)..(431)
   <223> YlPex3p; GenBank Accession No. CAG78565
<400> 110
   Met Asp Phe Phe Arg Arg His Gln Lys Lys Val Leu Ala Leu Val Gly
   1               5                   10                  15
   Val Ala Leu Ser Ser Tyr Leu Phe Ile Asp Tyr Val Lys Lys Lys Phe
               20                  25                  30
   Phe Glu Ile Gln Gly Arg Leu Ser Ser Glu Arg Thr Ala Lys Gln Asn
           35                  40                  45
   Leu Arg Arg Arg Phe Glu Gln Asn Gln Gln Asp Ala Asp Phe Thr Ile
       50                  55                  60
   Met Ala Leu Leu Ser Ser Leu Thr Thr Pro Val Met Glu Arg Tyr Pro
   65                  70                  75                  80
   Val Asp Gln Ile Lys Ala Glu Leu Gln Ser Lys Arg Arg Pro Thr Asp
                   85                  90                  95
   Arg Val Leu Ala Leu Glu Ser Ser Thr Ser Ser Ser Ala Thr Ala Gln
               100                 105                 110
   Thr Val Pro Thr Met Thr Ser Gly Ala Thr Glu Glu Gly Glu Lys Ser
   115 120 125
   Lys Thr Gln Leu Trp Gln Asp Leu Lys Arg Thr Thr Ile Ser Arg Ala
   130 135 140
   Phe Ser Leu Val Tyr Ala Asp Ala Leu Leu Ile Phe Phe Thr Arg Leu
   145 150 155 160
   Gln Leu Asn Ile Leu Gly Arg Arg Asn Tyr Val Asn Ser Val Val Ala
   165 170 175
   Leu Ala Gln Gln Gly Arg Glu Gly Asn Ala Glu Gly Arg Val Ala Pro
   180 185 190
   Ser Phe Gly Asp Leu Ala Asp Met Gly Tyr Phe Gly Asp Leu Ser Gly
   195 200 205
   Ser Ser Ser Phe Gly Glu Thr Ile Val Asp Pro Asp Leu Asp Glu Gln
   210 215 220
   Tyr Leu Thr Phe Ser Trp Trp Leu Leu Asn Glu Gly Trp Val Ser Leu
   225 230 235 240
   Ser Glu Arg Val Glu Glu Ala Val Arg Arg Val Trp Asp Pro Val Ser
   245 250 255
   Pro Lys Ala Glu Leu Gly Phe Asp Glu Leu Ser Glu Leu Ile Gly Arg
   260 265 270
   Thr Gln Met Leu Ile Asp Arg Pro Leu Asn Pro Ser Ser Pro Leu Asn
   275 280 285
   Phe Leu Ser Gln Leu Leu Pro Pro Arg Glu Gln Glu Glu Tyr Val Leu
   290 295 300
   Ala Gln Asn Pro Ser Asp Thr Ala Ala Pro Ile Val Gly Pro Thr Leu
   305 310 315 320
   Arg Arg Leu Leu Asp Glu Thr Ala Asp Phe Ile Glu Ser Pro Asn Ala
   325 330 335
   Ala Glu Val Ile Glu Arg Leu Val His Ser Gly Leu Ser Val Phe Met
   340 345 350
   Asp Lys Leu Ala Val Thr Phe Gly Ala Thr Pro Ala Asp Ser Gly Ser
   355 360 365
   Pro Tyr Pro Val Val Leu Pro Thr Ala Lys Val Lys Leu Pro Ser Ile
   370 375 380
   Leu Ala Asn Met Ala Arg Gln Ala Gly Gly Met Ala Gln Gly Ser Pro
   385 390 395 400
   Gly Val Glu Asn Glu Tyr Ile Asp Val Met Asn Gln Val Gln Glu Leu
   405 410 415
   Thr Ser Phe Ser Ala Val Val Tyr Ser Ser Phe Asp Trp Ala Leu
   420 425 430
<210> 111
   <211> 395
   <212> PRT
   <213> Yarrowia lipolytica
<220>
   <221> MISC_FEATURE
   <222> (1)..(395)
   <223> YlPex3Bp; GenBank Accession No. CAG83356
<400> 111
   Met Leu Gln Ser Leu Asn Arg Asn Lys Lys Arg Leu Ala Val Ser Thr
   1               5                   10                  15
   Gly Leu Ile Ala Val Ala Tyr Val Val Ile Ser Tyr Thr Thr Lys Arg
               20                  25                  30
   Leu Ile Glu Lys Gln Glu Gln Lys Leu Glu Glu Glu Arg Ala Lys Glu
           35                  40                  45
   Arg Leu Lys Gln Leu Phe Ala Gln Thr Gln Asn Glu Ala Ala Phe His
       50                  55                  60
   Thr Ala Ser Val Leu Pro Gln Leu Cys Glu Gln Ile Met Glu Phe Val
   65                  70                  75                  80
   Ala Val Glu Lys Ile Ala Glu Gln Leu Gln Asn Met Arg Ala Glu Lys
                   85                  90                  95
   Arg Lys Lys Gln Asn Met Asp Asp Asp Lys His Ser Val Leu Ser Leu
               100                 105                 110
   Gly Thr Glu Thr Thr Ala Ser Met Ala Asp Gly Gln Lys Met Ser Lys
           115                 120                 125
   Ile Gln Leu Trp Asp Glu Leu Lys Ile Glu Ser Leu Thr Arg Ile Val
       130                 135                 140
   Thr Leu Ile Tyr Cys Val Ser Leu Leu Asn Tyr Leu Ile Arg Leu Gln
   145                 150                 155                 160
   Thr Asn Ile Val Gly Arg Lys Arg Tyr Gln Asn Glu Ala Gly Pro Ala
                   165                 170                 175
   Gly Ala Thr Tyr Asp Met Ser Leu Glu Gln Cys Tyr Thr Trp Leu Leu
               180                 185                 190
   Thr Arg Gly Trp Lys Ser Val Val Asp Asn Val Arg Arg Ser Val Gln
           195                 200                 205
   Gln Val Phe Thr Gly Val Asn Pro Arg Gln Asn Leu Ser Leu Asp Glu
       210                 215                 220
   Phe Ala Thr Leu Leu Lys Arg Val Gln Thr Leu Val Asn Ser Pro Pro
   225                 230                 235                 240
   Tyr Ser Thr Thr Pro Asn Thr Phe Leu Thr Ser Leu Leu Pro Pro Arg
                   245                 250                 255
   Glu Leu Glu Gln Leu Arg Leu Glu Lys Glu Lys Gln Ser Leu Ser Pro
               260                 265                 270
   Asn Tyr Thr Tyr Gly Ser Pro Leu Lys Asp Leu Val Phe Glu Ser Ala
           275                 280                 285
   Gln His Ile Gln Ser Pro Gln Gly Met Ser Ser Phe Arg Ala Ile Ile
       290                 295                 300
   Asp Gln Ser Phe Lys Val Phe Leu Glu Lys Val Asn Glu Ser Gln Tyr
   305                 310                 315                 320
   Val Asn Pro Pro Ser Thr Gly Gly Lys Arg Ile Ala Val Gly Ala Leu
                   325                 330                 335
   Gln Pro Pro Ile Ile Ser Gly Gly Pro Lys Lys Val Lys Leu Ala Ser
               340                 345                 350
   Leu Leu Ser Val Ala Thr Arg Gln Ser Ser Val Ile Ser His Ala Gln
           355                 360                 365
   Pro Asn Pro Tyr Val Asp Ala Ile Asn Ser Val Ala Glu Tyr Asn Gly
       370                 375                 380
   Leu Cys Ala Val Ile Tyr Ser Ser Phe Glu Gln
   385                 390                 395
<210> 112
   <211> 153
   <212> PRT
   <213> Yarrowia lipolytica
<220>
   <221> MISC_FEATURE
   <222> (1)..(153)
   <223> YlPex4p; GenBank Accession No. CAG79130
<400> 112
   Met Ala Ser Gln Lys Arg Leu Ile Lys Glu Leu Ala Ala Tyr Lys Lys
   1               5                   10                  15
   Asp Pro Asn Pro Cys Leu Ala Ser Leu Thr Ala Asp Gly Asp Ser Leu
               20                  25                  30
   Tyr Lys Trp Thr Ala Val Met Arg Gly Thr Glu Gly Thr Ala Tyr Glu
           35                  40                  45
   Asn Gly Leu Trp Gln Val Glu Ile Asn Ile Pro Glu Asn Tyr Pro Leu
       50                  55                  60
   Gln Pro Pro Thr Met Phe Phe Arg Thr Lys Ile Cys His Pro Asn Ile
   65                  70                  75                  80
   His Phe Glu Thr Gly Glu Val Cys Ile Asp Val Leu Lys Thr Gln Trp
                   85                  90                  95
   Ser Pro Ala Trp Thr Ile Ser Ser Ala Cys Thr Ala Val Ser Ala Met
               100                 105                 110
   Leu Ser Leu Pro Glu Pro Asp Ser Pro Leu Asn Ile Asp Ala Ala Asn
           115                 120                 125
   Leu Val Arg Cys Gly Asp Glu Ser Ala Met Glu Gly Leu Val Arg Tyr
       130                 135                 140
   Tyr Val Asn Lys Tyr Ala Ser Gly Asn
   145                 150
<210> 113
   <211> 598
   <212> PRT
   <213> Yarrowia lipolytica
<220>
   <221> MISC_FEATURE
   <222> (1)..(598)
   <223> YlPex5p; GenBank Accession No. CAG78803
<400> 113
   Met Ser Phe Met Arg Gly Gly Ser Glu Cys Ser Thr Gly Arg Asn Pro
   1               5                   10                  15
   Leu Ser Gln Phe Thr Lys His Thr Ala Glu Asp Arg Ser Leu Gln His
               20                  25                  30
   Asp Arg Val Ala Gly Pro Ser Gly Gly Arg Val Gly Gly Met Arg Ser
           35                  40                  45
   Asn Thr Gly Glu Met Ser Gln Gln Asp Arg Glu Met Met Ala Arg Phe
       50                  55                  60
   Gly Ala Ala Gly Pro Glu Gln Ser Ser Phe Asn Tyr Glu Gln Met Arg
   65                  70                  75                  80
   His Glu Leu His Asn Met Gly Ala Gln Gly Gly Gln Ile Pro Gln Val
                   85                  90                  95
   Pro Ser Gln Gln Gly Ala Ala Asn Gly Gly Gln Trp Ala Arg Asp Phe
               100                 105                 110
   Gly Gly Gln Gln Thr Ala Pro Gly Ala Ala Pro Gln Asp Ala Lys Asn
           115                 120                 125
   Trp Asn Ala Glu Phe Gln Arg Gly Gly Ser Pro Ala Glu Ala Met Gln
       130                 135                 140
   Gln Gln Gly Pro Gly Pro Met Gln Gly Gly Met Gly Met Gly Gly Met
   145                 150                 155                 160
   Pro Met Tyr Gly Met Ala Arg Pro Met Tyr Ser Gly Met Ser Ala Asn
                   165                 170                 175
   Met Ala Pro Gln Phe Gln Pro Gln Gln Ala Asn Ala Arg Val Val Glu
               180                 185                 190
   Leu Asp Glu Gln Asn Trp Glu Glu Gln Phe Lys Gln Met Asp Ser Ala
           195                 200                 205
   Val Gly Lys Gly Lys Glu Val Glu Glu Gln Thr Ala Glu Thr Ala Thr
       210                 215                 220
   Ala Thr Glu Thr Val Thr Glu Thr Glu Thr Thr Thr Glu Asp Lys Pro
   225                 230                 235                 240
   Met Asp Ile Lys Asn Met Asp Phe Glu Asn Ile Trp Lys Asn Leu Gln
                   245                 250                 255
   Val Asn Val Leu Asp Asn Met Asp Glu Trp Leu Glu Glu Thr Asn Ser
   260 265 270
   Pro Ala Trp Glu Arg Asp Phe His Glu Tyr Thr His Asn Arg Pro Glu
   275 280 285
   Phe Ala Asp Tyr Gln Phe Glu Glu Asn Asn Gln Phe Met Glu His Pro
   290 295 300
   Asp Pro Phe Lys Ile Gly Val Glu Leu Met Glu Thr Gly Gly Arg Leu
   305 310 315 320
   Ser Glu Ala Ala Leu Ala Phe Glu Ala Ala Val Gln Lys Asn Thr Glu
   325 330 335
   His Ala Glu Ala Trp Gly Arg Leu Gly Ala Cys Gln Ala Gln Asn Glu
   340 345 350
   Lys Glu Asp Pro Ala Ile Arg Ala Leu Glu Arg Cys Ile Lys Leu Glu
   355 360 365
   Pro Gly Asn Leu Ser Ala Leu Met Asn Leu Ser Val Ser Tyr Thr Asn
   370 375 380
   Glu Gly Tyr Glu Asn Ala Ala Tyr Ala Thr Leu Glu Arg Trp Leu Ala
   385 390 395 400
   Thr Lys Tyr Pro Glu Val Val Asp Gln Ala Arg Asn Gln Glu Pro Arg
   405 410 415
   Leu Gly Asn Glu Asp Lys Phe Gln Leu His Ser Arg Val Thr Glu Leu
   420 425 430
   Phe Ile Arg Ala Ala Gln Leu Ser Pro Asp Gly Ala Asn Ile Asp Ala
   435 440 445
   Asp Val Gln Val Gly Leu Gly Val Leu Phe Tyr Gly Asn Glu Glu Tyr
   450 455 460
   Asp Lys Ala Ile Asp Cys Phe Asn Ala Ala Ile Ala Val Arg Pro Asp
   465 470 475 480
   Asp Ala Leu Leu Trp Asn Arg Leu Gly Ala Thr Leu Ala Asn Ser His
   485 490 495
   Arg Ser Glu Glu Ala Ile Asp Ala Tyr Tyr Lys Ala Leu Glu Leu Arg
   500 505 510
   Pro Ser Phe Val Arg Ala Arg Tyr Asn Leu Gly Val Ser Cys Ile Asn
   515 520 525
   Ile Gly Cys Tyr Lys Glu Ala Ala Gln Tyr Leu Leu Gly Ala Leu Ser
       530                 535                 540
   Met His Lys Val Glu Gly Val Gln Asp Asp Val Leu Ala Asn Gln Ser
   545                 550                 555                 560
   Thr Asn Leu Tyr Asp Thr Leu Lys Arg Val Phe Leu Gly Met Asp Arg
                   565                 570                 575
   Arg Asp Leu Val Ala Lys Val Gly Asn Gly Met Asp Val Asn Gln Phe
               580                 585                 590
   Arg Asn Glu Phe Glu Phe
           595
<210> 114
   <211> 1024
   <212> PRT
   <213> Yarrowia lipolytica
<220>
   <221> MISC_FEATURE
   <222> (1)..(1024)
   <223> YlPex6p; GenBank Accession No. CAG82306
<400> 114
   Met Pro Ser Ile Ser His Lys Pro Ile Thr Ala Lys Leu Val Ala Ala
   1               5                   10                  15
   Pro Asp Ala Thr Lys Leu Glu Leu Ser Ser Tyr Leu Tyr Gln Gln Leu
               20                  25                  30
   Phe Ser Asp Lys Pro Ala Glu Pro Tyr Val Ala Phe Glu Ala Pro Gly
           35                  40                  45
   Ile Lys Trp Ala Leu Tyr Pro Ala Ser Glu Asp Arg Ser Leu Pro Gln
       50                  55                  60
   Tyr Thr Cys Lys Ala Asp Ile Arg His Val Ala Gly Ser Leu Lys Lys
   65                  70                  75                  80
   Phe Met Pro Val Val Leu Lys Arg Val Asn Pro Val Thr Ile Glu His
                   85                  90                  95
   Ala Ile Val Thr Val Pro Ala Ser Gln Tyr Glu Thr Leu Asn Thr Pro
               100                 105                 110
   Glu Gln Val Leu Lys Ala Leu Glu Pro Gln Leu Asp Lys Asp Arg Pro
           115                 120                 125
   Val Ile Arg Gln Gly Asp Val Leu Leu Asn Gly Cys Arg Val Arg Leu
   130 135 140
   Cys Glu Pro Val Asn Gln Gly Lys Val Val Lys Gly Thr Thr Lys Leu
   145 150 155 160
   Thr Val Ala Lys Glu Gln Glu Thr Ile Gln Pro Ala Asp Glu Ala Ala
   165 170 175
   Asp Val Ala Phe Asp Ile Ala Glu Phe Leu Asp Phe Asp Thr Ser Val
   180 185 190
   Ala Lys Thr Arg Glu Ser Thr Asn Leu Gln Val Ala Pro Leu Glu Gly
   195 200 205
   Ala Ile Pro Thr Pro Leu Ser Asp Arg Phe Asp Asp Cys Glu Ser Arg
   210 215 220
   Gly Phe Val Lys Ser Glu Thr Met Ser Lys Leu Gly Val Phe Ser Gly
   225 230 235 240
   Asp Ile Val Ser Ile Lys Thr Lys Asn Gly Ala Glu Arg Val Leu Arg
   245 250 255
   Leu Phe Ala Tyr Pro Glu Pro Asn Thr Val Lys Tyr Asp Val Val Tyr
   260 265 270
   Val Ser Pro Ile Leu Tyr His Asn Ile Gly Asp Lys Glu Ile Glu Val
   275 280 285
   Thr Pro Asn Gly Glu Thr His Lys Ser Val Gly Glu Ala Leu Asp Ser
   290 295 300
   Val Leu Glu Ala Ala Glu Glu Val Lys Leu Ala Arg Val Leu Gly Pro
   305 310 315 320
   Thr Thr Thr Asp Arg Thr Phe Gln Thr Ala Tyr His Ala Gly Leu Gln
   325 330 335
   Ala Tyr Phe Lys Pro Val Lys Arg Ala Val Arg Val Gly Asp Leu Ile
   340 345 350
   Pro Ile Pro Phe Asp Ser Ile Leu Ala Arg Thr Ile Gly Glu Asp Pro
   355 360 365
   Glu Met Ser His Ile Pro Leu Glu Ala Leu Ala Val Lys Pro Asp Ser
   370 375 380
   Val Ala Trp Phe Gln Val Thr Ser Leu Asn Gly Ser Glu Asp Pro Ala
   385 390 395 400
   Ser Lys Gln Tyr Leu Val Asp Ser Ser Gln Thr Lys Leu Ile Glu Gly
   405 410 415
   Gly Thr Thr Ser Ser Ala Val Ile Pro Thr Ser Val Pro Trp Arg Glu
   420 425 430
   Tyr Leu Gly Leu Asp Thr Leu Pro Lys Phe Gly Ser Glu Phe Ala Tyr
   435 440 445
   Ala Asp Lys Ile Arg Asn Leu Val Gln Ile Ser Thr Ser Ala Leu Ser
   450 455 460
   His Ala Lys Leu Asn Thr Ser Val Leu Leu His Ser Ala Lys Arg Gly
   465 470 475 480
   Val Gly Lys Ser Thr Val Leu Arg Ser Val Ala Ala Gln Cys Gly Ile
   485 490 495
   Ser Val Phe Glu Ile Ser Cys Phe Gly Leu Ile Gly Asp Asn Glu Ala
   500 505 510
   Gln Thr Leu Gly Thr Leu Arg Ala Lys Leu Asp Arg Ala Tyr Gly Cys
   515 520 525
   Ser Pro Cys Val Val Val Leu Gln His Leu Glu Ser Ile Ala Lys Lys
   530 535 540
   Ser Asp Gln Asp Gly Lys Asp Glu Gly Ile Val Ser Lys Leu Val Asp
   545 550 555 560
   Val Leu Ala Asp Tyr Ser Gly His Gly Val Leu Leu Ala Ala Thr Ser
   565 570 575
   Asn Asp Pro Asp Lys Ile Ser Glu Ala Ile Arg Ser Arg Phe Gln Phe
   580 585 590
   Glu Ile Glu Ile Gly Val Pro Ser Glu Pro Gln Arg Arg Gln Ile Phe
   595 600 605
   Ser His Leu Thr Lys Ser Gly Pro Gly Gly Asp Ser Ile Arg Asn Ala
   610 615 620
   Pro Ile Ser Leu Arg Ser Asp Val Ser Val Glu Asn Leu Ala Leu Gln
   625 630 635 640
   Ser Ala Gly Leu Thr Pro Pro Asp Leu Thr Ala Ile Val Gln Thr Thr
   645 650 655
   Arg Leu Arg Ala Ile Asp Arg Leu Asn Lys Leu Thr Lys Asp Ser Asp
   660 665 670
   Thr Thr Leu Asp Asp Leu Leu Thr Leu Ser His Gly Thr Leu Gln Leu
   675 680 685
   Thr Pro Ser Asp Phe Asp Asp Ala Ile Ala Asp Ala Arg Gln Lys Tyr
   690 695 700
   Ser Asp Ser Ile Gly Ala Pro Arg Ile Pro Asn Val Gly Trp Asp Asp
   705 710 715 720
   Val Gly Gly Met Glu Gly Val Lys Lys Asp Ile Leu Asp Thr Ile Glu
   725 730 735
   Thr Pro Leu Lys Tyr Pro His Trp Phe Ser Asp Gly Val Lys Lys Arg
   740 745 750
   Ser Gly Ile Leu Phe Tyr Gly Pro Pro Gly Thr Gly Lys Thr Leu Leu
   755 760 765
   Ala Lys Ala Ile Ala Thr Thr Phe Ser Leu Asn Phe Phe Ser Val Lys
   770 775 780
   Gly Pro Glu Leu Leu Asn Met Tyr Ile Gly Glu Ser Glu Ala Asn Val
   785 790 795 800
   Arg Arg Val Phe Gln Lys Ala Arg Asp Ala Lys Pro Cys Val Val Phe
   805 810 815
   Phe Asp Glu Leu Asp Ser Val Ala Pro Gln Arg Gly Asn Gln Gly Asp
   820 825 830
   Ser Gly Gly Val Met Asp Arg Ile Val Ser Gln Leu Leu Ala Glu Leu
   835 840 845
   Asp Gly Met Ser Thr Ala Gly Gly Glu Gly Val Phe Val Val Gly Ala
   850 855 860
   Thr Asn Arg Pro Asp Leu Leu Asp Glu Ala Leu Leu Arg Pro Gly Arg
   865 870 875 880
   Phe Asp Lys Met Leu Tyr Leu Gly Ile Ser Asp Thr His Glu Lys Gln
   885 890 895
   Gln Thr Ile Met Glu Ala Leu Thr Arg Lys Phe Arg Leu Ala Ala Asp
   900 905 910
   Val Ser Leu Glu Ala Ile Ser Lys Arg Cys Pro Phe Thr Phe Thr Gly
   915 920 925
   Ala Asp Phe Tyr Ala Leu Cys Ser Asp Ala Met Leu Asn Ala Met Thr
   930 935 940
   Arg Thr Ala Asn Glu Val Asp Ala Lys Ile Lys Leu Leu Asn Lys Asn
   945 950 955 960
   Arg Glu Glu Ala Gly Glu Glu Pro Val Ser Ile Arg Trp Trp Phe Asp
   965 970 975
   His Glu Ala Thr Lys Ser Asp Ile Glu Val Glu Val Ala Gln Gln Asp
   980 985 990
   Phe Glu Lys Ala Lys Asp Glu Leu Ser Pro Ser Val Ser Ala Glu Glu
   995 1000 1005
   Leu Gln His Tyr Leu Lys Leu Arg Gln Gln Phe Glu Gly Gly Lys
   1010 1015 1020
   Lys
<210> 115
   <211> 356
   <212> PRT
   <213> Yarrowia lipolytica
<220>
   <221> MISC_FEATURE
   <222> (1)..(356)
   <223> YlPex7p; GenBank Accession No. CAG78389
<400> 115
   Met Leu Gly Phe Lys Thr Gln Gly Phe Asn Gly Tyr Ala Ala Asn Tyr
   1               5                   10                  15
   Ser Pro Phe Phe Asn Asp Lys Ile Ala Val Gly Thr Ala Ala Asn Tyr
               20                  25                  30
   Gly Leu Val Gly Asn Gly Lys Leu Phe Ile Leu Gly Ile Ser Pro Glu
           35                  40                  45
   Gly Arg Met Val Cys Glu Gly Gln Phe Asp Thr Gln Asp Gly Ile Phe
       50                  55                  60
   Asp Val Ala Trp Ser Glu Gln His Glu Asn His Val Ala Thr Ala Cys
   65                  70                  75                  80
   Gly Asp Gly Ser Val Lys Leu Phe Asp Ile Lys Ala Gly Ala Phe Pro
                   85                  90                  95
   Leu Val Ser Phe Lys Glu His Thr Arg Glu Val Phe Ser Val Asn Trp
               100                 105                 110
   Asn Met Ala Asn Lys Ala Leu Phe Cys Thr Ser Ser Trp Asp Ser Thr
           115                 120                 125
   Ile Lys Ile Trp Thr Pro Glu Arg Thr Asn Ser Ile Met Thr Leu Gly
       130                 135                 140
   Gln Pro Ala Pro Ala Gln Gly Thr Asn Ala Ser Ala His Ile Gly Arg
   145                 150                 155                 160
   Gln Thr Ala Pro Asn Gln Ala Ala Ala Gln Glu Cys Ile Tyr Ser Ala
                   165                 170                 175
   Lys Phe Ser Pro His Thr Asp Ser Ile Ile Ala Ser Ala His Ser Thr
               180                 185                 190
   Gly Met Val Lys Val Trp Asp Thr Arg Ala Pro Gln Pro Leu Gln Gln
           195                 200                 205
   Gln Phe Ser Thr Gln Gln Thr Glu Ser Gly Gly Pro Pro Glu Val Leu
       210                 215                 220
   Ser Leu Asp Trp Asn Lys Tyr Arg Pro Thr Val Ile Ala Thr Gly Gly
   225                 230                 235                 240
   Val Asp Arg Ser Val Gln Val Tyr Asp Ile Arg Met Thr Gln Pro Ala
                   245                 250                 255
   Ala Asn Gln Pro Val Gln Pro Leu Ser Leu Ile Leu Gly His Arg Leu
               260                 265                 270
   Pro Val Arg Gly Val Ser Trp Ser Pro His His Ala Asp Leu Leu Leu
           275                 280                 285
   Ser Cys Ser Tyr Asp Met Thr Ala Arg Val Trp Arg Asp Ala Ser Thr
       290                 295                 300
   Gly Gly Asn Tyr Leu Ala Arg Gln Arg Gly Gly Thr Glu Val Lys Cys
   305                 310                 315                 320
   Met Asp Arg His Thr Glu Phe Val Ile Gly Gly Asp Trp Ser Leu Trp
                   325                 330                 335
   Gly Asp Pro Gly Trp Ile Thr Thr Val Gly Trp Asp Gln Met Val Tyr
               340                 345                 350
   Val Trp His Ala
           355
<210> 116
   <211> 671
   <212> PRT
   <213> Yarrowia lipolytica
<220>
   <221> MISC_FEATURE
   <222> (1)..(671)
   <223> YlPex8p; GenBank Accession No. CAG80447
<400> 116
   Met Asn Lys Tyr Leu Val Pro Pro Pro Gln Ala Asn Arg Thr Val Thr
   1               5                   10                  15
   Asn Leu Asp Leu Leu Ile Asn Asn Leu Arg Gly Ser Ser Thr Pro Gly
               20                  25                  30
   Ala Ala Glu Val Asp Thr Arg Asp Ile Leu Gln Arg Ile Val Phe Ile
           35                  40                  45
   Leu Pro Thr Ile Lys Asn Pro Leu Asn Leu Asp Leu Val Ile Lys Glu
       50                  55                  60
   Ile Ile Asn Ser Pro Arg Leu Leu Pro Pro Leu Ile Asp Leu His Asp
   65                  70                  75                  80
   Tyr Gln Gln Leu Thr Asp Ala Phe Arg Ala Thr Ile Lys Arg Lys Ala
                   85                  90                  95
   Leu Val Thr Asp Pro Thr Ile Ser Phe Glu Ala Trp Leu Glu Thr Cys
               100                 105                 110
   Phe Gln Val Ile Thr Arg Phe Ala Gly Pro Gly Trp Lys Lys Leu Pro
           115                 120                 125
   Leu Leu Ala Gly Leu Ile Leu Ala Asp Tyr Asp Ile Ser Ala Asp Gly
       130                 135                 140
   Pro Thr Leu Glu Arg Lys Pro Gly Phe Pro Ser Lys Leu Lys His Leu
   145                 150                 155                 160
   Leu Lys Arg Glu Phe Val Thr Thr Phe Asp Gln Cys Leu Ser Ile Asp
                   165                 170                 175
   Thr Arg Asn Arg Ser Asp Ala Thr Lys Trp Val Pro Val Leu Ala Cys
   180 185 190
   Ile Ser Ile Ala Gln Val Tyr Ser Leu Leu Gly Asp Val Ala Ile Asn
   195 200 205
   Tyr Arg Arg Phe Leu Gln Val Gly Leu Asp Leu Ile Phe Ser Asn Tyr
   210 215 220
   Gly Leu Glu Met Gly Thr Ala Leu Ala Arg Leu His Ala Glu Ser Gly
   225 230 235 240
   Gly Asp Ala Thr Thr Ala Gly Gly Leu Ile Gly Lys Lys Leu Lys Glu
   245 250 255
   Pro Val Val Ala Leu Leu Asn Thr Phe Ala His Ile Ala Ser Ser Cys
   260 265 270
   Ile Val His Val Asp Ile Asp Tyr Ile Asp Arg Ile Gln Asn Lys Ile
   275 280 285
   Ile Leu Val Cys Glu Asn Gln Ala Glu Thr Trp Arg Ile Leu Thr Ile
   290 295 300
   Glu Ser Pro Thr Val Met His His Gln Glu Ser Val Gln Tyr Leu Lys
   305 310 315 320
   Trp Glu Leu Phe Thr Leu Cys Ile Ile Met Gln Gly Ile Ala Asn Met
   325 330 335
   Leu Leu Thr Gln Lys Met Asn Gln Phe Met Tyr Leu Gln Leu Ala Tyr
   340 345 350
   Lys Gln Leu Gln Ala Leu His Ser Ile Tyr Phe Ile Val Asp Gln Met
   355 360 365
   Gly Ser Gln Phe Ala Ala Tyr Asp Tyr Val Phe Phe Ser Ala Ile Asp
   370 375 380
   Val Leu Leu Ser Glu Tyr Ala Pro Tyr Ile Lys Asn Arg Gly Thr Ile
   385 390 395 400
   Pro Pro Asn Lys Glu Phe Val Ala Glu Arg Leu Ala Ala Asn Leu Ala
   405 410 415
   Gly Thr Ser Asn Val Gly Ser His Leu Pro Ile Asp Arg Ser Arg Val
   420 425 430
   Leu Phe Ala Leu Asn Tyr Tyr Glu Gln Leu Val Thr Val Cys His Asp
   435 440 445
   Ser Cys Val Glu Thr Ile Ile Tyr Pro Met Ala Arg Ser Phe Leu Tyr
   450 455 460
   Pro Thr Ser Asp Ile Gln Gln Leu Lys Pro Leu Val Glu Ala Ala His
   465 470 475 480
   Ser Val Ile Leu Ala Gly Leu Ala Val Pro Thr Asn Ala Val Val Asn
   485 490 495
   Ala Lys Leu Ile Pro Glu Tyr Met Gly Gly Val Leu Pro Leu Phe Pro
   500 505 510
   Gly Val Phe Ser Trp Asn Gln Phe Val Leu Ala Ile Gln Ser Ile Val
   515 520 525
   Asn Thr Val Ser Pro Pro Ser Glu Val Phe Lys Thr Asn Gln Lys Leu
   530 535 540
   Phe Arg Leu Val Leu Asp Ser Leu Met Lys Lys Cys Arg Asp Thr Pro
   545 550 555 560
   Val Gly Ile Pro Val Pro His Ser Val Thr Val Ser Gln Glu Gln Glu
   565 570 575
   Asp Ile Pro Pro Thr Gln Arg Ala Val Val Met Leu Ala Leu Ile Asn
   580 585 590
   Ser Leu Pro Tyr Val Asp Ile Arg Ser Phe Glu Leu Trp Leu Gln Glu
   595 600 605
   Thr Trp Asn Met Ile Glu Ala Thr Pro Met Leu Ala Glu Asn Ala Pro
   610 615 620
   Asn Lys Glu Leu Ala His Ala Glu His Glu Phe Leu Val Leu Glu Met
   625 630 635 640
   Trp Lys Met Ile Ser Gly Asn Ile Asp Gln Arg Leu Asn Asp Val Ala
   645 650 655
   Ile Arg Trp Trp Tyr Lys Lys Asn Ala Arg Val His Gly Thr Leu
   660 665 670
<210> 117
   <211> 377
   <212> PRT
   <213> Yarrowia lipolytica
<220>
   <221> MISC_FEATURE
   <222> (1)..(377)
   <223> YlPex10p; GenBank Accession No. CAG81606
<400> 117
   Met Trp Gly Ser Ser His Ala Phe Ala Gly Glu Ser Asp Leu Thr Leu
   1               5                   10                  15
   Gln Leu His Thr Arg Ser Asn Met Ser Asp Asn Thr Thr Ile Lys Lys
               20                  25                  30
   Pro Ile Arg Pro Lys Pro Ile Arg Thr Glu Arg Leu Pro Tyr Ala Gly
           35                  40                  45
   Ala Ala Glu Ile Ile Arg Ala Asn Gln Lys Asp His Tyr Phe Glu Ser
       50                  55                  60
   Val Leu Glu Gln His Leu Val Thr Phe Leu Gln Lys Trp Lys Gly Val
   65                  70                  75                  80
   Arg Phe Ile His Gln Tyr Lys Glu Glu Leu Glu Thr Ala Ser Lys Phe
                   85                  90                  95
   Ala Tyr Leu Gly Leu Cys Thr Leu Val Gly Ser Lys Thr Leu Gly Glu
               100                 105                 110
   Glu Tyr Thr Asn Leu Met Tyr Thr Ile Arg Asp Arg Thr Ala Leu Pro
           115                 120                 125
   Gly Val Val Arg Arg Phe Gly Tyr Val Leu Ser Asn Thr Leu Phe Pro
       130                 135                 140
   Tyr Leu Phe Val Arg Tyr Met Gly Lys Leu Arg Ala Lys Leu Met Arg
   145                 150                 155                 160
   Glu Tyr Pro His Leu Val Glu Tyr Asp Glu Asp Glu Pro Val Pro Ser
                   165                 170                 175
   Pro Glu Thr Trp Lys Glu Arg Val Ile Lys Thr Phe Val Asn Lys Phe
               180                 185                 190
   Asp Lys Phe Thr Ala Leu Glu Gly Phe Thr Ala Ile His Leu Ala Ile
           195                 200                 205
   Phe Tyr Val Tyr Gly Ser Tyr Tyr Gln Leu Ser Lys Arg Ile Trp Gly
       210                 215                 220
   Met Arg Tyr Val Phe Gly His Arg Leu Asp Lys Asn Glu Pro Arg Ile
   225                 230                 235                 240
   Gly Tyr Glu Met Leu Gly Leu Leu Ile Phe Ala Arg Phe Ala Thr Ser
                   245                 250                 255
   Phe Val Gln Thr Gly Arg Glu Tyr Leu Gly Ala Leu Leu Glu Lys Ser
               260                 265                 270
   Val Glu Lys Glu Ala Gly Glu Lys Glu Asp Glu Lys Glu Ala Val Val
           275                 280                 285
   Pro Lys Lys Lys Ser Ser Ile Pro Phe Ile Glu Asp Thr Glu Gly Glu
       290                 295                 300
   Thr Glu Asp Lys Ile Asp Leu Glu Asp Pro Arg Gln Leu Lys Phe Ile
   305                 310                 315                 320
   Pro Glu Ala Ser Arg Ala Cys Thr Leu Cys Leu Ser Tyr Ile Ser Ala
                   325                 330                 335
   Pro Ala Cys Thr Pro Cys Gly His Phe Phe Cys Trp Asp Cys Ile Ser
               340                 345                 350
   Glu Trp Val Arg Glu Lys Pro Glu Cys Pro Leu Cys Arg Gln Gly Val
           355                 360                 365
   Arg Glu Gln Asn Leu Leu Pro Ile Arg
       370                 375
<210> 118
   <211> 408
   <212> PRT
   <213> Yarrowia lipolytica
<220>
   <221> MISC_FEATURE
   <222> (1)..(408)
   <223> YlPex12p; GenBank Accession No. CAG81532
<400> 118
   Met Asp Tyr Phe Ser Ser Leu Asn Ala Ser Gln Leu Asp Pro Asp Val
   1               5                   10                  15
   Pro Thr Leu Phe Glu Leu Leu Ser Ala Lys Gln Leu Glu Gly Leu Ile
               20                  25                  30
   Ala Pro Ser Val Arg Tyr Ile Leu Ala Phe Tyr Ala Gln Arg His Pro
           35                  40                  45
   Arg Tyr Leu Leu Arg Ile Val Asn Arg Tyr Asp Glu Leu Tyr Ala Leu
       50                  55                  60
   Phe Met Gly Leu Val Glu Tyr Tyr Asn Leu Lys Thr Trp Asn Ala Ser
   65                  70                  75                  80
   Phe Thr Glu Lys Phe Tyr Gly Leu Lys Arg Thr Gln Ile Leu Thr Asn
                   85                  90                  95
   Pro Ala Leu Arg Thr Arg Gln Ala Val Pro Asp Leu Val Glu Ala Glu
               100                 105                 110
   Lys Arg Leu Ser Lys Lys Lys Ile Trp Gly Ser Leu Phe Phe Leu Ile
           115                 120                 125
   Val Val Pro Tyr Val Lys Glu Lys Leu Asp Ala Arg Tyr Glu Arg Leu
       130                 135                 140
   Lys Gly Arg Tyr Leu Ala Arg Asp Ile Asn Glu Glu Arg Ile Glu Ile
   145                 150                 155                 160
   Lys Arg Thr Gly Thr Ala Gln Gln Ile Ala Val Phe Glu Phe Asp Tyr
                   165                 170                 175
   Trp Leu Leu Lys Leu Tyr Pro Ile Val Thr Met Gly Cys Thr Thr Ala
               180                 185                 190
   Thr Leu Ala Phe His Met Leu Phe Leu Phe Ser Val Thr Arg Ala Tyr
           195                 200                 205
   Ser Ile Asp Asp Phe Leu Leu Asn Ile Gln Phe Ser Arg Met Thr Arg
       210                 215                 220
   Tyr Asp Tyr Gln Met Glu Thr Gln Arg Asp Ser Arg Asn Ala Ala Asn
   225                 230                 235                 240
   Val Ala His Thr Met Lys Ser Ile Ser Glu Tyr Pro Val Ala Glu Arg
                   245                 250                 255
   Val Met Leu Leu Leu Thr Thr Lys Ala Gly Ala Asn Ala Met Arg Ser
               260                 265                 270
   Ala Ala Leu Ser Gly Leu Ser Tyr Val Leu Pro Thr Ser Ile Phe Ala
           275                 280                 285
   Leu Lys Phe Leu Glu Trp Trp Tyr Ala Ser Asp Phe Ala Arg Gln Leu
       290                 295                 300
   Asn Gln Lys Arg Arg Gly Asp Leu Glu Asp Asn Leu Pro Val Pro Asp
   305                 310                 315                 320
   Lys Val Lys Gly Ala Asp Lys Leu Ala Glu Ser Val Ala Lys Trp Lys
                   325                 330                 335
   Glu Asp Thr Ser Lys Cys Pro Leu Cys Ser Lys Glu Leu Val Asn Pro
               340                 345                 350
   Thr Val Ile Glu Ser Gly Tyr Val Phe Cys Tyr Thr Cys Ile Tyr Arg
           355                 360                 365
   His Leu Glu Asp Gly Asp Glu Glu Thr Gly Gly Arg Cys Pro Val Thr
       370                 375                 380
   Gly Gln Lys Leu Leu Gly Cys Arg Trp Gln Asp Asp Val Trp Gln Val
   385                 390                 395                 400
   Thr Gly Leu Arg Arg Leu Met Val
                   405
<210> 119
   <211> 412
   <212> PRT
   <213> Yarrowia lipolytica
<220>
   <221> MISC_FEATURE
   <222> (1)..(412)
   <223> YlPex13p; GenBank Accession No. CAG81789
<400> 119
   Met Ser Val Pro Arg Pro Lys Pro Trp Glu Gly Ala Ser Gly Ser Ser
   1               5                   10                  15
   Ala Ala Thr Ala Thr Pro Ala Ala Thr Ala Thr Pro Ala Ser Thr Asp
               20                  25                  30
   Ala Val Ser Ser Ser Ala Gly Ser Ala Thr Gly Ala Pro Glu Leu Pro
           35                  40                  45
   Ser Arg Pro Ser Ala Met Gly Ser Thr Ser Asn Ala Leu Ser Ser Pro
       50                  55                  60
   Met Gly Ser Ser Met Asn Ser Gly Tyr Gly Gly Met Asn Ser Gly Tyr
   65                  70                  75                  80
   Gly Gly Met Gly Ser Ser Tyr Gly Ser Gly Tyr Gly Ser Ser Tyr Gly
                   85                  90                  95
   Met Gly Ser Ser Tyr Gly Ser Gly Tyr Gly Ser Gly Leu Gly Gly Tyr
   100 105 110
   Gly Ser Tyr Gly Gly Met Gly Gly Met Gly Gly Met Tyr Gly Ser Arg
   115 120 125
   Tyr Gly Gly Tyr Gly Ser Tyr Gly Gly Met Gly Gly Tyr Gly Gly Tyr
   130 135 140
   Gly Gly Met Gly Gly Gly Pro Met Gly Gln Asn Gly Leu Ala Gly Gly
   145 150 155 160
   Thr Gln Ala Thr Phe Gln Leu Ile Glu Ser Ile Val Gly Ala Val Gly
   165 170 175
   Gly Phe Ala Gln Met Leu Glu Ser Thr Tyr Met Ala Thr Gln Ser Ser
   180 185 190
   Phe Phe Ala Met Val Ser Val Ala Glu Gln Phe Gly Asn Leu Lys Asn
   195 200 205
   Thr Leu Gly Ser Leu Leu Gly Ile Tyr Ala Ile Met Arg Trp Ala Arg
   210 215 220
   Arg Leu Val Ala Lys Leu Ser Gly Gln Pro Val Thr Gly Ala Asn Gly
   225 230 235 240
   Ile Thr Pro Ala Gly Phe Ala Lys Phe Glu Ala Thr Gly Gly Ala Ala
   245 250 255
   Gly Pro Gly Arg Gly Pro Arg Pro Ser Tyr Lys Pro Leu Leu Phe Phe
   260 265 270
   Leu Thr Ala Val Phe Gly Leu Pro Tyr Leu Leu Gly Arg Leu Ile Lys
   275 280 285
   Ala Leu Ala Ala Lys Gln Glu Gly Met Tyr Asp Glu His Gly Asn Leu
   290 295 300
   Leu Pro Gly Ala Gln Met Gly Met Gly Gly Pro Gly Met Glu Gly Gly
   305 310 315 320
   Ala Glu Ile Asp Pro Ser Lys Leu Glu Phe Cys Arg Ala Asn Phe Asp
   325 330 335
   Phe Val Pro Glu Asn Pro Gln Leu Glu Leu Glu Leu Arg Lys Gly Asp
   340 345 350
   Leu Val Ala Val Leu Ala Lys Thr Asp Pro Met Gly Asn Pro Ser Gln
   355 360 365
   Trp Trp Arg Val Arg Thr Arg Asp Gly Arg Ser Gly Tyr Val Pro Ala
   370 375 380
   Asn Tyr Leu Glu Val Ile Pro Arg Pro Ala Val Glu Ala Pro Lys Lys
   385 390 395 400
   Val Glu Glu Ile Gly Ala Ser Ala Val Pro Val Asn
   405 410
<210> 120
   <211> 380
   <212> PRT
   <213> Yarrowia lipolytica
<220>
   <221> MISC_FEATURE
   <222> (1)..(380)
   <223> YlPex14p; GenBank Accession No. CAG79323
<400> 120
   Met Ile Pro Ser Cys Leu Ser Thr Gln His Met Ala Pro Arg Glu Asp
   1               5                   10                  15
   Leu Val Gln Ser Ala Val Ala Phe Leu Asn Asp Pro Gln Ala Ala Thr
               20                  25                  30
   Ala Pro Leu Ala Lys Arg Ile Glu Phe Leu Glu Ser Lys Asp Met Thr
           35                  40                  45
   Pro Glu Glu Ile Glu Glu Ala Leu Lys Arg Ala Gly Ser Gly Ser Ala
       50                  55                  60
   Gln Ser His Pro Gly Ser Val Val Ser His Gly Gly Ala Ala Pro Thr
   65                  70                  75                  80
   Val Pro Ala Ser Tyr Ala Phe Gln Ser Ala Pro Pro Leu Pro Glu Arg
                   85                  90                  95
   Asp Trp Lys Asp Val Phe Ile Met Ala Thr Val Thr Val Gly Val Gly
               100                 105                 110
   Phe Gly Leu Tyr Thr Val Ala Lys Arg Tyr Leu Met Pro Leu Ile Leu
           115                 120                 125
   Pro Pro Thr Pro Pro Ser Leu Glu Ala Asp Lys Glu Ala Leu Glu Ala
       130                 135                 140
   Glu Phe Ala Arg Val Gln Gly Leu Leu Asp Gln Val Gln Gln Asp Thr
   145                 150                 155                 160
   Glu Glu Val Lys Asn Ser Gln Val Glu Val Ala Lys Arg Val Thr Asp
                   165                 170                 175
   Ala Leu Lys Gly Val Glu Glu Thr Ile Asp Gln Leu Lys Ser Gln Thr
               180                 185                 190
   Lys Lys Arg Asp Asp Glu Met Lys Leu Val Thr Ala Glu Val Glu Arg
           195                 200                 205
   Ile Arg Asp Arg Leu Pro Lys Asn Ile Asp Lys Leu Lys Asp Ser Gln
       210                 215                 220
   Glu Gln Gly Leu Ala Asp Ile Gln Ser Glu Leu Lys Ser Leu Lys Gln
   225                 230                 235                 240
   Leu Leu Ser Thr Arg Thr Ala Ala Ser Ser Gly Pro Lys Leu Pro Pro
                   245                 250                 255
   Ile Pro Pro Pro Ser Ser Tyr Leu Thr Arg Lys Ala Ser Pro Ala Val
               260                 265                 270
   Pro Ala Ala Ala Pro Ala Pro Val Thr Pro Gly Ser Pro Val His Asn
           275                 280                 285
   Val Ser Ser Ser Ser Thr Val Pro Ala Asp Arg Asp Asp Phe Ile Pro
       290                 295                 300
   Thr Pro Ala Gly Ala Val Pro Met Ile Pro Gln Pro Ala Ser Met Ser
   305                 310                 315                 320
   Ser Ser Ser Thr Ser Thr Val Pro Asn Ser Ala Ile Ser Ser Ala Pro
                   325                 330                 335
   Ser Pro Ile Gln Glu Pro Glu Pro Phe Val Pro Glu Pro Gly Asn Ser
               340                 345                 350
   Ala Val Lys Lys Pro Ala Pro Lys Ala Ser Ile Pro Ala Trp Gln Leu
           355                 360                 365
   Ala Ala Leu Glu Lys Glu Lys Glu Lys Glu Lys Glu
       370                 375                 380
<210> 121
   <211> 391
   <212> PRT
   <213> Yarrowia lipolytica
<220>
   <221> MISC_FEATURE
   <222> (1)..(391)
   <223> YlPex16p; GenBank Accession No. CAG79622
<400> 121
   Met Thr Asp Lys Leu Val Lys Val Met Gln Lys Lys Lys Ser Ala Pro
   1               5                   10                  15
   Gln Thr Trp Leu Asp Ser Tyr Asp Lys Phe Leu Val Arg Asn Ala Ala
               20                  25                  30
   Ser Ile Gly Ser Ile Glu Ser Thr Leu Arg Thr Val Ser Tyr Val Leu
           35                  40                  45
   Pro Gly Arg Phe Asn Asp Val Glu Ile Ala Thr Glu Thr Leu Tyr Ala
       50                  55                  60
   Val Leu Asn Val Leu Gly Leu Tyr His Asp Thr Ile Ile Ala Arg Ala
   65                  70                  75                  80
   Val Ala Ala Ser Pro Asn Ala Ala Ala Val Tyr Arg Pro Ser Pro His
                   85                  90                  95
   Asn Arg Tyr Thr Asp Trp Phe Ile Lys Asn Arg Lys Gly Tyr Lys Tyr
               100                 105                 110
   Ala Ser Arg Ala Val Thr Phe Val Lys Phe Gly Glu Leu Val Ala Glu
           115                 120                 125
   Met Val Ala Lys Lys Asn Gly Gly Glu Met Ala Arg Trp Lys Cys Ile
       130                 135                 140
   Ile Gly Ile Glu Gly Ile Lys Ala Gly Leu Arg Ile Tyr Met Leu Gly
   145                 150                 155                 160
   Ser Thr Leu Tyr Gln Pro Leu Cys Thr Thr Pro Tyr Pro Asp Arg Glu
                   165                 170                 175
   Val Thr Gly Glu Leu Leu Glu Thr Ile Cys Arg Asp Glu Gly Glu Leu
               180                 185                 190
   Asp Ile Glu Lys Gly Leu Met Asp Pro Gln Trp Lys Met Pro Arg Thr
           195                 200                 205
   Gly Arg Thr Ile Pro Glu Ile Ala Pro Thr Asn Val Glu Gly Tyr Leu
       210                 215                 220
   Leu Thr Lys Val Leu Arg Ser Glu Asp Val Asp Arg Pro Tyr Asn Leu
   225                 230                 235                 240
   Leu Ser Arg Leu Asp Asn Trp Gly Val Val Ala Glu Leu Leu Ser Ile
   245 250 255
   Leu Arg Pro Leu Ile Tyr Ala Cys Leu Leu Phe Arg Gln His Val Asn
   260 265 270
   Lys Thr Val Pro Ala Ser Thr Lys Ser Lys Phe Pro Phe Leu Asn Ser
   275 280 285
   Pro Trp Ala Pro Trp Ile Ile Gly Leu Val Ile Glu Ala Leu Ser Arg
   290 295 300
   Lys Met Met Gly Ser Trp Leu Leu Arg Gln Arg Gln Ser Gly Lys Thr
   305 310 315 320
   Pro Thr Ala Leu Asp Gln Met Glu Val Lys Gly Arg Thr Asn Leu Leu
   325 330 335
   Gly Trp Trp Leu Phe Arg Gly Glu Phe Tyr Gln Ala Tyr Thr Arg Pro
   340 345 350
   Leu Leu Tyr Ser Ile Val Ala Arg Leu Glu Lys Ile Pro Gly Leu Gly
   355 360 365
   Leu Phe Gly Ala Leu Ile Ser Asp Tyr Leu Tyr Leu Phe Asp Arg Tyr
   370 375 380
   Tyr Phe Thr Ala Ser Thr Leu
   385 390
<210> 122
   <211> 225
   <212> PRT
   <213> Yarrowia lipolytica
<220>
   <221> MISC_FEATURE
   <222> (1)..(225)
   <223> YlPex17p; GenBank Accession No. CAG84025
<400> 122
   Met Ser Ala Phe Pro Glu Pro Ser Ser Phe Glu Ile Glu Phe Ala Lys
   1               5                   10                  15
   Gln Met Asn Arg Pro Arg Thr Val Gln Phe Lys Gln Leu Val Ala Val
               20                  25                  30
   Leu Tyr Ile Phe Gly Gly Thr Ser Ala Leu Ile Tyr Ile Ile Ser Lys
           35                  40                  45
   Thr Ile Leu Asn Pro Leu Phe Glu Glu Leu Thr Phe Ala Arg Ser Glu
       50                  55                  60
   Tyr Ala Ile His Ala Arg Arg Leu Met Glu Gln Leu Asn Ala Lys Leu
   65                  70                  75                  80
   Ser Ser Met Ala Ser Tyr Ile Pro Pro Val Arg Ala Leu Gln Gly Gln
                   85                  90                  95
   Arg Phe Val Asp Ala Gln Thr Gln Thr Glu Asp Glu Glu Gly Glu Asp
               100                 105                 110
   Ile Pro Asn Pro Ser Leu Gly Lys Ser Ser His Val Ser Phe Gly Glu
           115                 120                 125
   Ser Pro Met Gln Leu Lys Leu Ala Glu Lys Glu Lys Gln Gln Lys Leu
       130                 135                 140
   Ile Asp Asp Ser Val Asp Asn Leu Glu Arg Leu Ala Asp Ser Leu Lys
   145                 150                 155                 160
   His Ala Gly Glu Val Ser Asp Leu Ser Ala Leu Ser Gly Phe Lys Tyr
                   165                 170                 175
   Gln Val Glu Glu Leu Thr Asn Tyr Ser Asp Gln Leu Ala Met Ser Gly
               180                 185                 190
   Tyr Ser Met Met Lys Ser Gly Leu Pro Gly His Glu Thr Ala Met Ser
           195                 200                 205
   Glu Thr Lys Lys Glu Ile Arg Ser Leu Lys Gly Ser Val Leu Ser Val
       210                 215                 220
   Arg
   225
<210> 123
   <211> 324
   <212> PRT
   <213> Yarrowia lipolytica
<220>
   <221> MISC_FEATURE
   <222> (1)..(324)
   <223> YlPex19p; GenBank Accession No. AAK84827
<400> 123
   Met Ser His Glu Glu Asp Leu Asp Asp Leu Asp Asp Phe Leu Asp Glu
   1               5                   10                  15
   Phe Asp Glu Gln Val Leu Ser Lys Pro Pro Gly Ala Gln Lys Asp Ala
               20                  25                  30
   Thr Pro Thr Thr Ser Thr Ala Pro Thr Thr Ala Glu Ala Lys Pro Asp
           35                  40                  45
   Ala Thr Lys Lys Ser Thr Glu Thr Ser Gly Thr Asp Ser Lys Thr Glu
       50                  55                  60
   Gly Ala Asp Thr Ala Asp Lys Asn Ala Ala Thr Asp Ser Ala Glu Ala
   65                  70                  75                  80
   Gly Ala Glu Lys Val Ser Leu Pro Asn Leu Glu Asp Gln Leu Ala Gly
                   85                  90                  95
   Leu Lys Met Asp Asp Phe Leu Lys Asp Ile Glu Ala Asp Pro Glu Ser
               100                 105                 110
   Lys Ala Gln Phe Glu Ser Leu Leu Lys Glu Ile Asn Asn Val Thr Ser
           115                 120                 125
   Ala Thr Ala Ser Glu Lys Ala Gln Gln Pro Lys Ser Phe Lys Glu Thr
       130                 135                 140
   Ile Ser Ala Thr Ala Asp Arg Leu Asn Gln Ser Asn Gln Glu Met Gly
   145                 150                 155                 160
   Asp Met Pro Leu Gly Asp Asp Met Leu Ala Gly Leu Met Glu Gln Leu
                   165                 170                 175
   Ser Gly Ala Gly Gly Phe Gly Glu Gly Gly Glu Gly Asp Phe Gly Asp
               180                 185                 190
   Met Leu Gly Gly Ile Met Arg Gln Leu Ala Ser Lys Glu Val Leu Tyr
           195                 200                 205
   Gln Pro Leu Lys Glu Met His Asp Asn Tyr Pro Lys Trp Trp Asp Glu
       210                 215                 220
   His Gly Ser Lys Val Thr Glu Glu Lys Glu Arg Asp Arg Leu Lys Leu
   225                 230                 235                 240
   Gln Gln Asp Ile Val Gly Lys Ile Cys Ala Lys Phe Glu Asp Pro Ser
                   245                 250                 255
   Tyr Ser Asp Asp Ser Glu Ala Asp Arg Ala Val Ile Thr Gln Leu Met
               260                 265                 270
   Asp Glu Met Gln Glu Thr Gly Ala Pro Pro Asp Glu Ile Met Ser Asn
   275 280 285
   Val Ala Asp Gly Ser Ile Pro Gly Gly Leu Asp Gly Leu Gly Leu Gly
   290 295 300
   Gly Leu Gly Gly Gly Lys Met Pro Glu Met Pro Glu Asn Met Pro Glu
   305 310 315 320
   Cys Asn Gln Gln
<210> 124
   <211> 417
   <212> PRT
   <213> Yarrowia lipolytica
<220>
   <221> MISC_FEATURE
   <222> (1)..(417)
   <223> YlPex20p; GenBank Accession No. CAG79226
<400> 124
   Met Ala Ser Cys Gly Pro Ser Asn Ala Leu Gln Asn Leu Ser Lys His
   1 5 10 15
   Ala Ser Ala Asp Arg Ser Leu Gln His Asp Arg Met Ala Pro Gly Gly
   20 25 30
   Ala Pro Gly Ala Gln Arg Gln Gln Phe Arg Ser Gln Thr Gln Gly Gly
   35 40 45
   Gln Leu Asn Asn Glu Phe Gln Gln Phe Ala Gln Ala Gly Pro Ala His
   50 55 60
   Asn Ser Phe Glu Gln Ser Gln Met Gly Pro His Phe Gly Gln Gln His
   65 70 75 80
   Phe Gly Gln Pro His Gln Pro Gln Met Gly Gln His Ala Pro Met Ala
   85 90 95
   His Gly Gln Gln Ser Asp Trp Ala Gln Ser Phe Ser Gln Leu Asn Leu
   100 105 110
   Gly Pro Gln Thr Gly Pro Gln His Thr Gln Gln Ser Asn Trp Gly Gln
   115 120 125
   Asp Phe Met Arg Gln Ser Pro Gln Ser His Gln Val Gln Pro Gln Met
   130 135 140
   Ala Asn Gly Val Met Gly Ser Met Ser Gly Met Ser Ser Phe Gly Pro
   145                 150                 155                 160
   Met Tyr Ser Asn Ser Gln Leu Met Asn Ser Thr Tyr Gly Leu Gln Thr
                   165                 170                 175
   Glu His Gln Gln Thr His Lys Thr Glu Thr Lys Ser Ser Gln Asp Ala
               180                 185                 190
   Ala Phe Glu Ala Ala Phe Gly Ala Val Glu Glu Ser Ile Thr Lys Thr
           195                 200                 205
   Ser Asp Lys Gly Lys Glu Val Glu Lys Asp Pro Met Glu Gln Thr Tyr
       210                 215                 220
   Arg Tyr Asp Gln Ala Asp Ala Leu Asn Arg Gln Ala Glu His Ile Ser
   225                 230                 235                 240
   Asp Asn Ile Ser Arg Glu Glu Val Asp Ile Lys Thr Asp Glu Asn Gly
                   245                 250                 255
   Glu Phe Ala Ser Ile Ala Arg Gln Ile Ala Ser Ser Leu Glu Glu Ala
               260                 265                 270
   Asp Lys Ser Lys Phe Glu Lys Ser Thr Phe Met Asn Leu Met Arg Arg
           275                 280                 285
   Ile Gly Asn His Glu Val Thr Leu Asp Gly Asp Lys Leu Val Asn Lys
       290                 295                 300
   Glu Gly Glu Asp Ile Arg Glu Glu Val Arg Asp Glu Leu Leu Arg Glu
   305                 310                 315                 320
   Gly Ala Ser Gln Glu Asn Gly Phe Gln Ser Glu Ala Gln Gln Thr Ala
                   325                 330                 335
   Pro Leu Pro Val His His Glu Ala Pro Pro Pro Glu Gln Ile His Pro
               340                 345                 350
   His Thr Glu Thr Gly Asp Lys Gln Leu Glu Asp Pro Met Val Tyr Ile
           355                 360                 365
   Glu Gln Glu Ala Ala Arg Arg Ala Ala Glu Ser Gly Arg Thr Val Glu
       370                 375                 380
   Glu Glu Lys Leu Asn Phe Tyr Ser Pro Phe Glu Tyr Ala Gln Lys Leu
   385                 390                 395                 400
   Gly Pro Gln Gly Val Ala Lys Gln Ser Asn Trp Glu Glu Asp Tyr Asp
   405 410 415
   Phe
<210> 125
   <211> 195
   <212> PRT
   <213> Yarrowia lipolytica
<220>
   <221> MISC_FEATURE
   <222> (1)..(195)
   <223> YlPex22p; GenBank Accession No. CAG77876
<400> 125
   Val Pro Arg Cys Thr Ser His Pro Cys Asn Leu Thr Leu His Leu Pro
   1               5                   10                  15
   Val Thr Thr Met Ala Pro Arg Lys Thr Arg Leu Pro Ala Val Ile Gly
               20                  25                  30
   Ala Ala Ala Ala Ala Ala Ala Val Ala Tyr Leu Val Tyr Ser Phe Val
           35                  40                  45
   Ala Lys Ser Asn Ser Asp Gln Asp Thr Phe Asp Ser Ser Val Gln Ser
       50                  55                  60
   Ser Ser Lys Ser Ser Thr Lys Ser Pro Lys Ser Thr Ala Thr Asn Ser
   65                  70                  75                  80
   Lys Ile Thr Val Val Val Ser Gln Glu Leu Val Gln Ser Gln Leu Val
                   85                  90                  95
   Asp Phe Lys His Leu Met Ser Val His Pro Asn Leu Val Val Ile Val
               100                 105                 110
   Pro Pro Met Val Ala Asn Lys Phe His Arg Ala Leu Lys Ser Ser Val
           115                 120                 125
   Gly His Asp His Gly Val Lys Val Ile Arg Cys Asp Thr Asp Val Gly
       130                 135                 140
   Val Ile His Val Ile Lys His Ile Arg Pro Asp Leu Ala Leu Ile Ala
   145                 150                 155                 160
   Asp Gly Val Gly Asp Asn Ile Gln Gly Glu Ile Lys Arg Phe Val Gly
                   165                 170                 175
   Ser Ser Glu Ala Leu Ser Gly Asp Val Asn Leu Ala Ala Glu Arg Leu
                   180                 185                 190
   Thr Gly Leu
           195
<210> 126
   <211> 386
   <212> PRT
   <213> Yarrowia lipolytica
<220>
   <221> MISC_FEATURE
   <222> (1)..(386)
   <223> YlPex26p; GenBank Accession No. NC_006072, antisense translation of nucleotides 117230-118387
<400> 126
   Met Pro Pro Ala Met Pro Gln Met Thr Thr Ser Thr Leu Leu Thr Asp
   1               5                   10                  15
   Ser Val Thr Ser Ala Val Asn Gln Ala Ala Thr Pro Lys Val Asp Gln
               20                  25                  30
   Met Tyr Gln Thr Phe Gly Glu Ser Ala Arg Glu Phe Val Asn Lys Asn
           35                  40                  45
   Phe Tyr Asn Ser Tyr Glu Leu Ile Arg Pro Phe Phe Asp Glu Ile Thr
       50                  55                  60
   Ala Lys Gly Ala Gln Gln Asn Gly Ser Thr Val Leu Asp Ala Glu Asn
   65                  70                  75                  80
   Pro His Asn Ile Pro Leu Ser Leu Trp Ile Lys Val Trp Ser Leu Tyr
                   85                  90                  95
   Leu Ala Ile Leu Asp Ala Ser Cys Lys Gln Ala Gly Glu Ala Leu Leu
               100                 105                 110
   Asn Ser Thr Gly Asp Leu Ser Gly Ser Asp Ser Gly Glu Trp Asn Gln
           115                 120                 125
   Thr Arg Lys Leu Leu Ala Arg Lys Leu Thr Ser Gly Ser Val Trp Asp
       130                 135                 140
   Glu Leu Val Thr Ala Ser Gly Gly Thr Gly Asn Ile His Pro Thr Ile
   145                 150                 155                 160
   Leu Ala Leu Leu Ala Ser Leu Ser Ile Arg His Asp Thr Asp Ala Lys
                   165                 170                 175
   Leu Met Ala Asp Asn Leu Glu Lys Phe Ile Val Thr Tyr Asn Asp Asn
   180 185 190
   Gly Ser Asp Asp Val Lys Thr Lys Thr Ala Phe Tyr Lys Val Leu Asp
   195 200 205
   Leu Tyr Leu Leu Arg Val Leu Pro Asp Leu Gly Gln Trp Asp Val Ala
   210 215 220
   His Ser Phe Val Asn Asn Thr Asn Leu Phe Ser His Glu Gln Lys Lys
   225 230 235 240
   Glu Met Thr His Lys Leu Asp Gln Ser Gln Lys His Ala Glu Gln Glu
   245 250 255
   His Lys Arg Leu Leu Glu Glu Ala Gln Glu Lys Glu Lys Ser Asp Ala
   260 265 270
   Lys Glu Lys Glu Arg Glu Glu Arg Val Ser Arg Asp Thr Gln Ser Arg
   275 280 285
   Glu Ile Lys Ser Pro Ile Val Asp Ser Ser Thr Ser Ser Arg Asp Val
   290 295 300
   Thr Arg Asp Thr Thr Arg Glu Leu Ser Lys Ser Ser Arg Gln Pro Arg
   305 310 315 320
   Thr Leu Ser Gln Ile Ile Ser Thr Ser Leu Lys Ser Gln Phe Asp Gly
   325 330 335
   Asn Ala Ile Phe Arg Thr Leu Ala Leu Ile Val Ile Val Ser Leu Ser
   340 345 350
   Ala Ala Asn Pro Leu Ile Arg Lys Arg Val Val Asp Thr Leu Lys Met
   355 360 365
   Leu Trp Ile Lys Ile Leu Gln Thr Leu Ser Met Gly Phe Lys Val Ser
   370 375 380
   Tyr Leu
   385
<210> 127
   <211> 8739
   <212> DNA
   <213> Artificial Sequence
<220>
   <223> Plasmid pY116
<400> 127
   ggccgccacc gcggcccgag attccggcct cttcggccgc caagcgaccc gggtggacgt 60
   ctagaggtac ctagcaatta acagatagtt tgccggtgat aattctctta acctcccaca 120
   ctcctttgac ataacgattt atgtaacgaa actgaaattt gaccagatat tgtgtccgcg 180
   gtggagctcc agcttttgtt ccctttagtg agggtttaaa cgagcttggc gtaatcatgg 240
   tcatagctgt ttcctgtgtg aaattgttat ccgctcacaa ttccacacaa cgtacgagcc 300
   ggaagcataa agtgtaaagc ctggggtgcc taatgagtga gctaactcac attaattgcg 360
   ttgcgctcac tgcccgcttt ccagtcggga aacctgtcgt gccagctgca ttaatgaatc 420
   ggccaacgcg cggggagagg cggtttgcgt attgggcgct cttccgcttc ctcgctcact 480
   gactcgctgc gctcggtcgt tcggctgcgg cgagcggtat cagctcactc aaaggcggta 540
   atacggttat ccacagaatc aggggataac gcaggaaaga acatgtgagc aaaaggccag 600
   caaaaggcca ggaaccgtaa aaaggccgcg ttgctggcgt ttttccatag gctccgcccc 660
   cctgacgagc atcacaaaaa tcgacgctca agtcagaggt ggcgaaaccc gacaggacta 720
   taaagatacc aggcgtttcc ccctggaagc tccctcgtgc gctctcctgt tccgaccctg 780
   ccgcttaccg gatacctgtc cgcctttctc ccttcgggaa gcgtggcgct ttctcatagc 840
   tcacgctgta ggtatctcag ttcggtgtag gtcgttcgct ccaagctggg ctgtgtgcac 900
   gaaccccccg ttcagcccga ccgctgcgcc ttatccggta actatcgtct tgagtccaac 960
   ccggtaagac acgacttatc gccactggca gcagccactg gtaacaggat tagcagagcg 1020
   aggtatgtag gcggtgctac agagttcttg aagtggtggc ctaactacgg ctacactaga 1080
   aggacagtat ttggtatctg cgctctgctg aagccagtta ccttcggaaa aagagttggt 1140
   agctcttgat ccggcaaaca aaccaccgct ggtagcggtg gtttttttgt ttgcaagcag 1200
   cagattacgc gcagaaaaaa aggatctcaa gaagatcctt tgatcttttc tacggggtct 1260
   gacgctcagt ggaacgaaaa ctcacgttaa gggattttgg tcatgagatt atcaaaaagg 1320
   atcttcacct agatcctttt aaattaaaaa tgaagtttta aatcaatcta aagtatatat 1380
   gagtaaactt ggtctgacag ttaccaatgc ttaatcagtg aggcacctat ctcagcgatc 1440
   tgtctatttc gttcatccat agttgcctga ctccccgtcg tgtagataac tacgatacgg 1500
   gagggcttac catctggccc cagtgctgca atgataccgc gagacccacg ctcaccggct 1560
   ccagatttat cagcaataaa ccagccagcc ggaagggccg agcgcagaag tggtcctgca 1620
   actttatccg cctccatcca gtctattaat tgttgccggg aagctagagt aagtagttcg 1680
   ccagttaata gtttgcgcaa cgttgttgcc attgctacag gcatcgtggt gtcacgctcg 1740
   tcgtttggta tggcttcatt cagctccggt tcccaacgat caaggcgagt tacatgatcc 1800
   cccatgttgt gcaaaaaagc ggttagctcc ttcggtcctc cgatcgttgt cagaagtaag 1860
   ttggccgcag tgttatcact catggttatg gcagcactgc ataattctct tactgtcatg 1920
   ccatccgtaa gatgcttttc tgtgactggt gagtactcaa ccaagtcatt ctgagaatag 1980
   tgtatgcggc gaccgagttg ctcttgcccg gcgtcaatac gggataatac cgcgccacat 2040
   agcagaactt taaaagtgct catcattgga aaacgttctt cggggcgaaa actctcaagg 2100
   atcttaccgc tgttgagatc cagttcgatg taacccactc gtgcacccaa ctgatcttca 2160
   gcatctttta ctttcaccag cgtttctggg tgagcaaaaa caggaaggca aaatgccgca 2220
   aaaaagggaa taagggcgac acggaaatgt tgaatactca tactcttcct ttttcaatat 2280
   tattgaagca tttatcaggg ttattgtctc atgagcggat acatatttga atgtatttag 2340
   aaaaataaac aaataggggt tccgcgcaca tttccccgaa aagtgccacc tgacgcgccc 2400
   tgtagcggcg cattaagcgc ggcgggtgtg gtggttacgc gcagcgtgac cgctacactt 2460
   gccagcgccc tagcgcccgc tcctttcgct ttcttccctt cctttctcgc cacgttcgcc 2520
   ggctttcccc gtcaagctct aaatcggggg ctccctttag ggttccgatt tagtgcttta 2580
   cggcacctcg accccaaaaa acttgattag ggtgatggtt cacgtagtgg gccatcgccc 2640
   tgatagacgg tttttcgccc tttgacgttg gagtccacgt tctttaatag tggactcttg 2700
   ttccaaactg gaacaacact caaccctatc tcggtctatt cttttgattt ataagggatt 2760
   ttgccgattt cggcctattg gttaaaaaat gagctgattt aacaaaaatt taacgcgaat 2820
   tttaacaaaa tattaacgct tacaatttcc attcgccatt caggctgcgc aactgttggg 2880
   aagggcgatc ggtgcgggcc tcttcgctat tacgccagct ggcgaaaggg ggatgtgctg 2940
   caaggcgatt aagttgggta acgccagggt tttcccagtc acgacgttgt aaaacgacgg 3000
   ccagtgaatt gtaatacgac tcactatagg gcgaattggg taccgggccc cccctcgagg 3060
   tcgatggtgt cgataagctt gatatcgaat tcatgtcaca caaaccgatc ttcgcctcaa 3120
   ggaaacctaa ttctacatcc gagagactgc cgagatccag tctacactga ttaattttcg 3180
   ggccaataat ttaaaaaaat cgtgttatat aatattatat gtattatata tatacatcat 3240
   gatgatactg acagtcatgt cccattgcta aatagacaga ctccatctgc cgcctccaac 3300
   tgatgttctc aatatttaag gggtcatctc gcattgttta ataataaaca gactccatct 3360
   accgcctcca aatgatgttc tcaaaatata ttgtatgaac ttatttttat tacttagtat 3420
   tattagacaa cttacttgct ttatgaaaaa cacttcctat ttaggaaaca atttataatg 3480
   gcagttcgtt catttaacaa tttatgtaga ataaatgtta taaatgcgta tgggaaatct 3540
   taaatatgga tagcataaat gatatctgca ttgcctaatt cgaaatcaac agcaacgaaa 3600
   aaaatccctt gtacaacata aatagtcatc gagaaatatc aactatcaaa gaacagctat 3660
   tcacacgtta ctattgagat tattattgga cgagaatcac acactcaact gtctttctct 3720
   cttctagaaa tacaggtaca agtatgtact attctcattg ttcatacttc tagtcatttc 3780
   atcccacata ttccttggat ttctctccaa tgaatgacat tctatcttgc aaattcaaca 3840
   attataataa gatataccaa agtagcggta tagtggcaat caaaaagctt ctctggtgtg 3900
   cttctcgtat ttatttttat tctaatgatc cattaaaggt atatatttat ttcttgttat 3960
   ataatccttt tgtttattac atgggctgga tacataaagg tattttgatt taattttttg 4020
   cttaaattca atcccccctc gttcagtgtc aactgtaatg gtaggaaatt accatacttt 4080
   tgaagaagca aaaaaaatga aagaaaaaaa aaatcgtatt tccaggttag acgttccgca 4140
   gaatctagaa tgcggtatgc ggtacattgt tcttcgaacg taaaagttgc gctccctgag 4200
   atattgtaca tttttgcttt tacaagtaca agtacatcgt acaactatgt actactgttg 4260
   atgcatccac aacagtttgt tttgtttttt tttgtttttt ttttttctaa tgattcatta 4320
   ccgctatgta tacctacttg tacttgtagt aagccgggtt attggcgttc aattaatcat 4380
   agacttatga atctgcacgg tgtgcgctgc gagttacttt tagcttatgc atgctacttg 4440
   ggtgtaatat tgggatctgt tcggaaatca acggatgctc aaccgatttc gacagtaatt 4500
   aattaatttg aatcgaatcg gagcctaaaa tgaacccgag tatatctcat aaaattctcg 4560
   gtgagaggtc tgtgactgtc agtacaaggt gccttcatta tgccctcaac cttaccatac 4620
   ctcactgaat gtagtgtacc tctaaaaatg aaatacagtg ccaaaagcca aggcactgag 4680
   ctcgtctaac ggacttgata tacaaccaat taaaacaaat gaaaagaaat acagttcttt 4740
   gtatcatttg taacaattac cctgtacaaa ctaaggtatt gaaatcccac aatattccca 4800
   aagtccaccc ctttccaaat tgtcatgcct acaactcata taccaagcac taacctacca 4860
   aacaccacta aaaccccaca aaatatatct taccgaatat acagtaacaa gctaccacca 4920
   cactcgttgg gtgcagtcgc cagcttaaag atatctatcc acatcagcca caactccctt 4980
   cctttaataa accgactaca cccttggcta ttgaggttat gagtgaatat actgtagaca 5040
   agacactttc aagaagactg tttccaaaac gtaccactgt cctccactac aaacacaccc 5100
   aatctgcttc ttctagtcaa ggttgctaca ccggtaaatt ataaatcatc atttcattag 5160
   cagggcaggg ccctttttat agagtcttat acactagcgg accctgccgg tagaccaacc 5220
   cgcaggcgcg tcagtttgct ccttccatca atgcgtcgta gaaacgactt actccttctt 5280
   gagcagctcc ttgaccttgt tggcaacaag tctccgacct cggaggtgga ggaagagcct 5340
   ccgatatcgg cggtagtgat accagcctcg acggactcct tgacggcagc ctcaacagcg 5400
   tcaccggcgg gcttcatgtt aagagagaac ttgagcatca tggcggcaga cagaatggtg 5460
   gcaatggggt tgaccttctg cttgccgaga tcgggggcag atccgtgaca gggctcgtac 5520
   agaccgaacg cctcgttggt gtcgggcaga gaagccagag aggcggaggg cagcagaccc 5580
   agagaaccgg ggatgacgga ggcctcgtcg gagatgatat cgccaaacat gttggtggtg 5640
   atgatgatac cattcatctt ggagggctgc ttgatgagga tcatggcggc cgagtcgatc 5700
   agctggtggt tgagctcgag ctgggggaat tcgtccttga ggactcgagt gacagtcttt 5760
   cgccaaagtc gagaggaggc cagcacgttg gccttgtcaa gagaccacac gggaagaggg 5820
   gggttgtgct gaagggccag gaaggcggcc attcgggcaa ttcgctcaac ctcaggaacg 5880
   gagtaggtct cggtgtcgga agcgacgcca gatccgtcat cctcctttcg ctctccaaag 5940
   tagatacctc cgacgagctc tcggacaatg atgaagtcgg tgccctcaac gtttcggatg 6000
   ggggagagat cggcgagctt gggcgacagc agctggcagg gtcgcaggtt ggcgtacagg 6060
   ttcaggtcct ttcgcagctt gaggagaccc tgctcgggtc gcacgtcggt tcgtccgtcg 6120
   ggagtggtcc atacggtgtt ggcagcgcct ccgacagcac cgagcataat agagtcagcc 6180
   tttcggcaga tgtcgagagt agcgtcggtg atgggctcgc cctccttctc aatggcagct 6240
   cctccaatga gtcggtcctc aaacacaaac tcggtgccgg aggcctcagc aacagacttg 6300
   agcaccttga cggcctcggc aatcacctcg gggccacaga agtcgccgcc gagaagaaca 6360
   atcttcttgg agtcagtctt ggtcttctta gtttcgggtt ccattgtgga tgtgtgtggt 6420
   tgtatgtgtg atgtggtgtg tggagtgaaa atctgtggct ggcaaacgct cttgtatata 6480
   tacgcacttt tgcccgtgct atgtggaaga ctaaacctcc gaagattgtg actcaggtag 6540
   tgcggtatcg gctagggacc caaaccttgt cgatgccgat agcgctatcg aacgtacccc 6600
   agccggccgg gagtatgtcg gaggggacat acgagatcgt caagggtttg tggccaactg 6660
   gtatttaaat gtagctaacg gtagcaggcg aactactggt acatacctcc cccggaatat 6720
   gtacaggcat aatgcgtatc tgtgggacat gtggtcgttg cgccattatg taagcagcgt 6780
   gtactcctct gactgtccat atggtttgct ccatctcacc ctcatcgttt tcattgttca 6840
   caggcggcca caaaaaaact gtcttctctc cttctctctt cgccttagtc tactcggacc 6900
   agttttagtt tagcttggcg ccactggata aatgagacct caggccttgt gatgaggagg 6960
   tcacttatga agcatgttag gaggtgcttg tatggataga gaagcaccca aaataataag 7020
   aataataata aaacaggggg cgttgtcatt tcatatcgtg ttttcaccat caatacacct 7080
   ccaaacaatg cccttcatgt ggccagcccc aatattgtcc tgtagttcaa ctctatgcag 7140
   ctcgtatctt attgagcaag taaaactctg tcagccgata ttgcccgacc cgcgacaagg 7200
   gtcaacaagg tggtgtaagg ccttcgcaga agtcaaaact gtgccaaaca aacatctaga 7260
   gtctctttgg tgtttctcgc atatatttwa tcggctgtct tacgtatttg cgcctcggta 7320
   ccggactaat ttcggatcat ccccaatacg ctttttcttc gcagctgtca acagtgtcca 7380
   tgatctatcc acctaaatgg gtcatatgag gcgtataatt tcgtggtgct gataataatt 7440
   cccatatatt tgacacaaaa cttccccccc tagacataca tctcacaatc tcacttcttg 7500
   tgcttctgtc acacatctcc tccagctgac ttcaactcac acctctgccc cagttggtct 7560
   acagcggtat aaggtttctc cgcatagagg tgcaccactc ctcccgatac ttgtttgtgt 7620
   gacttgtggg tcacgacata tatatctaca cacattgcgc caccctttgg ttcttccagc 7680
   acaacaaaaa cacgacacgc taaccatggc caatttactg accgtacacc aaaatttgcc 7740
   tgcattaccg gtcgatgcaa cgagtgatga ggttcgcaag aacctgatgg acatgttcag 7800
   ggatcgccag gcgttttctg agcatacctg gaaaatgctt ctgtccgttt gccggtcgtg 7860
   ggcggcatgg tgcaagttga ataaccggaa atggtttccc gcagaacctg aagatgttcg 7920
   cgattatctt ctatatcttc aggcgcgcgg tctggcagta aaaactatcc agcaacattt 7980
   gggccagcta aacatgcttc atcgtcggtc cgggctgcca cgaccaagtg acagcaatgc 8040
   tgtttcactg gttatgcggc ggatccgaaa agaaaacgtt gatgccggtg aacgtgcaaa 8100
   acaggctcta gcgttcgaac gcactgattt cgaccaggtt cgttcactca tggaaaatag 8160
   cgatcgctgc caggatatac gtaatctggc atttctgggg attgcttata acaccctgtt 8220
   acgtatagcc gaaattgcca ggatcagggt taaagatatc tcacgtactg acggtgggag 8280
   aatgttaatc catattggca gaacgaaaac gctggttagc accgcaggtg tagagaaggc 8340
   acttagcctg ggggtaacta aactggtcga gcgatggatt tccgtctctg gtgtagctga 8400
   tgatccgaat aactacctgt tttgccgggt cagaaaaaat ggtgttgccg cgccatctgc 8460
   caccagccag ctatcaactc gcgccctgga agggattttt gaagcaactc atcgattgat 8520
   ttacggcgct aaggatgact ctggtcagag atacctggcc tggtctggac acagtgcccg 8580
   tgtcggagcc gcgcgagata tggcccgcgc tggagtttca ataccggaga tcatgcaagc 8640
   tggtggctgg accaatgtaa atattgtcat gaactatatc cgtaacctgg atagtgaaac 8700
   aggggcaatg gtgcgcctgc tggaagatgg cgattaagc 8739
<210> 128
   <211> 13975
   <212> DNA
   <213> Artificial Sequence
<220>
   <223> Plasmid pZKSL-5S5A5
<400> 128
   cgatggttaa tgctgctgtg tgctgtgtgt gtgtgttgtt tggcgctcat tgttgcgtta      60
   tgcagcgtac accacaatat tggaagctta ttagcctttc tattttttcg tttgcaaggc    120
   ttaacaacat tgctgtggag agggatgggg atatggaggc cgctggaggg agtcggagag     180
   gcgttttgga gcggcttggc ctggcgccca gctcgcgaaa cgcacctagg accctttggc     240
   acgccgaaat gtgccacttt tcagtctagt aacgccttac ctacgtcatt ccatgcgtgc     300
   atgtttgcgc cttttttccc ttgcccttga tcgccacaca gtacagtgca ctgtacagtg    360
   gaggttttgg gggggtctta gatgggagct aaaagcggcc tagcggtaca ctagtgggat     420
   tgtatggagt ggcatggagc ctaggtggag cctgacagga cgcacgaccg gctagcccgt     480
   gacagacgat gggtggctcc tgttgtccac cgcgtacaaa tgtttgggcc aaagtcttgt     540
   cagccttgct tgcgaaccta attcccaatt ttgtcacttc gcacccccat tgatcgagcc    600
   ctaacccctg cccatcaggc aatccaatta agctcgcatt gtctgccttg tttagtttgg    660
   ctcctgcccg tttcggcgtc cacttgcaca aacacaaaca agcattatat ataaggctcg     720
   tctctccctc ccaaccacac tcactttttt gcccgtcttc ccttgctaac acaaaagtca 780
   agaacacaaa caaccacccc aaccccctta cacacaagac atatctacag caatggccat 840
   ggctctcagt cttaccacag aacagctgtt agaacgccct gatttggttg cgattgatgg 900
   catcctctac gaccttgaag ggcttgccaa agttcatcca ggatccgatt tgattctcgc 960
   ttctggtgcc tctgatgcct cccctctctt ttattcaatg catccatacg tcaaaccgga 1020
   gaactccaaa ttgcttcaac agttcgtccg agggaagcat gaccgcacct cgaaggacat 1080
   tgtctacacg tatgattctc ccttcgcaca agacgttaag cggacaatgc gcgaggtgat 1140
   gaaagggagg aactggtacg caacccctgg cttctggctg cgcaccgttg ggatcatcgc 1200
   cgtgacggcc ttttgcgagt ggcactgggc taccacgggg atggtgctgt ggggcctgtt 1260
   gactggattc atgcacatgc agatcggctt atccatccag catgatgcgt cccacggggc 1320
   catcagcaag aagccttggg tcaacgccct cttcgcctac ggcattgacg tcatcggatc 1380
   gtcccggtgg atttggctgc agtcgcacat catgcggcac cacacctaca ccaaccagca 1440
   cggcctcgac ctggatgcgg agtcggcaga gccgttcctg gtgttccaca actaccccgc 1500
   cgcaaacacc gcccgaaagt ggttccaccg cttccaggct tggtacatgt accttgtgct 1560
   gggggcatac ggggtatcgc tggtgtacaa cccgctctac attttccgga tgcagcacaa 1620
   tgacaccatc ccagagtctg tcacggccat gcgggaaaat ggctttctgc ggcgctaccg 1680
   cacacttgca ttcgtgatgc gagctttctt catcttccgg accgcattct tgccctggta 1740
   cctcactggg acctcattgc tgatcaccat tcctctggtg cccaccgcaa ctggtgcctt 1800
   cttgacgttc ttcttcattt tgtcccacaa ttttgatggc tccgaacgga tccccgacaa 1860
   gaactgcaag gttaagcgat ctgagaagga cgttgaggct gaccaaattg actggtatcg 1920
   ggcgcaggtg gagacgtcct ccacatacgg tggccccatc gccatgttct tcactggcgg 1980
   tctcaatttc cagatcgagc accacctctt tccccggatg tcgtcttggc actacccctt 2040
   cgtccagcag gcggtccggg agtgttgcga acgacatgga gtgcgatatg ttttctaccc 2100
   taccatcgtc ggcaacatca tctccaccct gaagtacatg cataaggtgg gtgtcgtcca 2160
   ctgcgtgaag gacgcacagg attcctaagc ggccgcattg atgattggaa acacacacat 2220
   gggttatatc taggtgagag ttagttggac agttatatat taaatcagct atgccaacgg 2280
   taacttcatt catgtcaacg aggaaccagt gactgcaagt aatatagaat ttgaccacct 2340
   tgccattctc ttgcactcct ttactatatc tcatttattt cttatataca aatcacttct 2400
   tcttcccagc atcgagctcg gaaacctcat gagcaataac atcgtggatc tcgtcaatag 2460
   agggcttttt ggactccttg ctgttggcca ccttgtcctt gctgtctggc tcattctgtt 2520
   tcaacgcctt ttaattaact ctcctgcagc ttcctcagcg agacactgct cctgtctgga 2580
   cccgagctaa ggctctgttc gacaaacacg ttctgcgaat tggcgagtaa tttcattaat 2640
   gcaaatagac gtgtatttga aaaggaggag atgatggtac cagtaattta cggtgtttga 2700
   atagacaaat tatatatata aaattaacct gatgaatgag tgtatgaatg gattgcttaa 2760
   tataccgagg gagagccggc attatcaata tttattgtcc taagaagcta aaatatgctg 2820
   ttccgttgat agcgatgtct gtttgagagt caatggcaga acgcggagga gtattgttag 2880
   ttggtgatcg gtttactcga catcgagtag ggggcgagac agaagatatc ccgaatccat 2940
   tgcattgttt attaggatgt tcacaacaca actccctcta gactctgggg atgtgcgtta 3000
   gaagaatgac ctggagcaag agtgttcaga ttgatccgtt caaattttca agattactgt 3060
   tggggttgtt tttgaatcca cattagctgg acgactattg catctgagcg ctcggaacgt 3120
   ctccctttcc cttcagcttt atacgagtcg attccataag cgcgacctct cgaattgcct 3180
   tcggttgtga agccataggc aaaggtgtgg ctatggaatg catgcgacgt cgggcccaat 3240
   tcgccctata gtgagtcgta ttacaattca ctggccgtcg ttttacaacg tcgtgactgg 3300
   gaaaaccctg gcgttaccca acttaatcgc cttgcagcac atcccccttt cgccagctgg 3360
   cgtaatagcg aagaggcccg caccgatcgc ccttcccaac agttgcgcag cctgaatggc 3420
   gaatggacgc gccctgtagc ggcgcattaa gcgcggcggg tgtggtggtt acgcgcagcg 3480
   tgaccgctac acttgccagc gccctagcgc ccgctccttt cgctttcttc ccttcctttc 3540
   tcgccacgtt cgccggcttt ccccgtcaag ctctaaatcg ggggctccct ttagggttcc 3600
   gatttagtgc tttacggcac ctcgacccca aaaaacttga ttagggtgat ggttcacgta 3660
   gtgggccatc gccctgatag acggtttttc gccctttgac gttggagtcc acgttcttta 3720
   atagtggact cttgttccaa actggaacaa cactcaaccc tatctcggtc tattcttttg 3780
   atttataagg gattttgccg atttcggcct attggttaaa aaatgagctg atttaacaaa 3840
   aatttaacgc gaattttaac aaaatattaa cgcttacaat ttcctgatgc ggtattttct 3900
   ccttacgcat ctgtgcggta tttcacaccg catcaggtgg cacttttcgg ggaaatgtgc 3960
   gcggaacccc tatttgttta tttttctaaa tacattcaaa tatgtatccg ctcatgagac 4020
   aataaccctg ataaatgctt caataatatt gaaaaaggaa gagtatgagt attcaacatt 4080
   tccgtgtcgc ccttattccc ttttttgcgg cattttgcct tcctgttttt gctcacccag 4140
   aaacgctggt gaaagtaaaa gatgctgaag atcagttggg tgcacgagtg ggttacatcg 4200
   aactggatct caacagcggt aagatccttg agagttttcg ccccgaagaa cgttttccaa 4260
   tgatgagcac ttttaaagtt ctgctatgtg gcgcggtatt atcccgtatt gacgccgggc 4320
   aagagcaact cggtcgccgc atacactatt ctcagaatga cttggttgag tactcaccag 4380
   tcacagaaaa gcatcttacg gatggcatga cagtaagaga attatgcagt gctgccataa 4440
   ccatgagtga taacactgcg gccaacttac ttctgacaac gatcggagga ccgaaggagc 4500
   taaccgcttt tttgcacaac atgggggatc atgtaactcg ccttgatcgt tgggaaccgg 4560
   agctgaatga agccatacca aacgacgagc gtgacaccac gatgcctgta gcaatggcaa 4620
   caacgttgcg caaactatta actggcgaac tacttactct agcttcccgg caacaattaa 4680
   tagactggat ggaggcggat aaagttgcag gaccacttct gcgctcggcc cttccggctg 4740
   gctggtttat tgctgataaa tctggagccg gtgagcgtgg gtctcgcggt atcattgcag 4800
   cactggggcc agatggtaag ccctcccgta tcgtagttat ctacacgacg gggagtcagg 4860
   caactatgga tgaacgaaat agacagatcg ctgagatagg tgcctcactg attaagcatt 4920
   ggtaactgtc agaccaagtt tactcatata tactttagat tgatttaaaa cttcattttt 4980
   aatttaaaag gatctaggtg aagatccttt ttgataatct catgaccaaa atcccttaac 5040
   gtgagttttc gttccactga gcgtcagacc ccgtagaaaa gatcaaagga tcttcttgag 5100
   atcctttttt tctgcgcgta atctgctgct tgcaaacaaa aaaaccaccg ctaccagcgg 5160
   tggtttgttt gccggatcaa gagctaccaa ctctttttcc gaaggtaact ggcttcagca 5220
   gagcgcagat accaaatact gttcttctag tgtagccgta gttaggccac cacttcaaga 5280
   actctgtagc accgcctaca tacctcgctc tgctaatcct gttaccagtg gctgctgcca 5340
   gtggcgataa gtcgtgtctt accgggttgg actcaagacg atagttaccg gataaggcgc 5400
   agcggtcggg ctgaacgggg ggttcgtgca cacagcccag cttggagcga acgacctaca 5460
   ccgaactgag atacctacag cgtgagctat gagaaagcgc cacgcttccc gaagggagaa 5520
   aggcggacag gtatccggta agcggcaggg tcggaacagg agagcgcacg agggagcttc 5580
   cagggggaaa cgcctggtat ctttatagtc ctgtcgggtt tcgccacctc tgacttgagc 5640
   gtcgattttt gtgatgctcg tcaggggggc ggagcctatg gaaaaacgcc agcaacgcgg 5700
   cctttttacg gttcctggcc ttttgctggc cttttgctca catgttcttt cctgcgttat 5760
   cccctgattc tgtggataac cgtattaccg cctttgagtg agctgatacc gctcgccgca 5820
   gccgaacgac cgagcgcagc gagtcagtga gcgaggaagc ggaagagcgc ccaatacgca 5880
   aaccgcctct ccccgcgcgt tggccgattc attaatgcag ctggcgcgcc gaggtttcca 5940
   acgagataac atcgtggcag ctgccaccat gaccgatatg cctgaaataa gtgaattgac 6000
   catcagaagt tctgcattca aatagaacta tatcatattc ggctcagttt tttcaataat 6060
   agtccaatcc ctaagtctcc tatccaaaat ggttcctgac cagccacatc catgatcatg 6120
   actgcgtgac aggaacagtc attccgtgga tgaacgactt tacgctcagg tactgtaaat 6180
   atctgtaaag ggcagacaac caaccaattg agtaacctgt gagacttgaa acgtaagatg 6240
   acttcacaca caaagtcact tgactcaacc gtggctctca attgcacaaa atcactctgc 6300
   actaatctat tgcaggagtc aggctatgaa caactagacg acagctactt atgtgttata 6360
   tagaggaata ttaaaaaatc taagaataat cataaagtta caaaataatt atcagatttc 6420
   gagccacagg tcacccctaa catgtgttat tgcacaccca caatcctcag cttgatgtca 6480
   tttaattctt ccagccacca tctctctctc caaccctaat ggcaaacttt attttggtgg 6540
   agcgatgact cttactcaac tgcagcatac ttaagcacaa ttgttcccca gcctgatacg 6600
   acacaccatc cattgtcaag cttcaccaca tacaacaaca cagcgtacgc aactaacatg 6660
   aatgaatacg atatacatca aagactatga tacgcagtat tgcacactgt acgagtaaga 6720
   gcactagcca ctgcactcaa gtgaaaccgt tgcccgggta cgagtatgag tatgtacagt 6780
   atgtttagta ttgtacttgg acagtgcttg tatcgtacat tctcaagtgt caaacataaa 6840
   tatccgttgc tatatcctcg caccaccacg tagctcgcta tatccctgtg ttgaatccat 6900
   ccatcttgga ttgccaattg tgcacacaga accgggcact cacttcccca tccacacttg 6960
   cggccgctta ggaatcctga gcgtccttga cacagtgaac cacaccgact ttgtgcatgt 7020
   acttgagggt ggaaatgatg ttgcccacaa tggtagggta gaagacgtac cgaactccgt 7080
   gtcgttcgca acactctcgg acagcttgct gcacgaaggg atagtgccaa gacgacattc 7140
   gaggaaagag gtgatgctcg atctggaagt tgagaccgcc agtaaagaac atggcaatgg 7200
   gtccaccgta ggtggaagag gtctccacct gagctctgta ccagtcgatc tgatcggctt 7260
   caacgtcctt ctcggagctc ttgaccttgc agttcttgtc ggggattcgc tccgagccat 7320
   cgaagttgtg agacaagatg aaaaagaagg tgaggaaggc accggtagca gtgggcacca 7380
   gaggaatggt gatgagcagg gaggttccag tgagatacca gggcaagaag gcggttcgaa 7440
   agatgaagaa agctcgcata acgaatgcaa gggttcggta ccgtcgcaga aagccgttct 7500
   ctcgcatggc tgtgacagac tcgggaatgg tgtcgttgtg ctgcattcgg aagatgtaga 7560
   gagggttgta caccagcgaa acgccgtagg ctccaagcac gaggtacatg taccaggcct 7620
   ggaatcggtg aaaccacttt cgagcagtgt tggcagcagg gtagttgtgg aacacaagga 7680
   atggttctgc ggactcggca tccaggtcga gaccatgctg attggtgtag gtgtgatgtc 7740
   gcatgatgtg agactgcagc cagatccatc tggacgatcc aatgacgtcg atgccgtagg 7800
   caaagagagc gttgacccag ggctttttgc tgatggcacc atgagaggca tcgtgctgaa 7860
   tggacaggcc gatctgcatg tgcatgaatc cagtcaagag accccacagc accattccgg 7920
   tagtagccca gtgccactcg caaaaggcgg tgacagcaat gatgccaacg gttcgcagcc 7980
   agaatccagg tgtggcatac cagttccgac ctttcatgac ctctcgcata gttcgcttga 8040
   cgtcctgtgc aaagggagag tcgtaggtgt agacaatgtc cttggaggtt cggtcgtgct 8100
   tgcctcgcac gaactgttga agcagcttcg agttctcggg cttgacgtaa gggtgcatgg 8160
   agtagaacag aggagaagca tcggaggcac cagaagcgag gatcaagtcg gatccgggat 8220
   ggaccttggc aagaccttcc agatcgtaga gaatgccgtc gatggcaacc aggtcgggtc 8280
   gctcgagcag ctgctcggta gtaagggaga gagccatgga gagctgggtt agtttgtgta 8340
   gagagtgtgt gttgctagcg actttcggat tgtgtcatta cacaaaacgc gtcgtctcga 8400
   cactgatctt gtcgtggata ctcacggctc ggacatcgtc gccgacgatg acaccggact 8460
   ttcgcttaag gacgtcagta acaggcattg tgtgatgtgt agtttagatt tcgaatctgt 8520
   ggggaaagaa aggaaaaaag agactggcaa ccgattggga gagccactgt ttatatatac 8580
   cctagacaag ccccccgctt gtaagatgtt ggtcaatgta aaccagtatt aaggttggca 8640
   agtgcaggag aagcaaggtg tgggtaccga gcaatggaaa tgtgcggaag gcaaaaaaat 8700
   gaggccacgg cctattgtcg gggctatatc cagggggcga ttgaagtaca ctaacatgac 8760
   atgtgtccac agaccctcaa tctggcctga tgagccaaat ccatacgcgc tttcgcagct 8820
   ctaaaggcta taacaagtca caccaccctg ctcgacctca gcgccctcac tttttgttaa 8880
   gacaaactgt acacgctgtt ccagcgtttt ctgcctgcac ctggtgggac atttggtgca 8940
   acctaaagtg ctcggaacct ctgtggtgtc cagatcagcg cagcagttcc gaggtagttt 9000
   tgaggccctt agatgatgca atggtgtcag tcgctggatc acgagtctta atggcagtat 9060
   tcgttcttat ttgtgccatt gagccccgtt atcctcgtat cttctacccc ccatcccatc 9120
   cctttgttgg tgcaacccta cccatttatt gttgggtgca gcccaaccga cgtggagagc 9180
   ttggcttggc catataaaaa ggcccccccc tagtggcaat ggcagaaagt cagctgtgag 9240
   ttgttgaatt tgtcatctag gcggcctggc cgtcttctcc ggggcaattg gggctgtttt 9300
   ttgggacaca aatacgccgc caacccggtc tctcctgaat tctgcagatg ggctgcagga 9360
   attccgtcgt cgcctgagtc gactccaact tttcacactg agcgtaaaat gtggagaaga 9420
   aatcggcact aaaaagtcag gtagactgga aaatgcgcca tgaaatgaat atctcttgct 9480
   acagtaatgc ccagcatcga ggggtattgt gtcaccaaca ctatagtggc agctgaagcg 9540
   ctcgtgattg tagtatgagt ctttattggt gatgggaaga gttcactcaa tattctcgtt 9600
   actgccaaaa caccacggta atcggccaga caccatggat gtagatcacc aagcctgtga 9660
   atgttattcg agctaaaatg cacatggttg gtgaaaggag tagttgctgt cgaattccgt 9720
   cgtcgcctga gtcatcattt atttaccagt tggccacaaa cccttgacga tctcgtatgt 9780
   cccctccgac atactcccgg ccggctgggg tacgttcgat agcgctatcg gcatcgacaa 9840
   ggtttgggtc cctagccgat accgcactac ctgagtcaca atcttcggag gtttagtctt 9900
   ccacatagca cgggcaaaag tgcgtatata tacaagagcg tttgccagcc acagattttc 9960
   actccacaca ccacatcaca catacaacca cacacatcca caatggaacc cgaaactaag 10020
   aagaccaaga ctgactccaa gaagattgtt cttctcggcg gcgacttctg tggccccgag 10080
   gtgattgccg aggccgtcaa ggtgctcaag tctgttgctg aggcctccgg caccgagttt 10140
   gtgtttgagg accgactcat tggaggagct gccattgaga aggagggcga gcccatcacc 10200
   gacgctactc tcgacatctg ccgaaaggct gactctatta tgctcggtgc tgtcggaggc 10260
   gctgccaaca ccgtatggac cactcccgac ggacgaaccg acgtgcgacc cgagcagggt 10320
   ctcctcaagc tgcgaaagga cctgaacctg tacgccaacc tgcgaccctg ccagctgctg 10380
   tcgcccaagc tcgccgatct ctcccccatc cgaaacgttg agggcaccga cttcatcatt 10440
   gtccgagagc tcgtcggagg tatctacttt ggagagcgaa aggaggatga cggatctggc 10500
   gtcgcttccg acaccgagac ctactccgtt cctgaggttg agcgaattgc ccgaatggcc 10560
   gccttcctgg cccttcagca caacccccct cttcccgtgt ggtctcttga caaggccaac 10620
   gtgctggcct cctctcgact ttggcgaaag actgtcactc gagtcctcaa ggacgaattc 10680
   ccccagctcg agctcaacca ccagctgatc gactcggccg ccatgatcct catcaagcag 10740
   ccctccaaga tgaatggtat catcatcacc accaacatgt ttggcgatat catctccgac 10800
   gaggcctccg tcatccccgg ttctctgggt ctgctgccct ccgcctctct ggcttctctg 10860
   cccgacacca acgaggcgtt cggtctgtac gagccctgtc acggatctgc ccccgatctc 10920
   ggcaagcaga aggtcaaccc cattgccacc attctgtctg ccgccatgat gctcaagttc 10980
   tctcttaaca tgaagcccgc cggtgacgct gttgaggctg ccgtcaagga gtccgtcgag 11040
   gctggtatca ctaccgccga tatcggaggc tcttcctcca cctccgaggt cggagacttg 11100
   ttgccaacaa ggtcaaggag ctgctcaaga aggagtaagt cgtttctacg acgcattgat 11160
   ggaaggagca aactgacgcg cctgcgggtt ggtctaccgg cagggtccgc tagtgtataa 11220
   gactctataa aaagggccct gccctgctaa tgaaatgatg atttataatt taccggtgta 11280
   gcaaccttga ctagaagaag cagattgggt gtgtttgtag tggaggacag tggtacgttt 11340
   tggaaacagt cttcttgaaa gtgtcttgtc tacagtatat tcactcataa cctcaatagc 11400
   caagggtgta gtcggtttat taaaggaagg gagttgtggc tgatgtggat agatatcttt 11460
   aagctggcga ctgcacccaa cgagtgtggt ggtagcttgt ttaaacagag tgtgaaagac 11520
   tcactatggt ccgggcttat ctcgaccaat agccaaagtc tggagtttct gagagaaaaa 11580
   ggcaagatac gtatgtaaca aagcgacgca tggtacaata ataccggagg catgtatcat 11640
   agagagttag tggttcgatg atggcactgg tgcctggtat gactttatac ggctgactac 11700
   atatttgtcc tcagacatac aattacagtc aagcacttac ccttggacat ctgtaggtac 11760
   cccccggcca agacgatctc agcgtgtcgt atgtcggatt ggcgtagctc cctcgctcgt 11820
   caattggctc ccatctactt tcttctgctt ggctacaccc agcatgtctg ctatggctcg 11880
   ttttcgtgcc ttatctatcc tcccagtatt accaactcta aatgacatga tgtgattggg 11940
   tctacacttt catatcagag ataaggagta gcacagttgc ataaaaagcc caactctaat 12000
   cagcttcttc ctttcttgta attagtacaa aggtgattag cgaaatctgg aagcttagtt 12060
   ggccctaaaa aaatcaaaaa aagcaaaaaa cgaaaaacga aaaaccacag ttttgagaac 12120
   agggaggtaa cgaaggatcg tatatatata tatatatata tatacccacg gatcccgaga 12180
   ccggcctttg attcttccct acaaccaacc attctcacca ccctaattca caaccatggc 12240
   caccatctcc ctgactaccg agcagctcct ggaacacccc gagctcgttg ccatcgacgg 12300
   agtcctgtac gatctcttcg gtctggccaa ggtccatgcc ggaggcaacc tcatcgaagc 12360
   tgccggtgca tccgacggaa ccgctctgtt ctactccatg catcctggag tcaagccaga 12420
   gaactcgaag cttctgcagc aatttgcccg aggcaagcac gaacgaagct ccaaggatcc 12480
   cgtgtacacc ttcgactctc cctttgctca ggacgtcaag cagtccgttc gagaggtcat 12540
   gaagggtcga aactggtacg ccactcctgg cttctggctg agaaccgcac tcatcatcgc 12600
   ttgtactgcc attggcgagt ggtactggat cacaaccgga gcagtgatgt ggggtatctt 12660
   tactggatac ttccactcgc agattggctt ggccattcaa cacgatgctt ctcacggagc 12720
   catcagcaaa aagccctggg tcaacgcctt tttcgcttat ggcatcgacg ccattggttc 12780
   ctctcgttgg atctggctgc agtcccacat tatgcgacat cacacttaca ccaaccagca 12840
   tggcctcgac ctggatgctg cctcggcaga gccgttcatc ttgttccact cctatcctgc 12900
   taccaacgcc tctcgaaagt ggtaccaccg atttcaggcg tggtacatgt acatcgttct 12960
   gggaatgtat ggtgtctcga tggtgtacaa tcccatgtac ctcttcacaa tgcagcacaa 13020
   cgacaccatt cccgaggcca cttctctcag accaggcagc tttttcaatc ggcagcgagc 13080
   tttcgccgtt tcccttcgac tgctcttcat cttccgaaac gcctttcttc cctggtacat 13140
   tgctggtgcc tctcctctgc tcaccattct tctggtgccc acggtcacag gcatcttcct 13200
   cacctttgtg ttcgttctgt cccataactt cgagggagcc gaacggaccc cagagaagaa 13260
   ctgcaaggcc aaacgagcta aggaaggcaa ggaggtcaga gacgtggaag aggatcgagt 13320
   cgactggtac cgagcacagg ccgagactgc tgccacctac ggtggcagcg tgggaatgat 13380
   gcttacaggc ggtctcaacc tgcagatcga gcatcacttg tttccccgaa tgtcctcttg 13440
   gcactatccc ttcattcaag acaccgttcg ggagtgttgc aagcgacatg gcgtccgtta 13500
   cacatactat cctaccattc tcgagaacat catgtccact cttcgataca tgcagaaggt 13560
   gggtgttgct cacaccattc aggatgccca ggagttctaa gcggccgcat gtacatacaa 13620
   gattatttat agaaatgaat cgcgatcgaa caaagagtac gagtgtacga gtaggggatg 13680
   atgataaaag tggaagaagt tccgcatctt tggatttatc aacgtgtagg acgatacttc 13740
   ctgtaaaaat gcaatgtctt taccataggt tctgctgtag atgttattaa ctaccattaa 13800
   catgtctact tgtacagttg cagaccagtt ggagtataga atggtacact taccaaaaag 13860
   tgttgatggt tgtaactacg atatataaaa ctgttgacgg gatccccgct gatatgccta 13920
   aggaacaatc aaagaggaag atattaattc agaatgctag tatacagtta gggat 13975
<210> 129
   <211> 13066
   <212> DNA
   <213> Artificial Sequence
<220>
   <223> Plasmid pZP3-Pa777U
<400> 129
   tctcggtcta ttcttttgat ttataaggga ttttgccgat ttcggcctat tggttaaaaa 60
   atgagctgat ttaacaaaaa tttaacgcga attttaacaa aatattaacg cttacaattt 120
   cctgatgcgg tattttctcc ttacgcatct gtgcggtatt tcacaccgca tcaggtggca 180
   cttttcgggg aaatgtgcgc ggaaccccta tttgtttatt tttctaaata cattcaaata 240
   tgtatccgct catgagacaa taaccctgat aaatgcttca ataatattga aaaaggaaga 300
   gtatgagtat tcaacatttc cgtgtcgccc ttattccctt ttttgcggca ttttgccttc 360
   ctgtttttgc tcacccagaa acgctggtga aagtaaaaga tgctgaagat cagttgggtg 420
   cacgagtggg ttacatcgaa ctggatctca acagcggtaa gatccttgag agttttcgcc 480
   ccgaagaacg ttttccaatg atgagcactt ttaaagttct gctatgtggc gcggtattat 540
   cccgtattga cgccgggcaa gagcaactcg gtcgccgcat acactattct cagaatgact 600
   tggttgagta ctcaccagtc acagaaaagc atcttacgga tggcatgaca gtaagagaat 660
   tatgcagtgc tgccataacc atgagtgata acactgcggc caacttactt ctgacaacga 720
   tcggaggacc gaaggagcta accgcttttt tgcacaacat gggggatcat gtaactcgcc 780
   ttgatcgttg ggaaccggag ctgaatgaag ccataccaaa cgacgagcgt gacaccacga 840
   tgcctgtagc aatggcaaca acgttgcgca aactattaac tggcgaacta cttactctag 900
   cttcccggca acaattaata gactggatgg aggcggataa agttgcagga ccacttctgc 960
   gctcggccct tccggctggc tggtttattg ctgataaatc tggagccggt gagcgtgggt 1020
   ctcgcggtat cattgcagca ctggggccag atggtaagcc ctcccgtatc gtagttatct 1080
   acacgacggg gagtcaggca actatggatg aacgaaatag acagatcgct gagataggtg 1140
   cctcactgat taagcattgg taactgtcag accaagttta ctcatatata ctttagattg 1200
   atttaaaact tcatttttaa tttaaaagga tctaggtgaa gatccttttt gataatctca 1260
   tgaccaaaat cccttaacgt gagttttcgt tccactgagc gtcagacccc gtagaaaaga 1320
   tcaaaggatc ttcttgagat cctttttttc tgcgcgtaat ctgctgcttg caaacaaaaa 1380
   aaccaccgct accagcggtg gtttgtttgc cggatcaaga gctaccaact ctttttccga 1440
   aggtaactgg cttcagcaga gcgcagatac caaatactgt tcttctagtg tagccgtagt 1500
   taggccacca cttcaagaac tctgtagcac cgcctacata cctcgctctg ctaatcctgt 1560
   taccagtggc tgctgccagt ggcgataagt cgtgtcttac cgggttggac tcaagacgat 1620
   agttaccgga taaggcgcag cggtcgggct gaacgggggg ttcgtgcaca cagcccagct 1680
   tggagcgaac gacctacacc gaactgagat acctacagcg tgagctatga gaaagcgcca 1740
   cgcttcccga agggagaaag gcggacaggt atccggtaag cggcagggtc ggaacaggag 1800
   agcgcacgag ggagcttcca gggggaaacg cctggtatct ttatagtcct gtcgggtttc 1860
   gccacctctg acttgagcgt cgatttttgt gatgctcgtc aggggggcgg agcctatgga 1920
   aaaacgccag caacgcggcc tttttacggt tcctggcctt ttgctggcct tttgctcaca 1980
   tgttctttcc tgcgttatcc cctgattctg tggataaccg tattaccgcc tttgagtgag 2040
   ctgataccgc tcgccgcagc cgaacgaccg agcgcagcga gtcagtgagc gaggaagcgg 2100
   aagagcgccc aatacgcaaa ccgcctctcc ccgcgcgttg gccgattcat taatgcagct 2160
   ggcgcgccac caatcacaat tctgaaaagc acatcttgat ctcctcattg cggggagtcc 2220
   aacggtggtc ttattccccc gaatttcccg ctcaatctcg ttccagaccg acccggacac 2280
   agtgcttaac gccgttccga aactctaccg cagatatgct ccaacggact gggctgcata 2340
   gatgtgatcc tcggcttgga gaaatggata aaagccggcc aaaaaaaaag cggaaaaaag 2400
   cggaaaaaaa gagaaaaaaa atcgcaaaat ttgaaaaata gggggaaaag acgcaaaaac 2460
   gcaaggaggg gggagtatat gacactgata agcaagctca caacggttcc tcttattttt 2520
   ttcctcatct tctgcctagg ttcccaaaat cccagatgct tctctccagt gccaaaagta 2580
   agtaccccac aggttttcgg ccgaaaattc cacgtgcagc aacgtcgtgt ggggtgttaa 2640
   aatgtggggg gggggaacca ggacaagagg ctcttgtggg agccgaatga gagcacaaag 2700
   cgggcgggtg tgataagggc atttttgccc attttccctt ctcctgtctc tccgacggtg 2760
   atggcgttgt gcgtcctcta tttcttttta tttctttttg ttttatttct ctgactaccg 2820
   atttggtttg atttcctcaa ccccacacaa ataagctcgg gccgaggaat atatatatac 2880
   acggacacag tcgccctgtg gacaacacgt cactacctct acgatacaca ccgtacgttg 2940
   tgtggaagct tgtgagcgga taacaatttc acacaggaaa cagctatgac catgattacg 3000
   ccaagctcga aattaaccct cactaaaggg aacaaaagct ggagctccac cgcggacaca 3060
   atatctggtc aaatttcagt ttcgttacat ttaaattcct tcacttcaag ttcattcttc 3120
   atctgcttct gttttacttt gacaggcaaa tgaagacatg gtacgacttg atggaggcca 3180
   agaacgccat ttcaccccga gacaccgaag tgcctgaaat cctggctgcc cccattgata 3240
   acatcggaaa ctacggtatt ccggaaagtg tatatagaac ctttccccag cttgtgtctg 3300
   tggatatgga tggtgtaatc ccctttgagt actcgtcttg gcttctctcc gagcagtatg 3360
   aggctctcta atctagcgca tttaatatct caatgtattt atatatttat cttctcatgc 3420
   ggccgcttag ttggctttgg tcttggcagc cttggcctcc ttgagggtaa acatcttggc 3480
   atccttgtcg accacgccgt acttggcgta cataagacca attcggatga aggtgggaat 3540
   gatgggagaa gccgactttc gcaccagttc gggaaaggcc tgagcgaagg cagcagtggc 3600
   ctcgttgagc ttgtagtgag gaatgatggg aaacagatgg tggatctgat gtgtaccaat 3660
   gttgtgggac aggttgtcga tgagggctcc gtagcttcgg tccacagagg acaagttgcc 3720
   cttgacatag gtccactccg aatcggcgta ccagggagtt tcctcgtcgt tgtgatggag 3780
   gaaggtagtg acaaccagca tggtggcgaa tccaaagaga ggtgcgaagt aatacagagc 3840
   catggtcttg aggccgtaga cgtaggtaag gtaggcgtac agaccagcaa aggccacgag 3900
   agagccgagg gaaatgatga cggcagacat tcttcgcagg tagagaggct cccagggatt 3960
   gaagtggttg acctttcggg gaggaaatcc agcaacgagg taggcaaacc aagccgaacc 4020
   aagggagatg accatgtgtc gggacagggg atgagagtcg gcttctcgct gagggtagaa 4080
   gatctcatcc ttgtcgatgt tgccggtgtt cttgtgatgg tgtcgatggc tgatcttcca 4140
   cgactcgtag ggagtcagaa tgatggagtg aatgagtgtg ccaacagaga agttgagcag 4200
   gtgggatcgc gagaaggcac catgtccaca gtcgtgaccg atggtaaaga atccccagaa 4260
   cacgataccc tggagcagaa tgtagccagt gcaaaggacg gcatcgagca gtgcaaactc 4320
   ctgcacgata gcaagggctc gagcatagta cagtccgaga gcaagggaac cggcaatgcc 4380
   cagagctcgc acggtatagt agagggacca gggaacagag gcttcgaagc agtgggcagg 4440
   cagggatcgc ttgatctcgg tgagagtagg gaactcgtag ggagcggcaa cggtagagga 4500
   agccatggtt gtgaattagg gtggtgagaa tggttggttg tagggaagaa tcaaaggccg 4560
   gtctcgggat ccgtgggtat atatatatat atatatatat acgatccttc gttacctccc 4620
   tgttctcaaa actgtggttt ttcgtttttc gttttttgct ttttttgatt tttttagggc 4680
   caactaagct tccagatttc gctaatcacc tttgtactaa ttacaagaaa ggaagaagct 4740
   gattagagtt gggcttttta tgcaactgtg ctactcctta tctctgatat gaaagtgtag 4800
   acccaatcac atcatgtcat ttagagttgg taatactggg aggatagata aggcacgaaa 4860
   acgagccata gcagacatgc tgggtgtagc caagcagaag aaagtagatg ggagccaatt 4920
   gacgagcgag ggagctacgc caatccgaca tacgacacgc tgagatcgtc ttggccgggg 4980
   ggtacctaca gatgtccaag ggtaagtgct tgactgtaat tgtatgtctg aggacaaata 5040
   tgtagtcagc cgtataaagt cataccaggc accagtgcca tcatcgaacc actaactctc 5100
   tatgatacat gcctccggta ttattgtacc atgcgtcgct ttgttacata cgtatcttgc 5160
   ctttttctct cagaaactcc agactttggc tattggtcga gataagcccg gaccatagtg 5220
   agtctttcac actctacatt tctcccttgc tccaactatc gattgttgtc tactaactat 5280
   cgtacgataa cttcgtatag catacattat acgaagttat cgcgtcgacg agtatctgtc 5340
   tgactcgtca ttgccgcctt tggagtacga ctccaactat gagtgtgctt ggatcacttt 5400
   gacgatacat tcttcgttgg aggctgtggg tctgacagct gcgttttcgg cgcggttggc 5460
   cgacaacaat atcagctgca acgtcattgc tggctttcat catgatcaca tttttgtcgg 5520
   caaaggcgac gcccagagag ccattgacgt tctttctaat ttggaccgat agccgtatag 5580
   tccagtctat ctataagttc aactaactcg taactattac cataacatat acttcactgc 5640
   cccagataag gttccgataa aaagttctgc agactaaatt tatttcagtc tcctcttcac 5700
   caccaaaatg ccctcctacg aagctcgagc taacgtccac aagtccgcct ttgccgctcg 5760
   agtgctcaag ctcgtggcag ccaagaaaac caacctgtgt gcttctctgg atgttaccac 5820
   caccaaggag ctcattgagc ttgccgataa ggtcggacct tatgtgtgca tgatcaaaac 5880
   ccatatcgac atcattgacg acttcaccta cgccggcact gtgctccccc tcaaggaact 5940
   tgctcttaag cacggtttct tcctgttcga ggacagaaag ttcgcagata ttggcaacac 6000
   tgtcaagcac cagtaccggt gtcaccgaat cgccgagtgg tccgatatca ccaacgccca 6060
   cggtgtaccc ggaaccggaa tcattgctgg cctgcgagct ggtgccgagg aaactgtctc 6120
   tgaacagaag aaggaggacg tctctgacta cgagaactcc cagtacaagg agttcctagt 6180
   cccctctccc aacgagaagc tggccagagg tctgctcatg ctggccgagc tgtcttgcaa 6240
   gggctctctg gccactggcg agtactccaa gcagaccatt gagcttgccc gatccgaccc 6300
   cgagtttgtg gttggcttca ttgcccagaa ccgacctaag ggcgactctg aggactggct 6360
   tattctgacc cccggggtgg gtcttgacga caagggagac gctctcggac agcagtaccg 6420
   aactgttgag gatgtcatgt ctaccggaac ggatatcata attgtcggcc gaggtctgta 6480
   cggccagaac cgagatccta ttgaggaggc caagcgatac cagaaggctg gctgggaggc 6540
   ttaccagaag attaactgtt agaggttaga ctatggatat gtaatttaac tgtgtatata 6600
   gagagcgtgc aagtatggag cgcttgttca gcttgtatga tggtcagacg acctgtctga 6660
   tcgagtatgt atgatactgc acaacctgtg tatccgcatg atctgtccaa tggggcatgt 6720
   tgttgtgttt ctcgatacgg agatgctggg tacagtgcta atacgttgaa ctacttatac 6780
   ttatatgagg ctcgaagaaa gctgacttgt gtatgactta ttctcaacta catccccagt 6840
   cacaatacca ccactgcact accactacac caaaaccatg atcaaaccac ccatggactt 6900
   cctggaggca gaagaacttg ttatggaaaa gctcaagaga gagatcataa cttcgtatag 6960
   catacattat acgaagttat cctgcaggta aaggaattca tgctgttcat cgtggttaat 7020
   gctgctgtgt gctgtgtgtg tgtgttgttt ggcgctcatt gttgcgttat gcagcgtaca 7080
   ccacaatatt ggaagcttat tagcctttct attttttcgt ttgcaaggct taacaacatt 7140
   gctgtggaga gggatgggga tatggaggcc gctggaggga gtcggagagg cgttttggag 7200
   cggcttggcc tggcgcccag ctcgcgaaac gcacctagga ccctttggca cgccgaaatg 7260
   tgccactttt cagtctagta acgccttacc tacgtcattc catgcgtgca tgtttgcgcc 7320
   ttttttccct tgcccttgat cgccacacag tacagtgcac tgtacagtgg aggttttggg 7380
   ggggtcttag atgggagcta aaagcggcct agcggtacac tagtgggatt gtatggagtg 7440
   gcatggagcc taggtggagc ctgacaggac gcacgaccgg ctagcccgtg acagacgatg 7500
   ggtggctcct gttgtccacc gcgtacaaat gtttgggcca aagtcttgtc agccttgctt 7560
   gcgaacctaa ttcccaattt tgtcacttcg cacccccatt gatcgagccc taacccctgc 7620
   ccatcaggca atccaattaa gctcgcattg tctgccttgt ttagtttggc tcctgcccgt 7680
   ttcggcgtcc acttgcacaa acacaaacaa gcattatata taaggctcgt ctctccctcc 7740
   caaccacact cacttttttg cccgtcttcc cttgctaaca caaaagtcaa gaacacaaac 7800
   aaccacccca acccccttac acacaagaca tatctacagc aatggccatg gcttcttcca 7860
   ctgttgctgc gccgtacgag ttcccgacgc tgacggagat caagcgctcg ctgccagcgc 7920
   actgctttga ggcctcggtc ccgtggtcgc tctactacac cgtgcgcgcg ctgggcatcg 7980
   ccggctcgct cgcgctcggc ctctactacg cgcgcgcgct cgcgatcgtg caggagtttg 8040
   ccctgctgga tgcggtgctc tgcacggggt acattctgct gcagggcatc gtattctggg 8100
   ggttcttcac catcggccat gactgcggcc acggcgcgtt ctcgcgttcg cacctgctca 8160
   acttcagcgt cggcacgctc attcactcga tcatcctcac gccgtacgag tcatggaaga 8220
   tctcgcaccg ccaccaccac aagaacacgg gcaacatcga caaggacgag attttctacc 8280
   cgcagcgcga ggccgactcg cacccactgt cccgacacat ggtgatctcg ctcggctcgg 8340
   cctggttcgc gtacctcgtt gcgggcttcc ctcctcgcaa ggtgaaccac ttcaaccctt 8400
   gggaaccgtt gtacctgcgc cgcatgtctg ccgtcatcat ctcactcggc tcgctcgtgg 8460
   cgttcgcggg cttgtatgcg tatctcacct acgtctatgg ccttaagacc atggcgctgt 8520
   actacttcgc ccctctcttt gggttcgcca cgatgctcgt ggtcactacc tttttgcacc 8580
   acaatgacga ggaaacgcca tggtacgccg actcggagtg gacgtacgtc aagggcaacc 8640
   tctcgtccgt ggaccgctcg tacggcgcgc tcatcgacaa cctgagccac aacatcggca 8700
   cgcaccagat ccaccacctg tttccgatca tcccgcacta caagctgaac gaggcgacgg 8760
   cagcgttcgc gcaggcgttc ccggagctcg tgcgcaagag cgcgtcgccg atcatcccga 8820
   cgttcatccg catcgggctc atgtacgcca agtacggcgt cgtggacaag gacgccaaga 8880
   tgtttacgct caaggaggcc aaggccgcca agaccaaggc caactaggcg gccgcattga 8940
   tgattggaaa cacacacatg ggttatatct aggtgagagt tagttggaca gttatatatt 9000
   aaatcagcta tgccaacggt aacttcattc atgtcaacga ggaaccagtg actgcaagta 9060
   atatagaatt tgaccacctt gccattctct tgcactcctt tactatatct catttatttc 9120
   ttatatacaa atcacttctt cttcccagca tcgagctcgg aaacctcatg agcaataaca 9180
   tcgtggatct cgtcaataga gggctttttg gactccttgc tgttggccac cttgtccttg 9240
   ctgtttaaac agtgtacgca gatctactat agaggaacat ttaaattgcc ccggagaaga 9300
   cggccaggcc gcctagatga caaattcaac aactcacagc tgactttctg ccattgccac 9360
   tagggggggg cctttttata tggccaagcc aagctctcca cgtcggttgg gctgcaccca 9420
   acaataaatg ggtagggttg caccaacaaa gggatgggat ggggggtaga agatacgagg 9480
   ataacggggc tcaatggcac aaataagaac gaatactgcc attaagactc gtgatccagc 9540
   gactgacacc attgcatcat ctaagggcct caaaactacc tcggaactgc tgcgctgatc 9600
   tggacaccac agaggttccg agcactttag gttgcaccaa atgtcccacc aggtgcaggc 9660
   agaaaacgct ggaacagcgt gtacagtttg tcttaacaaa aagtgagggc gctgaggtcg 9720
   agcagggtgg tgtgacttgt tatagccttt agagctgcga aagcgcgtat ggatttggct 9780
   catcaggcca gattgagggt ctgtggacac atgtcatgtt agtgtacttc aatcgccccc 9840
   tggatatagc cccgacaata ggccgtggcc tcattttttt gccttccgca catttccatt 9900
   gctcggtacc cacaccttgc ttctcctgca cttgccaacc ttaatactgg tttacattga 9960
   ccaacatctt acaagcgggg ggcttgtcta gggtatatat aaacagtggc tctcccaatc 10020
   ggttgccagt ctcttttttc ctttctttcc ccacagattc gaaatctaaa ctacacatca 10080
   cagaattccg agccgtgagt atccacgaca agatcagtgt cgagacgacg cgttttgtgt 10140
   aatgacacaa tccgaaagtc gctagcaaca cacactctct acacaaacta acccagctct 10200
   ggtaccatgg cttcttccac tgttgctgcg ccgtacgagt tcccgacgct gacggagatc 10260
   aagcgctcgc tgccagcgca ctgctttgag gcctcggtcc cgtggtcgct ctactacacc 10320
   gtgcgcgcgc tgggcatcgc cggctcgctc gcgctcggcc tctactacgc gcgcgcgctc 10380
   gcgatcgtgc aggagtttgc cctgctggat gcggtgctct gcacggggta cattctgctg 10440
   cagggcatcg tattctgggg gttcttcacc atcggccatg actgcggcca cggcgcgttc 10500
   tcgcgttcgc acctgctcaa cttcagcgtc ggcacgctca ttcactcgat catcctcacg 10560
   ccgtacgagt catggaagat ctcgcaccgc caccaccaca agaacacggg caacatcgac 10620
   aaggacgaga ttttctaccc gcagcgcgag gccgactcgc acccactgtc ccgacacatg 10680
   gtgatctcgc tcggctcggc ctggttcgcg tacctcgttg cgggcttccc tcctcgcaag 10740
   gtgaaccact tcaacccttg ggaaccgttg tacctgcgcc gcatgtctgc cgtcatcatc 10800
   tcactcggct cgctcgtggc gttcgcgggc ttgtatgcgt atctcaccta cgtctatggc 10860
   cttaagacca tggcgctgta ctacttcgcc cctctctttg ggttcgccac gatgctcgtg 10920
   gtcactacct ttttgcacca caatgacgag gaaacgccat ggtacgccga ctcggagtgg 10980
   acgtacgtca agggcaacct ctcgtccgtg gaccgctcgt acggcgcgct catcgacaac 11040
   ctgagccaca acatcggcac gcaccagatc caccacctgt ttccgatcat cccgcactac 11100
   aagctgaacg aggcgacggc agcgttcgcg caggcgttcc cggagctcgt gcgcaagagc 11160
   gcgtcgccga tcatcccgac gttcatccgc atcgggctca tgtacgccaa gtacggcgtc 11220
   gtggacaagg acgccaagat gtttacgctc aaggaggcca aggccgccaa gaccaaggcc 11280
   aactaggcgg ccgcatggag cgtgtgttct gagtcgatgt tttctatgga gttgtgagtg 11340
   ttagtagaca tgatgggttt atatatgatg aatgaataga tgtgattttg atttgcacga 11400
   tggaattgag aactttgtaa acgtacatgg gaatgtatga atgtgggggt tttgtgactg 11460
   gataactgac ggtcagtgga cgccgttgtt caaatatcca agagatgcga gaaactttgg 11520
   gtcaagtgaa catgtcctct ctgttcaagt aaaccatcaa ctatgggtag tatatttagt 11580
   aaggacaaga gttgagattc tttggagtcc tagaaacgta ttttcgcgtt ccaagatcaa 11640
   attagtagag taatacgggc acgggaatcc attcatagtc tcaatcctgc aggtgagtta 11700
   attaagatga cgacatttgc gagctggacg aggaatagat ggagcgtgtg ttctgagtcg 11760
   atgttttcta tggagttgtg agtgttagta gacatgatgg gtttatatat gatgaatgaa 11820
   tagatgtgat tttgatttgc acgatggaat tgagaacttt gtaaacgtac atgggaatgt 11880
   atgaatgtgg gggttttgtg actggataac tgacggtcag tggacgccgt tgttcaaata 11940
   tccaagagat gcgagaaact ttgggtcaag tgaacatgtc ctctctgttc aagtaaacca 12000
   tcaactatgg gtagtatatt tagtaaggac aagagttgag attctttgga gtcctagaaa 12060
   cgtattttcg cgttccaaga tcaaattagt agagtaatac gggcacggga atccattcat 12120
   agtctcaatt ttcccatagg tgtgctacaa ggtgttgaga tgtggtacag taccaccatg 12180
   attcgaggta aagagcccag aagtcattga tgaggtcaag aaatacacag atctacagct 12240
   caatacaatg aatatcttct ttcatattct tcaggtgaca ccaagggtgt ctattttccc 12300
   cagaaatgcg tgaaaaggcg cgtgtgtagc gtggagtatg ggttcggttg gcgtatcctt 12360
   catatatcga cgaaatagta gggcaagaga tgacaaaaag tatctatatg tagacagcgt 12420
   agaatatgga tttgattggt ataaattcat ttattgcgtg tctcacaaat actctcgata 12480
   agttggggtt aaactggaga tggaacaatg tcgatatctc gacgcatgcg acgtcgggcc 12540
   caattcgccc tatagtgagt cgtattacaa ttcactggcc gtcgttttac aacgtcgtga 12600
   ctgggaaaac cctggcgtta cccaacttaa tcgccttgca gcacatcccc ctttcgccag 12660
   ctggcgtaat agcgaagagg cccgcaccga tcgcccttcc caacagttgc gcagcctgaa 12720
   tggcgaatgg acgcgccctg tagcggcgca ttaagcgcgg cgggtgtggt ggttacgcgc 12780
   agcgtgaccg ctacacttgc cagcgcccta gcgcccgctc ctttcgcttt cttcccttcc 12840
   tttctcgcca cgttcgccgg ctttccccgt caagctctaa atcgggggct ccctttaggg 12900
   ttccgattta gtgctttacg gcacctcgac cccaaaaaac ttgattaggg tgatggttca 12960
   cgtagtgggc catcgccctg atagacggtt tttcgccctt tgacgttgga gtccacgttc     13020
   tttaatagtg gactcttgtt ccaaactgga acaacactca acccta                    13066
<210> 130
   <211> 4313
   <212> DNA
   <213> Artificial Sequence
<220>
   <223> Plasmid pZKUM
<400> 130
   taatcgagct tggcgtaatc atggtcatag ctgtttcctg tgtgaaattg ttatccgctc 60
   acaattccac acaacatacg agccggaagc ataaagtgta aagcctgggg tgcctaatga 120
   gtgagctaac tcacattaat tgcgttgcgc tcactgcccg ctttccagtc gggaaacctg 180
   tcgtgccagc tgcattaatg aatcggccaa cgcgcgggga gaggcggttt gcgtattggg 240
   cgctcttccg cttcctcgct cactgactcg ctgcgctcgg tcgttcggct gcggcgagcg 300
   gtatcagctc actcaaaggc ggtaatacgg ttatccacag aatcagggga taacgcagga 360
   aagaacatgt gagcaaaagg ccagcaaaag gccaggaacc gtaaaaaggc cgcgttgctg 420
   gcgtttttcc ataggctccg cccccctgac gagcatcaca aaaatcgacg ctcaagtcag 480
   aggtggcgaa acccgacagg actataaaga taccaggcgt ttccccctgg aagctccctc 540
   gtgcgctctc ctgttccgac cctgccgctt accggatacc tgtccgcctt tctcccttcg 600
   ggaagcgtgg cgctttctca tagctcacgc tgtaggtatc tcagttcggt gtaggtcgtt 660
   cgctccaagc tgggctgtgt gcacgaaccc cccgttcagc ccgaccgctg cgccttatcc 720
   ggtaactatc gtcttgagtc caacccggta agacacgact tatcgccact ggcagcagcc 780
   actggtaaca ggattagcag agcgaggtat gtaggcggtg ctacagagtt cttgaagtgg 840
   tggcctaact acggctacac tagaaggaca gtatttggta tctgcgctct gctgaagcca 900
   gttaccttcg gaaaaagagt tggtagctct tgatccggca aacaaaccac cgctggtagc 960
   ggtggttttt ttgtttgcaa gcagcagatt acgcgcagaa aaaaaggatc tcaagaagat 1020
   cctttgatct tttctacggg gtctgacgct cagtggaacg aaaactcacg ttaagggatt 1080
   ttggtcatga gattatcaaa aaggatcttc acctagatcc ttttaaatta aaaatgaagt 1140
   tttaaatcaa tctaaagtat atatgagtaa acttggtctg acagttacca atgcttaatc 1200
   agtgaggcac ctatctcagc gatctgtcta tttcgttcat ccatagttgc ctgactcccc 1260
   gtcgtgtaga taactacgat acgggagggc ttaccatctg gccccagtgc tgcaatgata 1320
   ccgcgagacc cacgctcacc ggctccagat ttatcagcaa taaaccagcc agccggaagg 1380
   gccgagcgca gaagtggtcc tgcaacttta tccgcctcca tccagtctat taattgttgc 1440
   cgggaagcta gagtaagtag ttcgccagtt aatagtttgc gcaacgttgt tgccattgct 1500
   acaggcatcg tggtgtcacg ctcgtcgttt ggtatggctt cattcagctc cggttcccaa 1560
   cgatcaaggc gagttacatg atcccccatg ttgtgcaaaa aagcggttag ctccttcggt 1620
   cctccgatcg ttgtcagaag taagttggcc gcagtgttat cactcatggt tatggcagca 1680
   ctgcataatt ctcttactgt catgccatcc gtaagatgct tttctgtgac tggtgagtac 1740
   tcaaccaagt cattctgaga atagtgtatg cggcgaccga gttgctcttg cccggcgtca 1800
   atacgggata ataccgcgcc acatagcaga actttaaaag tgctcatcat tggaaaacgt 1860
   tcttcggggc gaaaactctc aaggatctta ccgctgttga gatccagttc gatgtaaccc 1920
   actcgtgcac ccaactgatc ttcagcatct tttactttca ccagcgtttc tgggtgagca 1980
   aaaacaggaa ggcaaaatgc cgcaaaaaag ggaataaggg cgacacggaa atgttgaata 2040
   ctcatactct tcctttttca atattattga agcatttatc agggttattg tctcatgagc 2100
   ggatacatat ttgaatgtat ttagaaaaat aaacaaatag gggttccgcg cacatttccc 2160
   cgaaaagtgc cacctgacgc gccctgtagc ggcgcattaa gcgcggcggg tgtggtggtt 2220
   acgcgcagcg tgaccgctac acttgccagc gccctagcgc ccgctccttt cgctttcttc 2280
   ccttcctttc tcgccacgtt cgccggcttt ccccgtcaag ctctaaatcg ggggctccct 2340
   ttagggttcc gatttagtgc tttacggcac ctcgacccca aaaaacttga ttagggtgat 2400
   ggttcacgta gtgggccatc gccctgatag acggtttttc gccctttgac gttggagtcc 2460
   acgttcttta atagtggact cttgttccaa actggaacaa cactcaaccc tatctcggtc 2520
   tattcttttg atttataagg gattttgccg atttcggcct attggttaaa aaatgagctg 2580
   atttaacaaa aatttaacgc gaattttaac aaaatattaa cgcttacaat ttccattcgc 2640
   cattcaggct gcgcaactgt tgggaagggc gatcggtgcg ggcctcttcg ctattacgcc 2700
   agctggcgaa agggggatgt gctgcaaggc gattaagttg ggtaacgcca gggttttccc 2760
   agtcacgacg ttgtaaaacg acggccagtg aattgtaata cgactcacta tagggcgaat 2820
   tgggtaccgg gccccccctc gaggtcgacg agtatctgtc tgactcgtca ttgccgcctt 2880
   tggagtacga ctccaactat gagtgtgctt ggatcacttt gacgatacat tcttcgttgg 2940
   aggctgtggg tctgacagct gcgttttcgg cgcggttggc cgacaacaat atcagctgca 3000
   acgtcattgc tggctttcat catgatcaca tttttgtcgg caaaggcgac gcccagagag 3060
   ccattgacgt tctttctaat ttggaccgat agccgtatag tccagtctat ctataagttc 3120
   aactaactcg taactattac cataacatat acttcactgc cccagataag gttccgataa 3180
   aaagttctgc agactaaatt tatttcagtc tcctcttcac caccaaaatg ccctcctacg 3240
   aagctcgagt gctcaagctc gtggcagcca agaaaaccaa cctgtgtgct tctctggatg 3300
   ttaccaccac caaggagctc attgagcttg ccgataaggt cggaccttat gtgtgcatga 3360
   tcaaaaccca tatcgacatc attgacgact tcacctacgc cggcactgtg ctccccctca 3420
   aggaacttgc tcttaagcac ggtttcttcc tgttcgagga cagaaagttc gcagatattg 3480
   gcaacactgt caagcaccag taccggtgtc accgaatcgc cgagtggtcc gatatcacca 3540
   acgcccacgg tgtacccgga accggaatcg attgctggcc tgcgagctgg tgcgtacgag 3600
   gaaactgtct ctgaacagaa gaaggaggac gtctctgact acgagaactc ccagtacaag 3660
   gagttcctag tcccctctcc caacgagaag ctggccagag gtctgctcat gctggccgag 3720
   ctgtcttgca agggctctct ggccactggc gagtactcca agcagaccat tgagcttgcc 3780
   cgatccgacc ccgagtttgt ggttggcttc attgcccaga accgacctaa gggcgactct 3840
   gaggactggc ttattctgac ccccggggtg ggtcttgacg acaagggaga cgctctcgga 3900
   cagcagtacc gaactgttga ggatgtcatg tctaccggaa cggatatcat aattgtcggc 3960
   cgaggtctgt acggccagaa ccgagatcct attgaggagg ccaagcgata ccagaaggct 4020
   ggctgggagg cttaccagaa gattaactgt tagaggttag actatggata tgtaatttaa 4080
   ctgtgtatat agagagcgtg caagtatgga gcgcttgttc agcttgtatg atggtcagac 4140
   gacctgtctg atcgagtatg tatgatactg cacaacctgt gtatccgcat gatctgtcca 4200
   atggggcatg ttgttgtgtt tctcgatacg gagatgctgg gtacagtgct aatacgttga 4260
   actacttata cttatatgag gctcgaagaa agctgacttg tgtatgactt aat 4313
<210> 131
   <211> 15991
   <212> DNA
   <213> Artificial Sequence
<220>
   <223> Plasmid pZKL2-5mB89C
<400> 131
   gtacgttatc atttgaacag tgaaaggcta cagtaacaga agcagttgta aacttcattc 60
   cgttgattct gtactacagt accccactac gccgcttccg ctgacactgt tcaacccaaa 120
   aactacatct gcgtgcgctg tgtaaggcta tcatcagata catactgtag attctgtaga 180
   tgcgaacctg cttgtatcat atacatcccc ctccccctga cctgcacaag caagcaatgt 240
   gacattgata ttgctgctta tctagtgccg aggatgtgaa agccgagact caaacatttc 300
   ttttactctc ttgttcctga ccagacctgg cggagattac gccagtatga ttcttgcagg 360
   tctgagacaa gcctggaaca gccaacattt atttttcgaa gcgagaaaca tgccacaccc 420
   cggcacgttc agagatgcat atgatttgtt tttcgagtaa cagtaccccc cccccccccc 480
   ccaatgaaac cagtattact cacaccatcc tcattcaaag cgttacactg attacgcgcc 540
   catcaacgac agcatgaggg gactgctgat ctgatctaat caaatgacta caaaaatcgc 600
   aataatgaag agcaaacgac aaaaaagaaa caggttaacc aatcccgctt caatgtctca 660
   ccacaatcca gcactgtttc tcattacctc ctccctctaa tttcagagtt gcatcagggt 720
   ccttgatggc gcgccagctg cattaatgaa tcggccaacg cgcggggaga ggcggtttgc 780
   gtattgggcg ctcttccgct tcctcgctca ctgactcgct gcgctcggtc gttcggctgc 840
   ggcgagcggt atcagctcac tcaaaggcgg taatacggtt atccacagaa tcaggggata 900
   acgcaggaaa gaacatgtga gcaaaaggcc agcaaaaggc caggaaccgt aaaaaggccg 960
   cgttgctggc gtttttccat aggctccgcc cccctgacga gcatcacaaa aatcgacgct 1020
   caagtcagag gtggcgaaac ccgacaggac tataaagata ccaggcgttt ccccctggaa 1080
   gctccctcgt gcgctctcct gttccgaccc tgccgcttac cggatacctg tccgcctttc 1140
   tcccttcggg aagcgtggcg ctttctcata gctcacgctg taggtatctc agttcggtgt 1200
   aggtcgttcg ctccaagctg ggctgtgtgc acgaaccccc cgttcagccc gaccgctgcg 1260
   ccttatccgg taactatcgt cttgagtcca acccggtaag acacgactta tcgccactgg 1320
   cagcagccac tggtaacagg attagcagag cgaggtatgt aggcggtgct acagagttct 1380
   tgaagtggtg gcctaactac ggctacacta gaagaacagt atttggtatc tgcgctctgc 1440
   tgaagccagt taccttcgga aaaagagttg gtagctcttg atccggcaaa caaaccaccg 1500
   ctggtagcgg tggttttttt gtttgcaagc agcagattac gcgcagaaaa aaaggatctc 1560
   aagaagatcc tttgatcttt tctacggggt ctgacgctca gtggaacgaa aactcacgtt 1620
   aagggatttt ggtcatgaga ttatcaaaaa ggatcttcac ctagatcctt ttaaattaaa 1680
   aatgaagttt taaatcaatc taaagtatat atgagtaaac ttggtctgac agttaccaat 1740
   gcttaatcag tgaggcacct atctcagcga tctgtctatt tcgttcatcc atagttgcct 1800
   gactccccgt cgtgtagata actacgatac gggagggctt accatctggc cccagtgctg 1860
   caatgatacc gcgagaccca cgctcaccgg ctccagattt atcagcaata aaccagccag 1920
   ccggaagggc cgagcgcaga agtggtcctg caactttatc cgcctccatc cagtctatta 1980
   attgttgccg ggaagctaga gtaagtagtt cgccagttaa tagtttgcgc aacgttgttg 2040
   ccattgctac aggcatcgtg gtgtcacgct cgtcgtttgg tatggcttca ttcagctccg 2100
   gttcccaacg atcaaggcga gttacatgat cccccatgtt gtgcaaaaaa gcggttagct 2160
   ccttcggtcc tccgatcgtt gtcagaagta agttggccgc agtgttatca ctcatggtta 2220
   tggcagcact gcataattct cttactgtca tgccatccgt aagatgcttt tctgtgactg 2280
   gtgagtactc aaccaagtca ttctgagaat agtgtatgcg gcgaccgagt tgctcttgcc 2340
   cggcgtcaat acgggataat accgcgccac atagcagaac tttaaaagtg ctcatcattg 2400
   gaaaacgttc ttcggggcga aaactctcaa ggatcttacc gctgttgaga tccagttcga 2460
   tgtaacccac tcgtgcaccc aactgatctt cagcatcttt tactttcacc agcgtttctg 2520
   ggtgagcaaa aacaggaagg caaaatgccg caaaaaaggg aataagggcg acacggaaat 2580
   gttgaatact catactcttc ctttttcaat attattgaag catttatcag ggttattgtc 2640
   tcatgagcgg atacatattt gaatgtattt agaaaaataa acaaataggg gttccgcgca 2700
   catttccccg aaaagtgcca cctgatgcgg tgtgaaatac cgcacagatg cgtaaggaga 2760
   aaataccgca tcaggaaatt gtaagcgtta atattttgtt aaaattcgcg ttaaattttt 2820
   gttaaatcag ctcatttttt aaccaatagg ccgaaatcgg caaaatccct tataaatcaa 2880
   aagaatagac cgagataggg ttgagtgttg ttccagtttg gaacaagagt ccactattaa 2940
   agaacgtgga ctccaacgtc aaagggcgaa aaaccgtcta tcagggcgat ggcccactac 3000
   gtgaaccatc accctaatca agttttttgg ggtcgaggtg ccgtaaagca ctaaatcgga 3060
   accctaaagg gagcccccga tttagagctt gacggggaaa gccggcgaac gtggcgagaa 3120
   aggaagggaa gaaagcgaaa ggagcgggcg ctagggcgct ggcaagtgta gcggtcacgc 3180
   tgcgcgtaac caccacaccc gccgcgctta atgcgccgct acagggcgcg tccattcgcc 3240
   attcaggctg cgcaactgtt gggaagggcg atcggtgcgg gcctcttcgc tattacgcca 3300
   gctggcgaaa gggggatgtg ctgcaaggcg attaagttgg gtaacgccag ggttttccca 3360
   gtcacgacgt tgtaaaacga cggccagtga attgtaatac gactcactat agggcgaatt 3420
   gggcccgacg tcgcatgctg gtttcgattt gtcttagagg aacgcatata cagtaatcat 3480
   agagaataaa cgatattcat ttattaaagt agatagttga ggtagaagtt gtaaagagtg 3540
   ataaatagct tagataccac agacaccctc ggtgacgaag tactgcagat ggtttccaat 3600
   cacattgacc tgctggagca gagtgttacc ggcagagcac tgtttattgc tctggccctg 3660
   gcacatgaca acgttggaga gaggagggtg gatcaggggc cagtcaataa agacctcacc 3720
   agagcagtgc tggtaaccgt cccagaaggg cacttgaggg acgatatctc ctcggtgggt 3780
   gattcggtag agctttcggt ctttggacac cttggagaca tcggggttct cctggccaaa 3840
   gaagagttta tcgacccagt tagcaaagcc agcgttaccg acaatgggct gaccaagagt 3900
   aacaacgagg ggatcgtggc cgttaacctt gaggttgatt ccgaacagaa gggctgcagc 3960
   tcctccgaga gagtgaccgg tgacagcaat ctggtagtcg ggatactgct caatcacaga 4020
   gtcgagcttg gggccgatct gattgtaggt gttgttgtag gactggatga agccattgtg 4080
   gacaagacag tcatcacaag tagcagtaga agagatgtta gcagcaagat caaagttaat 4140
   taactcacct gcaggattga gactatgaat ggattcccgt gcccgtatta ctctactaat 4200
   ttgatcttgg aacgcgaaaa tacgtttcta ggactccaaa gaatctcaac tcttgtcctt 4260
   actaaatata ctacccatag ttgatggttt acttgaacag agaggacatg ttcacttgac 4320
   ccaaagtttc tcgcatctct tggatatttg aacaacggcg tccactgacc gtcagttatc 4380
   cagtcacaaa acccccacat tcatacattc ccatgtacgt ttacaaagtt ctcaattcca 4440
   tcgtgcaaat caaaatcaca tctattcatt catcatatat aaacccatca tgtctactaa 4500
   cactcacaac tccatagaaa acatcgactc agaacacacg ctccatgcgg ccgcttagga 4560
   atcctgagcg tccttgacac agtgaaccac accgactttg tgcatgtact tgagggtgga 4620
   aatgatgttg cccacaatgg tagggtagaa gacgtaccga actccgtgtc gttcgcaaca 4680
   ctctcggaca gcttgctgca cgaagggata gtgccaagac gacattcgag gaaagaggtg 4740
   atgctcgatc tggaagttga gaccgccagt aaagaacatg gcaatgggtc caccgtaggt 4800
   ggaagaggtc tccacctgag ctctgtacca gtcgatctga tcggcttcaa cgtccttctc 4860
   ggagctcttg accttgcagt tcttgtcggg gattcgctcc gagccatcga agttgtgaga 4920
   caagatgaaa aagaaggtga ggaaggcacc ggtagcagtg ggcaccagag gaatggtgat 4980
   gagcagggag gttccagtga gataccaggg caagaaggcg gttcgaaaga tgaagaaagc 5040
   tcgcataacg aatgcaaggg ttcggtaccg tcgcagaaag ccgttctctc gcatggctgt 5100
   gacagactcg ggaatggtgt cgttgtgctg cattcggaag atgtagagag ggttgtacac 5160
   cagcgaaacg ccgtaggctc caagcacgag gtacatgtac caggcctgga atcggtgaaa 5220
   ccactttcga gcagtgttgg cagcagggta gttgtggaac acaaggaatg gttctgcgga 5280
   ctcggcatcc aggtcgagac catgctgatt ggtgtaggtg tgatgtcgca tgatgtgaga 5340
   ctgcagccag atccatctgg acgatccaat gacgtcgatg ccgtaggcaa agagagcgtt 5400
   gacccagggc tttttgctga tggcaccatg agaggcatcg tgctgaatgg acaggccgat 5460
   ctgcatgtgc atgaatccag tcaagagacc ccacagcacc attccggtag tagcccagtg 5520
   ccactcgcaa aaggcggtga cagcaatgat gccaacggtt cgcagccaga atccaggtgt 5580
   ggcataccag ttccgacctt tcatgacctc tcgcatagtt cgcttgacgt cctgtgcaaa 5640
   gggagagtcg taggtgtaga caatgtcctt ggaggttcgg tcgtgcttgc ctcgcacgaa 5700
   ctgttgaagc agcttcgagt tctcgggctt gacgtaaggg tgcatggagt agaacagagg 5760
   agaagcatcg gaggcaccag aagcgaggat caagtcggat ccgggatgga ccttggcaag 5820
   accttccaga tcgtagagaa tgccgtcgat ggcaaccagg tcgggtcgct cgagcagctg 5880
   ctcggtagta agggagagag ccatgggcag gacctgtgtt agtacattgt cggggagtca 5940
   tcaattggtt cgacaggttg tcgactgtta gtatgagctc aattgggctc tggtgggtcg 6000
   atgacacttg tcatctgttt ctgttgggtc atgtttccat caccttctat ggtactcaca 6060
   attcgtccga ttcgcccgaa tccgttaata ccgactttga tggccatgtt gatgtgtgtt 6120
   taattcaaga atgaatatag agaagagaag aagaaaaaag attcaattga gccggcgatg 6180
   cagaccctta tataaatgtt gccttggaca gacggagcaa gcccgcccaa acctacgttc 6240
   ggtataatat gttaagcttt ttaacacaaa ggtttggctt ggggtaacct gatgtggtgc 6300
   aaaagaccgg gcgttggcga gccattgcgc gggcgaatgg ggccgtgact cgtctcaaat 6360
   tcgagggcgt gcctcaattc gtgcccccgt ggctttttcc cgccgtttcc gccccgtttg 6420
   caccactgca gccgcttctt tggttcggac accttgctgc gagctaggtg ccttgtgcta 6480
   cttaaaaagt ggcctcccaa caccaacatg acatgagtgc gtgggccaag acacgttggc 6540
   ggggtcgcag tcggctcaat ggcccggaaa aaacgctgct ggagctggtt cggacgcagt 6600
   ccgccgcggc gtatggatat ccgcaaggtt ccatagcgcc attgccctcc gtcggcgtct 6660
   atcccgcaac ctctaaatag agcgggaata taacccaagc ttcttttttt tcctttaaca 6720
   cgcacacccc caactatcat gttgctgctg ctgtttgact ctactctgtg gaggggtgct 6780
   cccacccaac ccaacctaca ggtggatccg gcgctgtgat tggctgataa gtctcctatc 6840
   cggactaatt ctgaccaatg ggacatgcgc gcaggaccca aatgccgcaa ttacgtaacc 6900
   ccaacgaaat gcctacccct ctttggagcc cagcggcccc aaatcccccc aagcagcccg 6960
   gttctaccgg cttccatctc caagcacaag cagcccggaa ttctctctct tgagcttttc 7020
   cataacaagt tcttctgcct ccaggaagtc catgggtggt ttgatcatgg ttttggtgta 7080
   gtggtagtgc agtggtggta ttgtgactgg ggatgtagtt gagaataagt catacacaag 7140
   tcagctttct tcgagcctca tataagtata agtagttcaa cgtattagca ctgtacccag 7200
   catctccgta tcgagaaaca caacaacatg ccccattgga cagatcatgc ggatacacag 7260
   gttgtgcagt atcatacata ctcgatcaga caggtcgtct gaccatcata caagctgaac 7320
   aagcgctcca tacttgcacg ctctctatat acacagttaa attacatatc catagtctaa 7380
   cctctaacag ttaatcttct ggtaagcctc ccagccagcc ttctggtatc gcttggcctc 7440
   ctcaatagga tctcggttct ggccgtacag acctcggccg acaattatga tatccgttcc 7500
   ggtagacatg acatcctcaa cagttcggta ctgctgtccg agagcgtctc ccttgtcgtc 7560
   aagacccacc ccgggggtca gaataagcca gtcctcagag tcgcccttag gtcggttctg 7620
   ggcaatgaag ccaaccacaa actcggggtc ggatcgggca agctcaatgg tctgcttgga 7680
   gtactcgcca gtggccagag agcccttgca agacagctcg gccagcatga gcagacctct 7740
   ggccagcttc tcgttgggag aggggactag gaactccttg tactgggagt tctcgtagtc 7800
   agagacgtcc tccttcttct gttcagagac agtttcctcg gcaccagctc gcaggccagc 7860
   aatgattccg gttccgggta caccgtgggc gttggtgata tcggaccact cggcgattcg 7920
   gtgacaccgg tactggtgct tgacagtgtt gccaatatct gcgaactttc tgtcctcgaa 7980
   caggaagaaa ccgtgcttaa gagcaagttc cttgaggggg agcacagtgc cggcgtaggt 8040
   gaagtcgtca atgatgtcga tatgggtttt gatcatgcac acataaggtc cgaccttatc 8100
   ggcaagctca atgagctcct tggtggtggt aacatccaga gaagcacaca ggttggtttt 8160
   cttggctgcc acgagcttga gcactcgagc ggcaaaggcg gacttgtgga cgttagctcg 8220
   agcttcgtag gagggcattt tggtggtgaa gaggagactg aaataaattt agtctgcaga 8280
   actttttatc ggaaccttat ctggggcagt gaagtatatg ttatggtaat agttacgagt 8340
   tagttgaact tatagataga ctggactata cggctatcgg tccaaattag aaagaacgtc 8400
   aatggctctc tgggcgtcgc ctttgccgac aaaaatgtga tcatgatgaa agccagcaat 8460
   gacgttgcag ctgatattgt tgtcggccaa ccgcgccgaa aacgcagctg tcagacccac 8520
   agcctccaac gaagaatgta tcgtcaaagt gatccaagca cactcatagt tggagtcgta 8580
   ctccaaaggc ggcaatgacg agtcagacag atactcgtcg accttttcct tgggaaccac 8640
   caccgtcagc ccttctgact cacgtattgt agccaccgac acaggcaaca gtccgtggat 8700
   agcagaatat gtcttgtcgg tccatttctc accaacttta ggcgtcaagt gaatgttgca 8760
   gaagaagtat gtgccttcat tgagaatcgg tgttgctgat ttcaataaag tcttgagatc 8820
   agtttggcca gtcatgttgt ggggggtaat tggattgagt tatcgcctac agtctgtaca 8880
   ggtatactcg ctgcccactt tatacttttt gattccgctg cacttgaagc aatgtcgttt 8940
   accaaaagtg agaatgctcc acagaacaca ccccagggta tggttgagca aaaaataaac 9000
   actccgatac ggggaatcga accccggtct ccacggttct caagaagtat tcttgatgag 9060
   agcgtatcga tcgaggaaga ggacaagcgg ctgcttctta agtttgtgac atcagtatcc 9120
   aaggcaccat tgcaaggatt caaggctttg aacccgtcat ttgccattcg taacgctggt 9180
   agacaggttg atcggttccc tacggcctcc acctgtgtca atcttctcaa gctgcctgac 9240
   tatcaggaca ttgatcaact tcggaagaaa cttttgtatg ccattcgatc acatgctggt 9300
   ttcgatttgt cttagaggaa cgcatataca gtaatcatag agaataaacg atattcattt 9360
   attaaagtag atagttgagg tagaagttgt aaagagtgat aaatagcggc cgctcactga 9420
   atctttttgg ctcccttgtg ctttcggacg atgtaggtct gcacgtagaa gttgaggaac 9480
   agacacagga cagtaccaac gtagaagtag ttgaaaaacc agccaaacat tctcattcca 9540
   tcttgtcggt agcagggaat gttccggtac ttccagacga tgtagaagcc aacgttgaac 9600
   tgaatgatct gcatagaagt aatcagggac ttgggcatag ggaacttgag cttgatcagt 9660
   cgggtccaat agtagccgta catgatccag tgaatgaagc cgttgagcag cacaaagatc 9720
   caaacggctt cgtttcggta gttgtagaac agccacatgt ccataggagc tccgagatgg 9780
   tgaaagaact gcaaccaggt cagaggcttg cccatgaggg gcagatagaa ggagtcaatg 9840
   tactcgagga acttgctgag gtagaacagc tgagtggtga ttcggaagac attgttgtcg 9900
   aaagccttct cgcagttgtc ggacatgaca ccaatggtgt acatggcgta ggccatagag 9960
   aggaaggagc ccagcgagta gatggacatg agcaggttgt agttggtgaa cacaaacttc 10020
   attcgagact gacccttggg tccgagagga ccaagggtga acttcaggat gacgaaggcg 10080
   atggagaggt acagcacctc gcagtgcgag gcatcagacc agagctgagc atagtcgacc 10140
   ttgggaagaa cctcctggcc aatggagacg atttcgttca cgacctccat ggttgtgaat 10200
   tagggtggtg agaatggttg gttgtaggga agaatcaaag gccggtctcg ggatccgtgg 10260
   gtatatatat atatatatat atatacgatc cttcgttacc tccctgttct caaaactgtg 10320
   gtttttcgtt tttcgttttt tgcttttttt gattttttta gggccaacta agcttccaga 10380
   tttcgctaat cacctttgta ctaattacaa gaaaggaaga agctgattag agttgggctt 10440
   tttatgcaac tgtgctactc cttatctctg atatgaaagt gtagacccaa tcacatcatg 10500
   tcatttagag ttggtaatac tgggaggata gataaggcac gaaaacgagc catagcagac 10560
   atgctgggtg tagccaagca gaagaaagta gatgggagcc aattgacgag cgagggagct 10620
   acgccaatcc gacatacgac acgctgagat cgtcttggcc ggggggtacc tacagatgtc 10680
   caagggtaag tgcttgactg taattgtatg tctgaggaca aatatgtagt cagccgtata 10740
   aagtcatacc aggcaccagt gccatcatcg aaccactaac tctctatgat acatgcctcc 10800
   ggtattattg taccatgcgt cgctttgtta catacgtatc ttgccttttt ctctcagaaa 10860
   ctccagactt tggctattgg tcgagataag cccggaccat agtgagtctt tcacactctg 10920
   tttaaacacc actaaaaccc cacaaaatat atcttaccga atatacagat ctactataga 10980
   ggaacaattg ccccggagaa gacggccagg ccgcctagat gacaaattca acaactcaca 11040
   gctgactttc tgccattgcc actagggggg ggccttttta tatggccaag ccaagctctc 11100
   cacgtcggtt gggctgcacc caacaataaa tgggtagggt tgcaccaaca aagggatggg 11160
   atggggggta gaagatacga ggataacggg gctcaatggc acaaataaga acgaatactg 11220
   ccattaagac tcgtgatcca gcgactgaca ccattgcatc atctaagggc ctcaaaacta 11280
   cctcggaact gctgcgctga tctggacacc acagaggttc cgagcacttt aggttgcacc 11340
   aaatgtccca ccaggtgcag gcagaaaacg ctggaacagc gtgtacagtt tgtcttaaca 11400
   aaaagtgagg gcgctgaggt cgagcagggt ggtgtgactt gttatagcct ttagagctgc 11460
   gaaagcgcgt atggatttgg ctcatcaggc cagattgagg gtctgtggac acatgtcatg 11520
   ttagtgtact tcaatcgccc cctggatata gccccgacaa taggccgtgg cctcattttt 11580
   ttgccttccg cacatttcca ttgctcggta cccacacctt gcttctcctg cacttgccaa 11640
   ccttaatact ggtttacatt gaccaacatc ttacaagcgg ggggcttgtc tagggtatat 11700
   ataaacagtg gctctcccaa tcggttgcca gtctcttttt tcctttcttt ccccacagat 11760
   tcgaaatcta aactacacat cacacaatgc ctgttactga cgtccttaag cgaaagtccg 11820
   gtgtcatcgt cggcgacgat gtccgagccg tgagtatcca cgacaagatc agtgtcgaga 11880
   cgacgcgttt tgtgtaatga cacaatccga aagtcgctag caacacacac tctctacaca 11940
   aactaaccca gctctccatg gtgaaggctt ctcgacaggc tctgcccctc gtcatcgacg 12000
   gaaaggtgta cgacgtctcc gcttgggtga acttccaccc tggtggagct gaaatcattg 12060
   agaactacca gggacgagat gctactgacg ccttcatggt tatgcactct caggaagcct 12120
   tcgacaagct caagcgaatg cccaagatca accaggcttc cgagctgcct ccccaggctg 12180
   ccgtcaacga agctcaggag gatttccgaa agctccgaga agagctgatc gccactggca 12240
   tgtttgacgc ctctcccctc tggtactcgt acaagatctt gaccaccctg ggtcttggcg 12300
   tgcttgcctt cttcatgctg gtccagtacc acctgtactt cattggtgct ctcgtgctcg 12360
   gtatgcacta ccagcaaatg ggatggctgt ctcatgacat ctgccaccac cagaccttca 12420
   agaaccgaaa ctggaataac gtcctgggtc tggtctttgg caacggactc cagggcttct 12480
   ccgtgacctg gtggaaggac agacacaacg cccatcattc tgctaccaac gttcagggtc 12540
   acgatcccga cattgataac ctgcctctgc tcgcctggtc cgaggacgat gtcactcgag 12600
   cttctcccat ctcccgaaag ctcattcagt tccaacagta ctatttcctg gtcatctgta 12660
   ttctcctgcg attcatctgg tgtttccagt ctgtgctgac cgttcgatcc ctcaaggacc 12720
   gagacaacca gttctaccga tctcagtaca agaaagaggc cattggactc gctctgcact 12780
   ggactctcaa gaccctgttc cacctcttct ttatgccctc catcctgacc tcgatgctgg 12840
   tgttctttgt ttccgagctc gtcggtggct tcggaattgc catcgtggtc ttcatgaacc 12900
   actaccctct ggagaagatc ggtgattccg tctgggacgg acatggcttc tctgtgggtc 12960
   agatccatga gaccatgaac attcgacgag gcatcattac tgactggttc tttggaggcc 13020
   tgaactacca gatcgagcac catctctggc ccaccctgcc tcgacacaac ctcactgccg 13080
   tttcctacca ggtggaacag ctgtgccaga agcacaacct cccctaccga aaccctctgc 13140
   cccatgaagg tctcgtcatc ctgctccgat acctgtccca gttcgctcga atggccgaga 13200
   agcagcccgg tgccaaggct cagtaagcgg ccgcatgaga agataaatat ataaatacat 13260
   tgagatatta aatgcgctag attagagagc ctcatactgc tcggagagaa gccaagacga 13320
   gtactcaaag gggattacac catccatatc cacagacaca agctggggaa aggttctata 13380
   tacactttcc ggaataccgt agtttccgat gttatcaatg ggggcagcca ggatttcagg 13440
   cacttcggtg tctcggggtg aaatggcgtt cttggcctcc atcaagtcgt accatgtctt 13500
   catttgcctg tcaaagtaaa acagaagcag atgaagaatg aacttgaagt gaaggaattt 13560
   aaatagttgg agcaagggag aaatgtagag tgtgaaagac tcactatggt ccgggcttat 13620
   ctcgaccaat agccaaagtc tggagtttct gagagaaaaa ggcaagatac gtatgtaaca 13680
   aagcgacgca tggtacaata ataccggagg catgtatcat agagagttag tggttcgatg 13740
   atggcactgg tgcctggtat gactttatac ggctgactac atatttgtcc tcagacatac 13800
   aattacagtc aagcacttac ccttggacat ctgtaggtac cccccggcca agacgatctc 13860
   agcgtgtcgt atgtcggatt ggcgtagctc cctcgctcgt caattggctc ccatctactt 13920
   tcttctgctt ggctacaccc agcatgtctg ctatggctcg ttttcgtgcc ttatctatcc 13980
   tcccagtatt accaactcta aatgacatga tgtgattggg tctacacttt catatcagag 14040
   ataaggagta gcacagttgc ataaaaagcc caactctaat cagcttcttc ctttcttgta 14100
   attagtacaa aggtgattag cgaaatctgg aagcttagtt ggccctaaaa aaatcaaaaa 14160
   aagcaaaaaa cgaaaaacga aaaaccacag ttttgagaac agggaggtaa cgaaggatcg 14220
   tatatatata tatatatata tatacccacg gatcccgaga ccggcctttg attcttccct 14280
   acaaccaacc attctcacca ccctaattca caaccatggg cgtattcatt aaacaggagc 14340
   agcttccggc tctcaagaag tacaagtact ccgccgagga tcactcgttc atctccaaca 14400
   acattctgcg ccccttctgg cgacagtttg tcaaaatctt ccctctgtgg atggccccca 14460
   acatggtgac tctgctgggc ttcttctttg tcattgtgaa cttcatcacc atgctcattg 14520
   ttgatcccac ccacgaccgc gagcctccca gatgggtcta cctcacctac gctctgggtc 14580
   tgttccttta ccagacattt gatgcctgtg acggatccca tgcccgacga actggccaga 14640
   gtggacccct tggagagctg tttgaccact gtgtcgacgc catgaatacc tctctgattc 14700
   tcacggtggt ggtgtccacc acccatatgg gatataacat gaagctactg attgtgcaga 14760
   ttgccgctct cggaaacttc tacctgtcga cctgggagac ctaccatacc ggaactctgt 14820
   acctttctgg cttctctggt cctgttgaag gtatcttgat tctggtggct cttttcgtcc 14880
   tcaccttctt cactggtccc aacgtgtacg ctctgaccgt ctacgaggct cttcccgagt 14940
   ccatcacttc gctgctgcct gccagcttcc tggacgtcac catcacccag atctacattg 15000
   gattcggagt gctgggcatg gtgttcaaca tctacggcgc ctgcggaaac gtgatcaagt 15060
   actacaacaa caagggcaag agcgctctcc ccgccattct cggaatcgcc ccctttggca 15120
   tcttctacgt cggcgtcttt gcctgggccc atgttgctcc tctgcttctc tccaagtacg 15180
   ccatcgtcta tctgtttgcc attggggctg cctttgccat gcaagtcggc cagatgattc 15240
   ttgcccatct cgtgcttgct ccctttcccc actggaacgt gctgctcttc ttcccctttg 15300
   tgggactggc agtgcactac attgcacccg tgtttggctg ggacgccgat atcgtgtcgg 15360
   ttaacactct cttcacctgt tttggcgcca ccctctccat ttacgccttc tttgtgcttg 15420
   agatcatcga cgagatcacc aactacctcg atatctggtg tctgcgaatc aagtaccctc 15480
   aggagaagaa gaccgaataa gcggccgcat ggagcgtgtg ttctgagtcg atgttttcta 15540
   tggagttgtg agtgttagta gacatgatgg gtttatatat gatgaatgaa tagatgtgat 15600
   tttgatttgc acgatggaat tgagaacttt gtaaacgtac atgggaatgt atgaatgtgg 15660
   gggttttgtg actggataac tgacggtcag tggacgccgt tgttcaaata tccaagagat 15720
   gcgagaaact ttgggtcaag tgaacatgtc ctctctgttc aagtaaacca tcaactatgg 15780
   gtagtatatt tagtaaggac aagagttgag attctttgga gtcctagaaa cgtattttcg 15840
   cgttccaaga tcaaattagt agagtaatac gggcacggga atccattcat agtctcaatc 15900
   ctgcaggtga gttaattaat cgagcttggc gtaatcatgg tcatagctgt ttcctgtgtg 15960
   aaattgttat ccgctcacaa ttccacacaa c 15991
<210> 132
   <211> 14554
   <212> DNA
   <213> Artificial Sequence
<220>
   <223> Plasmid pZKL1-2SR9G85
<400> 132
   cgatagttgg agcaagggag aaatgtagag tgtgaaagac tcactatggt ccgggcttat      60
   ctcgaccaat agccaaagtc tggagtttct gagagaaaaa ggcaagatac gtatgtaaca     120
   aagcgacgca tggtacaata ataccggagg catgtatcat agagagttag tggttcgatg     180
   atggcactgg tgcctggtat gactttatac ggctgactac atatttgtcc tcagacatac     240
   aattacagtc aagcacttac ccttggacat ctgtaggtac cccccggcca agacgatctc     300
   agcgtgtcgt atgtcggatt ggcgtagctc cctcgctcgt caattggctc ccatctactt     360
   tcttctgctt ggctacaccc agcatgtctg ctatggctcg ttttcgtgcc ttatctatcc     420
   tcccagtatt accaactcta aatgacatga tgtgattggg tctacacttt catatcagag     480
   ataaggagta gcacagttgc ataaaaagcc caactctaat cagcttcttc ctttcttgta     540
   attagtacaa aggtgattag cgaaatctgg aagcttagtt ggccctaaaa aaatcaaaaa     600
   aagcaaaaaa cgaaaaacga aaaaccacag ttttgagaac agggaggtaa cgaaggatcg     660
   tatatatata tatatatata tatacccacg gatcccgaga ccggcctttg attcttccct     720
   acaaccaacc attctcacca ccctaattca caaccatggc tgccgtcatc gaggtggcca     780
   acgagttcgt cgctatcact gccgagaccc ttcccaaggt ggactatcag cgactctggc     840
   gagacatcta ctcctgcgag ctcctgtact tctccattgc tttcgtcatc ctcaagttta 900
   cccttggcga gctctcggat tctggcaaaa agattctgcg agtgctgttc aagtggtaca 960
   acctcttcat gtccgtcttt tcgctggtgt ccttcctctg tatgggttac gccatctaca 1020
   ccgttggact gtactccaac gaatgcgaca gagctttcga caacagcttg ttccgatttg 1080
   ccaccaaggt cttctactat tccaagtttc tggagtacat cgactctttc taccttcccc 1140
   tcatggccaa gcctctgtcc tttctgcagt tctttcatca cttgggagct cctatggaca 1200
   tgtggctctt cgtgcagtac tctggcgaat ccatttggat ctttgtgttc ctgaacggat 1260
   tcattcactt tgtcatgtac ggctactatt ggacacggct gatgaagttc aactttccca 1320
   tgcccaagca gctcattacc gcaatgcaga tcacccagtt caacgttggc ttctacctcg 1380
   tgtggtggta caaggacatt ccctgttacc gaaaggatcc catgcgaatg ctggcctgga 1440
   tcttcaacta ctggtacgtc ggtaccgttc ttctgctctt catcaacttc tttgtcaagt 1500
   cctacgtgtt tcccaagcct aagactgccg acaaaaaggt ccagggcgcc ggtcccgctc 1560
   gacctgccgg acttcctccc gctacctact acgactctct ggccgtcatg ggatccgtga 1620
   aggcttctcg acaggctctg cccctcgtca tcgacggaaa ggtgtacgac gtctccgctt 1680
   gggtgaactt ccaccctggt ggagctgaaa tcattgagaa ctaccaggga cgagatgcta 1740
   ctgacgcctt catggttatg cactctcagg aagccttcga caagctcaag cgaatgccca 1800
   agatcaacca ggcttccgag ctgcctcccc aggctgccgt caacgaagct caggaggatt 1860
   tccgaaagct ccgagaagag ctgatcgcca ctggcatgtt tgacgcctct cccctctggt 1920
   actcgtacaa gatcttgacc accctgggtc ttggcgtgct tgccttcttc atgctggtcc 1980
   agtaccacct gtacttcatt ggtgctctcg tgctcggtat gcactaccag caaatgggat 2040
   ggctgtctca tgacatctgc caccaccaga ccttcaagaa ccgaaactgg aataacgtcc 2100
   tgggtctggt ctttggcaac ggactccagg gcttctccgt gacctggtgg aaggacagac 2160
   acaacgccca tcattctgct accaacgttc agggtcacga tcccgacatt gataacctgc 2220
   ctctgctcgc ctggtccgag gacgatgtca ctcgagcttc tcccatctcc cgaaagctca 2280
   ttcagttcca acagtactat ttcctggtca tctgtattct cctgcgattc atctggtgtt 2340
   tccagtctgt gctgaccgtt cgatccctca aggaccgaga caaccagttc taccgatctc 2400
   agtacaagaa agaggccatt ggactcgctc tgcactggac tctcaagacc ctgttccacc 2460
   tcttctttat gccctccatc ctgacctcga tgctggtgtt ctttgtttcc gagctcgtcg 2520
   gtggcttcgg aattgccatc gtggtcttca tgaaccacta ccctctggag aagatcggtg 2580
   attccgtctg ggacggacat ggcttctctg tgggtcagat ccatgagacc atgaacattc 2640
   gacgaggcat cattactgac tggttctttg gaggcctgaa ctaccagatc gagcaccatc 2700
   tctggcccac cctgcctcga cacaacctca ctgccgtttc ctaccaggtg gaacagctgt 2760
   gccagaagca caacctcccc taccgaaacc ctctgcccca tgaaggtctc gtcatcctgc 2820
   tccgatacct gtcccagttc gctcgaatgg ccgagaagca gcccggtgcc aaggctcagt 2880
   aagcggccgc atgagaagat aaatatataa atacattgag atattaaatg cgctagatta 2940
   gagagcctca tactgctcgg agagaagcca agacgagtac tcaaagggga ttacaccatc 3000
   catatccaca gacacaagct ggggaaaggt tctatataca ctttccggaa taccgtagtt 3060
   tccgatgtta tcaatggggg cagccaggat ttcaggcact tcggtgtctc ggggtgaaat 3120
   ggcgttcttg gcctccatca agtcgtacca tgtcttcatt tgcctgtcaa agtaaaacag 3180
   aagcagatga agaatgaact tgaagtgaag gaatttaaat gtaacgaaac tgaaatttga 3240
   ccagatattg tgtccgcggt ggagctccag cttttgttcc ctttagtgag ggttaatttc 3300
   gagcttggcg taatcatggt catagctgtt tcctgtgtga aattgttatc cgctcacaag 3360
   cttccacaca acgtacgata gttagtagac aacaatcaga acatctccct ccttatataa 3420
   tcacacaggc cagaacgcgc taaactaaag cgctttggac actatgttac attggcattg 3480
   attgaactga aaccacagtc tccctcgcct gaatcgagca atggatgttg tcggaagtca 3540
   acttcactag aagagcggtt ctatgccttg tcaagatcat atcataaact cactctgtat 3600
   taccccatct atagaacact tgttatgaat gggcggaaac attccgctat atgcaccttt 3660
   ccacactaat gcaaagatgt gcatcttcaa cgggtagtaa gactggttcc gacttccgtt 3720
   gcatggagag caatgacctc gataatgcga acatccccca catatacact cttacacagg 3780
   ccaatataat ctgtgcattt actaaatatt taagtctatg cacctgcttg atgaaaagcg 3840
   gcacggatgg tatcatctag tttccgccaa tccaagaacc aactgtgttg gcagtggtgt 3900
   agcccatggc acacagacca aagatgaaaa tacagacatc ggcggttcga gccgtggtgc 3960
   ctcgagcaac acccttgtaa tgcaaaagag gagggtaaat gtacaccaga ggcacacatg 4020
   caaacgatcc ggtgagagcg acgaaccgat cgagatcgtc ggcacctccc catgcaacaa 4080
   aggcggtgac aaacacaagg aagaaccgga aaatgttctt ctgccacttg atggtagagt 4140
   tgtacttgcc tgatcgggtg aagagaccat tctcgatgat tcggatggcg cgccagctgc 4200
   attaatgaat cggccaacgc gcggggagag gcggtttgcg tattgggcgc tcttccgctt 4260
   cctcgctcac tgactcgctg cgctcggtcg ttcggctgcg gcgagcggta tcagctcact 4320
   caaaggcggt aatacggtta tccacagaat caggggataa cgcaggaaag aacatgtgag 4380
   caaaaggcca gcaaaaggcc aggaaccgta aaaaggccgc gttgctggcg tttttccata 4440
   ggctccgccc ccctgacgag catcacaaaa atcgacgctc aagtcagagg tggcgaaacc 4500
   cgacaggact ataaagatac caggcgtttc cccctggaag ctccctcgtg cgctctcctg 4560
   ttccgaccct gccgcttacc ggatacctgt ccgcctttct cccttcggga agcgtggcgc 4620
   tttctcatag ctcacgctgt aggtatctca gttcggtgta ggtcgttcgc tccaagctgg 4680
   gctgtgtgca cgaacccccc gttcagcccg accgctgcgc cttatccggt aactatcgtc 4740
   ttgagtccaa cccggtaaga cacgacttat cgccactggc agcagccact ggtaacagga 4800
   ttagcagagc gaggtatgta ggcggtgcta cagagttctt gaagtggtgg cctaactacg 4860
   gctacactag aagaacagta tttggtatct gcgctctgct gaagccagtt accttcggaa 4920
   aaagagttgg tagctcttga tccggcaaac aaaccaccgc tggtagcggt ggtttttttg 4980
   tttgcaagca gcagattacg cgcagaaaaa aaggatctca agaagatcct ttgatctttt 5040
   ctacggggtc tgacgctcag tggaacgaaa actcacgtta agggattttg gtcatgagat 5100
   tatcaaaaag gatcttcacc tagatccttt taaattaaaa atgaagtttt aaatcaatct 5160
   aaagtatata tgagtaaact tggtctgaca gttaccaatg cttaatcagt gaggcaccta 5220
   tctcagcgat ctgtctattt cgttcatcca tagttgcctg actccccgtc gtgtagataa 5280
   ctacgatacg ggagggctta ccatctggcc ccagtgctgc aatgataccg cgagacccac 5340
   gctcaccggc tccagattta tcagcaataa accagccagc cggaagggcc gagcgcagaa 5400
   gtggtcctgc aactttatcc gcctccatcc agtctattaa ttgttgccgg gaagctagag 5460
   taagtagttc gccagttaat agtttgcgca acgttgttgc cattgctaca ggcatcgtgg 5520
   tgtcacgctc gtcgtttggt atggcttcat tcagctccgg ttcccaacga tcaaggcgag 5580
   ttacatgatc ccccatgttg tgcaaaaaag cggttagctc cttcggtcct ccgatcgttg 5640
   tcagaagtaa gttggccgca gtgttatcac tcatggttat ggcagcactg cataattctc 5700
   ttactgtcat gccatccgta agatgctttt ctgtgactgg tgagtactca accaagtcat 5760
   tctgagaata gtgtatgcgg cgaccgagtt gctcttgccc ggcgtcaata cgggataata 5820
   ccgcgccaca tagcagaact ttaaaagtgc tcatcattgg aaaacgttct tcggggcgaa 5880
   aactctcaag gatcttaccg ctgttgagat ccagttcgat gtaacccact cgtgcaccca 5940
   actgatcttc agcatctttt actttcacca gcgtttctgg gtgagcaaaa acaggaaggc 6000
   aaaatgccgc aaaaaaggga ataagggcga cacggaaatg ttgaatactc atactcttcc 6060
   tttttcaata ttattgaagc atttatcagg gttattgtct catgagcgga tacatatttg 6120
   aatgtattta gaaaaataaa caaatagggg ttccgcgcac atttccccga aaagtgccac 6180
   ctgatgcggt gtgaaatacc gcacagatgc gtaaggagaa aataccgcat caggaaattg 6240
   taagcgttaa tattttgtta aaattcgcgt taaatttttg ttaaatcagc tcatttttta 6300
   accaataggc cgaaatcggc aaaatccctt ataaatcaaa agaatagacc gagatagggt 6360
   tgagtgttgt tccagtttgg aacaagagtc cactattaaa gaacgtggac tccaacgtca 6420
   aagggcgaaa aaccgtctat cagggcgatg gcccactacg tgaaccatca ccctaatcaa 6480
   gttttttggg gtcgaggtgc cgtaaagcac taaatcggaa ccctaaaggg agcccccgat 6540
   ttagagcttg acggggaaag ccggcgaacg tggcgagaaa ggaagggaag aaagcgaaag 6600
   gagcgggcgc tagggcgctg gcaagtgtag cggtcacgct gcgcgtaacc accacacccg 6660
   ccgcgcttaa tgcgccgcta cagggcgcgt ccattcgcca ttcaggctgc gcaactgttg 6720
   ggaagggcga tcggtgcggg cctcttcgct attacgccag ctggcgaaag ggggatgtgc 6780
   tgcaaggcga ttaagttggg taacgccagg gttttcccag tcacgacgtt gtaaaacgac 6840
   ggccagtgaa ttgtaatacg actcactata gggcgaattg ggcccgacgt cgcatgctta 6900
   gaagtgagga ttacaagaag cctctggata tcaatgatga acgtactcag cggctggtca 6960
   agcatttcga ccgtcgaatc gacgaggtgt tcacctttga caagcgaggg ttcccaattg 7020
   atcacgttct cgagttgttc aaatcttctc tcaacatctc tctgcatgaa ctatctctgt 7080
   tgacgaacgt gtcacccact gttcctcgaa cgcccttctc cgagtttggt ctgaacatct 7140
   tcgatctcaa actgaccccc gcagtgatca atagtgccat gccactgccg atgcggtgcg 7200
   aacatccctg gagggattct cggagctcta cacaatgcag attctgtcgt cgagtactct 7260
   ctaccttgct cgaatgactt attgtgctac tactgcactc atgcttcgat catgtgccct 7320
   actgcacccc aaatttggtg atctgattga gacagagtac cctcttcagc tgattcagaa 7380
   gatcatcagc aacatgaatg atgtggttga ccaggcaggc tgttgtagtc acgtccttca 7440
   cttcaagttc attcttcatc tgcttctgtt ttactttgac aggcaaatga agacatggta 7500
   cgacttgatg gaggccaaga acgccatttc accccgagac accgaagtgc ctgaaatcct 7560
   ggctgccccc attgataaca tcggaaacta cggtattccg gaaagtgtat atagaacctt 7620
   tccccagctt gtgtctgtgg atatggatgg tgtaatcccc ttaattaact cacctgcagg 7680
   attgagacta tgaatggatt cccgtgcccg tattactcta ctaatttgat cttggaacgc 7740
   gaaaatacgt ttctaggact ccaaagaatc tcaactcttg tccttactaa atatactacc 7800
   catagttgat ggtttacttg aacagagagg acatgttcac ttgacccaaa gtttctcgca 7860
   tctcttggat atttgaacaa cggcgtccac tgaccgtcag ttatccagtc acaaaacccc 7920
   cacattcata cattcccatg tacgtttaca aagttctcaa ttccatcgtg caaatcaaaa 7980
   tcacatctat tcattcatca tatataaacc catcatgtct actaacactc acaactccat 8040
   agaaaacatc gactcagaac acacgctcca tgcggccgct taggcaacgg gcttgatgac 8100
   agcgggagga gtgcccacat tgtttcggtt tcgaaagaac aggacaccct tgccagctcc 8160
   ctcggcacca gcggagggtt caacccactg gcacattcgt gcagatcggt acatggctcg 8220
   aatgaatcct cgaggaccgt cctggacatc agctcgatag tgcttgccca tgataggttt 8280
   gatggcctcg gtagcttcgt ccgcattgta gaagggaatg gaagagacgt agtgatgcag 8340
   gacgtgagtc tcgataatgc cgtggagcag atgacgtcca atgaagccca tctctcggtc 8400
   gatggttgca gcggcacctc gcacaaagtt ccactcgtcg ttggtgtagt ggggaagagt 8460
   aggatctgtg tgctgcagaa aggtaatggc gacgagccag tggttaaccc acaagtaggg 8520
   aacgaagtac cagatggcca tgttgtagaa tccgaacttc tgaacgagaa agtacagagc 8580
   ggtggccata agaccaatgc caatgtcgga gagcacgatg agcttggcgt cgctgttctc 8640
   gtacagagga gatcggggat cgaaatggtt aactccaccg ccaagaccgt tgtgctttcc 8700
   cttgcctcga ccctctcgct gccgctcatg gtagttgtgt ccagtaacgt tggtaatgag 8760
   atagttgggc caaccgacca gttgctgaag cacaagcatg agcagggtga aagcaggagt 8820
   ttcctcggta agatgggcga gttcgtgggt catcttgccg agtcgagtag cttgctgctc 8880
   tcgggttcga ggaacgaaga ccatgtctcg ctccatgttt ccagtggcct tgtgatgctt 8940
   ccggtgggag atttgccagc tgaagtaggg aacaagcagg gaagagtgaa gcacccagcc 9000
   agtaatgtcg ttgatgattc gggaatcgga gaaagcacca tgtccacact cgtgggcaat 9060
   gacccacagt ccagtaccga agagtccctg aagaacggtg tacacagccc acagaccggc 9120
   tcgagcagga gtggagggaa tgtactcggg tgtcacaaag ttgtaccaga tgctgaaagt 9180
   ggtagtcagg aggacaatgt ctcgaagaat gtagccgtat cccttgagag cagatcgctt 9240
   gaagcagtgc ttgggaatag cgttgtagat gtccttgatg gtgaagtcgg gaacttcgaa 9300
   ctggttgccg taggtatcca gcatgacacc gtactcggac ttgggcttgg caatgtccac 9360
   ctcggacatg gaagacagcg atgtagagga ggccgagtgt ctgggagaat cggagggaga 9420
   gacggcagca gactccgagt cggtcacagt ggtggaagtg acggttcgtc ggagggcagg 9480
   gttctgcttg ggcagagccg aggtggaggc catggccatt gctgtagata tgtcttgtgt 9540
   gtaagggggt tggggtggtt gtttgtgttc ttgacttttg tgttagcaag ggaagacggg 9600
   caaaaaagtg agtgtggttg ggagggagag acgagcctta tatataatgc ttgtttgtgt 9660
   ttgtgcaagt ggacgccgaa acgggcagga gccaaactaa acaaggcaga caatgcgagc 9720
   ttaattggat tgcctgatgg gcaggggtta gggctcgatc aatgggggtg cgaagtgaca 9780
   aaattgggaa ttaggttcgc aagcaaggct gacaagactt tggcccaaac atttgtacgc 9840
   ggtggacaac aggagccacc catcgtctgt cacgggctag ccggtcgtgc gtcctgtcag 9900
   gctccaccta ggctccatgc cactccatac aatcccacta gtgtaccgct aggccgcttt 9960
   tagctcccat ctaagacccc cccaaaacct ccactgtaca gtgcactgta ctgtgtggcg 10020
   atcaagggca agggaaaaaa ggcgcaaaca tgcacgcatg gaatgacgta ggtaaggcgt 10080
   tactagactg aaaagtggca catttcggcg tgccaaaggg tcctaggtgc gtttcgcgag 10140
   ctgggcgcca ggccaagccg ctccaaaacg cctctccgac tccctccagc ggcctccata 10200
   tccccatccc tctccacagc aatgttgtta agccttgcaa acgaaaaaat agaaaggcta 10260
   ataagcttcc aatattgtgg tgtacgctgc ataacgcaac aatgagcgcc aaacaacaca 10320
   cacacacagc acacagcagc attaaccacg atgaacagca tgaattctct ctcttgagct 10380
   tttccataac aagttcttct gcctccagga agtccatggg tggtttgatc atggttttgg 10440
   tgtagtggta gtgcagtggt ggtattgtga ctggggatgt agttgagaat aagtcataca 10500
   caagtcagct ttcttcgagc ctcatataag tataagtagt tcaacgtatt agcactgtac 10560
   ccagcatctc cgtatcgaga aacacaacaa catgccccat tggacagatc atgcggatac 10620
   acaggttgtg cagtatcata catactcgat cagacaggtc gtctgaccat catacaagct 10680
   gaacaagcgc tccatacttg cacgctctct atatacacag ttaaattaca tatccatagt 10740
   ctaacctcta acagttaatc ttctggtaag cctcccagcc agccttctgg tatcgcttgg 10800
   cctcctcaat aggatctcgg ttctggccgt acagacctcg gccgacaatt atgatatccg 10860
   ttccggtaga catgacatcc tcaacagttc ggtactgctg tccgagagcg tctcccttgt 10920
   cgtcaagacc caccccgggg gtcagaataa gccagtcctc agagtcgccc ttaggtcggt 10980
   tctgggcaat gaagccaacc acaaactcgg ggtcggatcg ggcaagctca atggtctgct 11040
   tggagtactc gccagtggcc agagagccct tgcaagacag ctcggccagc atgagcagac 11100
   ctctggccag cttctcgttg ggagagggga ctaggaactc cttgtactgg gagttctcgt 11160
   agtcagagac gtcctccttc ttctgttcag agacagtttc ctcggcacca gctcgcaggc 11220
   cagcaatgat tccggttccg ggtacaccgt gggcgttggt gatatcggac cactcggcga 11280
   ttcggtgaca ccggtactgg tgcttgacag tgttgccaat atctgcgaac tttctgtcct 11340
   cgaacaggaa gaaaccgtgc ttaagagcaa gttccttgag ggggagcaca gtgccggcgt 11400
   aggtgaagtc gtcaatgatg tcgatatggg ttttgatcat gcacacataa ggtccgacct 11460
   tatcggcaag ctcaatgagc tccttggtgg tggtaacatc cagagaagca cacaggttgg 11520
   ttttcttggc tgccacgagc ttgagcactc gagcggcaaa ggcggacttg tggacgttag 11580
   ctcgagcttc gtaggagggc attttggtgg tgaagaggag actgaaataa atttagtctg 11640
   cagaactttt tatcggaacc ttatctgggg cagtgaagta tatgttatgg taatagttac 11700
   gagttagttg aacttataga tagactggac tatacggcta tcggtccaaa ttagaaagaa 11760
   cgtcaatggc tctctgggcg tcgcctttgc cgacaaaaat gtgatcatga tgaaagccag 11820
   caatgacgtt gcagctgata ttgttgtcgg ccaaccgcgc cgaaaacgca gctgtcagac 11880
   ccacagcctc caacgaagaa tgtatcgtca aagtgatcca agcacactca tagttggagt 11940
   cgtactccaa aggcggcaat gacgagtcag acagatactc gtcgaccatt aattctcacg 12000
   tgacacagat tattaacgtc tcgtaccaac cacagattac gacccattcg cagtcacagt 12060
   tcactagggt ttgggttgca tccgttgaga gcggtttgtt tttaaccttc tccatgtgct 12120
   cactcaggtt ttgggttcag atcaaatcaa ggcgtgaacc actttgtttg aggacaaatg 12180
   tgacacaacc aaccagtgtc aggggcaagt ccgtgacaaa ggggaagata caatgcaatt 12240
   actgacagtt acagactgcc tcgatgccct aaccttgccc caaaataaga caactgtcct 12300
   cgtttaagcg caaccctatt cagcgtcacg tcataatagc gtttggatag cactagtcta 12360
   tgaggagcgt tttatgttgc ggtgagggcg attggtgctc atatgggttc aattgaggtg 12420
   gcggaacgag cttagtcttc aattgaggtg cgagcgacac aattgggtgt cacgtggcct 12480
   aattgacctc gggtcgtgga gtccccagtt atacagcaac cacgaggtgc atgggtagga 12540
   gacgtcacca gacaataggg ttttttttgg actggagagg gttgggcaaa agcgctcaac 12600
   gggctgtttg gggagctgtg ggggaggaat tggcgatatt tgtgaggtta acggctccga 12660
   tttgcgtgtt ttgtcgctcc tgcatctccc catacccata tcttccctcc ccacctcttt 12720
   ccacgataat tttacggatc agcaataagg ttccttctcc tagtttccac gtccatatat 12780
   atctatgctg cgtcgtcctt ttcgtgacat caccaaaaca catacaacca tggctctctc 12840
   ccttactacc gagcagctgc tcgagcgacc cgacctggtt gccatcgacg gcattctcta 12900
   cgatctggaa ggtcttgcca aggtccatcc cggatccgac ttgatcctcg cttctggtgc 12960
   ctccgatgct tctcctctgt tctactccat gcacccttac gtcaagcccg agaactcgaa 13020
   gctgcttcaa cagttcgtgc gaggcaagca cgaccgaacc tccaaggaca ttgtctacac 13080
   ctacgactct ccctttgcac aggacgtcaa gcgaactatg cgagaggtca tgaaaggtcg 13140
   gaactggtat gccacacctg gattctggct gcgaaccgtt ggcatcattg ctgtcaccgc 13200
   cttttgcgag tggcactggg ctactaccgg aatggtgctg tggggtctct tgactggatt 13260
   catgcacatg cagatcggcc tgtccattca gcacgatgcc tctcatggtg ccatcagcaa 13320
   aaagccctgg gtcaacgctc tctttgccta cggcatcgac gtcattggat cgtccagatg 13380
   gatctggctg cagtctcaca tcatgcgaca tcacacctac accaatcagc atggtctcga 13440
   cctggatgcc gagtccgcag aaccattcct tgtgttccac aactaccctg ctgccaacac 13500
   tgctcgaaag tggtttcacc gattccaggc ctggtacatg tacctcgtgc ttggagccta 13560
   cggcgtttcg ctggtgtaca accctctcta catcttccga atgcagcaca acgacaccat 13620
   tcccgagtct gtcacagcca tgcgagagaa cggctttctg cgacggtacc gaacccttgc 13680
   attcgttatg cgagctttct tcatctttcg aaccgccttc ttgccctggt atctcactgg 13740
   aacctccctg ctcatcacca ttcctctggt gcccactgct accggtgcct tcctcacctt 13800
   ctttttcatc ttgtctcaca acttcgatgg ctcggagcga atccccgaca agaactgcaa 13860
   ggtcaagagc tccgagaagg acgttgaagc cgatcagatc gactggtaca gagctcaggt 13920
   ggagacctct tccacctacg gtggacccat tgccatgttc tttactggcg gtctcaactt 13980
   ccagatcgag catcacctct ttcctcgaat gtcgtcttgg cactatccct tcgtgcagca 14040
   agctgtccga gagtgttgcg aacgacacgg agttcggtac gtcttctacc ctaccattgt 14100
   gggcaacatc atttccaccc tcaagtacat gcacaaagtc ggtgtggttc actgtgtcaa 14160
   ggacgctcag gattcctaag cggccgcatg tacatacaag attatttata gaaatgaatc 14220
   gcgatcgaac aaagagtacg agtgtacgag taggggatga tgataaaagt ggaagaagtt 14280
   ccgcatcttt ggatttatca acgtgtagga cgatacttcc tgtaaaaatg caatgtcttt 14340
   accataggtt ctgctgtaga tgttattaac taccattaac atgtctactt gtacagttgc 14400
   agaccagttg gagtatagaa tggtacactt accaaaaagt gttgatggtt gtaactacga 14460
   tatataaaac tgttgacggg atccccgctg atatgcctaa ggaacaatca aagaggaaga 14520
   tattaattca gaatgctagt atacagttag ggat 14554
<210> 133
   <211> 15119
   <212> DNA
   <213> Artificial Sequence
<220>
   <223> Plasmid pZSCP-Ma83
<400> 133
   gtacgacccc tctcaggcca agcagaaggc tgagtccatc aagaaggcca acgctatcat      60
   tgtcttcaac ctcaagaaca aggctggcaa gaccgagtct tggtaccttg acctcaagaa     120
   cgacggtgac gtcggcaagg gcaacaagtc ccccaagggt gatgctgaca tccagctcac     180
   tctctctgac gaccacttcc agcagctcgt tgagggtaag gctaacgccc agcgactctt     240
   catgaccggc aagctcaagg ttaagggcaa cgtcatgaag gctgccgcca ttgagggtat     300
   cctcaagaac gctcagaaca acctctaagc gcatcattta ttgattaatt gatgatttac     360
   tatattgatt tcgcaactgt agtgtgattg tatgtgatct ggctcgtagg cttcagtaaa     420
   tactagacgg gtatcctacg tagttgtatc atacatcgag cctgtggtta cttgtacaat     480
   aattcgtaat gtagagatac cccttgatcc attgcctgtt tctaacatac aatgatctcc     540
   acgcaataat cccactcttg actaaaagtt gctactcttg cacggttacc tcggcatagt     600
   cacgcctctc ttgtctcgtc tcgaacgcac aaagtcaatt gacaacgcca ctcactcgag     660
   tgtgccccaa cagggcacca tatcgactaa tttgaggcca actagggtga ttttggatgg     720
   aatttgatcg gaaaaaatag ctgcagaaat tcctggagag aaaaattgac cgcatccaca     780
   tggtttgacc aaaaaatcgt ctccatctct gtgctcaact ctcctgacga gatatgcgcg     840
   cgcaccccca catgatgtga ttgatctcaa caaacttcac ccagaccctt atctttccgg     900
   gaaacttact gtataagtgg tcgtgcgaac agaaagtgtg cgcactttag gtgtctagat     960
   ccgattgttc tcgttctgat aatgagccag ccccgcgagg caatgttttt tacaattgaa    1020
   aacttcgtta accactcaca ttaccgtttt tgccccatat ttaccctctg gtacactccc    1080
   tcttgcatac acacacactg cagtgaaaat gcactccgtt agcaccgttg tgattggttc    1140
   agggcacgag tttggtggtt taaggcgcaa ctacatcaat atgaaaacag gagacgctga    1200
   aaaggggtaa tatcggactg ctgctatgtt gtatgtactg catgacgaat tggtgttatt    1260
   caagaccgtg gcacaggttg ctgcggtacg agacctggta gcttctctaa acggcatgtc    1320
   taggtggcgc gccagctgca ttaatgaatc ggccaacgcg cggggagagg cggtttgcgt    1380
   attgggcgct cttccgcttc ctcgctcact gactcgctgc gctcggtcgt tcggctgcgg    1440
   cgagcggtat cagctcactc aaaggcggta atacggttat ccacagaatc aggggataac    1500
   gcaggaaaga acatgtgagc aaaaggccag caaaaggcca ggaaccgtaa aaaggccgcg    1560
   ttgctggcgt ttttccatag gctccgcccc cctgacgagc atcacaaaaa tcgacgctca 1620
   agtcagaggt ggcgaaaccc gacaggacta taaagatacc aggcgtttcc ccctggaagc 1680
   tccctcgtgc gctctcctgt tccgaccctg ccgcttaccg gatacctgtc cgcctttctc 1740
   ccttcgggaa gcgtggcgct ttctcatagc tcacgctgta ggtatctcag ttcggtgtag 1800
   gtcgttcgct ccaagctggg ctgtgtgcac gaaccccccg ttcagcccga ccgctgcgcc 1860
   ttatccggta actatcgtct tgagtccaac ccggtaagac acgacttatc gccactggca 1920
   gcagccactg gtaacaggat tagcagagcg aggtatgtag gcggtgctac agagttcttg 1980
   aagtggtggc ctaactacgg ctacactaga agaacagtat ttggtatctg cgctctgctg 2040
   aagccagtta ccttcggaaa aagagttggt agctcttgat ccggcaaaca aaccaccgct 2100
   ggtagcggtg gtttttttgt ttgcaagcag cagattacgc gcagaaaaaa aggatctcaa 2160
   gaagatcctt tgatcttttc tacggggtct gacgctcagt ggaacgaaaa ctcacgttaa 2220
   gggattttgg tcatgagatt atcaaaaagg atcttcacct agatcctttt aaattaaaaa 2280
   tgaagtttta aatcaatcta aagtatatat gagtaaactt ggtctgacag ttaccaatgc 2340
   ttaatcagtg aggcacctat ctcagcgatc tgtctatttc gttcatccat agttgcctga 2400
   ctccccgtcg tgtagataac tacgatacgg gagggcttac catctggccc cagtgctgca 2460
   atgataccgc gagacccacg ctcaccggct ccagatttat cagcaataaa ccagccagcc 2520
   ggaagggccg agcgcagaag tggtcctgca actttatccg cctccatcca gtctattaat 2580
   tgttgccggg aagctagagt aagtagttcg ccagttaata gtttgcgcaa cgttgttgcc 2640
   attgctacag gcatcgtggt gtcacgctcg tcgtttggta tggcttcatt cagctccggt 2700
   tcccaacgat caaggcgagt tacatgatcc cccatgttgt gcaaaaaagc ggttagctcc 2760
   ttcggtcctc cgatcgttgt cagaagtaag ttggccgcag tgttatcact catggttatg 2820
   gcagcactgc ataattctct tactgtcatg ccatccgtaa gatgcttttc tgtgactggt 2880
   gagtactcaa ccaagtcatt ctgagaatag tgtatgcggc gaccgagttg ctcttgcccg 2940
   gcgtcaatac gggataatac cgcgccacat agcagaactt taaaagtgct catcattgga 3000
   aaacgttctt cggggcgaaa actctcaagg atcttaccgc tgttgagatc cagttcgatg 3060
   taacccactc gtgcacccaa ctgatcttca gcatctttta ctttcaccag cgtttctggg 3120
   tgagcaaaaa caggaaggca aaatgccgca aaaaagggaa taagggcgac acggaaatgt 3180
   tgaatactca tactcttcct ttttcaatat tattgaagca tttatcaggg ttattgtctc 3240
   atgagcggat acatatttga atgtatttag aaaaataaac aaataggggt tccgcgcaca 3300
   tttccccgaa aagtgccacc tgatgcggtg tgaaataccg cacagatgcg taaggagaaa 3360
   ataccgcatc aggaaattgt aagcgttaat attttgttaa aattcgcgtt aaatttttgt 3420
   taaatcagct cattttttaa ccaataggcc gaaatcggca aaatccctta taaatcaaaa 3480
   gaatagaccg agatagggtt gagtgttgtt ccagtttgga acaagagtcc actattaaag 3540
   aacgtggact ccaacgtcaa agggcgaaaa accgtctatc agggcgatgg cccactacgt 3600
   gaaccatcac cctaatcaag ttttttgggg tcgaggtgcc gtaaagcact aaatcggaac 3660
   cctaaaggga gcccccgatt tagagcttga cggggaaagc cggcgaacgt ggcgagaaag 3720
   gaagggaaga aagcgaaagg agcgggcgct agggcgctgg caagtgtagc ggtcacgctg 3780
   cgcgtaacca ccacacccgc cgcgcttaat gcgccgctac agggcgcgtc cattcgccat 3840
   tcaggctgcg caactgttgg gaagggcgat cggtgcgggc ctcttcgcta ttacgccagc 3900
   tggcgaaagg gggatgtgct gcaaggcgat taagttgggt aacgccaggg ttttcccagt 3960
   cacgacgttg taaaacgacg gccagtgaat tgtaatacga ctcactatag ggcgaattgg 4020
   gcccgacgtc gcatgcgtca ctaatcaagg atacctacca tgccactatg atgtttgcag 4080
   gaggtgtacc tcggcagtca tcaaaaaatg gaactactgg ctttagatct tgttgtatgg 4140
   catcgcgcct aaaaaagaaa cccccttcca gcgagctact acaagtagtt gtagttgcgg 4200
   gcgttggata ccgaaagtca caagcacatg tcgaagctct catctgaaac accgacagtc 4260
   gtctgcaccc cgcaagtctc ggttcgtacc agcaccaatg ttaggcagaa ctatacacaa 4320
   gagggcggac gatcacttcg gcgttaggca actgaaggct attttcggct ggtactgtag 4380
   gggacagagg aaacgcaagt gattagtaaa tcggataata ggcctgttag tttaccgaaa 4440
   tggtggggga ggggttccgt ggatatcttg aagttatgga ggctgatcgt tatttgtggg 4500
   gatggatatc attgtatgga catactgtag ctactgtata aacaacggat cttacacctg 4560
   cctcttgtat gcccattgct tgatcatcta tcgtgttact gtacatatac aatagatata 4620
   gggaagaaaa gccggaagta gagaccatag tctggcagaa gtaacggcct cgggtcgaga 4680
   gaactataac aaagtccaac ggcgggtctt agaatagccc caaggatcac acagttccgc 4740
   aatccagttt cacatgttcc gttgcatgga cttttgcatg tctactgttg ctacgattcc 4800
   cccattgcaa ccacagtttg gggttacccc gcattatatt agcatgatta cgaaagagat 4860
   aagtatcata tggaacatgt gaagggtagt atgcaggtcc ggcggagaaa gagaatgacg 4920
   ttttcattaa gcgattcgct tggcggcttg tgggggatgt gacgatactt acggtaaaga 4980
   ccctgtgtga gagctggtac tcgctcgtta cttcgctgat ctgttgggcc gtcaatcgaa 5040
   tctcgtggaa cttgcattct tcttaactgt gtctatacaa gacacctaat gaaacataca 5100
   agctaccgaa atcattttac tcgtactgac cggtacggta cttgcacaag tagtgaaact 5160
   tccgaaaata gccagcctca tgcatcatcg cttcacccct tctgttgacc tcaaaagcat 5220
   tccaacggta aaaaattata acgccgccaa ctggatggtt gtgacggcgt tgaccaccaa 5280
   tgtgtggggg ctggcggtag gaccgagctt attcgtccca ataagctctt tggatttgat 5340
   tctttggggt gtgtggtaaa attcacatgg ggaagaacac ggtggcagtt tgaggcagag 5400
   gcccagcgtg tagttcctag ggcatgaata taccgaactc atggcgcaga attgagctga 5460
   atgcgcaaaa agctacagga tcaaccgcgt tagaaatgcc gcaaatgtcc actaattccc 5520
   cggactgttc caaatgattc tgtggggata aatctcaaac tgggttaggc tttgtcacgt 5580
   ttctttgtgt cgtgtcggtt cgtccggggc aatgtgccca cgcttggctg tctccctaca 5640
   cctcggtaaa aactatcaca tgctgcccct ctcgagcaag cattaaatgc atatagtcaa 5700
   tctaacgaca tatatatagg tagggtgcat cctccggttt agctccccag aatatctctt 5760
   attcattaca caaaaacaac aatgtctctc aaggtcgacg gcttcacttc ttaattaagt 5820
   tgcgacacat gtcttgatag tatcttgaat tctctctctt gagcttttcc ataacaagtt 5880
   cttctgcctc caggaagtcc atgggtggtt tgatcatggt tttggtgtag tggtagtgca 5940
   gtggtggtat tgtgactggg gatgtagttg agaataagtc atacacaagt cagctttctt 6000
   cgagcctcat ataagtataa gtagttcaac gtattagcac tgtacccagc atctccgtat 6060
   cgagaaacac aacaacatgc cccattggac agatcatgcg gatacacagg ttgtgcagta 6120
   tcatacatac tcgatcagac aggtcgtctg accatcatac aagctgaaca agcgctccat 6180
   acttgcacgc tctctatata cacagttaaa ttacatatcc atagtctaac ctctaacagt 6240
   taatcttctg gtaagcctcc cagccagcct tctggtatcg cttggcctcc tcaataggat 6300
   ctcggttctg gccgtacaga cctcggccga caattatgat atccgttccg gtagacatga 6360
   catcctcaac agttcggtac tgctgtccga gagcgtctcc cttgtcgtca agacccaccc 6420
   cgggggtcag aataagccag tcctcagagt cgcccttagg tcggttctgg gcaatgaagc 6480
   caaccacaaa ctcggggtcg gatcgggcaa gctcaatggt ctgcttggag tactcgccag 6540
   tggccagaga gcccttgcaa gacagctcgg ccagcatgag cagacctctg gccagcttct 6600
   cgttgggaga ggggactagg aactccttgt actgggagtt ctcgtagtca gagacgtcct 6660
   ccttcttctg ttcagagaca gtttcctcgg caccagctcg caggccagca atgattccgg 6720
   ttccgggtac accgtgggcg ttggtgatat cggaccactc ggcgattcgg tgacaccggt 6780
   actggtgctt gacagtgttg ccaatatctg cgaactttct gtcctcgaac aggaagaaac 6840
   cgtgcttaag agcaagttcc ttgaggggga gcacagtgcc ggcgtaggtg aagtcgtcaa 6900
   tgatgtcgat atgggttttg atcatgcaca cataaggtcc gaccttatcg gcaagctcaa 6960
   tgagctcctt ggtggtggta acatccagag aagcacacag gttggttttc ttggctgcca 7020
   cgagcttgag cactcgagcg gcaaaggcgg acttgtggac gttagctcga gcttcgtagg 7080
   agggcatttt ggtggtgaag aggagactga aataaattta gtctgcagaa ctttttatcg 7140
   gaaccttatc tggggcagtg aagtatatgt tatggtaata gttacgagtt agttgaactt 7200
   atagatagac tggactatac ggctatcggt ccaaattaga aagaacgtca atggctctct 7260
   gggcgtcgcc tttgccgaca aaaatgtgat catgatgaaa gccagcaatg acgttgcagc 7320
   tgatattgtt gtcggccaac cgcgccgaaa acgcagctgt cagacccaca gcctccaacg 7380
   aagaatgtat cgtcaaagtg atccaagcac actcatagtt ggagtcgtac tccaaaggcg 7440
   gcaatgacga gtcagacaga tactcgtcga ccttttcctt gggaaccacc accgtcagcc 7500
   cttctgactc acgtattgta gccaccgaca caggcaacag tccgtggata gcagaatatg 7560
   tcttgtcggt ccatttctca ccaactttag gcgtcaagtg aatgttgcag aagaagtatg 7620
   tgccttcatt gagaatcggt gttgctgatt tcaataaagt cttgagatca gtttggccag 7680
   tcatgttgtg gggggtaatt ggattgagtt atcgcctaca gtctgtacag gtatactcgc 7740
   tgcccacttt atactttttg attccgctgc acttgaagca atgtcgttta ccaaaagtga 7800
   gaatgctcca cagaacacac cccagggtat ggttgagcaa aaaataaaca ctccgatacg 7860
   gggaatcgaa ccccggtctc cacggttctc aagaagtatt cttgatgaga gcgtatcgat 7920
   ggaagccggt agaaccgggc tgcttgtgct tggagatgga agccggtaga accgggctgc 7980
   ttggggggat ttggggccgc tgggctccaa agaggggtag gcatttcgtt ggggttacgt 8040
   aattgcggca tttgggtcct gcgcgcatgt cccattggtc agaattagtc cggataggag 8100
   acttatcagc caatcacagc gccggatcca cctgtaggtt gggttgggtg ggagcacccc 8160
   tccacagagt agagtcaaac agcagcagca acatgatagt tgggggtgtg cgtgttaaag 8220
   gaaaaaaaag aagcttgggt tatattcccg ctctatttag aggttgcggg atagacgccg 8280
   acggagggca atggcgctat ggaaccttgc ggatatccat acgccgcggc ggactgcgtc 8340
   cgaaccagct ccagcagcgt tttttccggg ccattgagcc gactgcgacc ccgccaacgt 8400
   gtcttggccc acgcactcat gtcatgttgg tgttgggagg ccacttttta agtagcacaa 8460
   ggcacctagc tcgcagcaag gtgtccgaac caaagaagcg gctgcagtgg tgcaaacggg 8520
   gcggaaacgg cgggaaaaag ccacgggggc acgaattgag gcacgccctc gaatttgaga 8580
   cgagtcacgg ccccattcgc ccgcgcaatg gctcgccaac gcccggtctt ttgcaccaca 8640
   tcaggttacc ccaagccaaa cctttgtgtt aaaaagctta acatattata ccgaacgtag 8700
   gtttgggcgg gcttgctccg tctgtccaag gcaacattta tataagggtc tgcatcgccg 8760
   gctcaattga atcttttttc ttcttctctt ctctatattc attcttgaat taaacacaca 8820
   tcaaccatgg tcaagcgacc cgctctgcct ctcaccgtgg acggtgtcac ctacgacgtt 8880
   tctgcctggc tcaaccacca tcccggaggt gccgacatta tcgagaacta ccgaggtcgg 8940
   gatgctaccg acgtcttcat ggttatgcac tccgagaacg ccgtgtccaa actcagacga 9000
   atgcccatca tggaaccttc ctctcccctg actccaacac ctcccaagcc aaactccgac 9060
   gaacctcagg aggatttccg aaagctgcga gacgagctca ttgctgcagg catgttcgat 9120
   gcctctccca tgtggtacgc ttacaagacc ctgtcgactc tcggactggg tgtccttgcc 9180
   gtgctgttga tgacccagtg gcactggtac ctggttggtg ctatcgtcct cggcattcac 9240
   tttcaacaga tgggatggct ctcgcacgac atttgccatc accagctgtt caaggaccga 9300
   tccatcaaca atgccattgg cctgctcttc ggaaacgtgc ttcagggctt ttctgtcact 9360
   tggtggaagg accgacacaa cgctcatcac tccgccacca acgtgcaggg tcacgatccc 9420
   gacatcgaca acctgcctct cctggcgtgg tccaaggagg acgtcgagcg agctggcccg 9480
   ttttctcgac ggatgatcaa gtaccaacag tattacttct ttttcatctg tgcccttctg 9540
   cgattcatct ggtgctttca gtccattcat actgccacgg gtctcaagga tcgaagcaat 9600
   cagtactatc gaagacagta cgagaaggag tccgtcggtc tggcactcca ctggggtctc 9660
   aaggccttgt tctactattt ctacatgccc tcgtttctca ccggactcat ggtgttcttt 9720
   gtctccgagc tgcttggtgg cttcggaatt gccatcgttg tcttcatgaa ccactaccct 9780
   ctggagaaga ttcaggactc cgtgtgggat ggtcatggct tctgtgctgg acagattcac 9840
   gagaccatga acgttcagcg aggcctcgtc acagactggt ttttcggtgg cctcaactac 9900
   cagatcgaac atcacctgtg gcctactctt cccagacaca acctcaccgc tgcctccatc 9960
   aaagtggagc agctgtgcaa gaagcacaac ctgccctacc gatctcctcc catgctcgaa 10020
   ggtgtcggca ttcttatctc ctacctgggc accttcgctc gaatggttgc caaggcagac 10080
   aaggcctaag cggccgcatt gatgattgga aacacacaca tgggttatat ctaggtgaga 10140
   gttagttgga cagttatata ttaaatcagc tatgccaacg gtaacttcat tcatgtcaac 10200
   gaggaaccag tgactgcaag taatatagaa tttgaccacc ttgccattct cttgcactcc 10260
   tttactatat ctcatttatt tcttatatac aaatcacttc ttcttcccag catcgagctc 10320
   ggaaacctca tgagcaataa catcgtggat ctcgtcaata gagggctttt tggactcctt 10380
   gctgttggcc accttgtcct tgctgtttaa acagagtgtg aaagactcac tatggtccgg 10440
   gcttatctcg accaatagcc aaagtctgga gtttctgaga gaaaaaggca agatacgtat 10500
   gtaacaaagc gacgcatggt acaataatac cggaggcatg tatcatagag agttagtggt 10560
   tcgatgatgg cactggtgcc tggtatgact ttatacggct gactacatat ttgtcctcag 10620
   acatacaatt acagtcaagc acttaccctt ggacatctgt aggtaccccc cggccaagac 10680
   gatctcagcg tgtcgtatgt cggattggcg tagctccctc gctcgtcaat tggctcccat 10740
   ctactttctt ctgcttggct acacccagca tgtctgctat ggctcgtttt cgtgccttat 10800
   ctatcctccc agtattacca actctaaatg acatgatgtg attgggtcta cactttcata 10860
   tcagagataa ggagtagcac agttgcataa aaagcccaac tctaatcagc ttcttccttt 10920
   cttgtaatta gtacaaaggt gattagcgaa atctggaagc ttagttggcc ctaaaaaaat 10980
   caaaaaaagc aaaaaacgaa aaacgaaaaa ccacagtttt gagaacaggg aggtaacgaa 11040
   ggatcgtata tatatatata tatatatata cccacggatc ccgagaccgg cctttgattc 11100
   ttccctacaa ccaaccattc tcaccaccct aattcacaac catggtctcc aaccacctgt 11160
   tcgacgccat gcgagctgcc gctcccggag acgcaccttt cattcgaatc gacaacgctc 11220
   ggacctggac ttacgatgac gccattgctc tttccggtcg aatcgctgga gctatggacg 11280
   cactcggcat tcgacccgga gacagagttg ccgtgcaggt cgagaagtct gccgaggcgt 11340
   tgattctcta cctggcctgt cttcgaaccg gagctgtcta cctgcctctc aacactgcct 11400
   acaccctggc cgagctcgac tacttcatcg gcgatgccga accgcgtctg gtggtcgttg 11460
   ctcccgcagc tcgaggtggc gtggagacaa ttgccaagcg acacggtgct atcgtcgaaa 11520
   ccctcgacgc cgatggacga ggctccttgc tggaccttgc tagagatgag cctgccgact 11580
   ttgtcgatgc ttcgcgatct gccgacgatc tggctgctat tctctacact tccggtacaa 11640
   ccggacgatc gaagggtgcc atgcttactc atggcaatct gctctccaac gctctcacct 11700
   tgcgagacta ttggagagtt accgcagacg atcgactcat ccatgccttg ccaatctttc 11760
   acactcatgg tctgttcgtt gctacgaacg tcacactgct tgcaggagcc tcgatgtttc 11820
   tgctctccaa gttcgatgcc gacgaggtcg tttctctcat gccacaggcc accatgctta 11880
   tgggcgtgcc cacattctac gttcgattgc tgcagagtcc tcgactcgag aagggtgctg 11940
   tggccagcat cagactgttc atttctggat cagctccctt gcttgccgaa acccacgccg 12000
   agtttcatgc tcgtactggt cacgccattc tcgagcgata cggcatgacg gaaaccaaca 12060
   tgaatacttc caacccctac gagggcaagc gtattgccgg aaccgttggt tttcctctgc 12120
   ccgacgtcac tgtgcgagtc accgatcccg ccaccggtct cgttcttcca cctgaagaga 12180
   ctggcatgat cgagatcaag ggacccaacg tcttcaaggg ctattggcga atgcccgaaa 12240
   agaccgctgc cgagtttacc gcagacggtt tctttatctc tggagatctc ggcaagatcg 12300
   accgagaagg ttacgttcac attgtgggac gaggcaagga cctggtcatt tccggtggct 12360
   acaacatcta tcccaaagag gtcgaaggcg agatcgacca gatcgagggt gtggtcgagt 12420
   ctgctgtcat tggtgttcct catcccgatt tcggagaagg tgtcaccgct gttgtcgtgt 12480
   gcaaacctgg tgccgttctc gacgaaaaga ccatcgtgtc tgctctgcag gaccgtcttg 12540
   cccgatacaa gcaacccaag cggattatct ttgccgacga tctgcctcga aacactatgg 12600
   gaaaggttca gaagaacatt cttcgacagc aatacgccga tctctacacc agacgataag 12660
   cggccgcatg agaagataaa tatataaata cattgagata ttaaatgcgc tagattagag 12720
   agcctcatac tgctcggaga gaagccaaga cgagtactca aaggggatta caccatccat 12780
   atccacagac acaagctggg gaaaggttct atatacactt tccggaatac cgtagtttcc 12840
   gatgttatca atgggggcag ccaggatttc aggcacttcg gtgtctcggg gtgaaatggc 12900
   gttcttggcc tccatcaagt cgtaccatgt cttcatttgc ctgtcaaagt aaaacagaag 12960
   cagatgaaga atgaacttga agtgaaggaa tttaaatgat gtcgacgcag taggatgtcc 13020
   tgcacgggtc tttttgtggg gtgtggagaa aggggtgctt ggagatggaa gccggtagaa 13080
   ccgggctgct tgtgcttgga gatggaagcc ggtagaaccg ggctgcttgg ggggatttgg 13140
   ggccgctggg ctccaaagag gggtaggcat ttcgttgggg ttacgtaatt gcggcatttg 13200
   ggtcctgcgc gcatgtccca ttggtcagaa ttagtccgga taggagactt atcagccaat 13260
   cacagcgccg gatccacctg taggttgggt tgggtgggag cacccctcca cagagtagag 13320
   tcaaacagca gcagcaacat gatagttggg ggtgtgcgtg ttaaaggaaa aaaaagaagc 13380
   ttgggttata ttcccgctct atttagaggt tgcgggatag acgccgacgg agggcaatgg 13440
   cgctatggaa ccttgcggat atccatacgc cgcggcggac tgcgtccgaa ccagctccag 13500
   cagcgttttt tccgggccat tgagccgact gcgaccccgc caacgtgtct tggcccacgc 13560
   actcatgtca tgttggtgtt gggaggccac tttttaagta gcacaaggca cctagctcgc 13620
   agcaaggtgt ccgaaccaaa gaagcggctg cagtggtgca aacggggcgg aaacggcggg 13680
   aaaaagccac gggggcacga attgaggcac gccctcgaat ttgagacgag tcacggcccc 13740
   attcgcccgc gcaatggctc gccaacgccc ggtcttttgc accacatcag gttaccccaa 13800
   gccaaacctt tgtgttaaaa agcttaacat attataccga acgtaggttt gggcgggctt 13860
   gctccgtctg tccaaggcaa catttatata agggtctgca tcgccggctc aattgaatct 13920
   tttttcttct tctcttctct atattcattc ttgaattaaa cacacatcaa ccatggagtc 13980
   tggacccatg cctgctggca ttcccttccc tgagtactat gacttcttta tggactggaa 14040
   gactcccctg gccatcgctg ccacctacac tgctgccgtc ggtctcttca accccaaggt 14100
   tggcaaggtc tcccgagtgg ttgccaagtc ggctaacgca aagcctgccg agcgaaccca 14160
   gtccggagct gccatgactg ccttcgtctt tgtgcacaac ctcattctgt gtgtctactc 14220
   tggcatcacc ttctactaca tgtttcctgc tatggtcaag aacttccgaa cccacacact 14280
   gcacgaagcc tactgcgaca cggatcagtc cctctggaac aacgcacttg gctactgggg 14340
   ttacctcttc tacctgtcca agttctacga ggtcattgac accatcatca tcatcctgaa 14400
   gggacgacgg tcctcgctgc ttcagaccta ccaccatgct ggagccatga ttaccatgtg 14460
   gtctggcatc aactaccaag ccactcccat ttggatcttt gtggtcttca actccttcat 14520
   tcacaccatc atgtactgtt actatgcctt cacctctatc ggattccatc ctcctggcaa 14580
   aaagtacctg acttcgatgc agattactca gtttctggtc ggtatcacca ttgccgtgtc 14640
   ctacctcttc gttcctggct gcatccgaac acccggtgct cagatggctg tctggatcaa 14700
   cgtcggctac ctgtttccct tgacctatct gttcgtggac tttgccaagc gaacctactc 14760
   caagcgatct gccattgccg ctcagaaaaa ggctcagtaa gcggccgcaa gtgtggatgg 14820
   ggaagtgagt gcccggttct gtgtgcacaa ttggcaatcc aagatggatg gattcaacac 14880
   agggatatag cgagctacgt ggtggtgcga ggatatagca acggatattt atgtttgaca 14940
   cttgagaatg tacgatacaa gcactgtcca agtacaatac taaacatact gtacatactc 15000
   atactcgtac ccgggcaacg gtttcacttg agtgcagtgg ctagtgctct tactcgtaca 15060
   gtgtgcaata ctgcgtatca tagtctttga tgtatatcgt attcattcat gttagttgc 15119
<210> 134
   <211> 14623
   <212> DNA
   <213> Artificial Sequence
<220>
   <223> Plasmid pZKL4-398F2
<220>
   <221> misc_feature
   <222> (1048)..(1048)
   <223> n is a, c, g, or t
<220>
   <221> misc_feature
   <222> (1053)..(1056)
   <223> n is a, c, g, or t
<400> 134
   taaaaggcgt tgaaacagaa tgagccagac agcaaggaca aggtggccaa cagcaaggag      60
   tccaaaaagc cctctattga cgagatccac gatgttattg ctcatgaggt ttccgagctc     120
   gatgctggga agaagaagtg atttgtatat aagaaataaa tgagatatag taaaggagtg     180
   caagagaatg gcaaggtggt caaattctat attacttgca gtcactggtt cctcgttgac     240
   atgaatgaag ttaccgttgg catagctgat ttaatatata actgtccaac taactctcac     300
   ctagatataa cccatgtgtg tgtttccaat catcaatgcg gccgcgccta cttaagcaac     360
   gggcttgata acagcggggg gggtgcccac gttgttgcgg ttgcggaaga acagaacacc     420
   cttaccagca ccctcggcac cagcgctggg ctcaacccac tggcacatac gcgcactgcg     480
   gtacatggcg cggatgaagc cacgaggacc atcctggaca tcagcccggt agtgcttgcc     540
   catgatgggc ttaatggcct cggtggcctc gtccgcgttg tagaagggga tgctgctgac     600
   gtagtggtgg aggacatgag tctcgatgat gccgtggaga aggtggcggc cgatgaagcc     660
   catctcacgg tcaatggtag cagcggcacc acggacgaag ttccactcgt cgttggtgta     720
   gtggggaagg gtagggtcgg tgtgctggag gaaggtgatg gcaacgagcc agtggttaac     780
   ccagaggtag ggaacaaagt accagatggc catgttgtag aaaccgaact tctgaacgag     840
   gaagtacaga gcagtggcca tcagaccgat accaatatcg ctgaggacga tgagcttagc     900
   gtcactgttc tcgtacagag ggctgcgggg atcgaagtgg ttaacaccac cgccgaggcc     960
   gttatgcttg cccttgccgc gaccctcacg ctggcgctcg tggtagttgt ggccggtaac    1020
   attggtgatg aggtagttgg gccagccnac gannnnctca gtaagatgag cgagctcgtg    1080
   ggtcatcttt ccgagacgag tagcctgctg ctcgcgggtt cggggaacga agaccatgtc    1140
   acgctccatg ttgccagtgg ccttgtggtg ctttcggtgg gagatttgcc agctgaagta    1200
   ggggacaagg agggaagagt gaagaaccca gccagtaatg tcgttgatga tgcgagaatc    1260
   ggagaaagca ccgtgaccgc actcatgggc aataacccag agaccagtac cgaaaagacc    1320
   ctgaagaacg gtgtacacgg cccacagacc agcgcgggcg ggggtggagg ggatatattc    1380
   gggggtcaca aagttgtacc agatgctgaa agtggtagtc aggaggacaa tgtcgcggag    1440
   gatataaccg tatcccttga gagcggagcg cttgaagcag tgcttaggga tggcattgta 1500
   gatgtccttg atggtaaagt cgggaacctc gaactggttg ccgtaggtgt cgagcatgac 1560
   accatactcg gacttgggct tggcgatatc aacctcggac atggacgaga gcgatgtgga 1620
   agaggccgag tggcggggag agtctgaagg agagacggcg gcagactcag aatccgtcac 1680
   agtagttgag gtgacggtgc gtctaagcgc agggttctgc ttgggcagag ccgaagtgga 1740
   cgccatgggc aggacctgtg ttagtacatt gtcggggagt catcaattgg ttcgacaggt 1800
   tgtcgactgt tagtatgagc tcaattgggc tctggtgggt cgatgacact tgtcatctgt 1860
   ttctgttggg tcatgtttcc atcaccttct atggtactca caattcgtcc gattcgcccg 1920
   aatccgttaa taccgacttt gatggccatg ttgatgtgtg tttaattcaa gaatgaatat 1980
   agagaagaga agaagaaaaa agattcaatt gagccggcga tgcagaccct tatataaatg 2040
   ttgccttgga cagacggagc aagcccgccc aaacctacgt tcggtataat atgttaagct 2100
   ttttaacaca aaggtttggc ttggggtaac ctgatgtggt gcaaaagacc gggcgttggc 2160
   gagccattgc gcgggcgaat ggggccgtga ctcgtctcaa attcgagggc gtgcctcaat 2220
   tcgtgccccc gtggcttttt cccgccgttt ccgccccgtt tgcaccactg cagccgcttc 2280
   tttggttcgg acaccttgct gcgagctagg tgccttgtgc tacttaaaaa gtggcctccc 2340
   aacaccaaca tgacatgagt gcgtgggcca agacacgttg gcggggtcgc agtcggctca 2400
   atggcccgga aaaaacgctg ctggagctgg ttcggacgca gtccgccgcg gcgtatggat 2460
   atccgcaagg ttccatagcg ccattgccct ccgtcggcgt ctatcccgca acctctaaat 2520
   agagcgggaa tataacccaa gcttcttttt tttcctttaa cacgcacacc cccaactatc 2580
   atgttgctgc tgctgtttga ctctactctg tggaggggtg ctcccaccca acccaaccta 2640
   caggtggatc cggcgctgtg attggctgat aagtctccta tccggactaa ttctgaccaa 2700
   tgggacatgc gcgcaggacc caaatgccgc aattacgtaa ccccaacgaa atgcctaccc 2760
   ctctttggag cccagcggcc ccaaatcccc ccaagcagcc cggttctacc ggcttccatc 2820
   tccaagcaca agcagcccgg aattctctct cttgagcttt tccataacaa gttcttctgc 2880
   ctccaggaag tccatgggtg gtttgatcat ggttttggtg tagtggtagt gcagtggtgg 2940
   tattgtgact ggggatgtag ttgagaataa gtcatacaca agtcagcttt cttcgagcct 3000
   catataagta taagtagttc aacgtattag cactgtaccc agcatctccg tatcgagaaa 3060
   cacaacaaca tgccccattg gacagatcat gcggatacac aggttgtgca gtatcataca 3120
   tactcgatca gacaggtcgt ctgaccatca tacaagctga acaagcgctc catacttgca 3180
   cgctctctat atacacagtt aaattacata tccatagtct aacctctaac agttaatctt 3240
   ctggtaagcc tcccagccag ccttctggta tcgcttggcc tcctcaatag gatctcggtt 3300
   ctggccgtac agacctcggc cgacaattat gatatccgtt ccggtagaca tgacatcctc 3360
   aacagttcgg tactgctgtc cgagagcgtc tcccttgtcg tcaagaccca ccccgggggt 3420
   cagaataagc cagtcctcag agtcgccctt aggtcggttc tgggcaatga agccaaccac 3480
   aaactcgggg tcggatcggg caagctcaat ggtctgcttg gagtactcgc cagtggccag 3540
   agagcccttg caagacagct cggccagcat gagcagacct ctggccagct tctcgttggg 3600
   agaggggact aggaactcct tgtactggga gttctcgtag tcagagacgt cctccttctt 3660
   ctgttcagag acagtttcct cggcaccagc tcgcaggcca gcaatgattc cggttccggg 3720
   tacaccgtgg gcgttggtga tatcggacca ctcggcgatt cggtgacacc ggtactggtg 3780
   cttgacagtg ttgccaatat ctgcgaactt tctgtcctcg aacaggaaga aaccgtgctt 3840
   aagagcaagt tccttgaggg ggagcacagt gccggcgtag gtgaagtcgt caatgatgtc 3900
   gatatgggtt ttgatcatgc acacataagg tccgacctta tcggcaagct caatgagctc 3960
   cttggtggtg gtaacatcca gagaagcaca caggttggtt ttcttggctg ccacgagctt 4020
   gagcactcga gcggcaaagg cggacttgtg gacgttagct cgagcttcgt aggagggcat 4080
   tttggtggtg aagaggagac tgaaataaat ttagtctgca gaacttttta tcggaacctt 4140
   atctggggca gtgaagtata tgttatggta atagttacga gttagttgaa cttatagata 4200
   gactggacta tacggctatc ggtccaaatt agaaagaacg tcaatggctc tctgggcgtc 4260
   gcctttgccg acaaaaatgt gatcatgatg aaagccagca atgacgttgc agctgatatt 4320
   gttgtcggcc aaccgcgccg aaaacgcagc tgtcagaccc acagcctcca acgaagaatg 4380
   tatcgtcaaa gtgatccaag cacactcata gttggagtcg tactccaaag gcggcaatga 4440
   cgagtcagac agatactcgt cgaccttttc cttgggaacc accaccgtca gcccttctga 4500
   ctcacgtatt gtagccaccg acacaggcaa cagtccgtgg atagcagaat atgtcttgtc 4560
   ggtccatttc tcaccaactt taggcgtcaa gtgaatgttg cagaagaagt atgtgccttc 4620
   attgagaatc ggtgttgctg atttcaataa agtcttgaga tcagtttggc cagtcatgtt 4680
   gtggggggta attggattga gttatcgcct acagtctgta caggtatact cgctgcccac 4740
   tttatacttt ttgattccgc tgcacttgaa gcaatgtcgt ttaccaaaag tgagaatgct 4800
   ccacagaaca caccccaggg tatggttgag caaaaaataa acactccgat acggggaatc 4860
   gaaccccggt ctccacggtt ctcaagaagt attcttgatg agagcgtatc gatcgaggaa 4920
   gaggacaagc ggctgcttct taagtttgtg acatcagtat ccaaggcacc attgcaagga 4980
   ttcaaggctt tgaacccgtc atttgccatt cgtaacgctg gtagacaggt tgatcggttc 5040
   cctacggcct ccacctgtgt caatcttctc aagctgcctg actatcagga cattgatcaa 5100
   cttcggaaga aacttttgta tgccattcga tcacatgctg gtttcgattt gtcttagagg 5160
   aacgcatata cagtaatcat agagaataaa cgatattcat ttattaaagt agatagttga 5220
   ggtagaagtt gtaaagagtg ataaatagcg gccgcttagg ccttgtctgc cttggcaacc 5280
   attcgagcga aggtgcccag gtaggagata agaatgccga caccttcgag catgggagga 5340
   gatcggtagg gcaggttgtg cttcttgcac agctgctcca ctttgatgga ggcagcggtg 5400
   aggttgtgtc tgggaagagt aggccacagg tgatgttcga tctggtagtt gaggccaccg 5460
   aaaaaccagt ctgtgacgag gcctcgctga acgttcatgg tctcgtgaat ctgtccagca 5520
   cagaagccat gaccatccca cacggagtcc tgaatcttct ccagagggta gtggttcatg 5580
   aagacaacga tggcaattcc gaagccacca agcagctcgg agacaaagaa caccatgagt 5640
   ccggtgagaa acgagggcat gtagaaatag tagaacaagg ccttgagacc ccagtggagt 5700
   gccagaccga cggactcctt ctcgtactgt cttcgatagt actgattgct tcgatccttg 5760
   agacccgtgg cagtatgaat ggactgaaag caccagatga atcgcagaag ggcacagatg 5820
   aaaaagaagt aatactgttg gtacttgatc atccgtcgag aaaacgggcc agctcgctcg 5880
   acgtcctcct tggaccacgc caggagaggc aggttgtcga tgtcgggatc gtgaccctgc 5940
   acgttggtgg cggagtgatg agcgttgtgt cggtccttcc accaagtgac agaaaagccc 6000
   tgaagcacgt ttccgaagag caggccaatg gcattgttga tggatcggtc cttgaacagc 6060
   tggtgatggc aaatgtcgtg cgagagccat cccatctgtt gaaagtgaat gccgaggacg 6120
   atagcaccaa ccaggtacca gtgccactgg gtcatcaaca gcacggcaag gacacccagt 6180
   ccgagagtcg acagggtctt gtaagcgtac cacatgggag aggcatcgaa catgcctgca 6240
   gcaatgagct cgtctcgcag ctttcggaaa tcctcctgag gttcgtcgga gtttggcttg 6300
   ggaggtgttg gagtcagggg agaggaaggt tccatgatgg gcattcgtct gagtttggac 6360
   acggcgttct cggagtgcat aaccatgaag acgtcggtag catcccgacc tcggtagttc 6420
   tcgataatgt cggcacctcc gggatggtgg ttgagccagg cagaaacgtc gtaggtgaca 6480
   ccgtccacgg tgagaggcag agcgggtcgc ttgaccatgg atcccatgac ggccagagag 6540
   tcgtagtagg tagcgggagg aagtccggca ggtcgagcgg gaccggcgcc ctcctttttg 6600
   cctcggttct ttcgaggctt ccgaatgtag gtctggacgt agaagttgag gaacagaagc 6660
   agcaccgtgc cgacatacca gtagttgaag atccaggcaa acattcgcat accgtcctgc 6720
   cggtagcagg gcacgtttcg gtacttccag acgatgtaga atccgacgtt gaactggata 6780
   atttgcatgg aggtaatgag gttcttgggc atagggaagt tcagcttgat gagtcgcgtc 6840
   caatagtaac cgtacatgat ccagtgaatg aagccgttga gcagaacaaa gatccagacg 6900
   ccttcgtttc gatacttgta gaacagccac atgtcgatgg gagctccgag atggtgaaag 6960
   aactgcagaa acgacaaggg cttgtccatg aggggaaggt agaaggagtc gatgtactcg 7020
   acgaacttgc tgaggtagaa cagctgagtg gtgattcgga acacgtcatt gttgaaggca 7080
   gtctcacaat cgccggaaag aataccagtg acagagagag cgtaggccat cgccaggaag 7140
   gagccgaacg agtagatgga catgagaaag ttgtatgcgg tgaacagctt tttgagggtg 7200
   gcttgtcgct tgaggtccag aggtcgaagc atgaacttga tggccacgaa agcgaccgag 7260
   aggtacagaa cctcgcaaga ggaggcgtcc tgccagagct gggcgtagtc caccttggga 7320
   agttcctcct ggacgatgga gaccagctcc ttggcagcct ccatggtacc agagctgggt 7380
   tagtttgtgt agagagtgtg tgttgctagc gactttcgga ttgtgtcatt acacaaaacg 7440
   cgtcgtctcg acactgatct tgtcgtggat actcacggct cggaattctg tgatgtgtag 7500
   tttagatttc gaatctgtgg ggaaagaaag gaaaaaagag actggcaacc gattgggaga 7560
   gccactgttt atatataccc tagacaagcc ccccgcttgt aagatgttgg tcaatgtaaa 7620
   ccagtattaa ggttggcaag tgcaggagaa gcaaggtgtg ggtatcgagc aatggaaatg 7680
   tgcggaaggc aaaaaaatga ggccacggcc tattgtcggg gctatatcca gggggcgatt 7740
   gaagtacact aacatgacat gtgtccacag accctcaatc tggcctgatg agccaaatcc 7800
   atacgcgctt tcgcagctct aaaggctata acaagtcaca ccaccctgct cgacctcagc 7860
   gccctcactt tttgttaaga caaactgtac acgctgttcc agcgttttct gcctgcacct 7920
   ggtgggacat ttggtgcaac ctaaagtgct cggaacctct gtggtgtcca gatcagcgca 7980
   gcagttccga ggtagttttg aggcccttag atgatgcaat ggtgtcagtc gctggatcac 8040
   gagtcttaat ggcagtattc gttcttattt gtgccattga gccccgttat cctcgtatct 8100
   tctacccccc atcccatccc tttgttggtg caaccctacc catttattgt tgggtgcagc 8160
   ccaaccgacg tggagagctt ggcttggcca tataaaaagg ccccccccta gtggcaatgg 8220
   cagaaagtca gctgtgagtt gttgaatttg tcatctaggc ggcctggccg tcttctccgg 8280
   ggcaatttaa atgttcctct atagtagatc tgcgtacact gtttaaacag agtgtgaaag 8340
   actcactatg gtccgggctt atctcgacca atagccaaag tctggagttt ctgagagaaa 8400
   aaggcaagat acgtatgtaa caaagcgacg catggtacaa taataccgga ggcatgtatc 8460
   atagagagtt agtggttcga tgatggcact ggtgcctggt atgactttat acggctgact 8520
   acatatttgt cctcagacat acaattacag tcaagcactt acccttggac atctgtaggt 8580
   accccccggc caagacgatc tcagcgtgtc gtatgtcgga ttggcgtagc tccctcgctc 8640
   gtcaattggc tcccatctac tttcttctgc ttggctacac ccagcatgtc tgctatggct 8700
   cgttttcgtg ccttatctat cctcccagta ttaccaactc taaatgacat gatgtgattg 8760
   ggtctacact ttcatatcag agataaggag tagcacagtt gcataaaaag cccaactcta 8820
   atcagcttct tcctttcttg taattagtac aaaggtgatt agcgaaatct ggaagcttag 8880
   ttggccctaa aaaaatcaaa aaaagcaaaa aacgaaaaac gaaaaaccac agttttgaga 8940
   acagggaggt aacgaaggat cgtatatata tatatatata tatataccca cggatcccga 9000
   gaccggcctt tgattcttcc ctacaaccaa ccattctcac caccctaatt cacaaccatg 9060
   gagtctggac ccatgcctgc tggcattccc ttccctgagt actatgactt ctttatggac 9120
   tggaagactc ccctggccat cgctgccacc tacactgctg ccgtcggtct cttcaacccc 9180
   aaggttggca aggtctcccg agtggttgcc aagtcggcta acgcaaagcc tgccgagcga 9240
   acccagtccg gagctgccat gactgccttc gtctttgtgc acaacctcat tctgtgtgtc 9300
   tactctggca tcaccttcta ctacatgttt cctgctatgg tcaagaactt ccgaacccac 9360
   acactgcacg aagcctactg cgacacggat cagtccctct ggaacaacgc acttggctac 9420
   tggggttacc tcttctacct gtccaagttc tacgaggtca ttgacaccat catcatcatc 9480
   ctgaagggac gacggtcctc gctgcttcag acctaccacc atgctggagc catgattacc 9540
   atgtggtctg gcatcaacta ccaagccact cccatttgga tctttgtggt cttcaactcc 9600
   ttcattcaca ccatcatgta ctgttactat gccttcacct ctatcggatt ccatcctcct 9660
   ggcaaaaagt acctgacttc gatgcagatt actcagtttc tggtcggtat caccattgcc 9720
   gtgtcctacc tcttcgttcc tggctgcatc cgaacacccg gtgctcagat ggctgtctgg 9780
   atcaacgtcg gctacctgtt tcccttgacc tatctgttcg tggactttgc caagcgaacc 9840
   tactccaagc gatctgccat tgccgctcag aaaaaggctc agtaagcggc cgcatgagaa 9900
   gataaatata taaatacatt gagatattaa atgcgctaga ttagagagcc tcatactgct 9960
   cggagagaag ccaagacgag tactcaaagg ggattacacc atccatatcc acagacacaa 10020
   gctggggaaa ggttctatat acactttccg gaataccgta gtttccgatg ttatcaatgg 10080
   gggcagccag gatttcaggc acttcggtgt ctcggggtga aatggcgttc ttggcctcca 10140
   tcaagtcgta ccatgtcttc atttgcctgt caaagtaaaa cagaagcaga tgaagaatga 10200
   acttgaagtg aaggaattta aatgtaacga aactgaaatt tgaccagata ttgtgtccgc 10260
   ggtggagctc cagcttttgt tccctttagt gagggttaat ttcgagcttg gcgtaatcat 10320
   ggtcatagct gtttcctgtg tgaaattgtt atccgctcac aagcttccac acaacgtacg 10380
   tagggatcag gtgcttagga agctcgacca accacggaga ctgttgaaac tggatgtcgg 10440
   taacagcatc tggaatgctg aatgttcctc gaataacaac atatttctcc ttgttgaggt 10500
   gatcataagc tatgtatccg gtgattgaag tggaatagaa gtctcctccg aagactgagt 10560
   ccaacgtcat gttcgggaaa taccgacaac tctctccaca tgtaaaatca gttcgtagag 10620
   gagtgactgg cgcattgaca cagtaggcga tgtttgcaat ccgagaaaac ttggccgtaa 10680
   agttgtacag ctcctgggag gcttgaactc gagtttttga aagtgtcgct ggtggctcgc 10740
   cgaagaggga ggcatagagg tacgcaacca cttgcccgag cgtgaggttc atgatgccaa 10800
   tagtgaatgt catttatcac cgtactgcgc agtatttata tagggctcat cggtccatgt 10860
   atagatctgt ccacttatga cacccccatg tctcattaat gtgtaaaggt ggagacgggt 10920
   ggagtacagg tacagagttg gaggaaatca ggatagtggg gttaagacat gctccgagtc 10980
   caaatttcaa ctctccattg tcacaagacc tctggtttca gagttattac agatctaggc 11040
   ctgtttcaag gtgaggggac ctcatctgga tcggcacgac gatcgtcacc ttacagagga 11100
   cgtctgtcgc agggaaaggt gatgtggcgc gccagctgca ttaatgaatc ggccaacgcg 11160
   cggggagagg cggtttgcgt attgggcgct cttccgcttc ctcgctcact gactcgctgc 11220
   gctcggtcgt tcggctgcgg cgagcggtat cagctcactc aaaggcggta atacggttat 11280
   ccacagaatc aggggataac gcaggaaaga acatgtgagc aaaaggccag caaaaggcca 11340
   ggaaccgtaa aaaggccgcg ttgctggcgt ttttccatag gctccgcccc cctgacgagc 11400
   atcacaaaaa tcgacgctca agtcagaggt ggcgaaaccc gacaggacta taaagatacc 11460
   aggcgtttcc ccctggaagc tccctcgtgc gctctcctgt tccgaccctg ccgcttaccg 11520
   gatacctgtc cgcctttctc ccttcgggaa gcgtggcgct ttctcatagc tcacgctgta 11580
   ggtatctcag ttcggtgtag gtcgttcgct ccaagctggg ctgtgtgcac gaaccccccg 11640
   ttcagcccga ccgctgcgcc ttatccggta actatcgtct tgagtccaac ccggtaagac 11700
   acgacttatc gccactggca gcagccactg gtaacaggat tagcagagcg aggtatgtag 11760
   gcggtgctac agagttcttg aagtggtggc ctaactacgg ctacactaga agaacagtat 11820
   ttggtatctg cgctctgctg aagccagtta ccttcggaaa aagagttggt agctcttgat 11880
   ccggcaaaca aaccaccgct ggtagcggtg gtttttttgt ttgcaagcag cagattacgc 11940
   gcagaaaaaa aggatctcaa gaagatcctt tgatcttttc tacggggtct gacgctcagt 12000
   ggaacgaaaa ctcacgttaa gggattttgg tcatgagatt atcaaaaagg atcttcacct 12060
   agatcctttt aaattaaaaa tgaagtttta aatcaatcta aagtatatat gagtaaactt 12120
   ggtctgacag ttaccaatgc ttaatcagtg aggcacctat ctcagcgatc tgtctatttc 12180
   gttcatccat agttgcctga ctccccgtcg tgtagataac tacgatacgg gagggcttac 12240
   catctggccc cagtgctgca atgataccgc gagacccacg ctcaccggct ccagatttat 12300
   cagcaataaa ccagccagcc ggaagggccg agcgcagaag tggtcctgca actttatccg 12360
   cctccatcca gtctattaat tgttgccggg aagctagagt aagtagttcg ccagttaata 12420
   gtttgcgcaa cgttgttgcc attgctacag gcatcgtggt gtcacgctcg tcgtttggta 12480
   tggcttcatt cagctccggt tcccaacgat caaggcgagt tacatgatcc cccatgttgt 12540
   gcaaaaaagc ggttagctcc ttcggtcctc cgatcgttgt cagaagtaag ttggccgcag 12600
   tgttatcact catggttatg gcagcactgc ataattctct tactgtcatg ccatccgtaa 12660
   gatgcttttc tgtgactggt gagtactcaa ccaagtcatt ctgagaatag tgtatgcggc 12720
   gaccgagttg ctcttgcccg gcgtcaatac gggataatac cgcgccacat agcagaactt 12780
   taaaagtgct catcattgga aaacgttctt cggggcgaaa actctcaagg atcttaccgc 12840
   tgttgagatc cagttcgatg taacccactc gtgcacccaa ctgatcttca gcatctttta 12900
   ctttcaccag cgtttctggg tgagcaaaaa caggaaggca aaatgccgca aaaaagggaa 12960
   taagggcgac acggaaatgt tgaatactca tactcttcct ttttcaatat tattgaagca 13020
   tttatcaggg ttattgtctc atgagcggat acatatttga atgtatttag aaaaataaac 13080
   aaataggggt tccgcgcaca tttccccgaa aagtgccacc tgatgcggtg tgaaataccg 13140
   cacagatgcg taaggagaaa ataccgcatc aggaaattgt aagcgttaat attttgttaa 13200
   aattcgcgtt aaatttttgt taaatcagct cattttttaa ccaataggcc gaaatcggca 13260
   aaatccctta taaatcaaaa gaatagaccg agatagggtt gagtgttgtt ccagtttgga 13320
   acaagagtcc actattaaag aacgtggact ccaacgtcaa agggcgaaaa accgtctatc 13380
   agggcgatgg cccactacgt gaaccatcac cctaatcaag ttttttgggg tcgaggtgcc 13440
   gtaaagcact aaatcggaac cctaaaggga gcccccgatt tagagcttga cggggaaagc 13500
   cggcgaacgt ggcgagaaag gaagggaaga aagcgaaagg agcgggcgct agggcgctgg 13560
   caagtgtagc ggtcacgctg cgcgtaacca ccacacccgc cgcgcttaat gcgccgctac 13620
   agggcgcgtc cattcgccat tcaggctgcg caactgttgg gaagggcgat cggtgcgggc 13680
   ctcttcgcta ttacgccagc tggcgaaagg gggatgtgct gcaaggcgat taagttgggt 13740
   aacgccaggg ttttcccagt cacgacgttg taaaacgacg gccagtgaat tgtaatacga 13800
   ctcactatag ggcgaattgg gcccgacgtc gcatgccaag gcgtatatta gttggtggga 13860
   accagtgtac gaccgggtcc tgtataacca ggttcagtgg catacttgta ggagttgttc 13920
   ccgtggtatt tgggcatggc taagacattt cgccgaccaa tgttaagtgc acaatagccg 13980
   atgtagtaga tgtaagccag atggttttgg agcaggtcga ttcgagacca cagattgaaa 14040
   gtgcctcgat ggcaggcctc gttttctcct ccttcacaga cagacacttt gtcgagtgca 14100
   gggtagacgc tttcttggtc aatgtacact tctccaatgg cgtgtgtgta attggaccaa 14160
   tctggcagtc caacgaagat atcgttccag tgggtgagcc tgtagtttcg tcgcttgtcg 14220
   tttacttcaa ggcctgtgtc attaaaccac agctggttga tgtagtttgc aaactctgag 14280
   tttcctactc tcggctggcc aaagttgatc atggtaggat catgtccaag taatttgaag 14340
   tgagttgcga aaagaagagc ttgagcagcg cccagcgagt gaccagtaac atacattttg 14400
   tagtcagtgt ggttggtgag gaacttttca aactgaggag cagcattgac catagtttcg 14460
   ttgaaggcct tggcgaaccc atcatggatc atgcagcctt tgcactcact agttttgatg 14520
   ggaataagac gggggtcttc aaccaccaga gcccgctctt tgaggttgtc aagacctttg 14580
   ttctccactt ccaagtctgg tcggactgcc catctctgtt aat 14623
<210> 135
   <211> 14619
   <212> DNA
   <213> Artificial Sequence
<220>
   <223> Plasmid pZP2-85m98F
<400> 135
   cgatggaagc cggtagaacc gggctgcttg tgcttggaga tggaagccgg tagaaccggg 60
   ctgcttgggg ggatttgggg ccgctgggct ccaaagaggg gtaggcattt cgttggggtt 120
   acgtaattgc ggcatttggg tcctgcgcgc atgtcccatt ggtcagaatt agtccggata 180
   ggagacttat cagccaatca cagcgccgga tccacctgta ggttgggttg ggtgggagca 240
   cccctccaca gagtagagtc aaacagcagc agcaacatga tagttggggg tgtgcgtgtt 300
   aaaggaaaaa aaagaagctt gggttatatt cccgctctat ttagaggttg cgggatagac 360
   gccgacggag ggcaatggcg ctatggaacc ttgcggatat ccatacgccg cggcggactg 420
   cgtccgaacc agctccagca gcgttttttc cgggccattg agccgactgc gaccccgcca 480
   acgtgtcttg gcccacgcac tcatgtcatg ttggtgttgg gaggccactt tttaagtagc 540
   acaaggcacc tagctcgcag caaggtgtcc gaaccaaaga agcggctgca gtggtgcaaa 600
   cggggcggaa acggcgggaa aaagccacgg gggcacgaat tgaggcacgc cctcgaattt 660
   gagacgagtc acggccccat tcgcccgcgc aatggctcgc caacgcccgg tcttttgcac 720
   cacatcaggt taccccaagc caaacctttg tgttaaaaag cttaacatat tataccgaac 780
   gtaggtttgg gcgggcttgc tccgtctgtc caaggcaaca tttatataag ggtctgcatc 840
   gccggctcaa ttgaatcttt tttcttcttc tcttctctat attcattctt gaattaaaca 900
   cacatcaacc atggtcaagc gacccgctct gcctctcacc gtggacggtg tcacctacga 960
   cgtttctgcc tggctcaacc accatcccgg aggtgccgac attatcgaga actaccgagg 1020
   tcgggatgct accgacgtct tcatggttat gcactccgag aacgccgtgt ccaaactcag 1080
   acgaatgccc atcatggaac cttcctctcc cctgactcca acacctccca agccaaactc 1140
   cgacgaacct caggaggatt tccgaaagct gcgagacgag ctcattgctg caggcatgtt 1200
   cgatgcctct cccatgtggt acgcttacaa gaccctgtcg actctcggac tgggtgtcct 1260
   tgccgtgctg ttgatgaccc agtggcactg gtacctggtt ggtgctatcg tcctcggcat 1320
   tcactttcaa cagatgggat ggctctcgca cgacatttgc catcaccagc tgttcaagga 1380
   ccgatccatc aacaatgcca ttggcctgct cttcggaaac gtgcttcagg gcttttctgt 1440
   cacttggtgg aaggaccgac acaacgctca tcactccgcc accaacgtgc agggtcacga 1500
   tcccgacatc gacaacctgc ctctcctggc gtggtccaag gaggacgtcg agcgagctgg 1560
   cccgttttct cgacggatga tcaagtacca acagtattac ttctttttca tctgtgccct 1620
   tctgcgattc atctggtgct ttcagtccat tcatactgcc acgggtctca aggatcgaag 1680
   caatcagtac tatcgaagac agtacgagaa ggagtccgtc ggtctggcac tccactgggg 1740
   tctcaaggcc ttgttctact atttctacat gccctcgttt ctcaccggac tcatggtgtt 1800
   ctttgtctcc gagctgcttg gtggcttcgg aattgccatc gttgtcttca tgaaccacta 1860
   ccctctggag aagattcagg actccgtgtg ggatggtcat ggcttctgtg ctggacagat 1920
   tcacgagacc atgaacgttc agcgaggcct cgtcacagac tggtttttcg gtggcctcaa 1980
   ctaccagatc gaacatcacc tgtggcctac tcttcccaga cacaacctca ccgctgcctc 2040
   catcaaagtg gagcagctgt gcaagaagca caacctgccc taccgatctc ctcccatgct 2100
   cgaaggtgtc ggcattctta tctcctacct gggcaccttc gctcgaatgg ttgccaaggc 2160
   agacaaggcc taagcggccg cattgatgat tggaaacaca cacatgggtt atatctaggt 2220
   gagagttagt tggacagtta tatattaaat cagctatgcc aacggtaact tcattcatgt 2280
   caacgaggaa ccagtgactg caagtaatat agaatttgac caccttgcca ttctcttgca 2340
   ctcctttact atatctcatt tatttcttat atacaaatca cttcttcttc ccagcatcga 2400
   gctcggaaac ctcatgagca ataacatcgt ggatctcgtc aatagagggc tttttggact 2460
   ccttgctgtt ggccaccttg tccttgctgt ttaaacatcg tggttaatgc tgctgtgtgc 2520
   tgtgtgtgtg tgttgtttgg cgctcattgt tgcgttatgc agcgtacacc acaatattgg 2580
   aagcttatta gcctttctat tttttcgttt gcaaggctta acaacattgc tgtggagagg 2640
   gatggggata tggaggccgc tggagggagt cggagaggcg ttttggagcg gcttggcctg 2700
   gcgcccagct cgcgaaacgc acctaggacc ctttggcacg ccgaaatgtg ccacttttca 2760
   gtctagtaac gccttaccta cgtcattcca tgcgtgcatg tttgcgcctt ttttcccttg 2820
   cccttgatcg ccacacagta cagtgcactg tacagtggag gttttggggg ggtcttagat 2880
   gggagctaaa agcggcctag cggtacacta gtgggattgt atggagtggc atggagccta 2940
   ggtggagcct gacaggacgc acgaccggct agcccgtgac agacgatggg tggctcctgt 3000
   tgtccaccgc gtacaaatgt ttgggccaaa gtcttgtcag ccttgcttgc gaacctaatt 3060
   cccaattttg tcacttcgca cccccattga tcgagcccta acccctgccc atcaggcaat 3120
   ccaattaagc tcgcattgtc tgccttgttt agtttggctc ctgcccgttt cggcgtccac 3180
   ttgcacaaac acaaacaagc attatatata aggctcgtct ctccctccca accacactca 3240
   cttttttgcc cgtcttccct tgctaacaca aaagtcaaga acacaaacaa ccaccccaac 3300
   ccccttacac acaagacata tctacagcaa tggccatggc tctctccctt actaccgagc 3360
   agctgctcga gcgacccgac ctggttgcca tcgacggcat tctctacgat ctggaaggtc 3420
   ttgccaaggt ccatcccgga tccgacttga tcctcgcttc tggtgcctcc gatgcttctc 3480
   ctctgttcta ctccatgcac ccttacgtca agcccgagaa ctcgaagctg cttcaacagt 3540
   tcgtgcgagg caagcacgac cgaacctcca aggacattgt ctacacctac gactctccct 3600
   ttgcacagga cgtcaagcga actatgcgag aggtcatgaa aggtcggaac tggtatgcca 3660
   cacctggatt ctggctgcga accgttggca tcattgctgt caccgccttt tgcgagtggc 3720
   actgggctac taccggaatg gtgctgtggg gtctcttgac tggattcatg cacatgcaga 3780
   tcggcctgtc cattcagcac gatgcctctc atggtgccat cagcaaaaag ccctgggtca 3840
   acgctctctt tgcctacggc atcgacgtca ttggatcgtc cagatggatc tggctgcagt 3900
   ctcacatcat gcgacatcac acctacacca atcagcatgg tctcgacctg gatgccgagt 3960
   ccgcagaacc attccttgtg ttccacaact accctgctgc caacactgct cgaaagtggt 4020
   ttcaccgatt ccaggcctgg tacatgtacc tcgtgcttgg agcctacggc gtttcgctgg 4080
   tgtacaaccc tctctacatc ttccgaatgc agcacaacga caccattccc gagtctgtca 4140
   cagccatgcg agagaacggc tttctgcgac ggtaccgaac ccttgcattc gttatgcgag 4200
   ctttcttcat ctttcgaacc gccttcttgc cctggtatct cactggaacc tccctgctca 4260
   tcaccattcc tctggtgccc actgctaccg gtgccttcct caccttcttt ttcatcttgt 4320
   ctcacaactt cgatggctcg gagcgaatcc ccgacaagaa ctgcaaggtc aagagctccg 4380
   agaaggacgt tgaagccgat cagatcgact ggtacagagc tcaggtggag acctcttcca 4440
   cctacggtgg acccattgcc atgttcttta ctggcggtct caacttccag atcgagcatc 4500
   acctctttcc tcgaatgtcg tcttggcact atcccttcgt gcagcaagct gtccgagagt 4560
   gttgcgaacg acacggagtt cggtacgtct tctaccctac cattgtgggc aacatcattt 4620
   ccaccctcaa gtacatgcac aaagtcggtg tggttcactg tgtcaaggac gctcaggatt 4680
   cctaagcggc cgcatgagaa gataaatata taaatacatt gagatattaa atgcgctaga 4740
   ttagagagcc tcatactgct cggagagaag ccaagacgag tactcaaagg ggattacacc 4800
   atccatatcc acagacacaa gctggggaaa ggttctatat acactttccg gaataccgta 4860
   gtttccgatg ttatcaatgg gggcagccag gatttcaggc acttcggtgt ctcggggtga 4920
   aatggcgttc ttggcctcca tcaagtcgta ccatgtcttc atttgcctgt caaagtaaaa 4980
   cagaagcaga tgaagaatga acttgaagtg aaggaattta aatgtaacga aactgaaatt 5040
   tgaccagata ttgtgtccgc ggtggagctc cagcttttgt tccctttagt gagggttaat 5100
   ttcgagcttg gcgtaatcat ggtcatagct gtttcctgtg tgaaattgtt atccgctcac 5160
   aagcttccac acaacgtacg ggcgtcgttg cttgtgtgat ttttgaggac ccatcccttt 5220
   ggtatataag tatactctgg ggttaaggtt gcccgtgtag tctaggttat agttttcatg 5280
   tgaaataccg agagccgagg gagaataaac gggggtattt ggacttgttt ttttcgcgga 5340
   aaagcgtcga atcaaccctg cgggccttgc accatgtcca cgacgtgttt ctcgccccaa 5400
   ttcgcccctt gcacgtcaaa attaggcctc catctagacc cctccataac atgtgactgt 5460
   ggggaaaagt ataagggaaa ccatgcaacc atagacgacg tgaaagacgg ggaggaacca 5520
   atggaggcca aagaaatggg gtagcaacag tccaggagac agacaaggag acaaggagag 5580
   ggcgcccgaa agatcggaaa aacaaacatg tccaattggg gcagtgacgg aaacgacacg 5640
   gacacttcag tacaatggac cgaccatctc caagccaggg ttattccggt atcaccttgg 5700
   ccgtaacctc ccgctggtac ctgatattgt acacgttcac attcaatata ctttcagcta 5760
   caataagaga ggctgtttgt cgggcatgtg tgtccgtcgt atggggtgat gtccgagggc 5820
   gaaattcgct acaagcttaa ctctggcgct tgtccagtat gaatagacaa gtcaagacca 5880
   gtggtgccat gattgacagg gaggtacaag acttcgatac tcgagcatta ctcggacttg 5940
   tggcgattga acagacgggc gatcgcttct cccccgtatt gccggcgcgc cagctgcatt 6000
   aatgaatcgg ccaacgcgcg gggagaggcg gtttgcgtat tgggcgctct tccgcttcct 6060
   cgctcactga ctcgctgcgc tcggtcgttc ggctgcggcg agcggtatca gctcactcaa 6120
   aggcggtaat acggttatcc acagaatcag gggataacgc aggaaagaac atgtgagcaa 6180
   aaggccagca aaaggccagg aaccgtaaaa aggccgcgtt gctggcgttt ttccataggc 6240
   tccgcccccc tgacgagcat cacaaaaatc gacgctcaag tcagaggtgg cgaaacccga 6300
   caggactata aagataccag gcgtttcccc ctggaagctc cctcgtgcgc tctcctgttc 6360
   cgaccctgcc gcttaccgga tacctgtccg cctttctccc ttcgggaagc gtggcgcttt 6420
   ctcatagctc acgctgtagg tatctcagtt cggtgtaggt cgttcgctcc aagctgggct 6480
   gtgtgcacga accccccgtt cagcccgacc gctgcgcctt atccggtaac tatcgtcttg 6540
   agtccaaccc ggtaagacac gacttatcgc cactggcagc agccactggt aacaggatta 6600
   gcagagcgag gtatgtaggc ggtgctacag agttcttgaa gtggtggcct aactacggct 6660
   acactagaag aacagtattt ggtatctgcg ctctgctgaa gccagttacc ttcggaaaaa 6720
   gagttggtag ctcttgatcc ggcaaacaaa ccaccgctgg tagcggtggt ttttttgttt 6780
   gcaagcagca gattacgcgc agaaaaaaag gatctcaaga agatcctttg atcttttcta 6840
   cggggtctga cgctcagtgg aacgaaaact cacgttaagg gattttggtc atgagattat 6900
   caaaaaggat cttcacctag atccttttaa attaaaaatg aagttttaaa tcaatctaaa 6960
   gtatatatga gtaaacttgg tctgacagtt accaatgctt aatcagtgag gcacctatct 7020
   cagcgatctg tctatttcgt tcatccatag ttgcctgact ccccgtcgtg tagataacta 7080
   cgatacggga gggcttacca tctggcccca gtgctgcaat gataccgcga gacccacgct 7140
   caccggctcc agatttatca gcaataaacc agccagccgg aagggccgag cgcagaagtg 7200
   gtcctgcaac tttatccgcc tccatccagt ctattaattg ttgccgggaa gctagagtaa 7260
   gtagttcgcc agttaatagt ttgcgcaacg ttgttgccat tgctacaggc atcgtggtgt 7320
   cacgctcgtc gtttggtatg gcttcattca gctccggttc ccaacgatca aggcgagtta 7380
   catgatcccc catgttgtgc aaaaaagcgg ttagctcctt cggtcctccg atcgttgtca 7440
   gaagtaagtt ggccgcagtg ttatcactca tggttatggc agcactgcat aattctctta 7500
   ctgtcatgcc atccgtaaga tgcttttctg tgactggtga gtactcaacc aagtcattct 7560
   gagaatagtg tatgcggcga ccgagttgct cttgcccggc gtcaatacgg gataataccg 7620
   cgccacatag cagaacttta aaagtgctca tcattggaaa acgttcttcg gggcgaaaac 7680
   tctcaaggat cttaccgctg ttgagatcca gttcgatgta acccactcgt gcacccaact 7740
   gatcttcagc atcttttact ttcaccagcg tttctgggtg agcaaaaaca ggaaggcaaa 7800
   atgccgcaaa aaagggaata agggcgacac ggaaatgttg aatactcata ctcttccttt 7860
   ttcaatatta ttgaagcatt tatcagggtt attgtctcat gagcggatac atatttgaat 7920
   gtatttagaa aaataaacaa ataggggttc cgcgcacatt tccccgaaaa gtgccacctg 7980
   atgcggtgtg aaataccgca cagatgcgta aggagaaaat accgcatcag gaaattgtaa 8040
   gcgttaatat tttgttaaaa ttcgcgttaa atttttgtta aatcagctca ttttttaacc 8100
   aataggccga aatcggcaaa atcccttata aatcaaaaga atagaccgag atagggttga 8160
   gtgttgttcc agtttggaac aagagtccac tattaaagaa cgtggactcc aacgtcaaag 8220
   ggcgaaaaac cgtctatcag ggcgatggcc cactacgtga accatcaccc taatcaagtt 8280
   ttttggggtc gaggtgccgt aaagcactaa atcggaaccc taaagggagc ccccgattta 8340
   gagcttgacg gggaaagccg gcgaacgtgg cgagaaagga agggaagaaa gcgaaaggag 8400
   cgggcgctag ggcgctggca agtgtagcgg tcacgctgcg cgtaaccacc acacccgccg 8460
   cgcttaatgc gccgctacag ggcgcgtcca ttcgccattc aggctgcgca actgttggga 8520
   agggcgatcg gtgcgggcct cttcgctatt acgccagctg gcgaaagggg gatgtgctgc 8580
   aaggcgatta agttgggtaa cgccagggtt ttcccagtca cgacgttgta aaacgacggc 8640
   cagtgaattg taatacgact cactataggg cgaattgggc ccgacgtcgc atgcgctgat 8700
   gacactttgg tctgaaagag atgcattttg aatcccaaac ttgcagtgcc caagtgacat 8760
   acatctccgc gttttggaaa atgttcagaa acagttgatt gtgttggaat ggggaatggg 8820
   gaatggaaaa atgactcaag tatcaattcc aaaaacttct ctggctggca gtacctactg 8880
   tccatactac tgcattttct ccagtcaggc cactctatac tcgacgacac agtagtaaaa 8940
   cccagataat ttcgacataa acaagaaaac agacccaata atatttatat atagtcagcc 9000
   gtttgtccag ttcagactgt aatagccgaa aaaaaatcca aagtttctat tctaggaaaa 9060
   tatattccaa tatttttaat tcttaatctc atttatttta ttctagcgaa atacatttca 9120
   gctacttgag acatgtgata cccacaaatc ggattcggac tcggttgttc agaagagcat 9180
   atggcattcg tgctcgcttg ttcacgtatt cttcctgttc catctcttgg ccgacaatca 9240
   cacaaaaatg gggttttttt tttaattcta atgattcatt acagcaaaat tgagatatag 9300
   cagaccacgt attccataat caccaaggaa gttcttgggc gtcttaatta actcacctgc 9360
   aggattgaga ctatgaatgg attcccgtgc ccgtattact ctactaattt gatcttggaa 9420
   cgcgaaaata cgtttctagg actccaaaga atctcaactc ttgtccttac taaatatact 9480
   acccatagtt gatggtttac ttgaacagag aggacatgtt cacttgaccc aaagtttctc 9540
   gcatctcttg gatatttgaa caacggcgtc cactgaccgt cagttatcca gtcacaaaac 9600
   ccccacattc atacattccc atgtacgttt acaaagttct caattccatc gtgcaaatca 9660
   aaatcacatc tattcattca tcatatataa acccatcatg tctactaaca ctcacaactc 9720
   catagaaaac atcgactcag aacacacgct ccatgcggcc gcttactgag ccttggcacc 9780
   gggctgcttc tcggccattc gagcgaactg ggacaggtat cggagcagga tgacgagacc 9840
   ttcatggggc agagggtttc ggtaggggag gttgtgcttc tggcacagct gttccacctg 9900
   gtaggaaacg gcagtgaggt tgtgtcgagg cagggtgggc cagagatggt gctcgatctg 9960
   gtagttcagg cctccaaaga accagtcagt aatgatgcct cgtcgaatgt tcatggtctc 10020
   atggatctga cccacagaga agccatgtcc gtcccagacg gaatcaccga tcttctccag 10080
   agggtagtgg ttcatgaaga ccacgatggc aattccgaag ccaccgacga gctcggaaac 10140
   aaagaacacc agcatcgagg tcaggatgga gggcataaag aagaggtgga acagggtctt 10200
   gagagtccag tgcagagcga gtccaatggc ctctttcttg tactgagatc ggtagaactg 10260
   gttgtctcgg tccttgaggg atcgaacggt cagcacagac tggaaacacc agatgaatcg 10320
   caggagaata cagatgacca ggaaatagta ctgttggaac tgaatgagct ttcgggagat 10380
   gggagaagct cgagtgacat cgtcctcgga ccaggcgagc agaggcaggt tatcaatgtc 10440
   gggatcgtga ccctgaacgt tggtagcaga atgatgggcg ttgtgtctgt ccttccacca 10500
   ggtcacggag aagccctgga gtccgttgcc aaagaccaga cccaggacgt tattccagtt 10560
   tcggttcttg aaggtctggt ggtggcagat gtcatgagac agccatccca tttgctggta 10620
   gtgcataccg agcacgagag caccaatgaa gtacaggtgg tactggacca gcatgaagaa 10680
   ggcaagcacg ccaagaccca gggtggtcaa gatcttgtac gagtaccaga ggggagaggc 10740
   gtcaaacatg ccagtggcga tcagctcttc tcggagcttt cggaaatcct cctgagcttc 10800
   gttgacggca gcctggggag gcagctcgga agcctggttg atcttgggca ttcgcttgag 10860
   cttgtcgaag gcttcctgag agtgcataac catgaaggcg tcagtagcat ctcgtccctg 10920
   gtagttctca atgatttcag ctccaccagg gtggaagttc acccaagcgg agacgtcgta 10980
   cacctttccg tcgatgacga ggggcagagc ctgtcgagaa gccttcacgg atcccatgac 11040
   ggccagagag tcgtagtagg tagcgggagg aagtccggca ggtcgagcgg gaccggcgcc 11100
   ctgaatcttt ttggctccct tgtgctttcg gacgatgtag gtctgcacgt agaagttgag 11160
   gaacagacac aggacagtac caacgtagaa gtagttgaaa aaccagccaa acattctcat 11220
   tccatcttgt cggtagcagg gaatgttccg gtacttccag acgatgtaga agccaacgtt 11280
   gaactgaatg atctgcatag aagtaatcag ggacttgggc atagggaact tgagcttgat 11340
   cagtcgggtc caatagtagc cgtacatgat ccagtgaatg aagccgttga gcagcacaaa 11400
   gatccaaacg gcttcgtttc ggtagttgta gaacagccac atgtccatag gagctccgag 11460
   atggtgaaag aactgcaacc aggtcagagg cttgcccatg aggggcagat agaaggagtc 11520
   aatgtactcg aggaacttgc tgaggtagaa cagctgagtg gtgattcgga agacattgtt 11580
   gtcgaaagcc ttctcgcagt tgtcggacat gacaccaatg gtgtacatgg cgtaggccat 11640
   agagaggaag gagcccagcg agtagatgga catgagcagg ttgtagttgg tgaacacaaa 11700
   cttcattcga gactgaccct tgggtccgag aggaccaagg gtgaacttca ggatgacgaa 11760
   ggcgatggag aggtacagca cctcgcagtg cgaggcatca gaccagagct gagcatagtc 11820
   gaccttggga agaacctcct ggccaatgga gacgatttcg ttcacgacct ccatggttgt 11880
   gaattagggt ggtgagaatg gttggttgta gggaagaatc aaaggccggt ctcgggatcc 11940
   gtgggtatat atatatatat atatatatac gatccttcgt tacctccctg ttctcaaaac 12000
   tgtggttttt cgtttttcgt tttttgcttt ttttgatttt tttagggcca actaagcttc 12060
   cagatttcgc taatcacctt tgtactaatt acaagaaagg aagaagctga ttagagttgg 12120
   gctttttatg caactgtgct actccttatc tctgatatga aagtgtagac ccaatcacat 12180
   catgtcattt agagttggta atactgggag gatagataag gcacgaaaac gagccatagc 12240
   agacatgctg ggtgtagcca agcagaagaa agtagatggg agccaattga cgagcgaggg 12300
   agctacgcca atccgacata cgacacgctg agatcgtctt ggccgggggg tacctacaga 12360
   tgtccaaggg taagtgcttg actgtaattg tatgtctgag gacaaatatg tagtcagccg 12420
   tataaagtca taccaggcac cagtgccatc atcgaaccac taactctcta tgatacatgc 12480
   ctccggtatt attgtaccat gcgtcgcttt gttacatacg tatcttgcct ttttctctca 12540
   gaaactccag aattctctct cttgagcttt tccataacaa gttcttctgc ctccaggaag 12600
   tccatgggtg gtttgatcat ggttttggtg tagtggtagt gcagtggtgg tattgtgact 12660
   ggggatgtag ttgagaataa gtcatacaca agtcagcttt cttcgagcct catataagta 12720
   taagtagttc aacgtattag cactgtaccc agcatctccg tatcgagaaa cacaacaaca 12780
   tgccccattg gacagatcat gcggatacac aggttgtgca gtatcataca tactcgatca 12840
   gacaggtcgt ctgaccatca tacaagctga acaagcgctc catacttgca cgctctctat 12900
   atacacagtt aaattacata tccatagtct aacctctaac agttaatctt ctggtaagcc 12960
   tcccagccag ccttctggta tcgcttggcc tcctcaatag gatctcggtt ctggccgtac 13020
   agacctcggc cgacaattat gatatccgtt ccggtagaca tgacatcctc aacagttcgg 13080
   tactgctgtc cgagagcgtc tcccttgtcg tcaagaccca ccccgggggt cagaataagc 13140
   cagtcctcag agtcgccctt aggtcggttc tgggcaatga agccaaccac aaactcgggg 13200
   tcggatcggg caagctcaat ggtctgcttg gagtactcgc cagtggccag agagcccttg 13260
   caagacagct cggccagcat gagcagacct ctggccagct tctcgttggg agaggggact 13320
   aggaactcct tgtactggga gttctcgtag tcagagacgt cctccttctt ctgttcagag 13380
   acagtttcct cggcaccagc tcgcaggcca gcaatgattc cggttccggg tacaccgtgg 13440
   gcgttggtga tatcggacca ctcggcgatt cggtgacacc ggtactggtg cttgacagtg 13500
   ttgccaatat ctgcgaactt tctgtcctcg aacaggaaga aaccgtgctt aagagcaagt 13560
   tccttgaggg ggagcacagt gccggcgtag gtgaagtcgt caatgatgtc gatatgggtt 13620
   ttgatcatgc acacataagg tccgacctta tcggcaagct caatgagctc cttggtggtg 13680
   gtaacatcca gagaagcaca caggttggtt ttcttggctg ccacgagctt gagcactcga 13740
   gcggcaaagg cggacttgtg gacgttagct cgagcttcgt aggagggcat tttggtggtg 13800
   aagaggagac tgaaataaat ttagtctgca gaacttttta tcggaacctt atctggggca 13860
   gtgaagtata tgttatggta atagttacga gttagttgaa cttatagata gactggacta 13920
   tacggctatc ggtccaaatt agaaagaacg tcaatggctc tctgggcgtc gcctttgccg 13980
   acaaaaatgt gatcatgatg aaagccagca atgacgttgc agctgatatt gttgtcggcc 14040
   aaccgcgccg aaaacgcagc tgtcagaccc acagcctcca acgaagaatg tatcgtcaaa 14100
   gtgatccaag cacactcata gttggagtcg tactccaaag gcggcaatga cgagtcagac 14160
   agatactcgt cgaccttttc cttgggaacc accaccgtca gcccttctga ctcacgtatt 14220
   gtagccaccg acacaggcaa cagtccgtgg atagcagaat atgtcttgtc ggtccatttc 14280
   tcaccaactt taggcgtcaa gtgaatgttg cagaagaagt atgtgccttc attgagaatc 14340
   ggtgttgctg atttcaataa agtcttgaga tcagtttggc cagtcatgtt gtggggggta 14400
   attggattga gttatcgcct acagtctgta caggtatact cgctgcccac tttatacttt 14460
   ttgattccgc tgcacttgaa gcaatgtcgt ttaccaaaag tgagaatgct ccacagaaca 14520
   caccccaggg tatggttgag caaaaaataa acactccgat acggggaatc gaaccccggt 14580
   ctccacggtt ctcaagaagt attcttgatg agagcgtat 14619
<210> 136
   <211> 15262
   <212> DNA
   <213> Artificial Sequence
<220>
   <223> Plasmid pZK16-ML8N
<400> 136
   gtacgcattc ttgacttgct ccccagttgc cctttttggt cataactcag ccccgactct    60
   ctccccaaag aggagatgga cttacaatag atacgagaca acagatattt tcggccacct   120
   ggtgcaattg gtctgtttta ttacccccaa taatatccac agcgtccgaa tcagctcgac    180
   aagctctttc tcagcatact gatcttaccc catacccccc agacagcggt gccatatcca    240
   tacccgcact acctgagaca gtaaactcag ggcaaggagc ggtcttggtc catcgcgacg   300
   ctgtaagctt tgcctttgcg ttttgtgatc caccacacgg cagtcaaggt ctcggttgaa   360
   agatgggtct ggcaatgtgg attgctgaat cattgacata taaatacgag tgatcctgcc   420
   atttcccaat ctttagaaat catcttttct tcacacatac aaaaaacaca catcacaatg   480
   cagttctccg ccgccgccgt ccttgccctc gccgccttcg tctctgccca ggccgcctcc   540
   gctgcttctg aagctggtgg tgccgttgtc tccggtgcct ccggtgccgc tgccgccgct   600
   tcttctgctg tcattggctc cgagtctcag gccgcttctt ctgctgcctc ttctgctgct   660
   gagtctgctt cttccgctgc ctctgagtcc gcctcctccg cttccgcctc cgagtccaag   720
   gcctctgagt ccgcttccaa gtccgcctcc aaggcttctg agtctgcttc caaggccgag   780
   tcttctgctg ccaaggcttc ctccgctgct ggttctgctt cttccgccgc tgcctctgcc 840
   accaagtctg gtgatgcctc cggctcttcc aagccttccg gctctgcctc ctctaccggt 900
   gacaagtccg gtgctggtat tgttgccccc gctgttggtg ccatcgttgt cggtgctctt 960
   gctcagctca tctaagcggt ctttgtacaa ctacccactt aatatttata ttatgatata 1020
   gactgcaata ctattgcatg tgtaatgctt cgtttgaaag tcgtagtccg gccattcatg 1080
   gcagtgtatg gcagtattgc aaccaattgg cttaataaca tatacaaagt ggcaattctg 1140
   tgttcgaacc atcacacaac ccaacatatt tgacattgct acaagtattc gacttgttct 1200
   tcggccgcac gacatgcatt gctagcagca cacttgcaga agcttggcca atggttaggt 1260
   tgacctttaa ccagtaaaac gcaatatagg ttgccaatta acaaacaaca cagcccagaa 1320
   ctggtgtaaa gggcttgggt tttgtgcaat caactcattg actcacctcc cccaatgctc 1380
   catgaaccca tgaatccata gtacctccat cccaattcag agaggctcag aaacacgcaa 1440
   ttggcaaaag taaacaaagg tctcgaaacc ttaaggggtg cagtgttgca aagttaaaat 1500
   tacactgttg gatggtgttc cactcaaact gtggatggat cagacggttg cactaaacca 1560
   agattcaata tgctctacaa gtagcgtgcc atgaggtgta taatatcaca catgatcaga 1620
   gttaaggcct ggaaaaaaag tgtccgcggc tatgtaacac tgctgaatac agtgtgtgcc 1680
   gtttccacgg actggaatga atgcactcaa cacgttggta gctaacttcc ccaactattg 1740
   acttcacttt actgtagcta ctctaagtac gataatgaca tcatgaatca ccaactttat 1800
   cttccccacc gaaagtaaga gatgaaagag aggctaacag cttttgtaag aaaatcacaa 1860
   aatctactta ccgtattaca aaagtacaag tatcagtaac caggcgcgcc agctgcatta 1920
   atgaatcggc caacgcgcgg ggagaggcgg tttgcgtatt gggcgctctt ccgcttcctc 1980
   gctcactgac tcgctgcgct cggtcgttcg gctgcggcga gcggtatcag ctcactcaaa 2040
   ggcggtaata cggttatcca cagaatcagg ggataacgca ggaaagaaca tgtgagcaaa 2100
   aggccagcaa aaggccagga accgtaaaaa ggccgcgttg ctggcgtttt tccataggct 2160
   ccgcccccct gacgagcatc acaaaaatcg acgctcaagt cagaggtggc gaaacccgac 2220
   aggactataa agataccagg cgtttccccc tggaagctcc ctcgtgcgct ctcctgttcc 2280
   gaccctgccg cttaccggat acctgtccgc ctttctccct tcgggaagcg tggcgctttc 2340
   tcatagctca cgctgtaggt atctcagttc ggtgtaggtc gttcgctcca agctgggctg 2400
   tgtgcacgaa ccccccgttc agcccgaccg ctgcgcctta tccggtaact atcgtcttga 2460
   gtccaacccg gtaagacacg acttatcgcc actggcagca gccactggta acaggattag 2520
   cagagcgagg tatgtaggcg gtgctacaga gttcttgaag tggtggccta actacggcta 2580
   cactagaaga acagtatttg gtatctgcgc tctgctgaag ccagttacct tcggaaaaag 2640
   agttggtagc tcttgatccg gcaaacaaac caccgctggt agcggtggtt tttttgtttg 2700
   caagcagcag attacgcgca gaaaaaaagg atctcaagaa gatcctttga tcttttctac 2760
   ggggtctgac gctcagtgga acgaaaactc acgttaaggg attttggtca tgagattatc 2820
   aaaaaggatc ttcacctaga tccttttaaa ttaaaaatga agttttaaat caatctaaag 2880
   tatatatgag taaacttggt ctgacagtta ccaatgctta atcagtgagg cacctatctc 2940
   agcgatctgt ctatttcgtt catccatagt tgcctgactc cccgtcgtgt agataactac 3000
   gatacgggag ggcttaccat ctggccccag tgctgcaatg ataccgcgag acccacgctc 3060
   accggctcca gatttatcag caataaacca gccagccgga agggccgagc gcagaagtgg 3120
   tcctgcaact ttatccgcct ccatccagtc tattaattgt tgccgggaag ctagagtaag 3180
   tagttcgcca gttaatagtt tgcgcaacgt tgttgccatt gctacaggca tcgtggtgtc 3240
   acgctcgtcg tttggtatgg cttcattcag ctccggttcc caacgatcaa ggcgagttac 3300
   atgatccccc atgttgtgca aaaaagcggt tagctccttc ggtcctccga tcgttgtcag 3360
   aagtaagttg gccgcagtgt tatcactcat ggttatggca gcactgcata attctcttac 3420
   tgtcatgcca tccgtaagat gcttttctgt gactggtgag tactcaacca agtcattctg 3480
   agaatagtgt atgcggcgac cgagttgctc ttgcccggcg tcaatacggg ataataccgc 3540
   gccacatagc agaactttaa aagtgctcat cattggaaaa cgttcttcgg ggcgaaaact 3600
   ctcaaggatc ttaccgctgt tgagatccag ttcgatgtaa cccactcgtg cacccaactg 3660
   atcttcagca tcttttactt tcaccagcgt ttctgggtga gcaaaaacag gaaggcaaaa 3720
   tgccgcaaaa aagggaataa gggcgacacg gaaatgttga atactcatac tcttcctttt 3780
   tcaatattat tgaagcattt atcagggtta ttgtctcatg agcggataca tatttgaatg 3840
   tatttagaaa aataaacaaa taggggttcc gcgcacattt ccccgaaaag tgccacctga 3900
   tgcggtgtga aataccgcac agatgcgtaa ggagaaaata ccgcatcagg aaattgtaag 3960
   cgttaatatt ttgttaaaat tcgcgttaaa tttttgttaa atcagctcat tttttaacca 4020
   ataggccgaa atcggcaaaa tcccttataa atcaaaagaa tagaccgaga tagggttgag 4080
   tgttgttcca gtttggaaca agagtccact attaaagaac gtggactcca acgtcaaagg 4140
   gcgaaaaacc gtctatcagg gcgatggccc actacgtgaa ccatcaccct aatcaagttt 4200
   tttggggtcg aggtgccgta aagcactaaa tcggaaccct aaagggagcc cccgatttag 4260
   agcttgacgg ggaaagccgg cgaacgtggc gagaaaggaa gggaagaaag cgaaaggagc 4320
   gggcgctagg gcgctggcaa gtgtagcggt cacgctgcgc gtaaccacca cacccgccgc 4380
   gcttaatgcg ccgctacagg gcgcgtccat tcgccattca ggctgcgcaa ctgttgggaa 4440
   gggcgatcgg tgcgggcctc ttcgctatta cgccagctgg cgaaaggggg atgtgctgca 4500
   aggcgattaa gttgggtaac gccagggttt tcccagtcac gacgttgtaa aacgacggcc 4560
   agtgaattgt aatacgactc actatagggc gaattgggcc cgacgtcgca tgctatgtac 4620
   actgtgtgag tgtagagggc aatggtggtg acaactgagc tactatacac acacagcttg 4680
   tactggtata agttgtgagc tacagtacaa gtaatactac aaaattcagt atgtacaagt 4740
   atgccggtac ggcagtagca tgtatcaaag agagacagaa agaaggctgg aaagcgaagg 4800
   ccagctgagc gtgggaaccg cagtacgtgg aagtgccaaa cctgcgacag aatgacatgg 4860
   tcttgcactt tttaccggta ccggtacagt acgataccgg tacgcacttg ctacagtaga 4920
   tcatagacca ccagaataac cctgcattag cgccacgttg ccaagccctc aactttcttt 4980
   gcagctacac cttttacacc ttttaggctg ccaggaagag cataaatgaa ttgtacttgg 5040
   gggcacagaa tttgggttga agtgttcctg ggagagaatg tgcttggctg tatagttggg 5100
   tggctctcct tcatagtaga tgtgaggtta cagcacttgt agattgtgta cgcgttctgt 5160
   ttaaggtggt accagtatta tgggttgacg tgaaagaaag ggaggaggca ggggtacagc 5220
   aagagtgtgg gcgcacactc acacatgaag caagacctgt ttcgtcattt ctatttgttt 5280
   aacaaggacg acataatata cttaggacag catgggccga ccggcaggaa taatctgcaa 5340
   acacaatcgt attgtggcgt cccctctcga tcacgatagt tgctaagttt agcagtctga 5400
   gagtttggct gtgacgcaag accgagagag agacagagag ttgtcccagt ttgagtactc 5460
   gtaccgtact acagagtaaa tcgaaatcga gcgctcactt tctgcacttt gtgaaggcgc 5520
   agcattcaac atttgtgtcc agcccctttc cgtttgaagc gacttgacca cgaaatgttg 5580
   gaataaagct gtgttacata cgagacctac aagtagagtg tcggatatgc caactgaaca 5640
   ggttagatga aagttactca acaacaggag agcggacacc aacgtgcgaa aatgtacgga 5700
   tgaacccatt gtctgtccgc atagcaatct gttttcgcca gcagtgcttg tgtcgttgga 5760
   ttgctgccat tgtactattt gtactttgtt ccattaacac tcgcatcttg ctcaccgatt 5820
   agcagcactc ctgttgcctc actgggcttg tctgtttcag ggtggaatgc atgagcaatg 5880
   cgatcgtcaa ggggaggttg aaagcgcgta tggaataact gtggttctat agctgagggg 5940
   aggatatcga ggacagccac ggttcagtgg gggatttcgg gagctactca gtaacatgta 6000
   ctcttgtccg aacgccctgc tgatggcaaa tatgacaaaa cgcaacaatg tggagcaaca 6060
   tttccatttc gagagatatt acatgggttg cattggggcc tgggcatccc gtgcaataac 6120
   cgagaccatg tgaaggtgca cgatcgacac ttatccagcc agtcatacgt ggtgtcgcaa 6180
   gctaagcaac aatgggattc acgaatctat gtgggggaac gtgcacaggg gaaatgaaac 6240
   aaaacggagg agggggtctt tgaaaatgta gagaaatata acaaaaaatc acaaaaacgc 6300
   accaaaaata tgccaaaatc gtcaacagtg gaaatcacgc gcccctggct cgacttccca 6360
   ctgacatacc ccccctcaac caatatcata tccaccctag ccaatcgctt aattaagttg 6420
   cgacacatgt cttgatagta tcttgaattc tctctcttga gcttttccat aacaagttct 6480
   tctgcctcca ggaagtccat gggtggtttg atcatggttt tggtgtagtg gtagtgcagt 6540
   ggtggtattg tgactgggga tgtagttgag aataagtcat acacaagtca gctttcttcg 6600
   agcctcatat aagtataagt agttcaacgt attagcactg tacccagcat ctccgtatcg 6660
   agaaacacaa caacatgccc cattggacag atcatgcgga tacacaggtt gtgcagtatc 6720
   atacatactc gatcagacag gtcgtctgac catcatacaa gctgaacaag cgctccatac 6780
   ttgcacgctc tctatataca cagttaaatt acatatccat agtctaacct ctaacagtta 6840
   atcttctggt aagcctccca gccagccttc tggtatcgct tggcctcctc aataggatct 6900
   cggttctggc cgtacagacc tcggccgaca attatgatat ccgttccggt agacatgaca 6960
   tcctcaacag ttcggtactg ctgtccgaga gcgtctccct tgtcgtcaag acccaccccg 7020
   ggggtcagaa taagccagtc ctcagagtcg cccttaggtc ggttctgggc aatgaagcca 7080
   accacaaact cggggtcgga tcgggcaagc tcaatggtct gcttggagta ctcgccagtg 7140
   gccagagagc ccttgcaaga cagctcggcc agcatgagca gacctctggc cagcttctcg 7200
   ttgggagagg ggactaggaa ctccttgtac tgggagttct cgtagtcaga gacgtcctcc 7260
   ttcttctgtt cagagacagt ttcctcggca ccagctcgca ggccagcaat gattccggtt 7320
   ccgggtacac cgtgggcgtt ggtgatatcg gaccactcgg cgattcggtg acaccggtac 7380
   tggtgcttga cagtgttgcc aatatctgcg aactttctgt cctcgaacag gaagaaaccg 7440
   tgcttaagag caagttcctt gagggggagc acagtgccgg cgtaggtgaa gtcgtcaatg 7500
   atgtcgatat gggttttgat catgcacaca taaggtccga ccttatcggc aagctcaatg 7560
   agctccttgg tggtggtaac atccagagaa gcacacaggt tggttttctt ggctgccacg 7620
   agcttgagca ctcgagcggc aaaggcggac ttgtggacgt tagctcgagc ttcgtaggag 7680
   ggcattttgg tggtgaagag gagactgaaa taaatttagt ctgcagaact ttttatcgga 7740
   accttatctg gggcagtgaa gtatatgtta tggtaatagt tacgagttag ttgaacttat 7800
   agatagactg gactatacgg ctatcggtcc aaattagaaa gaacgtcaat ggctctctgg 7860
   gcgtcgcctt tgccgacaaa aatgtgatca tgatgaaagc cagcaatgac gttgcagctg 7920
   atattgttgt cggccaaccg cgccgaaaac gcagctgtca gacccacagc ctccaacgaa 7980
   gaatgtatcg tcaaagtgat ccaagcacac tcatagttgg agtcgtactc caaaggcggc 8040
   aatgacgagt cagacagata ctcgtcgacc ttttccttgg gaaccaccac cgtcagccct 8100
   tctgactcac gtattgtagc caccgacaca ggcaacagtc cgtggatagc agaatatgtc 8160
   ttgtcggtcc atttctcacc aactttaggc gtcaagtgaa tgttgcagaa gaagtatgtg 8220
   ccttcattga gaatcggtgt tgctgatttc aataaagtct tgagatcagt ttggccagtc 8280
   atgttgtggg gggtaattgg attgagttat cgcctacagt ctgtacaggt atactcgctg 8340
   cccactttat actttttgat tccgctgcac ttgaagcaat gtcgtttacc aaaagtgaga 8400
   atgctccaca gaacacaccc cagggtatgg ttgagcaaaa aataaacact ccgatacggg 8460
   gaatcgaacc ccggtctcca cggttctcaa gaagtattct tgatgagagc gtatcgatag 8520
   ttggagcaag ggagaaatgt agagtgtgaa agactcacta tggtccgggc ttatctcgac 8580
   caatagccaa agtctggagt ttctgagaga aaaaggcaag atacgtatgt aacaaagcga 8640
   cgcatggtac aataataccg gaggcatgta tcatagagag ttagtggttc gatgatggca 8700
   ctggtgcctg gtatgacttt atacggctga ctacatattt gtcctcagac atacaattac 8760
   agtcaagcac ttacccttgg acatctgtag gtaccccccg gccaagacga tctcagcgtg 8820
   tcgtatgtcg gattggcgta gctccctcgc tcgtcaattg gctcccatct actttcttct 8880
   gcttggctac acccagcatg tctgctatgg ctcgttttcg tgccttatct atcctcccag 8940
   tattaccaac tctaaatgac atgatgtgat tgggtctaca ctttcatatc agagataagg 9000
   agtagcacag ttgcataaaa agcccaactc taatcagctt cttcctttct tgtaattagt 9060
   acaaaggtga ttagcgaaat ctggaagctt agttggccct aaaaaaatca aaaaaagcaa 9120
   aaaacgaaaa acgaaaaacc acagttttga gaacagggag gtaacgaagg atcgtatata 9180
   tatatatata tatatatacc cacggatccc gagaccggcc tttgattctt ccctacaacc 9240
   aaccattctc accaccctaa ttcacaacca tgtctattgg ttcgtccaac cccgtgctct 9300
   tggctgcgat tcccttcgtc tacctgtttg tcctcccacg agtcctggct ttcctgcctc 9360
   agaaggctca gttcctggcc aaatgtattg tggtcctgat tgccacgctt atcatgtccg 9420
   ttgcaggctg cttcatctcg atcgtgtgcg ctcttctgga caagagatac gtcatcaatt 9480
   acgttgtgtc gcgattgttc tccttccttg ccgctcgacc gtgtggtgtg acctataaga 9540
   ttgttggtga ggaacacctc gataagtacc ctgctatcgt ggtctgtaac catcaatcct 9600
   ctatggatat gatggttttg ggacgagttt ttccaaagca ctgcgttgtc atggcgaaga 9660
   aggaactcct gtactttccc tttttgggaa tgtttatgaa actgagcaac gctatcttca 9720
   tcgaccggaa gaaccacaag aaagccatcg agtctaccac ccaagccgtg gcggacatga 9780
   agaagcacaa ctctggaatc tggattttcc cagagggcac ccggtctaga ctggacaagg 9840
   cagacctgct gcccttcaag aaaggtgcct ttcatcttgc aattcaggcc cagctcccta 9900
   ttctccccat tatctcgcag ggctattccc atatctacga ctcttcgaag cggtacttcc 9960
   ccggtggaga gctcgagatc agagtcctgg agcccattcc tacaactggc ctcactactg 10020
   atgatgtgaa cgacctgatg gacaagacac gaaaccttat gctcaagcac ttgaaggaga 10080
   tggattccca gtattcgtcg agcactgctg aaaatggatc cacgcacatc gacgccgata 10140
   ttgccaagtc tacagccacc agcattggca acactgacga cgcaattaca aaacgtcgta 10200
   cccctaagga ataagcggcc gcattgatga ttggaaacac acacatgggt tatatctagg 10260
   tgagagttag ttggacagtt atatattaaa tcagctatgc caacggtaac ttcattcatg 10320
   tcaacgagga accagtgact gcaagtaata tagaatttga ccaccttgcc attctcttgc 10380
   actcctttac tatatctcat ttatttctta tatacaaatc acttcttctt cccagcatcg 10440
   agctcggaaa cctcatgagc aataacatcg tggatctcgt caatagaggg ctttttggac 10500
   tccttgctgt tggccacctt gtccttgctg tttaaaccat catctaaggg cctcaaaact 10560
   acctcggaac tgctgcgctg atctggacac cacagaggtt ccgagcactt taggttgcac 10620
   caaatgtccc accaggtgca ggcagaaaac gctggaacag cgtgtacagt ttgtcttaac 10680
   aaaaagtgag ggcgctgagg tcgagcaggg tggtgtgact tgttatagcc tttagagctg 10740
   cgaaagcgcg tatggatttg gctcatcagg ccagattgag ggtctgtgga cacatgtcat 10800
   gttagtgtac ttcaatcgcc ccctggatat agccccgaca ataggccgtg gcctcatttt 10860
   tttgccttcc gcacatttcc attgctcggt acccacacct tgcttctcct gcacttgcca 10920
   accttaatac tggtttacat tgaccaacat cttacaagcg gggggcttgt ctagggtata 10980
   tataaacagt ggctctccca atcggttgcc agtctctttt ttcctttctt tccccacaga 11040
   ttcgaaatct aaactacaca tcacaccatg gtctccaacc acctgttcga cgccatgcga 11100
   gctgccgctc ccggagacgc acctttcatt cgaatcgaca acgctcggac ctggacttac 11160
   gatgacgcca ttgctctttc cggtcgaatc gctggagcta tggacgcact cggcattcga 11220
   cccggagaca gagttgccgt gcaggtcgag aagtctgccg aggcgttgat tctctacctg 11280
   gcctgtcttc gaaccggagc tgtctacctg cctctcaaca ctgcctacac cctggccgag 11340
   ctcgactact tcatcggcga tgccgaaccg cgtctggtgg tcgttgctcc cgcagctcga 11400
   ggtggcgtgg agacaattgc caagcgacac ggtgctatcg tcgaaaccct cgacgccgat 11460
   ggacgaggct ccttgctgga ccttgctaga gatgagcctg ccgactttgt cgatgcttcg 11520
   cgatctgccg acgatctggc tgctattctc tacacttccg gtacaaccgg acgatcgaag 11580
   ggtgccatgc ttactcatgg caatctgctc tccaacgctc tcaccttgcg agactattgg 11640
   agagttaccg cagacgatcg actcatccat gccttgccaa tctttcacac tcatggtctg 11700
   ttcgttgcta cgaacgtcac actgcttgca ggagcctcga tgtttctgct ctccaagttc 11760
   gatgccgacg aggtcgtttc tctcatgcca caggccacca tgcttatggg cgtgcccaca 11820
   ttctacgttc gattgctgca gagtcctcga ctcgagaagg gtgctgtggc cagcatcaga 11880
   ctgttcattt ctggatcagc tcccttgctt gccgaaaccc acgccgagtt tcatgctcgt 11940
   actggtcacg ccattctcga gcgatacggc atgacggaaa ccaacatgaa tacttccaac 12000
   ccctacgagg gcaagcgtat tgccggaacc gttggttttc ctctgcccga cgtcactgtg 12060
   cgagtcaccg atcccgccac cggtctcgtt cttccacctg aagagactgg catgatcgag 12120
   atcaagggac ccaacgtctt caagggctat tggcgaatgc ccgaaaagac cgctgccgag 12180
   tttaccgcag acggtttctt tatctctgga gatctcggca agatcgaccg agaaggttac 12240
   gttcacattg tgggacgagg caaggacctg gtcatttccg gtggctacaa catctatccc 12300
   aaagaggtcg aaggcgagat cgaccagatc gagggtgtgg tcgagtctgc tgtcattggt 12360
   gttcctcatc ccgatttcgg agaaggtgtc accgctgttg tcgtgtgcaa acctggtgcc 12420
   gttctcgacg aaaagaccat cgtgtctgct ctgcaggacc gtcttgcccg atacaagcaa 12480
   cccaagcgga ttatctttgc cgacgatctg cctcgaaaca ctatgggaaa ggttcagaag 12540
   aacattcttc gacagcaata cgccgatctc tacaccagac gataagcggc cgcatgagaa 12600
   gataaatata taaatacatt gagatattaa atgcgctaga ttagagagcc tcatactgct 12660
   cggagagaag ccaagacgag tactcaaagg ggattacacc atccatatcc acagacacaa 12720
   gctggggaaa ggttctatat acactttccg gaataccgta gtttccgatg ttatcaatgg 12780
   gggcagccag gatttcaggc acttcggtgt ctcggggtga aatggcgttc ttggcctcca 12840
   tcaagtcgta ccatgtcttc atttgcctgt caaagtaaaa cagaagcaga tgaagaatga 12900
   acttgaagtg aaggaattta aatagttgga gcaagggaga aatgtagagt gtgaaagact 12960
   cactatggtc cgggcttatc tcgaccaata gccaaagtct ggagtttctg agagaaaaag 13020
   gcaagatacg tatgtaacaa agcgacgcat ggtacaataa taccggaggc atgtatcata 13080
   gagagttagt ggttcgatga tggcactggt gcctggtatg actttatacg gctgactaca 13140
   tatttgtcct cagacataca attacagtca agcacttacc cttggacatc tgtaggtacc 13200
   ccccggccaa gacgatctca gcgtgtcgta tgtcggattg gcgtagctcc ctcgctcgtc 13260
   aattggctcc catctacttt cttctgcttg gctacaccca gcatgtctgc tatggctcgt 13320
   tttcgtgcct tatctatcct cccagtatta ccaactctaa atgacatgat gtgattgggt 13380
   ctacactttc atatcagaga taaggagtag cacagttgca taaaaagccc aactctaatc 13440
   agcttcttcc tttcttgtaa ttagtacaaa ggtgattagc gaaatctgga agcttagttg 13500
   gccctaaaaa aatcaaaaaa agcaaaaaac gaaaaacgaa aaaccacagt tttgagaaca 13560
   gggaggtaac gaaggatcgt atatatatat atatatatat atacccacgg atcccgagac 13620
   cggcctttga ttcttcccta caaccaacca ttctcaccac cctaattcac aaccatggtg 13680
   aaggcttctc gacaggctct gcccctcgtc atcgacggaa aggtgtacga cgtctccgct 13740
   tgggtgaact tccaccctgg tggagctgaa atcattgaga actaccaggg acgagatgct 13800
   actgacgcct tcatggttat gcactctcag gaagccttcg acaagctcaa gcgaatgccc 13860
   aagatcaacc aggcttccga gctgcctccc caggctgccg tcaacgaagc tcaggaggat 13920
   ttccgaaagc tccgagaaga gctgatcgcc actggcatgt ttgacgcctc tcccctctgg 13980
   tactcgtaca agatcttgac caccctgggt cttggcgtgc ttgccttctt catgctggtc 14040
   cagtaccacc tgtacttcat tggtgctctc gtgctcggta tgcactacca gcaaatggga 14100
   tggctgtctc atgacatctg ccaccaccag accttcaaga accgaaactg gaataacgtc 14160
   ctgggtctgg tctttggcaa cggactccag ggcttctccg tgacctggtg gaaggacaga 14220
   cacaacgccc atcattctgc taccaacgtt cagggtcacg atcccgacat tgataacctg 14280
   cctctgctcg cctggtccga ggacgatgtc actcgagctt ctcccatctc ccgaaagctc 14340
   attcagttcc aacagtacta tttcctggtc atctgtattc tcctgcgatt catctggtgt 14400
   ttccagtctg tgctgaccgt tcgatccctc aaggaccgag acaaccagtt ctaccgatct 14460
   cagtacaaga aagaggccat tggactcgct ctgcactgga ctctcaagac cctgttccac 14520
   ctcttcttta tgccctccat cctgacctcg atgctggtgt tctttgtttc cgagctcgtc 14580
   ggtggcttcg gaattgccat cgtggtcttc atgaaccact accctctgga gaagatcggt 14640
   gattccgtct gggacggaca tggcttctct gtgggtcaga tccatgagac catgaacatt 14700
   cgacgaggca tcattactga ctggttcttt ggaggcctga actaccagat cgagcaccat 14760
   ctctggccca ccctgcctcg acacaacctc actgccgttt cctaccaggt ggaacagctg 14820
   tgccagaagc acaacctccc ctaccgaaac cctctgcccc atgaaggtct cgtcatcctg 14880
   ctccgatacc tgtcccagtt cgctcgaatg gccgagaagc agcccggtgc caaggctcag 14940
   taagcggccg caagtgtgga tggggaagtg agtgcccggt tctgtgtgca caattggcaa 15000
   tccaagatgg atggattcaa cacagggata tagcgagcta cgtggtggtg cgaggatata 15060
   gcaacggata tttatgtttg acacttgaga atgtacgata caagcactgt ccaagtacaa 15120
   tactaaacat actgtacata ctcatactcg tacccgggca acggtttcac ttgagtgcag 15180
   tggctagtgc tcttactcgt acagtgtgca atactgcgta tcatagtctt tgatgtatat 15240
   cgtattcatt catgttagtt gc 15262
<210> 137
   <211> 13075
   <212> DNA
   <213> Artificial Sequence
<220>
   <223> Plasmid pZK16-ML
<400> 137
   gtacgcattc ttgacttgct ccccagttgc cctttttggt cataactcag ccccgactct      60
   ctccccaaag aggagatgga cttacaatag atacgagaca acagatattt tcggccacct     120
   ggtgcaattg gtctgtttta ttacccccaa taatatccac agcgtccgaa tcagctcgac     180
   aagctctttc tcagcatact gatcttaccc catacccccc agacagcggt gccatatcca     240
   tacccgcact acctgagaca gtaaactcag ggcaaggagc ggtcttggtc catcgcgacg     300
   ctgtaagctt tgcctttgcg ttttgtgatc caccacacgg cagtcaaggt ctcggttgaa     360
   agatgggtct ggcaatgtgg attgctgaat cattgacata taaatacgag tgatcctgcc     420
   atttcccaat ctttagaaat catcttttct tcacacatac aaaaaacaca catcacaatg     480
   cagttctccg ccgccgccgt ccttgccctc gccgccttcg tctctgccca ggccgcctcc     540
   gctgcttctg aagctggtgg tgccgttgtc tccggtgcct ccggtgccgc tgccgccgct     600
   tcttctgctg tcattggctc cgagtctcag gccgcttctt ctgctgcctc ttctgctgct     660
   gagtctgctt cttccgctgc ctctgagtcc gcctcctccg cttccgcctc cgagtccaag     720
   gcctctgagt ccgcttccaa gtccgcctcc aaggcttctg agtctgcttc caaggccgag     780
   tcttctgctg ccaaggcttc ctccgctgct ggttctgctt cttccgccgc tgcctctgcc 840
   accaagtctg gtgatgcctc cggctcttcc aagccttccg gctctgcctc ctctaccggt 900
   gacaagtccg gtgctggtat tgttgccccc gctgttggtg ccatcgttgt cggtgctctt 960
   gctcagctca tctaagcggt ctttgtacaa ctacccactt aatatttata ttatgatata 1020
   gactgcaata ctattgcatg tgtaatgctt cgtttgaaag tcgtagtccg gccattcatg 1080
   gcagtgtatg gcagtattgc aaccaattgg cttaataaca tatacaaagt ggcaattctg 1140
   tgttcgaacc atcacacaac ccaacatatt tgacattgct acaagtattc gacttgttct 1200
   tcggccgcac gacatgcatt gctagcagca cacttgcaga agcttggcca atggttaggt 1260
   tgacctttaa ccagtaaaac gcaatatagg ttgccaatta acaaacaaca cagcccagaa 1320
   ctggtgtaaa gggcttgggt tttgtgcaat caactcattg actcacctcc cccaatgctc 1380
   catgaaccca tgaatccata gtacctccat cccaattcag agaggctcag aaacacgcaa 1440
   ttggcaaaag taaacaaagg tctcgaaacc ttaaggggtg cagtgttgca aagttaaaat 1500
   tacactgttg gatggtgttc cactcaaact gtggatggat cagacggttg cactaaacca 1560
   agattcaata tgctctacaa gtagcgtgcc atgaggtgta taatatcaca catgatcaga 1620
   gttaaggcct ggaaaaaaag tgtccgcggc tatgtaacac tgctgaatac agtgtgtgcc 1680
   gtttccacgg actggaatga atgcactcaa cacgttggta gctaacttcc ccaactattg 1740
   acttcacttt actgtagcta ctctaagtac gataatgaca tcatgaatca ccaactttat 1800
   cttccccacc gaaagtaaga gatgaaagag aggctaacag cttttgtaag aaaatcacaa 1860
   aatctactta ccgtattaca aaagtacaag tatcagtaac caggcgcgcc agctgcatta 1920
   atgaatcggc caacgcgcgg ggagaggcgg tttgcgtatt gggcgctctt ccgcttcctc 1980
   gctcactgac tcgctgcgct cggtcgttcg gctgcggcga gcggtatcag ctcactcaaa 2040
   ggcggtaata cggttatcca cagaatcagg ggataacgca ggaaagaaca tgtgagcaaa 2100
   aggccagcaa aaggccagga accgtaaaaa ggccgcgttg ctggcgtttt tccataggct 2160
   ccgcccccct gacgagcatc acaaaaatcg acgctcaagt cagaggtggc gaaacccgac 2220
   aggactataa agataccagg cgtttccccc tggaagctcc ctcgtgcgct ctcctgttcc 2280
   gaccctgccg cttaccggat acctgtccgc ctttctccct tcgggaagcg tggcgctttc 2340
   tcatagctca cgctgtaggt atctcagttc ggtgtaggtc gttcgctcca agctgggctg 2400
   tgtgcacgaa ccccccgttc agcccgaccg ctgcgcctta tccggtaact atcgtcttga 2460
   gtccaacccg gtaagacacg acttatcgcc actggcagca gccactggta acaggattag 2520
   cagagcgagg tatgtaggcg gtgctacaga gttcttgaag tggtggccta actacggcta 2580
   cactagaaga acagtatttg gtatctgcgc tctgctgaag ccagttacct tcggaaaaag 2640
   agttggtagc tcttgatccg gcaaacaaac caccgctggt agcggtggtt tttttgtttg 2700
   caagcagcag attacgcgca gaaaaaaagg atctcaagaa gatcctttga tcttttctac 2760
   ggggtctgac gctcagtgga acgaaaactc acgttaaggg attttggtca tgagattatc 2820
   aaaaaggatc ttcacctaga tccttttaaa ttaaaaatga agttttaaat caatctaaag 2880
   tatatatgag taaacttggt ctgacagtta ccaatgctta atcagtgagg cacctatctc 2940
   agcgatctgt ctatttcgtt catccatagt tgcctgactc cccgtcgtgt agataactac 3000
   gatacgggag ggcttaccat ctggccccag tgctgcaatg ataccgcgag acccacgctc 3060
   accggctcca gatttatcag caataaacca gccagccgga agggccgagc gcagaagtgg 3120
   tcctgcaact ttatccgcct ccatccagtc tattaattgt tgccgggaag ctagagtaag 3180
   tagttcgcca gttaatagtt tgcgcaacgt tgttgccatt gctacaggca tcgtggtgtc 3240
   acgctcgtcg tttggtatgg cttcattcag ctccggttcc caacgatcaa ggcgagttac 3300
   atgatccccc atgttgtgca aaaaagcggt tagctccttc ggtcctccga tcgttgtcag 3360
   aagtaagttg gccgcagtgt tatcactcat ggttatggca gcactgcata attctcttac 3420
   tgtcatgcca tccgtaagat gcttttctgt gactggtgag tactcaacca agtcattctg 3480
   agaatagtgt atgcggcgac cgagttgctc ttgcccggcg tcaatacggg ataataccgc 3540
   gccacatagc agaactttaa aagtgctcat cattggaaaa cgttcttcgg ggcgaaaact 3600
   ctcaaggatc ttaccgctgt tgagatccag ttcgatgtaa cccactcgtg cacccaactg 3660
   atcttcagca tcttttactt tcaccagcgt ttctgggtga gcaaaaacag gaaggcaaaa 3720
   tgccgcaaaa aagggaataa gggcgacacg gaaatgttga atactcatac tcttcctttt 3780
   tcaatattat tgaagcattt atcagggtta ttgtctcatg agcggataca tatttgaatg 3840
   tatttagaaa aataaacaaa taggggttcc gcgcacattt ccccgaaaag tgccacctga 3900
   tgcggtgtga aataccgcac agatgcgtaa ggagaaaata ccgcatcagg aaattgtaag 3960
   cgttaatatt ttgttaaaat tcgcgttaaa tttttgttaa atcagctcat tttttaacca 4020
   ataggccgaa atcggcaaaa tcccttataa atcaaaagaa tagaccgaga tagggttgag 4080
   tgttgttcca gtttggaaca agagtccact attaaagaac gtggactcca acgtcaaagg 4140
   gcgaaaaacc gtctatcagg gcgatggccc actacgtgaa ccatcaccct aatcaagttt 4200
   tttggggtcg aggtgccgta aagcactaaa tcggaaccct aaagggagcc cccgatttag 4260
   agcttgacgg ggaaagccgg cgaacgtggc gagaaaggaa gggaagaaag cgaaaggagc 4320
   gggcgctagg gcgctggcaa gtgtagcggt cacgctgcgc gtaaccacca cacccgccgc 4380
   gcttaatgcg ccgctacagg gcgcgtccat tcgccattca ggctgcgcaa ctgttgggaa 4440
   gggcgatcgg tgcgggcctc ttcgctatta cgccagctgg cgaaaggggg atgtgctgca 4500
   aggcgattaa gttgggtaac gccagggttt tcccagtcac gacgttgtaa aacgacggcc 4560
   agtgaattgt aatacgactc actatagggc gaattgggcc cgacgtcgca tgctatgtac 4620
   actgtgtgag tgtagagggc aatggtggtg acaactgagc tactatacac acacagcttg 4680
   tactggtata agttgtgagc tacagtacaa gtaatactac aaaattcagt atgtacaagt 4740
   atgccggtac ggcagtagca tgtatcaaag agagacagaa agaaggctgg aaagcgaagg 4800
   ccagctgagc gtgggaaccg cagtacgtgg aagtgccaaa cctgcgacag aatgacatgg 4860
   tcttgcactt tttaccggta ccggtacagt acgataccgg tacgcacttg ctacagtaga 4920
   tcatagacca ccagaataac cctgcattag cgccacgttg ccaagccctc aactttcttt 4980
   gcagctacac cttttacacc ttttaggctg ccaggaagag cataaatgaa ttgtacttgg 5040
   gggcacagaa tttgggttga agtgttcctg ggagagaatg tgcttggctg tatagttggg 5100
   tggctctcct tcatagtaga tgtgaggtta cagcacttgt agattgtgta cgcgttctgt 5160
   ttaaggtggt accagtatta tgggttgacg tgaaagaaag ggaggaggca ggggtacagc 5220
   aagagtgtgg gcgcacactc acacatgaag caagacctgt ttcgtcattt ctatttgttt 5280
   aacaaggacg acataatata cttaggacag catgggccga ccggcaggaa taatctgcaa 5340
   acacaatcgt attgtggcgt cccctctcga tcacgatagt tgctaagttt agcagtctga 5400
   gagtttggct gtgacgcaag accgagagag agacagagag ttgtcccagt ttgagtactc 5460
   gtaccgtact acagagtaaa tcgaaatcga gcgctcactt tctgcacttt gtgaaggcgc 5520
   agcattcaac atttgtgtcc agcccctttc cgtttgaagc gacttgacca cgaaatgttg 5580
   gaataaagct gtgttacata cgagacctac aagtagagtg tcggatatgc caactgaaca 5640
   ggttagatga aagttactca acaacaggag agcggacacc aacgtgcgaa aatgtacgga 5700
   tgaacccatt gtctgtccgc atagcaatct gttttcgcca gcagtgcttg tgtcgttgga 5760
   ttgctgccat tgtactattt gtactttgtt ccattaacac tcgcatcttg ctcaccgatt 5820
   agcagcactc ctgttgcctc actgggcttg tctgtttcag ggtggaatgc atgagcaatg 5880
   cgatcgtcaa ggggaggttg aaagcgcgta tggaataact gtggttctat agctgagggg 5940
   aggatatcga ggacagccac ggttcagtgg gggatttcgg gagctactca gtaacatgta 6000
   ctcttgtccg aacgccctgc tgatggcaaa tatgacaaaa cgcaacaatg tggagcaaca 6060
   tttccatttc gagagatatt acatgggttg cattggggcc tgggcatccc gtgcaataac 6120
   cgagaccatg tgaaggtgca cgatcgacac ttatccagcc agtcatacgt ggtgtcgcaa 6180
   gctaagcaac aatgggattc acgaatctat gtgggggaac gtgcacaggg gaaatgaaac 6240
   aaaacggagg agggggtctt tgaaaatgta gagaaatata acaaaaaatc acaaaaacgc 6300
   accaaaaata tgccaaaatc gtcaacagtg gaaatcacgc gcccctggct cgacttccca 6360
   ctgacatacc ccccctcaac caatatcata tccaccctag ccaatcgctt aattaagttg 6420
   cgacacatgt cttgatagta tcttgaattc tctctcttga gcttttccat aacaagttct 6480
   tctgcctcca ggaagtccat gggtggtttg atcatggttt tggtgtagtg gtagtgcagt 6540
   ggtggtattg tgactgggga tgtagttgag aataagtcat acacaagtca gctttcttcg 6600
   agcctcatat aagtataagt agttcaacgt attagcactg tacccagcat ctccgtatcg 6660
   agaaacacaa caacatgccc cattggacag atcatgcgga tacacaggtt gtgcagtatc 6720
   atacatactc gatcagacag gtcgtctgac catcatacaa gctgaacaag cgctccatac 6780
   ttgcacgctc tctatataca cagttaaatt acatatccat agtctaacct ctaacagtta 6840
   atcttctggt aagcctccca gccagccttc tggtatcgct tggcctcctc aataggatct 6900
   cggttctggc cgtacagacc tcggccgaca attatgatat ccgttccggt agacatgaca 6960
   tcctcaacag ttcggtactg ctgtccgaga gcgtctccct tgtcgtcaag acccaccccg 7020
   ggggtcagaa taagccagtc ctcagagtcg cccttaggtc ggttctgggc aatgaagcca 7080
   accacaaact cggggtcgga tcgggcaagc tcaatggtct gcttggagta ctcgccagtg 7140
   gccagagagc ccttgcaaga cagctcggcc agcatgagca gacctctggc cagcttctcg 7200
   ttgggagagg ggactaggaa ctccttgtac tgggagttct cgtagtcaga gacgtcctcc 7260
   ttcttctgtt cagagacagt ttcctcggca ccagctcgca ggccagcaat gattccggtt 7320
   ccgggtacac cgtgggcgtt ggtgatatcg gaccactcgg cgattcggtg acaccggtac 7380
   tggtgcttga cagtgttgcc aatatctgcg aactttctgt cctcgaacag gaagaaaccg 7440
   tgcttaagag caagttcctt gagggggagc acagtgccgg cgtaggtgaa gtcgtcaatg 7500
   atgtcgatat gggttttgat catgcacaca taaggtccga ccttatcggc aagctcaatg 7560
   agctccttgg tggtggtaac atccagagaa gcacacaggt tggttttctt ggctgccacg 7620
   agcttgagca ctcgagcggc aaaggcggac ttgtggacgt tagctcgagc ttcgtaggag 7680
   ggcattttgg tggtgaagag gagactgaaa taaatttagt ctgcagaact ttttatcgga 7740
   accttatctg gggcagtgaa gtatatgtta tggtaatagt tacgagttag ttgaacttat 7800
   agatagactg gactatacgg ctatcggtcc aaattagaaa gaacgtcaat ggctctctgg 7860
   gcgtcgcctt tgccgacaaa aatgtgatca tgatgaaagc cagcaatgac gttgcagctg 7920
   atattgttgt cggccaaccg cgccgaaaac gcagctgtca gacccacagc ctccaacgaa 7980
   gaatgtatcg tcaaagtgat ccaagcacac tcatagttgg agtcgtactc caaaggcggc 8040
   aatgacgagt cagacagata ctcgtcgacc ttttccttgg gaaccaccac cgtcagccct 8100
   tctgactcac gtattgtagc caccgacaca ggcaacagtc cgtggatagc agaatatgtc 8160
   ttgtcggtcc atttctcacc aactttaggc gtcaagtgaa tgttgcagaa gaagtatgtg 8220
   ccttcattga gaatcggtgt tgctgatttc aataaagtct tgagatcagt ttggccagtc 8280
   atgttgtggg gggtaattgg attgagttat cgcctacagt ctgtacaggt atactcgctg 8340
   cccactttat actttttgat tccgctgcac ttgaagcaat gtcgtttacc aaaagtgaga 8400
   atgctccaca gaacacaccc cagggtatgg ttgagcaaaa aataaacact ccgatacggg 8460
   gaatcgaacc ccggtctcca cggttctcaa gaagtattct tgatgagagc gtatcgatag 8520
   ttggagcaag ggagaaatgt agagtgtgaa agactcacta tggtccgggc ttatctcgac 8580
   caatagccaa agtctggagt ttctgagaga aaaaggcaag atacgtatgt aacaaagcga 8640
   cgcatggtac aataataccg gaggcatgta tcatagagag ttagtggttc gatgatggca 8700
   ctggtgcctg gtatgacttt atacggctga ctacatattt gtcctcagac atacaattac 8760
   agtcaagcac ttacccttgg acatctgtag gtaccccccg gccaagacga tctcagcgtg 8820
   tcgtatgtcg gattggcgta gctccctcgc tcgtcaattg gctcccatct actttcttct 8880
   gcttggctac acccagcatg tctgctatgg ctcgttttcg tgccttatct atcctcccag 8940
   tattaccaac tctaaatgac atgatgtgat tgggtctaca ctttcatatc agagataagg 9000
   agtagcacag ttgcataaaa agcccaactc taatcagctt cttcctttct tgtaattagt 9060
   acaaaggtga ttagcgaaat ctggaagctt agttggccct aaaaaaatca aaaaaagcaa 9120
   aaaacgaaaa acgaaaaacc acagttttga gaacagggag gtaacgaagg atcgtatata 9180
   tatatatata tatatatacc cacggatccc gagaccggcc tttgattctt ccctacaacc 9240
   aaccattctc accaccctaa ttcacaacca tgtctattgg ttcgtccaac cccgtgctct 9300
   tggctgcgat tcccttcgtc tacctgtttg tcctcccacg agtcctggct ttcctgcctc 9360
   agaaggctca gttcctggcc aaatgtattg tggtcctgat tgccacgctt atcatgtccg 9420
   ttgcaggctg cttcatctcg atcgtgtgcg ctcttctgga caagagatac gtcatcaatt 9480
   acgttgtgtc gcgattgttc tccttccttg ccgctcgacc gtgtggtgtg acctataaga 9540
   ttgttggtga ggaacacctc gataagtacc ctgctatcgt ggtctgtaac catcaatcct 9600
   ctatggatat gatggttttg ggacgagttt ttccaaagca ctgcgttgtc atggcgaaga 9660
   aggaactcct gtactttccc tttttgggaa tgtttatgaa actgagcaac gctatcttca 9720
   tcgaccggaa gaaccacaag aaagccatcg agtctaccac ccaagccgtg gcggacatga 9780
   agaagcacaa ctctggaatc tggattttcc cagagggcac ccggtctaga ctggacaagg 9840
   cagacctgct gcccttcaag aaaggtgcct ttcatcttgc aattcaggcc cagctcccta 9900
   ttctccccat tatctcgcag ggctattccc atatctacga ctcttcgaag cggtacttcc 9960
   ccggtggaga gctcgagatc agagtcctgg agcccattcc tacaactggc ctcactactg 10020
   atgatgtgaa cgacctgatg gacaagacac gaaaccttat gctcaagcac ttgaaggaga 10080
   tggattccca gtattcgtcg agcactgctg aaaatggatc cacgcacatc gacgccgata 10140
   ttgccaagtc tacagccacc agcattggca acactgacga cgcaattaca aaacgtcgta 10200
   cccctaagga ataagcggcc gcattgatga ttggaaacac acacatgggt tatatctagg 10260
   tgagagttag ttggacagtt atatattaaa tcagctatgc caacggtaac ttcattcatg 10320
   tcaacgagga accagtgact gcaagtaata tagaatttga ccaccttgcc attctcttgc 10380
   actcctttac tatatctcat ttatttctta tatacaaatc acttcttctt cccagcatcg 10440
   agctcggaaa cctcatgagc aataacatcg tggatctcgt caatagaggg ctttttggac 10500
   tccttgctgt tggccacctt gtccttgctg tttaaaccat catctaaggg cctcaaaact 10560
   acctcggaac tgctgcgctg atctggacac cacagaggtt ccgagcactt taggttgcac 10620
   caaatgtccc accaggtgca ggcagaaaac gctggaacag cgtgtacagt ttgtcttaac 10680
   aaaaagtgag ggcgctgagg tcgagcaggg tggtgtgact tgttatagcc tttagagctg 10740
   cgaaagcgcg tatggatttg gctcatcagg ccagattgag ggtctgtgga cacatgtcat 10800
   gttagtgtac ttcaatcgcc ccctggatat agccccgaca ataggccgtg gcctcatttt 10860
   tttgccttcc gcacatttcc attgctcggt acccacacct tgcttctcct gcacttgcca 10920
   accttaatac tggtttacat tgaccaacat cttacaagcg gggggcttgt ctagggtata 10980
   tataaacagt ggctctccca atcggttgcc agtctctttt ttcctttctt tccccacaga 11040
   ttcgaaatct aaactacaca tcacaccatg gtctccaacc acctgttcga cgccatgcga 11100
   gctgccgctc ccggagacgc acctttcatt cgaatcgaca acgctcggac ctggacttac 11160
   gatgacgcca ttgctctttc cggtcgaatc gctggagcta tggacgcact cggcattcga 11220
   cccggagaca gagttgccgt gcaggtcgag aagtctgccg aggcgttgat tctctacctg 11280
   gcctgtcttc gaaccggagc tgtctacctg cctctcaaca ctgcctacac cctggccgag 11340
   ctcgactact tcatcggcga tgccgaaccg cgtctggtgg tcgttgctcc cgcagctcga 11400
   ggtggcgtgg agacaattgc caagcgacac ggtgctatcg tcgaaaccct cgacgccgat 11460
   ggacgaggct ccttgctgga ccttgctaga gatgagcctg ccgactttgt cgatgcttcg 11520
   cgatctgccg acgatctggc tgctattctc tacacttccg gtacaaccgg acgatcgaag 11580
   ggtgccatgc ttactcatgg caatctgctc tccaacgctc tcaccttgcg agactattgg 11640
   agagttaccg cagacgatcg actcatccat gccttgccaa tctttcacac tcatggtctg 11700
   ttcgttgcta cgaacgtcac actgcttgca ggagcctcga tgtttctgct ctccaagttc 11760
   gatgccgacg aggtcgtttc tctcatgcca caggccacca tgcttatggg cgtgcccaca 11820
   ttctacgttc gattgctgca gagtcctcga ctcgagaagg gtgctgtggc cagcatcaga 11880
   ctgttcattt ctggatcagc tcccttgctt gccgaaaccc acgccgagtt tcatgctcgt 11940
   actggtcacg ccattctcga gcgatacggc atgacggaaa ccaacatgaa tacttccaac 12000
   ccctacgagg gcaagcgtat tgccggaacc gttggttttc ctctgcccga cgtcactgtg 12060
   cgagtcaccg atcccgccac cggtctcgtt cttccacctg aagagactgg catgatcgag 12120
   atcaagggac ccaacgtctt caagggctat tggcgaatgc ccgaaaagac cgctgccgag 12180
   tttaccgcag acggtttctt tatctctgga gatctcggca agatcgaccg agaaggttac 12240
   gttcacattg tgggacgagg caaggacctg gtcatttccg gtggctacaa catctatccc 12300
   aaagaggtcg aaggcgagat cgaccagatc gagggtgtgg tcgagtctgc tgtcattggt 12360
   gttcctcatc ccgatttcgg agaaggtgtc accgctgttg tcgtgtgcaa acctggtgcc 12420
   gttctcgacg aaaagaccat cgtgtctgct ctgcaggacc gtcttgcccg atacaagcaa 12480
   cccaagcgga ttatctttgc cgacgatctg cctcgaaaca ctatgggaaa ggttcagaag 12540
   aacattcttc gacagcaata cgccgatctc tacaccagac gataagcggc cgcatgagaa 12600
   gataaatata taaatacatt gagatattaa atgcgctaga ttagagagcc tcatactgct 12660
   cggagagaag ccaagacgag tactcaaagg ggattacacc atccatatcc acagacacaa 12720
   gctggggaaa ggttctatat acactttccg gaataccgta gtttccgatg ttatcaatgg 12780
   gggcagccag gatttcaggc acttcggtgt ctcggggtga aatggcgttc ttggcctcca 12840
   tcaagtcgta ccatgtcttc atttgcctgt caaagtaaaa cagaagcaga tgaagaatga 12900
   acttgaagtg aaggaattta aatgtaacga aactgaaatt tgaccagata ttgtgtccgc 12960
   ggtggagctc cagcttttgt tccctttagt gagggttaat ttcgagcttg gcgtaatcat 13020
   ggtcatagct gtttcctgtg tgaaattgtt atccgctcac aagcttccac acaac 13075
<210> 138
   <211> 15799
   <212> DNA
   <213> Artificial Sequence
<220>
   <223> Plasmid pZKL2-5m89C
<400> 138
   gtacgttatc atttgaacag tgaaaggcta cagtaacaga agcagttgta aacttcattc 60
   cgttgattct gtactacagt accccactac gccgcttccg ctgacactgt tcaacccaaa 120
   aactacatct gcgtgcgctg tgtaaggcta tcatcagata catactgtag attctgtaga 180
   tgcgaacctg cttgtatcat atacatcccc ctccccctga cctgcacaag caagcaatgt 240
   gacattgata ttgctgctta tctagtgccg aggatgtgaa agccgagact caaacatttc 300
   ttttactctc ttgttcctga ccagacctgg cggagattac gccagtatga ttcttgcagg 360
   tctgagacaa gcctggaaca gccaacattt atttttcgaa gcgagaaaca tgccacaccc 420
   cggcacgttc agagatgcat atgatttgtt tttcgagtaa cagtaccccc cccccccccc 480
   ccaatgaaac cagtattact cacaccatcc tcattcaaag cgttacactg attacgcgcc 540
   catcaacgac agcatgaggg gactgctgat ctgatctaat caaatgacta caaaaatcgc 600
   aataatgaag agcaaacgac aaaaaagaaa caggttaacc aatcccgctt caatgtctca 660
   ccacaatcca gcactgtttc tcattacctc ctccctctaa tttcagagtt gcatcagggt 720
   ccttgatggc gcgccagctg cattaatgaa tcggccaacg cgcggggaga ggcggtttgc 780
   gtattgggcg ctcttccgct tcctcgctca ctgactcgct gcgctcggtc gttcggctgc 840
   ggcgagcggt atcagctcac tcaaaggcgg taatacggtt atccacagaa tcaggggata 900
   acgcaggaaa gaacatgtga gcaaaaggcc agcaaaaggc caggaaccgt aaaaaggccg 960
   cgttgctggc gtttttccat aggctccgcc cccctgacga gcatcacaaa aatcgacgct 1020
   caagtcagag gtggcgaaac ccgacaggac tataaagata ccaggcgttt ccccctggaa 1080
   gctccctcgt gcgctctcct gttccgaccc tgccgcttac cggatacctg tccgcctttc 1140
   tcccttcggg aagcgtggcg ctttctcata gctcacgctg taggtatctc agttcggtgt 1200
   aggtcgttcg ctccaagctg ggctgtgtgc acgaaccccc cgttcagccc gaccgctgcg 1260
   ccttatccgg taactatcgt cttgagtcca acccggtaag acacgactta tcgccactgg 1320
   cagcagccac tggtaacagg attagcagag cgaggtatgt aggcggtgct acagagttct 1380
   tgaagtggtg gcctaactac ggctacacta gaagaacagt atttggtatc tgcgctctgc 1440
   tgaagccagt taccttcgga aaaagagttg gtagctcttg atccggcaaa caaaccaccg 1500
   ctggtagcgg tggttttttt gtttgcaagc agcagattac gcgcagaaaa aaaggatctc 1560
   aagaagatcc tttgatcttt tctacggggt ctgacgctca gtggaacgaa aactcacgtt 1620
   aagggatttt ggtcatgaga ttatcaaaaa ggatcttcac ctagatcctt ttaaattaaa 1680
   aatgaagttt taaatcaatc taaagtatat atgagtaaac ttggtctgac agttaccaat 1740
   gcttaatcag tgaggcacct atctcagcga tctgtctatt tcgttcatcc atagttgcct 1800
   gactccccgt cgtgtagata actacgatac gggagggctt accatctggc cccagtgctg 1860
   caatgatacc gcgagaccca cgctcaccgg ctccagattt atcagcaata aaccagccag 1920
   ccggaagggc cgagcgcaga agtggtcctg caactttatc cgcctccatc cagtctatta 1980
   attgttgccg ggaagctaga gtaagtagtt cgccagttaa tagtttgcgc aacgttgttg 2040
   ccattgctac aggcatcgtg gtgtcacgct cgtcgtttgg tatggcttca ttcagctccg 2100
   gttcccaacg atcaaggcga gttacatgat cccccatgtt gtgcaaaaaa gcggttagct 2160
   ccttcggtcc tccgatcgtt gtcagaagta agttggccgc agtgttatca ctcatggtta 2220
   tggcagcact gcataattct cttactgtca tgccatccgt aagatgcttt tctgtgactg 2280
   gtgagtactc aaccaagtca ttctgagaat agtgtatgcg gcgaccgagt tgctcttgcc 2340
   cggcgtcaat acgggataat accgcgccac atagcagaac tttaaaagtg ctcatcattg 2400
   gaaaacgttc ttcggggcga aaactctcaa ggatcttacc gctgttgaga tccagttcga 2460
   tgtaacccac tcgtgcaccc aactgatctt cagcatcttt tactttcacc agcgtttctg 2520
   ggtgagcaaa aacaggaagg caaaatgccg caaaaaaggg aataagggcg acacggaaat 2580
   gttgaatact catactcttc ctttttcaat attattgaag catttatcag ggttattgtc 2640
   tcatgagcgg atacatattt gaatgtattt agaaaaataa acaaataggg gttccgcgca 2700
   catttccccg aaaagtgcca cctgatgcgg tgtgaaatac cgcacagatg cgtaaggaga 2760
   aaataccgca tcaggaaatt gtaagcgtta atattttgtt aaaattcgcg ttaaattttt 2820
   gttaaatcag ctcatttttt aaccaatagg ccgaaatcgg caaaatccct tataaatcaa 2880
   aagaatagac cgagataggg ttgagtgttg ttccagtttg gaacaagagt ccactattaa 2940
   agaacgtgga ctccaacgtc aaagggcgaa aaaccgtcta tcagggcgat ggcccactac 3000
   gtgaaccatc accctaatca agttttttgg ggtcgaggtg ccgtaaagca ctaaatcgga 3060
   accctaaagg gagcccccga tttagagctt gacggggaaa gccggcgaac gtggcgagaa 3120
   aggaagggaa gaaagcgaaa ggagcgggcg ctagggcgct ggcaagtgta gcggtcacgc 3180
   tgcgcgtaac caccacaccc gccgcgctta atgcgccgct acagggcgcg tccattcgcc 3240
   attcaggctg cgcaactgtt gggaagggcg atcggtgcgg gcctcttcgc tattacgcca 3300
   gctggcgaaa gggggatgtg ctgcaaggcg attaagttgg gtaacgccag ggttttccca 3360
   gtcacgacgt tgtaaaacga cggccagtga attgtaatac gactcactat agggcgaatt 3420
   gggcccgacg tcgcatgctg gtttcgattt gtcttagagg aacgcatata cagtaatcat 3480
   agagaataaa cgatattcat ttattaaagt agatagttga ggtagaagtt gtaaagagtg 3540
   ataaatagct tagataccac agacaccctc ggtgacgaag tactgcagat ggtttccaat 3600
   cacattgacc tgctggagca gagtgttacc ggcagagcac tgtttattgc tctggccctg 3660
   gcacatgaca acgttggaga gaggagggtg gatcaggggc cagtcaataa agacctcacc 3720
   agagcagtgc tggtaaccgt cccagaaggg cacttgaggg acgatatctc ctcggtgggt 3780
   gattcggtag agctttcggt ctttggacac cttggagaca tcggggttct cctggccaaa 3840
   gaagagttta tcgacccagt tagcaaagcc agcgttaccg acaatgggct gaccaagagt 3900
   aacaacgagg ggatcgtggc cgttaacctt gaggttgatt ccgaacagaa gggctgcagc 3960
   tcctccgaga gagtgaccgg tgacagcaat ctggtagtcg ggatactgct caatcacaga 4020
   gtcgagcttg gggccgatct gattgtaggt gttgttgtag gactggatga agccattgtg 4080
   gacaagacag tcatcacaag tagcagtaga agagatgtta gcagcaagat caaagttaat 4140
   taactcacct gcaggattga gactatgaat ggattcccgt gcccgtatta ctctactaat 4200
   ttgatcttgg aacgcgaaaa tacgtttcta ggactccaaa gaatctcaac tcttgtcctt 4260
   actaaatata ctacccatag ttgatggttt acttgaacag agaggacatg ttcacttgac 4320
   ccaaagtttc tcgcatctct tggatatttg aacaacggcg tccactgacc gtcagttatc 4380
   cagtcacaaa acccccacat tcatacattc ccatgtacgt ttacaaagtt ctcaattcca 4440
   tcgtgcaaat caaaatcaca tctattcatt catcatatat aaacccatca tgtctactaa 4500
   cactcacaac tccatagaaa acatcgactc agaacacacg ctccatgcgg ccgcttagga 4560
   atcctgagcg tccttgacac agtgaaccac accgactttg tgcatgtact tgagggtgga 4620
   aatgatgttg cccacaatgg tagggtagaa gacgtaccga actccgtgtc gttcgcaaca 4680
   ctctcggaca gcttgctgca cgaagggata gtgccaagac gacattcgag gaaagaggtg 4740
   atgctcgatc tggaagttga gaccgccagt aaagaacatg gcaatgggtc caccgtaggt 4800
   ggaagaggtc tccacctgag ctctgtacca gtcgatctga tcggcttcaa cgtccttctc 4860
   ggagctcttg accttgcagt tcttgtcggg gattcgctcc gagccatcga agttgtgaga 4920
   caagatgaaa aagaaggtga ggaaggcacc ggtagcagtg ggcaccagag gaatggtgat 4980
   gagcagggag gttccagtga gataccaggg caagaaggcg gttcgaaaga tgaagaaagc 5040
   tcgcataacg aatgcaaggg ttcggtaccg tcgcagaaag ccgttctctc gcatggctgt 5100
   gacagactcg ggaatggtgt cgttgtgctg cattcggaag atgtagagag ggttgtacac 5160
   cagcgaaacg ccgtaggctc caagcacgag gtacatgtac caggcctgga atcggtgaaa 5220
   ccactttcga gcagtgttgg cagcagggta gttgtggaac acaaggaatg gttctgcgga 5280
   ctcggcatcc aggtcgagac catgctgatt ggtgtaggtg tgatgtcgca tgatgtgaga 5340
   ctgcagccag atccatctgg acgatccaat gacgtcgatg ccgtaggcaa agagagcgtt 5400
   gacccagggc tttttgctga tggcaccatg agaggcatcg tgctgaatgg acaggccgat 5460
   ctgcatgtgc atgaatccag tcaagagacc ccacagcacc attccggtag tagcccagtg 5520
   ccactcgcaa aaggcggtga cagcaatgat gccaacggtt cgcagccaga atccaggtgt 5580
   ggcataccag ttccgacctt tcatgacctc tcgcatagtt cgcttgacgt cctgtgcaaa 5640
   gggagagtcg taggtgtaga caatgtcctt ggaggttcgg tcgtgcttgc ctcgcacgaa 5700
   ctgttgaagc agcttcgagt tctcgggctt gacgtaaggg tgcatggagt agaacagagg 5760
   agaagcatcg gaggcaccag aagcgaggat caagtcggat ccgggatgga ccttggcaag 5820
   accttccaga tcgtagagaa tgccgtcgat ggcaaccagg tcgggtcgct cgagcagctg 5880
   ctcggtagta agggagagag ccatggttgt gaattagggt ggtgagaatg gttggttgta 5940
   gggaagaatc aaaggccggt ctcgggatcc gtgggtatat atatatatat atatatatac 6000
   gatccttcgt tacctccctg ttctcaaaac tgtggttttt cgtttttcgt tttttgcttt 6060
   ttttgatttt tttagggcca actaagcttc cagatttcgc taatcacctt tgtactaatt 6120
   acaagaaagg aagaagctga ttagagttgg gctttttatg caactgtgct actccttatc 6180
   tctgatatga aagtgtagac ccaatcacat catgtcattt agagttggta atactgggag 6240
   gatagataag gcacgaaaac gagccatagc agacatgctg ggtgtagcca agcagaagaa 6300
   agtagatggg agccaattga cgagcgaggg agctacgcca atccgacata cgacacgctg 6360
   agatcgtctt ggccgggggg tacctacaga tgtccaaggg taagtgcttg actgtaattg 6420
   tatgtctgag gacaaatatg tagtcagccg tataaagtca taccaggcac cagtgccatc 6480
   atcgaaccac taactctcta tgatacatgc ctccggtatt attgtaccat gcgtcgcttt 6540
   gttacatacg tatcttgcct ttttctctca gaaactccag aattctctct cttgagcttt 6600
   tccataacaa gttcttctgc ctccaggaag tccatgggtg gtttgatcat ggttttggtg 6660
   tagtggtagt gcagtggtgg tattgtgact ggggatgtag ttgagaataa gtcatacaca 6720
   agtcagcttt cttcgagcct catataagta taagtagttc aacgtattag cactgtaccc 6780
   agcatctccg tatcgagaaa cacaacaaca tgccccattg gacagatcat gcggatacac 6840
   aggttgtgca gtatcataca tactcgatca gacaggtcgt ctgaccatca tacaagctga 6900
   acaagcgctc catacttgca cgctctctat atacacagtt aaattacata tccatagtct 6960
   aacctctaac agttaatctt ctggtaagcc tcccagccag ccttctggta tcgcttggcc 7020
   tcctcaatag gatctcggtt ctggccgtac agacctcggc cgacaattat gatatccgtt 7080
   ccggtagaca tgacatcctc aacagttcgg tactgctgtc cgagagcgtc tcccttgtcg 7140
   tcaagaccca ccccgggggt cagaataagc cagtcctcag agtcgccctt aggtcggttc 7200
   tgggcaatga agccaaccac aaactcgggg tcggatcggg caagctcaat ggtctgcttg 7260
   gagtactcgc cagtggccag agagcccttg caagacagct cggccagcat gagcagacct 7320
   ctggccagct tctcgttggg agaggggact aggaactcct tgtactggga gttctcgtag 7380
   tcagagacgt cctccttctt ctgttcagag acagtttcct cggcaccagc tcgcaggcca 7440
   gcaatgattc cggttccggg tacaccgtgg gcgttggtga tatcggacca ctcggcgatt 7500
   cggtgacacc ggtactggtg cttgacagtg ttgccaatat ctgcgaactt tctgtcctcg 7560
   aacaggaaga aaccgtgctt aagagcaagt tccttgaggg ggagcacagt gccggcgtag 7620
   gtgaagtcgt caatgatgtc gatatgggtt ttgatcatgc acacataagg tccgacctta 7680
   tcggcaagct caatgagctc cttggtggtg gtaacatcca gagaagcaca caggttggtt 7740
   ttcttggctg ccacgagctt gagcactcga gcggcaaagg cggacttgtg gacgttagct 7800
   cgagcttcgt aggagggcat tttggtggtg aagaggagac tgaaataaat ttagtctgca 7860
   gaacttttta tcggaacctt atctggggca gtgaagtata tgttatggta atagttacga 7920
   gttagttgaa cttatagata gactggacta tacggctatc ggtccaaatt agaaagaacg 7980
   tcaatggctc tctgggcgtc gcctttgccg acaaaaatgt gatcatgatg aaagccagca 8040
   atgacgttgc agctgatatt gttgtcggcc aaccgcgccg aaaacgcagc tgtcagaccc 8100
   acagcctcca acgaagaatg tatcgtcaaa gtgatccaag cacactcata gttggagtcg 8160
   tactccaaag gcggcaatga cgagtcagac agatactcgt cgaccttttc cttgggaacc 8220
   accaccgtca gcccttctga ctcacgtatt gtagccaccg acacaggcaa cagtccgtgg 8280
   atagcagaat atgtcttgtc ggtccatttc tcaccaactt taggcgtcaa gtgaatgttg 8340
   cagaagaagt atgtgccttc attgagaatc ggtgttgctg atttcaataa agtcttgaga 8400
   tcagtttggc cagtcatgtt gtggggggta attggattga gttatcgcct acagtctgta 8460
   caggtatact cgctgcccac tttatacttt ttgattccgc tgcacttgaa gcaatgtcgt 8520
   ttaccaaaag tgagaatgct ccacagaaca caccccaggg tatggttgag caaaaaataa 8580
   acactccgat acggggaatc gaaccccggt ctccacggtt ctcaagaagt attcttgatg 8640
   agagcgtatc gatcgaggaa gaggacaagc ggctgcttct taagtttgtg acatcagtat 8700
   ccaaggcacc attgcaagga ttcaaggctt tgaacccgtc atttgccatt cgtaacgctg 8760
   gtagacaggt tgatcggttc cctacggcct ccacctgtgt caatcttctc aagctgcctg 8820
   actatcagga cattgatcaa cttcggaaga aacttttgta tgccattcga tcacatgctg 8880
   gtttcgattt gtcttagagg aacgcatata cagtaatcat agagaataaa cgatattcat 8940
   ttattaaagt agatagttga ggtagaagtt gtaaagagtg ataaatagcg gccgctcact 9000
   gaatcttttt ggctcccttg tgctttcgga cgatgtaggt ctgcacgtag aagttgagga 9060
   acagacacag gacagtacca acgtagaagt agttgaaaaa ccagccaaac attctcattc 9120
   catcttgtcg gtagcaggga atgttccggt acttccagac gatgtagaag ccaacgttga 9180
   actgaatgat ctgcatagaa gtaatcaggg acttgggcat agggaacttg agcttgatca 9240
   gtcgggtcca atagtagccg tacatgatcc agtgaatgaa gccgttgagc agcacaaaga 9300
   tccaaacggc ttcgtttcgg tagttgtaga acagccacat gtccatagga gctccgagat 9360
   ggtgaaagaa ctgcaaccag gtcagaggct tgcccatgag gggcagatag aaggagtcaa 9420
   tgtactcgag gaacttgctg aggtagaaca gctgagtggt gattcggaag acattgttgt 9480
   cgaaagcctt ctcgcagttg tcggacatga caccaatggt gtacatggcg taggccatag 9540
   agaggaagga gcccagcgag tagatggaca tgagcaggtt gtagttggtg aacacaaact 9600
   tcattcgaga ctgacccttg ggtccgagag gaccaagggt gaacttcagg atgacgaagg 9660
   cgatggagag gtacagcacc tcgcagtgcg aggcatcaga ccagagctga gcatagtcga 9720
   ccttgggaag aacctcctgg ccaatggaga cgatttcgtt cacgacctcc atggttgtga 9780
   attagggtgg tgagaatggt tggttgtagg gaagaatcaa aggccggtct cgggatccgt 9840
   gggtatatat atatatatat atatatacga tccttcgtta cctccctgtt ctcaaaactg 9900
   tggtttttcg tttttcgttt tttgcttttt ttgatttttt tagggccaac taagcttcca 9960
   gatttcgcta atcacctttg tactaattac aagaaaggaa gaagctgatt agagttgggc 10020
   tttttatgca actgtgctac tccttatctc tgatatgaaa gtgtagaccc aatcacatca 10080
   tgtcatttag agttggtaat actgggagga tagataaggc acgaaaacga gccatagcag 10140
   acatgctggg tgtagccaag cagaagaaag tagatgggag ccaattgacg agcgagggag 10200
   ctacgccaat ccgacatacg acacgctgag atcgtcttgg ccggggggta cctacagatg 10260
   tccaagggta agtgcttgac tgtaattgta tgtctgagga caaatatgta gtcagccgta 10320
   taaagtcata ccaggcacca gtgccatcat cgaaccacta actctctatg atacatgcct 10380
   ccggtattat tgtaccatgc gtcgctttgt tacatacgta tcttgccttt ttctctcaga 10440
   aactccagac tttggctatt ggtcgagata agcccggacc atagtgagtc tttcacactc 10500
   tgtttaaaca ccactaaaac cccacaaaat atatcttacc gaatatacag atctactata 10560
   gaggaacaat tgccccggag aagacggcca ggccgcctag atgacaaatt caacaactca 10620
   cagctgactt tctgccattg ccactagggg ggggcctttt tatatggcca agccaagctc 10680
   tccacgtcgg ttgggctgca cccaacaata aatgggtagg gttgcaccaa caaagggatg 10740
   ggatgggggg tagaagatac gaggataacg gggctcaatg gcacaaataa gaacgaatac 10800
   tgccattaag actcgtgatc cagcgactga caccattgca tcatctaagg gcctcaaaac 10860
   tacctcggaa ctgctgcgct gatctggaca ccacagaggt tccgagcact ttaggttgca 10920
   ccaaatgtcc caccaggtgc aggcagaaaa cgctggaaca gcgtgtacag tttgtcttaa 10980
   caaaaagtga gggcgctgag gtcgagcagg gtggtgtgac ttgttatagc ctttagagct 11040
   gcgaaagcgc gtatggattt ggctcatcag gccagattga gggtctgtgg acacatgtca 11100
   tgttagtgta cttcaatcgc cccctggata tagccccgac aataggccgt ggcctcattt 11160
   ttttgccttc cgcacatttc cattgctcgg tacccacacc ttgcttctcc tgcacttgcc 11220
   aaccttaata ctggtttaca ttgaccaaca tcttacaagc ggggggcttg tctagggtat 11280
   atataaacag tggctctccc aatcggttgc cagtctcttt tttcctttct ttccccacag 11340
   attcgaaatc taaactacac atcacacaat gcctgttact gacgtcctta agcgaaagtc 11400
   cggtgtcatc gtcggcgacg atgtccgagc cgtgagtatc cacgacaaga tcagtgtcga 11460
   gacgacgcgt tttgtgtaat gacacaatcc gaaagtcgct agcaacacac actctctaca 11520
   caaactaacc cagctctcca tggtgaaggc ttctcgacag gctctgcccc tcgtcatcga 11580
   cggaaaggtg tacgacgtct ccgcttgggt gaacttccac cctggtggag ctgaaatcat 11640
   tgagaactac cagggacgag atgctactga cgccttcatg gttatgcact ctcaggaagc 11700
   cttcgacaag ctcaagcgaa tgcccaagat caaccaggct tccgagctgc ctccccaggc 11760
   tgccgtcaac gaagctcagg aggatttccg aaagctccga gaagagctga tcgccactgg 11820
   catgtttgac gcctctcccc tctggtactc gtacaagatc ttgaccaccc tgggtcttgg 11880
   cgtgcttgcc ttcttcatgc tggtccagta ccacctgtac ttcattggtg ctctcgtgct 11940
   cggtatgcac taccagcaaa tgggatggct gtctcatgac atctgccacc accagacctt 12000
   caagaaccga aactggaata acgtcctggg tctggtcttt ggcaacggac tccagggctt 12060
   ctccgtgacc tggtggaagg acagacacaa cgcccatcat tctgctacca acgttcaggg 12120
   tcacgatccc gacattgata acctgcctct gctcgcctgg tccgaggacg atgtcactcg 12180
   agcttctccc atctcccgaa agctcattca gttccaacag tactatttcc tggtcatctg 12240
   tattctcctg cgattcatct ggtgtttcca gtctgtgctg accgttcgat ccctcaagga 12300
   ccgagacaac cagttctacc gatctcagta caagaaagag gccattggac tcgctctgca 12360
   ctggactctc aagaccctgt tccacctctt ctttatgccc tccatcctga cctcgatgct 12420
   ggtgttcttt gtttccgagc tcgtcggtgg cttcggaatt gccatcgtgg tcttcatgaa 12480
   ccactaccct ctggagaaga tcggtgattc cgtctgggac ggacatggct tctctgtggg 12540
   tcagatccat gagaccatga acattcgacg aggcatcatt actgactggt tctttggagg 12600
   cctgaactac cagatcgagc accatctctg gcccaccctg cctcgacaca acctcactgc 12660
   cgtttcctac caggtggaac agctgtgcca gaagcacaac ctcccctacc gaaaccctct 12720
   gccccatgaa ggtctcgtca tcctgctccg atacctgtcc cagttcgctc gaatggccga 12780
   gaagcagccc ggtgccaagg ctcagtaagc ggccgcatga gaagataaat atataaatac 12840
   attgagatat taaatgcgct agattagaga gcctcatact gctcggagag aagccaagac 12900
   gagtactcaa aggggattac accatccata tccacagaca caagctgggg aaaggttcta 12960
   tatacacttt ccggaatacc gtagtttccg atgttatcaa tgggggcagc caggatttca 13020
   ggcacttcgg tgtctcgggg tgaaatggcg ttcttggcct ccatcaagtc gtaccatgtc 13080
   ttcatttgcc tgtcaaagta aaacagaagc agatgaagaa tgaacttgaa gtgaaggaat 13140
   ttaaatgatg tcgacgcagt aggatgtcct gcacgggtct tttgtggggg tgtggagaaa 13200
   ggggtgcttg gatcgatgga agccggtaga accgggctgc ttgtgcttgg agatggaagc 13260
   cggtagaacc gggctgcttg gggggatttg gggccgctgg gctccaaaga ggggtaggca 13320
   tttcgttggg gttacgtaat tgcggcattt gggtcctgcg cgcatgtccc attggtcaga 13380
   attagtccgg ataggagact tatcagccaa tcacagcgcc ggatccacct gtaggttggg 13440
   ttgggtggga gcacccctcc acagagtaga gtcaaacagc agcagcaaca tgatagttgg 13500
   gggtgtgcgt gttaaaggaa aaaaaagaag cttgggttat attcccgctc tatttagagg 13560
   ttgcgggata gacgccgacg gagggcaatg gcgctatgga accttgcgga tatccatacg 13620
   ccgcggcgga ctgcgtccga accagctcca gcagcgtttt ttccgggcca ttgagccgac 13680
   tgcgaccccg ccaacgtgtc ttggcccacg cactcatgtc atgttggtgt tgggaggcca 13740
   ctttttaagt agcacaaggc acctagctcg cagcaaggtg tccgaaccaa agaagcggct 13800
   gcagtggtgc aaacggggcg gaaacggcgg gaaaaagcca cgggggcacg aattgaggca 13860
   cgccctcgaa tttgagacga gtcacggccc cattcgcccg cgcaatggct cgccaacgcc 13920
   cggtcttttg caccacatca ggttacccca agccaaacct ttgtgttaaa aagcttaaca 13980
   tattataccg aacgtaggtt tgggcgggct tgctccgtct gtccaaggca acatttatat 14040
   aagggtctgc atcgccggct caattgaatc ttttttcttc ttctcttctc tatattcatt 14100
   cttgaattaa acacacatca accatgggcg tattcattaa acaggagcag cttccggctc 14160
   tcaagaagta caagtactcc gccgaggatc actcgttcat ctccaacaac attctgcgcc 14220
   ccttctggcg acagtttgtc aaaatcttcc ctctgtggat ggcccccaac atggtgactc 14280
   tgctgggctt cttctttgtc attgtgaact tcatcaccat gctcattgtt gatcccaccc 14340
   acgaccgcga gcctcccaga tgggtctacc tcacctacgc tctgggtctg ttcctttacc 14400
   agacatttga tgcctgtgac ggatcccatg cccgacgaac tggccagagt ggaccccttg 14460
   gagagctgtt tgaccactgt gtcgacgcca tgaatacctc tctgattctc acggtggtgg 14520
   tgtccaccac ccatatggga tataacatga agctactgat tgtgcagatt gccgctctcg 14580
   gaaacttcta cctgtcgacc tgggagacct accataccgg aactctgtac ctttctggct 14640
   tctctggtcc tgttgaaggt atcttgattc tggtggctct tttcgtcctc accttcttca 14700
   ctggtcccaa cgtgtacgct ctgaccgtct acgaggctct tcccgagtcc atcacttcgc 14760
   tgctgcctgc cagcttcctg gacgtcacca tcacccagat ctacattgga ttcggagtgc 14820
   tgggcatggt gttcaacatc tacggcgcct gcggaaacgt gatcaagtac tacaacaaca 14880
   agggcaagag cgctctcccc gccattctcg gaatcgcccc ctttggcatc ttctacgtcg 14940
   gcgtctttgc ctgggcccat gttgctcctc tgcttctctc caagtacgcc atcgtctatc 15000
   tgtttgccat tggggctgcc tttgccatgc aagtcggcca gatgattctt gcccatctcg 15060
   tgcttgctcc ctttccccac tggaacgtgc tgctcttctt cccctttgtg ggactggcag 15120
   tgcactacat tgcacccgtg tttggctggg acgccgatat cgtgtcggtt aacactctct 15180
   tcacctgttt tggcgccacc ctctccattt acgccttctt tgtgcttgag atcatcgacg 15240
   agatcaccaa ctacctcgat atctggtgtc tgcgaatcaa gtaccctcag gagaagaaga 15300
   ccgaataagc ggccgcatgg agcgtgtgtt ctgagtcgat gttttctatg gagttgtgag 15360
   tgttagtaga catgatgggt ttatatatga tgaatgaata gatgtgattt tgatttgcac 15420
   gatggaattg agaactttgt aaacgtacat gggaatgtat gaatgtgggg gttttgtgac 15480
   tggataactg acggtcagtg gacgccgttg ttcaaatatc caagagatgc gagaaacttt 15540
   gggtcaagtg aacatgtcct ctctgttcaa gtaaaccatc aactatgggt agtatattta 15600
   gtaaggacaa gagttgagat tctttggagt cctagaaacg tattttcgcg ttccaagatc 15660
   aaattagtag agtaatacgg gcacgggaat ccattcatag tctcaatcct gcaggtgagt 15720
   taattaatcg agcttggcgt aatcatggtc atagctgttt cctgtgtgaa attgttatcc 15780
   gctcacaatt ccacacaac 15799
<210> 139
   <211> 9641
   <212> DNA
   <213> Artificial Sequence
<220>
   <223> Plasmid pY201
<400> 139
   gtacgataac ttcgtatagc atacattata cgaagttatc gcgtcgacga gtatctgtct 60
   gactcgtcat tgccgccttt ggagtacgac tccaactatg agtgtgcttg gatcactttg 120
   acgatacatt cttcgttgga ggctgtgggt ctgacagctg cgttttcggc gcggttggcc 180
   gacaacaata tcagctgcaa cgtcattgct ggctttcatc atgatcacat ttttgtcggc 240
   aaaggcgacg cccagagagc cattgacgtt ctttctaatt tggaccgata gccgtatagt 300
   ccagtctatc tataagttca actaactcgt aactattacc ataacatata cttcactgcc 360
   ccagataagg ttccgataaa aagttctgca gactaaattt atttcagtct cctcttcacc 420
   accaaaatgc cctcctacga agctcgagct aacgtccaca agtccgcctt tgccgctcga 480
   gtgctcaagc tcgtggcagc caagaaaacc aacctgtgtg cttctctgga tgttaccacc 540
   accaaggagc tcattgagct tgccgataag gtcggacctt atgtgtgcat gatcaaaacc 600
   catatcgaca tcattgacga cttcacctac gccggcactg tgctccccct caaggaactt 660
   gctcttaagc acggtttctt cctgttcgag gacagaaagt tcgcagatat tggcaacact 720
   gtcaagcacc agtaccggtg tcaccgaatc gccgagtggt ccgatatcac caacgcccac 780
   ggtgtacccg gaaccggaat cattgctggc ctgcgagctg gtgccgagga aactgtctct 840
   gaacagaaga aggaggacgt ctctgactac gagaactccc agtacaagga gttcctagtc 900
   ccctctccca acgagaagct ggccagaggt ctgctcatgc tggccgagct gtcttgcaag 960
   ggctctctgg ccactggcga gtactccaag cagaccattg agcttgcccg atccgacccc 1020
   gagtttgtgg ttggcttcat tgcccagaac cgacctaagg gcgactctga ggactggctt 1080
   attctgaccc ccggggtggg tcttgacgac aagggagacg ctctcggaca gcagtaccga 1140
   actgttgagg atgtcatgtc taccggaacg gatatcataa ttgtcggccg aggtctgtac 1200
   ggccagaacc gagatcctat tgaggaggcc aagcgatacc agaaggctgg ctgggaggct 1260
   taccagaaga ttaactgtta gaggttagac tatggatatg taatttaact gtgtatatag 1320
   agagcgtgca agtatggagc gcttgttcag cttgtatgat ggtcagacga cctgtctgat 1380
   cgagtatgta tgatactgca caacctgtgt atccgcatga tctgtccaat ggggcatgtt 1440
   gttgtgtttc tcgatacgga gatgctgggt acagtgctaa tacgttgaac tacttatact 1500
   tatatgaggc tcgaagaaag ctgacttgtg tatgacttat tctcaactac atccccagtc 1560
   acaataccac cactgcacta ccactacacc aaaaccatga tcaaaccacc catggacttc 1620
   ctggaggcag aagaacttgt tatggaaaag ctcaagagag agatcataac ttcgtatagc 1680
   atacattata cgaagttatc ctgcaggtaa aggaattcag gagagaccgg gttggcggcg 1740
   tatttgtgtc ccaaaaaaca gccccaattg ccccaattga ccccaaattg acccagtagc 1800
   gggcccaacc ccggcgagag cccccttcac cccacatatc aaacctcccc cggttcccac 1860
   acttgccgtt aagggcgtag ggtactgcag tctggaatct acgcttgttc agactttgta 1920
   ctagtttctt tgtctggcca tccgggtaac ccatgccgga cgcaaaatag actactgaaa 1980
   atttttttgc tttgtggttg ggactttagc caagggtata aaagaccacc gtccccgaat 2040
   tacctttcct cttcttttct ctctctcctt gtcaactcac acccgaaatc gttaagcatt 2100
   tccttctgag tataagaatc attcaccatg gacttcctgg aggcagaaga acttgttatg 2160
   gaaaagctca agagagagaa gccaagatac tatcaagaca tgtgtcgcaa cttaattaag 2220
   atgacgacat ttgcgagctg gacgaggaat agatggagcg tgtgttctga gtcgatgttt 2280
   tctatggagt tgtgagtgtt agtagacatg atgggtttat atatgatgaa tgaatagatg 2340
   tgattttgat ttgcacgatg gaattgagaa ctttgtaaac gtacatggga atgtatgaat 2400
   gtgggggttt tgtgactgga taactgacgg tcagtggacg ccgttgttca aatatccaag 2460
   agatgcgaga aactttgggt caagtgaaca tgtcctctct gttcaagtaa accatcaact 2520
   atgggtagta tatttagtaa ggacaagagt tgagattctt tggagtccta gaaacgtatt 2580
   ttcgcgttcc aagatcaaat tagtagagta atacgggcac gggaatccat tcatagtctc 2640
   aattttccca taggtgtgct acaaggtgtt gagatgtggt acagtaccac catgattcga 2700
   ggtaaagagc ccagaagtca ttgatgaggt caagaaatac acagatctac agctcaatac 2760
   aatgaatatc ttctttcata ttcttcaggt gacaccaagg gtgtctattt tccccagaaa 2820
   tgcgtgaaaa ggcgcgtgtg tagcgtggag tatgggttcg gttggcgtat ccttcatata 2880
   tcgacgaaat agtagggcaa gagatgacaa aaagtatcta tatgtagaca gcgtagaata 2940
   tggatttgat tggtataaat tcatttattg cgtgtctcac aaatactctc gataagttgg 3000
   ggttaaactg gagatggaac aatgtcgata tctcgacgca tgcgacgtcg ggcccaattc 3060
   gccctatagt gagtcgtatt acaattcact ggccgtcgtt ttacaacgtc gtgactggga 3120
   aaaccctggc gttacccaac ttaatcgcct tgcagcacat ccccctttcg ccagctggcg 3180
   taatagcgaa gaggcccgca ccgatcgccc ttcccaacag ttgcgcagcc tgaatggcga 3240
   atggacgcgc cctgtagcgg cgcattaagc gcggcgggtg tggtggttac gcgcagcgtg 3300
   accgctacac ttgccagcgc cctagcgccc gctcctttcg ctttcttccc ttcctttctc 3360
   gccacgttcg ccggctttcc ccgtcaagct ctaaatcggg ggctcccttt agggttccga 3420
   tttagtgctt tacggcacct cgaccccaaa aaacttgatt agggtgatgg ttcacgtagt 3480
   gggccatcgc cctgatagac ggtttttcgc cctttgacgt tggagtccac gttctttaat 3540
   agtggactct tgttccaaac tggaacaaca ctcaacccta tctcggtcta ttcttttgat 3600
   ttataaggga ttttgccgat ttcggcctat tggttaaaaa atgagctgat ttaacaaaaa 3660
   tttaacgcga attttaacaa aatattaacg cttacaattt cctgatgcgg tattttctcc 3720
   ttacgcatct gtgcggtatt tcacaccgca tcaggtggca cttttcgggg aaatgtgcgc 3780
   ggaaccccta tttgtttatt tttctaaata cattcaaata tgtatccgct catgagacaa 3840
   taaccctgat aaatgcttca ataatattga aaaaggaaga gtatgagtat tcaacatttc 3900
   cgtgtcgccc ttattccctt ttttgcggca ttttgccttc ctgtttttgc tcacccagaa 3960
   acgctggtga aagtaaaaga tgctgaagat cagttgggtg cacgagtggg ttacatcgaa 4020
   ctggatctca acagcggtaa gatccttgag agttttcgcc ccgaagaacg ttttccaatg 4080
   atgagcactt ttaaagttct gctatgtggc gcggtattat cccgtattga cgccgggcaa 4140
   gagcaactcg gtcgccgcat acactattct cagaatgact tggttgagta ctcaccagtc 4200
   acagaaaagc atcttacgga tggcatgaca gtaagagaat tatgcagtgc tgccataacc 4260
   atgagtgata acactgcggc caacttactt ctgacaacga tcggaggacc gaaggagcta 4320
   accgcttttt tgcacaacat gggggatcat gtaactcgcc ttgatcgttg ggaaccggag 4380
   ctgaatgaag ccataccaaa cgacgagcgt gacaccacga tgcctgtagc aatggcaaca 4440
   acgttgcgca aactattaac tggcgaacta cttactctag cttcccggca acaattaata 4500
   gactggatgg aggcggataa agttgcagga ccacttctgc gctcggccct tccggctggc 4560
   tggtttattg ctgataaatc tggagccggt gagcgtgggt ctcgcggtat cattgcagca 4620
   ctggggccag atggtaagcc ctcccgtatc gtagttatct acacgacggg gagtcaggca 4680
   actatggatg aacgaaatag acagatcgct gagataggtg cctcactgat taagcattgg 4740
   taactgtcag accaagttta ctcatatata ctttagattg atttaaaact tcatttttaa 4800
   tttaaaagga tctaggtgaa gatccttttt gataatctca tgaccaaaat cccttaacgt 4860
   gagttttcgt tccactgagc gtcagacccc gtagaaaaga tcaaaggatc ttcttgagat 4920
   cctttttttc tgcgcgtaat ctgctgcttg caaacaaaaa aaccaccgct accagcggtg 4980
   gtttgtttgc cggatcaaga gctaccaact ctttttccga aggtaactgg cttcagcaga 5040
   gcgcagatac caaatactgt tcttctagtg tagccgtagt taggccacca cttcaagaac 5100
   tctgtagcac cgcctacata cctcgctctg ctaatcctgt taccagtggc tgctgccagt 5160
   ggcgataagt cgtgtcttac cgggttggac tcaagacgat agttaccgga taaggcgcag 5220
   cggtcgggct gaacgggggg ttcgtgcaca cagcccagct tggagcgaac gacctacacc 5280
   gaactgagat acctacagcg tgagctatga gaaagcgcca cgcttcccga agggagaaag 5340
   gcggacaggt atccggtaag cggcagggtc ggaacaggag agcgcacgag ggagcttcca 5400
   gggggaaacg cctggtatct ttatagtcct gtcgggtttc gccacctctg acttgagcgt 5460
   cgatttttgt gatgctcgtc aggggggcgg agcctatgga aaaacgccag caacgcggcc 5520
   tttttacggt tcctggcctt ttgctggcct tttgctcaca tgttctttcc tgcgttatcc 5580
   cctgattctg tggataaccg tattaccgcc tttgagtgag ctgataccgc tcgccgcagc 5640
   cgaacgaccg agcgcagcga gtcagtgagc gaggaagcgg aagagcgccc aatacgcaaa 5700
   ccgcctctcc ccgcgcgttg gccgattcat taatgcagct ggcgcgccac caatcacaat 5760
   tctgaaaagc acatcttgat ctcctcattg cggggagtcc aacggtggtc ttattccccc 5820
   gaatttcccg ctcaatctcg ttccagaccg acccggacac agtgcttaac gccgttccga 5880
   aactctaccg cagatatgct ccaacggact gggctgcata gatgtgatcc tcggcttgga 5940
   gaaatggata aaagccggcc aaaaaaaaag cggaaaaaag cggaaaaaaa gagaaaaaaa 6000
   atcgcaaaat ttgaaaaata gggggaaaag acgcaaaaac gcaaggaggg gggagtatat 6060
   gacactgata agcaagctca caacggttcc tcttattttt ttcctcatct tctgcctagg 6120
   ttcccaaaat cccagatgct tctctccagt gccaaaagta agtaccccac aggttttcgg 6180
   ccgaaaattc cacgtgcagc aacgtcgtgt ggggtgttaa aatgtggggg gggggaacca 6240
   ggacaagagg ctcttgtggg agccgaatga gagcacaaag cgggcgggtg tgataagggc 6300
   atttttgccc attttccctt ctcctgtctc tccgacggtg atggcgttgt gcgtcctcta 6360
   tttcttttta tttctttttg ttttatttct ctgactaccg atttggtttg atttcctcaa 6420
   ccccacacaa ataagctcgg gccgaggaat atatatatac acggacacag tcgccctgtg 6480
   gacaacacgt cactacctct acgatacaca ccgtacgata gttagtagac aacaatcgat 6540
   agttggagca agggagaaat gtagagtgtg aaagactcac tatggtccgg gcttatctcg 6600
   accaatagcc aaagtctgga gtttctgaga gaaaaaggca agatacgtat gtaacaaagc 6660
   gacgcatggt acaataatac cggaggcatg tatcatagag agttagtggt tcgatgatgg 6720
   cactggtgcc tggtatgact ttatacggct gactacatat ttgtcctcag acatacaatt 6780
   acagtcaagc acttaccctt ggacatctgt aggtaccccc cggccaagac gatctcagcg 6840
   tgtcgtatgt cggattggcg tagctccctc gctcgtcaat tggctcccat ctactttctt 6900
   ctgcttggct acacccagca tgtctgctat ggctcgtttt cgtgccttat ctatcctccc 6960
   agtattacca actctaaatg acatgatgtg attgggtcta cactttcata tcagagataa 7020
   ggagtagcac agttgcataa aaagcccaac tctaatcagc ttcttccttt cttgtaatta 7080
   gtacaaaggt gattagcgaa atctggaagc ttagttggcc ctaaaaaaat caaaaaaagc 7140
   aaaaaacgaa aaacgaaaaa ccacagtttt gagaacaggg aggtaacgaa ggatcgtata 7200
   tatatatata tatatatata cccacggatc ccgagaccgg cctttgattc ttccctacaa 7260
   ccaaccattc tcaccaccct aattcacaac catgtacaac cccgtggacg cagtgttgac 7320
   taagattatt acaaactacg gaattgattc ttttaccctg cgatatgcca tttgtctgtt 7380
   gggatctttt cctcttaacg ctattctgaa gcggattcct gaaaagcgaa tcggcctgaa 7440
   gtgttgtttt atcatttcta tgtccatgtt ttatctcttc ggcgttctga atctcgtgag 7500
   cggatttcga accctcttca tttccacaat gttcacatac cttatctctc ggttctaccg 7560
   atccaagttt atgccccatc tcaacttcat gttcgtcatg ggccacttgg ctatcaacca 7620
   cattcatgct cagttcctga acgaacaaac tcaaacgacc gtcgatatta catcctcgca 7680
   gatggtcctg gctatgaagc tgacaagctt tgcctggtct tactatgacg gttcgtgtac 7740
   gagcgagtcc gacttcaagg accttaccga acaccagaag tcccgagccg tccgaggcca 7800
   tcctcccctt ctgaaatttt tggcttacgc ctttttctac tctacccttc tcaccggtcc 7860
   ctccttcgat tacgctgatt tcgactcttg gctgaactgc gaaatgttcc gggaccttcc 7920
   cgagtccaag aaacccatgc gaagacatca tcctggtgag cggcgtcaga ttcccaagaa 7980
   cggcaagctc gccctgtgga aggttgtcca gggcctcgcc tggatgattc tgagcacgtt 8040
   gggtatgaag cacttccccg tgaagtacgt gctggacaag gacggatttc ctacccgttc 8100
   ctttatcttc cgtattcatt atctgtttct gctgggattc atccaccgat ttaagtatta 8160
   cgctgcgtgg acgattagcg aaggttcgtg cattctctgt ggtcttggtt ataatggata 8220
   cgattctaag acccagaaga tccggtggga tcgagtgcgg aatattgata tttggacagt 8280
   ggagactgca caaaacaccc gagagatgct ggaagcgtgg aacatgaata ctaacaaatg 8340
   gctgaagtat agcgtgtatc ttagagtgac taagaagggt aagaagccag gttttcgatc 8400
   taccctgttt accttcctga cctccgcctt ttggcacggt acccgtcctg gatactacct 8460
   taccttcgca actggtgccc tgtaccaaac ctgtggaaag atctatagac gaaactttcg 8520
   tcccatcttt ctgagagaag atggcgtgac acctctcccg tccaagaaga tttacgacct 8580
   ggtcggcatt tacgctatta agctggcctt tggttacatg gttcaaccct tcattatcct 8640
   tgacctgaag ccctctctta tggtttgggg atccgtgtat ttctacgtgc atattattgt 8700
   ggccttctcg ttctttctgt tccgaggacc atacgctaag caggttactg aatttttcaa 8760
   aagcaagcaa ccgaaggaga tcttcatccg aaagcagaag aagttggaaa aagacatctc 8820
   tgcctcttcc cccaacctcg gaggtattct taaggcaaaa atcgaacatg agaagggaaa 8880
   gacggcagag gaggaagaga tgaacttggg cattccaccc atcgaactgg agaagtggga 8940
   caacgccaag gaggactggg aggatttctg caaggactac aaggagtggc ggaacaagaa 9000
   cggactggaa attgaagagg agaacctgtc caaggccttc gagcgattta agcaggaatt 9060
   ttccaacgct gcgtcgggct ctggtgaacg ggttcggaaa atgtccttct ccggatattc 9120
   tcctaaaccc atctcgaaga aagaagaata ggcggccgca tgagaagata aatatataaa 9180
   tacattgaga tattaaatgc gctagattag agagcctcat actgctcgga gagaagccaa 9240
   gacgagtact caaaggggat tacaccatcc atatccacag acacaagctg gggaaaggtt 9300
   ctatatacac tttccggaat accgtagttt ccgatgttat caatgggggc agccaggatt 9360
   tcaggcactt cggtgtctcg ggtgaaatgg gcgttcttgg cctccatcaa gtcgtaccat 9420
   gtcttcattt gcctgtcaaa gtaaaacaga agcagatgaa gaatgaactt gaagtgaagg 9480
   aatttaaatg taacgaaact gaaatttgac cagatattgt gtccgcggtg gagctccagc 9540
   ttttgttccc tttagtgagg gttaatttcg agcttggcgt aatcatggtc atagctgttt 9600
   cctgtgtgaa attgttatcc gctcacaagc ttccacacaa c 9641
<210> 140
   <211> 34
   <212> DNA
   <213> Escherichia coli
<400> 140
   ataacttcgt ataatgtatg ctatacgaag ttat 34
<210> 141
   <211> 40
   <212> DNA
   <213> Artificial Sequence
<220>
   <223> Primer 798
<400> 141
   ctaattcaca accatggcct ttccatgggc agataagtgg 40
<210> 142
   <211> 36
   <212> DNA
   <213> Artificial Sequence
<220>
   <223> Primer 799
<400> 142
   ctcatgcggc cgcttacttg gtcttgatgg tgtcct 36
<210> 143
   <211> 40
   <212> DNA
   <213> Artificial Sequence
<220>
   <223> Primer 800
<400> 143
   cgatagttag tagacaacaa tcgatagttg gagcaaggga 40
<210> 144
   <211> 36
   <212> DNA
   <213> Artificial Sequence
<220>
   <223> Primer 801
<400> 144
   gaaaggccat ggttgtgaat tagggtggtg agaatg 36
<210> 145
   <211> 9320
   <212> DNA
   <213> Artificial Sequence
<220>
   <223> Plasmid pY168
<400> 145
   gtacgataac ttcgtatagc atacattata cgaagttatc gcgtcgacga gtatctgtct 60
   gactcgtcat tgccgccttt ggagtacgac tccaactatg agtgtgcttg gatcactttg 120
   acgatacatt cttcgttgga ggctgtgggt ctgacagctg cgttttcggc gcggttggcc 180
   gacaacaata tcagctgcaa cgtcattgct ggctttcatc atgatcacat ttttgtcggc 240
   aaaggcgacg cccagagagc cattgacgtt ctttctaatt tggaccgata gccgtatagt 300
   ccagtctatc tataagttca actaactcgt aactattacc ataacatata cttcactgcc 360
   ccagataagg ttccgataaa aagttctgca gactaaattt atttcagtct cctcttcacc 420
   accaaaatgc cctcctacga agctcgagct aacgtccaca agtccgcctt tgccgctcga 480
   gtgctcaagc tcgtggcagc caagaaaacc aacctgtgtg cttctctgga tgttaccacc 540
   accaaggagc tcattgagct tgccgataag gtcggacctt atgtgtgcat gatcaaaacc 600
   catatcgaca tcattgacga cttcacctac gccggcactg tgctccccct caaggaactt 660
   gctcttaagc acggtttctt cctgttcgag gacagaaagt tcgcagatat tggcaacact 720
   gtcaagcacc agtaccggtg tcaccgaatc gccgagtggt ccgatatcac caacgcccac 780
   ggtgtacccg gaaccggaat cattgctggc ctgcgagctg gtgccgagga aactgtctct 840
   gaacagaaga aggaggacgt ctctgactac gagaactccc agtacaagga gttcctagtc 900
   ccctctccca acgagaagct ggccagaggt ctgctcatgc tggccgagct gtcttgcaag 960
   ggctctctgg ccactggcga gtactccaag cagaccattg agcttgcccg atccgacccc 1020
   gagtttgtgg ttggcttcat tgcccagaac cgacctaagg gcgactctga ggactggctt 1080
   attctgaccc ccggggtggg tcttgacgac aagggagacg ctctcggaca gcagtaccga 1140
   actgttgagg atgtcatgtc taccggaacg gatatcataa ttgtcggccg aggtctgtac 1200
   ggccagaacc gagatcctat tgaggaggcc aagcgatacc agaaggctgg ctgggaggct 1260
   taccagaaga ttaactgtta gaggttagac tatggatatg taatttaact gtgtatatag 1320
   agagcgtgca agtatggagc gcttgttcag cttgtatgat ggtcagacga cctgtctgat 1380
   cgagtatgta tgatactgca caacctgtgt atccgcatga tctgtccaat ggggcatgtt 1440
   gttgtgtttc tcgatacgga gatgctgggt acagtgctaa tacgttgaac tacttatact 1500
   tatatgaggc tcgaagaaag ctgacttgtg tatgacttat tctcaactac atccccagtc 1560
   acaataccac cactgcacta ccactacacc aaaaccatga tcaaaccacc catggacttc 1620
   ctggaggcag aagaacttgt tatggaaaag ctcaagagag agatcataac ttcgtatagc 1680
   atacattata cgaagttatc ctgcaggtaa aggaattcag gagagaccgg gttggcggcg 1740
   tatttgtgtc ccaaaaaaca gccccaattg ccccaattga ccccaaattg acccagtagc 1800
   gggcccaacc ccggcgagag cccccttcac cccacatatc aaacctcccc cggttcccac 1860
   acttgccgtt aagggcgtag ggtactgcag tctggaatct acgcttgttc agactttgta 1920
   ctagtttctt tgtctggcca tccgggtaac ccatgccgga cgcaaaatag actactgaaa 1980
   atttttttgc tttgtggttg ggactttagc caagggtata aaagaccacc gtccccgaat 2040
   tacctttcct cttcttttct ctctctcctt gtcaactcac acccgaaatc gttaagcatt 2100
   tccttctgag tataagaatc attcaccatg gacttcctgg aggcagaaga acttgttatg 2160
   gaaaagctca agagagagaa gccaagatac tatcaagaca tgtgtcgcaa cttaattaag 2220
   atgacgacat ttgcgagctg gacgaggaat agatggagcg tgtgttctga gtcgatgttt 2280
   tctatggagt tgtgagtgtt agtagacatg atgggtttat atatgatgaa tgaatagatg 2340
   tgattttgat ttgcacgatg gaattgagaa ctttgtaaac gtacatggga atgtatgaat 2400
   gtgggggttt tgtgactgga taactgacgg tcagtggacg ccgttgttca aatatccaag 2460
   agatgcgaga aactttgggt caagtgaaca tgtcctctct gttcaagtaa accatcaact 2520
   atgggtagta tatttagtaa ggacaagagt tgagattctt tggagtccta gaaacgtatt 2580
   ttcgcgttcc aagatcaaat tagtagagta atacgggcac gggaatccat tcatagtctc 2640
   aattttccca taggtgtgct acaaggtgtt gagatgtggt acagtaccac catgattcga 2700
   ggtaaagagc ccagaagtca ttgatgaggt caagaaatac acagatctac agctcaatac 2760
   aatgaatatc ttctttcata ttcttcaggt gacaccaagg gtgtctattt tccccagaaa 2820
   tgcgtgaaaa ggcgcgtgtg tagcgtggag tatgggttcg gttggcgtat ccttcatata 2880
   tcgacgaaat agtagggcaa gagatgacaa aaagtatcta tatgtagaca gcgtagaata 2940
   tggatttgat tggtataaat tcatttattg cgtgtctcac aaatactctc gataagttgg 3000
   ggttaaactg gagatggaac aatgtcgata tctcgacgca tgcgacgtcg ggcccaattc 3060
   gccctatagt gagtcgtatt acaattcact ggccgtcgtt ttacaacgtc gtgactggga 3120
   aaaccctggc gttacccaac ttaatcgcct tgcagcacat ccccctttcg ccagctggcg 3180
   taatagcgaa gaggcccgca ccgatcgccc ttcccaacag ttgcgcagcc tgaatggcga 3240
   atggacgcgc cctgtagcgg cgcattaagc gcggcgggtg tggtggttac gcgcagcgtg 3300
   accgctacac ttgccagcgc cctagcgccc gctcctttcg ctttcttccc ttcctttctc 3360
   gccacgttcg ccggctttcc ccgtcaagct ctaaatcggg ggctcccttt agggttccga 3420
   tttagtgctt tacggcacct cgaccccaaa aaacttgatt agggtgatgg ttcacgtagt 3480
   gggccatcgc cctgatagac ggtttttcgc cctttgacgt tggagtccac gttctttaat 3540
   agtggactct tgttccaaac tggaacaaca ctcaacccta tctcggtcta ttcttttgat 3600
   ttataaggga ttttgccgat ttcggcctat tggttaaaaa atgagctgat ttaacaaaaa 3660
   tttaacgcga attttaacaa aatattaacg cttacaattt cctgatgcgg tattttctcc 3720
   ttacgcatct gtgcggtatt tcacaccgca tcaggtggca cttttcgggg aaatgtgcgc 3780
   ggaaccccta tttgtttatt tttctaaata cattcaaata tgtatccgct catgagacaa 3840
   taaccctgat aaatgcttca ataatattga aaaaggaaga gtatgagtat tcaacatttc 3900
   cgtgtcgccc ttattccctt ttttgcggca ttttgccttc ctgtttttgc tcacccagaa 3960
   acgctggtga aagtaaaaga tgctgaagat cagttgggtg cacgagtggg ttacatcgaa 4020
   ctggatctca acagcggtaa gatccttgag agttttcgcc ccgaagaacg ttttccaatg 4080
   atgagcactt ttaaagttct gctatgtggc gcggtattat cccgtattga cgccgggcaa 4140
   gagcaactcg gtcgccgcat acactattct cagaatgact tggttgagta ctcaccagtc 4200
   acagaaaagc atcttacgga tggcatgaca gtaagagaat tatgcagtgc tgccataacc 4260
   atgagtgata acactgcggc caacttactt ctgacaacga tcggaggacc gaaggagcta 4320
   accgcttttt tgcacaacat gggggatcat gtaactcgcc ttgatcgttg ggaaccggag 4380
   ctgaatgaag ccataccaaa cgacgagcgt gacaccacga tgcctgtagc aatggcaaca 4440
   acgttgcgca aactattaac tggcgaacta cttactctag cttcccggca acaattaata 4500
   gactggatgg aggcggataa agttgcagga ccacttctgc gctcggccct tccggctggc 4560
   tggtttattg ctgataaatc tggagccggt gagcgtgggt ctcgcggtat cattgcagca 4620
   ctggggccag atggtaagcc ctcccgtatc gtagttatct acacgacggg gagtcaggca 4680
   actatggatg aacgaaatag acagatcgct gagataggtg cctcactgat taagcattgg 4740
   taactgtcag accaagttta ctcatatata ctttagattg atttaaaact tcatttttaa 4800
   tttaaaagga tctaggtgaa gatccttttt gataatctca tgaccaaaat cccttaacgt 4860
   gagttttcgt tccactgagc gtcagacccc gtagaaaaga tcaaaggatc ttcttgagat 4920
   cctttttttc tgcgcgtaat ctgctgcttg caaacaaaaa aaccaccgct accagcggtg 4980
   gtttgtttgc cggatcaaga gctaccaact ctttttccga aggtaactgg cttcagcaga 5040
   gcgcagatac caaatactgt tcttctagtg tagccgtagt taggccacca cttcaagaac 5100
   tctgtagcac cgcctacata cctcgctctg ctaatcctgt taccagtggc tgctgccagt 5160
   ggcgataagt cgtgtcttac cgggttggac tcaagacgat agttaccgga taaggcgcag 5220
   cggtcgggct gaacgggggg ttcgtgcaca cagcccagct tggagcgaac gacctacacc 5280
   gaactgagat acctacagcg tgagctatga gaaagcgcca cgcttcccga agggagaaag 5340
   gcggacaggt atccggtaag cggcagggtc ggaacaggag agcgcacgag ggagcttcca 5400
   gggggaaacg cctggtatct ttatagtcct gtcgggtttc gccacctctg acttgagcgt 5460
   cgatttttgt gatgctcgtc aggggggcgg agcctatgga aaaacgccag caacgcggcc 5520
   tttttacggt tcctggcctt ttgctggcct tttgctcaca tgttctttcc tgcgttatcc 5580
   cctgattctg tggataaccg tattaccgcc tttgagtgag ctgataccgc tcgccgcagc 5640
   cgaacgaccg agcgcagcga gtcagtgagc gaggaagcgg aagagcgccc aatacgcaaa 5700
   ccgcctctcc ccgcgcgttg gccgattcat taatgcagct ggcgcgccac caatcacaat 5760
   tctgaaaagc acatcttgat ctcctcattg cggggagtcc aacggtggtc ttattccccc 5820
   gaatttcccg ctcaatctcg ttccagaccg acccggacac agtgcttaac gccgttccga 5880
   aactctaccg cagatatgct ccaacggact gggctgcata gatgtgatcc tcggcttgga 5940
   gaaatggata aaagccggcc aaaaaaaaag cggaaaaaag cggaaaaaaa gagaaaaaaa 6000
   atcgcaaaat ttgaaaaata gggggaaaag acgcaaaaac gcaaggaggg gggagtatat 6060
   gacactgata agcaagctca caacggttcc tcttattttt ttcctcatct tctgcctagg 6120
   ttcccaaaat cccagatgct tctctccagt gccaaaagta agtaccccac aggttttcgg 6180
   ccgaaaattc cacgtgcagc aacgtcgtgt ggggtgttaa aatgtggggg gggggaacca 6240
   ggacaagagg ctcttgtggg agccgaatga gagcacaaag cgggcgggtg tgataagggc 6300
   atttttgccc attttccctt ctcctgtctc tccgacggtg atggcgttgt gcgtcctcta 6360
   tttcttttta tttctttttg ttttatttct ctgactaccg atttggtttg atttcctcaa 6420
   ccccacacaa ataagctcgg gccgaggaat atatatatac acggacacag tcgccctgtg 6480
   gacaacacgt cactacctct acgatacaca ccgtacgata gttagtagac aacaatcgat 6540
   agttggagca agggagaaat gtagagtgtg aaagactcac tatggtccgg gcttatctcg 6600
   accaatagcc aaagtctgga gtttctgaga gaaaaaggca agatacgtat gtaacaaagc 6660
   gacgcatggt acaataatac cggaggcatg tatcatagag agttagtggt tcgatgatgg 6720
   cactggtgcc tggtatgact ttatacggct gactacatat ttgtcctcag acatacaatt 6780
   acagtcaagc acttaccctt ggacatctgt aggtaccccc cggccaagac gatctcagcg 6840
   tgtcgtatgt cggattggcg tagctccctc gctcgtcaat tggctcccat ctactttctt 6900
   ctgcttggct acacccagca tgtctgctat ggctcgtttt cgtgccttat ctatcctccc 6960
   agtattacca actctaaatg acatgatgtg attgggtcta cactttcata tcagagataa 7020
   ggagtagcac agttgcataa aaagcccaac tctaatcagc ttcttccttt cttgtaatta 7080
   gtacaaaggt gattagcgaa atctggaagc ttagttggcc ctaaaaaaat caaaaaaagc 7140
   aaaaaacgaa aaacgaaaaa ccacagtttt gagaacaggg aggtaacgaa ggatcgtata 7200
   tatatatata tatatatata cccacggatc ccgagaccgg cctttgattc ttccctacaa 7260
   ccaaccattc tcaccaccct aattcacaac catggccttt ccctgggcag ataagtgggc 7320
   agccgatgcg tctgcatcta cagggctgcc tccggacctc ctcaagattg cattcactct 7380
   ggtcatgtct tatccgctga gttctctcat gaaacggctg ccagatgacg ccaaaaacct 7440
   caagatcatc tatatcatct ccgtgtccat cttctacatg gtgggtgtct tctccctcta 7500
   tggcggagct gccactctgc tcttctcctc aatgggtacc ttcttcatca cccaatggaa 7560
   gagcccttac atgccctggg tcaattttgg ttttgtcatg acccatctct tcgtcaatca 7620
   cctgcgttcg cagtttttcc ccgaaacata cgaccccaat gtcattgaca tcaccggagc 7680
   acagatggtt ctgtgtatga agctatcgtc ttttggatgg aacgtctacg atggatggca 7740
   gattgagaag ggtgagcagc tcagcgagtt ccagactaaa agggctgttc tcaagcaccc 7800
   cagtcttatg gacttcctag cttttgtgtt ctacttccct tccattctga caggtccttc 7860
   ttacgactat atggagttcc ataactggct cgatctcagc ctgttcaagg agctggagaa 7920
   agataaggac cccaagcgag ctgctcgacg aaagcgacac aagatccccc gatctggaat 7980
   cgctgcttcc aagaaactcg ccgctggtat cttctggatc gttctgtgga cccaggtgga 8040
   ctctcgaatc tccaccgcct acgcttactc agacgcattc accaaggagc acaacatctt 8100
   tggacgaatt gtgtacctct acatgctcgg tttcatgtac cgactcaagt actacggagc 8160
   ctggtccatt tccgagggag cctgcatctt gtctggcctc ggattccatg gcgtggaccc 8220
   caaaactggc aagtacaagt gggaccgtgt ccagaacgtg gacccgtggg gattcgaaac 8280
   tggtcaaaac acaaaggctc tgctggaggc ctggaaccag aacactaaca agtggctacg 8340
   aaactatgtg tacctccgag tggtgcccaa aggccaaaag cctggattcc gagccactat 8400
   cttcacattt gtggtttccg ccttctggca tggaactcga cctggctact atctcacctt 8460
   tgtgaccgct gccatgtacc agtctgttgg taagttcttc cgacgatacc tgcgaccctt 8520
   cttcatggag tctgatggaa agactgccgg tccctataag atctactacg acattgtgtg 8580
   ttggatcgtt gtccaaaccg catttggata cgctacccag tcctttatga ttctagactt 8640
   ctggctgtcg ctcaagtgtt ggaagaactc ctggttcctg taccacattg ctctgggcgc 8700
   catctttgca atttctagcc cctacaaggc atgggcgatt cccaagatca agaaaaagca 8760
   ggctggagcc gtcactgaca agaaggacgc caaggaggag gtgaagaagg acaccatcaa 8820
   gaccaagtaa gcggccgcat gagaagataa atatataaat acattgagat attaaatgcg 8880
   ctagattaga gagcctcata ctgctcggag agaagccaag acgagtactc aaaggggatt 8940
   acaccatcca tatccacaga cacaagctgg ggaaaggttc tatatacact ttccggaata 9000
   ccgtagtttc cgatgttatc aatgggggca gccaggattt caggcacttc ggtgtctcgg 9060
   ggtgaaatgg cgttcttggc ctccatcaag tcgtaccatg tcttcatttg cctgtcaaag 9120
   taaaacagaa gcagatgaag aatgaacttg aagtgaagga atttaaatgt aacgaaactg 9180
   aaatttgacc agatattgtg tccgcggtgg agctccagct tttgttccct ttagtgaggg 9240
   ttaatttcga gcttggcgta atcatggtca tagctgtttc ctgtgtgaaa ttgttatccg 9300
   ctcacaagct tccacacaac 9320
<210> 146
   <211> 8726
   <212> DNA
   <213> Artificial Sequence
<220>
   <223> Plasmid pY208
<400> 146
   gtacgataac ttcgtatagc atacattata cgaagttatc gcgtcgacga gtatctgtct 60
   gactcgtcat tgccgccttt ggagtacgac tccaactatg agtgtgcttg gatcactttg 120
   acgatacatt cttcgttgga ggctgtgggt ctgacagctg cgttttcggc gcggttggcc 180
   gacaacaata tcagctgcaa cgtcattgct ggctttcatc atgatcacat ttttgtcggc 240
   aaaggcgacg cccagagagc cattgacgtt ctttctaatt tggaccgata gccgtatagt 300
   ccagtctatc tataagttca actaactcgt aactattacc ataacatata cttcactgcc 360
   ccagataagg ttccgataaa aagttctgca gactaaattt atttcagtct cctcttcacc 420
   accaaaatgc cctcctacga agctcgagct aacgtccaca agtccgcctt tgccgctcga 480
   gtgctcaagc tcgtggcagc caagaaaacc aacctgtgtg cttctctgga tgttaccacc 540
   accaaggagc tcattgagct tgccgataag gtcggacctt atgtgtgcat gatcaaaacc 600
   catatcgaca tcattgacga cttcacctac gccggcactg tgctccccct caaggaactt 660
   gctcttaagc acggtttctt cctgttcgag gacagaaagt tcgcagatat tggcaacact 720
   gtcaagcacc agtaccggtg tcaccgaatc gccgagtggt ccgatatcac caacgcccac 780
   ggtgtacccg gaaccggaat cattgctggc ctgcgagctg gtgccgagga aactgtctct 840
   gaacagaaga aggaggacgt ctctgactac gagaactccc agtacaagga gttcctagtc 900
   ccctctccca acgagaagct ggccagaggt ctgctcatgc tggccgagct gtcttgcaag 960
   ggctctctgg ccactggcga gtactccaag cagaccattg agcttgcccg atccgacccc 1020
   gagtttgtgg ttggcttcat tgcccagaac cgacctaagg gcgactctga ggactggctt 1080
   attctgaccc ccggggtggg tcttgacgac aagggagacg ctctcggaca gcagtaccga 1140
   actgttgagg atgtcatgtc taccggaacg gatatcataa ttgtcggccg aggtctgtac 1200
   ggccagaacc gagatcctat tgaggaggcc aagcgatacc agaaggctgg ctgggaggct 1260
   taccagaaga ttaactgtta gaggttagac tatggatatg taatttaact gtgtatatag 1320
   agagcgtgca agtatggagc gcttgttcag cttgtatgat ggtcagacga cctgtctgat 1380
   cgagtatgta tgatactgca caacctgtgt atccgcatga tctgtccaat ggggcatgtt 1440
   gttgtgtttc tcgatacgga gatgctgggt acagtgctaa tacgttgaac tacttatact 1500
   tatatgaggc tcgaagaaag ctgacttgtg tatgacttat tctcaactac atccccagtc 1560
   acaataccac cactgcacta ccactacacc aaaaccatga tcaaaccacc catggacttc 1620
   ctggaggcag aagaacttgt tatggaaaag ctcaagagag agatcataac ttcgtatagc 1680
   atacattata cgaagttatc ctgcaggtaa aggaattcag gagagaccgg gttggcggcg 1740
   tatttgtgtc ccaaaaaaca gccccaattg ccccaattga ccccaaattg acccagtagc 1800
   gggcccaacc ccggcgagag cccccttcac cccacatatc aaacctcccc cggttcccac 1860
   acttgccgtt aagggcgtag ggtactgcag tctggaatct acgcttgttc agactttgta 1920
   ctagtttctt tgtctggcca tccgggtaac ccatgccgga cgcaaaatag actactgaaa 1980
   atttttttgc tttgtggttg ggactttagc caagggtata aaagaccacc gtccccgaat 2040
   tacctttcct cttcttttct ctctctcctt gtcaactcac acccgaaatc gttaagcatt 2100
   tccttctgag tataagaatc attcaccatg gacttcctgg aggcagaaga acttgttatg 2160
   gaaaagctca agagagagaa gccaagatac tatcaagaca tgtgtcgcaa cttaattaag 2220
   atgacgacat ttgcgagctg gacgaggaat agatggagcg tgtgttctga gtcgatgttt 2280
   tctatggagt tgtgagtgtt agtagacatg atgggtttat atatgatgaa tgaatagatg 2340
   tgattttgat ttgcacgatg gaattgagaa ctttgtaaac gtacatggga atgtatgaat 2400
   gtgggggttt tgtgactgga taactgacgg tcagtggacg ccgttgttca aatatccaag 2460
   agatgcgaga aactttgggt caagtgaaca tgtcctctct gttcaagtaa accatcaact 2520
   atgggtagta tatttagtaa ggacaagagt tgagattctt tggagtccta gaaacgtatt 2580
   ttcgcgttcc aagatcaaat tagtagagta atacgggcac gggaatccat tcatagtctc 2640
   aattttccca taggtgtgct acaaggtgtt gagatgtggt acagtaccac catgattcga 2700
   ggtaaagagc ccagaagtca ttgatgaggt caagaaatac acagatctac agctcaatac 2760
   aatgaatatc ttctttcata ttcttcaggt gacaccaagg gtgtctattt tccccagaaa 2820
   tgcgtgaaaa ggcgcgtgtg tagcgtggag tatgggttcg gttggcgtat ccttcatata 2880
   tcgacgaaat agtagggcaa gagatgacaa aaagtatcta tatgtagaca gcgtagaata 2940
   tggatttgat tggtataaat tcatttattg cgtgtctcac aaatactctc gataagttgg 3000
   ggttaaactg gagatggaac aatgtcgata tctcgacgca tgcgacgtcg ggcccaattc 3060
   gccctatagt gagtcgtatt acaattcact ggccgtcgtt ttacaacgtc gtgactggga 3120
   aaaccctggc gttacccaac ttaatcgcct tgcagcacat ccccctttcg ccagctggcg 3180
   taatagcgaa gaggcccgca ccgatcgccc ttcccaacag ttgcgcagcc tgaatggcga 3240
   atggacgcgc cctgtagcgg cgcattaagc gcggcgggtg tggtggttac gcgcagcgtg 3300
   accgctacac ttgccagcgc cctagcgccc gctcctttcg ctttcttccc ttcctttctc 3360
   gccacgttcg ccggctttcc ccgtcaagct ctaaatcggg ggctcccttt agggttccga 3420
   tttagtgctt tacggcacct cgaccccaaa aaacttgatt agggtgatgg ttcacgtagt 3480
   gggccatcgc cctgatagac ggtttttcgc cctttgacgt tggagtccac gttctttaat 3540
   agtggactct tgttccaaac tggaacaaca ctcaacccta tctcggtcta ttcttttgat 3600
   ttataaggga ttttgccgat ttcggcctat tggttaaaaa atgagctgat ttaacaaaaa 3660
   tttaacgcga attttaacaa aatattaacg cttacaattt cctgatgcgg tattttctcc 3720
   ttacgcatct gtgcggtatt tcacaccgca tcaggtggca cttttcgggg aaatgtgcgc 3780
   ggaaccccta tttgtttatt tttctaaata cattcaaata tgtatccgct catgagacaa 3840
   taaccctgat aaatgcttca ataatattga aaaaggaaga gtatgagtat tcaacatttc 3900
   cgtgtcgccc ttattccctt ttttgcggca ttttgccttc ctgtttttgc tcacccagaa 3960
   acgctggtga aagtaaaaga tgctgaagat cagttgggtg cacgagtggg ttacatcgaa 4020
   ctggatctca acagcggtaa gatccttgag agttttcgcc ccgaagaacg ttttccaatg 4080
   atgagcactt ttaaagttct gctatgtggc gcggtattat cccgtattga cgccgggcaa 4140
   gagcaactcg gtcgccgcat acactattct cagaatgact tggttgagta ctcaccagtc 4200
   acagaaaagc atcttacgga tggcatgaca gtaagagaat tatgcagtgc tgccataacc 4260
   atgagtgata acactgcggc caacttactt ctgacaacga tcggaggacc gaaggagcta 4320
   accgcttttt tgcacaacat gggggatcat gtaactcgcc ttgatcgttg ggaaccggag 4380
   ctgaatgaag ccataccaaa cgacgagcgt gacaccacga tgcctgtagc aatggcaaca 4440
   acgttgcgca aactattaac tggcgaacta cttactctag cttcccggca acaattaata 4500
   gactggatgg aggcggataa agttgcagga ccacttctgc gctcggccct tccggctggc 4560
   tggtttattg ctgataaatc tggagccggt gagcgtgggt ctcgcggtat cattgcagca 4620
   ctggggccag atggtaagcc ctcccgtatc gtagttatct acacgacggg gagtcaggca 4680
   actatggatg aacgaaatag acagatcgct gagataggtg cctcactgat taagcattgg 4740
   taactgtcag accaagttta ctcatatata ctttagattg atttaaaact tcatttttaa 4800
   tttaaaagga tctaggtgaa gatccttttt gataatctca tgaccaaaat cccttaacgt 4860
   gagttttcgt tccactgagc gtcagacccc gtagaaaaga tcaaaggatc ttcttgagat 4920
   cctttttttc tgcgcgtaat ctgctgcttg caaacaaaaa aaccaccgct accagcggtg 4980
   gtttgtttgc cggatcaaga gctaccaact ctttttccga aggtaactgg cttcagcaga 5040
   gcgcagatac caaatactgt tcttctagtg tagccgtagt taggccacca cttcaagaac 5100
   tctgtagcac cgcctacata cctcgctctg ctaatcctgt taccagtggc tgctgccagt 5160
   ggcgataagt cgtgtcttac cgggttggac tcaagacgat agttaccgga taaggcgcag 5220
   cggtcgggct gaacgggggg ttcgtgcaca cagcccagct tggagcgaac gacctacacc 5280
   gaactgagat acctacagcg tgagctatga gaaagcgcca cgcttcccga agggagaaag 5340
   gcggacaggt atccggtaag cggcagggtc ggaacaggag agcgcacgag ggagcttcca 5400
   gggggaaacg cctggtatct ttatagtcct gtcgggtttc gccacctctg acttgagcgt 5460
   cgatttttgt gatgctcgtc aggggggcgg agcctatgga aaaacgccag caacgcggcc 5520
   tttttacggt tcctggcctt ttgctggcct tttgctcaca tgttctttcc tgcgttatcc 5580
   cctgattctg tggataaccg tattaccgcc tttgagtgag ctgataccgc tcgccgcagc 5640
   cgaacgaccg agcgcagcga gtcagtgagc gaggaagcgg aagagcgccc aatacgcaaa 5700
   ccgcctctcc ccgcgcgttg gccgattcat taatgcagct ggcgcgccac caatcacaat 5760
   tctgaaaagc acatcttgat ctcctcattg cggggagtcc aacggtggtc ttattccccc 5820
   gaatttcccg ctcaatctcg ttccagaccg acccggacac agtgcttaac gccgttccga 5880
   aactctaccg cagatatgct ccaacggact gggctgcata gatgtgatcc tcggcttgga 5940
   gaaatggata aaagccggcc aaaaaaaaag cggaaaaaag cggaaaaaaa gagaaaaaaa 6000
   atcgcaaaat ttgaaaaata gggggaaaag acgcaaaaac gcaaggaggg gggagtatat 6060
   gacactgata agcaagctca caacggttcc tcttattttt ttcctcatct tctgcctagg 6120
   ttcccaaaat cccagatgct tctctccagt gccaaaagta agtaccccac aggttttcgg 6180
   ccgaaaattc cacgtgcagc aacgtcgtgt ggggtgttaa aatgtggggg gggggaacca 6240
   ggacaagagg ctcttgtggg agccgaatga gagcacaaag cgggcgggtg tgataagggc 6300
   atttttgccc attttccctt ctcctgtctc tccgacggtg atggcgttgt gcgtcctcta 6360
   tttcttttta tttctttttg ttttatttct ctgactaccg atttggtttg atttcctcaa 6420
   ccccacacaa ataagctcgg gccgaggaat atatatatac acggacacag tcgccctgtg 6480
   gacaacacgt cactacctct acgatacaca ccgtacgata gttagtagac aacaatcgat 6540
   agttggagca agggagaaat gtagagtgtg aaagactcac tatggtccgg gcttatctcg 6600
   accaatagcc aaagtctgga gtttctgaga gaaaaaggca agatacgtat gtaacaaagc 6660
   gacgcatggt acaataatac cggaggcatg tatcatagag agttagtggt tcgatgatgg 6720
   cactggtgcc tggtatgact ttatacggct gactacatat ttgtcctcag acatacaatt 6780
   acagtcaagc acttaccctt ggacatctgt aggtaccccc cggccaagac gatctcagcg 6840
   tgtcgtatgt cggattggcg tagctccctc gctcgtcaat tggctcccat ctactttctt 6900
   ctgcttggct acacccagca tgtctgctat ggctcgtttt cgtgccttat ctatcctccc 6960
   agtattacca actctaaatg acatgatgtg attgggtcta cactttcata tcagagataa 7020
   ggagtagcac agttgcataa aaagcccaac tctaatcagc ttcttccttt cttgtaatta 7080
   gtacaaaggt gattagcgaa atctggaagc ttagttggcc ctaaaaaaat caaaaaaagc 7140
   aaaaaacgaa aaacgaaaaa ccacagtttt gagaacaggg aggtaacgaa ggatcgtata 7200
   tatatatata tatatatata cccacggatc ccgagaccgg cctttgattc ttccctacaa 7260
   ccaaccattc tcaccaccct aattcacaac catgtctatt ggttcgtcca accccgtgct 7320
   cttggctgcg attcccttcg tctacctgtt tgtcctccca cgagtcctgg ctttcctgcc 7380
   tcagaaggct cagttcctgg ccaaatgtat tgtggtcctg attgccacgc ttatcatgtc 7440
   cgttgcaggc tgcttcatct cgatcgtgtg cgctcttctg gacaagagat acgtcatcaa 7500
   ttacgttgtg tcgcgattgt tctccttcct tgccgctcga ccgtgtggtg tgacctataa 7560
   gattgttggt gaggaacacc tcgataagta ccctgctatc gtggtctgta accatcaatc 7620
   ctctatggat atgatggttt tgggacgagt tttccaaaag cactgcgttg tcatggcgaa 7680
   gaaggaactc ctgtactttc cctttttggg aatgtttatg aaactgagca acgctatctt 7740
   catcgaccgg aagaaccaca agaaagccat cgagtctacc acccaagccg tggcggacat 7800
   gaagaagcac aactctggaa tctggatttt cccagagggc acccggtcta gactggacaa 7860
   ggcagacctg ctgcccttca agaaaggtgc ctttcatctt gcaattcagg cccagctccc 7920
   tattctcccc attatctcgc agggctattc ccatatctac gactcttcga agcggtactt 7980
   ccccggtgga gagctcgaga tcagagtcct ggagcccatt cctacaactg gcctcactac 8040
   tgatgatgtg aacgacctga tggacaagac acgaaacctt atgctcaagc acttgaagga 8100
   gatggattcc cagtattcgt cgagcactgc tgaaaatgga tccacgcaca tcgacgccga 8160
   tattgccaag tctacagcca ccagcattgg caacactgac gacgcaatta caaaacgtcg 8220
   tacccctaag gaataagcgg ccgcatgaga agataaatat ataaatacat tgagatatta 8280
   aatgcgctag attagagagc ctcatactgc tcggagagaa gccaagacga gtactcaaag 8340
   gggattacac catccatatc cacagacaca agctggggaa aggttctata tacactttcc 8400
   ggaataccgt agtttccgat gttatcaatg ggggcagcca ggatttcagg cacttcggtg 8460
   tctcggggtg aaatggcgtt cttggcctcc atcaagtcgt accatgtctt catttgcctg 8520
   tcaaagtaaa acagaagcag atgaagaatg aacttgaagt gaaggaattt aaatgtaacg 8580
   aaactgaaat ttgaccagat attgtgtccg cggtggagct ccagcttttg ttccctttag 8640
   tgagggttaa tttcgagctt ggcgtaatca tggtcatagc tgtttcctgt gtgaaattgt 8700
   tatccgctca caagcttcca cacaac 8726
<210> 147
   <211> 31
   <212> DNA
   <213> Artificial Sequence
<220>
   <223> Primer 856
<400> 147
   tcacaacaca tgtccgttgc atccaagctc g 31
<210> 148
   <211> 28
   <212> DNA
   <213> Artificial Sequence
<220>
   <223> Primer 857
<400> 148
   tttagcggcc gcctactgag tcttctgg 28
<210> 149
   <211> 8630
   <212> DNA
   <213> Artificial Sequence
<220>
   <223> Plasmid pY207
<400> 149
   gtacgataac ttcgtatagc atacattata cgaagttatc gcgtcgacga gtatctgtct 60
   gactcgtcat tgccgccttt ggagtacgac tccaactatg agtgtgcttg gatcactttg 120
   acgatacatt cttcgttgga ggctgtgggt ctgacagctg cgttttcggc gcggttggcc 180
   gacaacaata tcagctgcaa cgtcattgct ggctttcatc atgatcacat ttttgtcggc 240
   aaaggcgacg cccagagagc cattgacgtt ctttctaatt tggaccgata gccgtatagt 300
   ccagtctatc tataagttca actaactcgt aactattacc ataacatata cttcactgcc 360
   ccagataagg ttccgataaa aagttctgca gactaaattt atttcagtct cctcttcacc 420
   accaaaatgc cctcctacga agctcgagct aacgtccaca agtccgcctt tgccgctcga 480
   gtgctcaagc tcgtggcagc caagaaaacc aacctgtgtg cttctctgga tgttaccacc 540
   accaaggagc tcattgagct tgccgataag gtcggacctt atgtgtgcat gatcaaaacc 600
   catatcgaca tcattgacga cttcacctac gccggcactg tgctccccct caaggaactt 660
   gctcttaagc acggtttctt cctgttcgag gacagaaagt tcgcagatat tggcaacact 720
   gtcaagcacc agtaccggtg tcaccgaatc gccgagtggt ccgatatcac caacgcccac 780
   ggtgtacccg gaaccggaat cattgctggc ctgcgagctg gtgccgagga aactgtctct 840
   gaacagaaga aggaggacgt ctctgactac gagaactccc agtacaagga gttcctagtc 900
   ccctctccca acgagaagct ggccagaggt ctgctcatgc tggccgagct gtcttgcaag 960
   ggctctctgg ccactggcga gtactccaag cagaccattg agcttgcccg atccgacccc 1020
   gagtttgtgg ttggcttcat tgcccagaac cgacctaagg gcgactctga ggactggctt 1080
   attctgaccc ccggggtggg tcttgacgac aagggagacg ctctcggaca gcagtaccga 1140
   actgttgagg atgtcatgtc taccggaacg gatatcataa ttgtcggccg aggtctgtac 1200
   ggccagaacc gagatcctat tgaggaggcc aagcgatacc agaaggctgg ctgggaggct 1260
   taccagaaga ttaactgtta gaggttagac tatggatatg taatttaact gtgtatatag 1320
   agagcgtgca agtatggagc gcttgttcag cttgtatgat ggtcagacga cctgtctgat 1380
   cgagtatgta tgatactgca caacctgtgt atccgcatga tctgtccaat ggggcatgtt 1440
   gttgtgtttc tcgatacgga gatgctgggt acagtgctaa tacgttgaac tacttatact 1500
   tatatgaggc tcgaagaaag ctgacttgtg tatgacttat tctcaactac atccccagtc 1560
   acaataccac cactgcacta ccactacacc aaaaccatga tcaaaccacc catggacttc 1620
   ctggaggcag aagaacttgt tatggaaaag ctcaagagag agatcataac ttcgtatagc 1680
   atacattata cgaagttatc ctgcaggtaa aggaattcag gagagaccgg gttggcggcg 1740
   tatttgtgtc ccaaaaaaca gccccaattg ccccaattga ccccaaattg acccagtagc     1800
   gggcccaacc ccggcgagag cccccttcac cccacatatc aaacctcccc cggttcccac     1860
   acttgccgtt aagggcgtag ggtactgcag tctggaatct acgcttgttc agactttgta 1920
   ctagtttctt tgtctggcca tccgggtaac ccatgccgga cgcaaaatag actactgaaa 1980
   atttttttgc tttgtggttg ggactttagc caagggtata aaagaccacc gtccccgaat 2040
   tacctttcct cttcttttct ctctctcctt gtcaactcac acccgaaatc gttaagcatt 2100
   tccttctgag tataagaatc attcaccatg gacttcctgg aggcagaaga acttgttatg 2160
   gaaaagctca agagagagaa gccaagatac tatcaagaca tgtgtcgcaa cttaattaag 2220
   atgacgacat ttgcgagctg gacgaggaat agatggagcg tgtgttctga gtcgatgttt 2280
   tctatggagt tgtgagtgtt agtagacatg atgggtttat atatgatgaa tgaatagatg 2340
   tgattttgat ttgcacgatg gaattgagaa ctttgtaaac gtacatggga atgtatgaat 2400
   gtgggggttt tgtgactgga taactgacgg tcagtggacg ccgttgttca aatatccaag 2460
   agatgcgaga aactttgggt caagtgaaca tgtcctctct gttcaagtaa accatcaact 2520
   atgggtagta tatttagtaa ggacaagagt tgagattctt tggagtccta gaaacgtatt 2580
   ttcgcgttcc aagatcaaat tagtagagta atacgggcac gggaatccat tcatagtctc 2640
   aattttccca taggtgtgct acaaggtgtt gagatgtggt acagtaccac catgattcga 2700
   ggtaaagagc ccagaagtca ttgatgaggt caagaaatac acagatctac agctcaatac 2760
   aatgaatatc ttctttcata ttcttcaggt gacaccaagg gtgtctattt tccccagaaa 2820
   tgcgtgaaaa ggcgcgtgtg tagcgtggag tatgggttcg gttggcgtat ccttcatata 2880
   tcgacgaaat agtagggcaa gagatgacaa aaagtatcta tatgtagaca gcgtagaata 2940
   tggatttgat tggtataaat tcatttattg cgtgtctcac aaatactctc gataagttgg 3000
   ggttaaactg gagatggaac aatgtcgata tctcgacgca tgcgacgtcg ggcccaattc 3060
   gccctatagt gagtcgtatt acaattcact ggccgtcgtt ttacaacgtc gtgactggga 3120
   aaaccctggc gttacccaac ttaatcgcct tgcagcacat ccccctttcg ccagctggcg 3180
   taatagcgaa gaggcccgca ccgatcgccc ttcccaacag ttgcgcagcc tgaatggcga 3240
   atggacgcgc cctgtagcgg cgcattaagc gcggcgggtg tggtggttac gcgcagcgtg 3300
   accgctacac ttgccagcgc cctagcgccc gctcctttcg ctttcttccc ttcctttctc 3360
   gccacgttcg ccggctttcc ccgtcaagct ctaaatcggg ggctcccttt agggttccga 3420
   tttagtgctt tacggcacct cgaccccaaa aaacttgatt agggtgatgg ttcacgtagt 3480
   gggccatcgc cctgatagac ggtttttcgc cctttgacgt tggagtccac gttctttaat 3540
   agtggactct tgttccaaac tggaacaaca ctcaacccta tctcggtcta ttcttttgat 3600
   ttataaggga ttttgccgat ttcggcctat tggttaaaaa atgagctgat ttaacaaaaa 3660
   tttaacgcga attttaacaa aatattaacg cttacaattt cctgatgcgg tattttctcc 3720
   ttacgcatct gtgcggtatt tcacaccgca tcaggtggca cttttcgggg aaatgtgcgc 3780
   ggaaccccta tttgtttatt tttctaaata cattcaaata tgtatccgct catgagacaa 3840
   taaccctgat aaatgcttca ataatattga aaaaggaaga gtatgagtat tcaacatttc 3900
   cgtgtcgccc ttattccctt ttttgcggca ttttgccttc ctgtttttgc tcacccagaa 3960
   acgctggtga aagtaaaaga tgctgaagat cagttgggtg cacgagtggg ttacatcgaa 4020
   ctggatctca acagcggtaa gatccttgag agttttcgcc ccgaagaacg ttttccaatg 4080
   atgagcactt ttaaagttct gctatgtggc gcggtattat cccgtattga cgccgggcaa 4140
   gagcaactcg gtcgccgcat acactattct cagaatgact tggttgagta ctcaccagtc 4200
   acagaaaagc atcttacgga tggcatgaca gtaagagaat tatgcagtgc tgccataacc 4260
   atgagtgata acactgcggc caacttactt ctgacaacga tcggaggacc gaaggagcta 4320
   accgcttttt tgcacaacat gggggatcat gtaactcgcc ttgatcgttg ggaaccggag 4380
   ctgaatgaag ccataccaaa cgacgagcgt gacaccacga tgcctgtagc aatggcaaca 4440
   acgttgcgca aactattaac tggcgaacta cttactctag cttcccggca acaattaata 4500
   gactggatgg aggcggataa agttgcagga ccacttctgc gctcggccct tccggctggc 4560
   tggtttattg ctgataaatc tggagccggt gagcgtgggt ctcgcggtat cattgcagca 4620
   ctggggccag atggtaagcc ctcccgtatc gtagttatct acacgacggg gagtcaggca 4680
   actatggatg aacgaaatag acagatcgct gagataggtg cctcactgat taagcattgg 4740
   taactgtcag accaagttta ctcatatata ctttagattg atttaaaact tcatttttaa 4800
   tttaaaagga tctaggtgaa gatccttttt gataatctca tgaccaaaat cccttaacgt 4860
   gagttttcgt tccactgagc gtcagacccc gtagaaaaga tcaaaggatc ttcttgagat 4920
   cctttttttc tgcgcgtaat ctgctgcttg caaacaaaaa aaccaccgct accagcggtg 4980
   gtttgtttgc cggatcaaga gctaccaact ctttttccga aggtaactgg cttcagcaga 5040
   gcgcagatac caaatactgt tcttctagtg tagccgtagt taggccacca cttcaagaac 5100
   tctgtagcac cgcctacata cctcgctctg ctaatcctgt taccagtggc tgctgccagt 5160
   ggcgataagt cgtgtcttac cgggttggac tcaagacgat agttaccgga taaggcgcag 5220
   cggtcgggct gaacgggggg ttcgtgcaca cagcccagct tggagcgaac gacctacacc 5280
   gaactgagat acctacagcg tgagctatga gaaagcgcca cgcttcccga agggagaaag 5340
   gcggacaggt atccggtaag cggcagggtc ggaacaggag agcgcacgag ggagcttcca 5400
   gggggaaacg cctggtatct ttatagtcct gtcgggtttc gccacctctg acttgagcgt 5460
   cgatttttgt gatgctcgtc aggggggcgg agcctatgga aaaacgccag caacgcggcc 5520
   tttttacggt tcctggcctt ttgctggcct tttgctcaca tgttctttcc tgcgttatcc 5580
   cctgattctg tggataaccg tattaccgcc tttgagtgag ctgataccgc tcgccgcagc 5640
   cgaacgaccg agcgcagcga gtcagtgagc gaggaagcgg aagagcgccc aatacgcaaa 5700
   ccgcctctcc ccgcgcgttg gccgattcat taatgcagct ggcgcgccac caatcacaat 5760
   tctgaaaagc acatcttgat ctcctcattg cggggagtcc aacggtggtc ttattccccc 5820
   gaatttcccg ctcaatctcg ttccagaccg acccggacac agtgcttaac gccgttccga 5880
   aactctaccg cagatatgct ccaacggact gggctgcata gatgtgatcc tcggcttgga 5940
   gaaatggata aaagccggcc aaaaaaaaag cggaaaaaag cggaaaaaaa gagaaaaaaa 6000
   atcgcaaaat ttgaaaaata gggggaaaag acgcaaaaac gcaaggaggg gggagtatat 6060
   gacactgata agcaagctca caacggttcc tcttattttt ttcctcatct tctgcctagg 6120
   ttcccaaaat cccagatgct tctctccagt gccaaaagta agtaccccac aggttttcgg 6180
   ccgaaaattc cacgtgcagc aacgtcgtgt ggggtgttaa aatgtggggg gggggaacca 6240
   ggacaagagg ctcttgtggg agccgaatga gagcacaaag cgggcgggtg tgataagggc 6300
   atttttgccc attttccctt ctcctgtctc tccgacggtg atggcgttgt gcgtcctcta 6360
   tttcttttta tttctttttg ttttatttct ctgactaccg atttggtttg atttcctcaa 6420
   ccccacacaa ataagctcgg gccgaggaat atatatatac acggacacag tcgccctgtg 6480
   gacaacacgt cactacctct acgatacaca ccgtacgata gttagtagac aacaatcgat 6540
   agttggagca agggagaaat gtagagtgtg aaagactcac tatggtccgg gcttatctcg 6600
   accaatagcc aaagtctgga gtttctgaga gaaaaaggca agatacgtat gtaacaaagc 6660
   gacgcatggt acaataatac cggaggcatg tatcatagag agttagtggt tcgatgatgg 6720
   cactggtgcc tggtatgact ttatacggct gactacatat ttgtcctcag acatacaatt 6780
   acagtcaagc acttaccctt ggacatctgt aggtaccccc cggccaagac gatctcagcg 6840
   tgtcgtatgt cggattggcg tagctccctc gctcgtcaat tggctcccat ctactttctt 6900
   ctgcttggct acacccagca tgtctgctat ggctcgtttt cgtgccttat ctatcctccc 6960
   agtattacca actctaaatg acatgatgtg attgggtcta cactttcata tcagagataa 7020
   ggagtagcac agttgcataa aaagcccaac tctaatcagc ttcttccttt cttgtaatta 7080
   gtacaaaggt gattagcgaa atctggaagc ttagttggcc ctaaaaaaat caaaaaaagc 7140
   aaaaaacgaa aaacgaaaaa ccacagtttt gagaacaggg aggtaacgaa ggatcgtata 7200
   tatatatata tatatatata cccacggatc ccgagaccgg cctttgattc ttccctacaa 7260
   ccaaccattc tcaccaccct aattcacaac catgtccgtt gcatccaagc tcgtcttcta 7320
   cgtccgcgcc gccatcgccg tggtcatctt tgccgcctgt gccacctacg gcgtgctggc 7380
   gtccaccatt ctcaccgcca tcggcaagca gggcctggcc caatggaccg ttgccagagc 7440
   cttctactac tcggtgcgca tcttcctggg tatcagcatc aagctgcgta gccggcaggt 7500
   gaccggaacc gccggtctgg atgcctccaa gatccaggtc gccaacacca ccaagcccat 7560
   tgacgacatc accaaacacc tgccccgacc atgcattctg atttccaacc accagaacga 7620
   aatggacatt ctggtgctcg gtcgcatctt cccccagtac tgctccgtca ccgccaaaaa 7680
   ggccctcaag tggtaccctc tgctgggcca gttcatggcg ctgtccggca ccatcttcct 7740
   ggaccgaaag gaccgaacca agtccgtgca gaccctcggc ggcgccgtca agaccatcca 7800
   gagcggcaac ggaggcaagg gccagagcgt cttcatgttc cccgagggaa cccgatccta 7860
   ctccaaggac gtcggcatca tgcccttcaa gaagggctgt ttccacctgg cggtccagtc 7920
   gggcgctccc attgtccccg tggtggtcca gaacacctcc cgaatgtttt ctttcggccg 7980
   aggcaagctg gacgccggag agatccttgt cgacgtcctg agccccattg agaccaaggg 8040
   tctggacgcc agcaacgtcg acgctctcat ggccaccact tataaggcca tgtgcgagac 8100
   tgccgaccag attggctacg ctggccagaa gactcagtag gcggccgcat gagaagataa 8160
   atatataaat acattgagat attaaatgcg ctagattaga gagcctcata ctgctcggag 8220
   agaagccaag acgagtactc aaaggggatt acaccatcca tatccacaga cacaagctgg 8280
   ggaaaggttc tatatacact ttccggaata ccgtagtttc cgatgttatc aatgggggca 8340
   gccaggattt caggcacttc ggtgtctcgg ggtgaaatgg cgttcttggc ctccatcaag 8400
   tcgtaccatg tcttcatttg cctgtcaaag taaaacagaa gcagatgaag aatgaacttg 8460
   aagtgaagga atttaaatgt aacgaaactg aaatttgacc agatattgtg tccgcggtgg 8520
   agctccagct tttgttccct ttagtgaggg ttaatttcga gcttggcgta atcatggtca 8580
   tagctgtttc ctgtgtgaaa ttgttatccg ctcacaagct tccacacaac 8630
<210> 150
   <211> 8630
   <212> DNA
   <213> Artificial Sequence
<220>
   <223> Plasmid pY175
<400> 150
   gtacgataac ttcgtatagc atacattata cgaagttatc gcgtcgacga gtatctgtct      60
   gactcgtcat tgccgccttt ggagtacgac tccaactatg agtgtgcttg gatcactttg     120
   acgatacatt cttcgttgga ggctgtgggt ctgacagctg cgttttcggc gcggttggcc     180
   gacaacaata tcagctgcaa cgtcattgct ggctttcatc atgatcacat ttttgtcggc      240
   aaaggcgacg cccagagagc cattgacgtt ctttctaatt tggaccgata gccgtatagt     300
   ccagtctatc tataagttca actaactcgt aactattacc ataacatata cttcactgcc     360
   ccagataagg ttccgataaa aagttctgca gactaaattt atttcagtct cctcttcacc     420
   accaaaatgc cctcctacga agctcgagct aacgtccaca agtccgcctt tgccgctcga     480
   gtgctcaagc tcgtggcagc caagaaaacc aacctgtgtg cttctctgga tgttaccacc     540
   accaaggagc tcattgagct tgccgataag gtcggacctt atgtgtgcat gatcaaaacc     600
   catatcgaca tcattgacga cttcacctac gccggcactg tgctccccct caaggaactt     660
   gctcttaagc acggtttctt cctgttcgag gacagaaagt tcgcagatat tggcaacact     720
   gtcaagcacc agtaccggtg tcaccgaatc gccgagtggt ccgatatcac caacgcccac     780
   ggtgtacccg gaaccggaat cattgctggc ctgcgagctg gtgccgagga aactgtctct     840
   gaacagaaga aggaggacgt ctctgactac gagaactccc agtacaagga gttcctagtc     900
   ccctctccca acgagaagct ggccagaggt ctgctcatgc tggccgagct gtcttgcaag     960
   ggctctctgg ccactggcga gtactccaag cagaccattg agcttgcccg atccgacccc    1020
   gagtttgtgg ttggcttcat tgcccagaac cgacctaagg gcgactctga ggactggctt    1080
   attctgaccc ccggggtggg tcttgacgac aagggagacg ctctcggaca gcagtaccga    1140
   actgttgagg atgtcatgtc taccggaacg gatatcataa ttgtcggccg aggtctgtac    1200
   ggccagaacc gagatcctat tgaggaggcc aagcgatacc agaaggctgg ctgggaggct    1260
   taccagaaga ttaactgtta gaggttagac tatggatatg taatttaact gtgtatatag    1320
   agagcgtgca agtatggagc gcttgttcag cttgtatgat ggtcagacga cctgtctgat    1380
   cgagtatgta tgatactgca caacctgtgt atccgcatga tctgtccaat ggggcatgtt    1440
   gttgtgtttc tcgatacgga gatgctgggt acagtgctaa tacgttgaac tacttatact 1500
   tatatgaggc tcgaagaaag ctgacttgtg tatgacttat tctcaactac atccccagtc 1560
   acaataccac cactgcacta ccactacacc aaaaccatga tcaaaccacc catggacttc 1620
   ctggaggcag aagaacttgt tatggaaaag ctcaagagag agatcataac ttcgtatagc 1680
   atacattata cgaagttatc ctgcaggtaa aggaattcag gagagaccgg gttggcggcg 1740
   tatttgtgtc ccaaaaaaca gccccaattg ccccaattga ccccaaattg acccagtagc      1800
   gggcccaacc ccggcgagag cccccttcac cccacatatc aaacctcccc cggttcccac      1860
   acttgccgtt aagggcgtag ggtactgcag tctggaatct acgcttgttc agactttgta 1920
   ctagtttctt tgtctggcca tccgggtaac ccatgccgga cgcaaaatag actactgaaa 1980
   atttttttgc tttgtggttg ggactttagc caagggtata aaagaccacc gtccccgaat 2040
   tacctttcct cttcttttct ctctctcctt gtcaactcac acccgaaatc gttaagcatt 2100
   tccttctgag tataagaatc attcaccatg gacttcctgg aggcagaaga acttgttatg 2160
   gaaaagctca agagagagaa gccaagatac tatcaagaca tgtgtcgcaa cttaattaag 2220
   atgacgacat ttgcgagctg gacgaggaat agatggagcg tgtgttctga gtcgatgttt 2280
   tctatggagt tgtgagtgtt agtagacatg atgggtttat atatgatgaa tgaatagatg 2340
   tgattttgat ttgcacgatg gaattgagaa ctttgtaaac gtacatggga atgtatgaat 2400
   gtgggggttt tgtgactgga taactgacgg tcagtggacg ccgttgttca aatatccaag 2460
   agatgcgaga aactttgggt caagtgaaca tgtcctctct gttcaagtaa accatcaact 2520
   atgggtagta tatttagtaa ggacaagagt tgagattctt tggagtccta gaaacgtatt 2580
   ttcgcgttcc aagatcaaat tagtagagta atacgggcac gggaatccat tcatagtctc 2640
   aattttccca taggtgtgct acaaggtgtt gagatgtggt acagtaccac catgattcga 2700
   ggtaaagagc ccagaagtca ttgatgaggt caagaaatac acagatctac agctcaatac 2760
   aatgaatatc ttctttcata ttcttcaggt gacaccaagg gtgtctattt tccccagaaa 2820
   tgcgtgaaaa ggcgcgtgtg tagcgtggag tatgggttcg gttggcgtat ccttcatata 2880
   tcgacgaaat agtagggcaa gagatgacaa aaagtatcta tatgtagaca gcgtagaata 2940
   tggatttgat tggtataaat tcatttattg cgtgtctcac aaatactctc gataagttgg 3000
   ggttaaactg gagatggaac aatgtcgata tctcgacgca tgcgacgtcg ggcccaattc 3060
   gccctatagt gagtcgtatt acaattcact ggccgtcgtt ttacaacgtc gtgactggga 3120
   aaaccctggc gttacccaac ttaatcgcct tgcagcacat ccccctttcg ccagctggcg 3180
   taatagcgaa gaggcccgca ccgatcgccc ttcccaacag ttgcgcagcc tgaatggcga 3240
   atggacgcgc cctgtagcgg cgcattaagc gcggcgggtg tggtggttac gcgcagcgtg 3300
   accgctacac ttgccagcgc cctagcgccc gctcctttcg ctttcttccc ttcctttctc 3360
   gccacgttcg ccggctttcc ccgtcaagct ctaaatcggg ggctcccttt agggttccga 3420
   tttagtgctt tacggcacct cgaccccaaa aaacttgatt agggtgatgg ttcacgtagt 3480
   gggccatcgc cctgatagac ggtttttcgc cctttgacgt tggagtccac gttctttaat 3540
   agtggactct tgttccaaac tggaacaaca ctcaacccta tctcggtcta ttcttttgat 3600
   ttataaggga ttttgccgat ttcggcctat tggttaaaaa atgagctgat ttaacaaaaa 3660
   tttaacgcga attttaacaa aatattaacg cttacaattt cctgatgcgg tattttctcc 3720
   ttacgcatct gtgcggtatt tcacaccgca tcaggtggca cttttcgggg aaatgtgcgc 3780
   ggaaccccta tttgtttatt tttctaaata cattcaaata tgtatccgct catgagacaa 3840
   taaccctgat aaatgcttca ataatattga aaaaggaaga gtatgagtat tcaacatttc 3900
   cgtgtcgccc ttattccctt ttttgcggca ttttgccttc ctgtttttgc tcacccagaa 3960
   acgctggtga aagtaaaaga tgctgaagat cagttgggtg cacgagtggg ttacatcgaa 4020
   ctggatctca acagcggtaa gatccttgag agttttcgcc ccgaagaacg ttttccaatg 4080
   atgagcactt ttaaagttct gctatgtggc gcggtattat cccgtattga cgccgggcaa 4140
   gagcaactcg gtcgccgcat acactattct cagaatgact tggttgagta ctcaccagtc 4200
   acagaaaagc atcttacgga tggcatgaca gtaagagaat tatgcagtgc tgccataacc 4260
   atgagtgata acactgcggc caacttactt ctgacaacga tcggaggacc gaaggagcta 4320
   accgcttttt tgcacaacat gggggatcat gtaactcgcc ttgatcgttg ggaaccggag 4380
   ctgaatgaag ccataccaaa cgacgagcgt gacaccacga tgcctgtagc aatggcaaca 4440
   acgttgcgca aactattaac tggcgaacta cttactctag cttcccggca acaattaata 4500
   gactggatgg aggcggataa agttgcagga ccacttctgc gctcggccct tccggctggc 4560
   tggtttattg ctgataaatc tggagccggt gagcgtgggt ctcgcggtat cattgcagca 4620
   ctggggccag atggtaagcc ctcccgtatc gtagttatct acacgacggg gagtcaggca 4680
   actatggatg aacgaaatag acagatcgct gagataggtg cctcactgat taagcattgg 4740
   taactgtcag accaagttta ctcatatata ctttagattg atttaaaact tcatttttaa 4800
   tttaaaagga tctaggtgaa gatccttttt gataatctca tgaccaaaat cccttaacgt 4860
   gagttttcgt tccactgagc gtcagacccc gtagaaaaga tcaaaggatc ttcttgagat 4920
   cctttttttc tgcgcgtaat ctgctgcttg caaacaaaaa aaccaccgct accagcggtg 4980
   gtttgtttgc cggatcaaga gctaccaact ctttttccga aggtaactgg cttcagcaga 5040
   gcgcagatac caaatactgt tcttctagtg tagccgtagt taggccacca cttcaagaac 5100
   tctgtagcac cgcctacata cctcgctctg ctaatcctgt taccagtggc tgctgccagt 5160
   ggcgataagt cgtgtcttac cgggttggac tcaagacgat agttaccgga taaggcgcag 5220
   cggtcgggct gaacgggggg ttcgtgcaca cagcccagct tggagcgaac gacctacacc 5280
   gaactgagat acctacagcg tgagctatga gaaagcgcca cgcttcccga agggagaaag 5340
   gcggacaggt atccggtaag cggcagggtc ggaacaggag agcgcacgag ggagcttcca 5400
   gggggaaacg cctggtatct ttatagtcct gtcgggtttc gccacctctg acttgagcgt 5460
   cgatttttgt gatgctcgtc aggggggcgg agcctatgga aaaacgccag caacgcggcc 5520
   tttttacggt tcctggcctt ttgctggcct tttgctcaca tgttctttcc tgcgttatcc 5580
   cctgattctg tggataaccg tattaccgcc tttgagtgag ctgataccgc tcgccgcagc 5640
   cgaacgaccg agcgcagcga gtcagtgagc gaggaagcgg aagagcgccc aatacgcaaa 5700
   ccgcctctcc ccgcgcgttg gccgattcat taatgcagct ggcgcgccac caatcacaat 5760
   tctgaaaagc acatcttgat ctcctcattg cggggagtcc aacggtggtc ttattccccc 5820
   gaatttcccg ctcaatctcg ttccagaccg acccggacac agtgcttaac gccgttccga 5880
   aactctaccg cagatatgct ccaacggact gggctgcata gatgtgatcc tcggcttgga 5940
   gaaatggata aaagccggcc aaaaaaaaag cggaaaaaag cggaaaaaaa gagaaaaaaa 6000
   atcgcaaaat ttgaaaaata gggggaaaag acgcaaaaac gcaaggaggg gggagtatat 6060
   gacactgata agcaagctca caacggttcc tcttattttt ttcctcatct tctgcctagg 6120
   ttcccaaaat cccagatgct tctctccagt gccaaaagta agtaccccac aggttttcgg 6180
   ccgaaaattc cacgtgcagc aacgtcgtgt ggggtgttaa aatgtggggg gggggaacca 6240
   ggacaagagg ctcttgtggg agccgaatga gagcacaaag cgggcgggtg tgataagggc 6300
   atttttgccc attttccctt ctcctgtctc tccgacggtg atggcgttgt gcgtcctcta 6360
   tttcttttta tttctttttg ttttatttct ctgactaccg atttggtttg atttcctcaa 6420
   ccccacacaa ataagctcgg gccgaggaat atatatatac acggacacag tcgccctgtg 6480
   gacaacacgt cactacctct acgatacaca ccgtacgata gttagtagac aacaatcgat 6540
   agttggagca agggagaaat gtagagtgtg aaagactcac tatggtccgg gcttatctcg 6600
   accaatagcc aaagtctgga gtttctgaga gaaaaaggca agatacgtat gtaacaaagc 6660
   gacgcatggt acaataatac cggaggcatg tatcatagag agttagtggt tcgatgatgg 6720
   cactggtgcc tggtatgact ttatacggct gactacatat ttgtcctcag acatacaatt 6780
   acagtcaagc acttaccctt ggacatctgt aggtaccccc cggccaagac gatctcagcg 6840
   tgtcgtatgt cggattggcg tagctccctc gctcgtcaat tggctcccat ctactttctt 6900
   ctgcttggct acacccagca tgtctgctat ggctcgtttt cgtgccttat ctatcctccc 6960
   agtattacca actctaaatg acatgatgtg attgggtcta cactttcata tcagagataa 7020
   ggagtagcac agttgcataa aaagcccaac tctaatcagc ttcttccttt cttgtaatta 7080
   gtacaaaggt gattagcgaa atctggaagc ttagttggcc ctaaaaaaat caaaaaaagc 7140
   aaaaaacgaa aaacgaaaaa ccacagtttt gagaacaggg aggtaacgaa ggatcgtata 7200
   tatatatata tatatatata cccacggatc ccgagaccgg cctttgattc ttccctacaa 7260
   ccaaccattc tcaccaccct aattcacaac catggagaac ttctggtcca tcgtcgtgtt     7320
   ctttctgctc tccattctgt tcatcctcta caacatttcg acagtctgcc actactacat     7380
   gcgaatctcc ttctactact ttaccatcct gcttcacggc atggaggtgt gcgttaccat     7440
   gattccctct tggctcaacg gcaagggtgc cgactacgtg tttcactcgt tcttctactg     7500
   gtgcaagtgg actggagtcc acaccactgt gtatggctac gagaagaccc aggtcgaagg     7560
   tcctgccgtg gtcatctgca accatcagtc ctcgctcgac attctgtcta tggcttccat     7620
   ctggcccaag aactgtgttg tcatgatgaa gcggattctt gcctacgttc ccttcttcaa     7680
   cctgggagcc tacttttcca acaccatctt catcgaccga tacaaccgag agcgagctat     7740
   ggcttctgtc gactactgtg cctccgagat gaagaaccga aacctgaagc tctgggtgtt     7800
   tcccgaaggc actcggaatc gagagggtgg attcattccc ttcaagaaag gtgccttcaa     7860
   catcgctgtt cgagcccaga ttcccatcat tcctgtcgtg ttctctgact atcgagactt     7920
   ctactccaag cctggccgat acttcaagaa cgatggagag gtcgtgatcc gagtcctgga     7980
   tgccattccc accaagggtc tgaccctcga tgacgtctct gagctttcgg acatgtgtcg     8040
   agacgtcatg ctggctgcct acaaggaagt taccctcgag gctcagcaac gaaacgccac     8100
   tcgaagagga gagaccaagg acggcaagaa atccgagtaa gcggccgcat gagaagataa     8160
   atatataaat acattgagat attaaatgcg ctagattaga gagcctcata ctgctcggag     8220
   agaagccaag acgagtactc aaaggggatt acaccatcca tatccacaga cacaagctgg     8280
   ggaaaggttc tatatacact ttccggaata ccgtagtttc cgatgttatc aatgggggca     8340
   gccaggattt caggcacttc ggtgtctcgg ggtgaaatgg cgttcttggc ctccatcaag     8400
   tcgtaccatg tcttcatttg cctgtcaaag taaaacagaa gcagatgaag aatgaacttg     8460
   aagtgaagga atttaaatgt aacgaaactg aaatttgacc agatattgtg tccgcggtgg     8520
   agctccagct tttgttccct ttagtgaggg ttaatttcga gcttggcgta atcatggtca     8580
   tagctgtttc ctgtgtgaaa ttgttatccg ctcacaagct tccacacaac                8630
<210> 151
   <211> 926
   <212> DNA
   <213> Saccharomyces cerevisiae
<220>
   <221> CDS
   <222> (4)..(915)
<400> 151
   aac atg tct gtt att gga cga ttt ctt tac tac ctg aga tcg gtg ctc          48
       Met Ser Val Ile Gly Arg Phe Leu Tyr Tyr Leu Arg Ser Val Leu
       1               5                   10                  15
   gtc gtt ttg gcc ctc gct gga tgt ggc ttc tat ggc gtg att gcc tct          96
   Val Val Leu Ala Leu Ala Gly Cys Gly Phe Tyr Gly Val Ile Ala Ser
                   20                  25                  30
   atc ctg tgt act ctc atc ggc aag cag cat ctc gcg caa tgg att acc      144
   Ile Leu Cys Thr Leu Ile Gly Lys Gln His Leu Ala Gln Trp Ile Thr
               35                  40                  45
   gcc cga tgc ttt tac cac gtg atg aaa ctg atg ctg gga ttg gac gtc      192
   Ala Arg Cys Phe Tyr His Val Met Lys Leu Met Leu Gly Leu Asp Val
           50                  55                  60
   aaa gtc gtg ggt gaa gag aac ctg gct aag aag ccc tat atc atg atc      240
   Lys Val Val Gly Glu Glu Asn Leu Ala Lys Lys Pro Tyr Ile Met Ile
       65                  70                  75
   gct aac cac cag tcc acc ctc gat atc ttt atg ctc ggc aga atc ttc      288
   Ala Asn His Gln Ser Thr Leu Asp Ile Phe Met Leu Gly Arg Ile Phe
   80                  85                  90                  95
   cct ccc gga tgc acc gtt acc gca aag aag agc ctt aag tac gtc ccc      336
   Pro Pro Gly Cys Thr Val Thr Ala Lys Lys Ser Leu Lys Tyr Val Pro
                   100                 105                 110
   ttc ctg ggc tgg ttt atg gcg ctt tcc ggt aca tac ttc ctc gac cgt      384
   Phe Leu Gly Trp Phe Met Ala Leu Ser Gly Thr Tyr Phe Leu Asp Arg
               115                 120                 125
   tcc aag cgg caa gag gct att gat acc ctg aac aaa gga ctg gag aac      432
   Ser Lys Arg Gln Glu Ala Ile Asp Thr Leu Asn Lys Gly Leu Glu Asn
           130                 135                 140
   gtc aaa aag aat aag cga gcc ctc tgg gtt ttt ccc gaa ggt acc cgg      480
   Val Lys Lys Asn Lys Arg Ala Leu Trp Val Phe Pro Glu Gly Thr Arg
       145                 150                 155
   tct tac acg tcg gag ctt acc atg ctg ccg ttc aag aag ggc gcc ttt      528
   Ser Tyr Thr Ser Glu Leu Thr Met Leu Pro Phe Lys Lys Gly Ala Phe
   160                 165                 170                 175
   cat ttg gcc cag cag gga aag atc cct atc gtc cca gtg gtt gtg tct      576
   His Leu Ala Gln Gln Gly Lys Ile Pro Ile Val Pro Val Val Val Ser
                   180                 185                 190
   aac act agc acg ctc gtc agc cct aag tac ggt gtg ttc aac cga ggc      624
   Asn Thr Ser Thr Leu Val Ser Pro Lys Tyr Gly Val Phe Asn Arg Gly
               195                 200                 205
   tgt atg att gtc cga att ctg aag ccc atc tcg act gag aac ctg aca      672
   Cys Met Ile Val Arg Ile Leu Lys Pro Ile Ser Thr Glu Asn Leu Thr
           210                 215                 220
   aaa gat aag att ggc gag ttc gct gag aaa gtg cga gat cag atg gtt      720
   Lys Asp Lys Ile Gly Glu Phe Ala Glu Lys Val Arg Asp Gln Met Val
       225                 230                 235
   gac aca ctg aag gaa atc ggt tat tcc ccc gca atc aac gac acc acc      768
   Asp Thr Leu Lys Glu Ile Gly Tyr Ser Pro Ala Ile Asn Asp Thr Thr
   240                 245                 250                 255
   ctt cct ccg cag gca att gag tac gcc gca ttg cag cat gac aag aag      816
   Leu Pro Pro Gln Ala Ile Glu Tyr Ala Ala Leu Gln His Asp Lys Lys
                   260                 265                 270
   gtg aac aaa aag att aag aac gaa cct gtt cca tcg gtg tcc att tct      864
   Val Asn Lys Lys Ile Lys Asn Glu Pro Val Pro Ser Val Ser Ile Ser
               275                 280                 285
   aat gat gtg aat act cac aac gaa gga tcg tct gtc aag aag atg cac        912
   Asn Asp Val Asn Thr His Asn Glu Gly Ser Ser Val Lys Lys Met His
           290                 295                 300
   tag gcggccgcat g                                                       926
<210> 152
   <211> 303
   <212> PRT
   <213> Saccharomyces cerevisiae
<400> 152
   Met Ser Val Ile Gly Arg Phe Leu Tyr Tyr Leu Arg Ser Val Leu Val
   1               5                   10                  15
   Val Leu Ala Leu Ala Gly Cys Gly Phe Tyr Gly Val Ile Ala Ser Ile
               20                  25                  30
   Leu Cys Thr Leu Ile Gly Lys Gln His Leu Ala Gln Trp Ile Thr Ala
           35                  40                  45
   Arg Cys Phe Tyr His Val Met Lys Leu Met Leu Gly Leu Asp Val Lys
       50                  55                  60
   Val Val Gly Glu Glu Asn Leu Ala Lys Lys Pro Tyr Ile Met Ile Ala
   65                  70                  75                  80
   Asn His Gln Ser Thr Leu Asp Ile Phe Met Leu Gly Arg Ile Phe Pro
                   85                  90                  95
   Pro Gly Cys Thr Val Thr Ala Lys Lys Ser Leu Lys Tyr Val Pro Phe
               100                 105                 110
   Leu Gly Trp Phe Met Ala Leu Ser Gly Thr Tyr Phe Leu Asp Arg Ser
           115                 120                 125
   Lys Arg Gln Glu Ala Ile Asp Thr Leu Asn Lys Gly Leu Glu Asn Val
       130                 135                 140
   Lys Lys Asn Lys Arg Ala Leu Trp Val Phe Pro Glu Gly Thr Arg Ser
   145                 150                 155                 160
   Tyr Thr Ser Glu Leu Thr Met Leu Pro Phe Lys Lys Gly Ala Phe His
                   165                 170                 175
   Leu Ala Gln Gln Gly Lys Ile Pro Ile Val Pro Val Val Val Ser Asn
               180                 185                 190
   Thr Ser Thr Leu Val Ser Pro Lys Tyr Gly Val Phe Asn Arg Gly Cys
           195                 200                 205
   Met Ile Val Arg Ile Leu Lys Pro Ile Ser Thr Glu Asn Leu Thr Lys
       210                 215                 220
   Asp Lys Ile Gly Glu Phe Ala Glu Lys Val Arg Asp Gln Met Val Asp
   225                 230                 235                 240
   Thr Leu Lys Glu Ile Gly Tyr Ser Pro Ala Ile Asn Asp Thr Thr Leu
                   245                 250                 255
   Pro Pro Gln Ala Ile Glu Tyr Ala Ala Leu Gln His Asp Lys Lys Val
               260                 265                 270
   Asn Lys Lys Ile Lys Asn Glu Pro Val Pro Ser Val Ser Ile Ser Asn
           275                 280                 285
   Asp Val Asn Thr His Asn Glu Gly Ser Ser Val Lys Lys Met His
       290                 295                 300
<210> 153
   <211> 7891
   <212> DNA
   <213> Artificial Sequence
<220>
   <223> Plasmid pY222
<400> 153
   tcgaccgtac gatagttagt agacaacaat cgatagttgg agcaagggag aaatgtagag      60
   tgtgaaagac tcactatggt ccgggcttat ctcgaccaat agccaaagtc tggagtttct     120
   gagagaaaaa ggcaagatac gtatgtaaca aagcgacgca tggtacaata ataccggagg     180
   catgtatcat agagagttag tggttcgatg atggcactgg tgcctggtat gactttatac     240
   ggctgactac atatttgtcc tcagacatac aattacagtc aagcacttac ccttggacat     300
   ctgtaggtac cccccggcca agacgatctc agcgtgtcgt atgtcggatt ggcgtagctc     360
   cctcgctcgt caattggctc ccatctactt tcttctgctt ggctacaccc agcatgtctg     420
   ctatggctcg ttttcgtgcc ttatctatcc tcccagtatt accaactcta aatgacatga     480
   tgtgattggg tctacacttt catatcagag ataaggagta gcacagttgc ataaaaagcc     540
   caactctaat cagcttcttc ctttcttgta attagtacaa aggtgattag cgaaatctgg     600
   aagcttagtt ggccctaaaa aaatcaaaaa aagcaaaaaa cgaaaaacga aaaaccacag     660
   ttttgagaac agggaggtaa cgaaggatcg tatatatata tatatatata tatacccacg     720
   gatcccgaga ccggcctttg attcttccct acaaccaacc attctcacca ccctaattca     780
   caaccatgtc tgttattgga cgatttcttt actacctgag atcggtgctc gtcgttttgg     840
   ccctcgctgg atgtggcttc tatggcgtga ttgcctctat cctgtgtact ctcatcggca     900
   agcagcatct cgcgcaatgg attaccgccc gatgctttta ccacgtgatg aaactgatgc     960
   tgggattgga cgtcaaagtc gtgggtgaag agaacctggc taagaagccc tatatcatga 1020
   tcgctaacca ccagtccacc ctcgatatct ttatgctcgg cagaatcttc cctcccggat 1080
   gcaccgttac cgcaaagaag agccttaagt acgtcccctt cctgggctgg tttatggcgc 1140
   tttccggtac atacttcctc gaccgttcca agcggcaaga ggctattgat accctgaaca 1200
   aaggactgga gaacgtcaaa aagaataagc gagccctctg ggtttttccc gaaggtaccc 1260
   ggtcttacac gtcggagctt accatgctgc cgttcaagaa gggcgccttt catttggccc 1320
   agcagggaaa gatccctatc gtcccagtgg ttgtgtctaa cactagcacg ctcgtcagcc 1380
   ctaagtacgg tgtgttcaac cgaggctgta tgattgtccg aattctgaag cccatctcga 1440
   ctgagaacct gacaaaagat aagattggcg agttcgctga gaaagtgcga gatcagatgg 1500
   ttgacacact gaaggaaatc ggttattccc ccgcaatcaa cgacaccacc cttcctccgc 1560
   aggcaattga gtacgccgca ttgcagcatg acaagaaggt gaacaaaaag attaagaacg 1620
   aacctgttcc atcggtgtcc atttctaatg atgtgaatac tcacaacgaa ggatcgtctg 1680
   tcaagaagat gcactaggcg gccgcatgag aagataaata tataaataca ttgagatatt 1740
   aaatgcgcta gattagagag cctcatactg ctcggagaga agccaagacg agtactcaaa 1800
   ggggattaca ccatccatat ccacagacac aagctgggga aaggttctat atacactttc 1860
   cggaataccg tagtttccga tgttatcaat gggggcagcc aggatttcag gcacttcggt 1920
   gtctcggggt gaaatggcgt tcttggcctc catcaagtcg taccatgtct tcatttgcct 1980
   gtcaaagtaa aacagaagca gatgaagaat gaacttgaag tgaaggaatt taaatgtaac 2040
   gaaactgaaa tttgaccaga tattgtgtcc gcggtggagc tccagctttt gttcccttta 2100
   gtgagggtta atttcgagct tggcgtaatc atggtcatag ctgtttcctg tgtgaaattg 2160
   ttatccgctc acaagcttcc acacaacgta cgagccggaa gcataaagtg taaagcctgg 2220
   ggtgcctaat gagtgagcta actcacatta attgcgttgc gctcactgcc cgctttccag 2280
   tcgggaaacc tgtcgtgcca gctgcattaa tgaatcggcc aacgcgcggg gagaggcggt 2340
   ttgcgtattg ggcgctcttc cgcttcctcg ctcactgact cgctgcgctc ggtcgttcgg 2400
   ctgcggcgag cggtatcagc tcactcaaag gcggtaatac ggttatccac agaatcaggg 2460
   gataacgcag gaaagaacat gtgagcaaaa ggccagcaaa aggccaggaa ccgtaaaaag 2520
   gccgcgttgc tggcgttttt ccataggctc cgcccccctg acgagcatca caaaaatcga 2580
   cgctcaagtc agaggtggcg aaacccgaca ggactataaa gataccaggc gtttccccct 2640
   ggaagctccc tcgtgcgctc tcctgttccg accctgccgc ttaccggata cctgtccgcc 2700
   tttctccctt cgggaagcgt ggcgctttct catagctcac gctgtaggta tctcagttcg 2760
   gtgtaggtcg ttcgctccaa gctgggctgt gtgcacgaac cccccgttca gcccgaccgc 2820
   tgcgccttat ccggtaacta tcgtcttgag tccaacccgg taagacacga cttatcgcca 2880
   ctggcagcag ccactggtaa caggattagc agagcgaggt atgtaggcgg tgctacagag 2940
   ttcttgaagt ggtggcctaa ctacggctac actagaagga cagtatttgg tatctgcgct 3000
   ctgctgaagc cagttacctt cggaaaaaga gttggtagct cttgatccgg caaacaaacc 3060
   accgctggta gcggtggttt ttttgtttgc aagcagcaga ttacgcgcag aaaaaaagga 3120
   tctcaagaag atcctttgat cttttctacg gggtctgacg ctcagtggaa cgaaaactca 3180
   cgttaaggga ttttggtcat gagattatca aaaaggatct tcacctagat ccttttaaat 3240
   taaaaatgaa gttttaaatc aatctaaagt atatatgagt aaacttggtc tgacagttac 3300
   caatgcttaa tcagtgaggc acctatctca gcgatctgtc tatttcgttc atccatagtt 3360
   gcctgactcc ccgtcgtgta gataactacg atacgggagg gcttaccatc tggccccagt 3420
   gctgcaatga taccgcgaga cccacgctca ccggctccag atttatcagc aataaaccag 3480
   ccagccggaa gggccgagcg cagaagtggt cctgcaactt tatccgcctc catccagtct 3540
   attaattgtt gccgggaagc tagagtaagt agttcgccag ttaatagttt gcgcaacgtt 3600
   gttgccattg ctacaggcat cgtggtgtca cgctcgtcgt ttggtatggc ttcattcagc 3660
   tccggttccc aacgatcaag gcgagttaca tgatccccca tgttgtgcaa aaaagcggtt 3720
   agctccttcg gtcctccgat cgttgtcaga agtaagttgg ccgcagtgtt atcactcatg 3780
   gttatggcag cactgcataa ttctcttact gtcatgccat ccgtaagatg cttttctgtg 3840
   actggtgagt actcaaccaa gtcattctga gaatagtgta tgcggcgacc gagttgctct 3900
   tgcccggcgt caatacggga taataccgcg ccacatagca gaactttaaa agtgctcatc 3960
   attggaaaac gttcttcggg gcgaaaactc tcaaggatct taccgctgtt gagatccagt 4020
   tcgatgtaac ccactcgtgc acccaactga tcttcagcat cttttacttt caccagcgtt 4080
   tctgggtgag caaaaacagg aaggcaaaat gccgcaaaaa agggaataag ggcgacacgg 4140
   aaatgttgaa tactcatact cttccttttt caatattatt gaagcattta tcagggttat 4200
   tgtctcatga gcggatacat atttgaatgt atttagaaaa ataaacaaat aggggttccg 4260
   cgcacatttc cccgaaaagt gccacctgac gcgccctgta gcggcgcatt aagcgcggcg 4320
   ggtgtggtgg ttacgcgcag cgtgaccgct acacttgcca gcgccctagc gcccgctcct 4380
   ttcgctttct tcccttcctt tctcgccacg ttcgccggct ttccccgtca agctctaaat 4440
   cgggggctcc ctttagggtt ccgatttagt gctttacggc acctcgaccc caaaaaactt 4500
   gattagggtg atggttcacg tagtgggcca tcgccctgat agacggtttt tcgccctttg 4560
   acgttggagt ccacgttctt taatagtgga ctcttgttcc aaactggaac aacactcaac 4620
   cctatctcgg tctattcttt tgatttataa gggattttgc cgatttcggc ctattggtta 4680
   aaaaatgagc tgatttaaca aaaatttaac gcgaatttta acaaaatatt aacgcttaca 4740
   atttccattc gccattcagg ctgcgcaact gttgggaagg gcgatcggtg cgggcctctt 4800
   cgctattacg ccagctggcg aaagggggat gtgctgcaag gcgattaagt tgggtaacgc 4860
   cagggttttc ccagtcacga cgttgtaaaa cgacggccag tgaattgtaa tacgactcac     4920
   tatagggcga attgggtacc gggccccccc tcgaggtcga tggtgtcgat aagcttgata     4980
   tcgaattcat gtcacacaaa ccgatcttcg cctcaaggaa acctaattct acatccgaga     5040
   gactgccgag atccagtcta cactgattaa ttttcgggcc aataatttaa aaaaatcgtg     5100
   ttatataata ttatatgtat tatatatata catcatgatg atactgacag tcatgtccca     5160
   ttgctaaata gacagactcc atctgccgcc tccaactgat gttctcaata tttaaggggt     5220
   catctcgcat tgtttaataa taaacagact ccatctaccg cctccaaatg atgttctcaa     5280
   aatatattgt atgaacttat ttttattact tagtattatt agacaactta cttgctttat     5340
   gaaaaacact tcctatttag gaaacaattt ataatggcag ttcgttcatt taacaattta     5400
   tgtagaataa atgttataaa tgcgtatggg aaatcttaaa tatggatagc ataaatgata     5460
   tctgcattgc ctaattcgaa atcaacagca acgaaaaaaa tcccttgtac aacataaata     5520
   gtcatcgaga aatatcaact atcaaagaac agctattcac acgttactat tgagattatt     5580
   attggacgag aatcacacac tcaactgtct ttctctcttc tagaaataca ggtacaagta     5640
   tgtactattc tcattgttca tacttctagt catttcatcc cacatattcc ttggatttct     5700
   ctccaatgaa tgacattcta tcttgcaaat tcaacaatta taataagata taccaaagta     5760
   gcggtatagt ggcaatcaaa aagcttctct ggtgtgcttc tcgtatttat ttttattcta     5820
   atgatccatt aaaggtatat atttatttct tgttatataa tccttttgtt tattacatgg     5880
   gctggataca taaaggtatt ttgatttaat tttttgctta aattcaatcc cccctcgttc     5940
   agtgtcaact gtaatggtag gaaattacca tacttttgaa gaagcaaaaa aaatgaaaga     6000
   aaaaaaaaat cgtatttcca ggttagacgt tccgcagaat ctagaatgcg gtatgcggta     6060
   cattgttctt cgaacgtaaa agttgcgctc cctgagatat tgtacatttt tgcttttaca     6120
   agtacaagta catcgtacaa ctatgtacta ctgttgatgc atccacaaca gtttgttttg     6180
   tttttttttg tttttttttt ttctaatgat tcattaccgc tatgtatacc tacttgtact     6240
   tgtagtaagc cgggttattg gcgttcaatt aatcatagac ttatgaatct gcacggtgtg     6300
   cgctgcgagt tacttttagc ttatgcatgc tacttgggtg taatattggg atctgttcgg     6360
   aaatcaacgg atgctcaatc gatttcgaca gtaattaatt aagtcataca caagtcagct     6420
   ttcttcgagc ctcatataag tataagtagt tcaacgtatt agcactgtac ccagcatctc     6480
   cgtatcgaga aacacaacaa catgccccat tggacagatc atgcggatac acaggttgtg     6540
   cagtatcata catactcgat cagacaggtc gtctgaccat catacaagct gaacaagcgc     6600
   tccatacttg cacgctctct atatacacag ttaaattaca tatccatagt ctaacctcta     6660
   acagttaatc ttctggtaag cctcccagcc agccttctgg tatcgcttgg cctcctcaat     6720
   aggatctcgg ttctggccgt acagacctcg gccgacaatt atgatatccg ttccggtaga     6780
   catgacatcc tcaacagttc ggtactgctg tccgagagcg tctcccttgt cgtcaagacc 6840
   caccccgggg gtcagaataa gccagtcctc agagtcgccc ttaggtcggt tctgggcaat 6900
   gaagccaacc acaaactcgg ggtcggatcg ggcaagctca atggtctgct tggagtactc 6960
   gccagtggcc agagagccct tgcaagacag ctcggccagc atgagcagac ctctggccag 7020
   cttctcgttg ggagagggga ctaggaactc cttgtactgg gagttctcgt agtcagagac 7080
   gtcctccttc ttctgttcag agacagtttc ctcggcacca gctcgcaggc cagcaatgat 7140
   tccggttccg ggtacaccgt gggcgttggt gatatcggac cactcggcga ttcggtgaca 7200
   ccggtactgg tgcttgacag tgttgccaat atctgcgaac tttctgtcct cgaacaggaa 7260
   gaaaccgtgc ttaagagcaa gttccttgag ggggagcaca gtgccggcgt aggtgaagtc 7320
   gtcaatgatg tcgatatggg ttttgatcat gcacacataa ggtccgacct tatcggcaag 7380
   ctcaatgagc tccttggtgg tggtaacatc cagagaagca cacaggttgg ttttcttggc 7440
   tgccacgagc ttgagcactc gagcggcaaa ggcggacttg tggacgttag ctcgagcttc 7500
   gtaggagggc attttggtgg tgaagaggag actgaaataa atttagtctg cagaactttt 7560
   tatcggaacc ttatctgggg cagtgaagta tatgttatgg taatagttac gagttagttg 7620
   aacttataga tagactggac tatacggcta tcggtccaaa ttagaaagaa cgtcaatggc 7680
   tctctgggcg tcgcctttgc cgacaaaaat gtgatcatga tgaaagccag caatgacgtt 7740
   gcagctgata ttgttgtcgg ccaaccgcgc cgaaaacgca gctgtcagac ccacagcctc 7800
   caacgaagaa tgtatcgtca aagtgatcca agcacactca tagttggagt cgtactccaa 7860
   aggcggcaat gacgagtcag acagatactc g 7891
<210> 154
   <211> 32
   <212> DNA
   <213> Artificial Sequence
<220>
   <223> Primer 869
<400> 154
   acttggcgcg ccactgccga gatccagtct ac 32
<210> 155
   <211> 28
   <212> DNA
   <213> Artificial Sequence
<220>
   <223> Primer 870
<400> 155
   tcagcctagg agcatccgtt gatttccg 28
<210> 156
   <211> 9598
   <212> DNA
   <213> Artificial Sequence
<220>
   <223> Plasmid pY177
<400> 156
   gtacgataac ttcgtatagc atacattata cgaagttatc gcgtcgacga gtatctgtct 60
   gactcgtcat tgccgccttt ggagtacgac tccaactatg agtgtgcttg gatcactttg 120
   acgatacatt cttcgttgga ggctgtgggt ctgacagctg cgttttcggc gcggttggcc 180
   gacaacaata tcagctgcaa cgtcattgct ggctttcatc atgatcacat ttttgtcggc 240
   aaaggcgacg cccagagagc cattgacgtt ctttctaatt tggaccgata gccgtatagt 300
   ccagtctatc tataagttca actaactcgt aactattacc ataacatata cttcactgcc 360
   ccagataagg ttccgataaa aagttctgca gactaaattt atttcagtct cctcttcacc 420
   accaaaatgc cctcctacga agctcgagct aacgtccaca agtccgcctt tgccgctcga 480
   gtgctcaagc tcgtggcagc caagaaaacc aacctgtgtg cttctctgga tgttaccacc 540
   accaaggagc tcattgagct tgccgataag gtcggacctt atgtgtgcat gatcaaaacc 600
   catatcgaca tcattgacga cttcacctac gccggcactg tgctccccct caaggaactt 660
   gctcttaagc acggtttctt cctgttcgag gacagaaagt tcgcagatat tggcaacact 720
   gtcaagcacc agtaccggtg tcaccgaatc gccgagtggt ccgatatcac caacgcccac 780
   ggtgtacccg gaaccggaat cattgctggc ctgcgagctg gtgccgagga aactgtctct 840
   gaacagaaga aggaggacgt ctctgactac gagaactccc agtacaagga gttcctagtc 900
   ccctctccca acgagaagct ggccagaggt ctgctcatgc tggccgagct gtcttgcaag 960
   ggctctctgg ccactggcga gtactccaag cagaccattg agcttgcccg atccgacccc 1020
   gagtttgtgg ttggcttcat tgcccagaac cgacctaagg gcgactctga ggactggctt 1080
   attctgaccc ccggggtggg tcttgacgac aagggagacg ctctcggaca gcagtaccga 1140
   actgttgagg atgtcatgtc taccggaacg gatatcataa ttgtcggccg aggtctgtac 1200
   ggccagaacc gagatcctat tgaggaggcc aagcgatacc agaaggctgg ctgggaggct 1260
   taccagaaga ttaactgtta gaggttagac tatggatatg taatttaact gtgtatatag 1320
   agagcgtgca agtatggagc gcttgttcag cttgtatgat ggtcagacga cctgtctgat 1380
   cgagtatgta tgatactgca caacctgtgt atccgcatga tctgtccaat ggggcatgtt 1440
   gttgtgtttc tcgatacgga gatgctgggt acagtgctaa tacgttgaac tacttatact 1500
   tatatgaggc tcgaagaaag ctgacttgtg tatgacttat tctcaactac atccccagtc 1560
   acaataccac cactgcacta ccactacacc aaaaccatga tcaaaccacc catggacttc 1620
   ctggaggcag aagaacttgt tatggaaaag ctcaagagag agatcataac ttcgtatagc 1680
   atacattata cgaagttatc ctgcaggtaa aggaattcag gagagaccgg gttggcggcg 1740
   tatttgtgtc ccaaaaaaca gccccaattg ccccaattga ccccaaattg acccagtagc 1800
   gggcccaacc ccggcgagag cccccttcac cccacatatc aaacctcccc cggttcccac 1860
   acttgccgtt aagggcgtag ggtactgcag tctggaatct acgcttgttc agactttgta 1920
   ctagtttctt tgtctggcca tccgggtaac ccatgccgga cgcaaaatag actactgaaa 1980
   atttttttgc tttgtggttg ggactttagc caagggtata aaagaccacc gtccccgaat 2040
   tacctttcct cttcttttct ctctctcctt gtcaactcac acccgaaatc gttaagcatt 2100
   tccttctgag tataagaatc attcaccatg gacttcctgg aggcagaaga acttgttatg 2160
   gaaaagctca agagagagaa gccaagatac tatcaagaca tgtgtcgcaa cttaattaag 2220
   atgacgacat ttgcgagctg gacgaggaat agatggagcg tgtgttctga gtcgatgttt 2280
   tctatggagt tgtgagtgtt agtagacatg atgggtttat atatgatgaa tgaatagatg 2340
   tgattttgat ttgcacgatg gaattgagaa ctttgtaaac gtacatggga atgtatgaat 2400
   gtgggggttt tgtgactgga taactgacgg tcagtggacg ccgttgttca aatatccaag 2460
   agatgcgaga aactttgggt caagtgaaca tgtcctctct gttcaagtaa accatcaact 2520
   atgggtagta tatttagtaa ggacaagagt tgagattctt tggagtccta gaaacgtatt 2580
   ttcgcgttcc aagatcaaat tagtagagta atacgggcac gggaatccat tcatagtctc 2640
   aattttccca taggtgtgct acaaggtgtt gagatgtggt acagtaccac catgattcga 2700
   ggtaaagagc ccagaagtca ttgatgaggt caagaaatac acagatctac agctcaatac 2760
   aatgaatatc ttctttcata ttcttcaggt gacaccaagg gtgtctattt tccccagaaa 2820
   tgcgtgaaaa ggcgcgtgtg tagcgtggag tatgggttcg gttggcgtat ccttcatata 2880
   tcgacgaaat agtagggcaa gagatgacaa aaagtatcta tatgtagaca gcgtagaata 2940
   tggatttgat tggtataaat tcatttattg cgtgtctcac aaatactctc gataagttgg 3000
   ggttaaactg gagatggaac aatgtcgata tctcgacgca tgcgacgtcg ggcccaattc 3060
   gccctatagt gagtcgtatt acaattcact ggccgtcgtt ttacaacgtc gtgactggga 3120
   aaaccctggc gttacccaac ttaatcgcct tgcagcacat ccccctttcg ccagctggcg 3180
   taatagcgaa gaggcccgca ccgatcgccc ttcccaacag ttgcgcagcc tgaatggcga 3240
   atggacgcgc cctgtagcgg cgcattaagc gcggcgggtg tggtggttac gcgcagcgtg 3300
   accgctacac ttgccagcgc cctagcgccc gctcctttcg ctttcttccc ttcctttctc 3360
   gccacgttcg ccggctttcc ccgtcaagct ctaaatcggg ggctcccttt agggttccga 3420
   tttagtgctt tacggcacct cgaccccaaa aaacttgatt agggtgatgg ttcacgtagt 3480
   gggccatcgc cctgatagac ggtttttcgc cctttgacgt tggagtccac gttctttaat 3540
   agtggactct tgttccaaac tggaacaaca ctcaacccta tctcggtcta ttcttttgat 3600
   ttataaggga ttttgccgat ttcggcctat tggttaaaaa atgagctgat ttaacaaaaa 3660
   tttaacgcga attttaacaa aatattaacg cttacaattt cctgatgcgg tattttctcc 3720
   ttacgcatct gtgcggtatt tcacaccgca tcaggtggca cttttcgggg aaatgtgcgc 3780
   ggaaccccta tttgtttatt tttctaaata cattcaaata tgtatccgct catgagacaa 3840
   taaccctgat aaatgcttca ataatattga aaaaggaaga gtatgagtat tcaacatttc 3900
   cgtgtcgccc ttattccctt ttttgcggca ttttgccttc ctgtttttgc tcacccagaa 3960
   acgctggtga aagtaaaaga tgctgaagat cagttgggtg cacgagtggg ttacatcgaa 4020
   ctggatctca acagcggtaa gatccttgag agttttcgcc ccgaagaacg ttttccaatg 4080
   atgagcactt ttaaagttct gctatgtggc gcggtattat cccgtattga cgccgggcaa 4140
   gagcaactcg gtcgccgcat acactattct cagaatgact tggttgagta ctcaccagtc 4200
   acagaaaagc atcttacgga tggcatgaca gtaagagaat tatgcagtgc tgccataacc 4260
   atgagtgata acactgcggc caacttactt ctgacaacga tcggaggacc gaaggagcta 4320
   accgcttttt tgcacaacat gggggatcat gtaactcgcc ttgatcgttg ggaaccggag 4380
   ctgaatgaag ccataccaaa cgacgagcgt gacaccacga tgcctgtagc aatggcaaca 4440
   acgttgcgca aactattaac tggcgaacta cttactctag cttcccggca acaattaata 4500
   gactggatgg aggcggataa agttgcagga ccacttctgc gctcggccct tccggctggc 4560
   tggtttattg ctgataaatc tggagccggt gagcgtgggt ctcgcggtat cattgcagca 4620
   ctggggccag atggtaagcc ctcccgtatc gtagttatct acacgacggg gagtcaggca 4680
   actatggatg aacgaaatag acagatcgct gagataggtg cctcactgat taagcattgg 4740
   taactgtcag accaagttta ctcatatata ctttagattg atttaaaact tcatttttaa 4800
   tttaaaagga tctaggtgaa gatccttttt gataatctca tgaccaaaat cccttaacgt 4860
   gagttttcgt tccactgagc gtcagacccc gtagaaaaga tcaaaggatc ttcttgagat 4920
   cctttttttc tgcgcgtaat ctgctgcttg caaacaaaaa aaccaccgct accagcggtg 4980
   gtttgtttgc cggatcaaga gctaccaact ctttttccga aggtaactgg cttcagcaga 5040
   gcgcagatac caaatactgt tcttctagtg tagccgtagt taggccacca cttcaagaac 5100
   tctgtagcac cgcctacata cctcgctctg ctaatcctgt taccagtggc tgctgccagt 5160
   ggcgataagt cgtgtcttac cgggttggac tcaagacgat agttaccgga taaggcgcag 5220
   cggtcgggct gaacgggggg ttcgtgcaca cagcccagct tggagcgaac gacctacacc 5280
   gaactgagat acctacagcg tgagctatga gaaagcgcca cgcttcccga agggagaaag 5340
   gcggacaggt atccggtaag cggcagggtc ggaacaggag agcgcacgag ggagcttcca 5400
   gggggaaacg cctggtatct ttatagtcct gtcgggtttc gccacctctg acttgagcgt 5460
   cgatttttgt gatgctcgtc aggggggcgg agcctatgga aaaacgccag caacgcggcc 5520
   tttttacggt tcctggcctt ttgctggcct tttgctcaca tgttctttcc tgcgttatcc 5580
   cctgattctg tggataaccg tattaccgcc tttgagtgag ctgataccgc tcgccgcagc 5640
   cgaacgaccg agcgcagcga gtcagtgagc gaggaagcgg aagagcgccc aatacgcaaa 5700
   ccgcctctcc ccgcgcgttg gccgattcat taatgcagct ggcgcgccac tgccgagatc 5760
   cagtctacac tgattaattt tcgggccaat aatttaaaaa aatcgtgtta tataatatta 5820
   tatgtattat atatatacat catgatgata ctgacagtca tgtcccattg ctaaatagac 5880
   agactccatc tgccgcctcc aactgatgtt ctcaatattt aaggggtcat ctcgcattgt 5940
   ttaataataa acagactcca tctaccgcct ccaaatgatg ttctcaaaat atattgtatg 6000
   aacttatttt tattacttag tattattaga caacttactt gctttatgaa aaacacttcc 6060
   tatttaggaa acaatttata atggcagttc gttcatttaa caatttatgt agaataaatg 6120
   ttataaatgc gtatgggaaa tcttaaatat ggatagcata aatgatatct gcattgccta 6180
   attcgaaatc aacagcaacg aaaaaaatcc cttgtacaac ataaatagtc atcgagaaat 6240
   atcaactatc aaagaacagc tattcacacg ttactattga gattattatt ggacgagaat 6300
   cacacactca actgtctttc tctcttctag aaatacaggt acaagtatgt actattctca 6360
   ttgttcatac ttctagtcat ttcatcccac atattccttg gatttctctc caatgaatga 6420
   cattctatct tgcaaattca acaattataa taagatatac caaagtagcg gtatagtggc 6480
   aatcaaaaag cttctctggt gtgcttctcg tatttatttt tattctaatg atccattaaa 6540
   ggtatatatt tatttcttgt tatataatcc ttttgtttat tacatgggct ggatacataa 6600
   aggtattttg atttaatttt ttgcttaaat tcaatccccc ctcgttcagt gtcaactgta 6660
   atggtaggaa attaccatac ttttgaagaa gcaaaaaaaa tgaaagaaaa aaaaaatcgt 6720
   atttccaggt tagacgttcc gcagaatcta gaatgcggta tgcggtacat tgttcttcga 6780
   acgtaaaagt tgcgctccct gagatattgt acatttttgc ttttacaagt acaagtacat 6840
   cgtacaacta tgtactactg ttgatgcatc cacaacagtt tgttttgttt ttttttgttt 6900
   tttttttttc taatgattca ttaccgctat gtatacctac ttgtacttgt agtaagccgg 6960
   gttattggcg ttcaattaat catagactta tgaatctgca cggtgtgcgc tgcgagttac 7020
   ttttagctta tgcatgctac ttgggtgtaa tattgggatc tgttcggaaa tcaacggatg 7080
   ctcctaggtt cccaaaatcc cagatgcttc tctccagtgc caaaagtaag taccccacag 7140
   gttttcggcc gaaaattcca cgtgcagcaa cgtcgtgtgg ggtgttaaaa tgtggggggg 7200
   gggaaccagg acaagaggct cttgtgggag ccgaatgaga gcacaaagcg ggcgggtgtg 7260
   ataagggcat ttttgcccat tttcccttct cctgtctctc cgacggtgat ggcgttgtgc 7320
   gtcctctatt tctttttatt tctttttgtt ttatttctct gactaccgat ttggtttgat 7380
   ttcctcaacc ccacacaaat aagctcgggc cgaggaatat atatatacac ggacacagtc 7440
   gccctgtgga caacacgtca ctacctctac gatacacacc gtacgatagt tagtagacaa 7500
   caatcgatag ttggagcaag ggagaaatgt agagtgtgaa agactcacta tggtccgggc 7560
   ttatctcgac caatagccaa agtctggagt ttctgagaga aaaaggcaag atacgtatgt 7620
   aacaaagcga cgcatggtac aataataccg gaggcatgta tcatagagag ttagtggttc 7680
   gatgatggca ctggtgcctg gtatgacttt atacggctga ctacatattt gtcctcagac 7740
   atacaattac agtcaagcac ttacccttgg acatctgtag gtaccccccg gccaagacga 7800
   tctcagcgtg tcgtatgtcg gattggcgta gctccctcgc tcgtcaattg gctcccatct 7860
   actttcttct gcttggctac acccagcatg tctgctatgg ctcgttttcg tgccttatct 7920
   atcctcccag tattaccaac tctaaatgac atgatgtgat tgggtctaca ctttcatatc 7980
   agagataagg agtagcacag ttgcataaaa agcccaactc taatcagctt cttcctttct 8040
   tgtaattagt acaaaggtga ttagcgaaat ctggaagctt agttggccct aaaaaaatca 8100
   aaaaaagcaa aaaacgaaaa acgaaaaacc acagttttga gaacagggag gtaacgaagg 8160
   atcgtatata tatatatata tatatatacc cacggatccc gagaccggcc tttgattctt 8220
   ccctacaacc aaccattctc accaccctaa ttcacaacca tgtccgttgc atccaagctc 8280
   gtcttctacg tccgcgccgc catcgccgtg gtcatctttg ccgcctgtgc cacctacggc 8340
   gtgctggcgt ccaccattct caccgccatc ggcaagcagg gcctggccca atggaccgtt 8400
   gccagagcct tctactactc ggtgcgcatc ttcctgggta tcagcatcaa gctgcgtagc 8460
   cggcaggtga ccggaaccgc cggtctggat gcctccaaga tccaggtcgc caacaccacc 8520
   aagcccattg acgacatcac caaacacctg ccccgaccat gcattctgat ttccaaccac 8580
   cagaacgaaa tggacattct ggtgctcggt cgcatcttcc cccagtactg ctccgtcacc 8640
   gccaaaaagg ccctcaagtg gtaccctctg ctgggccagt tcatggcgct gtccggcacc 8700
   atcttcctgg accgaaagga ccgaaccaag tccgtgcaga ccctcggcgg cgccgtcaag 8760
   accatccaga gcggcaacgg aggcaagggc cagagcgtct tcatgttccc cgagggaacc 8820
   cgatcctact ccaaggacgt cggcatcatg cccttcaaga agggctgttt ccacctggcg 8880
   gtccagtcgg gcgctcccat tgtccccgtg gtggtccaga acacctcccg aatgttttct 8940
   ttcggccgag gcaagctgga cgccggagag atccttgtcg acgtcctgag ccccattgag 9000
   accaagggtc tggacgccag caacgtcgac gctctcatgg ccaccactta taaggccatg 9060
   tgcgagactg ccgaccagat tggctacgct ggccagaaga ctcagtaggc ggccgcatga 9120
   gaagataaat atataaatac attgagatat taaatgcgct agattagaga gcctcatact 9180
   gctcggagag aagccaagac gagtactcaa aggggattac accatccata tccacagaca 9240
   caagctgggg aaaggttcta tatacacttt ccggaatacc gtagtttccg atgttatcaa 9300
   tgggggcagc caggatttca ggcacttcgg tgtctcgggg tgaaatggcg ttcttggcct 9360
   ccatcaagtc gtaccatgtc ttcatttgcc tgtcaaagta aaacagaagc agatgaagaa 9420
   tgaacttgaa gtgaaggaat ttaaatgtaa cgaaactgaa atttgaccag atattgtgtc 9480
   cgcggtggag ctccagcttt tgttcccttt agtgagggtt aatttcgagc ttggcgtaat 9540
   catggtcata gctgtttcct gtgtgaaatt gttatccgct cacaagcttc cacacaac        9598

## Description

This application claims the benefit of U.S. Provisional Applications No. 61/187366, No. 61/187368 and No. 61/187359.

### FIELD OF THE INVENTION

This invention is in the field of biotechnology. More specifically, this invention pertains to an engineered strain of the oleaginous yeast *Yarrowia lipolytica* that is capable of efficiently producing eicosapentaenoic acid, an ω-3 polyunsaturated fatty acid ["PUFA"], in high concentrations.

### BACKGROUND OF THE INVENTION

The clinical and pharmaceutical value of eicosapentaenoic acid ["EPA"; *cis-5,* 8, 11, 14, 17-eicosapentaenoic acid; ω-3] are well known (U.S. Pat. Appl. Pub. No. 2009-0093543-A1). Similarly, the advantages of producing EPA in microbes using recombinant means, as opposed to producing EPA from natural microbial sources or via isolation from fish oil and marine plankton, are also well recognized.

Although the literature reports a number of recent examples whereby various portions of the ω-3/ω-6 polyunsaturated fatty acid ["PUFA"] biosynthetic pathway, responsible for EPA production, have been introduced into plants and non-oleaginous yeast, significant efforts by the Applicants' Assignee has focused on the use of the oleaginous yeast, *Yarrowia lipolytica* (U.S. Patent 7,238,482; U.S. Pat. Appl. Pub. No. 2006-0115881-A1; U.S. Pat. Appl. Pub. No. 2009-0093543-A1). Oleaginous yeast are defined as those yeast that are naturally capable of oil synthesis and accumulation, wherein oil accumulation is at least 25% of the cellular dry weight.

More specifically, U.S. Pat. Appl. Pub. No. 2006-0115881-A1 demonstrated production of 9% EPA of total fatty acids in a recombinant *Yarrowia lipolytica* strain without co-synthesis of γ-linolenic acid ["GLA"; ω-6], by expression of the following genes: Δ9 elongase, Δ8 desaturase, Δ5 desaturase, Δ17 desaturase, Δ12 desaturase and C_{16/18} elongase.

U.S. Pat. Appl. Pub. No. 2009-0093543-A1 describes optimized recombinant *Yarrowia lipolytica* strains for EPA production and demonstrated production of up to 55.6% EPA of total fatty acids in a recombinant Y. *lipolytica* strain by expression of the following genes: Δ9 elongase, Δ8 desaturase, Δ5 desaturase, Δ17 desaturase, Δ12 desaturase, C_{16/18} elongase and diacylglycerol cholinephosphotransferase, within a host cell comprising a disruption in the native peroxisome biogenesis factor 10 protein (PEX10).

U.S. Pat. Appl. Pub. No. 2008-254191-A1 describes a multizyme comprising for example a delta-8 desaturase and a delta-9 elongase connected by a linker for enhancing the production of PUFA. A recombinant *Yarrowia lipolytica* comprising a multizyme is disclosed.

U.S. Pat. Appl. Pub. No. 2006-0094092-A1 describes engineered recombinant *Yarrowia lipolytica* strains for ARA production, wherein the cells comprise a variety of heterologous desaturases, and/or acyltransferases.

U.S. Pat. Appl. Pub. No. 2009-0082286-A1 describes methods for enhancing the production of resveratrol in recombinant oleaginous organisms. Described therein is a recombinant *Yarrowia lipolytica* comprising a heterologous malonyl-CoA synthetase enzyme.

Despite the disclosures cited above, strain improvements are necessary for commercial production of EPA that will permit production of high EPA as a weight percent of the total fatty acids in addition to high total lipid content, while minimizing production of intermediate fatty acids, such as linoleic acid ["LA"; ω-6], and byproduct fatty acids in the final oil product. Applicants have solved the stated problem by engineering improved optimized strains of *Yarrowia lipolytica,* wherein the improvement enables at least one of the following: production of 61.8% EPA in the total oil fraction, production of 39.6% total fatty acids as a percent of the dry cell weight, or production of lipids having an EPA to LA ratio of 6.1.

### SUMMARY OF THE INVENTION

In a first embodiment, the invention concerns a recombinant *Yarrowia* sp. host cell that produces an oil comprising:
(a) at least 50 weight percent of eicosapentaenoic acid measured as a weight percent of total fatty acids; and
(b) said oil has a ratio of at least 3.5 of eicosapentaenoic acid, measured as a weight percent of total fatty acids, to linoleic acid, measured as a weight percent of total fatty acids, wherein said host cell comprises:
   (a) at least at least one multizyme which comprises a polypeptide having at least one delta-9 elongase linked to at least one delta-8 desaturase;
   (b) at least one peroxisome biogenesis factor protein whose expression has been down-regulated; and
   (c) at least one recombinant construct comprising a non-native chimeric gene comprising a nucleotide sequence encoding a heterologous malonyl-CoA synthetase, and at least one recombinant construct comprising a non-native chimeric gene comprising a nucleotide sequence encoding an acyl-CoA lysophospholipid acyltransferase (LPLAT) such that the LPLAT is overexpressed in the host cell, wherein said chimeric genes comprise regulatory and coding sequences not found together in nature.

Preferably, the nucleotide sequence encoding the malonyl-CoA synthetase encodes a polypeptide consisting essentially of an amino acid sequence selected from the group consisting of SEQ ID NO:40 and SEQ ID NO:42.

Preferably, the nucleotide sequence encoding the acyl-CoA lysophospholipid acyltransferase encodes a polypeptide consisting essentially of an amino acid sequence selected from the group consisting of SEQ ID NO:15, SEQ ID NO:17, SEQ ID NO:25, SEQ ID NO:29, SEQ ID NO:31 and SEQ ID NO:32.

Preferably, the multizyme comprises a linker between the delta-9 elongase and delta-8 desaturase, which linker is selected from the group consisting of SEQ ID NO:1, SEQ ID NO:2, SEQ ID NO:3, SEQ ID NO:4, SEQ ID NO:5, SEQ ID NO:6 and SEQ ID NO:7.

Preferably, the multizyme consists essentially of a sequence selected from the group consisting of: SEQ ID NO:9, SEQ ID NO:11 and SEQ ID NO:13.

In a second embodiment, the invention concerns a method for making a microbial oil comprising eicosapentaenoic acid comprising:
a) culturing the host cell of any of the invention wherein a microbial oil comprising eicosapentaenoic acid is produced; and,
b) optionally recovering the microbial oil of step (a).

### BIOLOGICAL DEPOSITS

The following biological materials have been deposited with the American Type Culture Collection (ATCC), 10801 University Boulevard, Manassas, VA 20110-2209, and bear the following designations, accession numbers and dates of deposit.

| Biological Material | Accession No. | Date of Deposit |
|---|---|---|
| *Yarrowia lipolytica* Y8406 | ATCC PTA-10025 | May 14, 2009 |
| *Yarrowia lipolytica* Y8412 | ATCC PTA-10026 | May 14, 2009 |
| *Yarrowia lipolytica* Y8259 | ATCC PTA-10027 | May 14, 2009 |

The biological materials listed above were deposited under the terms of the Budapest Treaty on the International Recognition of the Deposit of Microorganisms for the Purposes of Patent Procedure. The listed deposit will be maintained in the indicated international depository for at least 30 years and will be made available to the public upon the grant of a patent disclosing it. The availability of a deposit does not constitute a license to practice the subject invention in derogation of patent rights granted by government action.

### BRIEF DESCRIPTION OF THE DRAWINGS AND

### SEQUENCE DESCRIPTIONS

FIG. 1A and FIG. 1 B illustrate the ω-3/ω-6 fatty acid biosynthetic pathway, and should be viewed together when considering the description of this pathway below.
FIG. 2 diagrams the development of *Yarrowia lipolytica* strains Y9481, Y9497 and Y9502, producing greater than 60.9% EPA in the total lipid fraction.
FIG. 3 provides a plasmid map for pY116.
FIG. 4 provides plasmid maps for the following: (A) pZKSL-5S5A5; and, (B) pZP3-Pa777U.
FIG. 5 provides plasmid maps for the following: (A) pZKUM; and, (B) pZKL2-5mB89C.
FIG. 6 provides plasmid maps for the following: (A) pZKL1-2SR9G85; and, (B) pZSCP-Ma83.
FIG. 7 provides plasmid maps for the following: (A) pZKL4-398F2; and, (B) pZP2-85m98F.
FIG. 8 provides plasmid maps for the following: (A) pZK16-ML8N; and, (B) pZK16-ML.
FIG. 9 diagrams the development of *Yarrowia lipolytica* strain Y8672, producing greater than 61.8% EPA in the total lipid fraction.
FIG. 10 provides plasmid maps for the following: (A) pZKL2-5m89C; and, (B) pY201, comprising a chimeric YAT1::ScAle1S::Lip1 gene.
FIG. 11 provides plasmid maps for the following: (A) pY168, comprising a chimeric YAT1::YIAle1::Lip1 gene; and, (B) pY208, comprising a chimeric YAT1 ::MaLPAAT1S::Lip1 gene.
FIG. 12 provides plasmid maps for the following: (A) pY207, comprising a chimeric YAT1::YILPAAT1::Lip1 gene; and, (B) pY175, comprising a chimeric YAT1 ::CeLPCATS::Lip1 gene.

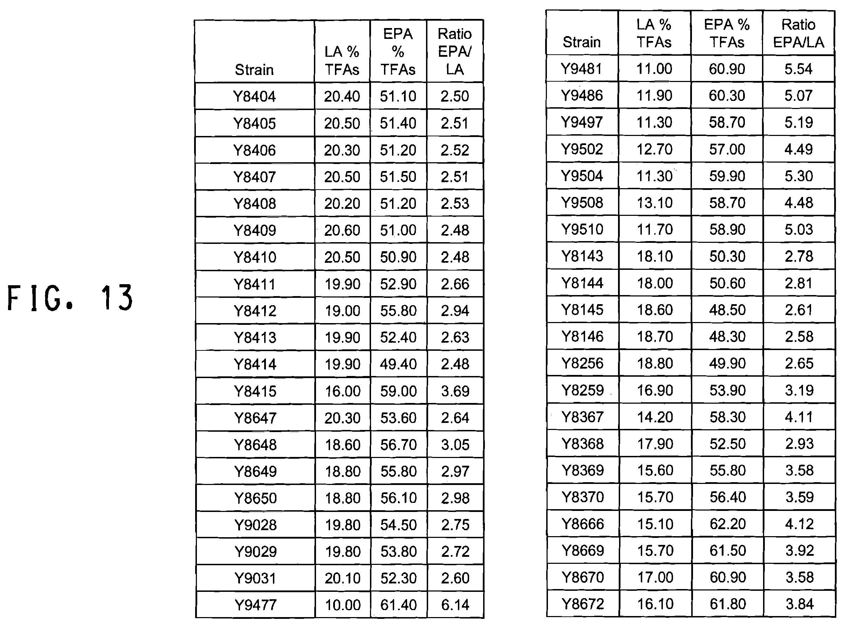

FIG. 13 provides a comparison of EPA % TFAs, LA % TFAs and the ratio of EPA % TFAs to LA % TFAs in each of the strains described in the Examples.
FIG. 14 provides plasmid maps for the following: (A) pY222, comprising a chimeric YAT1::ScLPAATS::Lip1 gene; and, (B) pY177, comprising a chimeric YAT1::YILPAAT1::Lip1 gene.

The invention can be more fully understood from the following detailed description and the accompanying sequence descriptions, which form a part of this application.

The following sequences comply with 37 C.F.R. §1.821-1.825 ("Requirements for Patent Applications Containing Nucleotide Sequences and/or Amino Acid Sequence Disclosures - the Sequence Rules") and are consistent with World Intellectual Property Organization (WIPO) Standard ST.25 (1998) and the sequence listing requirements of the EPO and PCT (Rules 5.2 and 49.5(a-bis), and Section 208 and Annex C of the Administrative Instructions). The symbols and format used for nucleotide and amino acid sequence data comply with the rules set forth in 37 C.F.R. §1.822.

SEQ ID NOs:1-156 are ORFs encoding promoters, genes or proteins (or fragments thereof) or plasmids, as identified in Table 1.

**Table 1: Summary of Gene and Protein SEQ ID Numbers**

| Description | Nucleic acid SEO ID NO. | Protein SEQ ID NO. |
|---|---|---|
| Multizyme linker GAGPARPAGLPPATYYDSLAVMGS | -- | 1 |
| Multizyme linker GPARPAGLPPATYYDSLAV | -- | 2 |
| Multizyme linker PARPAGLPPATYYDSLAV | -- | 3 |
| Multizyme linker PTRPAGPPPATYYDSLAV | -- | 4 |
| Multizyme linker PGGPGKPSEIASLPPPIRPVGNPPAAYYDALAT | -- | 5 |
| Multizyme linker PARPAGLPPATYYDSLAVSGRT | -- | 6 |
| Multizyme linker PGGPGKPSEIASLPPPIRPVGNPPAAYYDALATGR T | -- | 7 |
| DGLA synthase, comprising EgD9eS/EgD8M gene fusion | 8 (2112 bp) | 9 (703 AA) |
| DGLA synthase, comprising EaD9eS/EaD8S gene fusion | 10 (2109 bp) | 11 (702 AA) |
| DGLA synthase, comprising E389D9eS/EgD8M gene fusion | 12 (2127 bp) | 13 (708 AA) |
| *Saccharomyces cerevisiae* Ale1 ("ScAle1"; also ORF "YOR175C") | 14 (1860 bp) | 15 (619 AA) |
| *Yarrowia lipolytica* Ale1 ("YIAle1") | 16 (1539 bp) | 17 (512 AA) |
| membrane bound *O*-acyltransferase motif M(V/I)LxxKL | -- | 18 |
| membrane bound *O*-acyltransferase motif RxKYYxxW | -- | 19 |
| membrane bound *O*-acyltransferase motif SAxWHG | -- | 20 |
| U.S. Pat. Appl. Pub. No. 2008-0145867-A1 motif EX₁₁WNX₂-[T/V]-X₂W | -- | 21 |
| Synthetic Ale1 derived from *Saccharomyces cerevisiae,* codon-optimized for expression in *Yarrowia lipolytica* ("ScAle1S") | 22 (1870 bp) | 23 (619 AA) |
| *Caenorhabditis elegans* LPCAT ("CeLPCAT") | 24 (849 bp) | 25 (282 AA) |
| Synthetic LPCAT derived from *Caenorhabditis elegans*, codon-optimized for expression in *Yarrowia lipolytica* ("CeLPCATS") | 26 (859 bp) | 27 (282 AA) |
| *Mortierella alpina* LPAAT1 ("MaLPAAT1") | 28 (945 bp) | 29 (314 AA) |
| *Yarrowia lipolytica* LPAAT1 ("YILPAAT1") | 30 (1549 bp) | 31 (282 AA) |
| *Saccharomyces cerevisiae* LPAAT ("ScLPAAT"; also ORF "YDL052C") | -- | 32 (303 AA) |
| 1-acyl-sn-glycerol-3-phosphate acyltransferase motif NHxxxxD | -- | 33 |
| 1-acyl-sn-glycerol-3-phosphate acyltransferase motif EGTR | -- | 34 |
| Synthetic LPAAT1 derived from *Mortierella alpina,* codon-optimized for expression in *Yarrowia lipolytica* ("MaLPAAT1S") | 35 (955 bp) | 36 (314 AA) |
| *Yarrowia lipolytica* diacylglycerol cholinephosphotransferase gene ("YICPT1") | 37 (1185 bp) | 38 (394 AA) |
| *Rhizobium leguminosarum* bv. viciae 3841 malonyl-CoA synthetase (GenBank Accession No. YP_766603) ("rMCS") | 39 (1515 bp) | 40 (504 AA) |
| Synthetic malonyl-CoA synthetase derived from *Rhizobium leguminosarum* bv. viciae 3841 (GenBank Accession No. YP_766603), codon-optimized for expression in *Yarrowia lipolytica* ("MCS") | 41 (1518 bp) | 42 (505 AA) |
| *Euglena gracilis* Δ9 elongase ("EgD9e") | 43 (777 bp) | 44 (258 AA) |
| Synthetic Δ9 elongase derived from *Euglena gracilis,* codon-optimized for expression in *Yarrowia lipolytica* ("EgD9eS") | 45 (777 bp) | 46 (258 AA) |
| *Eutreptiella sp.* CCMP389 Δ9 elongase ("E389D9e") | 47 (792 bp) | 48 (263 AA) |
| Synthetic Δ9 elongase derived from *Eutreptiella* sp. CCMP389 codon-optimized for expression in *Yarrowia lipolytica* ("E389D9eS") | 49 (792 bp) | 50 (263 AA) |
| *Euglena anabaena* UTEX 373 Δ9 elongase ("EaD9Elo1") | 51 (774 bp) | 52 (258 AA) |
| Synthetic Δ9 elongase derived from *Euglena anabaena* UTEX 373, codon-optimized for expression in *Yarrowia lipolytica* ("EaD9eS") | 53 (774 bp) | 54 (258 AA) |
| *Euglena gracilis* Δ8 desaturase ("Eg5" or "EgD8") | 55 (1271 bp) | 56 (421 AA) |
| Synthetic Δ8 desaturase derived from *Euglena gracilis,* codon-optimized for expression in *Yarrowia lipolytica* ("D8SF" or "EgD8S") | 57 (1272 bp) | 58 (422 AA) |
| Synthetic mutant Δ8 desaturase ("EgD8M"), derived from *Euglena gracilis* ("EgD8S") | 59 (1272 bp) | 60 (422 AA) |
| *Euglena anabaena* UTEX 373 Δ8 desaturase ("EaD8es3") | 61 (1260 bp) | 62 (420 AA) |
| Synthetic Δ8 desaturase derived from *Euglena anabaena* UTEX 373, codon-optimized for expression in *Yarrowia lipolytica* ("EaD8S") | 63 (1260 bp) | 64 (420 AA) |
| *Euglena gracilis* Δ5 desaturase ("EgD5") | 65 (1350 bp) | 66 (449 AA) |
| Synthetic Δ5 desaturase derived from *Euglena gracilis,* codon-optimized for expression in *Yarrowia lipolytica* ("EgD5S") | 67 (1350 bp) | 68 (449 AA) |
| Mutant Δ5 desaturase ("EgD5M"), derived from | 69 | 70 |
| Euglena gracilis ("EgD5") (U.S. Pat. Pub. No. 2010-0075386-A1) | (1350 bp) | (449 AA) |
| Synthetic mutant Δ5 desaturase ("EgD5SM"), derived from *Euglena gracilis* ("EgD5S") (U.S. Pat. Pub. No. 2010-0075386-A1) | 71 (1350 bp) | 72 (449 AA) |
| *Peridinium sp.* CCMP626 Δ5 desaturase ("RD5") | 73 (1392 bp) | 74 (463 AA) |
| Synthetic Δ5 desaturase derived from *Peridinium sp.* CCMP626, codon-optimized for expression in *Yarrowia lipolytica* ("RD5S") | 75 (1392 bp) | 76 (463 AA) |
| *Euglena anabaena* UTEX 373 Δ5 desaturase ("EaD5") | 77 (1362 bp) | 78 (454 AA) |
| Synthetic Δ5 desaturase derived from *Euglena anabaena* UTEX 373, codon-optimized for expression in *Yarrowia lipolytica* ("EaD5S") | 79 (1362 bp) | 80 (454 AA) |
| Synthetic mutant Δ5 desaturase ("EaD5SM"), derived from *Euglena anabaena* ("EaD5S") (U.S. Pat. Pub. No. 2010-0075386-A1) | 81 (1365 bp) | 82 (454 AA) |
| *Phytophthora ramorum* Δ17 desaturase ("PrD17") | 83 (1086 bp) | 84 (361 AA) |
| Synthetic Δ17 desaturase derived from *Phytophthora ramorum,* codon-optimized for expression in *Yarrowia lipolytica* ("PrD17S") | 85 (1086 bp) | 86 (361 AA) |
| *Pythium aphanidermatum* Δ17 desaturase ("PaD17") | 87 (1080 bp) | 88 (359 AA) |
| Synthetic Δ17 desaturase derived from *Pythium aphanidermatum,* codon-optimized for expression in *Yarrowia lipolytica* ("PaD17S") | 89 (1080 bp) | 90 (359 AA) |
| *Fusarium moniliforme* Δ12 desaturase ("FmD12") | 91 (1434 bp) | 92 (477 AA) |
| Synthetic Δ12 desaturase derived from *Fusarium moniliforme,* codon-optimized for expression in *Yarrowia lipolytica* ("FmD12S") | 93 (1434 bp) | 94 (477 AA) |
| *Mortierella alpina* C_{16/18} elongase | 95 (828 bp) | 96 (275 AA) |
| Synthetic C_{16/18} elongase derived from *Mortierella alpina* ELO3, codon-optimized for expression in *Yarrowia lipolytica* ("ME3S") | 97 (828 bp) | 98 (275 AA) |
| Shindou et al. membrane bound *O*-acyltransferase motif WHGxxxGYxxxF | -- | 99 |
| Shindou et al. membrane bound *O*-acyltransferase motif YxxxxF | -- | 100 |
| Shindou et al. membrane bound *O*-acyltransferase motif YxxxYFxxH | -- | 101 |
| U.S. Pat. Appl. Pub. No. 2008-0145867-A1 motif M-[V/I]-[L/I]-xxK-[L/V/I]-xxxxxxDG | -- | 102 |
| U.S. Pat. Appl. Pub. No. 2008-0145867-A1 motif RxKYYxxWxxx-[E/D]-[A/G]xxxxGxG-[F/Y]-xG | -- | 103 |
| U.S. Pat. Appl. Pub. No. 2008-0145867-A1 motif SAxWHGxxPGYxx-[T/F]-F | -- | 104 |
| Lewin, T.W. et al. & Yamashita et al. 1-acyl-*sn-*glycerol-3-phosphate acyltransferase motif GxxFI-[D/R]-R | -- | 105 |
| Lewin, T.W. et al. 1-acyl-*sn*-glycerol-3-phosphate acyltransferase motif [V/I]-[P/X]-[I/V/L]-[I/V]-P-[V/I] | -- | 106 |
| Yamashita et al. 1-acyl-*sn*-glycerol-3-phosphate acyltransferase motif IVPIVM | -- | 107 |
| *Yarrowia lipolytica* Pex1p (GenBank Accession No. CAG82178) | -- | 108 (1024 AA) |
| *Yarrowia lipolytica* Pex2p (GenBank Accession No. CAG77647) | -- | 109 (381 AA) |
| *Yarrowia lipolytica* Pex3p (GenBank Accession No. CAG78565) | -- | 110 (431 AA) |
| *Yarrowia lipolytica* Pex3Bp (GenBank Accession No. CAG83356) | -- | 111 (395 AA) |
| *Yarrowia lipolytica* Pex4p (GenBank Accession No. CAG79130) | -- | 112 (153 AA) |
| *Yarrowia lipolytica* Pex5p (GenBank Accession No. CAG78803) | -- | 113 (598 AA) |
| *Yarrowia lipolytica* Pex6p (GenBank Accession No. CAG82306) | -- | 114 (1024 AA) |
| *Yarrowia lipolytica* Pex7p (GenBank Accession No. CAG78389) | -- | 115 (356 AA) |
| *Yarrowia lipolytica* Pex8p (GenBank Accession No. CAG80447) | -- | 116 (671 AA) |
| *Yarrowia lipolytica* Pex10p (GenBank Accession No. CAG81606) | -- | 117 (377 AA) |
| *Yarrowia lipolytica* Pex12p (GenBank Accession No. CAG81532) | -- | 118 (408 AA) |
| *Yarrowia lipolytica* Pex13p (GenBank Accession No. CAG81789) | -- | 119 (412 AA) |
| *Yarrowia lipolytica* Pex14p (GenBank Accession No. CAG79323) | -- | 120 (380 AA) |
| *Yarrowia lipolytica* Pex16p (GenBank Accession No. CAG79622) | -- | 121 (391 AA) |
| *Yarrowia lipolytica* Pex17p (GenBank Accession No. CAG84025) | -- | 122 (225 AA) |
| *Yarrowia lipolytica* Pex19p (GenBank Accession No. AAK84827) | -- | 123 (324 AA) |
| *Yarrowia lipolytica* Pex20p (GenBank Accession No. CAG79226) | -- | 124 (417 AA) |
| *Yarrowia lipolytica* Pex22p (GenBank Accession No. CAG77876) | -- | 125 (195 AA) |
| *Yarrowia lipolytica* Pex26p (GenBank Accession No. NC_006072, antisense translation of nucleotides 117230-118387) | -- | 126 (386 AA) |
| Plasmid pY116 | 127 (8739 bp) | -- |
| Plasmid pZKSL-5S5A5 | 128 (13975 bp) | -- |
| Plasmid pZP3-Pa777U | 129 (13066 bp) | -- |
| Plasmid pZKUM | 130 (4313 bp) | - |
| Plasmid pZKL2-5mB89C | 131 (15991 bp) | -- |
| Plasmid pZKL1-2SR9G85 | 132 (14554 bp) | -- |
| Plasmid pZSCP-Ma83 | 133 (15119 bp) | -- |
| Plasmid pZKL4-398F2 | 134 (14623 bp) | -- |
| Plasmid pZP2-85m98F | 135 (14619 bp) | -- |
| Plasmid pZK16-ML8N | 136 (15262 bp) | -- |
| Plasmid pZK16-ML | 137 (13075bp) | -- |
| Plasmid pZKL2-5m89C | 138 (15799 bp) | -- |
| Plasmid pY201 | 139 (9641 bp) | -- |
| *Escherichia coli* LoxP recombination site, recognized by a Cre recombinase enzyme | 140 (34 bp) | -- |
| Primer 798 | 141 | -- |
| Primer 799 | 142 | -- |
| Primer 800 | 143 | -- |
| Primer 801 | 144 | -- |
| Plasmid pY168 | 145 (9320 bp) | -- |
| Plasmid pY208 | 146 (8726 bp) | -- |
| Primer 856 | 147 | -- |
| Primer 857 | 148 | -- |
| Plasmid pY207 | 149 (8630 bp) | -- |
| Plasmid pY175 | 150 (8630 bp) | -- |
| Synthetic LPAAT derived from *Saccharomyces cerevisiae,* codon-optimized for expression in *Yarrowia lipolytica* ("ScLPAATS") | 151 (926 bp) | 152 (303 AA) |
| Plasmid pY222 | 153 (7891 bp) | -- |
| Primer 869 | 154 | -- |
| Primer 870 | 155 | -- |
| Plasmid pY177 | 156 (9598 bp) | -- |

### DETAILED DESCRIPTION OF THE INVENTION

Described herein are production host strains of *Yarrowia lipolytica* that are capable of producing greater than 50% eicosapentaenoic acid ["EPA"; 20:5 ω-3]. Accumulation of this particular polyunsaturated fatty acid ["PUFA"] is accomplished by introduction of a functional ω-3/ω-6 fatty acid biosynthetic pathway comprising proteins with Δ9 elongase, Δ8 desaturase, Δ5 desaturase, Δ17 desaturase, Δ12 desaturase and C_{16/18} elongase activities, which thereby enables production of an EPA oil with minimal γ-linolenic acid ["GLA"]. Thus, this disclosure demonstrates that *Y. lipolytica* can be engineered to enable commercial production of EPA and derivatives thereof. Methods of production, are also claimed.

PUFAs, such as EPA (or derivatives thereof), are used as dietary substitutes, or supplements, particularly infant formulas, for patients undergoing intravenous feeding or for preventing or treating malnutrition. Alternatively, the purified PUFAs (or derivatives thereof) may be incorporated into cooking oils, fats or margarines formulated so that in normal use the recipient would receive the desired amount for dietary supplementation. The PUFAs may also be incorporated into infant formulas, nutritional supplements or other food products and may find use as anti-inflammatory or cholesterol lowering agents. Optionally, the compositions may be used for pharmaceutical use, either human or veterinary.

Supplementation of humans or animals with PUFAs produced by recombinant means can result in increased levels of the added PUFAs, as well as their metabolic progeny. For example, treatment with EPA can result not only in increased levels of EPA, but also downstream products of EPA such as eicosanoids (i.e., prostaglandins, leukotrienes, thromboxanes), docosapentaenoic acid ["DPA"; *cis*-7, 10, 13, 16, 19-docosapentaenoic; 22:5 ω-3] and docosahexaenoic acid ["DHA"; *cis*-4, 7, 10, 13, 16, 19-docosahexaenoic acid; 22:6 ω-3]. Complex regulatory mechanisms can make it desirable to combine various PUFAs, or add different conjugates of PUFAs, in order to prevent, control or overcome such mechanisms to achieve the desired levels of specific PUFAs in an individual.

Alternately, PUFAs, or derivatives thereof, made by the methodology disclosed herein can be utilized in the synthesis of animal and aquaculture feeds, such as dry feeds, semi-moist and wet feeds, since these formulations generally require at least 1-2% of the nutrient composition to be ω-3 and/or ω-6 PUFAs.

In this disclosure, a number of terms and abbreviations are used. The following definitions are provided.
"Open reading frame" is abbreviated as "ORF".
"Polymerase chain reaction" is abbreviated as "PCR".
"American Type Culture Collection" is abbreviated as "ATCC".
"Polyunsaturated fatty acid(s)" is abbreviated as "PUFA(s)".
"Triacylglycerols" are abbreviated as "TAGs".
"Co-enzyme A" is abbreviated as "CoA".
"Total fatty acids" are abbreviated as "TFAs".
"Fatty acid methyl esters" are abbreviated as "FAMEs".
"Dry cell weight" is abbreviated as "DCW".

As used herein the term "invention" or "present invention" is intended to refer to all aspects and embodiments of the invention as described in the claims herein and should not be read so as to be limited to any particular embodiment or aspect.

The term "food product" refers to any food generally suitable for human consumption. Typical food products include, but are not limited to: meat products, cereal products, baked foods, snack foods, dairy products, beverages and the like. The terms "food analog", "functional food", "medical food" and "medical nutritional" are defined as in U.S. Pat. Appl. Pub. No. 2006-0115881-A1.

The term "pharmaceutical" as used herein means a compound or substance which if sold in the United States would be controlled by Section 503 or 505 of the Federal Food, Drug and Cosmetic Act.

The term "infant formula" means a food which is designed exclusively for consumption by the human infant by reason of its simulation of human breast milk. Typical commercial examples of infant formula include, but are not limited to: Similac® and Isomil®.

The term "dietary supplement" refers to a product that: (i) is intended to supplement the diet and thus is not represented for use as a conventional food or as a sole item of a meal or the diet; (ii) contains one or more dietary ingredients (including, e.g., vitamins, minerals, herbs or other botanicals, amino acids, enzymes and glandulars) or their constituents; (iii) is intended to be taken by mouth as a pill, capsule, tablet, or liquid; and, (iv) is labeled as being a dietary supplement.

The term "animal feed" refers to feeds intended exclusively for consumption by animals, including domestic animals such as pets, farm animals, etc. or for animals raised for the production of food, such as for e.g., fish farming. The terms "aquaculture feed", "aquafeed" and "feed nutrient" are as defined in U.S. Pat. Appl. Pub. No. 2006-0115881-A1.

As used herein the term "biomass" refers specifically to spent or used yeast cellular material from the fermentation of a recombinant production host producing EPA in commercially significant amounts, wherein the preferred production host is a recombinant strain of the oleaginous yeast, *Yarrowia lipolytica.* The biomass may be in the form of whole cells, whole cell lysates, homogenized cells, partially hydrolyzed cellular material, and/or partially purified cellular material (e.g., microbially produced oil).

The term "'lipids" refer to any fat-soluble (i.e., lipophilic), naturally-occurring molecule. A general overview of lipids is provided in U.S. Pat. Appl. Pub. No. 2009-0093543-A1 (see Table 2 therein).

The term "glycerophospholipids" refers to a broad class of molecules, having a glycerol core with fatty acids at the sn-1 position and sn-2 position, and a polar head group (e.g., phosphate, choline, ethanolamine, glycerol, inositol, serine, cardiolipin) joined at the *sn*-3 position via a phosphodiester bond. Glycerophospholipids thus include phosphatidic acid ["PA"], phosphatidylcholines ["PC"], phosphatidylethanolamines ["PE"], phosphatidylglycerols ["PG"], phosphatidylinositols ["PI"], phosphatidylserines ["PS"] and cardiolipins ["CL"]. Glycerophospholipids possess tremendous diversity, not only resulting from variable phosphoyl head groups, but also as a result of differing chain lengths and degrees of saturation of their fatty acids. Generally, saturated and monounsaturated fatty acids are esterified at the sn-1 position, while polyunsaturated fatty acids are esterified at the sn-2 position.

"Lysophospholipids" are derived from glycerophospholipids, by deacylation of the sn-2 position fatty acid. Lysophospholipids include, e.g., lysophosphatidic acid ["LPA"], lysophosphatidylcholine ["LPC"], lysophosphatidyletanolamine ["LPE"], lysophosphatidylserine ["LPS"], lysophosphatidylglycerol ["LPG"] and lysophosphatidylinositol ["LPI"].

The term "oil" refers to a lipid substance that is liquid at 25 °C and usually polyunsaturated. In oleaginous organisms, oil constitutes a major part of the total lipid. "Oil" is composed primarily of triacylglycerols ["TAGs"] but may also contain other neutral lipids, phospholipids and free fatty acids. The fatty acid composition in the oil and the fatty acid composition of the total lipid are generally similar; thus, an increase or decrease in the concentration of PUFAs in the total lipid will correspond with an increase or decrease in the concentration of PUFAs in the oil, and vice versa.

"Neutral lipids" refer to those lipids commonly found in cells in lipid bodies as storage fats and are so called because at cellular pH, the lipids bear no charged groups. Generally, they are completely non-polar with no affinity for water. Neutral lipids generally refer to mono-, di-, and/or triesters of glycerol with fatty acids, also called monoacylglycerol, diacylglycerol or triacylglycerol, respectively, or collectively, acylglycerols. A hydrolysis reaction must occur to release free fatty acids from acylglycerols.

The term "triacylglycerols" ["TAGs"] refers to neutral lipids composed of three fatty acyl residues esterified to a glycerol molecule. TAGs can contain long chain PUFAs and saturated fatty acids, as well as shorter chain saturated and unsaturated fatty acids.

The term "total fatty acids" ["TFAs"] herein refer to the sum of all cellular fatty acids that can be derivitized to fatty acid methyl esters ["FAMEs"] by the base transesterification method (as known in the art) in a given sample, which may be the biomass or oil, for example. Thus, total fatty acids include fatty acids from neutral lipid fractions (including diacylglycerols, monoacylglycerols and TAGs) and from polar lipid fractions (including, e.g., the PC and the PE fractions) but not free fatty acids.

The term "total lipid content" of cells is a measure of TFAs as a percent of the dry cell weight ["DCW"], although total lipid content can be approximated as a measure of FAMEs as a percent of the DCW ["FAMEs % DCW"]. Thus, total lipid content ["TFAs % DCW"] is equivalent to, e.g., milligrams of total fatty acids per 100 milligrams of DCW.

The concentration of a fatty acid in the total lipid is expressed herein as a weight percent of TFAs ["% TFAs"], e.g., milligrams of the given fatty acid per 100 milligrams of TFAs. Unless otherwise specifically stated in the disclosure herein, reference to the percent of a given fatty acid with respect to total lipids is equivalent to concentration of the fatty acid as % TFAs (e.g., % EPA of total lipids is equivalent to EPA % TFAs).

In some cases, it is useful to express the content of a given fatty acid(s) in a cell as its weight percent of the dry cell weight ["% DCW"]. Thus, for example, EPA % DCW would be determined according to the following formula: (EPA % TFAs) * (TFAs % DCW)]/100. The content of a given fatty acid(s) in a cell as its weight percent of the dry cell weight ["% DCW"] can be approximated, however, as: (EPA % TFAs) * (FAMEs % DCW)]/100.

The terms "lipid profile" and "lipid composition" are interchangeable and refer to the amount of individual fatty acids contained in a particular lipid fraction, such as in the total lipid or the oil, wherein the amount is expressed as a weight percent of TFAs. The sum of each individual fatty acid present in the mixture should be 100.

The term "extracted oil" refers to an oil that has been separated from other cellular materials, such as the microorganism in which the oil was synthesized. Extracted oils are obtained through a wide variety of methods, the simplest of which involves physical means alone. For example, mechanical crushing using various press configurations (e.g., screw, expeller, piston, bead beaters, etc.) can separate oil from cellular materials. Alternately, oil extraction can occur via treatment with various organic solvents (e.g., hexane), via enzymatic extraction, via osmotic shock, via ultrasonic extraction, via supercritical fluid extraction (e.g., CO₂ extraction), via saponification and via combinations of these methods. An extracted oil does not require that it is not necessarily purified or further concentrated. The extracted oils described herein will comprise at least 50 EPA % TFAs.

The term "blended oil" refers to an oil that is obtained by admixing, or blending, the extracted oil described herein with any combination of, or individual, oil to obtain a desired composition. Thus, for example, types of oils from different microbes can be mixed together to obtain a desired PUFA composition. Alternatively, or additionally, the PUFA-containing oils disclosed herein can be blended with fish oil, vegetable oil or a mixture of both to obtain a desired composition.

The term "fatty acids" refers to long chain aliphatic acids (alkanoic acids) of varying chain lengths, from about C₁₂ to C₂₂, although both longer and shorter chain-length acids are known. The predominant chain lengths are between C₁₆ and C₂₂. The structure of a fatty acid is represented by a simple notation system of "X:Y", where X is the total number of carbon ["C"] atoms in the particular fatty acid and Y is the number of double bonds. Additional details concerning the differentiation between "saturated fatty acids" versus "unsaturated fatty acids", "monounsaturated fatty acids" versus "polyunsaturated fatty acids" ["PUFAs"], and "omega-6 fatty acids" ["ω-6" or "n-6"] versus "omega-3 fatty acids" ["ω-3" or "n-3"] are provided in U.S. Patent 7,238,482.

Nomenclature used to describe PUFAs herein is given in Table 2. In the column titled "Shorthand Notation", the omega-reference system is used to indicate the number of carbons, the number of double bonds and the position of the double bond closest to the omega carbon, counting from the omega carbon, which is numbered 1 for this purpose. The remainder of the Table summarizes the common names of ω-3 and ω-6 fatty acids and their precursors, the abbreviations that will be used throughout the specification and the chemical name of each compound.

**Table 2: Nomenclature of Polyunsaturated Fatty Acids And Precursors**

| Common Name | Abbreviation | Chemical Name | Shorthand Notation |
|---|---|---|---|
| Myristic | -- | tetradecanoic | 14:0 |
| Palmitic | Palmitate | hexadecanoic | 16:0 |
| Palmitoleic | -- | 9-hexadecenoic | 16:1 |
| Stearic | -- | octadecanoic | 18:0 |
| Oleic | -- | *cis*-9-octadecenoic | 18:1 |
| Linoleic | LA | *cis*-9, 12-octadecadienoic | 18:2 ω-6 |
| γ-Linolenic | GLA | *cis*-6, 9, 12-octadecatrienoic | 18:3 ω-6 |
| Eicosadienoic | EDA | *cis*-11, 14-eicosadienoic | 20:2 ω-6 |
| Dihomo-γ-Linolenic | DGLA | *cis*-8, 11, 14-eicosatrienoic | 20:3 ω-6 |
| Arachidonic | ARA | *cis*-5, 8, 11, 14-eicosatetraenoic | 20:4 ω-6 |
| α-Linolenic | ALA | *cis*-9, 12, 15-octadecatrienoic | 18:3 ω-3 |
| Stearidonic | STA | *cis*-6, 9, 12, 15-octadecatetraenoic | 18:4 ω-3 |
| Eicosatrienoic | ETrA | *cis*-11, 14, 17-eicosatrienoic | 20:3 ω-3 |
| Sciadonic | SCI | *cis*-5, 11, 14-eicosatrienoic | 20:3b ω-6 |
| Juniperonic | JUP | *cis*-5, 11, 14, 17-eicosatetraenoic | 20:4b ω-3 |
| Eicosatetraenoic | ETA | *cis*-8, 11, 14, 17-eicosatetraenoic | 20:4 ω-3 |
| Eicosapentaenoic | EPA | *cis*-5, 8, 11, 14, 17-eicosapentaenoic | 20:5 ω-3 |
| Docosatetraenoic | DTA | *cis*-7, 10, 13, 16-docosatetraenoic | 22:4 ω-6 |
| Docosapentaenoic | DPAn-6 | *cis*-4, 7, 10, 13, 16-docosapentaenoic | 22:5 ω-6 |
| Docosapentaenoic | DPA | *cis*-7, 10, 13, 16, 19-docosapentaenoic | 22:5 ω-3 |
| Docosahexaenoic | DHA | *cis*-4, 7, 10, 13, 16, 19-docosahexaenoic | 22:6 ω-3 |

The term "PUFA biosynthetic pathway" refers to a metabolic process that converts oleic acid to ω-6 fatty acids such as LA, EDA, GLA, DGLA, ARA, DTA and DPAn-6 and ω-3 fatty acids such as ALA, STA, ETrA, ETA, EPA, DPA and DHA. This process is well described in the literature (e.g., see U.S. Pat. Appl. Pub. No. 2006-0115881-A1). Briefly, this process involves elongation of the carbon chain through the addition of carbon atoms and desaturation of the molecule through the addition of double bonds, via a series of special elongation and desaturation enzymes termed "PUFA biosynthetic pathway enzymes" that are present in the endoplasmic reticulum membrane. More specifically, "PUFA biosynthetic pathway enzymes" refer to any of the following enzymes (and genes which encode said enzymes) associated with the biosynthesis of a PUFA, including: Δ4 desaturase, Δ5 desaturase, Δ6 desaturase, Δ12 desaturase, Δ15 desaturase, Δ17 desaturase, Δ9 desaturase, Δ8 desaturase, Δ9 elongase, C_{14/16} elongase, C_{16/18} elongase, C_{18/20} elongase and/or C_{20/22} elongase.

The term "Δ9 elongase/Δ8 desaturase pathway" will refer to a PUFA biosynthetic pathway that minimally includes at least one Δ9 elongase and at least one Δ8 desaturase, thereby enabling biosynthesis of DGLA and/or ETA from LA and ALA, respectively, with EDA and/or ETrA as intermediate fatty acids. With expression of other desaturases and elongases, ARA, DTA, DPAn-6, EPA, DPA and DHA may also be synthesized.

The term "desaturase" refers to a polypeptide that can desaturate, i.e., introduce a double bond, in one or more fatty acids to produce a fatty acid or precursor of interest. Despite use of the omega-reference system throughout the specification to refer to specific fatty acids, it is more convenient to indicate the activity of a desaturase by counting from the carboxyl end of the substrate using the delta-system. Of particular interest herein are: Δ8 desaturases, Δ5 desaturases, Δ17 desaturases and Δ12 desaturases. Other useful desaturases can include Δ4 desaturases, Δ6 desaturases, Δ15 desaturases and Δ9 desaturases.

The term "elongase" refers to a polypeptide that can elongate a fatty acid carbon chain to produce an acid 2 carbons longer than the fatty acid substrate that the elongase acts upon. This process of elongation occurs in a multi-step mechanism in association with fatty acid synthase, as described in Intl. App. Pub. No. WO 2005/047480. Examples of reactions catalyzed by elongase systems are the conversion of GLA to DGLA, STA to ETA, ARA to DTA and EPA to DPA. In general, the substrate selectivity of elongases is somewhat broad but segregated by both chain length and the degree and type of unsaturation. For example, a C_{14/16} elongase will utilize a C₁₄ substrate (e.g., myristic acid), a C_{16/18} elongase will utilize a C₁₆ substrate (e.g., palmitate), a C_{18/20} elongase will utilize a C₁₈ substrate (e.g., GLA, STA) and a C_{20/22} elongase [also referred to as a Δ5 elongase or C20 elongase] will utilize a C₂₀ substrate (e.g., ARA, EPA). For the purposes herein, two distinct types of C_{18/20} elongases can be defined: a Δ6 elongase will catalyze conversion of GLA and STA to DGLA and ETA, respectively, while a Δ9 elongase is able to catalyze the conversion of LA and ALA to EDA and ETrA, respectively.

The term "multizyme" or "fusion protein" refers to a single polypeptide having at least two independent and separable enzymatic activities, wherein the first enzymatic activity is preferably linked to the second enzymatic activity (U.S. Pat. Appl. Pub. No. 2008-0254191-A1). The "link" or "bond" between the at least two independent and separable enzymatic activities is minimally comprised of a single polypeptide bond, although the link may also be comprised of one amino acid residue, such as proline, or a polypeptide comprising at least one proline amino acid residue. Preferred linkers are selected from the group consisting of: SEQ ID NO:1, SEQ ID NO:2, SEQ ID NO:3, SEQ ID NO:4, SEQ ID NO:5, SEQ ID NO:6 and SEQ ID NO:7.

The term "DGLA synthase" refers to a multizyme, wherein a Δ9 elongase is linked to a Δ8 desaturase. The term "EgD9eS/EgD8M" refers to a DGLA synthase (SEQ ID NOs:8 and 9) created by linking the Δ9 elongase "EgD9eS" (U.S. Patent 7,645,604) to the Δ8 desaturase "EgD8M" (U.S. Patent 7,709,239) with a linker sequence (i.e., SEQ ID NO:1 [GAGPARPAGLPPATYYDSLAVMGS]; U.S. Pat. Appl. Pub. No. 2008-0254191-A1). Similarly, the term "EaD9eS/EaD8S" refers to a DGLA synthase (SEQ ID NOs:10 and 11) created by linking the Δ9 elongase "EaD9eS" (U.S. Pat. Appl. Pub. No. 2008-0254522-A1) to the Δ8 desaturase "EaD8S" (U.S. Pat. Appl. Pub. No. 2008-0254521-A1) with the linker sequence set forth as SEQ ID NO:1. And, the term "E389D9eS/EgD8M" refers to a DGLA synthase (SEQ ID NOs:12 and 13) created by linking the Δ9 elongase "E389D9eS" (U.S. Patent 7,645,604) to the Δ8 desaturase "EgD8M" (*supra*) with the linker sequence set forth as SEQ ID NO:1.

The terms "conversion efficiency" and "percent substrate conversion" refer to the efficiency by which a particular enzyme, such as a desaturase, elongase or multizyme, can convert substrate to product. The conversion efficiency is measured according to the following formula: ([product]/[substrate+product])*100, where 'product' includes the immediate product and all products in the pathway derived from it.

The term "C₁₈ to C₂₀ elongation conversion efficiency" refers to the efficiency by which C_{18//20} elongases can convert C₁₈ substrates (i.e., LA, ALA, GLA, STA) to C₂₀ products (i.e., EDA, ETrA, DGLA, ETA). These C_{18//20} elongases can be either Δ9 elongases or Δ6 elongases.

The term "Δ9 elongation conversion efficiency" refers to the efficiency by which Δ9 elongase can convert C₁₈ substrates (i.e., LA, ALA) to C₂₀ products (i.e., EDA, ETrA).

The term "acyltransferase" refers to an enzyme responsible for transferring an acyl group from a donor lipid to an acceptor lipid molecule.

The term "acyl-CoA:lysophospholipid acyltransferase" ["LPLAT"] refers to a broad class of acyltransferases, having the ability to acylate a variety of lysophospholipid substrates at the sn-2 position. More specifically, LPLATs include LPA acyltransferases ["LPAATs"] having the ability to catalyze conversion of LPA to PA, LPC acyltransferases ["LPCATs"] having the ability to catalyze conversion of LPC to PC, LPE acyltransferases ["LPEATs"] having the ability to catalyze conversion of LPE to PE, LPS acyltransferases ["LPSATs"] having the ability to catalyze conversion of LPS to PS, LPG acyltransferases ["LPGATs"] having the ability to catalyze conversion of LPG to PG, and LPI acyltransferases ["LPIATs"] having the ability to catalyze conversion of LPI to PI. Standardization of LPLAT nomenclature has not been formalized, so various other designations are used in the art (for example, LPAATs have also been referred to as acyl-CoA:1-acyl-*sn*-glycerol-3-phosphate 2-O-acyltransferases, 1-acyl-*sn*-glycerol-3-phosphate acyltransferases and/or 1-acylglycerolphosphate acyltransferases ["AGPATs"] and LPCATs are often referred to as acyl-CoA:1-acyl lysophosphatidyl-choline acyltransferases). Additionally, it is important to note that some LPLATs, such as the *Saccharomyces cerevisiae* Ale1 (ORF YOR175C; SEQ ID NO:15), have broad specificity and thus a single enzyme may be capable of catalyzing several LPLAT reactions, including LPAAT, LPCAT and LPEAT reactions (Tamaki, H. et al., J. Biol. Chem., 282:34288-34298 (2007); Ståhl, U. et al., FEBS Letters, 582:305-309 (2008); Chen, Q. et al., FEBS Letters, 581:5511-5516 (2007); Benghezal, M. et al., J. Biol. Chem., 282:30845-30855 (2007); Riekhof, et al., J. Biol. Chem., 282:28344-28352 (2007)).

More specifically, the term "polypeptide having at least lysophosphtidylcholine acyltransferase ["LPCAT"] activity" will refer to those enzymes capable of catalyzing the reaction: acyl-CoA + 1-acyl-*sn-*glycero-3-phosphocholine → CoA + 1,2-diacyl-*sn*-glycero-3-phosphocholine (EC 2.3.1.23). LPCAT activity has been described in two structurally distinct protein families, i.e., the LPAAT protein family (Hishikawa, et al., Proc. Natl. Acad. Sci. U.S.A., 105:2830-2835 (2008); Intl. App. Pub. No. WO 2004/076617) and the ALE1 protein family (Tamaki, H. et al., *supra;* Ståhl, U. et al., *supra;* Chen, Q. et al., *supra;* Benghezal, M. et al., *supra;* Riekhof, et al., *supra*).

The term "LPCAT" refers to a protein of the ALE1 protein family that: 1) has LPCAT activity (EC 2.3.1.23) and shares at least about 45% amino acid identity, based on the Clustal W method of alignment, when compared to an amino acid sequence selected from the group consisting of SEQ ID NO:15 (ScAle1) and SEQ ID NO:17 (YlAle1); and/or 2) has LPCAT activity (EC 2.3.1.23) and has at least one membrane bound *O-*acyltransferase ["MBOAT"] protein family motif selected from the group consisting of: M(V/I)LxxKL (SEQ ID NO:18), RxKYYxxW (SEQ ID NO:19), SAxWHG (SEQ ID NO:20) and EX₁₁WNX₂-[T/V]-X₂W (SEQ ID NO:21). Examples of ALE1 polypeptides include ScAle1 and YlAle1.

The term "ScAle1" refers to a LPCAT (SEQ ID NO:15) isolated from *Saccharomyces cerevisiae* (ORF "YOR175C"), encoded by the nucleotide sequence set forth as SEQ ID NO:14. In contrast, the term "ScAle1S" refers to a synthetic LPCAT derived from S. *cerevisiae* that is codon-optimized for expression in *Yarrowia lipolytica* (i.e., SEQ ID NOs:22 and 23).

The term "YlAle1" refers to a LPCAT (SEQ ID NO:17) isolated from *Yarrowia lipolytica,* encoded by the nucleotide sequence set forth as SEQ ID NO:16.

The term "LPCAT" also refers to a protein that has LPCAT activity (EC 2.3.1.23) and shares at least about 90% amino acid identity, based on the Clustal W method of alignment, when compared to an amino acid sequence as set forth in SEQ ID NO:25 (CeLPCAT).

The term "CeLPCAT" refers to a LPCAT enzyme (SEQ ID NO:25) isolated from *Caenorhabditis elegans,* encoded by the nucleotide sequence set forth as SEQ ID NO:24. In contrast, the term "CeLPCATS" refers to a synthetic LPCAT derived from C. *elegans* that is codon-optimized for expression in *Yarrowia lipolytica* (i.e., SEQ ID NOs:26 and 27).

The term "polypeptide having at least lysophosphatidic acid acyltransferase ["LPAAT"] activity" will refer to those enzymes capable of catalyzing the reaction: acyl-CoA + 1-acyl-sn-glycerol 3-phosphate → CoA + 1,2-diacyl-sn-glycerol 3-phosphate (EC 2.3.1.51).

The term "LPAAT" refers to a protein that: 1) has LPAAT activity and shares at least about 43.9% amino acid identity, based on the Clustal W method of alignment, when compared to an amino acid sequence selected from the group consisting of SEQ ID NO:29 (MaLPAAT1), SEQ ID NO:31 (YILPAAT1) and SEQ ID NO:32 (ScLPAAT1); and/or, 2) has LPAAT activity and has at least one 1-acyl-sn-glycerol-3-phosphate acyltransferase family motif selected from the group consisting of: NHxxxxD (SEQ ID NO:33) and EGTR (SEQ ID NO:34). Examples of LPAAT polypeptides include ScLPAAT, MaLPAAT1 and YILPAAT1.

The term "ScLPAAT" refers to a LPAAT (SEQ ID NO:32) isolated from *Saccharomyces cerevisiae* (ORF "YDL052C").

The term "MaLPAAT1" refers to a LPAAT (SEQ ID NO:29) isolated from *Mortierella alpina,* encoded by the nucleotide sequence set forth as SEQ ID NO:28. In contrast, the term "MaLPAAT1 S" refers to a synthetic LPAAT derived from *M. alpina* that is codon-optimized for expression in *Yarrowia lipolytica* (i.e., SEQ ID NOs:35 and 36).

The term "YILPAAT1" refers to a LPAAT (SEQ ID NO:31) isolated from *Yarrowia lipolytica,* encoded by the nucleotide sequence set forth as SEQ ID NO:30.

The term "ortholog" refers to a homologous protein from a different species that evolved from a common ancestor protein as evidenced by being in one clade of phylogenetic tree analysis and that catalyzes the same enzymatic reaction.

The term "diacylglycerol cholinephosphotransferase" refers to an enzyme (EC 2.7.8.2) that catalyses the synthesis of phosphatidylcholines from CDP-choline and 1,2-diacylglycerols. This enzyme is part of the CDP-choline pathway, responsible for phosphatidylcholine ["PC"] biosynthesis.

The term "YICPT1" refers to a diacylglycerol cholinephosphotransferase enzyme (SEQ ID NO:38) isolated from *Yarrowia lipolytica,* encoded by SEQ ID NO:37. YICPT1 is described in Intl. App. Pub. No. WO 2006/052870 (see also GenBank Accession No. XM_501703 (YALI0C10989g)).

The term "malonic acid", also referred to as propanedioic acid according to International Union of Pure and Applied Chemistry ["IUPAC"] systematic nomenclature, refers to a dicarboxylic acid having the chemical structure set forth as CH₂(COOH)₂. The malonate or propanedioate ion is derived from malonic acid by loss of two hydrogen ions (i.e., CH₂(COO)₂²⁻). Salts and esters of malonic acid include, but are not limited to, diethyl malonate [(C₂H₅)₂(C₃H₂O₄)], dimethyl malonate [(CH₃)₂(C₃H₂O₄)] and disodium malonate [Na₂(C₃H₂O₄)].

"Malonates" refer to the ionised form of malonic acid, as well as its esters and salts. All of these are referred to herein collectively as "malonates".

"Malonyl-CoA" [CAS Registry No. 524-14-1] refers to an acyl thioester that can be formed by the carboxylation of acetyl-CoA to malonyl-CoA. Alternatively, malonyl-CoA is produced enzymatically from the substrate malonate, via a malonyl-CoA synthetase.

"Malonyl-CoA synthetase" [EC 6.2.1.-] catalyzes the following enzymatic reaction: malonate + ATP + CoA → malonyl-CoA + AMP + pyrophosphate (PPi). The enzyme was first purified from malonate-grown *Pseudomonas fluorescens* (Kim, Y.S. and S.K. Bang, J. Biol. Chem., 260:5098-5104 (1985)), although various *Rhizobia* homologs have since been isolated from bacteroids within legume nodules (see, for example, Kim, Y.S. and H.Z. Chae, Biochem. J., 273:511-516 (1991) and Kim, Y.S. and S.W. Kang, Biochem. J., 297:327-333 (1994)).

As used herein, the term "rMCS" refers to a gene (SEQ ID NO:39) encoding malonyl-CoA synthetase (SEQ ID NO:40) isolated from *Rhizobium leguminosarum* bv. viciae 3841 (GenBank Accession No. YP_766603). Similarly, the term "MCS" refers to a synthetic gene encoding malonyl-CoA synthetase derived from *Rhizobium leguminosarum* bv. viciae 3841 that is codon-optimized for expression in *Yarrowia lipolytica* (i.e., SEQ ID NOs:41 and 42).

The term "peroxisomes" refers to ubiquitous organelles found in all eukaryotic cells. They have a single lipid bilayer membrane that separates their contents from the cytosol and that contains various membrane proteins essential to the functions described below. Peroxisomes selectively import proteins via an "extended shuttle mechanism". More specifically, there are at least 32 known peroxisomal proteins, called peroxins, which participate in the process of importing proteins by means of ATP hydrolysis through the peroxisomal membrane. Once cellular proteins are imported into the peroxisome, they are typically subjected to some means of degradation. For example, peroxisomes contain oxidative enzymes, such as e.g., catalase, D-amino acid oxidase and uric acid oxidase, that enable degradation of substances that are toxic to the cell. Alternatively, peroxisomes breakdown fatty acid molecules to produce free molecules of acetyl-CoA which are exported back to the cytosol, in a process called β-oxidation.

The terms "peroxisome biogenesis factor protein", "peroxin" and "Pex protein" are interchangeable and refer to proteins involved in peroxisome biogenesis and/or that participate in the process of importing cellular proteins by means of ATP hydrolysis through the peroxisomal membrane. The acronym of a gene that encodes any of these proteins is "Pex gene". A system for nomenclature is described by Distel et al., J. Cell Biol., 135:1-3 (1996). At least 32 different Pex genes have been identified so far in various eukaryotic organisms. Many Pex genes have been isolated from the analysis of mutants that demonstrated abnormal peroxisomal functions or structures. Based on a review by Kiel, J. A. K. W., et al. (Traffic, 7:1291-1303 (2006)), wherein *in silico* analysis of the genomic sequences of 17 different fungal species was performed, the following Pex proteins were identified: Pex1p, Pex2p, Pex3p, Pex3Bp, Pex4p, Pex5p, Pex5Bp, Pex5Cp, Pex5/20p, Pex6p, Pex7p, Pex8p, Pex10p, Pex12p, Pex13p, Pex14p, Pex15p, Pex16p, Pex17p, Pex14/17p, Pex18p, Pex19p, Pex20p, Pex21 p, Pex21 Bp, Pex22p, Pex22p-like and Pex26p. Collectively, these proteins will be referred to herein as "Pex proteins", encoded by "Pex genes".

The term "conserved domain" or "motif' means a set of amino acids conserved at specific positions along an aligned sequence of evolutionarily related proteins. While amino acids at other positions can vary between homologous proteins, amino acids that are highly conserved at specific positions indicate amino acids that are essential in the structure, the stability, or the activity of a protein. Because they are identified by their high degree of conservation in aligned sequences of a family of protein homologues, they can be used as identifiers, or "signatures", to determine if a protein with a newly determined sequence belongs to a previously identified protein family.

The term "down-regulated" in or in connection with at least one peroxisome biogenesis factor protein refers to reduction in, or abolishment of, the activity of a native peroxisome biogenesis factor protein, as compared to the activity of the wildtype protein. Down-regulation typically occurs when a native Pex gene has a "disruption", referring to an insertion, deletion, or targeted mutation within a portion of that gene, that results in either a complete gene knockout such that the gene is deleted from the genome and no protein is translated or a translated Pex protein having an insertion, deletion, amino acid substitution or other targeted mutation. The location of the disruption in the protein may be, for example, within the N-terminal portion of the protein or within the C-terminal portion of the protein. The disrupted Pex protein will have impaired activity with respect to the Pex protein that was not disrupted, and can be non-functional. Down-regulation that results in low or lack of expression of the Pex protein, could also result via manipulating the regulatory sequences, transcription and translation factors and/or signal transduction pathways or by use of sense, antisense or RNAi technology, etc.

The term "oleaginous" refers to those organisms that tend to store their energy source in the form of oil (Weete, In: Fungal Lipid Biochemistry, 2nd Ed., Plenum, 1980). The term "oleaginous yeast" refers to those microorganisms classified as yeasts that can make oil. Generally, the cellular oil content of oleaginous microorganisms follows a sigmoid curve, wherein the concentration of lipid increases until it reaches a maximum at the late logarithmic or early stationary growth phase and then gradually decreases during the late stationary and death phases (Yongmanitchai and Ward, Appl. Environ. Microbiol., 57:419-25 (1991)). It is not uncommon for oleaginous microorganisms to accumulate in excess of about 25% of their dry cell weight as oil. Examples of oleaginous yeast include, but are no means limited to, the following genera: *Yarrowia, Candida, Rhodotorula, Rhodosporidium, Cryptococcus, Trichosporon* and *Lipomyces.*

The term "fermentable carbon source" means a carbon source that a microorganism will metabolize to derive energy. Typical carbon sources include, but are not limited to: monosaccharides, disaccharides, oligosaccharides, polysaccharides, alkanes, fatty acids, esters of fatty acids, monoglycerides, diglycerides, triglycerides, carbon dioxide, methanol, formaldehyde, formate and carbon-containing amines.

The terms "polynucleotide", "polynucleotide sequence", "nucleic acid sequence", "nucleic acid fragment" and "isolated nucleic acid fragment" are used interchangeably herein. These terms encompass nucleotide sequences and the like. A polynucleotide may be a polymer of RNA or DNA that is single- or double-stranded, that optionally contains synthetic, non-natural or altered nucleotide bases. A polynucleotide in the form of a polymer of DNA may be comprised of one or more segments of cDNA, genomic DNA, synthetic DNA, or mixtures thereof. Nucleotides (usually found in their 5'-monophosphate form) are referred to by a single letter designation as follows: "A" for adenylate or deoxyadenylate (for RNA or DNA, respectively), "C" for cytidylate or deoxycytidylate, "G" for guanylate or deoxyguanylate, "U" for uridylate, "T" for deoxythymidylate, "R" for purines (A or G), "Y" for pyrimidines (C or T), "K" for G or T, "H" for A or C or T, "I" for inosine, and "N" for any nucleotide.

A "substantial portion" of an amino acid or nucleotide sequence is that portion comprising enough of the amino acid sequence of a polypeptide or the nucleotide sequence of a gene to putatively identify that polypeptide or gene, either by manual evaluation of the sequence by one skilled in the art, or by computer-automated sequence comparison and identification using algorithms such as BLAST (Basic Local Alignment Search Tool; Altschul, S. F., et al., J. Mol. Biol., 215:403-410 (1993)). In general, a sequence of ten or more contiguous amino acids or thirty or more nucleotides is necessary in order to identify putatively a polypeptide or nucleic acid sequence as homologous to a known protein or gene. Moreover, with respect to nucleotide sequences, gene-specific oligonucleotide probes comprising 20-30 contiguous nucleotides may be used in sequence-dependent methods of gene identification (e.g., Southern hybridization) and isolation, such as *in situ* hybridization of bacterial colonies or bacteriophage plaques. In addition, short oligonucleotides of 12-15 bases may be used as amplification primers in PCR in order to obtain a particular nucleic acid fragment comprising the primers. Accordingly, a "substantial portion" of a nucleotide sequence comprises enough of the sequence to specifically identify and/or isolate a nucleic acid fragment comprising the sequence.

The term "complementary" is used to describe the relationship between nucleotide bases that are capable of hybridizing to one another. For example, with respect to DNA, adenosine is complementary to thymine and cytosine is complementary to guanine.

"Codon degeneracy" refers to the nature in the genetic code permitting variation of the nucleotide sequence without effecting the amino acid sequence of an encoded polypeptide. The skilled artisan is well aware of the "codon-bias" exhibited by a specific host cell in usage of nucleotide codons to specify a given amino acid. Therefore, when synthesizing a gene for improved expression in a host cell, it is desirable to design the gene such that its frequency of codon usage approaches the frequency of preferred codon usage of the host cell.

"Synthetic genes" can be assembled from oligonucleotide building blocks that are chemically synthesized using procedures known to those skilled in the art. These oligonucleotide building blocks are annealed and then ligated to form gene segments that are then enzymatically assembled to construct the entire gene. Accordingly, the genes can be tailored for optimal gene expression based on optimization of nucleotide sequence to reflect the codon bias of the host cell. The skilled artisan appreciates the likelihood of successful gene expression if codon usage is biased towards those codons favored by the host. Determination of preferred codons can be based on a survey of genes derived from the host cell, where sequence information is available. For example, the codon usage profile for *Yarrowia lipolytica* is provided in U.S. Patent 7,125,672.

"Gene" refers to a nucleic acid fragment that expresses a specific protein, and which may refer to the coding region alone or may include regulatory sequences preceding (5' non-coding sequences) and following (3' non-coding sequences) the coding sequence. "Native gene" refers to a gene as found in nature with its own regulatory sequences. "Chimeric gene" refers to any gene that is not a native gene, comprising regulatory and coding sequences that are not found together in nature. Accordingly, a chimeric gene may comprise regulatory sequences and coding sequences that are derived from different sources, or regulatory sequences and coding sequences derived from the same source, but arranged in a manner different than that found in nature. "Endogenous gene" refers to a native gene in its natural location in the genome of an organism. A "foreign" gene refers to a gene that is introduced into the host organism by gene transfer. Foreign genes can comprise native genes inserted into a non-native organism, native genes introduced into a new location within the native host, or chimeric genes. A "transgene" is a gene that has been introduced into the genome by a transformation procedure. A "codon-optimized gene" is a gene having its frequency of codon usage designed to mimic the frequency of preferred codon usage of the host cell.

"Coding sequence" refers to a DNA sequence that codes for a specific amino acid sequence. "Suitable regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include promoters, enhancers, silencers, 5' untranslated leader sequence (e.g., between the transcription start site and the translation initiation codon), introns, polyadenylation recognition sequences, RNA processing sites, effector binding sites and stem-loop structures.

"Promoter" refers to a DNA sequence capable of controlling the expression of a coding sequence or functional RNA. In general, a coding sequence is located 3' to a promoter sequence. Promoters may be derived in their entirety from a native gene, or be composed of different elements derived from different promoters found in nature, or even comprise synthetic DNA segments. It is understood by those skilled in the art that different promoters may direct the expression of a gene in different tissues or cell types, or at different stages of development, or in response to different environmental or physiological conditions. Promoters that cause a gene to be expressed in most cell types at most times are commonly referred to as "constitutive promoters". It is further recognized that since in most cases the exact boundaries of regulatory sequences have not been completely defined, DNA fragments of different lengths may have identical promoter activity.

The terms "3' non-coding sequence" and "transcription terminator" refer to DNA sequences located downstream of a coding sequence. This includes polyadenylation recognition sequences and other sequences encoding regulatory signals capable of affecting mRNA processing or gene expression. The polyadenylation signal is usually characterized by affecting the addition of polyadenylic acid tracts to the 3' end of the mRNA precursor. The 3' region can influence the transcription, RNA processing or stability, or translation of the associated coding sequence.

The term "operably linked" refers to the association of nucleic acid sequences on a single nucleic acid fragment so that the function of one is affected by the other. For example, a promoter is operably linked with a coding sequence when it is capable of affecting the expression of that coding sequence. That is, the coding sequence is under the transcriptional control of the promoter. Coding sequences can be operably linked to regulatory sequences in sense or antisense orientation.

The term "expression", as used herein, refers to the transcription and stable accumulation of sense (mRNA) or antisense RNA. Expression may also refer to translation of mRNA into a polypeptide.

"Transformation" refers to the transfer of a nucleic acid molecule into a host organism. The nucleic acid molecule may be a plasmid that replicates autonomously, for example, or, it may integrate into the genome of the host organism. Host organisms containing the transformed nucleic acid fragments are referred to as "transgenic" or "recombinant" or "transformed" organisms or "transformant".

"Stable transformation" refers to the transfer of a nucleic acid fragment into the genome of a host organism, including both nuclear and organellar genomes, resulting in genetically stable inheritance (i.e., the nucleic acid fragment is "stably integrated"). In contrast, "transient transformation" refers to the transfer of a nucleic acid fragment into the nucleus, or DNA-containing organelle, of a host organism resulting in gene expression without integration or stable inheritance.

The terms "plasmid" and "vector" refer to an extra chromosomal element often carrying genes that are not part of the central metabolism of the cell, and usually in the form of circular double-stranded DNA fragments. Such elements may be autonomously replicating sequences, genome integrating sequences, phage or nucleotide sequences, linear or circular, of a single- or double-stranded DNA or RNA, derived from any source, in which a number of nucleotide sequences have been joined or recombined into a unique construction that is capable of introducing an expression cassette(s) into a cell.

The term "expression cassette" refers to a fragment of DNA comprising the coding sequence of a selected gene and regulatory sequences preceding (5' non-coding sequences) and following (3' non-coding sequences) the coding sequence that are required for expression of the selected gene product. Thus, an expression cassette is typically composed of: 1) a promoter sequence; 2) a coding sequence (i.e., ORF) and, 3) a 3' untranslated region (i.e., a terminator) that, in eukaryotes, usually contains a polyadenylation site. The expression cassette(s) is usually included within a vector, to facilitate cloning and transformation. Different expression cassettes can be transformed into different organisms including bacteria, yeast, plants and mammalian cells, as long as the correct regulatory sequences are used for each host.

The term "sequence analysis software" refers to any computer algorithm or software program that is useful for the analysis of nucleotide or amino acid sequences. "Sequence analysis software" may be commercially available or independently developed. Typical sequence analysis software will include, but is not limited to: 1) the GCG suite of programs (Wisconsin Package Version 9.0, Genetics Computer Group (GCG), Madison, WI); 2) BLASTP, BLASTN, BLASTX (Altschul et al., J. Mol. Biol., 215:403-410 (1990)); 3) DNASTAR (DNASTAR, Inc. Madison, WI); 4) Sequencher (Gene Codes Corporation, Ann Arbor, MI); and, 5) the FASTA program incorporating the Smith-Waterman algorithm (W. R. Pearson, Comput. Methods Genome Res., [Proc. Int. Symp.] (1994), Meeting Date 1992, 111-20. Editor(s): Suhai, Sandor. Plenum: New York, NY). Within the context of this application it will be understood that where sequence analysis software is used for analysis, that the results of the analysis will be based on the "default values" of the program referenced, unless otherwise specified. As used herein "default values" will mean any set of values or parameters that originally load with the software when first initialized.

Standard recombinant DNA and molecular cloning techniques used herein are well known in the art and are described by Sambrook, J., Fritsch, E. F. and Maniatis, T., Molecular Cloning: A Laboratory Manual, 2nd ed., Cold Spring Harbor Laboratory: Cold Spring Harbor, NY (1989) (hereinafter "Maniatis"); by Silhavy, T. J., Bennan, M. L. and Enquist, L. W., Experiments with Gene Fusions, Cold Spring Harbor Laboratory: Cold Spring Harbor, NY (1984); and by Ausubel, F. M. et al., Current Protocols in Molecular Biology, published by Greene Publishing Assoc. and Wiley-Interscience, Hoboken, NJ (1987).

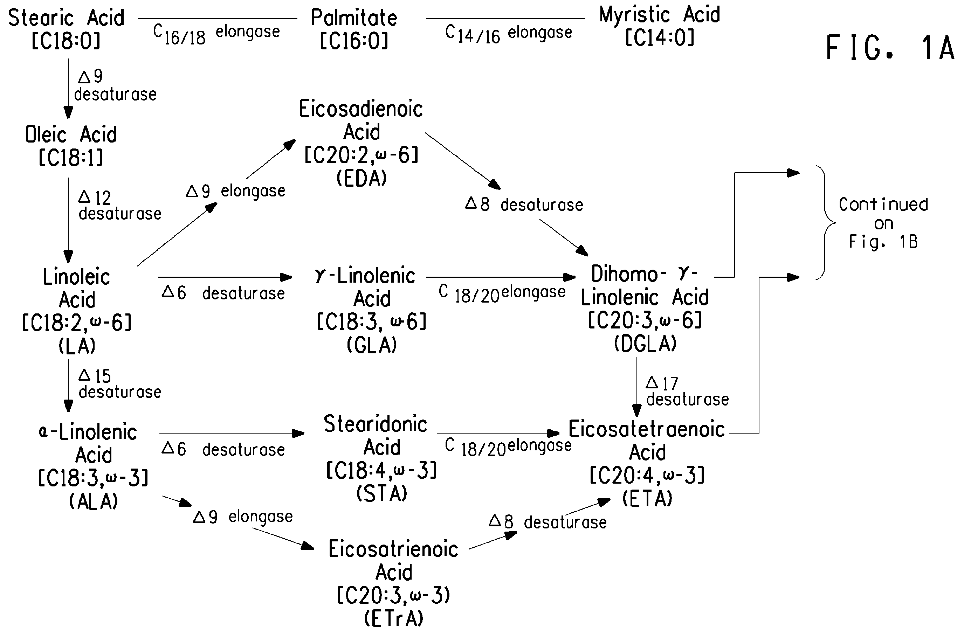

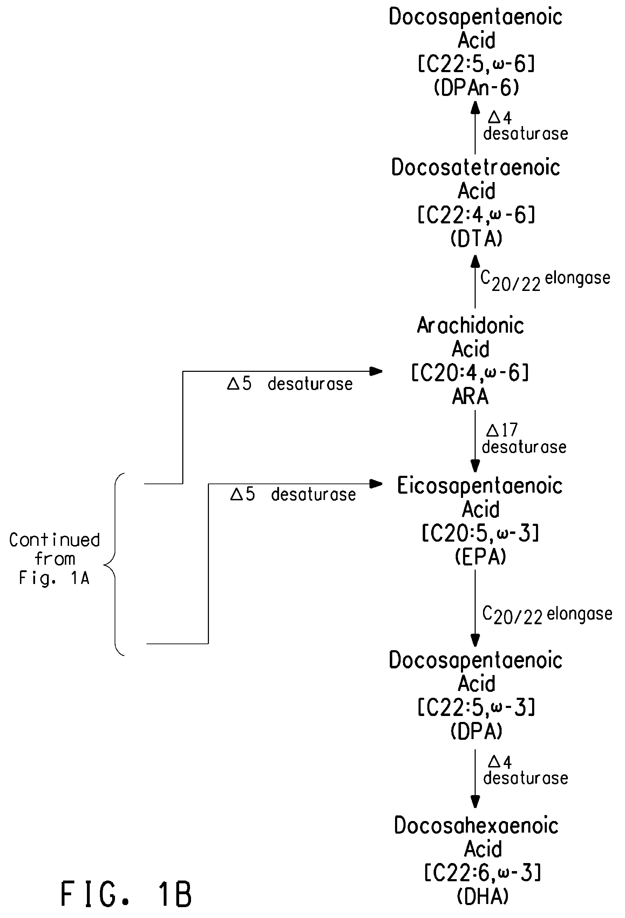

In general, lipid accumulation in oleaginous microorganisms is triggered in response to the overall carbon to nitrogen ratio present in the growth medium. This process, leading to the *de novo* synthesis of free palmitate (16:0) in oleaginous microorganisms, is described in detail in U.S. Patent 7,238,482. Palmitate is the precursor of longer-chain saturated and unsaturated fatty acid derivates, which are formed through the action of elongases and desaturases (FIG. 1).

A wide spectrum of fatty acids (including saturated and unsaturated fatty acids and short-chain and long-chain fatty acids) can be incorporated into TAGs, the primary storage unit for fatty acids. In the methods and host cells described herein, incorporation of EPA into TAGs is most desirable, although the structural form of the EPA is not limiting (thus, for example, the EPA may exist in the total lipids as free fatty acids or in esterified forms such as acylglycerols, phospholipids, sulfolipids or glycolipids).

Although most PUFAs are incorporated into TAGs as neutral lipids and are stored in lipid bodies, it is important to note that a measurement of the total PUFAs within an oleaginous organism should minimally include those PUFAs that are located in the PC, PE and TAG fractions.

The metabolic process wherein oleic acid is converted to EPA involves elongation of the carbon chain through the addition of carbon atoms and desaturation of the molecule through the addition of double bonds. This requires a series of special desaturation and elongation enzymes present in the endoplasmic reticulum membrane. However, as seen in FIG. 1 and as described below, multiple alternate pathways exist for EPA production.

Specifically, FIG. 1 depicts the pathways described below. All pathways require the initial conversion of oleic acid to linoleic acid ["LA"], the first of the ω-6 fatty acids, by a Δ12 desaturase. Then, using the "Δ9 elongase/ Δ8 desaturase pathway" and LA as substrate, long-chain ω-6 fatty acids are formed as follows: 1) LA is converted to eicosadienoic acid ["EDA"] by a Δ9 elongase; 2) EDA is converted to dihomo-γ-linolenic acid ["DGLA"] by a Δ8 desaturase; 3) DGLA is converted to arachidonic acid ["ARA"] by a Δ5 desaturase; 4) ARA is converted to docosatetraenoic acid ["DTA"] by a C_{20/22} elongase; and, 5) DTA is converted to docosapentaenoic acid ["DPAn-6"] by a Δ4 desaturase.

The "Δ9 elongase/ Δ8 desaturase pathway" can also use α-linolenic acid ["ALA"] as substrate to produce long-chain ω-3 fatty acids as follows: 1) LA is converted to ALA, the first of the ω-3 fatty acids, by a Δ15 desaturase; 2) ALA is converted to eicosatrienoic acid ["ETrA"] by a Δ9 elongase; 3) ETrA is converted to eicosatetraenoic acid ["ETA"] by a Δ8 desaturase; 4) ETA is converted to eicosapentaenoic acid ["EPA"] by a Δ5 desaturase; 5) EPA is converted to docosapentaenoic acid ["DPA"] by a C_{20/22} elongase; and, 6) DPA is converted to docosahexaenoic acid ["DHA"] by a Δ4 desaturase. Optionally, ω-6 fatty acids may be converted to ω-3 fatty acids. For example, ETA and EPA are produced from DGLA and ARA, respectively, by Δ17 desaturase activity. Advantageously for the purposes herein, the Δ9 elongase/ Δ8 desaturase pathway enables production of an EPA oil that lacks significant amounts of γ-linolenic acid ["GLA"].

Alternate pathways for the biosynthesis of ω-3/ω-6 fatty acids utilize a Δ6 desaturase and C_{18/20} elongase, that is, the "Δ6 desaturase/ Δ6 elongase pathway". More specifically, LA and ALA may be converted to GLA and stearidonic acid ["STA"], respectively, by a Δ6 desaturase; then, a C_{18/20} elongase converts GLA to DGLA and/or STA to ETA.

Economical commercial production of EPA in a recombinant *Yarrowia* sp. host cell requires consideration of a variety of variables, including the EPA concentration ["EPA % TFAs"] and total lipid content ["TFAs % DCW"]. Furthermore, it is desirable to reduce the production of intermediate fatty acids and byproduct fatty acids in the final oil product, in order to maximize production of the desired fatty acid, i.e., EPA.

Intermediate fatty acids are those fatty acids (e.g., oleic acid, LA, ALA, EDA, DGLA, ETA) that can be further converted to EPA by the action of other metabolic pathway enzymes. In contrast, by-product fatty acids (e.g., sciadonic acid, juniperonic acid) refer to any fatty acid produced that is neither EPA nor an intermediate fatty acid that can be further converted to EPA.

U.S. Pat. Appl. Pub. No. 2009-0093543-A1 describes optimized strains of recombinant *Yarrowia lipolytica* having the ability to produce microbial oils comprising at least about 43.3 EPA % TFAs, with less than about 23.6 LA % TFAs (an EPA:LA ratio of 1.83). The preferred strain was Y4305, whose maximum production was 55.6 EPA % TFAs, with an EPA:LA ratio of 3.03. Generally, the EPA strains of U.S. Pat. Appl. Pub. No. 2009-0093543-A1 comprised the following genes of the ω-3/ω-6 fatty acid biosynthetic pathway:
a) at least one gene encoding Δ9 elongase; and,
b) at least one gene encoding Δ8 desaturase; and,
c) at least one gene encoding Δ5 desaturase; and,
d) at least one gene encoding Δ17 desaturase; and,
e) at least one gene encoding Δ12 desaturase; and,
f) at least one gene encoding C_{16/18} elongase; and,
g) optionally, at least one gene encoding diacylglycerol cholinephosphotransferase (CPT1).

Examples of preferred genes having the enzymatic functionalities described above are set forth in Table 3 (although these genes are not intended to be limiting).

**Table 3: Preferred Desaturases And Elongates For EPA Biosynthesis In Yarrowia lipolytica**

| ORF | Organism | Co-pending Patent Application References | Wildtype Abbreviation and SEQ ID NO | Codon-Optimized Abbreviation and SEQ ID NO | Mutant Abbreviation and SEQ ID NO |
|---|---|---|---|---|---|
| Δ9 elongase | *Euglena gracillis* | U.S. Patent 7,645,604 | "EgD9e" (SEQ ID NOs:43 and 44) | "EgD9eS" (SEQ ID NOs:45 and 46) | -- |
| | *Eutreptiella sp.* CCMP389 | U.S. Patent 7,645,604 | "E389D9e" (SEQ ID NOs:47 and 48) | "E389D9eS" (SEQ ID NOs:49 and 50) | -- |
| | *Euglena anabaena* UTEX 373 | U.S. Pat. Appl. Pub. No. 2008-0254522-A1; Intl. App. Pub. No. WO 2008/128241 | "EaD9e" * (SEQ ID NOs:51 and 52) | "EaD9eS" (SEQ ID NOs:53 and 54) | -- |
| Δ8 desaturase | *Euglena gracilis* | U.S. Patent 7,256,033; U.S. Patent 7,709,239 | "EgD8" * (SEQ ID NOs:55 and 56) | "EgD8S" * (SEQ ID NOs:57 and 58) | "EgD8M" * (SEQ ID NOs:59 and 60) |
| | *Euglena anabaena* UTEX 373 | U.S. Pat. Appl. Pub. No. 2008-0254521-A1; Intl. App. Pub. No. WO 2008/124194 | "EaD8" * (SEQ ID NOs:61 and 62) | "EaD8S" (SEQ ID NOs:63 and 64) | -- |
| Δ5 desaturase | *Euglena gracilis* | U.S. Patent 7,678,560; U.S. Pat. Pub. No. 2010-0075386-A1 | "EgD5" (SEQ ID NOs:65 and 66) | "EgD5S" (SEQ ID NOs:67 and 68) | "EgD5M" (SEQ ID NOs:69 and 70); "EgD5SM" (SEQ ID NOs:71 and 72) |
| | *Peridinium sp.* CCMP626 | U.S. Patent 7,695,950; U.S. Pat. Pub. No. 2010-0075386-A1 | "RD5" (SEQ ID NOs:73 and 74) | "RD5S" (SEQ ID NOs:75 and 76) | -- |
| | *Euglena anabaena* UTEX 373 | U.S. Pat. Appl. Pub. No. 2008-0274521-A1; U.S. Pat. Pub. No. 2010-0075386-A1 | "EaD5" * (SEQ ID NOs:77 and 78) | "EaD5S" * (SEQ ID NOs:79 and 80) | "EaD5SM" (SEQ ID NOs:81 and 82) |
| Δ17 desaturase | *Phytophthora ramorum* | U.S. Patent 7,465,793 | "PrD17" (SEQ ID NOs:83 and 84) | "PrD17S" (SEQ ID NOs:85 and 86) | -- |
| | *Pythium aphanidernatum* | U.S. Patent 7,556,949 | "PaD17" (SEQ ID NOs:87 and 88) | "PaD17S" (SEQ ID NOs:89 and 90) | -- |
| Δ12 desaturase | *Fusarium moniliforme* | U.S. Patent 7,504,259 | "FmD12" * (SEQ ID NOs:91 and 92) | "FmD12S" (SEQ ID NOs:93 and 94) | -- |
| C_{16/18} elongase | *Mortierella alpina* | U.S. Patent 7,470,532 | "ELO3" (SEQ ID NOs:95 and 96) | "ME3S" (SEQ ID NOs:97 and 98) | -- |
| Diacyl-glycerol choline-phosphotransferase | *Yarrowia lipolytica* | Intl. App. Pub. No. WO 2006/052870 | "YICPT" (SEQ ID NOs:37 and 38) | -- | -- |

| | | | | | |
|---|---|---|---|---|---|
| * Notes: EaD9e was identified as "EaD9Elo1" in U.S. Pat. Appl. Pub. No. 2008-0254522-A1; EgD8 was identified as "Eg5" in U.S. Patent 7,256,033; EgD8S was identified as "D8SF" in U.S. Patent 7,256,033; EgD8M was identified as "EgD8S-23" in U.S. Patent 7,709,239; EaD8 was identified as "EaD8Des3" in U.S. Pat. Appl. Pub. No. 2008-0254521-A1; EaD5 was identified as "EaD5Des1" in U.S. Pat. Appl. Pub. No. 2008-0274521-A1; and, FmD12 was identified as "Fm2" in U.S. Patent 7,504,259. | | | | | |

Provided herein are optimized strains of recombinant *Yarrowia lipolytica* having the ability to produce improved microbial oils relative to those strains described in U.S. Pat. Appl. Pub. No. 2009-0093543-A1, based on the EPA % TFAs and the ratio of EPA:LA. In addition to expressing genes of the ω-3/ω-6 fatty acid biosynthetic pathway as defined above and as detailed in U.S. Pat. Appl. Pub. No. 2009-0093543-A1, these improved strains are distinguished by:
1) comprising at least one multizyme, wherein said multizyme comprises a polypeptide having at least one fatty acid Δ9 elongase linked to at least one fatty acid Δ8 desaturase [a "DGLA synthase"];
2) comprising at least one polynucleotide encoding an enzyme selected from the group consisting of a malonyl CoA synthetase and an acyl-CoA lysophospholipid acyltransferase ["LPLAT"];
3) comprising at least one peroxisome biogenesis factor protein whose expression has been down-regulated;
4) producing at least about 50 EPA % TFAs; and,
5) having a ratio of EPA:LA of at least about 3.5.

U.S. Pat. Appl. Pub. No. 2008-0254191-A1, and especially Examples 55 and 56, describes DGLA synthases that possess improved enzymatic activity with respect to their individual Δ9 elongase and/or Δ8 desaturase counterparts, when heterologously expressed in *Yarrowia lipolytica.* Particularly relevant to the disclosure herein, a linker sequence (i.e., SEQ ID NO:1 [GAGPARPAGLPPATYYDSLAVMGS]) was used to fuse a Δ9 elongase (i.e., EgD9eS, EaD9eS or E389D9eS) to a Δ8 desaturase (i.e., EgD8M or EaD8S), thereby creating EgD9eS/EgD8M (SEQ ID NOs:8 and 9), EaD9eS/EaD8S (SEQ ID NOs:10 and 11) and E389D9eS/EgD8M (SEQ ID NOs:12 and 13). Surprisingly, fusing the two independent enzymes together as one fusion protein separated by a linker region increased flux from LA to DGLA, suggesting that the product of Δ9 elongase may be directly channeled as substrate of Δ8 desaturase in the fusion protein.

Table 4 below provides a summary of the improvements noted in conversion efficiency in U.S. Pat. Appl. Pub. No. 2008-0254191-A1, as a result of the gene fusion. Specifically, the number shown in bold text is the percent improvement in elongase or desaturase activity, while the details shown in parentheses provide the elongase or desaturase conversion efficiency in the gene fusion versus when the elongase or desaturase conversion efficiency when the gene was expressed alone.

**Table 4: Improvement In Δ9 Elongase And Δ8 Desaturase Conversion As A Result Of Gene Fusion**

| Gene fusion | Δ9 Improvement | Δ8 Improvement |
|---|---|---|
| EgD9eS/EgD8M | **5%** | **97%** |
| (SEQ ID NOs:8 and 9) | (21% versus 20% conversion) | (73% versus 37% conversion) |
| EaD9eS/EaD8S | **38%** | **32%** |
| (SEQ ID NOs:10 and 11) | (18% versus 13% conversion) | (58% versus 41% conversion) |
| E389D9eS/EgD8M | **50%** | **89%** |
| (SEQ ID NOs:12 and 13) | (18% versus 12% conversion) | (70% versus 37% conversion) |

Based on the results described above, expression of at least one DGLA synthase, such as the EgD9eS/EgD8M, EaD9eS/EaD8S and E389D9eS/EgD8M gene fusions described above, is preferred in improved optimized strains of recombinant *Yarrowia lipolytica* having the ability to produce improved EPA % TFAs. This gene fusion can be created using any combination of preferred Δ9 elongases and Δ8 desaturases suitable for expression in *Y. lipolytica;* and, the linker can be selected from the group consisting of: SEQ ID NO:1, SEQ ID NO:2, SEQ ID NO:3, SEQ ID NO:4, SEQ ID NO:5, SEQ ID NO:6 and SEQ ID NO:7.

Previous studies have determined that many of the genetic mutations relating to engineering production of PUFAs in *Yarrowia lipolytica* result in increased by production of malonates during the fermentation (malonates accounted for ∼45% of the total organic acids accumulated). Expression of a heterologous malonyl-CoA synthetase reversed this effect and resulted in substantially reduced by production of malonates.

More specifically, U.S. Patent Appl. Pub. No. US 2010-0159558 A1 describes generalized methods to avoid accumulation of organic acid (and in particular, malonate) "byproducts" that cannot be further utilized during a fermentation, during production of a product. This avoids carbon and energy waste within the organism, reduces the amount of base required to maintain an optimal pH range during the fermentation process, and reduces the amount of byproduct organic acids that require neutralization within the fermentation waste steam.

Malonyl-CoA synthetase [EC 6.2.1.-] catalyzes the following enzymatic reaction: malonate + ATP + CoA → malonyl-CoA + AMP + pyrophosphate (PPi). By converting the byproduct (i.e., malonate) into malonyl-CoA, this substrate becomes available for use during the synthesis of fatty acids within the organism. Specifically, fatty acid synthesis can be summarized by the following equation (ignoring H⁺ and water): acetyl-CoA + 7 malonyl-CoA + 14 NADPH → palmitate + 7 CO₂ + 14 NADP⁺ + 8 CoA.

A codon-optimized malonyl-CoA synthetase was created and expressed in *Yarrowia lipolytica* in U.S. Patent Application No. 12/637877. Specifically, the codon-optimized malonyl-CoA synthetase gene ("MCS", SEQ ID NO:41) was designed based on the coding sequence of the malonyl-CoA synthetase gene from *Rhizobium leguminosarum* bv. viciae 3841 (rMCS; SEQ ID NOs:39 and 40, corresponding to GenBank Accession No. YP_766603). In addition to modification of the translation initiation site, 233 bp of the 1515 bp coding region (including the stop codon) were modified (15.4%), 219 codons were optimized (43.4%), the GC content was reduced from 61.4% within the wild type gene (i.e., rMCS) to 55.6% within the synthetic gene (i.e., MCS) and the translation initiation codon "ATG" was added in front of the rMCS gene (SEQ ID NO:39) since *Yarrowia* cannot use the "GTG" codon for translation initiation. The codon-optimized MCS gene (SEQ ID NO:41) is 1518 bp encoding a peptide of 505 amino acids and a stop codon (SEQ ID NO:42).

Expression of MCS (SEQ ID NO:42) in *Yarrowia lipolytica* strain Y4305U, producing 49 EPA % TFAs, lowered the total amount of malonates (g/g DCW) ∼94% without impacting either the fatty acid profile or the total lipid yield (TFAs % DCW).

Based on the results described above, expression of at least one malonyl-CoA synthetase in improved optimized strains of recombinant *Yarrowia lipolytica* is desirable, as a means to reduce generation of unwanted byproducts and thereby decrease the cost of manufacture. Preferred malonyl-CoA synthetases are set forth as SEQ ID NOs:40 and 42, but these are should not be limiting to the disclosure herein. One skilled in the art could readily identify alternate heterologous malonyl-CoA synthetases suitable for expression in *Y. lipolytica.*

Glycerophospholipids, the main component of biological membranes, contain a glycerol core with fatty acids attached as R groups at the sn-1 position and *sn*-2 position, and a polar head group joined at the sn-3 position via a phosphodiester bond. Table 5 below summarizes the steps in the *de novo* biosynthetic pathway, originally described by Kennedy and Weiss (J. Biol. Chem., 222:193-214 (1956)):

**Table 5: General Reactions Of de Novo Glycerophospholipid Biosynthesis**

| | |
|---|---|
| *sn*-Glycerol-3-Phosphate → Lysophosphatidic Acid (1-acyl-*sn*-glycerol 3-phosphate or "LPA") | Glycerol-3-phosphate acyltransferase (GPAT) [E.C. 2.3.1.15] esterifies 1 st acyl-CoA to *sn-*1 position of *sn*-glycerol 3-phosphate |
| LPA → Phosphatidic Acid (1,2-diacylglycerol phosphate or "PA") | Lysophosphatidic acid acyltransferase (LPAAT) [E.C. 2.3.1.51] esterifies 2^{nd} acyl-CoA to *sn-2* position of LPA |
| PA → 1,2-Diacylglycerol ("DAG") | Phosphatidic acid phosphatase [E.C. 3.1.3.4] removes a phosphate from PA; DAG can subsequently be converted to phosphatidylcholines ["PC"], phosphatidylethanolamines ["PE"] or TAG (TAG synthesis requires either a diacylglycerol acyltransferase (DGAT) [E.C. 2.3.1.20] or a phospholipid:diacylglycerol acyltransferase (PDAT) [E.C.2.3.1.158]) |
| **Or** | |
| PA → Cytidine Diphosphate Diacylglycerol ("CDP-DG") | |
| | CDP-diacylglycerol synthase [EC 2.7.7.41] causes condensation of PA and cytidine triphosphate, with elimination of pyrophosphate; CDP-DG can subsequently be converted to phosphatidylglycerols ["PG"], phosphatidylinositols ["PI"], phosphatidylserines ["PS"] or cardiolipins ["CL"] |

Following their *de novo* synthesis, glycerophospholipids can undergo rapid turnover of their fatty acyl composition at the sn-2 position. This "remodeling", or "acyl editing", has been attributed to deacylation of the glycerophospholipid and subsequent reacylation of the resulting lysophospholipid.

In the Lands' cycle (Lands, W.E., J. Biol. Chem., 231:883-888 (1958)), remodeling occurs through the concerted action of: 1) a phospholipase, such as phospholipase A₂, that releases fatty acids from the sn-2 position of phosphatidylcholine; and, 2) acyl-CoA:lysophospholipid acyltransferases ["LPLATs"], such as lysophosphatidylcholine acyltransferase ["LPCAT"] that reacylates the lysophosphatidylcholine ["LPC"] at the sn-2 position. Other glycerophospholipids can also be involved in the remodeling with their respective lysophospholipid acyltransferase activity, including LPLAT enzymes having lysophosphatidylethanolamine acyltransferase ["LPEAT"] activity, lysophosphatidylserine acyltransferase ["LPSAT"] activity, lysophosphatidylglycerol acyltransferase ["LPGAT"] activity and lysophosphatidylinositol acyltransferase ["LPIAT"] activity. In all cases, LPLATs are responsible for removing acyl-CoA fatty acids from the cellular acyl-CoA pool and acylating various lysophospholipid substrates at the *sn-*2 position in the phospholipid pool. Finally, LPLATs also include LPAAT enzymes that are involved in the *de novo* biosynthesis of PA from LPA.

In other cases, this *sn-*2 position remodeling has been attributed to the forward and reverse reactions of enzymes having LPCAT activity (Stymne S. and A.K. Stobart, Biochem J., 223(2):305-314 (1984)).

Several recent reviews by Shindou et al. provide an overview of glycerophospholipid biosynthesis and the role of LPLATs (J. Biol. Chem., 284(1):1-5 (2009); J. Lipid Res., 50:S46-S51 (2009)). And, numerous LPLATs have been reported in public and patent literature, based on the presence of conserved motifs.

More specifically, a variety of LPLAT motifs have been proposed, with slight variation based on the specific species that are included in analyzed alignments. For example, Shindou et al. (Biochem. Biophys. Res. Comm., 383:320-325 (2009)) proposed the following membrane bound *O*-acyltransferase ["MBOAT"] family motifs to be important for LPLAT activity, based on alignment of sequences from *Homo sapiens, Gallus gallus, Danio rerio* and *Caenorhabditis elegans:* WD, WHGxxxGYxxxF (SEQ ID NO:99), YxxxxF (SEQ ID NO:100) and YxxxYFxxH (SEQ ID NO:101). Of these, the WD, WHGxxxGYxxxF and YxxxxF motifs are present in ScAle (SEQ ID NO:15) and YIAle1 (SEQ ID NO:17), but the YxxxYFxxH motif is not. Alternate non-plant motifs for Ale1 homologs are also described in U.S. Pat. Appl. Pub. No. 2008-0145867-A1; specifically, these include: M-[V/I]-[L/I]-xxK-[L/V/I]-xxxxxxDG (SEQ ID NO:102), RxKYYxxWxxx-[E/D]-[A/G]xxxxGxG-[F/Y]-xG (SEQ ID NO:103), EX₁₁WNX₂-[T/V]-X₂W (SEQ ID NO:21) and SAxWHGxxPGYxx-[T/F]-F (SEQ ID NO:104).

Similarly, Lewin, T.W. et al. (Biochemistry, 38:5764-5771 (1999) and Yamashita et al. (Biochim. Biophys. Acta, 1771:1202-1215 (2007)) proposed the following 1-acyl-*sn*-glycerol-3-phosphate acyltransferase ["LPAAT"] family motifs to be important for LPLAT activity, based on alignment of sequences from bacteria, yeast, nematodes and mammals: NHxxxxD (SEQ ID NO:33), GxxFI-[D/R]-R (SEQ ID NO:105), EGTR (SEQ ID NO:34) and either [V/I]-[P/X]-[I/V/L]-[I/V]-P-[V/I] (SEQ ID NO:106) or IVPIVM (SEQ ID NO:107). The NHxxxxD and EGTR motifs are present in MaLPAAT1 (SEQ ID NO:29), YILPAAT1 (SEQ ID NO:31) and CeLPCAT (SEQ ID NO:25), but the other LPAAT family motifs are not.

Based on publicly available Ale1, LPCAT and LPAAT protein sequences, including those described herein, LPLATs for inclusion in the improved optimized strains of recombinant *Yarrowia lipolytica* herein possess either MBOAT family motifs selected from the group consisting of: M(V/I)LxxKL (SEQ ID NO:18), RxKYYxxW (SEQ ID NO:19), SAxWHG (SEQ ID NO:20) and EX₁₁WNX₂-[T/V]-X₂W (SEQ ID NO:21) or 1-acyl-*sn-*glycerol-3-phosphate acyltransferase family motifs selected from the group consisting of: NHxxxxD (SEQ ID NO:33) and EGTR (SEQ ID NO:34).

The effect of LPLATs on PUFA production has been contemplated, since fatty acid biosynthesis requires rapid exchange of acyl groups between the acyl-CoA pool and the phospholipid pool. Specifically, desaturations occur mainly at the sn-2 position of phospholipids, while elongation occurs in the acyl-CoA pool.

More specifically, it has been previously hypothesized that LPCATs could be important in the accumulation of EPA in the TAG fraction of *Yarrowia lipolytica* (U.S. Pat. Appl. Pub. No. 2006-0115881-A1). As described therein, this hypothesis was based on the following studies: 1) Stymne S. and A.K. Stobart (Biochem. J., 223(2):305-314(1984)), who hypothesized that the exchange between the acyl-CoA pool and PC pool may be attributed to the forward and backward reaction of LPCAT; 2) Domergue, F. et al. (J. Bio. Chem., 278:35115 (2003)), who suggested that accumulation of GLA at the *sn-*2 position of PC and the inability to efficiently synthesize ARA in yeast was a result of the elongation step involved in PUFA biosynthesis occurring within the acyl-CoA pool, while the Δ5 and Δ6 desaturation steps occurred predominantly at the *sn-2* position of PC; 3) Abbadi, A. et al. (The Plant Cell, 16:2734-2748 (2004)), who suggested that LPCAT plays a critical role in the successful reconstitution of a Δ6 desaturase/Δ6 elongase pathway, based on analysis on the constraints of PUFA accumulation in transgenic oilseed plants; and, 4) the work of Renz, A. et al. in Intl. App. Publications No. WO 2004/076617 A2 and No. WO 2004/087902 A2.

More specifically, Intl. App. Pub. No. WO 2004/076617 A2 describes the isolation of a LPCAT from *Caenorhabditis elegans* (clone T06E8.1) ["CeLPCAT"] and reports increase in the efficiency of Δ6 desaturation and Δ6 elongation, as well as an increase in biosynthesis of the long-chain PUFAs eicosadienoic acid ["EDA"; 20:2] and eicosatetraenoic acid ["ETA"; 20:4], respectively, when the LPCAT was expressed in an engineered strain of *Saccharomyces cerevisiae* that was fed exogenous 18:2 or α-linolenic ["ALA"; 18:3] fatty acids, respectively.

Intl. App. Pub. No. WO 2004/087902 A2 (Example 16) describes the isolation of *Mortierella alpina* LPAAT-like proteins (encoded by proteins having 417 amino acids in length or 389 amino acids in length, respectively, that are identical except for an N-terminal extension of 28 amino acid residues) and reports expression of one of these proteins using similar methods to those of Intl. App. Pub. No. WO 2004/076617 A2, which results in similar improvements in EDA and ETA biosynthesis.

Both Intl. App. Publications No. WO 2004/076617 and No. WO 2004/087902 teach that the improvement in EDA and ETA biosynthesis is due to reversible LPCAT activity in CeLPLAT and some LPAAT-like proteins, although not all LPAAT-like proteins have LPCAT activity. Furthermore, Renz, A. et al. concluded that LPCAT allowed efficient and continuous exchange of the newly synthesized fatty acids between phospholipids and the acyl-CoA pool, since desaturases catalyze the introduction of double bonds in PC-coupled fatty acids while elongases exclusively catalyze the elongation of CoA esterified fatty acids (acyl-CoAs).

Numerous other references generally describe benefits of co-expressing LPLATs with PUFA biosynthetic genes, to increase the amount of a desired fatty acid in the oil of a transgenic organism, increase total oil content or selectively increase the content of desired fatty acids (e.g., Intl. App. Publications No. WO 2006/069936, No. WO 2006/052870, No. WO 2009/001315, No. WO 2009/014140).

Herein, it is demonstrated that LPAAT and LPCAT are indeed important in the accumulation of EPA in the TAG fraction of *Yarrowia lipolytica.* Specifically, it was found that over-expression of LPLATs can result in an improvement in the Δ9 elongase conversion efficiency. As previously defined, conversion efficiency is a term that refers to the efficiency by which a particular enzyme, such as a Δ9 elongase, can convert substrate (e.g., LA) to product (e.g., EDA). Thus, in a strain engineered to produce EPA, improvement in Δ9 elongase conversion efficiency was demonstrated to result in increased EPA % TFAs and/or EPA % DCW.

These results, and additional supporting work, are the cornerstone of a method disclosed below for improving C₁₈ to C₂₀ elongation conversion efficiency in a LC-PUFA-producing recombinant oleaginous microbial host cell, wherein said method comprises:
a) introducing into said LC-PUFA-producing recombinant host cell at least one isolated polynucleotide encoding a polypeptide having at least acyl-CoA:lysophospholipid acyltransferase activity wherein the polypeptide is selected from the group consisting of:
   (i) a polypeptide having at least 45% amino acid identity, based on the Clustal W method of alignment, when compared to an amino acid sequence selected from the group consisting of SEQ ID NO:15 (ScAle1) and SEQ ID NO:17 (YIAle1);
   (ii) a polypeptide having at least one membrane bound *O-*acyltransferase protein family motif selected from the group consisting of: M(V/I)LxxKL (SEQ ID NO:18), RxKYYxxW (SEQ ID NO:19), SAxWHG (SEQ ID NO:20) and EX₁₁WNX₂-[T/V]-X₂W (SEQ ID NO:21);
   (iii) a polypeptide having at least 90% amino acid identity, based on the Clustal W method of alignment, when compared to an amino acid sequence as set forth in SEQ ID NO:25 (CeLPCAT);
   (iv) a polypeptide having at least 43.9% amino acid identity, based on the Clustal W method of alignment, when compared to an amino acid sequence selected from the group consisting of SEQ ID NO:29 (MaLPAAT1), SEQ ID NO:31 (YILPAAT1) and SEQ ID NO:32 (ScLPAAT1); and,
   (v) a polypeptide having at least one 1-acyl-sn-glycerol-3-phosphate acyltransferase protein family motif selected from the group consisting of: NHxxxxD (SEQ ID NO:33) and EGTR (SEQ ID NO:34);
      wherein the at least one isolated polynucleotide encoding a polypeptide having at least acylCoA:lysophospholipid acyltransferase activity is operably linked to at least one regulatory sequence, said regulatory sequence being the same or different; and,
b) growing the oleaginous microbial host cell;
wherein the C₁₈ to C₂₀ elongation conversion efficiency of the oleaginous microbial host cell is increased relative to the control host cell.

Preferably, the polynucleotide encoding a polypeptide having at least acyl-CoA:lysophospholipid acyltransferase activity is stably integrated and the an increase in C₁₈ to C₂₀ elongation conversion is at least about 4%,

More preferred, the increase in C₁₈ to C₂₀ elongation conversion efficiency is at least about 4-10%, more preferred at least about 10-20%, more preferred at least about 20-40%, and most preferred at least about 40-60% or greater in at least one LC-PUFA-producing oleaginous microbial host cell when compared to the control host cell.

Based on the improvement in C₁₈ to C₂₀ elongation conversion efficiency described above, optimized strains of recombinant *Yarrowia lipolytica* having the ability to produce improved EPA % TFAs, relative to those strains described in U.S. Pat. Appl. Pub. No. 2009-0093543-A1, will optionally comprise at least one acyl-CoA lysophospholipid acyltransferase ["LPLAT"] as defined in the methods described above. As described herein, the amino acid sequence of the LPLAT is selected from the group consisting of: SEQ ID NO:15 (ScAle1), SEQ ID NO:16 (YIAle1), SEQ ID NO:25 (CeLPCAT), SEQ ID NO:29 (MaLPAAT1), SEQ ID NO:31 (YILPAAT1) and SEQ ID NO:32 (ScLPAAT1).

U.S. Pat. Appl. Pub. No. 2009-0093543-A1 describes a variety of knockouts useful in recombinant *Yarrowia* sp., including those useful for selection of transformants, those that diminish fatty acid degradation and TAG degradation and those that appear to result in a phenotypically "neutral" mutation (wherein the *Yarrowia* host cell seems unaffected). Most preferred, however, are those gene knockouts (e.g., diacylglycerol acyltransferase gene knockouts, peroxisome biogenesis factor protein ["PEX"] gene knockouts) that result in increases in the concentration of EPA relative to the total fatty acids ["EPA % TFAs"].

More specifically, U.S. Pat. Appl. Pub. No. 2009-0093543-A1 contemplates that in some preferred recombinant *Yarrowia* production hosts, the host is devoid of a native gene encoding a peroxisome biogenesis factor protein selected from the group consisting of: Pex1p (SEQ ID NO:108), Pex2p (SEQ ID NO:109), Pex3p (SEQ ID NO:110), Pex3Bp (SEQ ID NO:111), Pex4p (SEQ ID NO:112), Pex5p (SEQ ID NO:113), Pex6p (SEQ ID NO:114), Pex7p (SEQ ID NO:115), Pex8p (SEQ ID NO:116), Pex10p (SEQ ID NO:117), Pex12p (SEQ ID NO:118), Pex13p (SEQ ID NO:119), Pex14p (SEQ ID NO:120), Pex16p (SEQ ID NO:121), Pex17p (SEQ ID NO:122), Pex19p (SEQ ID NO:123), Pex20p (SEQ ID NO:124), Pex22p (SEQ ID NO:125) and Pex26p (SEQ ID NO:126). More preferred disrupted peroxisome biogenesis factor proteins are Pex2p, Pex3p, Pex10p, Pex12p and Pex16p, although data is provided only concerning Pex10p.

Intl. App. Pub. No. WO 2009/046248 confirms and expands the hypotheses and studies presented in U.S. Pat. Appl. Pub. No. 2009-0093543-A1, by comparing ΔPex16, ΔPex3 and ΔPex10 strains of *Yarrowia lipolytica.* Results therein demonstrated that Pex10 disruption was responsible for a 3.3 fold increase in EPA % TFAs and a 1.7 fold increase in the amount of C₂₀ PUFAs relative to the non-disrupted strain engineered for EPA production. Similarly, a 1.65 fold increase in DGLA % TFAs and a 1.3 fold increase in C₂₀ PUFAs % TFAs was observed in a ΔPex16 strain engineered for DGLA production. A 2.0 fold increase in DGLA % TFAs and a 1.7 fold increase in C₂₀ PUFAs % TFAs was observed in a ΔPex3 strain engineered for DGLA production.

These results, and additional supporting work, are the cornerstone of a method disclosed below for increasing the weight percent of one PUFA or a combination of PUFAs relative to the weight percent of total fatty acids in an oleaginous eukaryotic organism having a total lipid content, a total lipid fraction and an oil fraction, said method comprising:
a) providing an oleaginous eukaryotic organism comprising a disruption in a native gene encoding a peroxisome biogenesis factor protein, which creates a PEX-disruption organism; and genes encoding a functional PUFA biosynthetic pathway; and,
b) growing the PEX-disrupted organism under conditions wherein the weight percent of at least one PUFA is increased in the total lipid fraction and in the oil fraction relative to the weight percent of the total fatty acids, when compared with those weight percents in an oleaginous eukaryotic organism whose native peroxisome biogenesis factor protein has not been disrupted.
The amount of PUFAs that increases as a percent of total fatty acids can be: 1) the PUFA that is the desired end product of a functional PUFA biosynthetic pathway, as opposed to PUFA intermediates or by-products; 2) C₂₀ to C₂₂ PUFAs; and/or, 3) total PUFAs.

In addition to the increase in the weight percent of one or a combination of PUFAs relative to the weight percent of the total fatty acids, in some cases, the total lipid content (TFA % DCW) of the cell may be increased or decreased. What this means is that regardless of whether the disruption in the PEX gene causes the amount of total lipids in the PEX-disrupted cell to increase or decrease, the disruption always causes the weight percent of a PUFA or of a combination of PUFAs to increase.

Based on the above, optimized strains of recombinant *Yarrowia lipolytica* having the ability to produce improved EPA % TFAs, relative to those strains described in U.S. Pat. Appl. Pub. No. 2009-0093543-A1, will comprise at least one peroxisome biogenesis factor protein whose expression has been down-regulated (i.e., thereby producing a PEX-disrupted organism). In preferred strains, the down-regulated peroxisome biogenesis factor protein is Pex3p (SEQ ID NO:110), Pex10p (SEQ ID NO:117), or Pex16p (SEQ ID NO:121).

Although numerous techniques are available to one of skill in the art to achieve disruption of a native *Yarrowia* gene, generally the endogenous activity of a particular gene can be reduced or eliminated by the following techniques, for example: 1) disrupting the gene through insertion, substitution and/or deletion of all or part of the target gene; or, 2) manipulating the regulatory sequences controlling the expression of the protein. Both of these techniques are discussed in U.S. Pat. Appl. Pub. No. 2009-0093543-A1, as well as Intl. App. Pub. No. WO 2009/046248. One skilled in the art would appreciate that these and other methods are well described in the existing literature and are not limiting to the methods, host cells, and products described herein. One skilled in the art will also appreciate the most appropriate technique for use with any particular oleaginous yeast.

The optimized strains will produce at least about 40-50 EPA % TFAs, preferably at least about 50-55 EPA % TFAs, more preferably at least about 55-60 EPA % TFAs, more preferably at least 60-70 EPA % TFAs, and most preferably at least about 70-80 EPA % TFAs.

As will be clear to one of skill in the art, a multitude of different optimized *Yarrrowia* strains producing at least about 50 EPA % TFAs could be engineered using the methodologies described herein. Selection of a preferred strain for commercial purposes will therefore also consider the total lipid content of the engineered strain, since both the concentration of EPA as a percent of the total fatty acids ["EPA % TFAs"] and total lipid content ["TFAs % DCW"] affect the cellular content of EPA as a percent of the dry cell weight ["EPA % DCW"]. That is, EPA % DCW is calculated as: (EPA % TFAs) * (TFA % DCW)]/100. For example, a strain producing 50 EPA % TFAs and having 24 TFAs % DCW, a strain producing 55 EPA % TFAs and having 21.82 TFAs % DCW, a strain producing 60 EPA % TFAs and having 20 TFAs % DCW, a strain producing 65 EPA % TFAs and having 18.46 TFAs % DCW and a strain producing 70 EPA % TFAs and having 17.14 TFAs % DCW all produce 12 EPA % DCW. As described herein, an improved optimized strain of *Yarrrowia lipolytica* may produce at least about 10-12 EPA % DCW, preferably at least about 12-14 EPA % DCW, more preferably at least about 14-16 EPA % DCW, more preferably at least about 16-18 EPA % DCW, more preferably at least about 18-20 EPA % DCW, more preferably at least about 20-22 EPA % DCW, more preferably at least about 22-24 EPA % DCW, and most preferably at least about 24-26 EPA % DCW.

In addition to possessing at least about 50 EPA % TFAs, the lipid profile within an improved optimized strain of *Yarrrowia lipolytica,* or within extracted or unconcentrated oil therefrom, will have a ratio of EPA % TFAs to LA % TFAs of at least about 3.1. As demonstrated in U.S. Pat. Appl. Pub. No. 2009-0093543-A1 (Table 23), EPA, LA and oleic acid constituted approximately 76-80% of the fatty acids present in the lipid profile of a strain of *Y*. *lipolytica* producing greater than 40 EPA % TFAs. Of this, LA % TFAs was ca. three-fold greater than oleic acid % TFAs. Based on these observations, one of skill in the art will appreciate that minimizing the concentration of the intermediate fatty acid, LA (resulting in increased ratios of EPA:LA), will result in greater "pushing" of the carbon through the PUFA biosynthetic pathway and permit increased synthesis of EPA. As described herein, the ratio of EPA:LA will be at least about 3.1-3.5, more preferably at least about 3.5-4.5, more preferably at least about 4.5-5.5, and most preferably at least about 5.5-6.5.

Lipids produced by an improved optimized recombinant *Y. lipolytica* strains described herein will also be distinguished as having less than about 0.5% GLA or DHA (when measured by GC analysis using equipment having a detectable level down to about 0.1 %) and having a saturated fatty acid content of less than about 8%. This low percent of saturated fatty acids (i.e., 16:0 and 18:0) results in substantial health benefits to humans and animals.

Microbial expression systems and expression vectors containing regulatory sequences that direct high-level expression of foreign proteins are well known to those skilled in the art. Any of these could be used to construct chimeric genes encoding the preferred desaturase, elongase, CPT1, DGLA synthase, malonyl CoA synthetase and acyl-CoA lysophospholipid acyltransferase proteins. These chimeric genes could then be introduced into *Yarrowia lipolytica* using standard methods of transformation to provide high-level expression of the encoded enzymes.

Vectors (e.g., constructs, plasmids) and DNA expression cassettes useful for the transformation of *Yarrowia* host cells are well known in the art. The specific choice of sequences present in the construct is dependent upon the desired expression products, the nature of the host cell, and the proposed means of separating transformed cells versus non-transformed cells. Typically, however, the vector contains at least one expression cassette, a selectable marker and sequences allowing autonomous replication or chromosomal integration. Suitable expression cassettes typically comprise a region 5' of the gene that controls transcriptional initiation (e.g., a promoter), the gene coding sequence, and a region 3' of the DNA fragment that controls transcriptional termination (i.e., a terminator). It is most preferred when both control regions are derived from genes from the transformed host cell, although they need not be derived from genes native to the production host (e.g., *Yarrowia lipolytica*)*.*

Where two or more genes are expressed from separate replicating vectors, it is desirable that each vector has a different means of selection and should lack homology to the other constructs to maintain stable expression and prevent reassortment of elements among constructs. Judicious choice of regulatory regions, selection means and method of propagation of the introduced construct can be experimentally determined so that all introduced genes are expressed at the necessary levels to provide for synthesis of the desired products.

Constructs or vectors comprising the gene(s) of interest may be introduced into a host cell such as *Yarrowia* by any standard technique. These techniques include transformation (e.g., lithium acetate transformation [Methods in Enzymology, 194:186-187 (1991)]), bolistic impact, electroporation, microinjection, or any other method that introduces the gene(s) of interest into the host cell. More preferred herein for *Yarrowia lipolytica* are integration techniques based on linearized fragments of DNA, as described in U.S. Patent 4,880,741 and U.S. Patent 5,071,764 and Chen, D. C. et al. (Appl. Microbiol. Biotechnol., 48(2):232-235 (1997)).

For convenience, a host cell that has been manipulated by any method to take up a DNA sequence (e.g., an expression cassette) is referred to herein as "transformed", "transformant" or "recombinant". The transformed host will have at least one copy of the expression cassette and may have two or more, depending upon whether the expression cassette is integrated into the genome or is present on an extrachromosomal element having multiple copy numbers. The transformed host cell can be identified by various selection techniques, as described in U.S. Patent 7,238,482 and U.S. Patent 7,259,255.

Preferred selection methods for use herein are resistance to kanamycin, hygromycin and the amino glycoside G418, as well as ability to grow on media lacking uracil, leucine, lysine, tryptophan or histidine. Alternatively, 5-fluoroorotic acid (5-fluorouracil-6-carboxylic acid monohydrate; "5-FOA") is used for selection of yeast Ura- mutants (U.S. Pat. Appl. Pub. No. 2009-0093543-A1), or a native acetohydroxyacid synthase (or acetolactate synthase; E.C. 4.1.3.18) that confers sulfonyl urea herbicide resistance (Intl. App. Pub. No. WO 2006/052870) is utilized for selection of transformants. A unique method of "recycling" a pair of preferred selection markers for their use in multiple sequential transformations, by use of site-specific recombinase systems, is also taught in U.S. Pat. Appl. Pub. No. 2009-0093543-A1.

As is well known to one of skill in the art, merely inserting a gene (e.g., a desaturase, elongase, CPT1, DGLA synthase, malonyl CoA synthetase, acyl-CoA lysophospholipid acyltransferase) into a cloning vector does not ensure its expression at the desired rate, concentration, amount, etc. It may be desirable to manipulate a number of different genetic elements that control aspects of transcription, RNA stability, translation, protein stability and protein location, oxygen limitation and secretion from the host cell. More specifically, gene expression may be controlled by altering the following: the nature of the relevant transcriptional promoter and terminator sequences; the number of copies of the cloned gene; whether the gene is plasmid-borne or integrated into the genome of the host cell; the final cellular location of the synthesized foreign protein; the efficiency of translation in the host organism; the intrinsic stability of the cloned gene protein within the host cell; and, the codon usage within the cloned gene, such that its frequency approaches the frequency of preferred codon usage of the host cell. Several of these methods of overexpression will be discussed below, and are useful in recombinant *Yarrowia* host cells as a means to overexpress e.g., desaturases, elongases, CPT1 proteins, DGLA synthases, malonyl CoA synthetases and acyl-CoA lysophospholipid acyltransferases.

Expression of the desired gene(s) can be increased at the transcriptional level through the use of a stronger promoter (either regulated or constitutive) to cause increased expression, by removing/deleting destabilizing sequences from either the mRNA or the encoded protein, or by adding stabilizing sequences to the mRNA (U.S. Patent 4,910,141).

Transcription initiation control regions or promoters which are useful to drive expression of heterologous genes or portions thereof in *Yarrowia* host cells are numerous and known to those skilled in the art. Expression can be accomplished in an induced or constitutive fashion. Induced expression can be accomplished by inducing the activity of a regulatable promoter operably linked to the gene of interest, while constitutive expression can be achieved by the use of a constitutive promoter operably linked to the gene of interest. Virtually any promoter (i.e., native, synthetic, or chimeric) capable of directing expression of desaturase, elongase, CPT1, DGLA synthase, malonyl CoA synthetase and acyl-CoA lysophospholipid acyltransferase genes in *Yarrowia* will be suitable, although transcriptional and translational regions from the host species are particularly useful.

In general, the termination region can be derived from the 3' region of the gene from which the initiation region was obtained or from a different gene. A large number of termination regions are known and function satisfactorily in a variety of hosts when utilized both in the same and different genera and species from which they were derived. The termination region usually is selected more as a matter of convenience rather than because of any particular property. Preferably, the termination region is derived from a yeast gene. The 3'-region can also be synthetic, as one of skill in the art can utilize available information to design and synthesize a 3'-region sequence that functions as a transcription terminator. A termination site may be unnecessary, but it is highly preferred.

Although not intended to be limiting, preferred promoter regions and termination regions useful in the disclosure herein are those taught in U.S. Pat. Pub. No. 2009-0093543-A1.

Additional copies (i.e., more than one copy) of the PUFA biosynthetic pathway desaturase, elongase and DGLA synthase genes and/or CPT1, malonyl CoA synthetase and acyl-CoA lysophospholipid acyltransferase genes may be introduced into *Yarrowia lipolytica* to thereby increase EPA production and accumulation. Specifically, additional copies of genes may be cloned within a single expression construct; and/or, additional copies of the cloned gene(s) may be introduced into the host cell by increasing the plasmid copy number or by multiple integration of the cloned gene into the genome (*infra*)*.*

It is important to note that the when preparing optimized strains of *Y. lipolytica* according to the methodology herein, copies of various desaturases, elongases, CPT1s, DGLA synthases, malonyl CoA synthetases and acyl-CoA lysophospholipid acyltransferases are often referred to. If, for example, 2 copies of a Δ9 elongase are required, this can refer to: 1) two copies of an identical coding sequence for a particular Δ9 elongase isolated from a single species; or, 2) one coding sequence for a Δ9 elongase isolated from a species "A" and one coding sequence for a Δ9 elongase isolated from a species "B", thus collectively resulting in two Δ9 elongases.

In general, once a DNA cassette (e.g., comprising a chimeric gene comprising a promoter, ORF and terminator) suitable for expression in an oleaginous yeast has been obtained, it is either placed in a plasmid vector capable of autonomous replication in a host cell or directly integrated into the genome of the host cell. Integration of expression cassettes can occur randomly within the host genome or can be targeted through the use of constructs containing regions of homology with the host genome sufficient to target recombination with the host locus. Although not relied on herein, all or some of the transcriptional and translational regulatory regions can be provided by the endogenous locus where constructs are targeted to an endogenous locus.

The preferred method of expressing genes in *Yarrowia lipolytica* is by integration of a linear DNA fragment into the genome of the host. Integration into multiple locations within the genome can be particularly useful when high level expression of genes are desired. Preferred loci include those taught in U.S. Pat. Pub.No. 2009-0093543-A1.

Juretzek et al. (Yeast, 18:97-113 (2001)) note that the stability of an integrated DNA fragment in *Yarrowia lipolytica* is dependent on the individual transformants, the recipient strain and the targeting platform used. Thus, the skilled artisan will recognize that multiple transformants must be screened in order to obtain a strain displaying the desired expression level and pattern. Such screening may be accomplished by Southern analysis of DNA blots (Southern, J. Mol. Biol., 98:503 (1975)), Northern analysis of mRNA expression (Kroczek, J. Chromatogr. Biomed. Appl., 618 (1-2):133-145 (1993)), Western analysis of protein expression, phenotypic analysis or GC analysis of the PUFA products.

The transformed microbial host cell is grown under conditions that optimize expression of chimeric genes (e.g., encoding desaturases, elongases, CPT1, DGLA synthases, malonyl CoA synthetases, acyl-CoA lysophospholipid acyltransferases) and produce the greatest and the most economical yield of EPA. In general, media conditions may be optimized by modifying the type and amount of carbon source, the type and amount of nitrogen source, the carbon-to-nitrogen ratio, the amount of different mineral ions, the oxygen level, growth temperature, pH, length of the biomass production phase, length of the oil accumulation phase and the time and method of cell harvest. *Yarrowia lipolytica* are generally grown in a complex media such as yeast extract-peptone-dextrose broth ["YPD"] or a defined minimal media that lacks a component necessary for growth and thereby forces selection of the desired expression cassettes (e.g., Yeast Nitrogen Base (DIFCO Laboratories, Detroit, MI)).

Fermentation media for the methods and host cells described herein must contain a suitable carbon source, such as are taught in U.S. Patent 7,238,482 and U.S. Pat. Appl. No. 12/641,929 (filed December 19, 2009). Although it is contemplated that the source of carbon utilized in the presently disclosed methods may encompass a wide variety of carbon-containing sources, preferred carbon sources are sugars, glycerol and/or fatty acids. Most preferred is glucose, sucrose, invert sucrose, fructose and/or fatty acids containing between 10-22 carbons. For example, the fermentable carbon source can be selected from the group consisting of invert sucrose, glucose, fructose and combinations of these, provided that glucose is used in combination with invert sucrose and/or fructose.

The term "invert sucrose", also referred to herein as "invert sugar", refers to a mixture comprising equal parts of fructose and glucose resulting from the hydrolysis of sucrose. Invert sucrose may be a mixture comprising 25 to 50% glucose and 25 to 50% fructose. Invert sucrose may also comprise sucrose, the amount of which depends on the degree of hydrolysis.

Nitrogen may be supplied from an inorganic (e.g., (NH₄)₂SO₄) or organic (e.g., urea or glutamate) source. In addition to appropriate carbon and nitrogen sources, the fermentation media must also contain suitable minerals, salts, cofactors, buffers, vitamins and other components known to those skilled in the art suitable for the growth of the high EPA-producing oleaginous yeast and the promotion of the enzymatic pathways for EPA production. Particular attention is given to several metal ions, such as Fe⁺², Cu⁺², Mn⁺², Co⁺², Zn⁺² and Mg⁺², that promote synthesis of lipids and PUFAs (Nakahara, T. et al., Ind. Appl. Single Cell Oils, D. J. Kyle and R. Colin, eds. pp 61-97 (1992)).

Preferred growth media for the methods and host cells described herein are common commercially prepared media, such as Yeast Nitrogen Base (DIFCO Laboratories, Detroit, MI). Other defined or synthetic growth media may also be used and the appropriate medium for growth of *Yarrowia lipolytica* will be known by one skilled in the art of microbiology or fermentation science. A suitable pH range for the fermentation is typically between about pH 4.0 to pH 8.0, wherein pH 5.5 to pH 7.5 is preferred as the range for the initial growth conditions. The fermentation may be conducted under aerobic or anaerobic conditions, wherein microaerobic conditions are preferred.

Typically, accumulation of high levels of PUFAs in oleaginous yeast cells requires a two-stage process, since the metabolic state must be "balanced" between growth and synthesis/storage of fats. Thus, most preferably, a two-stage fermentation process is necessary for the production of EPA in *Yarrowia lipolytica.* This approach is described in U.S. Patent 7,238,482, as are various suitable fermentation process designs (i.e., batch, fed-batch and continuous) and considerations during growth.

In some aspects herein, the primary product is oleaginous yeast biomass. As such, isolation and purification of the EPA-containing oils from the biomass may not be necessary (i.e., wherein the whole cell biomass is the product).

However, certain end uses and/or product forms may require partial and/or complete isolation/purification of the EPA-containing oil from the biomass, to result in partially purified biomass, purified oil, and/or purified EPA. PUFAs, including EPA, may be found in the host microorganism (e.g., *Yarrowia*) as free fatty acids or in esterified forms such as acylglycerols, phospholipids, sulfolipids or glycolipids. These fatty acids may be extracted from the host cell through a variety of means well-known in the art. One review of extraction techniques, quality analysis and acceptability standards for yeast lipids is that of Z. Jacobs (Critical Reviews in Biotechnology, 12(5/6):463-491 (1992)). A brief review of downstream processing is also available by A. Singh and O. Ward (Adv. Appl. Microbiol., 45:271-312 (1997)).

In general, means for the purification of EPA and other PUFAs from *Yarrowia* biomass may include extraction (e.g., U.S. Patent 6,797,303 and U.S. Patent 5,648,564) with organic solvents, sonication, supercritical fluid extraction (e.g., using carbon dioxide), saponification and physical means such as presses, bead beaters, or combinations thereof. One is referred to the teachings of U.S. Patent 7,238,482 for additional details.

Oils containing EPA that have been refined and/or purified can be hydrogenated, to thereby result in fats with various melting properties and textures. Many processed fats, including spreads, confectionary fats, hard butters, margarines, baking shortenings, etc., require varying degrees of solidity at room temperature and can only be produced through alteration of the source oil's physical properties. This is most commonly achieved through catalytic hydrogenation as described in WO 2006/052870 .

Food products, infant formulas, functional foods, medical foods, medical nutritionals, dietary supplements, pharmaceutical compositions, animal feeds, and personal care products comprising oleaginous yeast biomass comprising EPA are provided herein. Similarly, also provided are food products, infant formulas, functional foods, medical foods, medical nutritionals, dietary supplements, pharmaceutical compositions, animal feeds, and personal care products comprising EPA or microbial oil comprising EPA isolated from the recombinant oleaginous yeast biomass.

One of skill in the art of processing and formulation will understand how the amount and composition of the biomass, partially purified biomass, purified oil, and/or purified EPA may be added to a particular product according to target species and/or end use. More specifically, an "effective" amount should be incorporated into a product formulation, although this amount will depend on the food or feed product, the diet that the product is intended to supplement or the medical condition that the medical food or medical nutritional is intended to correct or treat. Most desirably, the effective amount of EPA will be sufficient to provide the desirable health characteristics associated with ω-3/ω-6 PUFA consumption. Typically, the amount of EPA incorporated into the product takes into account losses associated with processing conditions, typical handling and storage conditions, the stability of EPA in the product, and the bioavailability/ bioabsorption efficiency with the target species, to name a few.

One of skill in the art of processing and formulation will be familiar with processes to concentrate the oil produced from the recombinant *Yarrowia* production host cells described herein, to thereby increase the concentration of EPA in the total lipid fraction such that it comprises at least about 60%, at least about 70%, at least about 80% or even at least about 90% EPA. Means to blend the purified oils described herein with other purified fatty acids (e.g., LA, GLA, EDA, DGLA, ARA, DTA, DPAn-6, ALA, STA, ETrA, ETA, DPA and DHA), or oils containing alternate fatty acids in preferred concentrations, are also well known to one of skill in the art. These techniques readily permit the creation of an oil comprising a uniquely tailored fatty acid profile.

Personal Care Products: Within the context of personal care products, ω-3 fatty acids have particular application in skin formulations where they may be used to enhance the skin conditioning effect. The skilled person will understand how to provide an effective amount of the relevant ω-3 fatty acid(s) or oil comprising the same to a skin care composition. In addition to the PUFA oil or ω-3 fatty acid, the skin care composition may further comprise a cosmetically acceptable medium for skin care compositions, examples of which are described by Philippe et al. in U.S. Patent 6,280,747. For example, the cosmetically acceptable medium may be an anhydrous composition containing a fatty substance in a proportion generally from about 10% to about 90% by weight relative to the total weight of the composition, where the fatty phase contains at least one liquid, solid or semi-solid fatty substance. The fatty substance includes, but is not limited to, oils, waxes, gums, and so-called pasty fatty substances. Alternatively, the compositions may be in the form of a stable dispersion such as a water-in-oil or oil-in-water emulsion. Additionally, the compositions may contain one or more conventional cosmetic or dermatological additives or adjuvants including, but not limited to, antioxidants, preserving agents, fillers, surfactants, UVA and/or UVB sunscreens, fragrances, thickeners, wetting agents, anionic or nonionic or amphoteric polymers, and dyes.

Foodstuffs: The market place currently supports a large variety of food and feed products, incorporating ω-3 and/or ω-6 fatty acids (particularly LA, GLA, ARA, EPA, DPA and DHA). It is contemplated that the yeast biomass, partially purified biomass, purified oil, and/or purified EPA described herein will function in food products to impart the health benefits of current formulations.

Food products will include, but not be limited to: food analogs, drinks, meat products, cereal products, baked foods, snack foods and dairy products.

Food analogs can be made using processes well known to those skilled in the art. There can be mentioned meat analogs, cheese analogs, milk analogs and the like. Meat analogs made from soybeans contain soy protein or tofu and other ingredients mixed together to simulate various kinds of meats. These meat alternatives are sold as frozen, canned or dried foods. Usually, they can be used the same way as the foods they replace. Examples of meat analogs include, but are not limited to: ham analogs, sausage analogs, bacon analogs, and the like.

Food analogs can be classified as imitation or substitutes depending on their functional and compositional characteristics. For example, an imitation cheese need only resemble the cheese it is designed to replace. However, a product can generally be called a substitute cheese only if it is nutritionally equivalent to the cheese it is replacing and meets the minimum compositional requirements for that cheese. Thus, substitute cheese will often have higher protein levels than imitation cheeses and be fortified with vitamins and minerals.

Milk analogs or nondairy food products include, but are not limited to: imitation milks and nondairy frozen desserts (e.g., those made from soybeans and/or soy protein products).

Meat products encompass a broad variety of products. In the United States "meat" includes "red meats" produced from cattle, hogs and sheep. In addition to the red meats there are poultry items which include chickens, turkeys, geese, guineas, ducks and the fish and shellfish. There is a wide assortment of seasoned and processed meat products: fresh, cured and fried, and cured and cooked. Sausages and hot dogs are examples of processed meat products. Thus, the term "meat products" as used herein includes, but is not limited to, processed meat products.

A cereal food product is a food product derived from the processing of a cereal grain. A cereal grain includes any plant from the grass family that yields an edible grain (seed). The most popular grains are barley, corn, millet, oats, quinoa, rice, rye, sorghum, triticale, wheat and wild rice. Examples of cereal food products include, but are not limited to: whole grain, crushed grain, grits, flour, bran, germ, breakfast cereals, extruded foods, pastas, and the like.

A baked goods product comprises any of the cereal food products mentioned above and which has been baked or processed in a manner comparable to baking, i.e., to dry or harden by subjecting to heat. Examples of a baked good product include, but are not limited to: bread, cakes, doughnuts, bars, pastas, bread crumbs, baked snacks, mini-biscuits, mini-crackers, mini-cookies, and mini-pretzels. As was mentioned above, oils from the recombinant EPA production host cells can be used as an ingredient.

A snack food product comprises any of the above or below described food products.

A fried food product comprises any of the above or below described food products that has been fried.

The beverage can be in a liquid or in a dry powdered form. For example, there can be mentioned: non-carbonated drinks; fruit juices, fresh, frozen, canned or concentrate; flavored or plain milk drinks, etc. Adult and infant nutritional formulas are well known in the art and commercially available (e.g., Similac®, Ensure®, Jevity®, and Alimentum® from Ross Products Division, Abbott Laboratories).

A dairy product is a product derived from milk. A milk analog or nondairy product is derived from a source other than milk, for example, soymilk as was discussed above. These products include, but are not limited to: whole milk, skim milk, fermented milk products such as yogurt or sour milk, cream, butter, condensed milk, dehydrated milk, coffee whitener, coffee creamer, ice cream, cheese, etc.

Additional food products into which the *Yarrowia* biomass, partially purified biomass, purified oil, and/or purified EPA could be included are, for example: chewing gums, confections and frostings, gelatins and puddings, hard and soft candies, jams and jellies, white granulated sugar, sugar substitutes, sweet sauces, toppings and syrups, and dry-blended powder mixes.

Infant Formulas: Infant formulas are liquids or reconstituted powders fed to infants and young children. "Infant formula" is defined herein as an enteral nutritional product which can be substituted for human breast milk in feeding infants and typically is composed of a desired percentage of fat mixed with desired percentages of carbohydrates and proteins in an aqueous solution (e.g., see U.S. Patent 4,670,285). Based on worldwide composition studies, as well as levels specified by expert groups, average human breast milk typically contains about 0.20% to 0.40% of total fatty acids (assuming about 50% of calories from fat); and, generally the ratio of DHA to ARA would range from about 1:1 to 1:2 (see, e.g., formulations of Enfamil LIPIL™ [Mead Johnson & Company] and Similac Advance™ [Ross Products Division, Abbott Laboratories]). Infant formulas have a special role to play in the diets of infants because they are often the only source of nutrients for infants. Although breast-feeding is still the best nourishment for infants, infant formula is a close enough second that babies not only survive but thrive.

Health Food Products And Pharmaceuticals: The present biomass, partially purified biomass, purified oil, and/or purified EPA may be used in formulations to impart health benefit in health food products, including functional foods, medical foods, medical nutritionals and dietary supplements. Additionally, *Yarrowia* biomass, partially purified biomass, purified oil, and/or purified EPA may be used in standard pharmaceutical compositions. The present engineered strains of *Yarrowia lipolytica* or the microbial oils produced therefrom comprising EPA could readily be incorporated into the any of the above mentioned food products, to thereby produce e.g., a functional or medical food. For example, more concentrated formulations comprising EPA include capsules, powders, tablets, softgels, gelcaps, liquid concentrates and emulsions which can be used as a dietary supplement in humans or animals other than humans.

Animal Feed Products: Animal feeds are generically defined herein as products intended for use as feed or for mixing in feed for animals other than humans. The *Yarrowia* biomass, partially purified biomass, purified oil, and/or purified EPA described herein can be used as an ingredient in various animal feeds.

More specifically, although not to be construed as limiting, it is expected that the EPA products from the recombinant *Yarrowia* host cells can be used within pet food products, ruminant and poultry food products and aquacultural food products. Pet food products are those products intended to be fed to a pet, such as a dog, cat, bird, reptile, rodent. These products can include the cereal and health food products above, as well as meat and meat byproducts, soy protein products, grass and hay products (such as alfalfa, timothy, oat or brome grass) and vegetables. Ruminant and poultry food products are those wherein the product is intended to be fed to e.g., turkeys, chickens, cattle and swine. As with the pet foods above, these products can include cereal and health food products, soy protein products, meat and meat byproducts, and grass and hay products as listed above. Aquacultural food products (or "aquafeeds") are those products intended to be used in aquafarming, which concerns the propagation, cultivation or farming of aquatic organisms and/or animals in fresh or marine waters.

It is contemplated that the present engineered strains of *Yarrowia lipolytica* that are producing high concentrations of EPA will be especially useful to include in most animal feed formulations. In addition to providing necessary ω-3 PUFAs, the yeast itself is a useful source of protein and other nutrients (e.g., vitamins, minerals, nucleic acids, complex carbohydrates, etc.) that can contribute to overall animal health and nutrition, as well as increase a formulation's palatablility. Accordingly it is contemplated that the addition of yeast biomass comprising the recombinant *Yarrowia* production hosts will be an excellent additional source of feed nutrients in animal feed formulations (see U.S. Pat. Appl. Pub. No. 2009-0093543-A1 for additional details).

It is clear then that the present engineered strains of *Yarrowia lipolytica* that are producing high concentrations of EPA will be especially useful to include in most aquaculture feeds. In addition to providing necessary ω-3 and/or ω-6 PUFAs, the yeast itself is a useful source of protein that can increase the formulation's palatablility. Alternatively, the oils produced by the present strains of *Y. lipolytica* could be introduced directly into the aquaculture feed formulations, following extraction and purification from the cell mass.

There is increasing awareness that EPA is an important ω-3 fatty acid in and of itself. As a result, it is expected herein that the EPA-enriched oils of the recombinant *Yarrowia* production hosts described herein will have very broad utility in a variety of therapeutic applications, e.g., inflammation, cardiovascular diseases, nutrient regulation of gene expression and dyslipidemia, and specifically in the treatment of clinical conditions including: coronary heart disease, high blood pressure, inflammatory disorders, Type II diabetes, ulcerative colitis, Crohn's disease, anorexia nervosa, burns, osteoarthritis, osteoporosis, depression, and attention deficit/hyperactivity disorder.

U.S. Pat. Appl. Pub. No. 2009-0093543-A1 describes additional clinical human studies, relating to EPA and inflammation, EPA and cardiovascular diseases, ω-3 PUFAs and nutrient regulation of gene expression, and ω-3 PUFAs and dyslipidemia and should be referred to therein. More recently, a randomized, double-blind placebo-controlled study was performed in 110 normal healthy subjects , wherein subjects were provided one of the following for 6 weeks, as a means to evaluate the effects of the oils on cardiovascular disease risk factors, adverse events and safety parameters: 600 mg/day EPA, 1800 mg/day EPA, 600 mg/day DHA or olive oil (placebo) (Gillies, P., "The New Science of Omega-3 Fatty Acids: Differential Nutritional Pharmacology" Texas Human Nutrition Conference, Texas A&M University, February 2010*..* The 600 mg EPA per day supplement was found to maintain healthy cholesterol levels already in the normal range. Notably, the EPA oils of the study were derived from engineered strains of *Yarrowia lipolytica.*

### EXAMPLES

The present invention is further defined in the following Examples. It should be understood that these Examples, while indicating preferred embodiments of the invention, are given by way of illustration only. From the above discussion and these Examples, one skilled in the art can ascertain the essential characteristics of this invention, and without departing from the spirit and scope thereof, can make various changes and modifications of the invention to adapt it to various usages and conditions, as defined in the claims.

### GENERAL METHODS

Standard recombinant DNA and molecular cloning techniques used in the Examples are well known in the art and are described by: 1) Sambrook, J., Fritsch, E. F. and Maniatis, T. Molecular Cloning: A Laboratory Manual; Cold Spring Harbor Laboratory: Cold Spring Harbor, NY (1989) (Maniatis); 2) T. J. Silhavy, M. L. Bennan, and L. W. Enquist, Experiments with Gene Fusions; Cold Spring Harbor Laboratory: Cold Spring Harbor, NY (1984); and, 3) Ausubel, F. M. et al., Current Protocols in Molecular Biology, published by Greene Publishing Assoc. and Wiley-Interscience, Hoboken, NJ (1987).

Materials and methods suitable for the maintenance and growth of microbial cultures are well known in the art. Techniques suitable for use in the following examples may be found as set out in Manual of Methods for General Bacteriology (Phillipp Gerhardt, R. G. E. Murray, Ralph N. Costilow, Eugene W. Nester, Willis A. Wood, Noel R. Krieg and G. Briggs Phillips, Eds), American Society for Microbiology: Washington, D.C. (1994)); or by Thomas D. Brock in Biotechnology: A Textbook of Industrial Microbiology, 2nd ed., Sinauer Associates: Sunderland, MA (1989). All reagents, restriction enzymes and materials used for the growth and maintenance of microbial cells were obtained from Aldrich Chemicals (Milwaukee, WI), DIFCO Laboratories (Detroit, MI), New England Biolabs, Inc. (Beverly, MA), GIBCO/BRL (Gaithersburg, MD), or Sigma Chemical Company (St. Louis, MO), unless otherwise specified. *E. coli* strains were typically grown at 37 °C on Luria Bertani ["LB"] plates.

General molecular cloning was performed according to standard methods (Sambrook et al., *supra*). When PCR or site-directed mutagenesis was involved in subcloning, the constructs were sequenced to confirm that no errors had been introduced to the sequence. PCR products were cloned into Promega's pGEM-T-easy vector (Madison, WI).

The meaning of abbreviations is as follows: "sec" means second(s), "min" means minute(s), "h" means hour(s), "d" means day(s), "µL" means microliter(s), "mL" means milliliter(s), "L" means liter(s), "µM" means micromolar, "mM" means millimolar, "M" means molar, "mmol" means millimole(s), "µmole" mean micromole(s), "g" means gram(s), "µg" means microgram(s), "ng" means nanogram(s), "U" means unit(s), "bp" means base pair(s), "kB" means kilobase(s), "DCW" means dry cell weight, and "TFAs" means total fatty acids.

### Nomenclature For Expression Cassettes

The structure of an expression cassette will be represented by a simple notation system of "X::Y::Z", wherein X describes the promoter fragment, Y describes the gene fragment, and Z describes the terminator fragment, which are all operably linked to one another.

### Transformation And Cultivation Of Yarrowia lipolytica

*Yarrowia lipolytica* strain ATCC #20362 was purchased from the American Type Culture Collection (Rockville, MD). *Yarrowia lipolytica* strains were routinely grown at 28-30 °C in several media, according to the recipes shown below. Agar plates were prepared as required by addition of 20 g/L agar to each liquid media, according to standard methodology.
YPD agar medium (per liter): 10 g of yeast extract [Difco], 20 g of Bacto peptone [Difco], and 20 g of glucose.
Basic Minimal Media ["MM"] (per liter): 20 g glucose, 1.7 g yeast nitrogen base without amino acids, 1.0 g proline, and pH 6.1 (do not need to adjust).
Minimal Media + Uracil ["MM+uracil or MMU"] (per liter): Prepare MM media as above and add 0.1 g uracil and 0.1 g uridine.
Minimal Media + Uracil + Sulfonylurea ["MMU+SU"] (per liter): Prepare MMU media as above and add 280 mg sulfonylurea.
Minimal Media + Uracil + Lysine ["MMUraLys"] (per liter): Prepare MM media as above and add 0.1 g uracil, 0.1 g uridine. and 0.1 g lysine.
Minimal Media + 5-Fluoroorotic Acid ["MM + 5-FOA"] (per liter): 20 g glucose, 6.7 g Yeast Nitrogen base, 75 mg uracil, 75 mg uridine and appropriate amount of FOA (Zymo Research Corp., Orange, CA), based on FOA activity testing against a range of concentrations from 100 mg/L to 1000 mg/L (since variation occurs within each batch received from the supplier).
High Glucose Media ["HGM"] (per liter): 80 glucose, 2.58 g KH₂PO₄ and 5.36 g K₂HPO₄, pH 7.5 (do not need to adjust).
Fermentation medium without Yeast Extract ["FM without YE"] (per liter): 6.70 g/L Yeast nitrogen base, 6.00 g KH₂PO₄, 2.00 g K₂HPO₄, 1.50 g MgSO₄*7H₂O, and 20 g glucose.
Fermentation medium ["FM"] (per liter): 6.70 g/L Yeast nitrogen base, 6.00 g KH₂PO₄, 2.00 g K₂HPO₄, 1.50 g MgSO₄*7H₂O, 20 g glucose and 5.00 g Yeast extract (BBL).

Transformation of Y. *lipolytica* was performed as described in U.S. Pat. Appl. Pub. No. 2009-0093543-A1.

### Fatty Acid Analysis Of Yarrowia lipolytica

For fatty acid ["FA"] analysis, cells were collected by centrifugation and lipids were extracted as described in Bligh, E. G. & Dyer, W. J. (Can. J. Biochem. Physiol., 37:911-917 (1959)). Fatty acid methyl esters ["FAMEs"] were prepared by transesterification of the lipid extract with sodium methoxide (Roughan, G., and Nishida I., Arch Biochem Biophys., 276(1):38-46 (1990)) and subsequently analyzed with a Hewlett-Packard 6890 GC fitted with a 30-m X 0.25 mm (i.d.) HP-INNOWAX (Hewlett-Packard) column. The oven temperature was from 170 °C (25 min hold) to 185 °C at 3.5 °C/min.

For direct base transesterification, *Yarrowia* cells (0.5 mL culture) were harvested, washed once in distilled water, and dried under vacuum in a Speed-Vac for 5-10 min. Sodium methoxide (100 µl of 1 %) and a known amount of C15:0 triacylglycerol (C15:0 TAG; Cat. No. T-145, Nu-Check Prep, Elysian, MN) was added to the sample, and then the sample was vortexed and rocked for 30 min at 50 °C. After adding 3 drops of 1 M NaCl and 400 µl hexane, the sample was vortexed and spun. The upper layer was removed and analyzed by GC.

FAME peaks recorded via GC analysis were identified by their retention times, when compared to that of known fatty acids, and quantitated by comparing the FAME peak areas with that of the internal standard (C15:0 TAG) of known amount. Thus, the approximate amount (µg) of any fatty acid FAME ["µg FAME"] is calculated according to the formula: (area of the FAME peak for the specified fatty acid/ area of the standard FAME peak) * (µg of the standard C15:0 TAG), while the amount (µg) of any fatty acid ["µg FA"] is calculated according to the formula: (area of the FAME peak for the specified fatty acid/area of the standard FAME peak) * (µg of the standard C15:0 TAG) * 0.9503, since 1 µg of C15:0 TAG is equal to 0.9503 µg fatty acids. Note that the 0.9503 conversion factor is an approximation of the value determined for most fatty acids, which range between 0.95 and 0.96.

The lipid profile, summarizing the amount of each individual fatty acid as a weight percent of TFAs, was determined by dividing the individual FAME peak area by the sum of all FAME peak areas and multiplying by 100.

### Analysis Of Total Lipid Content And Composition In Yarrowia lipolytica By Flask Assay

For a detailed analysis of the total lipid content and composition in a particular strain of *Y. lipolytica,* flask assays were conducted as followed. Specifically, one loop of freshly streaked cells was inoculated into 3 mL FM medium and grown overnight at 250 rpm and 30 °C. The OD₆₀₀ₙₘ was measured and an aliquot of the cells were added to a final OD₆₀₀ₙₘ of 0.3 in 25 mL FM medium in a 125 mL flask. After 2 days in a shaker incubator at 250 rpm and at 30 °C, 6 mL of the culture was harvested by centrifugation and resuspended in 25 mL HGM in a 125 mL flask. After 5 days in a shaker incubator at 250 rpm and at 30 °C, a 1 mL aliquot was used for fatty acid analysis (*supra*) and 10 mL dried for dry cell weight ["DCW"] determination.

For DCW determination, 10 mL culture was harvested by centrifugation for 5 min at 4000 rpm in a Beckman GH-3.8 rotor in a Beckman GS-6R centrifuge. The pellet was resuspended in 25 mL of water and re-harvested as above. The washed pellet was re-suspended in 20 mL of water and transferred to a pre-weighed aluminum pan. The cell suspension was dried overnight in a vacuum oven at 80 °C. The weight of the cells was determined.

Total lipid content of cells ["TFAs % DCW"] is calculated and considered in conjunction with data tabulating the concentration of each fatty acid as a weight percent of TFAs ["% TFAs"] and the EPA content as a percent of the dry cell weight ["EPA % DCW"]. Data from flask assays will be presented as a table that summarizes the total dry cell weight of the cells ["DCW"], the total lipid content of cells ["TFAs % DCW"], the concentration of each fatty acid as a weight percent of TFAs ["% TFAs"] and the EPA content as a percent of the dry cell weight ["EPA % DCW"]. More specifically, fatty acids will be identified as 16:0 (palmitate), 16:1 (palmitoleic acid), 18:0 (stearic acid), 18:1 (oleic acid), 18:2 (LA), ALA, EDA, DGLA, ARA, ETrA, ETA, EPA and other.

### EXAMPLE 1

### Generation Of Yarrowia lipolytica Strain L135 (Ura3+, Leu-, Δpex3) To Produce About 46% DGLA Of Total Fatty Acids

The present Example describes the construction of strain L135, derived from *Yarrowia lipolytica* ATCC #20362, capable of producing about 46% DGLA relative to the total lipids via expression of a Δ9 elongase/Δ8 desaturase pathway.

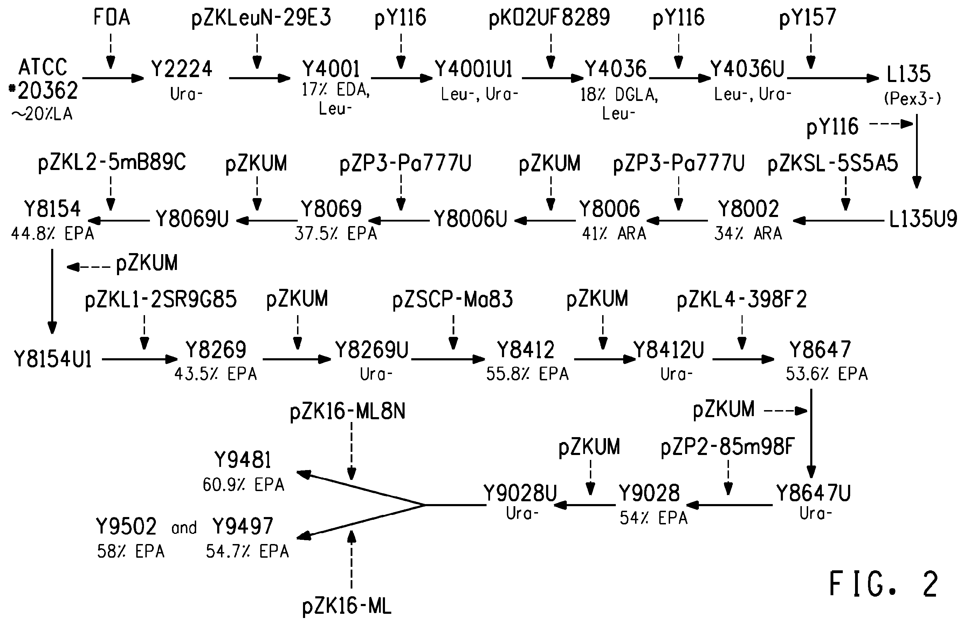

Briefly, as diagrammed in FIG. 2, strain L135 was derived from *Yarrowia lipolytica* ATCC #20362 via construction of strain Y2224 (a FOA resistant mutant from an autonomous mutation of the *Ura3* gene of wildtype *Yarrowia* strain ATCC #20362), strain Y4001 (producing 17% EDA with a *Leu-* phenotype), strain Y4001 U1 *(Leu-* and *Ura-*)*,* strain Y4036 (producing 18% DGLA with a Leu- phenotype) and strain Y4036U (L*eu-* and *Ura*-). Further details regarding the construction of strains Y2224, Y4001, Y4001 U, Y4036 and Y4036U are described in the General Methods of U.S. Pat. App. Pub. No. 2008-0254191.

The final genotype of strain Y4036U with respect to wild type *Yarrowia lipolytica* ATCC #20362 was *Ura3-,* YAT1::ME3S::Pex16, EXP1::EgD9eS::Lip1, FBAINm::EgD9eS::Lip2, GPAT::EgD9e::Lip2, FBAINm::EgDBM::Pex20, EXP1::EgDBM::Pex16, GPD::FmD12::Pex20, YAT1::FmD12::OCT (wherein FmD12 is a *Fusarium moniliforme* Δ12 desaturase gene [U.S. Patent 7,504,259]; ME3S is a codon-optimized C_{16/18} elongase gene, derived from *Mortierella alpina* [U.S. Patent 7,470,532]; EgD9e is a *Euglena gracilis* Δ9 elongase gene [U.S. Patent 7,645,604]; EgD9eS is a codon-optimized Δ9 elongase gene, derived from *Euglena gracilis* [U.S. Patent 7,645,604]; EgD8M is a synthetic mutant Δ8 desaturase [U.S. Patent 7,709,239], derived from *Euglena gracilis* [U.S. Patent 7,256,033]).

### Generation Of L135 Strain With Chromosomal Deletion Of Pex3

Construction of strain L135 is described in Example 12 of Intl. App. Pub. No. WO 2009/046248. Briefly, construct pY157 was used to knock out the chromosomal gene encoding the peroxisome biogenesis factor 3 protein [peroxisomal assembly protein Peroxin 3 or "Pex3p"] in strain Y4036U, thereby producing strain L135 (also referred to as strain Y4036 (Δpex3)). Knockout of the chromosomal Pex3 gene in strain L135, as compared to in strain Y4036 (whose native Pex3p had not been knocked out) resulted in the following: higher lipid content (TFAs % DCW) (ca. 6.0% versus 4.7%), higher DGLA % TFAs (46% versus 19%), higher DGLA % DCW (ca. 2.8% versus 0.9%) and reduced LA % TFAs (12% versus 30%). Additionally, the Δ9 elongase percent conversion efficiency was increased from ca. 48% in strain Y4036 to 83% in strain L135.

The final genotype of strain L135 with respect to wildtype *Yarrowia lipolytica* ATCC #20362 was *Ura3+, Leu-, Pex3-, unknownn1-,* YAT1::ME3S::Pex16, EXP1::EgD9eS::Lip1, FBAINm::EgD9eS::Lip2, GPAT::EgD9e::Lip2, FBAINm::EgD8M::Pex20, EXP1::EgDBM::Pex16, GPD::FmD12::Pex20, YAT1::FmD12::OCT.

### EXAMPLE 2

### Generation Of Yarrowia lipolytica Strains Producing From About 18% To About 41 % ARA Of Total Fatty Acids ["TFAs"]

The present Example describes the construction of strain Y8006, derived from *Yarrowia lipolytica* ATCC #20362, capable of producing about 41 % ARA relative to the total lipids via expression of a Δ9 elongase/Δ8 desaturase pathway.

The development of strain Y8006 (FIG. 2) required the construction of strains Y2224, Y4001, Y4001 U, Y4036, Y4036U and L135 (described in Example 1), as well as construction of strains L135U9 and Y8002.

### Generation of L135U9 (Leu-, Ura3-) Strain

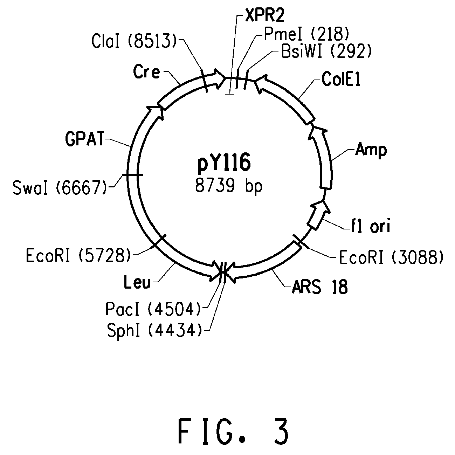

Strain L135U was created *via* temporary expression of the *Cre* recombinase enzyme in plasmid pY116 (FIG. 3; SEQ ID NO:127; described in Example 7 of Intl. App. Pub. No. WO 2008/073367) within strain L135 to produce a *Leu-* and *Ura-* phenotype. Plasmid pY116 was used for transformation of freshly grown L135 cells according to the General Methods. The transformant cells were plated onto MMLeuUra and maintained at 30 °C for 3 to 4 days. Three colonies were picked, inoculated into 3 mL liquid YPD media at 30 °C and shaken at 250 rpm/min for 1 day. The cultures were diluted to 1:50,000 with liquid MMLeuUra media, and 100 µL was plated onto new YPD plates and maintained at 30 °C for 2 days. Eight colonies were picked from each of three plates (24 colonies total) and streaked onto MMLeu and MMLeuUra selection plates. The colonies that could grow on MMLeuUra plates but not on MMLeu plates were selected and analyzed by GC to confirm the presence of C20:2 (EDA). One strain, having a *Leu-*and *Ura-* phenotype, was designated as L135U9.

### Generation Of Y8002 Strain To Produce About 32% ARA Of TFAs

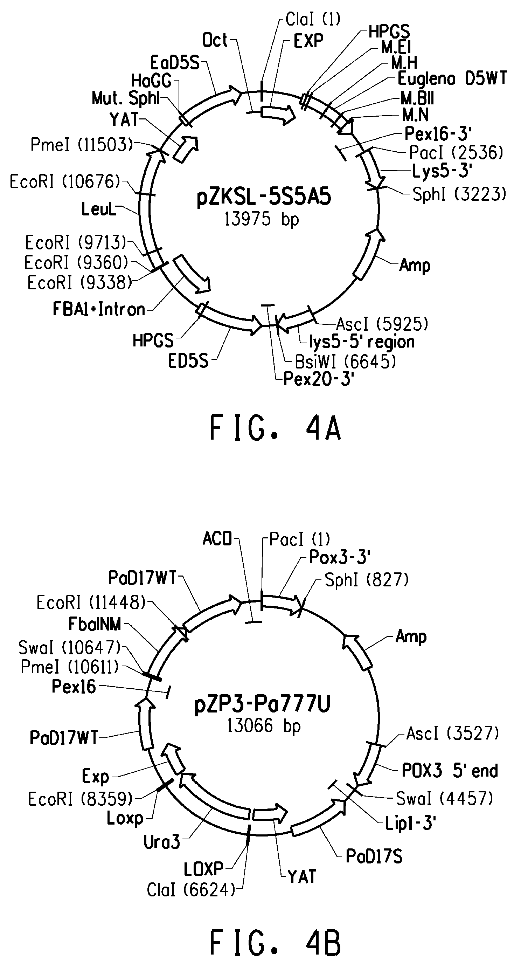

Construct pZKSL-5S5A5 (FIG. 4A; SEQ ID NO:128) was generated to integrate three Δ5 desaturase genes into the *Lys* loci of strain L135U9, to thereby enable production of ARA. The pZKSL-5S5A5 plasmid contained the following components:

**Table 6: Description of Plasmid pZKSL-5S5A5 (SEQ ID NO:128)**

| RE Sites And Nucleotides Within SEQ ID NO:128 | Description Of Fragment And Chimeric Gene Components |
|---|---|
| *Asc*I/*Bsi*WI (5925-6645) | 720 bp 5' portion of *Yarrowia Lys5* gene (GenBank Accession No. M34929; labeled as "lys5 5' region" in Figure) |
| *Pac*l/*Sph*l (2536-3225) | 689 bp 3' portion of *Yarrowia Lys5* gene (GenBank Accession No. M34929; labeled as "Lys5-3"' in Figure) |
| *EcoR*I /*BsiW*I (9338-6645) | FBAIN::EgD5SM::Pex20, comprising: |
| | • FBAIN: *Yarrowia lipolytica* FBAIN promoter (U.S. Patent 7,202,356); |
| | • EgD5SM: Synthetic mutant Δ5 desaturase (SEQ ID NO:71; U.S. Pat. Pub. No. 2010-0075386-A1), derived from *Euglena gracilis* (U.S. Patent 7,678,560) (labeled as "ED5S" in Figure); |
| | • Pex20: Pex20 terminator sequence from *Yarrowia Pex20* gene (GenBank Accession No. AF054613) |
| *Pme*I /*Cla*I (11503-1) | YAT1::EaD5SM::OCT, comprising: |
| | • YAT1: *Yarrowia lipolytica* YAT1 promoter (labeled as "YAT" in Figure; U.S. Pat. Appl. Pub. No. 2006-0094102-A1); |
| | • EaD5SM: Synthetic mutant Δ5 desaturase (SEQ ID NO:81; |
| | U.S. Pat. Pub. No. 2010-0075386-A1), derived from *Euglena anabaena* (U.S. Pat. Appl. Pub. No. 2008-0274521-A1) (labeled as "EaD5S" in Figure); |
| | • OCT: OCT terminator sequence of *Yarrowia* OCT gene (GenBank Accession No. X69988) |
| *Cla*I/*Pac*I (1-2536) | EXP1::EgD5M::Pex16, comprising: |
| | • EXP1: *Yarrowia lipolytica* export protein (EXP1) promoter (labeled as "EXP" in Figure; Intl. App. Pub. No. WO 2006/052870); |
| | • EgD5M: Mutant Δ5 desaturase (SEQ ID NO:69; U.S. Pat. Pub. No. 2010-0075386-A1) with elimination of internal *EcoR*I*, Bgl*II*, Hind*III and *Nco*I restriction enzyme sites, derived from *Euglena gracilis* (U.S. Patent 7,678,560) (labeled as "Euglena D5WT" in Figure); |
| | • Pex16: Pex16 terminator sequence from *Yarrowia Pex16* gene (GenBank Accession No. U75433) |
| *Eco*RI/*Pme*I (9360-11503) | *Yarrowia Leu2* gene (GenBank Accession No. M37309) |

The pZKSL-5S5A5 plasmid was digested with *Asc*I/*Sph*I*,* and then used for transformation of strain L135U9 according to the General Methods. The transformant cells were plated onto MMUraLys plates and maintained at 30 °C for 2 to 3 days. Single colonies were then re-streaked onto MMUraLys plates, and then inoculated into liquid MMUraLys at 30 °C and shaken at 250 rpm/min for 2 days. The cells were subjected to fatty acid analysis, according to the General Methods.

GC analyses showed the presence of ARA in the transformants containing the 3 chimeric genes of pZKSL-5S5A5, but not in the parent L135U9 strain. Five strains (i.e., #28, #62, #73, #84 and #95) that produced about 32.2%, 32.9%, 34.4%, 32.1% and 38.6% ARA of TFAs were designated as strains Y8000, Y8001, Y8002, Y8003 and Y8004, respectively. Further analyses showed that the three chimeric genes of pZKSL-5S5A5 were not integrated into the *Lys5* site in the Y8000, Y8001, Y8002, Y8003 and Y8004 strains. All strains possessed a *Lys*+ phenotype.

The final genotype of strains Y8000, Y8001, Y8002, Y8003 and Y8004 with respect to wildtype *Yarrowia lipolytica* ATCC #20362 was *Ura-, Pex3-, unknown 1-, unknown 2*-, *Leu*+, *Lys*+*,* YAT1::ME3S::Pex16, GPD::FmD12::Pex20, YAT1::FmD12::Oct, GPAT::EgD9e::Lip2, FBAINm::EgD9eS::Lip2, EXP1::EgD9eS::Lip1, FBAINm::EgD8M::Pex20, EXP1::EgDBM::Pex16, FBAIN::EgD5SM::Pex20, EXP1::EgD5M::Pex16, YAT1::EaD5SM::Oct.

### Generation Of Y8006 Strain To Produce About 41 % ARA Of TFAs

Construct pZP3-Pa777U (FIG. 4B; SEQ ID NO:129; described in Table 9 of U.S. Pat. Appl. Pub. No. 2009-0093543-A1) was generated to integrate three Δ17 desaturase genes into the *Pox3* loci (GenBank Accession No. AJ001301) of strain Y8002.

The pZP3-Pa777U plasmid was digested with *Asc*I*lSph*I*,* and then used for transformation of strain Y8002 according to the General Methods. The transformant cells were plated onto MM plates and maintained at 30 zu for 2 to 3 days. Single colonies were then re-streaked onto MM plates, and then inoculated into liquid MM at 30 °C and shaken at 250 rpm/min for 2 days. The cells were subjected to fatty acid analysis, according to the General Methods.

GC analyses showed the presence of 26% to 31 % EPA of TFAs in most of the selected 96 transformants containing the 3 chimeric genes of pZP3-Pa777U, but not in the parent Y8002 strain. Strain #69 produced about 38% EPA of TFAs and was designated as Y8007. There was one strain (i.e., strain #9) that did not produce EPA, but produced about 41% ARA of TFAs. This strain was designated as Y8006. Based on the lack of EPA production in strain Y8006, its genotype with respect to wildtype *Yarrowia lipolytica* ATCC #20362 is assumed to be *Pex3-, unknown 1-, unknown 2-, unknown 3-, Leu+, Lys*+*,* Ura+, YAT1::ME3S::Pex16, GPD::FmD12::Pex20, YAT1::FmD12::Oct, GPAT::EgD9e::Lip2, FBAINm::EgD9eS::Lip2, EXP1::EgD9eS::Lip1, FBAINm::EgD8M::Pex20, EXP1::EgDBM::Pex16, FBAIN::EgD5SM::Pex20, EXP1::EgD5M::Pex16, YAT1::EaD5SM::Oct.

In contrast, the final genotype of strain Y8007 with respect to wildtype *Yarrowia lipolytica* ATCC #20362 was *Pex3-, unknown 1-, unknown 2-, unknown 3-, Leu+, Lys*+, *Ura+,* YAT1::ME3S::Pex16, GPD::FmD12::Pex20, YAT1::FmD12::Oct, GPAT::EgD9e::Lip2, FBAINm::EgD9eS::Lip2, EXP1::EgD9eS::Lip1, FBAINm::EgD8M::Pex20, EXP1::EgDBM::Pex16, FBAIN::EgD5SM::Pex20, EXP1::EgD5M::Pex16, YAT1::EaD5SM::Oct, YAT1 ::PaD17S::Lip1, EXP1::PaD17::Pex16, FBAINm::PaD17::Aco (wherein PaD17 is a *Pythium aphanidermatum* Δ17 desaturase [U.S. Patent 7,556,949] and PaD17S is a codon-optimized Δ17 desaturase, derived from *Pythium aphanidermatum* [U.S. Patent 7,556,949].

Integration of the 3 chimeric genes of pZP3-Pa777U into the *Pox3* loci (GenBank Accession No. AJ001301) in strains Y8006 and Y8007 was not confirmed.

### EXAMPLE 3

### Generation Of Yarrowia lipolytica Strains Producing From About 24% To About 56% EPA Of Total Fatty Acids ["TFAs"]

The present Example describes the construction of strain Y8412, derived from *Yarrowia lipolytica* ATCC #20362, capable of producing about 56% EPA relative to the total lipids via expression of a Δ9 elongase/Δ8 desaturase pathway.

The development of strain Y8412 (FIG. 2) required the construction of strains Y2224, Y4001, Y4001 U, Y4036, Y4036U and L135 (described in Example 1), strains L135U9 and Y8002 (described in Example 2), and strains Y8006U6, Y8069, Y8069U, Y8154, Y8154U, Y8269 and Y8269U.

### Generation Of Strain Y8006U6 (Ura3-)

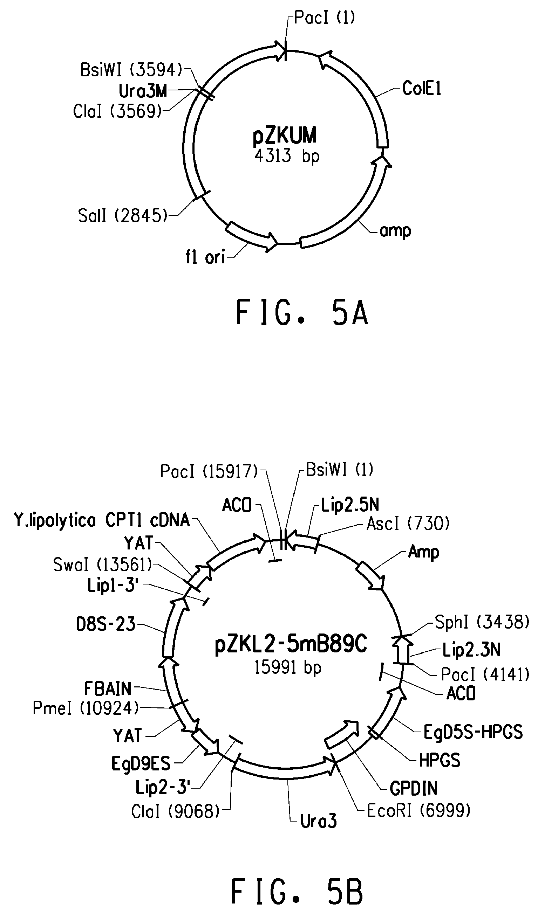

In order to disrupt the *Ura3* gene, construct pZKUM (FIG. 5A; SEQ ID NO:130; described in Table 15 of U.S. Pat. Appl. Pub. No. 2009-0093543-A1) was used to integrate a Ura3 mutant gene into the Ura3 gene of strain Y8006.

Plasmid pZKUM was digested with *Sal*I*lPac*I*,* and then used to transform strain Y8006 according to the General Methods. Following transformation, cells were plated onto MM + 5-FOA selection plates and maintained at 30 °C for 2 to 3 days.

A total of 8 transformants grown on MM + 5-FOA plates were picked and re-streaked onto MM plates and MM + 5-FOA plates, separately. All 8 strains had a *Ura-* phenotype (i.e., cells could grow on MM + 5-FOA plates, but not on MM plates). The cells were scraped from the MM + 5-FOA plates and subjected to fatty acid analysis, according to the General Methods.

GC analyses showed the presence of 22.9%, 25.5%, 23.6% 21.6%, 21.6% and 25% ARA in the pZKUM-transformant strains #1, #2, #4, #5, #6 and #7, respectively, grown on MM + 5-FOA plates. These six strains were designated as strains Y8006U1, Y8006U2, Y8006U3, Y8006U4, Y8006U5 and Y8006U6, respectively (collectively, Y8006U).

### Generation Of Y8069 Strain To Produce About 37.5% EPA Of TFAs

Construct pZP3-Pa777U (FIG. 4B; SEQ ID NO:129; described in Table 9 of U.S. Pat. Appl. Pub. No. 2009-0093543-A1) was used to integrate three Δ17 desaturase genes into the *Pox3* loci (GenBank Accession No. AJ001301) of strain Y8006U6.

The pZP3-Pa777U plasmid was digested with *Asc*I/*Sph*I*,* and then used for transformation of strain Y8006U6 according to the General Methods. The transformant cells were plated onto MM plates and maintained at 30 °C for 2 to 3 days. Single colonies were then re-streaked onto MM plates, and then inoculated into liquid MM at 30 °C and shaken at 250 rpm/min for 2 days. The cells were subjected to fatty acid analysis, according to the General Methods.

GC analyses showed the presence of EPA in the transformants containing the 3 chimeric genes of pZP3-Pa777U, but not in the parent Y8006U6 strain. Most of the selected 24 strains produced 24-37% EPA of TFAs. Four strains (i.e., #1, #6, #11 and #14) that produced 37.5%, 43.7%, 37.9% and 37.5% EPA of TFAs were designated as Y8066, Y8067, Y8068 and Y8069, respectively. Integration of the 3 chimeric genes of pZP3-Pa777U into the *Pox3* loci (GenBank Accession No. AJ001301) of strains Y8066, Y8067, Y8068 and Y8069 was not confirmed.

The final genotype of strains Y8066, Y8067, Y8068 and Y8069 with respect to wildtype *Yarrowia lipolytica* ATCC #20362 was Ura+, *Pex3-, unknown 1-, unknown 2-, unknown 3-, unknown 4-, Leu+, Lys*+*,* YAT1::ME3S::Pex16, GPD::FmD12::Pex20, YAT1::FmD12::Oct, GPAT::EgD9e::Lip2, FBAINm::EgD9eS::Lip2, EXP1::EgD9eS::Lip1, FBAINm::EgD8M::Pex20, EXP1::EgDBM::Pex16, FBAIN::EgD5SM::Pex20, EXP1::EgD5M::Pex16, YAT1::EaD5SM::Oct, YAT1::PaD17S::Lip1, EXP1::PaD17::Pex16, FBAINm::PaD17::Aco.

### Generation Of Strain Y8069U (Ura3-)

In order to disrupt the *Ura3* gene, construct pZKUM (FIG. 5A; SEQ ID NO:130; described in Table 15 of U.S. Pat. Appl. Pub. No. 2009-0093543-A1) was used to integrate a *Ura3* mutant gene into the *Ura3* gene of strain Y8069, in a manner similar to that described for pZKUM transformation of strain Y8006 *(supra).* A total of 3 transformants were grown and identified to possess a *Ura-* phenotype.

GC analyses showed the presence of 22.4%, 21.9% and 21.5% EPA in the pZKUM-transformant strains #1, #2 and #3, respectively, grown on MM + 5-FOA plates. These three strains were designated as strains Y8069U1, Y8069U2, and Y8069U3, respectively (collectively, Y8069U).

### Generation Of Strain Y8154 To Produce about 44.8% EPA Of TFAs

Construct pZKL2-5mB89C (FIG. 5B; SEQ ID NO:131) was generated to integrate one Δ5 desaturase gene, one Δ9 elongase gene, one Δ8 desaturase gene, and one *Yarrowia lipolytica* diacylglycerol cholinephosphotransferase gene (CPT1) into the *Lip2* loci (GenBank Accession No. AJ012632) of strain Y8069U3 to thereby enable higher level production of EPA. The pZKL2-5mB89C plasmid contained the following components:

**Table 7: Description of Plasmid pZKL2-5mB89C (SEQ ID NO:131)**

| RE Sites And Nucleotides Within SEQ ID NO:131 | Description Of Fragment And Chimeric Gene Components |
|---|---|
| *Asc*I/*Bsi*WI (730-1) | 722 bp 5' portion of *Yarrowia Lip2* gene (labeled as "Lip2.5N" in Figure; GenBank Accession No. AJ012632) |
| *Pac*I/*Sph*I (4141-3438) | 697 bp 3' portion of *Yarrowia Lip2* gene (labeled as "Lip2.3N" in Figure; GenBank Accession No. AJ012632) |
| *Swa*I/*Bsi*WI (13561-1) | YAT1::YICPT1::Aco, comprising: |
| | • YAT1: *Yarrowia lipolytica* YAT1 promoter (labeled as "YAT" in Figure; U.S. Pat. Appl. Pub. No. 2006-0094102-A1); |
| | • YICPT1: *Yarrowia lipolytica* diacylglycerol cholinephosphotransferase gene (SEQ ID NO:37) (labeled as "Y. lipolytica CPT1 cDNA" in Figure; Intl. App. Pub. No. WO 2006/052870); |
| | • Aco: Aco terminator sequence from *Yarrowia Aco* gene (GenBank Accession No. AJ001300) |
| *Pme*I/*Swa*I (10924-13561) | FBAIN::EgD8M::Lip1 comprising: |
| | • FBAIN: *Yarrowia lipolytica* FBAIN promoter (U.S. Patent 7,202,356); |
| | • EgD8M: Synthetic mutant Δ8 desaturase (SEQ ID NO:59; U.S. Patent 7,709,239), derived from *Euglena gracilis* ("EgD8S"; U.S. Patent 7,256,033) (labeled as "D8S-23" in Figure); |
| | • Lip1: Lip1 terminator sequence from *Yarrowia Lip1* gene (GenBank Accession No. Z50020) |
| *Pme*I/*Cla*I (10924-9068) | YAT1::EgD9eS::Lip2, comprising: |
| | • YAT1: *Yarrowia lipolytica* YAT1 promoter (labeled as "YAT" in Figure; U.S. Pat. Appl. Pub. No. 2006-0094102-A1); |
| | • EgD9eS: codon-optimized Δ9 elongase (SEQ ID NO:45), derived from *Euglena gracilis* (U.S. Patent 7,645,604); |
| | • Lip2: Lip2 terminator sequence from *Yarrowia Lip2* gene (GenBank Accession No. AJ012632) |
| *Cla*I/EcoRI (9068-6999) | *Yarrowia* Ura3 gene (GenBank Accession No. AJ306421) |
| *Eco*RI/*Pac*I (6999-4141) | GPDIN::EgD5SM::ACO, comprising: |
| | • GPDIN: *Yarrowia lipolytica* GPDIN promoter (U.S. Patent 7,459,546); |
| | • EgD5SM: Synthetic mutant Δ5 desaturase (SEQ ID NO:71; U.S. Pat. Pub. No. 2010-0075386-A1), derived from *Euglena gracilis* (U.S. Patent 7,678,560) (labeled as "EgD5S-HPGS" in Figure); |
| | • Aco: Aco terminator sequence from *Yarrowia Aco* gene (GenBank Accession No. AJ001300) |

The pZKL2-5mB89C plasmid was digested with *Asc*l/*Sph*l*,* and then used for transformation of strain Y8069U3 according to the General Methods. The transformant cells were plated onto MM plates and maintained at 30 °C for 3 to 4 days. Single colonies were re-streaked onto MM plates, and then inoculated into liquid MM at 30 °C and shaken at 250 rpm/min for 2 days. The cells were collected by centrifugation, resuspended in HGM and then shaken at 250 rpm/min for 5 days. The cells were subjected to fatty acid analysis, according to the General Methods.

GC analyses showed that most of the selected 96 strains produced approximately 38-44% EPA of TFAs. Seven strains (i.e., #1, #39, #49, #62, #70, #85 and #92) that produced about 44.7%, 45.2%, 45.4%, 44.8%, 46.1 %, 48.6% and 45.9% EPA of TFAs were designated as strains Y8151, Y8152, Y8153, Y8154, Y8155, Y8156 and Y8157, respectively. Knockout of the Lip2 gene was not confirmed in these EPA strains.

The final genotype of strains Y8151, Y8152, Y8153, Y8154, Y8155, Y8156 and Y8157 with respect to wildtype *Yarrowia lipolytica* ATCC #20362 was *Ura+, Pex3-, unknown 1-, unknown* 2-, *unknown 3-, unknown 4-, unknown* 5-, *Leu+, Lys+,* YAT1::ME3S::Pex16, FBAINm::EgD9eS::Lip2, EXP1::EgD9eS::Lip1, GPAT::EgD9e::Lip2, YAT1::EgD9eS::Lip2, FBAINm::EgD8M::Pex20, EXP1::EgD8M::Pex16, FBAIN::EgD8M::Lip1, GPD::FmD12::Pex20, YAT1::FmD12::Oct, EXP1::EgD5M::Pex16, YAT1::EaD5SM::Oct, FBAIN::EgD5SM::Pex20, GPDIN::EgD5SM::Aco, FBAINm::PaD17::Aco, EXP1::PaD17::Pex16, YAT1 ::PaD17S::Lip1, YAT1::YICPT::Aco.

### Generation Of Strain Y8154U1 (Ura3-)

In order to disrupt the *Ura3* gene, construct pZKUM (FIG. 5A; SEQ ID NO:130; described in Table 15 of U.S. Pat. Appl. Pub. No. 2009-0093543-A1) was used to integrate a *Ura3* mutant gene into the *Ura3* gene of strain Y8154, in a manner similar to that described for pZKUM transformation of strain Y8006 (*supra*). A total of 8 transformants were grown and identified to possess a *Ura-* phenotype.

GC analyses showed that there was 23.1 % EPA of TFAs in the pZKUM-transformant strain #7. This strain was designated as strain Y8154U1.

### Generation Of Strain Y8269 To Produce About 45.3% EPA Of TFAs

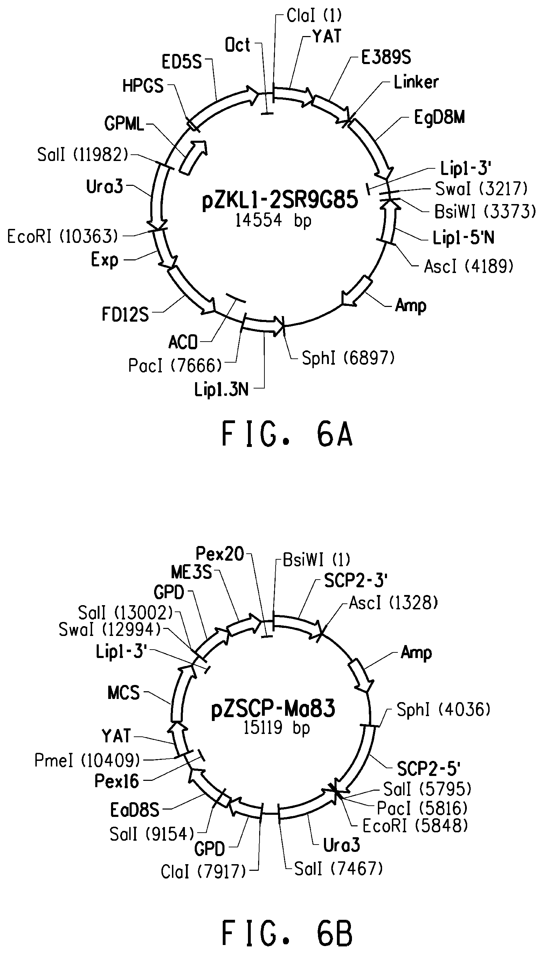

Construct pZKL1-2SR9G85 (FIG. 6A; SEQ ID NO:132) was generated to integrate one DGLA synthase, one Δ12 desaturase gene and one Δ5 desaturase gene into the *Lip1* loci (GenBank Accession No. Z50020) of strain Y8154U1 to thereby enable higher level production of EPA. A DGLA synthase is a multizyme comprising a Δ9 elongase linked to a Δ8 desaturase.

The pZKL1-2SR9G85 plasmid contained the following components:

**Table 8: Description of Plasmid pZKL1-2SR9G85 (SEQ ID NO:132)**

| RE Sites And Nucleotides Within SEQ ID NO:132 | Description Of Fragment And Chimeric Gene Components |
|---|---|
| *Asc*I/*Bsi*WI (4189-3373) | 809 bp 5' portion of *Yarrowia Lip1* gene (labeled as "Lip1-5'N" in Figure; GenBank Accession No. Z50020) |
| *Pac*I/*Sph*I (7666-6879) | 763 bp 3' portion of *Yarrowia Lip1* gene (labeled as "Lip1.3N" in Figure; GenBank Accession No. Z50020) |
| *Cla*I/*Swa*I (1-3217) | YAT1::E389D9eS/EgDBM::Lip1, comprising: |
| | • YAT1: *Yarrowia lipolytica* YAT1 promoter (labeled as "YAT" in Figure; U.S. Pat. Appl. Pub. No. 2006-0094102-A1); |
| | • E389D9eS/EgD8M: gene fusion comprising a codon-optimized Δ9 elongase derived from *Eutreptiella* sp. CCMP389 ("E389D9eS"), a linker, and the synthetic mutant Δ8 desaturase derived from *Euglena gracilis* ("EgD8M") (SEQ ID NO:12) (labeled as individually as "E389S", "Linker" and "EgD8M" in Figure; U.S. Pat. Appl. Pub. No. 2008-0254191-A1); |
| | • Lip1: Lip1 terminator sequence from *Yarrowia Lip1* gene (GenBank Accession No. Z50020) |
| *Sal*I/*Cla*I (11982-1) | GPM::EgD5SM::Oct comprising: |
| | • GPM: *Yarrowia lipolytica* GPM promoter (labeled as "GPML" in Figure; U.S. Patent 7,202,356); |
| | • EgD5SM: Synthetic mutant Δ5 desaturase (SEQ ID NO:71; U.S. Pat. Pub. No. 2010-0075386-A1), derived from *Euglena gracilis* (U.S. Patent 7,678,560) (labeled as "ED5S" in Figure); |
| | • OCT: OCT terminator sequence of *Yarrowia* OCT gene (GenBank Accession No. X69988) |
| *Sal*I/*Eco*RI (11982-10363) | *Yarrowia* Ura3 gene (GenBank Accession No. AJ306421) |
| *EcoR*I/*Pac*I (10363-7666) | EXP1::FmD12S::ACO, comprising: |
| | • EXP1: *Yarrowia lipolytica* export protein (EXP1) promoter (labeled as "Exp" in Figure; Intl. App. Pub. No. WO 2006/052870); |
| | • FmD12S: codon-optimized Δ12 elongase (SEQ ID NO:93), derived from *Fusarium moniliforme* (labeled as "FD12S" in Figure; U.S. Patent 7,504,259); |
| | • Aco: Aco terminator sequence from *Yarrowia Aco* gene (GenBank Accession No. AJ001300) |

The pZKL1-2SR9G85 plasmid was digested with *Asc*I/*Sph*I*,* and then used for transformation of strain Y8154U1 according to the General Methods. The transformant cells were plated onto MM plates and maintained at 30 °C for 3 to 4 days. Single colonies were re-streaked onto MM plates, and then inoculated into liquid MM at 30 °C and shaken at 250 rpm/min for 2 days. The cells were collected by centrifugation, resuspended in HGM and then shaken at 250 rpm/min for 5 days. The cells were subjected to fatty acid analysis, according to the General Methods.

GC analyses showed that most of the selected 96 strains produced 40-44.5% EPA of total lipids. Five strains (i.e., #44, #46, #47, #66 and #87) that produced about 44.8%, 45.3%, 47%, 44.6% and 44.7% EPA of TFAs were designated as Y8268, Y8269, Y8270, Y8271 and Y8272, respectively. Knockout of the *Lip1* loci (GenBank Accession No. Z50020) was not confirmed in these EPA strains.

The final genotype of strains Y8268, Y8269, Y8270, Y8271 and Y8272 with respect to wildtype *Yarrowia lipolytica* ATCC #20362 was Ura+, *Pex3-, unknown 1-, unknown 2-*, *unknown 3-, unknown 4-, unknown 5-, unknown6-,* YAT1::ME3S::Pex16, FBAINm::EgD9eS::Lip2, EXP1::EgD9eS::Lip1, GPAT::EgD9e::Lip2, YAT1::EgD9eS::Lip2, FBAINm::EgD8M::Pex20, EXP1::EgD8M::Pex16, FBAIN::EgD8M::Lip1, YAT1::E389D9eS/EgD8M::Lip1, GPD::FmD12::Pex20, YAT1::FmD12::Oct, EXP1::FmD12S::Aco, EXP1::EgD5M::Pex16, YAT1::EaD5SM::Oct, FBAIN::EgD5SM::Pex20, GPDIN::EgD5SM::Aco, GPM::EgD5SM::Oct, FBAINm::PaD17::Aco, EXP1::PaD17::Pex16, YAT1::PaD17S::Lip1, YAT1::YICPT::Aco.

### Generation Of Strain Y8269U (Ura3-)

In order to disrupt the *Ura3* gene, construct pZKUM (FIG. 5A; SEQ ID NO:130; described in Table 15 of U.S. Pat. Appl. Pub. No. 2009-0093543-A1) was used to integrate a *Ura3* mutant gene into the *Ura3* gene of strain Y8269, in a manner similar to that described for pZKUM transformation of strain Y8006 (*supra*). A total of 8 transformants were grown and identified to possess a *Ura-* phenotype.

GC analyses showed that there were 23.0%, 23.1% and 24.2% EPA of TFAs in pZKUM-transformant strains #2, #3 and #5, respectively. These strains were designated as strains Y8269U1, Y8269U2 and Y8269U3, respectively (collectively, Y8269U).

### Generation Of Strain Y8406 And Strain Y8412 To Produce About 51.2% EPA And 55.8% EPA Of TFAs

Construct pZSCP-Ma83 (FIG. 6B; SEQ ID NO:133) was generated to integrate one Δ8 desaturase gene, one C_{16/18} elongase gene and one malonyl-CoA synthetase gene into the *SCP2* loci (GenBank Accession No. XM_503410) of strain Y8269U1 to thereby enable higher level production of EPA. The pZSCP-Ma83 plasmid contained the following components:

**Table 9: Description of Plasmid pZSCP-Ma83 (SEQ ID NO:133)**

| RE Sites And Nucleotides Within SEQ ID NO:133 | Description Of Fragment And Chimeric Gene Components |
|---|---|
| *Bsi*WI/*Asc*I (1-1328) | 1327 bp 3' portion of *Yarrowia SCP2* gene (labeled as "SCP2-3"' in Figure; GenBank Accession No. XM_503410) |
| *Sph*I/*Pac*I (4036-5816) | 1780 bp 5' portion of *Yarrowia SCP2* gene (labeled as "SCP2-5"' in Figure; GenBank Accession No. XM_503410) |
| *Swa*I/*Bsi*WI (12994-1) | GPD::ME3S::Pex20, comprising: |
| | • GPD: *Yarrowia lipolytica* GPD promoter (U.S. Patent 7,259,255); |
| | • ME3S: codon-optimized C_{16/18} elongase gene (SEQ ID NO:97), derived from *M*. *alpina* (U.S. Patent 7,470,532); |
| | • Pex20: Pex20 terminator sequence from *Yarrowia Pex20* gene (GenBank Accession No. AF054613) |
| *Pme*I/*Swa*I (10409-12994) | YAT1::MCS::Lip1 comprising: |
| | • YAT1: *Yarrowia lipolytica* YAT1 promoter (labeled as "YAT" in Figure; U.S. Pat. Appl. Pub. No. 2006/0094102-A1); |
| | • MCS: codon-optimized malonyl-CoA synthetase gene (SEQ ID NO:41), derived from *Rhizobium leguminosarum* bv. viciae 3841 (U.S. Patent Application No. 12/637877); |
| | • Lip1: Lip1 terminator sequence from *Yarrowia Lip1* gene (GenBank Accession No. Z50020) |
| *Cla*I/*Pme*I (7917-10409) | GPD::EaD8S::Pex16 comprising: |
| | • GPD: *Yarrowia lipolytica* GPD promoter (U.S. Patent 7,259,255); |
| | • EaD8S: codon-optimized Δ8 desaturase gene (SEQ ID NO:63), derived from *Euglena anabaena* (U.S. Pat. Appl. Pub. No. 2008-0254521-A1); |
| | • Pex16: Pex16 terminator sequence from *Yarrowia Pex16* gene (GenBank Accession No. U75433) |
| *Sal*I/*Eco*RI (7467-5848) | *Yarrowia* Ura3 gene (GenBank Accession No. AJ306421) |

The pZSCP-Ma83 plasmid was digested with *Asc*I/*Sph*I*,* and then used for transformation of strains Y8269U1, Y8269U2 and Y8269U3, separately, according to the General Methods. The transformant cells were plated onto MM plates and maintained at 30 °C for 3 to 4 days. Single colonies were re-streaked onto MM plates, and then inoculated into liquid MM at 30 °C and shaken at 250 rpm/min for 2 days. The cells were collected by centrifugation, resuspended in HGM and then shaken at 250 rpm/min for 5 days. The cells were subjected to fatty acid analysis, according to the General Methods.

A total of 96 strains resulting from each pZSCP-Ma83 transformation (i.e., into Y8269U1, Y8269U2 and Y8269U3) were analyzed by GC. Most of the selected 288 strains produced 43-47% EPA of TFAs. Seven strains of Y8269U1 transformed with pZSCP-Ma83 (i.e., #59, #61, #65, #67, #70, #81 and #94) that produced about 51.3%, 47.9%, 50.8%, 48%, 47.8%, 47.8% and 47.8% EPA of TFAs were designated as strains Y8404, Y8405, Y8406, Y8407, Y8408, Y8409 and Y8410, respectively. Three strains of Y8269U2 transformed with pZSCP-Ma83 (i.e., #4, #13 and #17) that produced about 48.8%, 50.8%, and 49.3% EPA of TFAs were designated as Y8411, Y8412 and Y8413, respectively. And, two strains of Y8269U3 transformed with pZSCP-Ma83 (i.e., #2 and #16) that produced about 49.3% and 53.5% EPA of TFAs were designated as Y8414 and Y8415, respectively.

Knockout of the *SCP2* loci (GenBank Accession No. XM_503410) in strains Y8404, Y8405, Y8406, Y8407, Y8408, Y8409, Y8410, Y8411, Y8412, Y8413, Y8414 and Y8415 was not confirmed in any of these EPA strains, produced by transformation with pZSCP-Ma83.

The final genotype of strains Y8404, Y8405, Y8406, Y8407, Y8408, Y8409, Y8410, Y8411, Y8412, Y8413, Y8414 and Y8415 with respect to wildtype *Yarrowia lipolytica* ATCC #20362 was *Ura+, Pex3-, unknown 1-, unknown* 2-, *unknown 3-, unknown 4-, unknown 5-, unknown6-, unknown* 7-, YAT1::ME3S::Pex16, GPD::ME3S::Pex20, FBAINm::EgD9eS::Lip2, EXP1::EgD9eS::Lip1, GPAT::EgD9e::Lip2, YAT1::EgD9eS::Lip2, FBAINm::EgD8M::Pex20, EXP1::EgD8M::Pex16, FBAIN::EgD8M::Lip1, GPD::EaD8S::Pex16, YAT1::E389D9eS/EgD8M::Lip1, GPD::FmD12::Pex20, YAT1::FmD12::Oct, EXP1::FmD12S::Aco, EXP1::EgD5M::Pex16, YAT1::EaD5SM::Oct, FBAIN::EgD5SM::Pex20, GPDIN::EgD5SM::Aco, GPM::EgD5SM::Oct, FBAINm::PaD17::Aco, EXP1::PaD17::Pex16, YAT1 ::PaD17S::Lip1, YAT1::YICPT::Aco, YAT1::MCS::Lip1.

*Yarrowia lipolytica* strain Y8406 was deposited with the American Type Culture Collection on May 14, 2009 and bears the designation ATCC PTA-10025. *Yarrowia lipolytica* strain Y8412 was deposited with the American Type Culture Collection on May 14, 2009 and bears the designation ATCC PTA-10026.

### Analysis Of Total Lipid Content And Composition By Flask Assay

Cells from YPD plates of strains Y8404, Y8405, Y8406, Y8407, Y8408, Y8409, Y8410, Y8411, Y8412, Y8413, Y8414 and Y8415 were grown and analyzed for total lipid content and composition, according to the General Methods. Table 10 summarizes the total dry cell weight of the cells ["DCW'], the total lipid content of cells ["TFAs % DCW"], the concentration of each fatty acid as a weight percent of TFAs ["% TFAs"] and the EPA content as a percent of the dry cell weight ["EPA % DCW"]. More specifically, fatty acids are identified as 16:0 (palmitate), 16:1 (palmitoleic acid), 18:0 (stearic acid), 18:1 (oleic acid), 18:2 (LA), ALA, EDA, DGLA, ARA, ETrA, ETA, EPA and other.

**Table 10: Total Lipid Content And Composition In Yarrowia Strains Y8404, Y8405, Y8406, Y8407, Y8408, Y8409, Y8410, Y8411, Y8412, Y8413, Y8414 And Y8415 By Flask Assay**

| Strain | DCW (g/L) | TFAs % DCW | % TFAs | | | | | | | | | | | | | EPA % DCW |
|---|---|---|---|---|---|---|---|---|---|---|---|---|---|---|---|---|
| | | | 16:0 | 16:1 | 18:0 | 18:1 | 18:2 | ALA | EDA | DGLA | ARA | EtrA | ETA | EPA | other | |
| Y8404 | 4.1 | 27.3 | 2.8 | 0.8 | 1.8 | 5.1 | 20.4 | 2.1 | 2.9 | 2.5 | 0.6 | 0.8 | 2.4 | 51.1 | 6.3 | 14.0 |
| Y8405 | 3.9 | 29.6 | 2.7 | 0.5 | 2.9 | 5.7 | 20.5 | 2.8 | 2.7 | 2.1 | 0.5 | 0.7 | 2.0 | 51.4 | 5.1 | 15.2 |
| **Y8406** | **4.0** | **30.7** | **2.6** | **0.5** | **2.9** | **5.7** | **20.3** | **2.8** | **2.8** | **2.1** | **0.5** | **0.8** | **2.1** | **51.2** | **5.4** | **15.7** |
| Y8407 | 4.0 | 29.4 | 2.6 | 0.5 | 3.0 | 5.6 | 20.5 | 2.8 | 2.7 | 2.1 | 0.4 | 0.7 | 2.1 | 51.5 | 5.1 | 15.2 |
| Y8408 | 4.1 | 29.8 | 2.9 | 0.6 | 2.7 | 5.7 | 20.2 | 2.8 | 2.6 | 2.1 | 0.5 | 0.9 | 2.1 | 51.2 | 5.5 | 15.3 |
| Y8409 | 3.9 | 30.8 | 2.8 | 0.5 | 2.9 | 5.7 | 20.6 | 2.7 | 2.7 | 2.1 | 0.5 | 0.8 | 2.1 | 51.0 | 5.2 | 15.7 |
| Y8410 | 4.0 | 31.8 | 2.7 | 0.5 | 3.0 | 5.7 | 20.5 | 2.9 | 2.7 | 2.1 | 0.5 | 0.7 | 2.1 | 50.9 | 5.3 | 16.2 |
| Y8411 | 3.6 | 30.5 | 2.7 | 0.3 | 3.3 | 5.1 | 19.9 | 2.6 | 2.4 | 2.0 | 0.5 | 0.6 | 1.8 | 52.9 | 5.7 | 16.1 |
| **Y8412** | **3.2** | **27.0** | **2.5** | **0.4** | **2.6** | **4.3** | **19.0** | **2.4** | **2.2** | **2.0** | **0.5** | **0.6** | **1.9** | **55.8** | **5.6** | **15.1** |
| Y8413 | 2.9 | 27.2 | 3.1 | 0.4 | 2.6 | 5.4 | 19.9 | 2.2 | 2.8 | 2.0 | 0.5 | 0.7 | 1.8 | 52.4 | 5.9 | 14.2 |
| Y8414 | 3.7 | 27.1 | 2.5 | 0.7 | 2.3 | 6.0 | 19.9 | 1.6 | 3.4 | 3.4 | 0.6 | 0.6 | 3.1 | 49.4 | 6.1 | 13.4 |
| Y8415 | 3.6 | 25.9 | 1.4 | 0.3 | 1.9 | 4.5 | 16.0 | 1.3 | 2.7 | 2.9 | 0.5 | 0.6 | 2.5 | 59.0 | 6.1 | 15.3 |

### EXAMPLE 4

### Generation Of Yarrowia lipolytica Strain Y8647 To Produce About 53.6% EPA Of Total Fatty Acids ["TFAs"] With 37.6% Total Lipid Content

The present Example describes the construction of strain Y8647, derived from *Yarrowia lipolytica* ATCC #20362, capable of producing about 53.6% EPA relative to the total lipids with 37.6% total lipid content ["TFAs % DCW"] via expression of a Δ9 elongase/Δ8 desaturase pathway. The development of strain Y8647 (FIG. 2) required the construction of strains Y2224, Y4001, Y4001 U, Y4036, Y4036U and L135 (described in Example 1), strains L135U9 and Y8002 (described in Example 2), strains Y8006U6, Y8069, Y8069U, Y8154, Y8154U, Y8269 and Y8269U (described in Example 3) and strain Y8412U6.

### Generation Of Strain Y8412U6 (Ura3-)

In order to disrupt the *Ura3* gene, construct pZKUM (FIG. 5A; SEQ ID NO:130; described in Table 15 of U.S. Pat. Appl. Pub. No. 2009-0093543-A1) was used to integrate a *Ura3* mutant gene into the *Ura3* gene of strain Y8412 (Example 3) in a manner similar to that described for pZKUM transformation of strain Y8006 (Example 3). A total of 8 transformants were grown and identified to possess a *Ura-* phenotype.

GC analyses showed that there were 25.9% and 26.9% EPA of TFAs in pZKUM-transformant strains #4 and #6, respectively. These two strains were designated as strains Y8412U6 and Y8412U8, respectively (collectively, Y8412U).

### Generation Of Strain Y8647

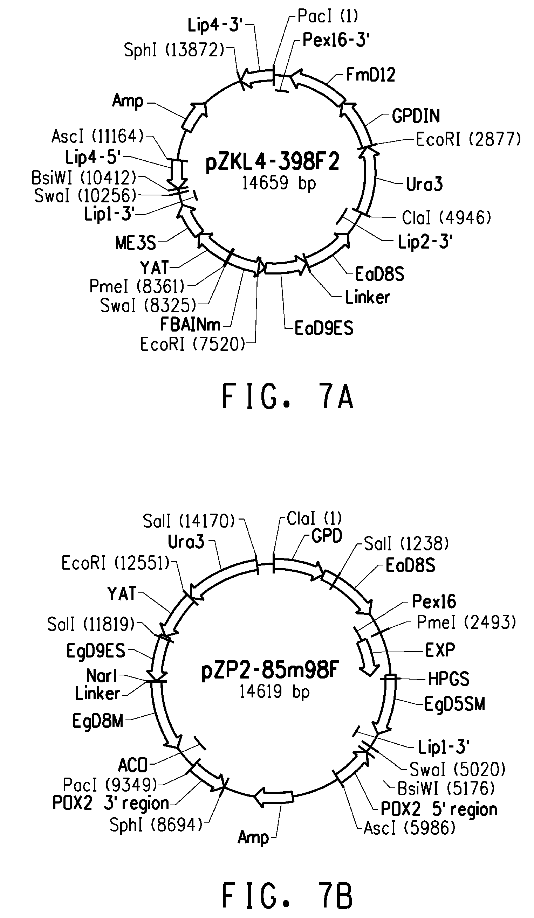

Construct pZKL4-398F2 (FIG. 7A; SEQ ID NO:134) was generated to integrate one C_{16/18} elongase gene, one DGLA synthase, and one Δ12 desaturase gene into the *Yarrowia* lipase-like locus (designated as *Lip4,* GenBank Accession No. XM_503825) of strain Y8412U6 to thereby enable higher level production of EPA. The pZKL4-398F2 plasmid contained the following components:

**Table 11: Description of Plasmid pZKL4-398F2 (SEQ ID NO:134)**

| RE Sites And Nucleotides Within SEQ ID NO:134 | Description Of Fragment And Chimeric Gene Components |
|---|---|
| *Asc*I / *Bsi*WI (11164-10412) | 745 bp 5' portion of *Yarrowia Lipase* 4 locus (labeled as "Lip4" in Figure; GenBank Accession No. XM_503825) |
| *Pac*I / *Sph*I (1-13872) | 782 bp 3' portion of *Yarrowia Lipase* 4 locus (labeled as "Lip4-3"' in Figure; GenBank Accession No. XM_503825) |
| *EcoR*I/*Pac*I (2877-1) | GPDIN::FmD12::Pex16, comprising: |
| | • GPDIN: *Yarrowia lipolytica* GPDIN promoter (U.S. Patent 7,459,546); |
| | • FmD12: *Fusarium moniliforme* Δ12 desaturase (SEQ ID NO:91) (labeled as "F.D12" in Figure; U.S. Patent 7,504,259); |
| | • Pex16: Pex16 terminator sequence from *Yarrowia Pex16* gene (GenBank Accession No. U75433) |
| *Pme*I/*Swa*I (8361-10256) | YAT1::ME3S::Lip1 comprising: |
| | • YAT1: *Yarrowia lipolytica* YAT1 promoter (labeled as "YAT" in Figure; U.S. Pat. Appl. Pub. No. 2006-0094102-A1); |
| | • ME3S: codon-optimized C_{16/18} elongase gene (SEQ ID NO:97), derived from M. *alpina* (U.S. Patent 7,470,532); |
| | • Lip1: Lip1 terminator sequence from *Yarrowia Lip1* gene (GenBank Accession No. Z50020) |
| SwaI/*Cl*aI (8325-4946) | FBAINm::EaD9eS/EaD8S::Lip2 comprising: |
| | • FBAINm: *Yarrowia lipolytica* FBAINm promoter (U.S. Patent 7,202,356); |
| | • EaD9eS/EaD8S: gene fusion comprising a codon-optimized Δ9 elongase derived from *Euglena anabaena* ("EaD9eS"), a linker, and a codon-optimized Δ8 desaturase derived from *Euglena anabaena* ("EaD8S") (SEQ ID NO:63) (labeled as individually as "EaD9E9S", "Linker" and "EaD8S" in Figure; U.S. Pat. Appl. Pub. No. 2008-0254191-A1); |
| | • Lip2: Lip2 terminator sequence from *Yarrowia Lip2* gene (GenBank Accession No. AJ012632) |
| *C*/*a*I/*Eco*RI (49146-2877) | *Yarrowia* Ura3 gene (GenBank Accession No. AJ306421) |

The pZKL4-398F2 plasmid was digested with *Asc*I/*Sph*I*,* and then used for transformation of strain Y8412U6, according to the General Methods. The transformant cells were plated onto MM plates and maintained at 30 °C for 3 to 4 days. Single colonies were re-streaked onto MM plates, and then inoculated into liquid MM at 30 °C and shaken at 250 rpm/min for 2 days. The cells were collected by centrifugation, resuspended in HGM and then shaken at 250 rpm/min for 5 days. The cells were subjected to fatty acid analysis, according to the General Methods.

GC analyses showed that most of the selected 96 transformant strains produced 50-52.7% EPA of TFAs. Seven strains (i.e., #31, #35, #38, #41, #60, #61 and #95) that produced about 52.8%, 53.1%, 52.8%, 53.2%, 53.1%, 52.8%, and 52.9% EPA of TFAs were designated as Y8646, Y8647, Y8648, Y8649, Y8650, Y8651 and Y8652, respectively.

Knockout of the *Lip4* locus (GenBank Accession No. XM_503825) in these EPA strains was not confirmed.

The final genotype of strains Y8646, Y8647, Y8648, Y8649, Y8650, Y8651 and Y8652 with respect to wildtype *Yarrowia lipolytica* ATCC #20362 was *Ura+, Pex3-, unknown 1-, unknown 2-*, *unknown 3-, unknown 4-, unknown 5-, unknown6-, unknown 7-*, *unknown* 8-, YAT1::ME3S::Pex16, GPD::ME3S::Pex20, YAT1::ME3S::Lip1, FBAINm::EgD9eS::Lip2, EXP1::EgD9eS::Lip1, GPAT::EgD9e::Lip2, YAT1::EgD9eS::Lip2, FBAINm::EgD8M::Pex20, EXP1::EgD8M::Pex16, FBAIN::EgD8M::Lip1, GPD::EaD8S::Pex16, YAT1::E389D9eS/EgD8M::Lip1, FBAINm::EaD9eS/EaD8S::Lip2, GPD::FmD12::Pex20, YAT1::FmD12::Oct, EXP1 ::FmD12S::Aco, GPDIN::FmD12::Pex16, EXP1::EgD5M::Pex16, FBAIN::EgD5SM:Pex20, GPDIN::EgD5SM::Aco, GPM::EgD5SM::Oct, YAT1::EaD5SM::Oct, FBAINm::PaD17::Aco, EXP1::PaD17::Pex16, YAT1::PaD17S::Lip1, YAT1::YICPT::Aco, YAT1::MCS::Lip1.

### Analysis Of Total Lipid Content And Composition By Flask Assay

Cells from YPD plates of strains Y8647, Y8648, Y8649 and Y8650 were grown and analyzed for total lipid content and composition, according to the General Methods. Table 12 summarizes the total dry cell weight of the cells ["DCW'], the total lipid content of cells ["TFAs % DCW"], the concentration of each fatty acid as a weight percent of TFAs ["% TFAs"] and the EPA content as a percent of the dry cell weight ["EPA % DCW"]. Fatty acids are identified as 16:0 (palmitate), 16:1 (palmitoleic acid), 18:0 (stearic acid), 18:1 (oleic acid), 18:2 (LA), ALA, EDA, DGLA, ARA, ETrA, ETA, EPA and other.

**Table 12: Total Lipid Content And Composition In Yarrowia Strains Y8647, Y8648, Y8649 And Y8650 By Flask Assay**

| Strain | DCW (g/L) | TFAs % DCW | % TFAs | | | | | | | | | | | | | EPA % DCW |
|---|---|---|---|---|---|---|---|---|---|---|---|---|---|---|---|---|
| | | | 16:0 | 16:1 | 18:0 | 18:1 | 18:2 | ALA | EDA | DGLA | ARA | EtrA | ETA | EPA | other | |
| Y8647 | 3.8 | 37.6 | 1.3 | 0.2 | 2.1 | 4.7 | 20.3 | 1.7 | 3.3 | 3.6 | 0.7 | 0.6 | 3.0 | 53.6 | 4.5 | 20.1 |
| Y8648 | 3.5 | 27.8 | 2.3 | 0.3 | 2.7 | 4.3 | 18.6 | 2.3 | 2.1 | 2.2 | 0.6 | 0.6 | 1.9 | 56.7 | 4.9 | 15.7 |
| Y8649 | 3.6 | 27.9 | 2.4 | 0.3 | 2.9 | 3.7 | 18.8 | 2.2 | 2.1 | 2.4 | 0.6 | 0.8 | 2.1 | 55.8 | 5.5 | 15.6 |
| Y8650 | 3.5 | 28.2 | 2.2 | 0.3 | 2.9 | 3.8 | 18.8 | 2.4 | 2.1 | 2.4 | 0.6 | 0.6 | 2.1 | 56.1 | 5.3 | 15.8 |

### EXAMPLE 5

### Generation Of Yarrowia lipolytica Strain Y9028 To Produce About 54.5% EPA Of Total Fatty Acids ["TFAs"] With 39.6% Total Lipid Content

The present Example describes the construction of strain Y9028, derived from *Yarrowia lipolytica* ATCC #20362, capable of producing about 54.5% EPA relative to the total lipids with 39.6% total lipid content ["TFAs % DCW"] via expression of a Δ9 elongase/Δ8 desaturase pathway. The development of strain Y9028 (FIG. 2) required the construction of strains Y2224, Y4001, Y4001 U, Y4036, Y4036U and L135 (described in Example 1), strains L135U9 and Y8002 (described in Example 2), strains Y8006U6, Y8069, Y8069U, Y8154, Y8154U, Y8269 and Y8269U (described in Example 3), strains Y8412U6 and Y8647 (described in Example 4) and strain Y8467U.

### Generation Of Strain Y8647U (Ura3-)

In order to disrupt the Ura3 gene, construct pZKUM (FIG. 5A; SEQ ID NO:130; described in Table 15 of U.S. Pat. Appl. Pub. No. 2009-0093543-A1) was used to integrate a *Ura3* mutant gene into the *Ura3* gene of strain Y8647 (Example 4) in a manner similar to that described for pZKUM transformation of strain Y8006 (Example 3). A total of 12 transformants were grown and identified to possess a *Ura-* phenotype.

GC analyses showed that there were 30.2%, 29.2%, 28.1% and 29.9% EPA of TFAs in pZKUM-transformant strains #1, #3, #4 and #12, respectively. These four strains were designated as strains Y8647U1, Y8647U2, Y8647U3, and Y8647U6, respectively (collectively, Y8647U).

### Generation Of Strain Y9028

Construct pZP2-85m98F (FIG. 7B; SEQ ID NO:135) was generated to integrate one Δ8 desaturase gene, one DGLA synthase and one Δ5 desaturase gene into the *Yarrowia* Pox2 locus (GenBank Accession No. AJ001300) of strain Y8647U3 to thereby enable higher level production of EPA. The pZP2-85m98F plasmid contained the following components:

**Table 13: Description of Plasmid pZP2-85m98F (SEQ ID NO:135)**

| RE Sites And Nucleotides Within SEQ ID NO:135 | Description Of Fragment And Chimeric Gene Components |
|---|---|
| *Asc*I/*Bsi*WI (5986-5176) | 810 bp 5' portion of *Yarrowia Pox2* gene (GenBank Accession No. AJ001300) |
| *Pac*I/*Sph*I (9349-8694) | 655 bp 3' portion of *Yarrowia Pox2* gene (GenBank Accession No. AJ001300) |
| *Pme*I/*Swa*I (2493-5020) | EXP1::EgD5SM::Lip1, comprising: |
| | • EXP1: *Yarrowia lipolytica* export protein (EXP1) promoter (labeled as "EXP" in Figure; Intl. App. Pub. No. WO 2006/052870); |
| | • EgD5SM: Synthetic mutant Δ5 desaturase (SEQ ID NO:71; U.S. Pat. Pub. No. 2010-0075386-A1), derived from *Euglena gracilis* (U.S. Patent 7,678,560); |
| | • Lip1: Lip1 terminator sequence from *Yarrowia Lip1* gene (GenBank Accession No. Z50020) |
| *Cla*I/*Pme*I (1-2493) | GPD::EaD8S::Pex16, comprising: |
| | • GPD: *Yarrowia lipolytica* GPD promoter (U.S. Patent 7,259,255); |
| | • EaD8S: codon-optimized Δ8 desaturase gene (SEQ ID NO:63), derived from *Euglena anabaena* (U.S. Pat. Appl. Pub. No. 2008-0254521-A1); |
| | • Pex16: Pex16 terminator sequence from *Yarrowia Pex16* gene (GenBank Accession No. U75433) |
| *Sal*I/*Eco*RI (14170-12551) | *Yarrowia* Ura3 gene (GenBank Accession No. AJ306421) |
| *Eco*RI/*Pac*I (12551-9349) | YAT1::EgD9eS/EgD8M::Aco, comprising: |
| | • YAT1: *Yarrowia lipolytica* YAT1 promoter (labeled as "YAT" in Figure; U.S. Pat. Appl. Pub. No. 2006/0094102-A1); |
| | • EgD9eS/EgD8M: gene fusion comprising a codon-optimized Δ9 elongase derived from *Euglena gracilis* ("EgD9eS"), a linker, and the synthetic mutant Δ8 desaturase derived from *Euglena gracilis* ("EgD8M") (SEQ ID NO:8) (labeled as individually as "EgD9eS", "Linker" and "EgD8M" in Figure; U.S. Pat. Appl. Pub. No. 2008-0254191-A1); |
| | • Aco: Aco terminator sequence from *Yarrowia Aco* gene (GenBank Accession No. AJ001300) |

The pZP2-85m98F plasmid was digested with *Asc*l/*Sph*l*,* and then used for transformation of strains of Y8647U1, Y8647U2, Y8647U3 and Y8647U6, individually, according to the General Methods. The transformant cells were plated onto MM plates and maintained at 30 °C for 3 to 4 days. Single colonies were re-streaked onto MM plates, and then inoculated into liquid MM at 30 °C and shaken at 250 rpm/min for 2 days. The cells were collected by centrifugation, resuspended in HGM and then shaken at 250 rpm/min for 5 days. The cells were subjected to fatty acid analysis, according to the General Methods.

GC analyses showed that most of the selected 48 strains of Y8647U1 transformed with pZP2-85m98F produced 49-52% EPA of TFAs. Two strains (i.e., #30 and #31) that produced about 52.6% and 52.1% EPA of TFAs were designated as Y9024 and Y9025, respectively.

Most of the selected 60 strains of Y8647U2 transformed with pZP2-85m98F produced 49-51.9% EPA of TFAs. Strain #6 produced about 52% EPA of TFAs and was designated as Y9026.

Most of the selected 60 strains of Y8647U3 transformed with pZP2-85m98F produced 50-52.2% EPA of TFAs. Six strains (i.e., #5, #6, #14, #15, #20 and #34) that produced about 53.2%, 53.7%, 54.0%, 52.9%, 53.4% and 52.3% EPA of TFAs were designated as Y9027, Y9028, Y9029, Y9030, Y9031 and Y9032, respectively.

Similarly, GC analyses showed that most of the selected 48 strains of Y8647U6 transformed with pZP2-85m98F produced 50-52.1% EPA of TFAs. Two strains (i.e., #27 and #44) that produced about 52.2% and 52.8% EPA of TFAs were designated as Y9033 and Y9034, respectively.

Knockout of the Pox2 locus (GenBank Accession No. AJ001300) in strains Y9024, Y9025, Y9026, Y9027, Y9028, Y9029, Y9030, Y9031, Y9032, Y9033 and Y9034 was not confirmed in any of these EPA strains, produced by transformation with pZP2-85m98F.

The final genotype of these strains with respect to wildtype *Yarrowia lipolytica* ATCC #20362 was *Ura+, Pex3-, unknown 1-, unknown 2-, unknown 3-, unknown 4-, unknown 5-, unknown6-, unknown 7-, unknown 8-, unknown9-,* YAT1::ME3S::Pex16, GPD::ME3S::Pex20, YAT1::ME3S::Lip1, FBAINm::EgD9eS::Lip2, EXP1::EgD9eS::Lip1, GPAT::EgD9e::Lip2, YAT1::EgD9eS::Lip2, FBAINm::EgD8M::Pex20, EXP1::EgD8M::Pex16, FBAIN::EgD8M::Lip1, GPD::EaD8S::Pex16 (2 copies), YAT1::E389D9eS/EgD8M::Lip1, YAT1::EgD9eS/EgD8M::Aco, FBAINm::EaD9eS/EaD8S::Lip2, GPD::FmD12::Pex20, YAT1::FmD12::Oct, EXP1::FmD12S::Aco, GPDIN::FmD12::Pex16, EXP1::EgD5M::Pex16, FBAIN::EgD5SM::;Pex20, GPDIN::EgD5SM::Aco, GPM::EgD5SM::Oct, EXP1::EgD5SM::Lip1, YAT1::EaD5SM::Oct, FBAINm::PaD17::Aco, EXP1::PaD17::Pex16, YAT1 ::PaD17S::Lip1, YAT1::YICPT::Aco, YAT1::MCS::Lip1.

### Analysis Of Total Lipid Content And Composition By Flask Assay

Cells from YPD plates of strains Y9028, Y9029 and Y9031 were grown and analyzed for total lipid content and composition, according to the General Methods.

Table 14 below summarizes the total dry cell weight of the cells ["DCW'], the total lipid content of cells ["TFAs % DCW"], the concentration of each fatty acid as a weight percent of TFAs ["% TFAs"] and the EPA content as a percent of the dry cell weight ["EPA % DCW"]. More specifically, fatty acids are identified as 16:0 (palmitate), 16:1 (palmitoleic acid), 18:0 (stearic acid), 18:1 (oleic acid), 18:2 (LA), ALA, EDA, DGLA, ARA, ETrA, ETA, EPA and other.

**Table 14: Total Lipid Content And Composition In Yarrowia Strains Y9028, Y9029 and Y9031 By Flask Assay**

| Strain | DCW (g/L) | TFAs % DCW | % TFAs | | | | | | | | | | | | | EPA % DCW |
|---|---|---|---|---|---|---|---|---|---|---|---|---|---|---|---|---|
| | | | 16:0 | 16:1 | 18:0 | 18:1 | 18:2 | ALA | EDA | DGLA | ARA | EtrA | ETA | EPA | other | |
| **Y9028** | **3.3** | **39.6** | **1.3** | **0.2** | **2.1** | **4.4** | **19.8** | **1.7** | **3.2** | **2.5** | **0.8** | **0.7** | **1.9** | **54.5** | **6.1** | **21.6** |
| Y9029 | 3.2 | 38.4 | 1.3 | 0.3 | 1.7 | 4.4 | 19.8 | 1.5 | 3.2 | 3.3 | 0.9 | 0.7 | 2.4 | 53.8 | 6.0 | 20.7 |
| Y9031 | 3.3 | 38.6 | 1.3 | 0.3 | 1.8 | 4.7 | 20.1 | 1.7 | 3.2 | 3.2 | 0.9 | 0.8 | 2.6 | 52.3 | 6.3 | 20.2 |

### EXAMPLE 6

### Generation Of Yarrowia lipolytica Strains Y9481 And Y9502, Producing At Least About 57% EPA Of Total Fatty Acids ["TFAs"] With At Least About 35% Total Lipid Content

The present Example describes the construction of strains Y9481 and Y9502, derived from *Yarrowia lipolytica* ATCC #20362 and expressing a Δ9 elongase/Δ8 desaturase pathway. Strain Y9481 is capable of producing about 60.9% EPA relative to the total lipids with 35% total lipid content ["TFAs % DCW"], while strain Y9502 is capable of producing about 57% EPA relative to the total lipids with 37.1% TFAs % DCW.

The development of strains Y9481 and Y9502 (FIG. 2) required the construction of strains Y2224, Y4001, Y4001 U, Y4036, Y4036U and L135 (described in Example 1), strains L135U9 and Y8002 (described in Example 2), strains Y8006U6, Y8069, Y8069U, Y8154, Y8154U, Y8269 and Y8269U (described in Example 3), strains Y8412U6 and Y8647 (described in Example 4), strains Y8467U and Y9028 (described in Example 5) and strain Y9028U.

### Generation Of Strain Y9028U (Ura3-)

In order to disrupt the Ura3 gene, construct pZKUM (FIG. 5A; SEQ ID NO:130; described in Table 15 of U.S. Pat. Appl. Pub. No. 2009-0093543-A1) was used to integrate a *Ura3* mutant gene into the *Ura3* gene of strain Y9028 (Example 5) in a manner similar to that described for pZKUM transformation of strain Y8006 (Example 3). A total of 8 transformants were grown and identified to possess a *Ura-* phenotype.

GC analyses showed that there were 24.1%, 24.9%, 24.5% and 24.5% EPA of TFAs in pZKUM-transformant strains #1, #3, #4, and #5, respectively. These four strains were designated as strains Y9028U1, Y9028U2, Y9028U3, and Y9028U4, respectively (collectively, Y9028U).

### Components Of Integration Vector pZK16-ML8N

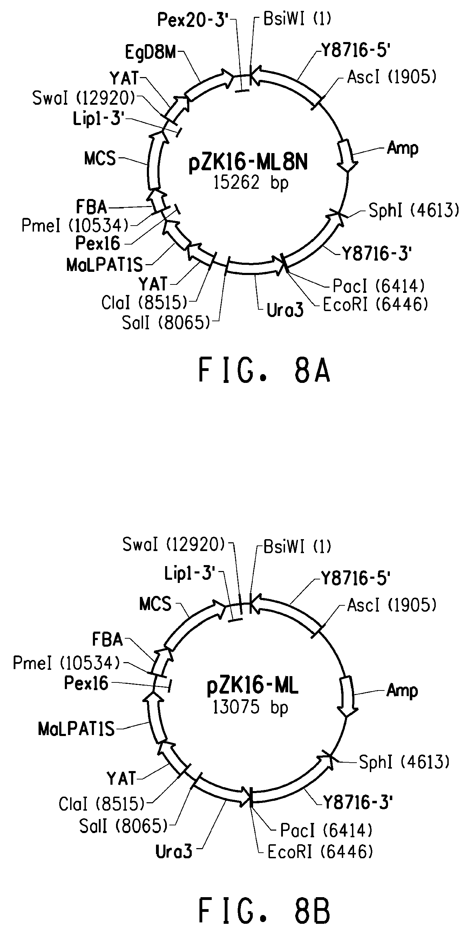

Construct pZK16-ML8N (FIG. 8A; SEQ ID NO:136) was generated to integrate one Δ8 desaturase gene, one malonyl-CoA synthetase gene, and one lysophosphatidic acid acyltransferase gene ["LPAAT"] into the *Yarrowia* YALI0B14795p locus (GenBank Accession No. XM_500900) of strain Y9028U2. The pZK16-ML8N plasmid contained the following components:

**Table 15: Description of Plasmid pZK16-ML8N (SEQ ID NO:136)**

| RE Sites And Nucleotides Within SEQ ID NO:136 | Description Of Fragment And Chimeric Gene Components |
|---|---|
| *Asc*I/*Bsi*WI (1905-1) | 1904 bp 5' portion of YALI0B14795p locus (GenBank Accession No. XM_500900, labeled as "Y8716-5"' in Figure) |
| *Pac*I/*Sph*I (6414-4613) | 1801 bp 3' portion of YALI0B14795p locus (GenBank Accession No. XM_500900, labeled as "Y8716-3"' in Figure) |
| *Swa*I/*Bsi*WI (12920 - 1) | YAT1::EgD8M::Pex20, comprising: |
| | • YAT1: *Yarrowia lipolytica* YAT1 promoter (labeled as "YAT" in Figure; U.S. Pat. Appl. Pub. No. 2006-0094102-A1); |
| | • EgD8M: Synthetic mutant Δ8 desaturase (SEQ ID NO:59; U.S. Patent 7,709,239), derived from *Euglena gracilis* ("EgD8S"; U.S. Patent 7,256,033); |
| | • Pex20: Pex20 terminator sequence from *Yarrowia Pex20* gene (GenBank Accession No. AF054613) |
| *Pme*I/*Swa*I (10534-12920) | FBA::MCS::Lip1, comprising: |
| | • FBA: *Yarrowia lipolytica* FBA promoter (U.S. Patent 7,202,356); |
| | • MCS: codon-optimized malonyl-CoA synthetase gene (SEQ ID NO:41), derived from *Rhizobium leguminosarum* bv. viciae 3841 (U.S. Patent Application No. 12/637877); |
| | • Lip1: Lip1 terminator sequence from *Yarrowia Lip1* gene (GenBank Accession No. Z50020) |
| *Cla*/I\|*Pme*I (8515-10534) | YAT1::MaLPAAT1S::Pex16, comprising: |
| | • YAT1: *Yarrowia lipolytica* YAT1 promoter (labeled as "YAT" in Figure; U.S. Pat. Appl. Pub. No. 2006-0094102-A1); |
| | • MaLPAAT1S: codon-optimized lysophosphatidic acid acyltransferase gene (SEQ ID NO:35), derived from *Mortierella alpina* (U.S. Pat. Appl. Pub. No. 2006-0115881-A1; U.S. Pat. Appl. Pub. No. 2009-0325265-A1); |
| | • Pex16: Pex16 terminator sequence from *Yarrowia Pex16* gene (GenBank Accession No. U75433) |
| *Sal*I/*Eco*RI (8065 - 6446) | *Yarrowia* Ura3 gene (GenBank Accession No. AJ306421) |

### Components Of Integration Vector pZK16-ML

Construct pZK16-ML (FIG. 8B; SEQ ID NO:137) was generated to integrate one malonyl-CoA synthetase gene and one lysophosphatidic acid acyltransferase gene ["LPAAT"] into the *Yarrowia* YALI0B14795p locus (GenBank Accession No. XM_500900) of strain Y9028U2. The components of the pZK16-ML plasmid are identical to those of pZK16-ML8N (*supra*); however, the chimeric YAT1::EgD8M::Pex20 gene of pZK16-ML8N is lacking.

### Generation Of Strains Y9481 And Y9502

The pZK16-ML8N plasmid and pZK16-ML plasmid were each individually digested with *Asc*I/*Sph*I*,* and then used separately for transformation of strain Y9028U2, according to the General Methods. The transformant cells were plated onto MM plates and maintained at 30 °C for 3 to 4 days. Single colonies were re-streaked onto MM plates, and then inoculated into liquid MM at 30 °C and shaken at 250 rpm/min for 2 days. The cells were collected by centrifugation, resuspended in HGM and then shaken at 250 rpm/min for 5 days. The cells were subjected to fatty acid analysis, according to the General Methods.

GC analyses showed that most of the selected 96 strains of Y9028U2 with pZK16-ML8N produced 50-55.4% EPA of TFAs. Fifteen strains (i.e., #8, #18, #21, #24, #29, #48, #60, #66, #68, #75, #76, #78, #90, #95 and #96) that produced about 58.1%, 61.4%, 56.2%, 58.1%, 57.5%, 57.0%, 55.9%, 57.6%, 57.8%, 55.5%, 57.6%, 58.1%, 57.1%, 56.2% and 58.6% EPA of TFAs were designated as Y9472, Y9473, Y9474, Y9475, Y9476, Y9477, Y9478, Y9479, Y9480, Y9481, Y9482, Y9483, Y9484, Y9485 and Y9486, respectively.

The final genotype of these pZK16-ML8N transformant strains with respect to wildtype *Yarrowia lipolytica* ATCC #20362 was *Ura+, Pex3-, unknown 1-, unknown 2-, unknown 3-, unknown 4-, unknown 5-, unknown6-, unknown 7-, unknown 8-, unknown9-, unknown 10-,* YAT1::ME3S::Pex16, GPD::ME3S::Pex20, YAT1::ME3S::Lip1, FBAINm::EgD9eS::Lip2, EXP1::EgD9eS::Lip1, GPAT::EgD9e::Lip2, YAT1::EgD9eS::Lip2, FBAINm::EgD8M::Pex20, EXP1::EgD8M::Pex16, FBAIN::EgD8M::Lip1, YAT1::EgD8M::Pex20, GPD::EaD8S::Pex16 (2 copies), YAT1::E389D9eS/EgD8M::Lip1, YAT1::EgD9eS/EgD8M::Aco, FBAINm::EaD9eS/EaD8S::Lip2, GPD::FmD12::Pex20, YAT1::FmD12::Oct, EXP1::FmD12S::Aco, GPDIN::FmD12::Pex16, EXP1::EgD5M::Pex16, FBAIN::EgD5SM::Pex20, GPDIN::EgD5SM::Aco, GPM::EgD5SM::Oct, EXP1::EgD5SM::Lip1, YAT1::EaD5SM::Oct, FBAINm::PaD17::Aco, EXP1::PaD17::Pex16, YAT1 ::PaD17S::Lip1, YAT1::YICPT::Aco, YAT1 ::MCS::Lip1, FBA::MCS::Lip1, YAT1::MaLPAAT1S::Pex16.

Similarly, GC analyses showed that most of the selected 96 strains of Y9028U2 with pZK16-ML produced 51-55.5% EPA of TFAs. Sixteen strains (i.e., #4, #8, #15, #16, #39, #44, #46, #63, #66, #80, #85, #86, #88, #89, #90 and #96) that produced about 56.5%, 57.4%, 56.8%, 57.0%, 56.4%, 57.3%, 58.2%, 55.6%, 57.8%, 55.6%, 57.6%, 56.8%, 55.8%, 56.4%, 56.1% and 57% EPA of TFAs were designated as Y9496, Y9497, Y9498, Y9499, Y9500, Y9501, Y9502, Y9503, Y9504, Y9505, Y9506, Y9507, Y9508, Y9509, Y9510 and Y9511, respectively.

The final genotype of these pZK16-ML transformant strains with respect to wildtype *Yarrowia lipolytica* ATCC #20362 was *Ura+, Pex3-, unknown 1-, unknown 2-, unknown 3-, unknown 4-, unknown 5-, unknown6-, unknown 7-, unknown 8-, unknown9-, unknown 10-,* YAT1::ME3S::Pex16, GPD::ME3S::Pex20, YAT1::ME3S::Lip1, FBAINm::EgD9eS::Lip2, EXP1::EgD9eS::Lip1, GPAT::EgD9e::Lip2, YAT1::EgD9eS::Lip2, FBAINm::EgD8M::Pex20, EXP1::EgD8M::Pex16, FBAIN::EgD8M::Lip1, GPD::EaD8S::Pex16 (2 copies), YAT1::E389D9eS/EgD8M::Lip1, YAT1::EgD9eS/EgD8M::Aco, FBAINm::EaD9eS/EaD8S::Lip2, GPD::FmD12::Pex20, YAT1::FmD12::Oct, EXP1::FmD12S::Aco, PDIN::FmD12::Pex16, EXP1::EgD5M::Pex16, FBAIN::EgD5SM::Pex20, GPDIN::EgD5SM::Aco, GPM::EgD5SM::Oct, EXP1::EgD5SM::Lip1, YAT1::EaD5SM::Oct, FBAINm::PaD17::Aco, EXP1::PaD17::Pex16, YAT1::PaD17S::Lip1, YAT1::YICPT::Aco, YAT1::MCS::Lip1, FBA::MCS::Lip1, YAT1::MaLPAAT1S::Pex16.

Knockout of the YALI0B14795p locus (GenBank Accession No. XM_500900) in strains Y9472, Y9473, Y9474, Y9475, Y9476, Y9477, Y9478, Y9479, Y9480, Y9481, Y94782, Y9483, Y9484, Y9485, Y9486, Y9496, Y9497, Y9498, Y9499, Y9500, Y9501, Y9502, Y9503, Y9504, Y9505, Y9506, Y9507, Y9508, Y9509, Y9510 and Y9511 was not confirmed in any of these EPA strains, produced by transformation with pZK16-ML8N or pZK16-ML.

### Analysis Of Total Lipid Content And Composition By Flask Assay

Cells from YPD plates of strains Y9477, Y9481, Y9486, Y9497, Y9502, Y9504, Y9508 and Y9510 were grown and analyzed for total lipid content and composition, according to the General Methods.

Table 16 below summarizes the total dry cell weight of the cells ["DCW'], the total lipid content of cells ["TFAs % DCW"], the concentration of each fatty acid as a weight percent of TFAs ["% TFAs"] and the EPA content as a percent of the dry cell weight ["EPA % DCW"]. More specifically, fatty acids are identified as 16:0 (palmitate), 16:1 (palmitoleic acid), 18:0 (stearic acid), 18:1 (oleic acid), 18:2 (LA), ALA, EDA, DGLA, ARA, ETrA, ETA, EPA and other.

**Table 16: Total Lipid Content And Composition In Yarrowia Strains Y9477, Y9481, Y9486, Y9497, Y9502, Y9504, Y9508 and Y9510 By Flask Assay**

| Strain | DCW (g/L) | TFAs % DCW | % TFAs | | | | | | | | | | | | | EPA % DCW |
|---|---|---|---|---|---|---|---|---|---|---|---|---|---|---|---|---|
| | | | 16:0 | 16:1 | 18:0 | 18:1 | 18:2 | ALA | EDA | DGLA | ARA | EtrA | ETA | EPA | other | |
| Y9477 | 3.2 | 32.6 | 2.6 | 0.5 | 3.4 | 4.8 | 10.0 | 0.5 | 2.5 | 3.7 | 1.0 | 0.5 | 2.1 | 61.4 | 6.9 | 20.0 |
| **Y9481** | **3.1** | **35.0** | **2.5** | **0.5** | **3.1** | **4.7** | **11.0** | **0.6** | **2.6** | **3.6** | **0.9** | **0.5** | **2.1** | **60.9** | **6.8** | **21.3** |
| Y9486 | 3.1 | 32.2 | 2.1 | 0.7 | 1.8 | 4.2 | 11.9 | 0.6 | 2.9 | 4.2 | 1.2 | 0.7 | 2.4 | 60.3 | 6.7 | 19.4 |
| | | | | | | | | | | | | | | | | |
| Y9497 | 3.7 | 33.7 | 2.4 | 0.5 | 3.2 | 4.6 | 11.3 | 0.8 | 3.1 | 3.6 | 0.9 | 0.7 | 2.3 | 58.7 | 7.1 | 19.8 |
| **Y9502** | **3.8** | **37.1** | **2.5** | **0.5** | **2.9** | **5.0** | **12.7** | **0.9** | **3.5** | **3.3** | **0.8** | **0.7** | **2.4** | **57.0** | **7.5** | **21.3** |
| Y9504 | 3.7 | 33.7 | 2.2 | 0.5 | 3.0 | 4.5 | 11.3 | 0.7 | 2.9 | 3.5 | 0.9 | 0.7 | 2.3 | 59.9 | 7.1 | 20.1 |
| Y9508 | 3.7 | 34.9 | 2.3 | 0.5 | 2.7 | 4.4 | 13.1 | 0.9 | 2.9 | 3.3 | 0.9 | 0.7 | 2.3 | 58.7 | 7.3 | 20.5 |
| Y9510 | 3.6 | 35.1 | 2.5 | 0.5 | 2.7 | 4.4 | 11.7 | 0.7 | 2.9 | 3.7 | 0.9 | 0.7 | 2.3 | 58.9 | 7.8 | 20.7 |

### EXAMPLE 7

### Generation Of Yarrowia lipolytica Strain Y8672 To Produce About 61.8% EPA Of Total Fatty Acids ["TFAs"] With 26.5% Total Lipid Content

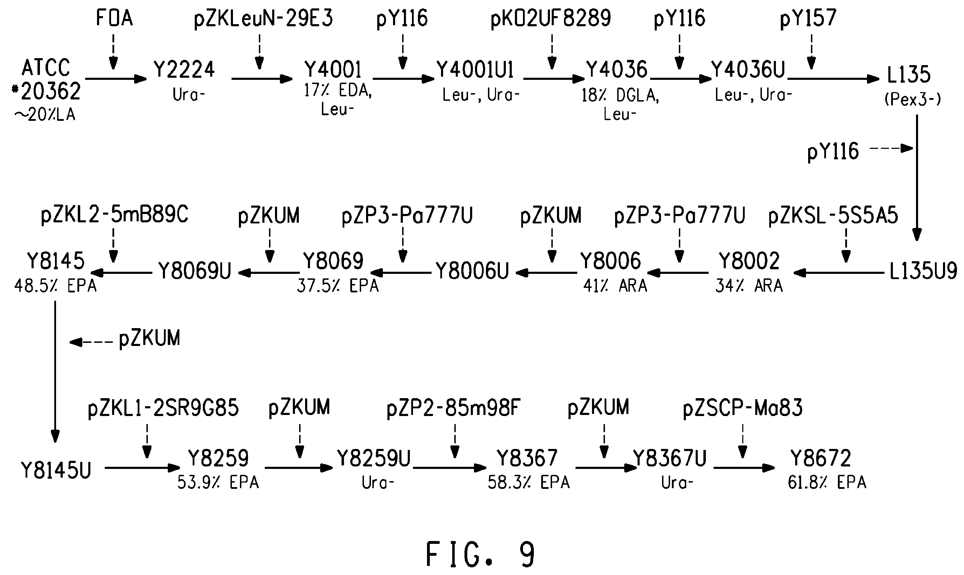

The present Example describes the construction of strain Y8672, derived from *Yarrowia lipolytica* ATCC #20362, capable of producing about 61.8% EPA relative to the total lipids with 26.5% total lipid content ["TFAs % DCW"] via expression of a Δ9 elongase/Δ8 desaturase pathway. The development of strain Y8672 (FIG. 9) required the construction of strains Y2224, Y4001, Y4001 U, Y4036, Y4036U and L135 (described in Example 1), strains L135U9 and Y8002 (described in Example 2), strains Y8006U6, Y8069, Y8069U (described in Example 3) and strains Y8145, Y8145U, Y8259, Y8259U, Y8367 and Y8367U.

### Generation Of Strain Y8145 To Produce About 48.5% EPA Of TFAs

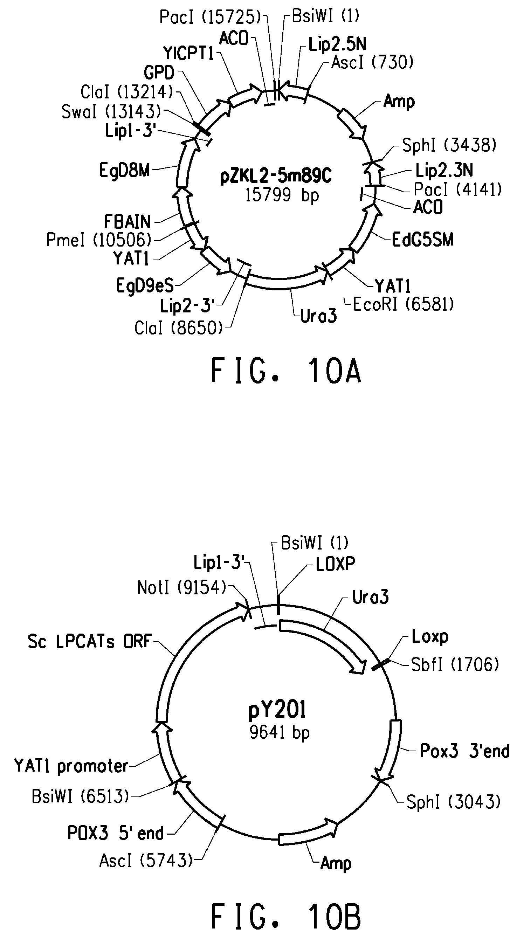

Construct pZKL2-5m89C (FIG. 10; SEQ ID NO:138) was generated to integrate one Δ5 desaturase gene, one Δ9 elongase gene, one Δ8 desaturase gene, and one Y. *lipolytica* diacylglycerol cholinephosphotransferase gene (CPT1) into the *Lip2* loci (GenBank Accession No. AJ012632) of strain Y8069U3 (Example 3) to thereby enable higher level production of EPA. The pZKL2-5m89C plasmid contained the following components:

**Table 17: Description of Plasmid pZKL2-5m89C (SEQ ID NO:138)**

| RE Sites And Nucleotides Within SEQ ID NO:138 | Description Of Fragment And Chimeric Gene Components |
|---|---|
| *Asc*I/*Bsi*WI (730-1) | 722 bp 5' portion of *Yarrowia Lip2* gene (labeled as "Lip2.5N" in Figure; GenBank Accession No. AJ012632) |
| *Pac*I/*Sph*I (4141-3438) | 697 bp 3' portion of *Yarrowia Lip2* gene (labeled as "Lip2.3N" in Figure; GenBank Accession No. AJ012632) |
| *Swa*I/*Bsi*WI (13143-1) | GPD::YICPT1::Aco, comprising: |
| | • GPD: *Yarrowia lipolytica* GPD promoter (U.S. Patent 7,259,255); |
| | • YICPT1: *Yarrowia lipolytica* diacylglycerol cholinephosphotransferase gene (SEQ ID NO:37) (Intl. App. Pub. No. WO 2006/052870); |
| | • Aco: Aco terminator sequence from *Yarrowia Aco* gene (GenBank Accession No. AJ001300) |
| *Pme*I/*Swa*I | FBAIN::EgD8M::Lip1 comprising: |
| (10506-13143) | • FBAIN: *Yarrowia lipolytica* FBAIN promoter (U.S. Patent 7,202,356); |
| | • EgD8M: Synthetic mutant Δ8 desaturase (SEQ ID NO:59; U.S. Patent 7,709,239), derived from *Euglena gracilis* ("EgD8S"; U.S. Patent 7,256,033); |
| | • Lip1: Lip1 terminator sequence from *Yarrowia Lip1* gene (GenBank Accession No. Z50020) |
| *Pme*I/*Cla*I (10506-8650) | YAT1::EgD9eS::Lip2, comprising: |
| | • YAT1: *Yarrowia lipolytica* YAT1 promoter (labeled as "YAT" in Figure; U.S. Pat. Appl. Pub. No. 2006-0094102-A1); |
| | • EgD9eS: codon-optimized Δ9 elongase gene (SEQ ID NO:45), derived from *Euglena gracilis* (U.S. Patent 7,645,604); |
| | • Lip2: Lip2 terminator sequence from *Yarrowia Lip2* gene (GenBank Accession No. AJ012632) |
| *Cla*I/*Eco*RI (8650-6581) | *Yarrowia* Ura3 gene (GenBank Accession No. AJ306421) |
| *Eco*RI/*Pac*I (6581-4141) | YAT1::EgD5SM::ACO, comprising: |
| | • YAT1: *Yarrowia lipolytica* YAT1 promoter (labeled as "YAT" in Figure; U.S. Pat. Appl. Pub. No. 2006-0094102-A1); |
| | • EgD5SM: Synthetic mutant Δ5 desaturase (SEQ ID NO:71; U.S. Pat. Pub. No. 2010-0075386-A1), derived from *Euglena gracilis* (U.S. Patent 7,678,560); |
| | • Aco: Aco terminator sequence from *Yarrowia Aco* gene (GenBank Accession No. AJ001300) |

The pZKL2-5m89C plasmid was digested with *Asc*l/*Sph*l*,* and then used for transformation of strain Y8069U3 according to the General Methods. The transformant cells were plated onto MM plates and maintained at 30 °C for 3 to 4 days. Single colonies were re-streaked onto MM plates, and then inoculated into liquid MM at 30 °C and shaken at 250 rpm/min for 2 days. The cells were collected by centrifugation, resuspended in HGM and then shaken at 250 rpm/min for 5 days. The cells were subjected to fatty acid analysis, according to the General Methods.

GC analyses showed that most of the selected 96 strains produced 38-44.5% EPA of TFAs. Four strains (i.e., #10, #50, #70 and #89) that produced about 45.1%, 45.6%, 45.0% and 45.6% EPA of TFAs were designated as Y8143, Y8144, Y8145 and Y8146, respectively. Knockout of the *Lip2* loci (GenBank Accession No. AJ012632) was not confirmed in these EPA strains.

The final genotype of strains Y8143, Y8144, Y8145 and Y8146 with respect to wildtype *Yarrowia lipolytica* ATCC #20362 was *Ura+, Pex3-, unknown 1-, unknown 2-, unknown 3-, unknown 4-, unknown 5-, Leu+, Lys*+, YAT1::ME3S::Pex16, GPD::FmD12::Pex20, YAT1::FmD12::Oct, GPAT::EgD9e::Lip2, FBAINm::EgD9eS::Lip2, EXP1::EgD9eS::Lip1, YAT1::EgD9eS::Lip2, FBAINm::EgD8M::Pex20, FBAIN::EgD8M::Lip1, EXP1::EgD8M::Pex16, FBAIN::EgD5SM::Pex20, YAT1::EgD5SM::Aco, EXP1::EgD5M::Pex16, YAT1::EaD5SM::Oct, YAT1 ::PaD17S::Lip1, EXP1::PaD17::Pex16, FBAINm::PaD17::Aco, GPD::YICPT1::Aco. Analysis Of Total Lipid Content And Composition By Flask Assay

Cells from YPD plates of strains Y8143, Y8144, Y8145 and Y8146 were grown and analyzed for total lipid content and composition, according to the General Methods.

**Table 18: Total Lipid Content And Composition In Yarrowia Strains Y8143, Y8144, Y8145 and Y8146 By Flask Assay**

| Strain | DCW (g/L) | TFAs % DCW | % TFAs | | | | | | | | | | | | | EPA % DCW |
|---|---|---|---|---|---|---|---|---|---|---|---|---|---|---|---|---|
| | | | 16:0 | 16:1 | 18:0 | 18:1 | 18:2 | ALA | EDA | DGLA | ARA | EtrA | ETA | EPA | other | |
| Y8143 | 4.6 | 22.3 | 4.2 | 1.5 | 1.4 | 3.6 | 18.1 | 2.6 | 1.7 | 1.6 | 0.6 | 2.2 | 1.6 | 50.3 | 11.6 | 11.2 |
| Y8144 | 4.3 | 23 | 4.0 | 1.5 | 1.4 | 3.3 | 18.0 | 2.6 | 1.8 | 1.7 | 0.7 | 2.3 | 1.6 | 50.6 | 11.5 | 11.6 |
| **Y8145** | **4.6** | **23.1** | **4.3** | **1.7** | **1.4** | **4.8** | **18.6** | **2.8** | **2.2** | **1.5** | **0.6** | **2.2** | **1.5** | **48.5** | **9.9** | **11.2** |
| Y8146 | 4.5 | 23.8 | 4.3 | 1.7 | 1.4 | 4.8 | 18.7 | 2.8 | 2.0 | 1.5 | 0.6 | 2.2 | 1.5 | 48.3 | 11.2 | 11.5 |

### Generation Of Strain Y8145U (Ura3-)

In order to disrupt the Ura3 gene, construct pZKUM (FIG. 5A; SEQ ID NO:130; described in Table 15 of U.S. Pat. Appl. Pub. No. 2009-0093543-A1) was used to integrate a *Ura3* mutant gene into the *Ura3* gene of strain Y8145 in a manner similar to that described for pZKUM transformation of strain Y8006 (Example 3). A total of 8 transformants were grown and identified to possess a *Ura-* phenotype.

GC analyses showed that there were 22.5%, 22.6% and 23.4% EPA of TFAs in pZKUM-transformant strains #5, #6 and #7, respectively. These three strains were designated as strains Y8145U1, Y8145U2 and Y8145U3, respectively (collectively, Y8145U).

### Generation Of Y8259 Strain To Produce About 53.9% EPA Of TFAs

Construct pZKL1-2SR9G85 (Example 3, FIG. 6A; SEQ ID NO:132) was generated to integrate one DGLA synthase gene, one Δ12 desaturase gene and one Δ5 desaturase gene into the *Lip1* loci (GenBank Accession No. Z50020) of strain Y8145U to thereby enable higher level production of EPA.

The pZKL1-2SR9G85 plasmid was digested with *Asc*I/*Sph*I*,* and then used for transformation of strain Y8145U1, in a manner similar to that described for pZKL1-2SR9G85 transformation of strain Y8154U1 (Example 3). The cells were subjected to fatty acid analysis, according to the General Methods.

GC analyses showed that most of the selected 96 strains produced 40-44.0% EPA of total lipids. Five strains (i.e., #7, #14, #48, #56 and #60) that produced about 45.2%, 47%, 44.4%, 44.3% and 45.2% EPA of TFAs were designated as Y8255, Y8256, Y8257, Y8258 and Y8259, respectively. Knockout of the *Lip1* loci (GenBank Accession No. Z50020) was not confirmed in these EPA strains.

The final genotype of these strains with respect to wildtype *Yarrowia lipolytica* ATCC #20362 was *Ura+, Pex3-, unknown 1-, unknown* 2-, *unknown 3-, unknown 4-, unknown 5-, unknown 6-, Leu+, Lys*+, YAT1::ME3S::Pex16, GPD::FmD12::Pex20, YAT1::FmD12::Oct, EXP1 ::FmD12S::ACO, GPAT::EgD9e::Lip2, FBAINm::EgD9eS::Lip2, EXP1::EgD9eS::Lip1, YAT1::EgD9eS::Lip2, FBAINm::EgD8M::Pex20, FBAIN::EgD8M::Lip1, EXP1::EgD8M::Pex16, YAT1::E389S/EgD8M::Lip1, FBAIN::EgD5SM::Pex20, YAT1::EgD5SM::Aco, GPM::EgD5SM::Oct, EXP1::EgD5M::Pex16, YAT1::EaD5SM::Oct; YAT1::PaD17S::Lip1, EXP1::PaD17::Pex16, FBAINm::PaD17::Aco, GPD::YICPT1::Aco.

*Yarrowia lipolytica* strain Y8259 was deposited with the American Type Culture Collection on May 14, 2009 and bears the designation ATCC PTA-10027.

### Analysis Of Total Lipid Content And Composition By Flask Assay

Cells from YPD plates of strains Y8256 and Y8259 were grown and analyzed for total lipid content and composition, according to the General Methods.

**Table 19: Total Lipid Content And Composition In Yarrowia Strains Y8256 and Y8259 By Flask Assay**

| Strain | DCW (g/L) | TFAs % DCW | % TFAs | | | | | | | | | | | | | EPA % DCW |
|---|---|---|---|---|---|---|---|---|---|---|---|---|---|---|---|---|
| | | | 16:0 | 16:1 | 18:0 | 18:1 | 18:2 | ALA | EDA | DGLA | ARA | EtrA | ETA | EPA | other | |
| Y8256 | 4.0 | 20.1 | 3.5 | 1.4 | 1.3 | 3.8 | 18.8 | 2.0 | 2.1 | 1.6 | 0.8 | 2.1 | 1.7 | 49.9 | 11.0 | 10.0 |
| **Y8259** | **4.7** | **20.5** | **3.5** | **1.3** | **1.3** | **4.8** | **16.9** | **2.3** | **1.9** | **1.7** | **0.6** | **1.8** | **1.6** | **53.9** | **8.4** | **11.0** |

### Generation Of Strain Y8259U (Ura3-)

In order to disrupt the Ura3 gene, construct pZKUM (FIG. 5A; SEQ ID NO:130; described in Table 15 of U.S. Pat. Appl. Pub. No. 2009-0093543-A1) was used to integrate a *Ura3* mutant gene into the *Ura3* gene of strain Y8259 in a manner similar to that described for pZKUM transformation of strain Y8006 (Example 3). A total of 8 transformants were grown and identified to possess a *Ura-* phenotype.

GC analyses showed that there was 26.6% EPA of TFAs in pZKUM-transformant strain #3. This strain was designated as strain Y8259U.

### Generation Of Y8367 Strain To Produce about 58.3% EPA Of TFAs

Construct pZP2-85m98F (Example 5, FIG. 7B; SEQ ID NO:135) was generated to integrate one Δ8 desaturase gene, one DGLA synthase, and one Δ5 desaturase gene into the *Yarrowia* Pox2 locus (GenBank Accession No. AJ001300) of strain Y8259U to thereby enable higher level production of EPA.

The pZP2-85m98F plasmid was digested with *Asc*I/*Sph*I, and then used for transformation of strain Y8259U, in a manner similar to that described for pZP2-85m98F transformation of strain Y8647U3 (Example 5). The cells were subjected to fatty acid analysis, according to the General Methods.

GC analyses showed that most of the selected 96 strains of Y8259U with pZP2-85m98F produced 41-46% EPA of TFAs. Four strains (i.e., #26, #33, #77 and #81) that produced about 46.7%, 46.5%, 47.4% and 46.9% EPA of TFAs were designated as Y8367, Y8368, Y8369 and Y8370, respectively. Knock out of the Pox2 locus (GenBank Accession No. AJ001300) was not confirmed in these EPA strains.

The final genotype of strains Y8367, Y8368, Y8369 and Y8370 with respect to wildtype *Yarrowia lipolytica* ATCC #20362 was *Ura*+, *Pex3-, unknown 1-, unknown 2*-, *unknown 3-, unknown 4-, unknown 5*-, *unknown 6-, unknown 7-, Leu+, Lys+,* YAT1::ME3S::Pex16, GPD::FmD12::Pex20, YAT1::FmD12::Oct, EXP1::FmD12S::ACO, GPAT::EgD9e::Lip2, FBAINm::EgD9eS::Lip2, EXP1::EgD9eS::Lip1, YAT1::EgD9eS::Lip2, FBAINm::EgD8M::Pex20, FBAIN::EgD8M::Lip1, EXP1::EgD8M::Pex16, GPD::EaD8S::Pex16, YAT1::E389D9eS/EgD8M::Lip1, YAT1::EgD9eS/EgD8M::Aco, FBAIN::EgD5SM::Pex20, YAT1::EgD5SM::Aco, GPM::EgD5SM::Oct, EXP1::EgD5M::Pex16, EXP1::EgD5SM::Lip1, YAT1::EaD5SM::Oct, YAT1::PaD17S::Lip1, EXP1::PaD17::Pex16, FBAINm::PaD17::Aco, GPD::YICPT1::Aco.

### Analysis Of Total Lipid Content And Composition By Flask Assay

Cells from YPD plates of strains Y8367, Y8368, Y8369 and Y8370 were grown and analyzed for total lipid content and composition, according to the General Methods.

**Table 20: Total Lipid Content And Composition In Yarrowia Strains Y8367, Y8368, Y8369 and Y8370 By Flask Assay**

| Strain | DCW (g/L) | TFAs % DCW | % TFAs | | | | | | | | | | | | | EPA % DCW |
|---|---|---|---|---|---|---|---|---|---|---|---|---|---|---|---|---|
| | | | 16:0 | 16:1 | 18:0 | 18:1 | 18:2 | ALA | EDA | DGLA | ARA | EtrA | ETA | EPA | other | |
| **Y8367** | **3.6** | **18.4** | **3.7** | **1.2** | **1.1** | **3.4** | **14.2** | **1.1** | **1.5** | **1.7** | **0.8** | **2.1** | **1.0** | **58.3** | **9.9** | **10.7** |
| Y8368 | 4.7 | 19.2 | 3.0 | 1.4 | 1.3 | 4.3 | 17.9 | 1.3 | 2.4 | 2.8 | 1.0 | 1.8 | 1.9 | 52.5 | 8.4 | 10.1 |
| Y8369 | 3.5 | 19.7 | 3.7 | 1.2 | 1.6 | 4.2 | 15.6 | 1.8 | 1.7 | 1.9 | 0.6 | 1.7 | 1.6 | 55.8 | 8.6 | 11.0 |
| Y8370 | 4.0 | 23.3 | 3.4 | 1.1 | 1.4 | 4.0 | 15.7 | 1.9 | 1.7 | 1.9 | 0.6 | 1.8 | 1.5 | 56.4 | 8.6 | 13.1 |

### Generation Of Strain Y8367U (Ura3-)

In order to disrupt the Ura3 gene, construct pZKUM (FIG. 5A; SEQ ID NO:130; described in Table 15 of U.S. Pat. Appl. Pub. No. 2009-0093543-A1) was used to integrate a *Ura3* mutant gene into the *Ura3* gene of strain Y8367 in a manner similar to that described for pZKUM transformation of strain Y8006 (Example 3). A total of 8 transformants were grown and identified to possess a *Ura-* phenotype.

GC analyses showed that there were 25.6%, 25.5% and 25.4% EPA of TFAs in pZKUM-transformant strains #2, #3 and #6, respectively. These three strains were designated as strains Y8367U1, Y8367U2 and Y8367U3, respectively (collectively, Y8367U).

### Generation Of Y8672 strain To Produce about 61.8% EPA Of TFAs

Construct pZSCP-Ma83 (Example 3, FIG. 6B; SEQ ID NO:133) was generated to integrate one Δ8 desaturase gene, one C_{16/18} elongase gene and one malonyl-CoA synthetase gene into the *SCP2* loci (GenBank Accession No. XM_503410) of strain Y8637U to thereby enable higher level production of EPA.

The pZSCP-Ma83 plasmid was digested with *Asc*I/*Sph*I, and then used for transformation of strain Y8367U1, in a manner similar to that described for pZSCP-Ma83 transformation of strain Y8269U1 (Example 3). The cells were subjected to fatty acid analysis, according to the General Methods.

GC analyses showed that most of the selected 96 strains of Y8367U1 with pZSCP-Ma83 produced 46-52.5% EPA of TFAs. Eight strains (i.e., #8, #40, #43, #44, #61, #63, #68 and #70) that produced about 53.2%, 52.8%, 52.7%, 52.9%, 53.0%, 52.6%, 53.1% and 52.7% EPA of TFAs were designated as Y8666, Y8667, Y8668, Y8669, Y8670, Y8671, Y8672 and Y8673, respectively. Knockout of the *SCP2* loci (GenBank Accession No. XM_503410) was not confirmed in these EPA strains.

The final genotype of strains Y8666, Y8667, Y8668, Y8669, Y8670, Y8671, Y8672 and Y8673 with respect to wildtype *Yarrowia lipolytica* ATCC #20362 was *Ura*+*, Pex3-, unknown 1-, unknown 2-, unknown 3-, unknown 4-, unknown 5-, unknown 6-, unknown 7-, unknown 8-, Leu+, Lys*+, YAT1::ME3S::Pex16, GPD::ME3S::Pex20, GPD::FmD12::Pex20, YAT1::FmD12::Oct, EXP1::FmD12S::ACO, GPAT::EgD9e::Lip2, FBAINm::EgD9eS::Lip2, EXP1::EgD9eS::Lip1, YAT1::EgD9eS::Lip2, FBAINm::EgD8M::Pex20, FBAIN::EgD8M::Lip1, EXP1::EgD8M::Pex16, GPD::EaD8S::Pex16 (2 copies), YAT1::E389D9eS/EgD8M::Lip1, YAT1::EgD9eS/EgD8M::Aco, FBAIN::EgD5SM::Pex20, YAT1::EgD5SM::Aco, GPM::EgD5SM::Oct, EXP1::EgD5M::Pex16, EXP1::EgD5SM::Lip1, YAT1::EaD5SM::Oct, YAT1::PaD17S::Lip1, EXP1::PaD17::Pex16, FBAINm::PaD17::Aco, GPD::YICPT1::Aco, YAT1::MCS::Lip1.

### Analysis Of Total Lipid Content And Composition By Flask Assay

Cells from YPD plates of strains Y8666, Y8669, Y8679 and Y8672 were grown and analyzed for total lipid content and composition, according to the General Methods.

**Table 21: Total Lipid Content And Composition In Yarrowia Strains Y8666, Y8669, Y8670 And Y8672 By Flask Assay**

| Strain | DCW (g/L) | TFAs % DCW | % TFAs | | | | | | | | | | | | | EPA % DCW |
|---|---|---|---|---|---|---|---|---|---|---|---|---|---|---|---|---|
| | | | 16:0 | 16:1 | 18:0 | 18:1 | 18:2 | ALA | EDA | DGLA | ARA | EtrA | ETA | EPA | other | |
| Y8666 | 3.2 | 25.2 | 2.3 | 0.3 | 2.3 | 4.1 | 15.1 | 1.3 | 1.7 | 1.4 | 0.7 | 0.6 | 1.3 | 62.2 | 6.7 | 15.6 |
| Y8669 | 3.2 | 26.4 | 2.3 | 0.3 | 2.3 | 4.1 | 15.7 | 1.4 | 1.8 | 1.6 | 0.7 | 0.5 | 1.1 | 61.5 | 6.7 | 16.3 |
| Y8670 | 3.2 | 27.3 | 1.9 | 0.4 | 3.4 | 4.3 | 17.0 | 1.5 | 2.2 | 1.7 | 0.6 | 0.5 | 1.1 | 60.9 | 4.5 | 16.6 |
| **Y8672** | **3.3** | **26.5** | **2.3** | **0.4** | **2.0** | **4.0** | **16.1** | **1.4** | **1.8** | **1.6** | **0.7** | **0.4** | **1.1** | **61.8** | **6.4** | **16.4** |

### EXAMPLE 8

### Construction Of Various Expression Vectors Comprising Different LPLAT ORFs

The present example describes the construction of a series of vectors, each comprising a LPLAT ORF, suitable for expression in *Yarrowia lipolytica.* LPLAT ORFs included the *Saccharomyces cerevisiae* Ale1, *Yarrowia lipolytica* Ale1, *Mortierella alpina* LPAAT1, *Yarrowia lipolytica* LPAAT1 and *Caenorhabditis elegans* LPCAT. Example 9 describes the results obtained following transformation of these vectors into *Yarrowia lipolytica* strain Y8406U.

### Origin Of LPLATs

A variety of LPLATs have been identified in the patent and open literature, but the functionality of these genes has not been previously directly compared. Table 22 summarizes publicly available LPLATs (i.e., ScAle1, ScLPAAT, MaLPAAT1 and CeLPCAT) and LPLAT orthologs identified herein (i.e., YIAle1 and YILPAAT1) that are utilized in the present Example, following codon-optimization of heterologous genes for expression in *Yarrowia lipolytica* (*infra*).

**Table 22: LPLATs Functionally Characterized**

| LPLAT | Organism | ORF Designation | References | SEQ ID NO |
|---|---|---|---|---|
| Ale1 | *Saccharomyces cerevisiae* * | ORF "YOR175C" or "ScAle1" | GenBank Accession No. | 14, 15 |
| | | | NP_014818; U.S. Pat. Appl. Pub. | |
| | | | No. 20080145867 (and corresponding to Intl. App. Pub. No. WO 2008/076377); Intl. App. Pub. No. WO 2009/001315 | |
| | *Yarrowia lipolytica* | "YALI0F19514p" or "YIAle1" | GenBank Accession No. | 16, 17 |
| | | | XP_505624; Intl. App. Pub. No. WO 2009/001315 | |
| LPAAT | *Saccharomyces cerevisiae* | ORF "YDL052C" or "ScLPAAT" | GenBank Accession No. | 32 |
| | | | NP_010231 | |
| | *Mortierella alpina* | "MaLPAAT1" | U.S. Pat. Appl. Pub. No. 2006-0115881-A1; U.S. Pat. Appl. Pub. No. 2009-0325265-A1 | 28,29 |
| | *Yarrowia lipolytica* | "YALI0E18964g" or "YILPAAT1" | GenBank Accession No. | 30, 31 |
| | | | XP_504127; U.S. Patent 7,189,559 | |
| LPCAT | *Caenorhabditis elegans** | "clone T06E8.1" or "CeLPCAT" | GenBank Accession No. CAA98276; Intl. App. Pub. No. WO 2004/076617 (corresponding to U.S. Pat. Appl. Pub. No. 2006-0168687-A1) | 24,25 |

| | | | | |
|---|---|---|---|---|
| *The *Saccharomyces cerevisiae Ale1* and *Caenorhabditis elegans* LPCAT were used as comparative Examples. | | | | |

More specifically, the ScLPAAT (SEQ ID NO:32) and ScAle1 (SEQ ID NO:15) protein sequences were used as queries to identify orthologs from the public *Y. lipolytica* protein database of the "Yeast project *Genolevures"* (Center for Bioinformatics, LaBRI, Talence Cedex, France) (see also Dujon, B. et al., Nature, 430(6995):35-44 (2004)). Based on analysis of the best hits, the Ale1 and LPAAT orthologs from *Yarrowia lipolytica* are identified herein as YIAle1 (SEQ ID NO:17) and YILPAAT (SEQ ID NO:31), respectively. The identity of YIAle1 and YILPAAT1 as orthologs of ScAle1 and ScLPAAT, respectively, was further confirmed by doing a reciprocal BLAST, i.e., using SEQ ID NOs:17 and 31 as a query against the *Saccharomyces cerevisiae* public protein database to find ScAle1 and ScLPAAT, respectively, as the best hits.

The LPLAT proteins identified above as ScAle1 (SEQ ID NO:15), YIAle1 (SEQ ID NO:17), ScLPAAT (SEQ ID NO:32), MaLPAAT1 (SEQ ID NO:29), YILPAAT1 (SEQ ID NO:31) and CeLPCAT (SEQ ID NO:25) were aligned using the method of Clustal W (slow, accurate, Gonnet option; Thompson et al., Nucleic Acids Res., 22:4673-4680 (1994)) of the MegAlign™ program (version 8.0.2) of the LASERGENE bioinformatics computing suite (DNASTAR, Inc., Madison, WI). This resulted in creation of Table 23, where percent similarity is shown in the upper triangle of the Table while percent divergence is shown in the lower triangle.

**Table 23: Percent Identity And Percent Divergence Among Various LPLATs**

| YILPAAT1 | CeLPCAT | MaLPAAT1 | ScAle1 | ScLPAAT | YIAle1 | |
|---|---|---|---|---|---|---|
| -- | 26.6 | 34.0 | 9.6 | 43.9 | 11.7 | YILPAAT1 |
| 184.3 | -- | 36.4 | 11.3 | 32.4 | 14.5 | CeLPCAT |
| 137.5 | 126.4 | -- | 11.1 | 34.6 | 15.0 | MaLPAAT1 |
| 545.0 | 442.0 | 456.0 | -- | 13.5 | 45.0 | ScAle1 |
| 97.9 | 145.7 | 134.5 | 365.0 | -- | 15.6 | ScLPAAT |
| 426.0 | 339.0 | 330.0 | 94.3 | 317.0 | -- | YIAle1 |

The percent identities revealed by this method allowed determination of the minimum percent identity between each of the LPAAT polypeptides and the minimum percent identity between each of the Ale1 polypeptides. The range of identity between LPAAT polypeptides was 34.0% identity (MaLPAAT1 and YILPAAT1) to 43.9% identity (ScLPAAT and YILPAAT1), while identity between the ScAle1 and YlAle1 polypeptides was 45%.

Membrane Bound *O*-Acyltransferase ["MBOAT"] Family Motifs: Orthologs of the ScAle1 protein sequence (SEQ ID NO:15) were identified by conducting a National Center for Biotechnology Information ["NCBI"] BLASTP 2.2.20 (protein-protein Basic Local Alignment Search Tool; Altschul et al., Nucleic Acids Res., 25:3389-3402 (1997); and Altschul et al., FEBS J., 272:5101-5109 (2005)) search using ScAle1 (SEQ ID NO:15) as the query sequence against fungal proteins in the "nr" protein database (comprising all non-redundant GenBank CDS translations, sequences derived from the 3-dimensional structure from Brookhaven Protein Data Bank ["PDB"], sequences included in the last major release of the SWISS-PROT protein sequence database, PIR and PRF excluding those environmental samples from WGS projects) using default parameters (expect threshold = 10; word size = 3; scoring parameters matrix = BLOSUM62; gap costs: existence = 11, extension = 1). The following hits were obtained:

**Table 24: Fungal Orthologs Of ScAle1 (SEQ ID NO:15) Based On BLAST Analysis**

| **Gen Bank Acession No.** | **Species** |
|---|---|
| NP_014818.1 | *Saccharomyces cerevisiae* |
| XP_001643411.1 | *Vandenwaltozyma polyspora* DSM 70294 |
| XP_448977.1 | *Candida glabrata* |
| XP_455985.1 | *Kluyveromyces lactis* |
| NP_986937.1 | *Ashbya gossypii* ATCC 10895 |
| XP_001385654.2 | *Pichia stipitis* CBS 6054 |
| XP_001487052.1 | *Pichia guilliermondii* ATCC 6260 |
| EDK36331.2 | *Pichia guilliermondii* ATCC 6260 |
| XP_001525914.1 | *Lodderomyces elongisporus* NRRL YB-4239 |
| XP_461358.1 | *Debaryomyces hansenii* CBS767 |
| XP_713184.1 | *Candida albicans* SC5314 |
| XP_001645053.1 | *Vandenwaltozyma polyspora* DSM 70294 |
| XP_505624.1 | *Yarrowia lipolytica* |
| XP_001805526.1 | *Phaeosphaeria nodorum* SN15 |
| XP_001598340.1 | *Sclerotinia sclerotiorum* 1980 |
| XP_001907785.1 | *Podospora anserina* |
| XP_001931658.1 | *Pyrenophora tritici-repentis* Pt-1C-BFP |
| XP_001560657.1 | *Botryotinia fuckeliana* B05.*10* |
| XP_963006.1 | *Neurospora crassa* OR74A |
| XP_364011.2 | *Magnaporthe grisea* 70-15 |
| XP_001209647.1 | *Aspergillus terreus* NIH2624 |
| XP_001822945.1 | *Aspergillus oryzae* RIB40 |
| XP_001257694.1 | *Neosartorya fischeri* NRRL 181 |
| XP_747591.2 | *Aspergillus fumigatus* Af293 |
| XP_001270060.1 | *Aspergillus clavatus* NRRL 1 |
| NP_596779.1 | *Schizosaccharomyces pombe* |
| XP_001396584.1 | *Aspergillus niger* |
| XP_001229385.1 | *Chaetomium globosum* CBS 148.51 |
| XP_001248887.1 | *Coccidioides immitis* RS |
| XP_664134.1 | *Aspergillus nidulans* FGSC A4 |
| XP_566668.1 | *Cryptococcus neoformans var. neoformans* JEC21 |
| XP_001839338.1 | *Coprinopsis cinerea okayama* 7#130 |
| XP_757554.1 | *Ustilago maydis* 521 |

| | |
|---|---|
| The yeast and fungal protein sequences of Table 24 were aligned using DNASTAR. Multiple sequence alignments and percent identity calculations were performed using the Clustal W method of alignment (*supra*)*.* | |

More specifically, default parameters for multiple protein alignment using the Clustal W method of alignment correspond to: GAP PENALTY=10, GAP LENGTH PENALTY=0.2, Delay Divergent Seqs(%)=30, DNA Transition Weight=0.5, Protein Weight Matrix=Gonnet Series, DNA Weight Matrix=IUB with the 'slow-accurate' option. The resulting alignment was analyzed to determine the presence or absence of the non-plant motifs for Ale1 homologs, as identified in U.S. Pat. Appl. Pub. No. 2008-0145867-A1. Specifically, these include: M-[V/I]-[L/I]-xxK-[L/V/I]-xxxxxxDG (SEQ ID NO:102), RxKYYxxWxxx-[E/D]-[A/G]xxxxGxG-[F/Y]-xG (SEQ ID NO:103), EX₁₁WNX₂-[T/V]-X₂W (SEQ ID NO:21) and SAxWHGxxPGYxx-[T/F]-F (SEQ ID NO:104), wherein X encodes any amino acid residue. The His residue in SEQ ID NO:104 has been reported to be a likely active site residue within the protein.

Only one motif, i.e., EX₁₁WNX₂-[T/V]-X₂W (SEQ ID NO:21), was completely conserved in all 33 of the organisms aligned. The remaining M-[V/I]-[L/I]-xxK-[L/V/I]-xxxxxxDG (SEQ ID NO:102), RxKYYxxWxxx-[E/D]-[A/G]xxxxGxG-[F/Y]-xG (SEQ ID NO:103) and SAxWHGxxPGYxx-[T/F]-F (SEQ ID NO:104) motifs were only partially conserved. Thus, these motifs were appropriately truncated to fit with 0 mismatch (i.e., SAxWHG [SEQ ID NO:20]), 1 mismatch (i.e., RxKYYxxW [SEQ ID NO:19]), or 2 mismatches (i.e., M(V/I)(L/I)xxK(LVI) [SEQ ID NO:18]) for the purposes of the present methodologies.

1-Acyl-*sn*-Glycerol-3-Phosphate Acyltransferase ["LPAAT"] Family Motifs: Analysis of the protein alignment comprising ScLPAAT (SEQ ID NO:18), MaLPAAT1 (SEQ ID NO:15) and YILPAAT1 (SEQ ID NO:17) revealed that the 1-acyl-*sn*-glycerol-3-phosphate acyltransferase family motif EGTR (SEQ ID NO:20) was present in each of the LPAAT orthologs. On this basis, MaLPAAT1 was identified as a likely LPAAT, that was clearly distinguishable from the *Morteriella alpina* LPAAT-like proteins disclosed in Intl. App. Pub. No. WO 2004/087902 (i.e., SEQ ID NOs:93 and 95).

It is noteworthy that the EGTR (SEQ ID NO:20) motif, while lacking in the LPCAT sequences in Intl. App. Pub. No. WO 2004/087902, is present in CeLPCAT (SEQ ID NO:2). It appears that other residues distinguish LPAAT and LPCAT sequences in LPAAT-like proteins. One such residue could be the extension of the EGTR (SEQ ID NO:20) motif. Specifically, whereas the EGTR motif in ScLPAAT (SEQ ID NO:18), MaLPAAT1 (SEQ ID NO:15) and YILPAAT1 (SEQ ID NO:17) is immediately followed by a serine residue, the EGTR motif in CeLPCAT is immediately followed by an asparagine residue. In contrast, the two LPCATs in Intl. App. Pub. No. WO 2004/087902 have a valine substituted for the arginine residue in the EGTR motif and the motif is immediately followed by a valine residue.

### Construction Of pY201, Comprising A Codon-Optimized Saccharomyces cerevisiae Ale1 Gene

The *Saccharomyces cerevisiae* ORF designated as "ScAle1" (SEQ ID NO:14) was optimized for expression in *Yarrowia lipolytica,* by DNA 2.0 (Menlo Park, CA). In addition to codon optimization, 5' *Pci*1 and 3' *Not*1 cloning sites were introduced within the synthetic gene (i.e., ScAle1S; SEQ ID NO:22). None of the modifications in the ScAle1S gene changed the amino acid sequence of the encoded protein (i.e., the protein sequence encoded by the codon-optimized gene [i.e., SEQ ID NO:23] is identical to that of the wildtype protein sequence [i.e., SEQ ID NO:15]). ScAle1S was cloned into pJ201 (DNA 2.0) to result in pJ201:ScAle1S.

A 1863 bp *Pci*1/*Not*1 fragment comprising ScAle1S was excised from pJ201:ScAle1S and used to create pY201 (SEQ ID NO:139; Table 25; FIG. 10A). In addition to comprising a chimeric YAT1::ScAle1S::Lip1 gene, pY201 also contains a *Y. lipolytica* URA3 selection marker flanked by LoxP sites for subsequent removal, if needed, by Cre recombinase-mediated recombination. Both the YAT1::ScAle1S::Lip1 chimeric gene and the URA3 gene were flanked by fragments having homology to 5' and 3' regions of the *Y. lipolytica* Pox3 gene to facilitate integration by double homologous recombination, although integration into *Y*. *lipolytica* is known to usually occur without homologous recombination. Thus, construct pY201 thereby contained the following components:

**Table 25: Description of Plasmid pY201 (SEQ ID NO:139)**

| RE Sites And Nucleotides Within SEQ ID NO:139 | Description Of Fragment And Chimeric Gene Components |
|---|---|
| *Bsi*W1/*Sbf*1 (1-1706 bp) | LoxP::*Ura3*::LoxP, comprising: |
| | • LoxP sequence (SEQ ID NO:140) |
| | • *Yarrowia lipolytica* Ura3 gene (GenBank Accession No. AJ306421); |
| | • LoxP sequence (SEQ ID NO:140) |
| *Sbf*1/*Sph*1 (1706-3043 bp) | 3' portion of *Yarrowia lipolytica* POX3 Acyl-CoA oxidase 3 (GenBank Accession No. YALI024750g) (i.e., bp 2215-3038 in pY201) |
| *Sph*1/*Asc*1 (3043-5743 bp) | • *ColE1* plasmid origin of replication; |
| | • Ampicillin-resistance gene (Amp^{R}) for selection in *E. coli* (i.e., bp 3598-4758 [complementary] in pY201); |
| | • *E. coli* f1 origin of replication |
| *Asc*I/*Bsi*WI (5743-6513 bp) | 5' portion of *Yarrowia lipolytica* POX3 Acyl-CoA oxidase 3 (GenBank Accession No. YALI0D24750g) (i.e., bp 5743-6512 in pY201) |
| *Bsi*WI/*Bsi*WI (6514-1 bp) | YAT1::ScAle1S::Lip1, comprising: |
| | • YAT1: *Yarrowia lipolytica* YAT1 promoter (U.S. Pat. Appl. Pub. No. 2006/0094102-A1) (i.e., bp 6514-7291 in pY201) |
| [a *Not*1 site, located between ScAle1S and Lip1 is present at bp 9154 bp] | • ScAle1S: codon-optimized Ale1 (SEQ ID NO:22) derived from *Saccharomyces cerevisiae* YOR175C (i.e., bp 7292-9151 in pY201; labeled as "Sc LPCATs ORF" in Figure); |
| | • Lip1: Lip1 terminator sequence from *Yarrowia Lip1* gene (GenBank Accession No. Z50020) (i.e., bp 9160-9481 pY201; labeled as "Lip1-3"' in Figure) |

### Construction Of pY168, Comprising A Yarrowia lipolytica Ale1 Gene

The *Yarrowia lipolytica* ORF designated as "YIAle1" (GenBank Accession No. XP_505624; SEQ ID NO:16) was amplified by PCR from *Yarrowia lipolytica* ATCC #20362 cDNA library using PCR primers 798 and 799 (SEQ ID NOs:141 and 142, respectively). Additionally, the YAT promoter was amplified by PCR primers 800 and 801 (SEQ ID NOs:143 and 144, respectively) from pY201 (SEQ ID NO:139). Since the primer pairs were designed to create two PCR products having some overlap with one another, a YAT1::YIAle1 fusion fragment was then amplified by overlapping PCR using primers 798 and 801 (SEQ ID NOs:141 and 144, respectively) and the two PCR fragments as template. The PCR was carried out in a RoboCycler Gradient 40 PCR machine (Stratagene) using the manufacturer's recommendations and Pfu Ultra™ High-Fidelity DNA Polymerase (Stratagene, Cat. No. 600380). Amplification was carried out as follows: initial denaturation at 95 °C for 4 min, followed by 30 cycles of denaturation at 95 °C for 30 sec, annealing at 55 °C for 1 min, and elongation at 72 °C for 1 min. A final elongation cycle at 72 °C for 10 min was carried out, followed by reaction termination at 4 °C.

The PCR product comprising the YAT1::YI Ale1 fusion fragment was gel purified and digested with *Cla*I/*Not*I*.* This *Cla*1-*Not*1 fragment was ligated into pY201 that had been similarly digested (thereby removing the YAT1::ScAle1S fragment) to create pY168 (SEQ ID NO:145), comprising a chimeric YAT1::YlAle1::Lip1 gene. The DNA sequence of the *Yarrowia* Ale1 ORF was confirmed by DNA sequencing. The components present in pY168 (FIG. 10B; SEQ ID NO:145) are identical to those present in pY201, with the exception of the YAT1::YIAle1::Lip1 gene in pY168, instead of the YAT1::ScAle1 S::Lip1 gene in pY201 (FIG. 10A). Note that YIAle1 is labeled as "YI LPCAT" in FIG. 10B.

### Construction Of pY208, Comprising A Mortierella alpina LPAAT1 Gene

The *Mortierella alpina* ORF designated as "MaLPAAT1" (SEQ ID NO:28) was optimized for expression in *Yarrowia lipolytica,* by DNA 2.0 (Menlo Park, CA). In addition to codon optimization, 5' *Pci*1 and 3' *Not*1 cloning sites were introduced within the synthetic gene (i.e., MaLPAAT1S; SEQ ID NO:35). None of the modifications in the MaLPAAT1S gene changed the amino acid sequence of the encoded protein (i.e., the protein sequence encoded by the codon-optimized gene [i.e., SEQ ID NO:36] is identical to that of the wildtype protein sequence [i.e., SEQ ID NO:29]). MaLPAAT1S was cloned into pJ201 (DNA 2.0) to result in pJ201:MaLPAAT1 S.

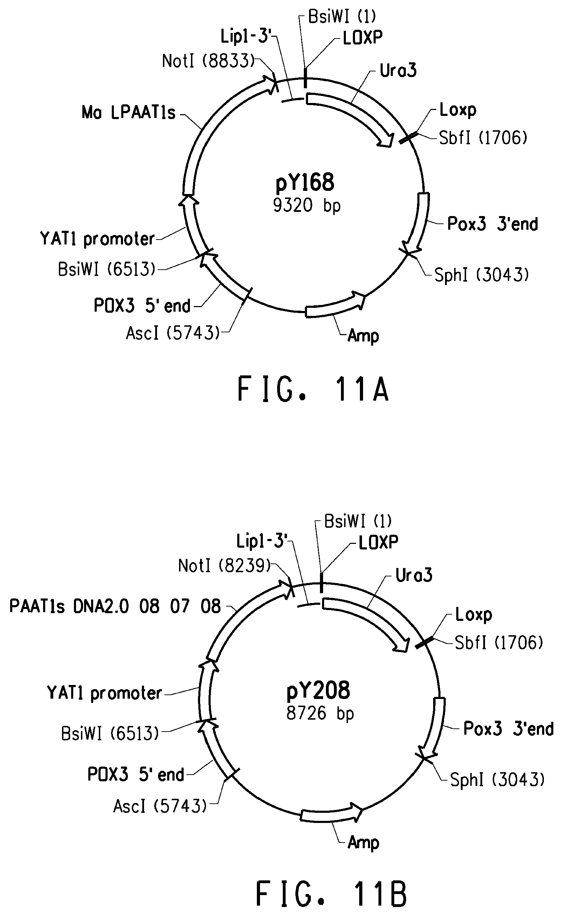

A 945 bp *Pci*1/*Not*1 fragment comprising MaLPAAT1S was excised from pJ201 :MaLPAAT1S and used to create pY208 (SEQ ID NO:146), in a 3-way ligation with two fragments of pY201 (SEQ ID NO:139). Specifically, the MaLPAAT1 fragment was ligated with a 3530 bp *Sph-Not*I pY201 fragment and a 4248 bp *Nco*I*-Sph*I pY201 fragment to result in pY208. The components present in pY208 (FIG. 11A; SEQ ID NO:146) are identical to those present in pY201, with the exception of the YAT1::MaLPAAT1S::Lip1 gene in pY208, instead of the YAT1::ScAle1S::Lip1 gene in pY201 (FIG. 10A).

### Construction Of pY207, Comprising A Yarrowia lipolytica LPAAT1 Gene

A putative LPAAT1 from *Yarrowia lipolytica* (designated herein as "YILPAAT1"; SEQ ID NOs:30 and 31) was described in U.S. Patent 7,189,559 and GenBank Accession No. XP_504127. The protein is annotated as "similar to uniprot|P33333 *Saccharomyces cerevisiae* YDL052c SLC1 fatty acyltransferase".

The YILPAAT1 ORF (SEQ ID NO:30) was amplified by PCR using a *Yarrowia lipolytica* ATCC #20362 cDNA library as a template and PCR primers 856 and 857 (SEQ ID NOs:147 and 148, respectively). The PCR was conducted using the same components and conditions as described above for amplification of the YAT1::Yl Ale1 fusion fragment, prior to synthesis of pY168.

The PCR product comprising the YILPAAT1 ORF was digested with *Pci*I and *Not*I and then utilized in a 3-way ligation with two fragments from pY168. Specifically, the YILPAAT1 fragment was ligated with a 3530 bp *Sph-Not*I pY168 fragment and a 4248 bp *Nco*I*-Sph*I pY168 fragment, to produce pY207, comprising a chimeric YAT1::YILPAAT1::Lip1 gene. The *Y. lipolytica* LPAAT1 ORF was confirmed by DNA sequencing. The components present in pY207 (FIG. 11B; SEQ ID NO:149) are identical to those present in pY201, with the exception of the chimeric YAT1::YILPAAT1::Lip1 gene in pY207, instead of the YAT1 ::ScAle1S::Lip1 gene in pY201 (FIG. 10A). Note that YILPAAT1 is labeled as "YI LPAT1 ORF" in FIG. 11 B.

### Construction Of pY175, Comprising A Caenorhabditis elegans LPCAT Gene

The *Caenorhabditis elegans* ORF designated as "CeLPCAT" (SEQ ID NO:24) was optimized for expression in *Yarrouwia lipolytica,* by GenScript Corporation (Piscataway, NJ). In addition to codon optimization, 5' *Nco*1 and 3' *Not*1 cloning sites were introduced within the synthetic gene (i.e., CeLPCATS; SEQ ID NO:26). None of the modifications in the CeLPCATS gene changed the amino acid sequence of the encoded protein (i.e., the protein sequence encoded by the codon-optimized gene [i.e., SEQ ID NO:27] is identical to that of the wildtype protein sequence [i.e., SEQ ID NO:25]).

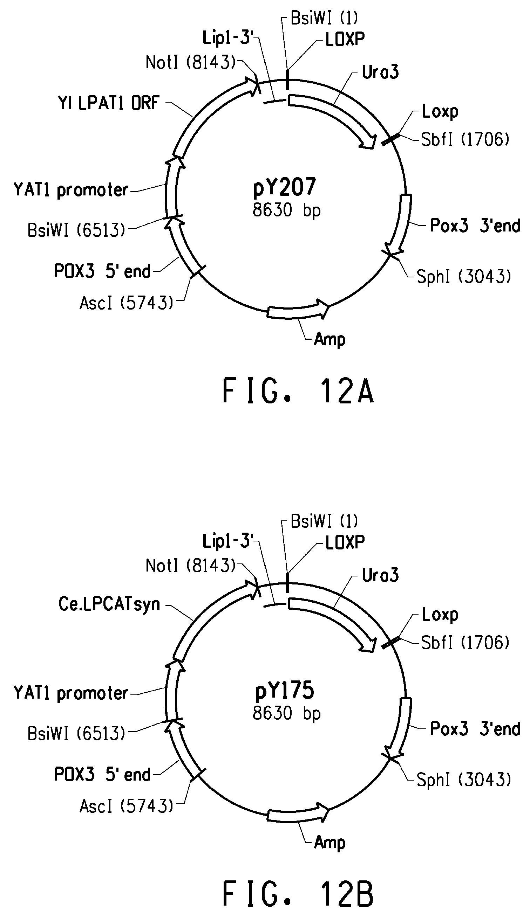

A *Nco*1-*Not*1 fragment comprising CeLPCATS was used to create pY175 (SEQ ID NO:150), in a 3-way ligation with two fragments from pY168 (SEQ ID NO:145). Specifically, the *Nco*1-*Not*1 fragment comprising CeLPCATS was ligated with a 3530 bp *Sph-Not*I pY168 fragment and a 4248 bp *Nco*I*-Sph*I pY168 fragment to result in pY175. The components present in pY175 (FIG. 12A; SEQ ID NO:150) are identical to those present in pY201, with the exception of the YAT1::CeLPCATS::Lip1 gene in pY175, instead of the YAT1 ::ScAle1S::Lip1 gene in pY201 (FIG. 10A). Note that CeLPCATS is labeled as "Ce.LPCATsyn" in FIG. 12A.

### EXAMPLE 9

### Functional Characterization Of Different LPLATs In EPA-Producing Yarrowia lipolytica Strain Y8406

*Yarrowia lipolytica* strain Y8406U, producing EPA, was used to functionally characterize the effects of overexpression of the *Saccharomyces cerevisiae* Ale1, *Yarrowia lipolytica* Ale1, *Mortierella alpina* LPAAT1, *Yarrowia lipolytica* LPAAT1 and *Caenorhabditis elegans* LPCAT, following their stable integration into the *Yarrowia* host chromosome. This was in spite of the host containing its native LPLATs, i.e., Ale1 and LPAAT1.

### Transformation And Growth

In order to disrupt the *Ura3* gene (Example 3), construct pZKUM (FIG. 5A; SEQ ID NO:130; described in Table 15 of U.S. Pat. Appl. Pub. No. 2009-0093543-A1) was used to integrate a *Ura3* mutant gene into the *Ura3* gene of strain Y8406 in a manner similar to that described for pZKUM transformation of strain Y8006 (Example 3). Several transformants were grown and identified to possess a *Ura-* phenotype.

GC analyses showed that there were 26.1% EPA of FAMEs in pZKUM-transformant strains #4 and #5. These two strains were designated as strains Y8406U1 and Y8406U2, respectively (collectively, Y8406U).

*Yarrowia lipolytica* strain Y8406U was then individually transformed with linear *Sph*I*-Asc*I fragments of the integrating vectors described in Example 8, wherein each LPLAT was under the control of the *Yarrowia* YAT1 promoter. Specifically, vectors pY201 (YAT1::ScAle1S::Lip1), pY168 (YAT1::YIAle1::Lip1), pY208 (YAT1::MaLPAAT1S::Lip1), pY207 (YAT1::YILPAAT1::Lip1) and pY175 (YAT1::CeLPCATS::Lip1) were transformed according to the General Methods.

Each transformation mix was plated on MM agar plates. Several resultant URA+ transformants were picked and inoculated into 3 mL FM medium (Biomyx Cat. No. CM-6681, Biomyx Technology, San Diego, CA) containing per L: 6.7 g Difco Yeast Nitrogen Base without amino acids, 5 g Yeast Extract, 6 g KH₂PO₄, 2 g K₂HPO₄, 1.5 g MgSO₄·7H₂0, 1.5 mg thiamine·HCl, and 20 g glucose. After 2 days growth on a shaker at 200 rpm and 30 °C, the cultures were harvested by centrifugation and resuspended in 3 mL HGM medium (Cat. No. 2G2080, Teknova Inc., Hollister, CA) containing 0.63% monopotassium phosphate, 2.7% dipotassium phosphate, 8.0% glucose, adjusted to pH 7.5. After 5 days growth on a shaker at 200 rpm and at 30 °C, 1 mL aliquots of the cultures were harvested by centrifugation and analyzed by GC. Specifically, the cultured cells were collected by centrifugation for 1 min at 13,000 rpm, total lipids were extracted, and fatty acid methyl esters ["FAMEs"] were prepared by trans-esterification, and subsequently analyzed with a Hewlett-Packard 6890 GC (General Methods).

Based on the fatty acid composition of the 3 mL cultures, selected transformants were further characterized. Specifically, clones #5 and #11 of strain Y8406U transformed with expression vector pY201 (comprising ScAle1S) were selected and designated as "Y8406U::ScAle1S-5" and "Y8406U::ScAle1S-11", respectively; clone #16 of strain Y8406U transformed with expression vector pY168 (comprising YlAle1) was selected and designated as "Y8406U::YlAle1"; clone #8 of strain Y8406U transformed with expression vector pY208 (comprising MaLPAAT1S) was selected and designated as "Y8406U::MaLPAAT1S"; clone #21 of strain Y8406U transformed with expression vector pY207 (comprising YILPAAT1) was selected and designated as "Y8406U::YILPAAT1"; and clone #23 of strain Y8406U transformed with expression vector pY175 (comprising CeLPCATS) was selected and designated as "Y8406U::CeLPCATS". Additionally, strain Y8406 (a Ura+ strain that was parent to strain Y8406U (Ura-)) was used as a control.

Each selected transformant and the control was streaked onto MM agar plates. Then, one loop of freshly streaked cells was inoculated into 3 mL FM medium and grown overnight at 250 rpm and 30 °C. The OD₆₀₀nm was measured and an aliquot of the cells were added to a final OD₆₀₀nm of 0.3 in 25 mL FM medium in a 125 mL flask. After 2 days in a shaker incubator at 250 rpm and at 30 °C, 6 mL of the culture was harvested by centrifugation and resuspended in 25 mL HGM in a 125 mL flask. After 5 days in a shaker incubator at 250 rpm and at 30 °C, a 1 mL aliquot was used for GC analysis (*supra*) and 10 mL dried for dry cell weight ["DCW"] determination.

For DCW determination, 10 mL culture was harvested by centrifugation for 5 min at 4000 rpm in a Beckman GH-3.8 rotor in a Beckman GS-6R centrifuge. The pellet was resuspended in 25 mL of water and re-harvested as above. The washed pellet was re-suspended in 20 mL of water and transferred to a pre-weighed aluminum pan. The cell suspension was dried overnight in a vacuum oven at 80 °C. The weight of the cells was determined.

### Lipid Content, Fatty Acid Composition And Conversion Efficiencies

A total of four separate experiments were conducted under identical conditions. Experiment 1 compared control strain Y8406 versus strain Y8406U::ScAle1S-5. Experiment 2 compared control strain Y8406 versus strain Y8406U::YlAle1. Experiment 3 compared control strain Y8406 versus strain Y8406U::YlAle1, strain Y8406U::ScAle1 S-11, and strain Y8406U::MaLPAAT1S. Experiment 4 compared control strain Y8406 versus strain Y8406U::MaLPAAT1S, strain Y8406U::YILPAAT1 and strain Y8406U::CeLPCATS.

In each experiment, the lipid content, fatty acid composition and EPA as a percent of the DCW are quantified for 1, 2 or 3 replicate cultures ["Replicates"] of the control Y8406 strain and the transformant Y8406U strain(s). Additionally, data for each Y8406U transformant is presented as a % of the Y8406 control. Table 26 below summarizes the total lipid content of cells ["TFAs % DCW"], the concentration of each fatty acid as a weight percent of TFAs ["% TFAs"] and the EPA content as a percent of the dry cell weight ["EPA % DCW"]. More specifically, fatty acids are identified as 16:0 (palmitate), 16:1 (palmitoleic acid), 18:0 (stearic acid), 18:1 (oleic acid), 18:2 (LA), ALA, EDA, DGLA, ARA, ETrA, ETA and EPA.

Table 27 summarizes the conversion efficiency of each desaturase and the Δ9 elongase functioning in the PUFA biosynthetic pathway and which are required for EPA production. Specifically, the Δ12 desaturase conversion efficiency ["Δ12 CE"], Δ8 desaturase conversion efficiency ["Δ8 CE"], Δ5 desaturase conversion efficiency ["Δ5 CE"], Δ17 desaturase conversion efficiency ["Δ17 CE"] and Δ9 elongation conversion efficiency ["Δ9e CE"] are provided for each control Y8406 strain and the transformant Y8406U strain(s); data for each Y8406U transformant is presented as a % of the Y8406 control. Conversion efficiency was calculated according to the formula:
([product]/[substrate+product])*100, where product includes both product and product derivatives.

**Table 26: Lipid Content And Composition In LPCAT Transformant Strains Of Yarrowia lipolytica Y8406**

| **Expt.** | **Strain** | **Repli cates** | **TFA % DCW** | **% TFAs** | | | | | | | | | | | | **EPA % DCW** |
|---|---|---|---|---|---|---|---|---|---|---|---|---|---|---|---|---|
| | | | | **16: 0** | **16: 1** | **18: 0** | **18: 1** | **18:2** | **ALA** | **EDA** | **DGLA** | **ARA** | **ERA** | **ETA** | **EPA** | |
| | Y8406 | AVG.3 | 17.6 | 3.8 | 0.7 | 3.3 | 6.4 | 22.6 | 2.5 | 2.8 | 2.2 | 0.5 | 1.9 | 2.0 | 48.9 | 8.6 |
| 1 | Y8406U:: | AVG.3 | 18.3 | 4.2 | 0.7 | 3.5 | 5.7 | 15.1 | 0.6 | 3.3 | 3.7 | 0.8 | 1.8 | 2.3 | 56.9 | 10.4 |
| | ScAle1S-5 | % Ctrl | 104 | 111 | 100 | 106 | 89 | 67 | 24 | 118 | 168 | 160 | 95 | 115 | 116 | 121 |
| | Y8406 | AVG.3 | 23.2 | 3.5 | 0.6 | 3.3 | 6.4 | 22.3 | 2.7 | 2.6 | 2.1 | 0.5 | 1.6 | 2.0 | 49.9 | 11.6 |
| 2 | Y8406U:: | AVG.3 | 22.3 | 3.8 | 0.7 | 2.9 | 3.9 | 12.7 | 0.4 | 3.0 | 3.8 | 0.8 | 1.6 | 2.4 | 60.9 | 13.6 |
| | YIAle1 | % Ctrl | 96 | 109 | 117 | 88 | 61 | 57 | 15 | 115 | 181 | 160 | 100 | 120 | 122 | 117 |
| | Y8406 | 1 | 26.1 | 2.7 | 0.7 | 2.8 | 6.5 | 20.5 | 2.5 | 3.2 | 2.3 | 0.7 | 0.8 | 0.0 | 50.8 | 13.3 |
| | Y8406U:: | AVG.2 | 23.3 | 3.3 | 0.7 | 2.4 | 3.6 | 12.1 | 0.5 | 3.2 | 3.5 | 0.9 | 0.0 | 2.3 | 62.2 | 14.5 |
| | YIAle1 | % Ctrl | 89 | 122 | 100 | 86 | 55 | 59 | 20 | 100 | 152 | 129 | 0 | na | 122 | 109 |
| 3 | Y8406U:: | AVG.2 | 28.0 | 3.0 | 0.7 | 3.0 | 5.5 | 13.1 | 0.6 | 3.5 | 3.8 | 0.9 | 0.0 | 2.4 | 58.5 | 16.4 |
| | ScAle1S-11 | % Ctrl | 107 | 111 | 100 | 107 | 85 | 64 | 24 | 109 | 165 | 129 | 0 | na | 115 | 123 |
| | Y8406U:: | AVG.2 | 23.7 | 4.4 | 0.8 | 4.2 | 6.6 | 11.2 | 0.7 | 2.7 | 3.7 | 0.9 | 0.0 | 2.5 | 57.0 | 13.5 |
| | MaLPAAT1S | % Ctrl | 91 | 163 | 114 | 150 | 102 | 55 | 28 | 84 | 161 | 129 | 0 | na | 112 | 102 |
| 4 | Y8406 | AVG.2 | 27.9 | 2.8 | 0.6 | 3.1 | 6.2 | 20.6 | 2.9 | 2.9 | 2.0 | 0.6 | 0.7 | 2.0 | 49.4 | 13.8 |
| | Y8406U:: | AVG.2 | 25.2 | 4.8 | 0.8 | 4.8 | 6.9 | 11.6 | 0.8 | 2.5 | 3.0 | 0.7 | 0.0 | 2.3 | 55.3 | 14.0 |
| | MaLPAAT1S | % Ctrl | 90 | 171 | 133 | 155 | 111 | 56 | 28 | 86 | 150 | 117 | 0 | 115 | 112 | 101 |
| | Y8406U:: | AVG.2 | 25.2 | 3.7 | 0.7 | 4.2 | 6.2 | 13.0 | 1.2 | 2.3 | 2.6 | 0.6 | 0.0 | 2.2 | 56.7 | 14.3 |
| | YILPAAT1 | % Ctrl | 90 | 132 | 117 | 135 | 100 | 63 | 41 | 79 | 130 | 100 | 0 | 110 | 115 | 104 |
| | Y8406U:: | AVG.2 | 24.7 | 3.8 | 0.6 | 4.6 | 7.1 | 13.9 | 1.6 | 2.3 | 2.6 | 0.6 | 0.4 | 2.2 | 53.6 | 13.2 |
| | CeLPCATS | % Ctrl | 89 | 136 | 100 | 148 | 115 | 67 | 55 | 79 | 130 | 100 | 57 | 110 | 109 | 96 |

**Table 27: Desaturase And Elongate Conversion Efficiency In LPCAT Transformant Strains Of Yarrowia lipolytica Y8406**

| **Expt.** | **Strain** | **Replicates** | **Δ12 CE** | **Δ9e CE** | **Δ8 CE** | **Δ5 CE** | **Δ17 CE** |
|---|---|---|---|---|---|---|---|
| | Y8406 | AVG.3 | 93 | 70 | 92 | 92 | 90 |
| 1 | Y8406U::ScAle1S-5 | AVG.3 | 94 | 81 | 93 | 91 | 89 |
| | | % Ctrl | 101 | 116 | 101 | 98 | 98 |
| | Y8406 | AVG.3 | 93 | 70 | 93 | 93 | 91 |
| 2 | Y8406U::YlAle1 | AVG.3 | 96 | 85 | 94 | 91 | 90 |
| | | % Ctrl | 103 | 121 | 101 | 98 | 98 |
| | Y8406 | 1 | 93 | 72 | 93 | 96 | 89 |
| | Y8406U::YlAle1 | AVG.2 | 96 | 85 | 96 | 92 | 89 |
| | | % Ctrl | 104 | 119 | 103 | 96 | 100 |
| 3 | Y8406U::ScAle1S-11 | AVG.2 | 94 | 83 | 95 | 91 | 88 |
| | | % Ctrl | 101 | 117 | 102 | 95 | 99 |
| | Y8406U::MaLPAAT1S | AVG.2 | 92 | 85 | 96 | 90 | 89 |
| | | % Ctrl | 100 | 119 | 103 | 94 | 100 |
| 4 | Y8406 | AVG.2 | 93 | 71 | 94 | 93 | 91 |
| | Y8406U::MaLPAAT1S | AVG.2 | 92 | 84 | 96 | 91 | 90 |
| | | % Ctrl | 99 | 118 | 102 | 99 | 100 |
| | Y8406U::YILPAAT1 | AVG.2 | 93 | 82 | 96 | 92 | 92 |
| | | % Ctrl | 100 | 115 | 103 | 100 | 101 |
| | Y8406U::CeLPCATS | AVG.2 | 92 | 80 | 96 | 92 | 91 |
| | | % Ctrl | 99 | 113 | 102 | 99 | 100 |

Based on the data concerning Experiments 1, 2 and 3 in Table 26 and Table 27, overexpression of LPLAT in EPA strains Y8406U::ScAle1S-5, Y8406U::ScAle1S-11, Y8406U::YlAle1 and Y8406U::MaLPAAT1 S results in significant reduction (to 67% or below of the control) of the concentration of LA (18:2) as a weight % of TFAs ["LA % TFAs"], an increase (to at least 12% of the control) in the concentration of EPA as a weight % of TFAs ["EPA % TFAs"], and an increase (to at least 16% of the control) in the conversion efficiency of the Δ9 elongase. Compared to Y8406U::ScAle1S-5 and Y8406U::ScAle1S-11, Y8406U::YlAle1 has lower LA % TFAs, higher EPA % TFAs, better Δ9 elongation conversion efficiency, and slightly lower TFAs % DCW and EPA % DCW. Y8406U::YlAle1 and Y8406U::MaLPAAT1S are similar except overexpression of MaLPAAT1S resulted in lower LA % TFAs, EPA % TFAs, and EPA % DCW.

Experiment 4 shows that overexpression of LPLAT in EPA strains Y8406U::YlAle1, Y8406U::MaLPAAT1 S and Y8406U::CeLPCATS results in significant reduction (to 67% or below of the control) of LA % TFAs, an increase (to at least 9% of the control) in EPA % TFAs, and an increase (to at least 13% of the control) in the conversion efficiency of the Δ9 elongase. Compared to Y8406U::CeLPCATS, Y8406U::YILPAAT1 and Y8406U::MaLPAAT1S both have lower LA % TFAs, higher EPA % TFAs, higher EPA % DCW, and slightly better TFAs % DCW. Y8406U::YILPAAT1 and Y8406U::MaLPAAT1S are similar except overexpression of MaLPAAT1S results in lower LA % TFAs, slightly lower EPA % TFAs and EPA % DCW, and slightly better Δ9 elongase conversion efficiency.

It is well known in the art that most desaturations occur at the sn-2 position of phospholipids, while fatty acid elongations occur on acyl-CoAs. Furthermore, ScAle1S, YlAle1, MaLPAAT1S and YILPAAT1 were expected to only incorporate acyl groups from the acyl-CoA pool into the sn-2 position of lysophospholipids, such as lysophosphatidic acid ["LPA"] and lysophosphatidylcholine ["LPC"]. Thus, it was expected that expression of ScAle1S, YlAle1, MaLPAAT1S, and YILPAAT1 would result in improved desaturations due to improved substrate availability in phospholipids, and not result in improved elongations that require improved substrate availability in the CoA pool. Our data (*supra*) shows that unexpectedly, expression of ScAle1S, YlAle1, MaLPAAT1S and YILPAAT1 significantly improved the Δ9 elongase conversion efficiency in strains of *Yarrowia* producing EPA but did not improve the desaturations (measured as Δ12 desaturase conversion efficiency, Δ8 desaturase conversion efficiency, Δ5 desaturase conversion efficiency or Δ17 desaturase conversion efficiency).

CeLPCAT was previously shown to improve Δ6 elongation conversion efficiency in *Saccharomyces cerevisiae* fed LA or GLA (Intl. App. Pub. No. WO 2004/076617). This was attributed to its reversible LPCAT activity that released fatty acids from phospholipids into the CoA pool. An improvement in Δ9 elongation conversion efficiency in an oleaginous microbe, such as *Yarrowia lipolytica,* engineered for high level LC-PUFA production in the absence of feeding fatty acids was not contemplated in Intl. App. Pub. No. WO 2004/076617.

Furthermore, expression of ScAle1S, YlAle1, MaLPAAT1S, YILPAAT1 and CeLPCATS did not significantly alter either the level of PUFAs accumulated or the total lipid content in strains of *Yarrowia* producing EPA.

Previous studies have shown that both Δ6 elongation and Δ9 elongation are bottlenecks in long chain PUFA biosynthesis due to poor transfer of acyl groups between phospholipid and acyl-CoA pools. Based on the improved Δ9 elongase conversion efficiency resulting from overexpression of LPLATs, demonstrated above, it is anticipated that the LPLATs described herein and their orthologs, such as ScLPAAT, will also improve Δ6 elongation conversion efficiency.

### EXAMPLE 10

### Construction Of Expression Vectors Comprising LPAAT ORFs And An Autonomously Replicating Sequence

The present example describes the construction of vectors comprising autonomously replicating sequences ["ARS"] and LPAAT ORFs suitable for LPAAT gene expression without integration in *Yarrowia lipolytica.* ORFs included the *Saccharomyces cerevisiae* LPAAT encoding SEQ ID NO:32 and the *Yarrowia lipolytica* LPAAT1 encoding SEQ ID NO:31. Example 11 describes the results obtained following transformation of these vectors into *Y. lipolytica.*

### Construction Of pY222, Comprising A Codon-Optimized Saccharomyces cerevisiae LPAAT Gene

The *Saccharomyces cerevisiae* ORF designated as "ScLPAAT" (SEQ ID NO:32) was optimized for expression in *Yarrowia lipolytica,* by DNA 2.0 (Menlo Park, CA). In addition to codon optimization, 5' *Pci*1 and 3' *Not*1 cloning sites were introduced within the synthetic gene (i.e., ScLPAATS; SEQ ID NO:151). None of the modifications in the ScLPAATS gene changed the amino acid sequence of the encoded protein (i.e., the protein sequence encoded by the codon-optimized gene [i.e., SEQ ID NO:152] is identical to that of the wildtype protein sequence [i.e., SEQ ID NO:32]). ScLPAATS was cloned into pJ201 (DNA 2.0) to result in pJ201 :ScLPAATS.

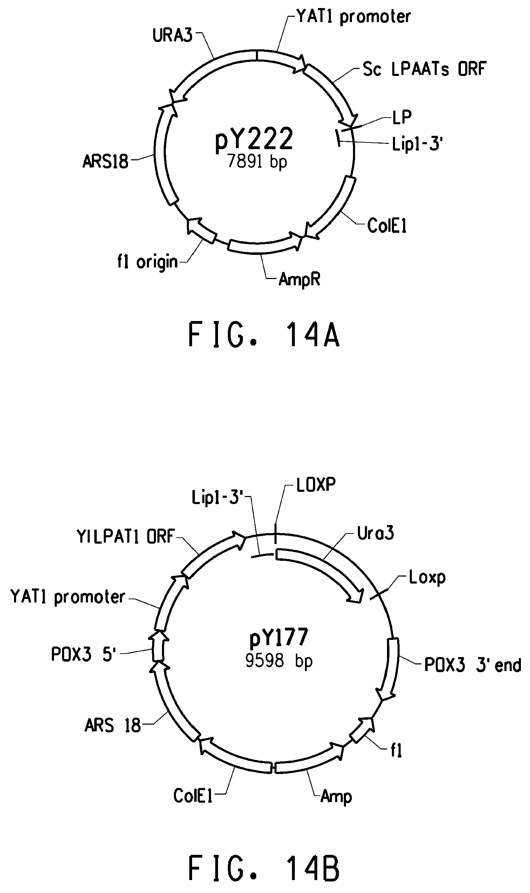

A 926 bp *Pci*1/*Not*1 fragment comprising ScLPAATS was excised from pJ201 :ScLPAATS and cloned into *Nco*I-*Not*1 cut pYAT-DG2-1 to create pY222 (SEQ ID NO:153; Table 28; FIG. 14A). Thus, pY222 contained the following components:

**Table 28: Description of Plasmid pY222 (SEQ ID NO:153)**

| RE Sites And Nucleotides Within SEO ID NO:153 | Description Of Fragment And Chimeric Gene Components |
|---|---|
| *Sal*1/*Swa*I (1-2032) | YAT1::ScLPAATS::Lip1, comprising: |
| | • YAT1: *Yarrowia lipolytica* YAT1 promoter (U.S. Pat. Appl. Pub. No. 2006/0094102-A1); |
| | • ScLPAATS: codon-optimized ScLPAATS (SEQ ID NO:151) (labeled as "Sc LPAATs ORF" in Figure); |
| | • Lip1: Lip1 terminator sequence from *Yarrowia Lip1* gene (GenBank Accession No. Z50020) (labeled as "Lip1-3"' in Figure) |
| *Swa*I/*Ava*I (2032-4946) | • *ColE1* plasmid origin of replication; |
| | • Ampicillin-resistance gene (Amp^{R}) for selection in *E. coli*; |
| | *• E. coli* f1 origin of replication |
| *Aval-Sphl* (4946-6330) | *Yarrowia lipolytica* centromere and autonomously replicating sequence ["ARS"] 18 locus |
| *Sph*I-*Sal*I (6330-1) | *Yarrowia lipolytica* URA3 gene (GenBank Accession No. AJ306421) |

### Construction Of pY177, Comprising A Yarrowia lipolytica LPAAT1 Gene

The *Yarrowia lipolytica* centromere and autonomously replicating sequence ["ARS"] was amplified by standard PCR using primer 869 (SEQ ID NO:154) and primer 870 (SEQ ID NO:155), with plasmid pYAT-DG2-1 as template. The PCR product was digested with *Asc*I/*Avr*II and cloned into *Asc*I*-Avr*II digested pY207 (SEQ ID NO:149; Example 8) to create pY177 (SEQ ID NO:156; Table 29; FIG. 14B). Thus, the components present in pY177 are identical to those in pY207 (FIG. 12A), except for the replacement of the 373 bp pY207 sequence between *Asc*I and *Avr*II with the 1341 bp sequence contaning ARS. More specifically, pY177 contained the following components:

**Table 29: Description of Plasmid pY177 (SEQ ID NO:156)**

| RE Sites And Nucleotides Within SEO ID NO:156 | Description Of Fragment And Chimeric Gene Components |
|---|---|
| *Bsi*W1/*Sbf*1 (1-1706 bp) | LoxP::*Ura3*::LoxP, comprising: |
| | • LoxP sequence (SEQ ID NO:140) |
| | • *Yarrowia lipolytica* Ura3 gene (GenBank Accession No. AJ306421); |
| | • LoxP sequence (SEQ ID NO:140) |
| *Sbf*1/*Sph*1 (1706-3043 bp) | 3' portion of *Yarrowia lipolytica* POX3 Acyl-CoA oxidase 3 (GenBank Accession No. YALI0D24750g) |
| *Sph*I/*Asc*I (3043-5743 bp) | • *ColE1* plasmid origin of replication; |
| | • Ampicillin-resistance gene (Amp^{R}) for selection in *E. coli;* |
| | • *E. coli* f1 origin of replication |
| AscI/*Bsi*WI (5743-6513 bp) | 5' portion of *Yarrowia lipolytica* POX3 Acyl-CoA oxidase 3 (GenBank Accession No. YALI0D24750g) |
| *AscI*/*Avr*II (5743-7084 bp) | *Yarrowia lipolytica* centromere and autonomously replicating sequence ["ARS"] 18 locus |
| *Avr*II/*Bsi*WI (7084-7481 bp) | 5' portion of *Yarrowia lipolytica* POX3 Acyl-CoA oxidase 3 (GenBank Accession No. YALI0D24750g) |
| *Bsi*WI/ *Bsi*WI | YAT1::YILPAAT1::Lip1, comprising: |
| (7481-1 bp) | • YAT1: *Yarrowia lipolytica* YAT1 promoter (U.S. Pat. Appl. Pub. No. 2006/0094102-A1); |
| | • YILPAAT1: *Yarrowia lipolytica* LPAAT1 ("YALI0E18964g"; GenBank Accession No. XP_504127) (SEQ ID NO:30) (labeled as "YI LPAT1 ORF" in Figure); |
| | • Lip1: Lip1 terminator sequence from *Yarrowia Lip1* gene (GenBank Accession No. Z50020) (labeled as "Lip1-3"' in Figure) |

### EXAMPLE 11

### Functional Characterization Of Different LPAATs In EPA-Producing Yarrowia lipolytica Strain Y8406

*Yarrowia lipolytica* strain Y8406U, producing EPA, was used to functionally characterize the effects of expression of the *Saccharomyces cerevisiae* LPAATS (SEQ ID NO:151) and *Yarrowia lipolytica* LPAAT1 (SEQ ID NO:30) without integration on self-replicating plasmids. This was in spite of the host containing its native LPAATs.

### Transformation And Growth

*Yarrowia lipolytica* strain Y8406U (Example 9) was individually transformed with uncut plasmids from Example 10. Specifically, vectors pY177 (YAT1::YILPAAT1::Lip1) [SEQ ID NO:156] and pY222 (YAT1::ScLPAATS::Lip1) [SEQ ID NO:153] were transformed according to the General Methods.

Each transformation mix was plated on MM agar plates. Several resultant URA+ transformants were picked and inoculated into 3 mL CSM-U medium (Teknova Cat. No. C8140, Teknova Inc., Hollister, CA), wherein CSM-U medium refers to CM Broth with glucose minus uracil containing 0.13% amino acid dropout powder minus uracil, 0.17% yeast nitrogen base, 0.5% (NH₄)₂SO₄, and 2.0% glucose. After 2 days growth on a shaker at 200 rpm and 30 °C, the cultures were harvested by centrifugation and resuspended in 3 mL HGM medium (Cat. No. 2G2080, Teknova Inc.). After 5 days growth on a shaker, 1 mL aliquots of the cultures were harvested and analyzed by GC, as described in Example 9.

Based on the fatty acid composition of the 3 mL cultures, selected transformants were further characterized by flask assay. Specifically, clones #5 and #6 of strain Y8406U transformed with expression vector pY222 (comprising ScLPAATS) were selected and designated as "Y8406U::ScLPAATS-5" and "Y8406U::ScLPAATS-6", respectively; clone #1 of strain Y8406U transformed with expression vector pY177 (comprising YILPAAT1) was selected and designated as "Y8406U::YILPAAT1". Additionally, strain Y8406 (a Ura+ strain that was parent to strain Y8406U (Ura-)) was used as a control.

Each selected transformant and the control was streaked onto MM agar plates. Then, one loop of freshly streaked cells was inoculated into 3 mL CSM-U medium and grown overnight at 250 rpm and 30 °C. The OD₆₀₀ₙₘ was measured and an aliquot of the cells were added to a final OD₆₀₀ₙₘ of 0.3 in 25 mL CSM-U medium in a 125 mL flask. After 2 days in a shaker incubator at 250 rpm and at 30 °C, 6 mL of the culture was harvested by centrifugation and resuspended in 25 mL HGM in a 125 mL flask. After 5 days in a shaker incubator at 250 rpm and at 30 °C, a 1 mL aliquot was used for GC analysis and 10 mL dried for dry cell weight ["DCW"] determination, as described in Example 9.

### Lipid Content, Fatty Acid Composition And Conversion Efficiencies

The lipid content, fatty acid composition and EPA as a percent of the DCW are quantified for 2 replicate cultures ["Replicates"] of the control Y8406 strain and the transformant Y8406U strain(s). Additionally, data for each Y8406U transformant is presented as a % of the Y8406 control. Table 30 below summarizes the total lipid content of cells ["TFAs % DCW"], the concentration of each fatty acid as a weight percent of TFAs ["% TFAs"] and the EPA content as a percent of the dry cell weight ["EPA % DCW"]. More specifically, fatty acids are identified as 16:0 (palmitate), 16:1 (palmitoleic acid), 18:0 (stearic acid), 18:1 (oleic acid), 18:2 (LA), ALA, EDA, DGLA, ARA, ETrA, ETA and EPA.

Table 31 summarizes the conversion efficiency of each desaturase and the Δ9 elongase functioning in the PUFA biosynthetic pathway and which are required for EPA production, in a manner identical to that described in Example 9.

**Table 30: Lipid Content And Composition In ScLPAATS and YILPAAT1 Transformant Strains Of Yarrowia lipolytica Y8406**

| **Strain** | **Repli cates** | **TFA % DCW** | **% TFAs** | | | | | | | | | | | | **EPA % DCW** |
|---|---|---|---|---|---|---|---|---|---|---|---|---|---|---|---|
| | | | **16: 0** | **16: 1** | **18: 0** | **18: 1** | **18:2** | **ALA** | **EDA** | **DGLA** | **ARA** | **ERA** | **ETA** | **EPA** | |
| Y8406 | AVG.2 | 22.0 | 2 | 0 | 2 | 4 | 19 | 2 | 3 | 4 | 1 | 2 | 3 | 55 | 12 |
| Y8406U:: YILPAAT1 | AVG.2 | 24.6 | 2 | 1 | 2 | 6 | 14 | 1 | 3 | 5 | 1 | 2 | 3 | 55 | 14 |
| | % Ctrl | 112 | 98 | 153 | 102 | 148 | 76 | 50 | 120 | 144 | 101 | 109 | 123 | 101 | 113 |
| Y8406U:: ScLPAATS-5 | AVG.2 | 21.6 | 3 | 1 | 3 | 6 | 14 | 1 | 3 | 4 | 1 | 2 | 3 | 57 | 12 |
| | % Ctrl | 98 | 131 | 137 | 125 | 131 | 74 | 56 | 100 | 117 | 86 | 101 | 108 | 104 | 102 |
| Y8406U:: ScLPAATS-6 | AVG.2 | 21.4 | 3 | 1 | 3 | 5 | 14 | 1 | 3 | 4 | 1 | 2 | 3 | 58 | 12 |
| | % Ctrl | 97 | 125 | 133 | 121 | 124 | 72 | 52 | 97 | 119 | 88 | 102 | 111 | 106 | 103 |

**Table 31: Desaturase And Elongate Conversion Efficiency In ScLPAATS and YILPAAT1 Transformant Strains Of Yarrowia lipolytica Y8406**

| **Strain** | **Replicates** | **Δ12 CE** | **Δ9e CE** | **Δ8 CE** | **Δ5 CE** | **Δ17 CE** |
|---|---|---|---|---|---|---|
| Y8406 | AVG.2 | 95 | 77 | 92 | 90 | 92 |
| Y8406U::YILPAAT1 | AVG.2 | 93 | 82 | 92 | 87 | 90 |
| | % Ctrl | 98 | 107 | 99 | 97 | 98 |
| Y8406U::ScLPAATS-5 | AVG.2 | 94 | 83 | 93 | 89 | 92 |
| | % Ctrl | 98 | 108 | 100 | 99 | 100 |
| Y8406U::ScLPAATS-6 | AVG.2 | 94 | 83 | 93 | 89 | 92 |
| | % Ctrl | 99 | 109 | 101 | 99 | 100 |

Based on the data in Table 30 and Table 31 above, overexpression of both ScLPAATS and YILPAAT1 in EPA strains Y8406U::YILPAAT1, Y8406U::ScLPAATS-5 and Y8406U::ScLPAATS-6 resulted in reduction (to 76% or below of the control) of the concentration of LA (18:2) as a weight % of TFAs ["LA % TFAs"], and an increase (to at least 7% of the control) in the conversion efficiency of the Δ9 elongase. ScLPAATS and YILPAAT1 have a similar effect on lipid profile.

The results obtained above were then compared to those obtained in Example 9, although different means were utilized to characterize the LPLATs. Specifically, in Example 9, linearized DNA carrying the LPLATs were transformed by chromosomal integration, since the vectors lacked ARS sequences. This resulted in stable integrations and the strains were grown in the relatively rich, non-selective FM growth medium during both preculture and 2 days growth prior to being transferred to HGM.

In Example 11, the functional characterization of YILPAAT1 and ScLPAATS was done on a replicating plasmid. Thus, *Yarrowia lipolytica* strain Y8406 was transformed with circular DNA carrying each LPAAT and ARS sequence. To maintain these plasmids and assay gene expression without integration, it was necessary to grow the transformants on selective medium (i.e., CSM-U medium) during both preculture and 2 days growth prior to being transferred to HGM.

These differences described above can contribute to differences in lipid profile and content, as illustrated by the expression of YILPAAT1 in Examples 9 and 11. The change over control in LA % TFAs, EPA % TFAs, and Δ9 elongase conversion efficiency were 63%, 115%, and 115%, respectively, upon expression of YILPAAT in Example 9, whereas the change over control in LA % TFAs, EPA % TFAs, and Δ9 elongase conversion efficiency were 76%, 101%, and 107%, respectively, upon expression of YILPAAT in Example 11. Thus, the improvements in Δ9 elongation and LC-PUFA biosynthesis in Example 11 are minimized when compared to those observed in Example 9. These differences can be attributed to the "position effects" of chromosomal integration and/or different growth conditions.

Since the improvements in LC-PUFA biosynthesis (measured as reduction in LA % TFAs, increase in EPA % TFAs and increase in Δ9 elongase conversion efficiency) are similar for both ScLPAATS and YILPAAT when transformed in *Yarrowia lipolytica* strain Y8406 on a replicating plasmid, it is anticipated that both LPLAATs will also function similarly when stably integrated into the host chromosome. Thus, ScLPAATS will likely improve the lipid profile in a manner similar to that observed in Example 9, when YILPAAT1 was stably integrated into the host chromosome.

## Claims

1. A recombinant *Yarrowia* sp. host cell that produces an oil comprising:
(i) at least 50 weight percent of eicosapentaenoic acid measured as a weight percent of total fatty acids; and
(ii) a ratio of at least 3.5 of eicosapentaenoic acid, measured as a weight percent of total fatty acids, to linoleic acid, measured as a weight percent of total fatty acids;
wherein said host cell comprises:
(a) at least one multizyme which comprises a polypeptide having at least one delta-9 elongase linked to at least one delta-8 desaturase;
(b) at least one peroxisome biogenesis factor protein whose expression has been down-regulated; and
(c) at least one recombinant construct comprising a non-native chimeric gene comprising a nucleotide sequence encoding a heterologous malonyl-CoA synthetase, and at least one recombinant construct comprising a non-native chimeric gene comprising a nucleotide sequence encoding an acyl-CoA lysophospholipid acyltransferase (LPLAT) such that the LPLAT is overexpressed in the host cell, wherein said chimeric genes comprise regulatory and coding sequences not found together in nature.

2. The recombinant host cell of claim 1, wherein the nucleotide sequence encoding the malonyl CoA synthetase encodes a polypeptide consisting essentially of an amino acid selected from the group consisting of SEQ ID NO:40 and SEQ ID NO:42.

3. The recombinant host cell of claims 1 or 2, wherein the nucleotide sequence encoding the acyl-CoA lysophospholipid acyltransferase encodes a polypeptide consisting essentially of an amino acid sequence selected from the group consisting of SEQ ID NO:15, SEQ ID NO:17, SEQ ID NO:25, SEQ ID NO:29, SEQ ID NO:31 and SEQ ID NO:32.

4. The recombinant host cell of any one of claims 1 to 3 wherein the LPLAT is an LPAAT.

5. The recombinant host cell of claim 4 wherein the LPAAT comprises a sequence of SEQ ID NO:29.

6. The recombinant host cell of any one of claims 1 to 5, wherein the multizyme comprises a linker between the delta-9 elongase and delta-8 desaturase, which linker is selected from the group consisting of: SEQ ID NO:1, SEQ ID NO:2, SEQ ID NO:3, SEQ ID NO:4, SEQ ID NO:5, SEQ ID NO:6 and SEQ ID NO:7.

7. The recombinant host cell of any one of claims 1 to 6, wherein the multizyme comprises a sequence selected from the group consisting of: SEQ ID NO:9, SEQ ID NO:11 and SEQ ID NO:13.

8. The recombinant host cell of any one of claims 1 to 7, wherein the ratio of eicosapentaenoic acid to linoleic acid in the oil is at least 4.5.

9. The recombinant host cell of any one of claims 1 to 7, wherein the cell produces at least 55 weight percent of eicosopentaenoic acid, measured as a weight percentage of total fatty acids.

10. The recombinant host cell of any one of claims 1 to 8, wherein the cell produces at least 60 weight percent of eicosapentaenoic acid, measured as a weight percentage of total fatty acids.

11. A method for making a microbial oil comprising eicosapentaenoic acid comprising:
a) culturing the host cell of any of claims 1-10, wherein a microbial oil comprising eicosapentaenoic acid is produced; and
b) optionally recovering the microbial oil of step (a).

## Patentansprüche

1. Rekombinante *Yarrowia*-sp.-Wirtszelle, die ein Öl erzeugt, umfassend:
i) mindestens 50 Gewichtsprozent von Eicosapentaensäure, gemessen als Gewichtsprozent von Gesamtfettsäuren; und
(ii) ein Verhältnis von mindestens 3,5 von Eicosapentaensäure, gemessen als Gewichtsprozent von Gesamtfettsäuren, zu Linolsäure, gemessen als Gewichtsprozent von Gesamtfettsäuren;
wobei die Wirtszelle umfasst:
(a) mindestens ein Multizym, welches ein Polypeptid, aufweisend mindestens eine delta-9-Elongase, verknüpft mit mindestens einer delta-8-Desaturase, umfasst;
(b) mindestens ein Peroxisom-Biogenese-Faktor-Protein, dessen Expression herabreguliert worden ist; und
(c) mindestens ein rekombinantes Konstrukt, umfassend ein nicht-natives chimäres Gen, umfassend eine Nucleotidsequenz, codierend eine heterologe Malonyl-CoA-Synthetase, und mindestens ein rekombinantes Konstrukt, umfassend ein nicht-natives chimäres Gen, umfassend eine Nucleotidsequenz, codierend eine Acyl-CoA-Lysophospholipid-Acyltransferase (LPLAT), derart, dass die LPLAT in der Wirtszelle überexprimiert wird, wobei die chimären Gene regulatorische und codierende Sequenzen, nicht zusammen in der Natur gefunden, umfassen.

2. Rekombinante Wirtszelle nach Anspruch 1, wobei die Nucleotidsequenz, codierend die Malonyl-CoA-Synthetase, ein Polypeptid, bestehend im Wesentlichen aus einer Aminosäure, ausgewählt aus der Gruppe, bestehend aus SEQ ID NO:40 und SEQ ID NO:42, codiert.

3. Rekombinante Wirtszelle nach den Ansprüchen 1 oder 2, wobei die Nucleotidsequenz, codierend die Acyl-CoA-Lysophospholipid-Acyltransferase, ein Polypeptid, bestehend im Wesentlichen aus einer Aminosäuresequenz, ausgewählt aus der Gruppe, bestehend aus SEQ ID NO:15, SEQ ID NO:17, SEQ ID NO:25, SEQ ID NO:29, SEQ ID NO:31 und SEQ ID NO:32, codiert.

4. Rekombinante Wirtszelle nach einem der Ansprüche 1 bis 3, wobei die LPLAT eine LPAAT ist.

5. Rekombinante Wirtszelle nach Anspruch 4, wobei die LPAAT eine Sequenz von SEQ ID NO:29 umfasst.

6. Rekombinante Wirtszelle nach einem der Ansprüche 1 bis 5, wobei das Multizym einen Linker zwischen der delta-9-Elongase und delta-8-Desaturase umfasst, welcher Linker aus der Gruppe, bestehend aus: SEQ ID NO:1, SEQ ID NO:2, SEQ ID NO:3, SEQ ID NO:4, SEQ ID NO:5, SEQ ID NO:6 und SEQ ID NO:7, ausgewählt ist.

7. Rekombinante Wirtszelle nach einem der Ansprüche 1 bis 6, wobei das Multizym eine Sequenz, ausgewählt aus der Gruppe, bestehend aus: SEQ ID NO:9, SEQ ID NO:11 und SEQ ID NO:13, umfasst.

8. Rekombinante Wirtszelle nach einem der Ansprüche 1 bis 7, wobei das Verhältnis von Eicosapentaensäure zu Linolsäure in dem Öl mindestens 4,5 beträgt.

9. Rekombinante Wirtszelle nach einem der Ansprüche 1 bis 7, wobei die Zelle mindestens 55 Gewichtsprozent von Eicosapentaensäure, gemessen als ein Gewichtsprozentsatz von Gesamtfettsäuren, erzeugt.

10. Rekombinante Wirtszelle nach einem der Ansprüche 1 bis 8, wobei die Zelle mindestens 60 Gewichtsprozent von Eicosapentaensäure, gemessen als ein Gewichtsprozentsatz von Gesamtfettsäuren, erzeugt.

11. Verfahren zum Herstellen eines mikrobiellen Öls, umfassend Eicosapentaensäure, umfassend:
a) Kultivieren der Wirtszelle nach einem der Ansprüche 1-10, wobei ein mikrobielles Öl, umfassend Eicosapentaensäure, erzeugt wird; und
b) gegebenenfalls Gewinnen des mikrobiellen Öls von Schritt (a).

## Revendications

1. Cellule hôte de *Yarrowia* sp. recombinante qui produit une huile comprenant:
(i) au moins 50 pour-cent en poids d'acide eicosapentaénoïque mesuré sous forme de pourcentage en poids de total d'acides gras; et
(ii) un rapport d'au moins 3,5 de l'acide eicosapentaénoïque, mesuré sous forme de pourcentage en poids de total d'acides gras, sur l'acide linoléique, mesuré sous forme de pourcentage en poids de total d'acides gras;
où ladite cellule hôte comprend:
(a) au moins une multienzyme qui comprend un polypeptide possédant au moins une delta-9 élongase liée à au moins une delta-8 désaturase;
(b) au moins une protéine de facteur de biogenèse de peroxysomes dont l'expression a été sous-régulée; et
(c) au moins une construction recombinante comprenant un gène non naturel chimérique comprenant une séquence de nucléotides codant pour une malonyl-CoA synthétase hétérologue et au moins une construction recombinante comprenant un gène non naturel chimérique comprenant une séquence de nucléotides codant pour une acyl-CoA lysophospholipide acyltransférase (LPLAT) de sorte que la LPLAT est surexprimée dans la cellule hôte, où lesdits gènes chimériques comprennent des séquences régulatrices et de codage non trouvées ensemble dans la nature.

2. Cellule hôte recombinante selon la revendication 1, dans laquelle la séquence de nucléotides codant pour la malonyl-CoA synthétase code pour un polypeptide constitué essentiellement d'un acide aminé choisi dans le groupe constitué de la SEQ ID NO:40 et la SEQ ID NO:42.

3. Cellule hôte recombinante selon la revendication 1 ou 2, dans laquelle la séquence de nucléotides codant pour l'acyl-CoA lysophospholipide acyltransférase code pour un polypeptide constitué essentiellement d'une séquence d'acides aminés choisie dans le groupe constitué de la SEQ ID NO:15, la SEQ ID NO:17, la SEQ ID NO:25, la SEQ ID NO:29, la SEQ ID NO:31 et la SEQ ID NO:32.

4. Cellule hôte recombinante selon l'une quelconque des revendications 1 à 3, dans laquelle la LPLAT est une LPAAT.

5. Cellule hôte recombinante selon la revendication 4, dans laquelle la LPAAT comprend une séquence de la SEQ ID NO:29.

6. Cellule hôte recombinante selon l'une quelconque des revendications 1 à 5, dans laquelle la multienzyme comprend un lieur entre la delta-9 élongase et la delta-8 désaturase, lequel lieur est choisi dans le groupe constitué de: la SEQ ID NO:1, la SEQ ID NO:2, la SEQ ID NO:3, la SEQ ID NO:4, la SEQ ID NO:5, la SEQ ID NO:6 et la SEQ ID NO:7.

7. Cellule hôte recombinante selon l'une quelconque des revendications 1 à 6, dans laquelle la multienzyme comprend une séquence choisie dans le groupe constitué de: la SEQ ID NO:9, la SEQ ID NO:11 et la SEQ ID NO:13.

8. Cellule hôte recombinante selon l'une quelconque des revendications 1 à 7, dans laquelle le rapport de l'acide eicosapentaénoïque sur l'acide linoléique dans l'huile est d'au moins 4,5.

9. Cellule hôte recombinante selon l'une quelconque des revendications 1 à 7, où la cellule produit au moins 55 pour-cent en poids d'acide eicosapentaénoïque, mesuré sous forme de pourcentage en poids de total d'acides gras.

10. Cellule hôte recombinante selon l'une quelconque des revendications 1 à 8, où la cellule produit au moins 60 pour-cent en poids d'acide eicosapentaénoïque, mesuré sous forme de pourcentage en poids de total d'acides gras.

11. Méthode pour la fabrication d'une huile microbienne comprenant de l'acide eicosapentaénoïque comprenant:
a) la culture de la cellule hôte selon l'une quelconque des revendications 1-10, dans laquelle une huile microbienne comprenant de l'acide eicosapentaénoïque est produite; et
b) optionnellement, la récupération de l'huile microbienne de l'étape (a).
